(19) Europäisches Patentamt
European Patent Office
Office européen des brevets

(11) EP 4 711 366 A1

(12) EUROPEAN PATENT APPLICATION
published in accordance with Art. 153(4) EPC

(43) Date of publication:
**18.03.2026 Bulletin 2026/12**

(21) Application number: **24803086.8**

(22) Date of filing: **10.05.2024**

(51) International Patent Classification (IPC):
***C07D 487/04*** (2006.01) ***C07D 487/14*** (2006.01)
***A61K 31/519*** (2006.01) ***A61K 31/4162*** (2006.01)
***A61P 35/00*** (2006.01) ***A61P 37/00*** (2006.01)

(52) Cooperative Patent Classification (CPC):
**A61K 31/519; A61P 35/00; C07D 471/04; C07D 487/04; C07D 498/04**

(86) International application number:
**PCT/CN2024/092244**

(87) International publication number:
**WO 2024/230807 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.05.2023 PCT/CN2023/093349**

(71) Applicant: **Mindrank AI Ltd.**
**Hangzhou, Zhejiang 310018 (CN)**

(72) Inventors:
- **ZHANG, Long**
  **Hangzhou, China (Zhejiang) Pilot Free Trade Zone,**
  **Hangzhou, Zhejiang 310018 (CN)**
- **NIU, Zhangming**
  **Hangzhou, China (Zhejiang) Pilot Free Trade Zone,**
  **Hangzhou, Zhejiang 310018 (CN)**

(74) Representative: **Monteiro Alves, Inês**
**Alameda Dos Oceanos, Nº 41K-21**
**Parque das Nações**
**1990-207 Lisboa (PT)**

# (54) PYRIMIDO MACROHETEROCYCLIC COMPOUND AS WEE1-YES DUAL-TARGET INHIBITOR AND USE THEREOF

(57) Disclosed in the present invention for the first time is a novel pyrimido macroheterocyclic compound having a WEE1 and Yes dual-target inhibitory activity. Specifically disclosed are a compound having a structure as shown in formula (I) as a WEE1 and YES dual-target inhibitor or a pharmaceutically acceptable salt, solvate, hydrate, or isotope substitute thereof, or an isomer thereof and a polymorph thereof.

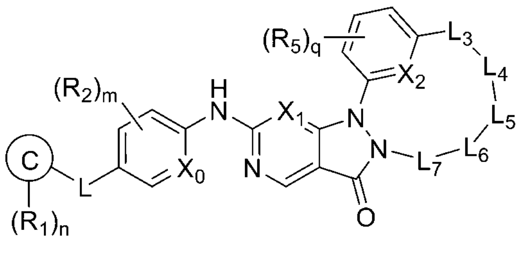

Formula (I)

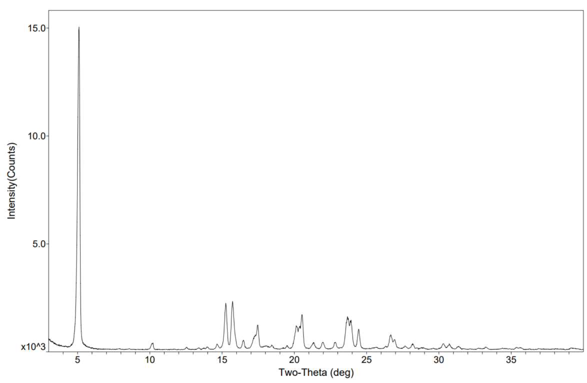


FIG. 1

EP 4 711 366 A1

## Description

The present disclosure claims priority of an International Application No. PCT/CN2023/093349 entitled "NOVEL PYRIMIDO-HETEROCYCLIC COMPOUND AS WEE1 INHIBITOR AND USE THEREOF", and filed with the International Bureau of the World Intellectual Property Organization on May 10, 2023.

## TECHNICAL FIELD

The present disclosure pertains to the field of medical chemistry and particularly discloses, for the first time worldwide, novel pyrimido-macroheterocyclic compounds capable of simultaneously inhibiting the WEE1 and Yes targets, compositions containing such compounds, and a method for manufacturing a medicament for the treatment or prevention of diseases associated with the WEE1 and/or Yes targets by using such compounds.

## BACKGROUND

The cell cycle is a highly regulated and controlled process. A normal cell cycle has checkpoints in the G1/S, S, and G2/M phases to allow sufficient time for DNA damage repair. Normally, after DNA damage occurs in a cell, TP53 can perform checks in the G1/S phase of the cell, and the cell cannot enter the G2 phase until the damaged DNA is repaired. If TP53 is dysfunctional and cannot perform checks in the G1/S phase as it normally does, the cell enters the G2 phase, carrying the damaged DNA. WEE1, a member of the serine/threonine protein kinase family, plays an important regulatory role at the checkpoint of the G2/M phase of the cell cycle. The WEE1 kinase inhibits the activity of CDKs by phosphorylating the Tyr14 and Tyr15 of the CDKs, arresting the cell cycle in the G2/M phase, thereby allowing sufficient time for DNA damage repair in the cell. Inhibition of WEE1 eliminates the cell cycle arrest and causes the cell to enter mitosis prematurely, which leads to cell replication stress and improper mitotic progression.

WEE1 is overexpressed in many types of cancer, including breast cancer, lung cancer, cervical cancer, head and neck cancer, ovarian cancer, prostate cancer, melanoma, leukemia, glioblastoma, medulloblastoma, liver cancer, and the like. The overexpression of WEE1 in cancer cells is a normal response to the elevated replication stress in cancer cells and has some correlation with tumor progression and low disease-free survival rates. Therefore, WEE1 inhibition has become a very promising cancer therapy, especially in conjunction with DNA damage treatment. Numerous studies have demonstrated that WEE1 and TP53 exhibit a synthetic lethal relationship. In tumor cells in which TP53 is inactivated due to mutation, the loss of the checking function of the G1/S phase makes the tumor cells highly dependent on the checks of the G2/M phase. In tumor cells lacking TP53 function, inhibiting WEE1 function disrupts damage repair, leading to the accumulation of errors and eventual apoptosis, thereby achieving an inhibitory effect on tumors. Therefore, WEE1 inhibitors have great synthetic lethal therapeutic value in cancers in which TP53 is mutated.

Oral small molecule WEE1 inhibitors have been reported in some literature or patent publications, and some candidate compounds have entered clinical trials, for example, ZN-c3, AZD-1775, IMP 7068, Debio 0123, and the like. However, single-target Wee1 selective inhibitors generally show relatively poor efficacy in inhibiting tumor proliferation, and the majority of the clinical candidate molecules generally face relatively significant challenges regarding druggability, such as relatively low efficacy, poor selectivity, rapid metabolism, poor oral absorption, low bioavailability, narrow safety windows, and the like.

Yes-associated protein (YAP) is a major transcriptional coactivator in the Hippo pathway. It binds to the transcription factor TEAD to regulate downstream targets related to cell proliferation and survival. Dysregulated YAP can cause metastasis and poor prognosis of aggressive tumors, including pancreatic cancer, gastric cancer, lung cancer, and the like. Therefore, Yes target inhibitors are likely to become a good tumor treatment strategy.

Studies have shown that both Yes and Wee1 are co-overexpressed in many malignant tumors, such as pancreatic cancer, small-cell lung cancer, gastric cancer, bowel cancer, ovarian cancer, or the like, and play significant roles in the proliferation, invasion, and metastasis of these malignant tumors. This suggests that inhibitors that simultaneously inhibit the Yes and Wee1 targets can have better efficacy. However, up to the filing date of this application, compounds capable of simultaneously inhibiting the Yes and Wee1 targets have not been reported in literature or patent publications worldwide.

### Technical Effects

The inventors have unexpectedly discovered for the first time that some of the novel pyrimido-macroheterocyclic compounds of the structure of formula (I) of the present disclosure can simultaneously inhibit the Yes and Wee1 targets. Compared to structurally known single-target Wee1 selective compounds, such as DEBIO0123, ZN-c3, NUV-569, or the like, these novel compounds have stronger anti-tumor activity and good PK profiles and are more suitable as candidate drugs for the development of treatments for preventing or treating tumors associated with the WEE1 and/or Yes target(s) or

signaling pathway(s).

## SUMMARY

An object of the present disclosure is to provide a compound represented by formula (I), a pharmaceutically acceptable salt, a solvate, an enantiomer, an isotopically substituted compound, or a polymorph thereof:

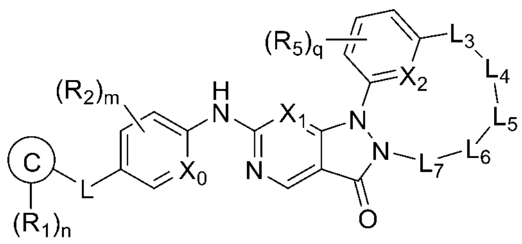

formula (I),

wherein

$X_0$, $X_1$, and $X_2$ are each independently selected from $-C(R_x)-$ and -N-;

$L_3$, $L_4$, $L_5$, $L_6$, and $L_7$ are identical or different and are each independently absent, or selected from single bond, double bond, alkyne bond, $-C(R_{d1})(R_{d2})-$, $-C(R_{d1})(R_{d2})C(R_{d1})(R_{d2})-$, $-OC(R_{d1})(R_{d2})-$, $-C(R_{d1})(R_{d2})O-$, $-C(=O)N(R_{d3})-$, $-N(R_{d3})C(=O)-$, $-N(R_{d3})-$, $-C(=NR_{d3})-$, $-S(=O)_2N(R_{d3})-$, $-N(R_{d3})S(=O)_2-$, $-C(R_{d1})(R_{d2})N(R_{d3})-$, $-N(R_{d3})C(R_{d1})(R_{d2})-$, -O-, -S-, -C(=O)O-, -OC(=O)-, -C(=O)-, -C(=S)-, -N-, -CH-, -S(=O)-, and $-S(=O)_2-$;

L is absent or is selected from O, S, $CH_2$, C(O), S(O), $S(O)_2$, NH, C(O)NH, and NHC(O); ring C is a heterocyclyl unsubstituted or substituted with $R_0$;

$R_0$ is independently selected from H, deuterium, halogen, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein a hydrogen or hydrogens on the aryl, saturated or partially saturated cycloalkyl, or heterocycloalkyl is/are optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, =O, and saturated or partially saturated $C_{3-6}$ cycloalkyl, wherein $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl;

$R_1$, $R_{d1}$, $R_{d2}$, and $R_x$ are independently selected from hydrogen, deuterium, halogen, cyano, oxo (=O), amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, and $C_{1-6}$ alkyl and $C_{1-6}$ alkoxy are optionally further substituted with one or more groups selected from

hydrogen, deuterium, halogen, oxo, CN, $CF_3$, OH, $OCH_3$, $OCH_2CH_3$, and saturated or partially saturated $C_{3-6}$ cycloalkyl; or $R_{d1}$ and $R_{d2}$, or $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;
$R_{d3}$ is optionally and independently selected from hydrogen, $NH_2$, $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, and $C_{3-10}$ heterocyclyl substituted with $C_{3-10}$ cycloalkyl or $C_{3-10}$ heterocycloalkyl, wherein the $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, or $C_{3-10}$ heterocyclyl substituted with $C_{3-10}$ cycloalkyl or $C_{3-10}$ heterocycloalkyl is further optionally substituted with one or more substituents selected from hydrogen, deuterium, halogen, oxo, CN, OH, and $C_{3-10}$ saturated or partially saturated cycloalkyl or heterocyclyl;
the hetero represents any heteroatom optionally and independently selected from O, N, S, P, S=O, and $S(=O)_2$ and isotopes thereof;
the halogen is optionally and independently selected from F, Cl, Br, and I and isotopes thereof; m is an integer optionally selected from 0, 1, 2, 3, and 4;
n is an integer optionally selected from 0, 1, 2, 3, 4, and 5; and
q is an integer optionally selected from 0, 1, 2, and 3.

According to an embodiment of the present disclosure, $X_0$, $X_1$, and $X_2$ are each independently selected from $-C(R_x)-$ and -N-, wherein $R_x$ is selected from H and $C_{1-6}$ alkyl (e.g., methyl).

According to an embodiment of the present disclosure, $X_0$ is -CH-.

According to an embodiment of the present disclosure, $X_1$ is N.

According to an embodiment of the present disclosure, $X_2$ is N.

According to an embodiment of the present disclosure, L is selected from O, S, and NH. According to an embodiment of the present disclosure, $L_3$, $L_4$, $L_5$, $L_6$, and $L_7$ are identical or different and are independently absent, or selected from single bond, and the following groups unsubstituted or optionally substituted with one, two, or more $R_{d1}$: $C_{1-6}$ alkylene and $C_{2-6}$ alkenylene;

According to an embodiment of the present disclosure, $L_3$, $L_4$, $L_5$, $L_6$, and $L_7$ are identical or different and are independently absent, or selected from single bond, and the following groups unsubstituted or optionally substituted with one, two, or more $R_{d1}$: $CH_2$, $CH_2CH_2$, $CH_2CH_2CH_2$, CH=CH, and $CH=CHCH_2$.

According to an embodiment of the present disclosure, $L_3$ is $-C(R_{d1})(R_{d1})-$, preferably - $C(CH_3)(OH)-$.

According to an embodiment of the present disclosure, $L_6$ is vinylene, preferably cis-vinylene. According to an embodiment of the present disclosure, $L_4$, $L_5$, and $L_7$ are identical or different and are independently $CH_2$.

According to an embodiment of the present disclosure, each $R_{d1}$ is identical or different and is independently selected from H, OH, and $C_{1-6}$ alkyl (e.g., methyl).

According to an embodiment of the present disclosure, ring C is a heterocyclyl unsubstituted or substituted with $R_0$, wherein the heterocyclyl may be selected from piperidyl (e.g.,

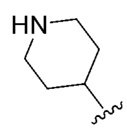

), piperazinyl (e.g.,

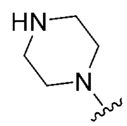

), morpholinyl (e.g.,

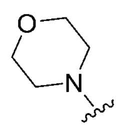

), azetidinyl (e.g.,

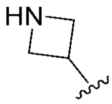

), tetrahydropyrrolidinyl (e.g.,

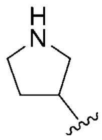 ), 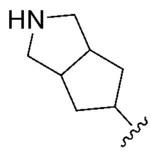 , 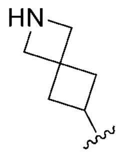 , 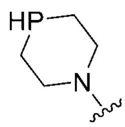 ,

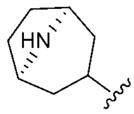 , and 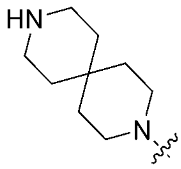 .

According to an embodiment of the present disclosure,

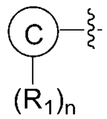

is selected from

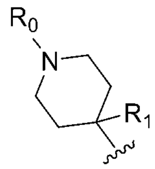 ,

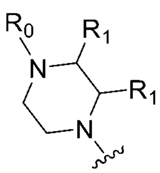 , 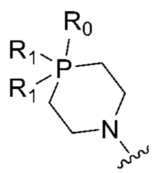 , 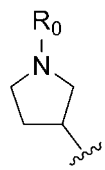 , 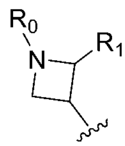 , 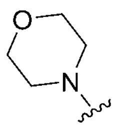 , 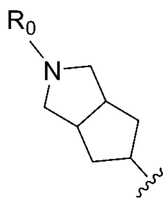 , 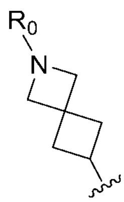 ,

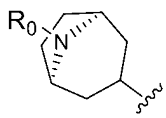 , and 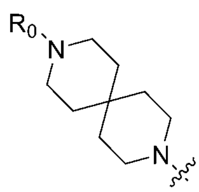 .

According to an embodiment of the present disclosure,

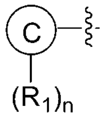

is selected from

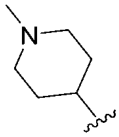,

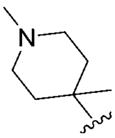, 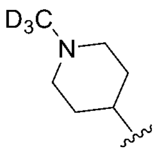, 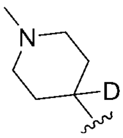, 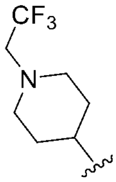, 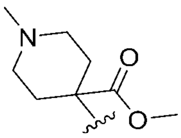, 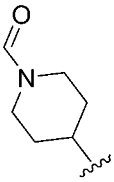, 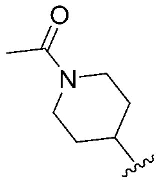,

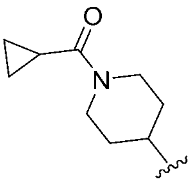, 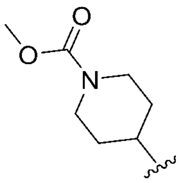, 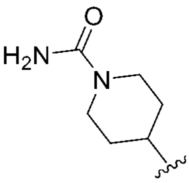, 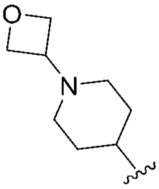, 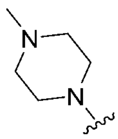, 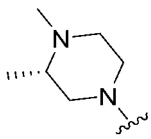,

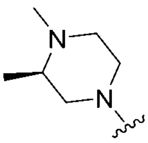, 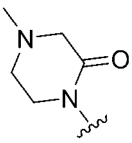, 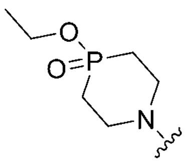, 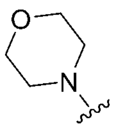, 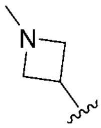, 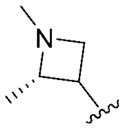, 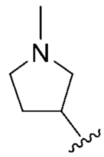,

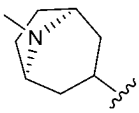, 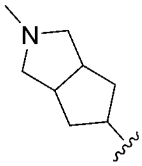, 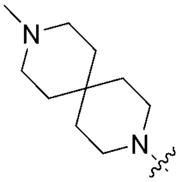, and 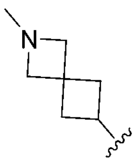.

According to an embodiment of the present disclosure, $R_0$ is selected from H, $C_{1-10}$ alkyl, $C_{1-10}$ alkoxy, deuterated $C_{1-10}$ alkyl, $C_{1-10}$ haloalkyl, HC(O)-, $NH_2C(O)$-, $C_{1-10}$ alkyl-C(O)-, $C_{1-10}$ alkoxy-C(O)-, $C_{3-8}$ cycloalkyl-C(O)-, and 3- to 8-membered heterocyclyl.

Preferably, $R_0$ is selected from H, $C_{1-6}$ alkyl, $C_{1-6}$ alkoxy, deuterated $C_{1-6}$ alkyl, $C_{1-6}$ haloalkyl, HC(O)-, $NH_2C(O)$-, $C_{1-6}$ alkyl-C(O)-, $C_{1-6}$ alkoxy-C(O)-, $C_{3-6}$ cycloalkyl-C(O)-, and 3- to 6-membered heterocyclyl.

According to an embodiment of the present disclosure, $R_0$ is selected from H, methyl, ethoxy, trideuterated methyl, $CF_3CH_2$-, HC(O)-, $CH_3C(O)$-,

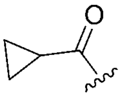, 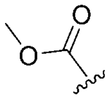, 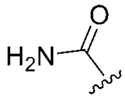,

and

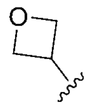

.

According to an embodiment of the present disclosure, each $R_1$ is identical or different and is independently selected from H, deuterium, oxo (=O), $C_{1-6}$ alkyl, $C_{1-6}$ alkoxy, $C_{1-6}$ alkoxy-C(O)-, $C_{1-6}$ haloalkyl, $C_{1-6}$ haloalkoxy, $C_{1-6}$ alkylamino, $C_{6-10}$ aryl, and 5- to 10-membered heteroaryl. Preferably, each $R_1$ is identical or different and is independently selected from H, deuterium, oxo (=O), $C_{1-6}$ alkyl, and $C_{1-6}$ alkoxy-C(O)-.

According to an embodiment of the present disclosure, each $R_1$ is identical or different and is independently selected from H, methyl, deuterium, oxo (=O), and

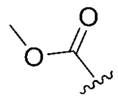

.

According to an embodiment of the present disclosure, each $R_2$ is identical or different and is independently selected from H, deuterium, oxo (=O), $C_{1-6}$ alkyl, $C_{1-6}$ alkoxy, $C_{1-6}$ alkoxy-C(O)-, $C_{1-6}$ haloalkyl, $C_{1-6}$ haloalkoxy, $C_{1-6}$ alkylamino, $C_{6-10}$ aryl, and 5- to 10-membered heteroaryl. According to an embodiment of the present disclosure, each $R_2$ is identical or different and is independently selected from H and $C_{1-6}$ alkyl (e.g., methyl).

According to an embodiment of the present disclosure, each $R_5$ is identical or different and is independently selected from H, deuterium, oxo (=O), $C_{1-6}$ alkyl, $C_{1-6}$ alkoxy, $C_{1-6}$ alkoxy-C(O)-, $C_{1-6}$ haloalkyl, $C_{1-6}$ haloalkoxy, $C_{1-6}$ alkylamino, $C_{6-10}$ aryl, and 5- to 10-membered heteroaryl. According to an embodiment of the present disclosure, each $R_5$ is identical or different and is independently selected from H and $C_{1-6}$ alkyl (e.g., methyl).

In one embodiment of the present disclosure, the compound, the pharmaceutically acceptable salt, the solvate, the enantiomer, the isotopically substituted compound, or the polymorph thereof has a structure of formula (II):

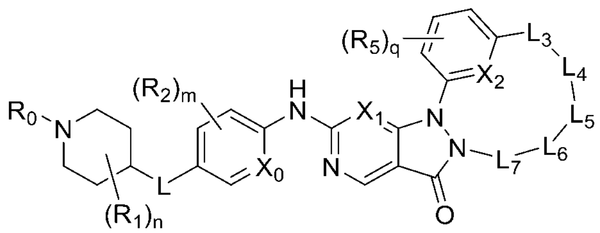

(II),

wherein L, $L_3$, $L_4$, $L_5$, $L_6$, $L_7$, $X_0$, $X_1$, $X_2$, $R_0$, $R_1$, $R_2$, $R_5$, m, n, and q are independently defined as described above.

According to an embodiment of the present disclosure, $X_0$, $X_1$, and $X_2$ are each independently selected from $-C(R_x)-$ and -N-;

$L_3$, $L_4$, $L_5$, $L_6$, and $L_7$ are independently absent, or selected from single bond, double bond, alkyne bond, $-C(R_{d1})(R_{d2})-$, $-C(R_{d1})(R_{d2})C(R_{d1})(R_{d2})-$, $-OC(R_{d1})(R_{d2})-$, $-C(R_{d1})(R_{d2})O-$, - C(=O)N($R_{d3}$)-, -N($R_{d3}$)C(=O)-, -N($R_{d3}$)-, -C(=N$R_{d3}$)-, $-S(=O)_2N(R_{d3})-$, $-N(R_{d3})S(=O)_2-$, - $C(R_{d1})(R_{d2})N(R_{d3})-$, $-N(R_{d3})C(R_{d1})(R_{d2})-$, -O-, -S-, -C(=O)O-, -OC(=O)-, -C(=O)-, -C(=S)-, - N-, -CH-, -S(=O)-, and $-S(=O)_2-$;

L is optionally and independently selected from O, S, and NH;

$R_0$ is independently selected from H, deuterium, halogen, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring

structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein a hydrogen or hydrogens on the aryl, saturated or partially saturated cycloalkyl, or heterocycloalkyl is/are optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, =O, and saturated or partially saturated $C_{3-6}$ cycloalkyl, and $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl;

$R_1$, $R_{d1}$, $R_{d2}$, and $R_x$ are independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, and $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or $R_{d1}$ and $R_{d2}$, or $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

$R_{d3}$ is optionally and independently selected from hydrogen, $NH_2$, $C_{1-10}$ alkyl, $C_{2-10}$ alkynyl or $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, and $C_{3-10}$ heterocyclyl substituted with $C_{3-10}$ cycloalkyl or $C_{3-10}$ heterocycloalkyl, wherein the $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, or $C_{3-10}$ heterocyclyl substituted with $C_{3-10}$ cycloalkyl or $C_{3-10}$ heterocycloalkyl is further optionally substituted with one or more substituents selected from hydrogen, deuterium, halogen, oxo, CN, OH, and $C_{3-10}$ saturated or partially saturated cycloalkyl or heterocyclyl;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5; and

q is an integer optionally selected from 0, 1, 2, and 3.

In one embodiment of the present disclosure, the compound, the pharmaceutically acceptable, the isotopically substituted compound, or the isomer thereof has a structure of formula (IA):

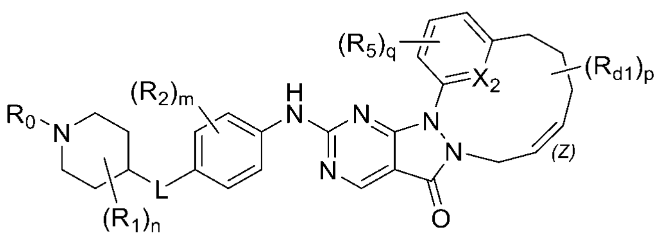

formula (IA),

wherein L, $X_2$, $R_0$, $R_1$, $R_2$, $R_5$, $R_{d1}$, m, n, and q are independently defined as described above, and p is an integer optionally selected from 0, 1, 2, 3, and 4.

According to an embodiment of the present disclosure, $X_2$ is independently selected from $C(R_x)$- and -N-;

L is optionally and independently selected from O, S, and NH;

$R_0$ is independently selected from H, deuterium, halogen, alkyl, haloalkyl, deuterated alkyl, cycloalkyl, heterocyclyl,

alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein a hydrogen or hydrogens on the aryl, saturated or partially saturated cycloalkyl, or heterocycloalkyl is/are optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}\ alkyl)_2$, =O, and saturated or partially saturated $C_{3-6}$ cycloalkyl, wherein $C_{1-6}$ alkyl and $C_{1-6}$ alkoxy are optionally substituted with or more groups selected from hydrogen, deuterium, halogen, oxo, CN, $CF_3$, OH, $OCH_3$, $OCH_2CH_3$, and saturated or partially saturated $C_{3-6}$ cycloalkyl;

$R_1$, $R_{d1}$, and $R_x$ are independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}\ alkyl)_2$, oxy, and saturated or partially saturated $C_{3-6}$ cycloalkyl, wherein $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or optionally, $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5;

q is an integer optionally selected from 0, 1, 2, and 3; and

p is an integer optionally selected from 0, 1, 2, 3, and 4.

In one embodiment of the present disclosure, the compound, the pharmaceutically acceptable salt, the isotopically substituted compound, or the isomer thereof has a structure of formula (IB):

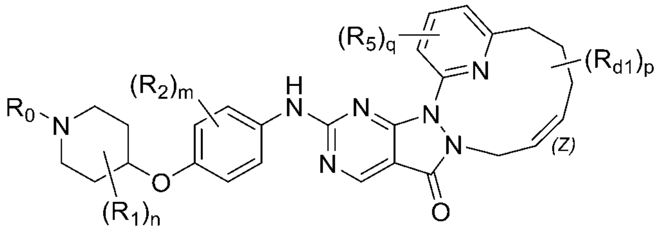

formula (IB),

wherein $R_0$, $R_1$, $R_2$, $R_5$, $R_{d1}$, m, n, and q are independently defined as described above.

According to an embodiment of the present disclosure, $R_0$ is independently selected from H, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein a hydrogen or hydrogens on the aryl, saturated or partially saturated cycloalkyl, or heterocycloalkyl is/are optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}\ alkyl)_2$, =O, and saturated or partially saturated $C_{3-6}$ cycloalkyl, wherein $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl;

$R_1$ and $R_{d1}$ are independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}\ alkyl)_2$, oxy, and saturated or partially saturated $C_{3-6}$ cycloalkyl, wherein $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or optionally, $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5;

q is an integer optionally selected from 0, 1, 2, and 3; and

p is an integer optionally selected from 0, 1, 2, 3, and 4.

In one embodiment of the present disclosure, the compound, the pharmaceutically acceptable salt, the isotopically substituted compound, or the isomer thereof has a structure of formula (IC):

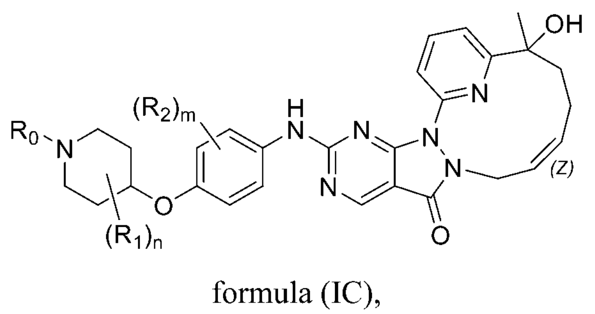

formula (IC),

wherein $R_0$, $R_1$, $R_2$, m, and n are independently defined as described above.

According to an embodiment of the present disclosure, $R_0$ is independently selected from H, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$, together with the carbon atoms to which they are attached on the ring form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

$R_1$ is independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, wherein $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or optionally, $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4; and

n is an integer optionally selected from 0, 1, 2, 3, and 4.

In one embodiment of the present disclosure, the compound, the pharmaceutically acceptable salt, the isotopically substituted compound, or the isomer thereof has a structure of formula (ID):

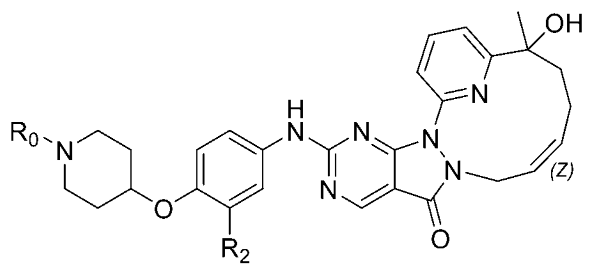

formula (ID),

wherein $R_0$ and $R_2$ are independently defined as described above.

According to an embodiment of the present disclosure, $R_0$ is independently selected from H, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

$R_2$ is independently selected from hydrogen, deuterium, halogen, -CN, -OH, -SH, $-NH_2$, - COOH, $C_{1-10}$ alkyl, $C_{2-10}$

alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$, together with the carbon atoms to which they are attached on the ring form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

the hetero represents any heteroatom optionally and independently selected from O, N, S, P, S=O, and $S(=O)_2$ and isotopes thereof; and

the halogen is optionally and independently selected from F, Cl, Br, and I and isotopes thereof.

In one embodiment of the present disclosure, the compound, the pharmaceutically acceptable salt, the isotopically substituted compound, or the isomer thereof has a structure of formula (III):

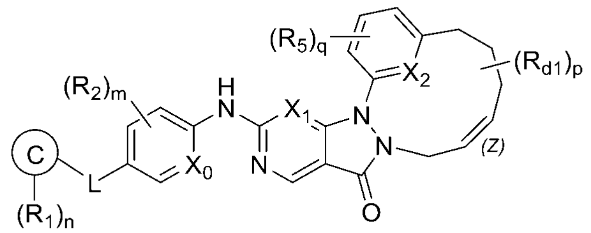

(III),

wherein ring C, L, $X_0$, $X_1$, $X_2$, $R_1$, $R_2$, $R_5$, $R_{d1}$, m, n, and q are independently defined as described above.

In one embodiment of the present disclosure, the compound, the pharmaceutically acceptable salt, the isotopically substituted compound, or the isomer thereof has a structure of formula (IIIA):

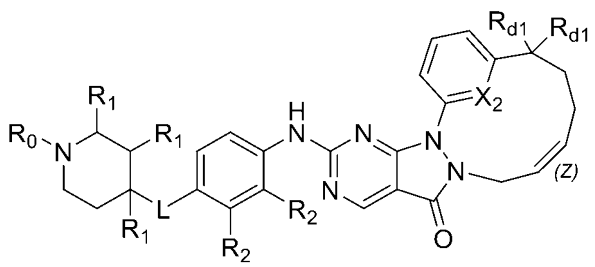

(IIIA),

wherein L, $X_2$, $R_0$, $R_1$, $R_2$, and $R_{d1}$ are independently defined as described above.

In one embodiment of the present disclosure, the compound, the pharmaceutically acceptable salt, the isotopically substituted compound, or the isomer thereof has the structures shown below:

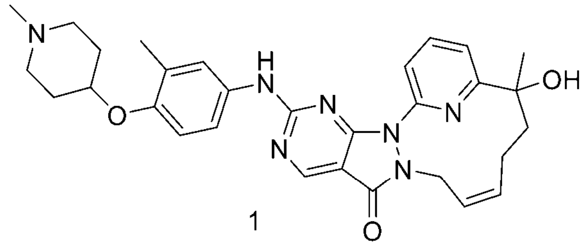

1

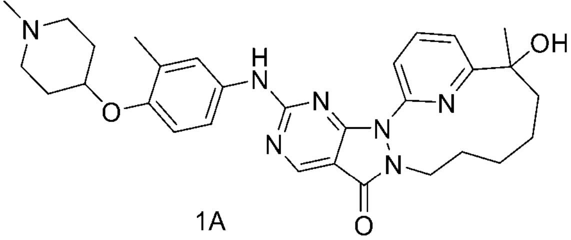

1A

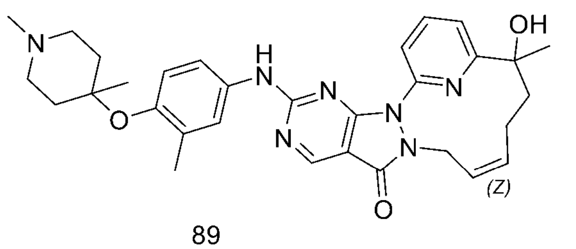

89

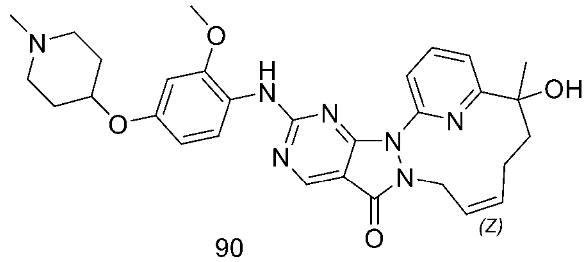

90

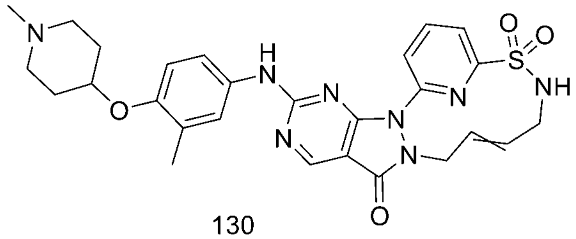

130

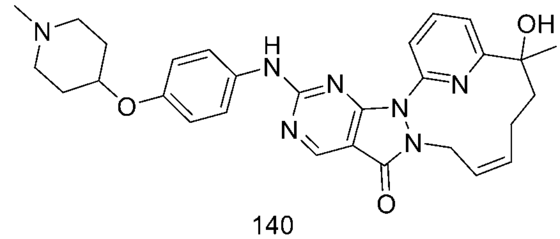

140

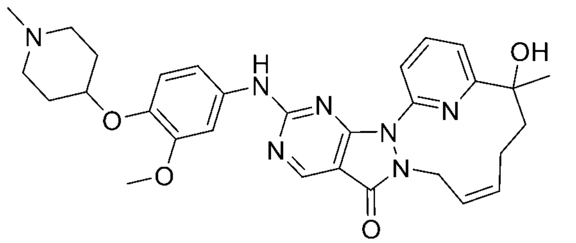

141

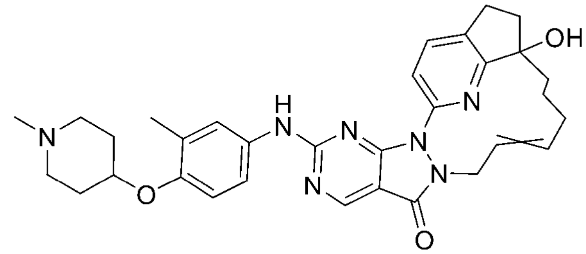

146

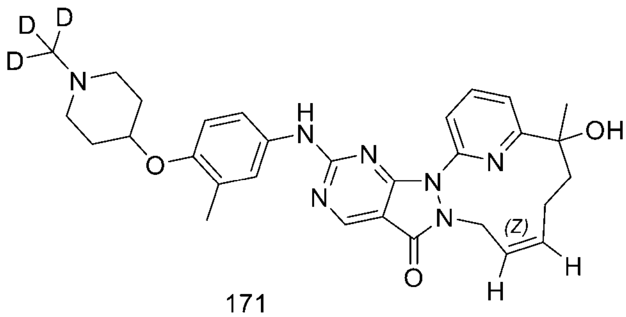

171

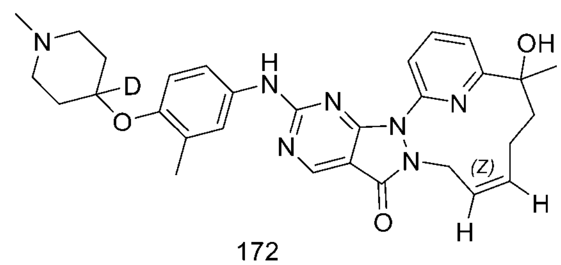

172

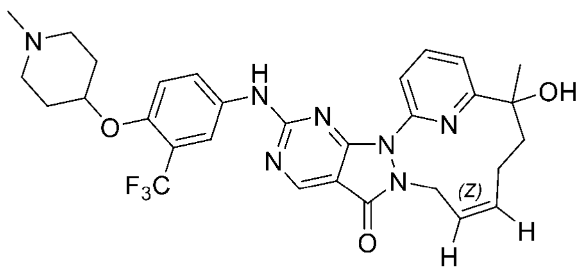

173

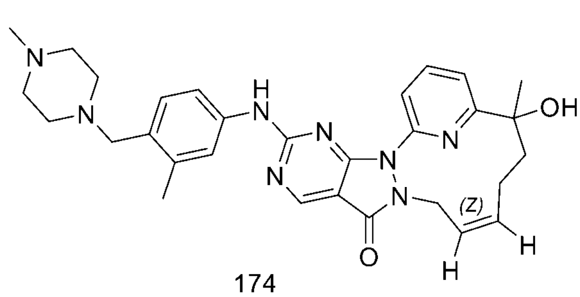

174

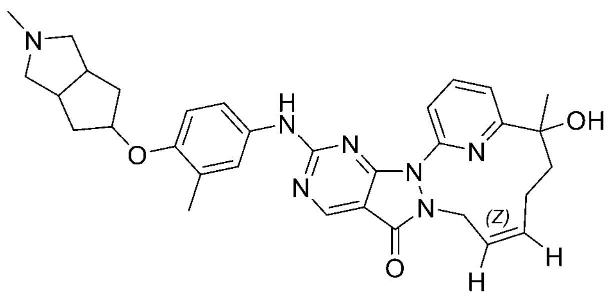

175

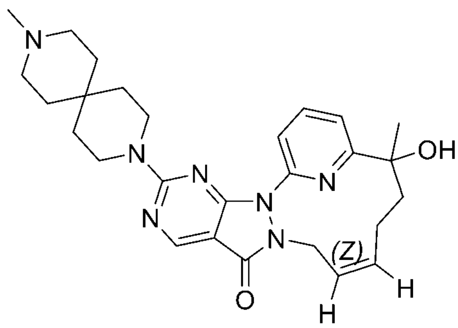

176

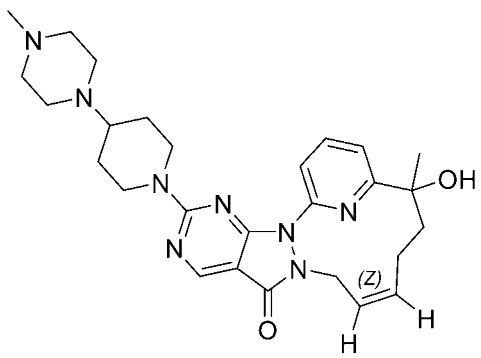

177

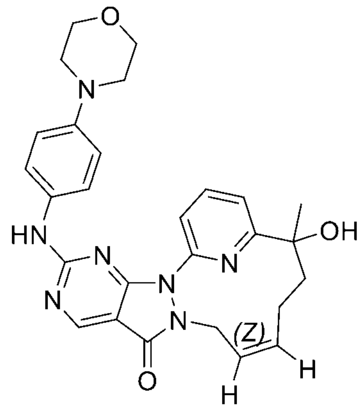

178

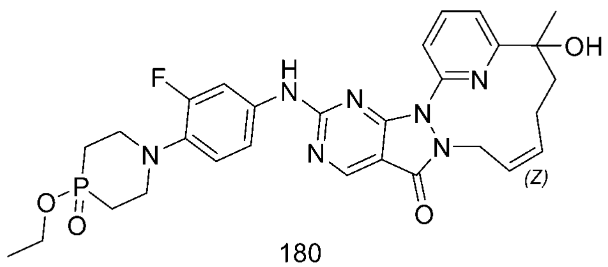

180

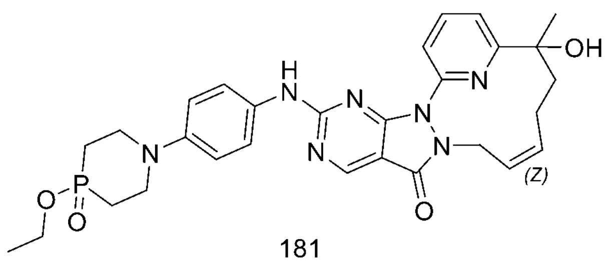

181

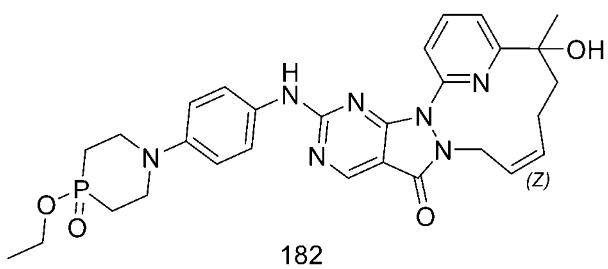

182

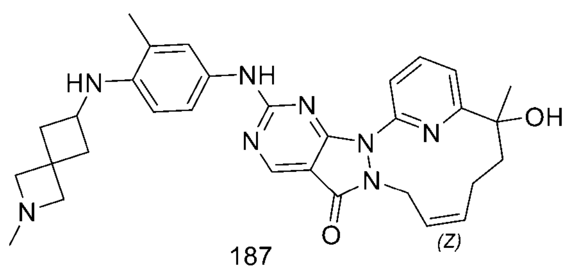

187

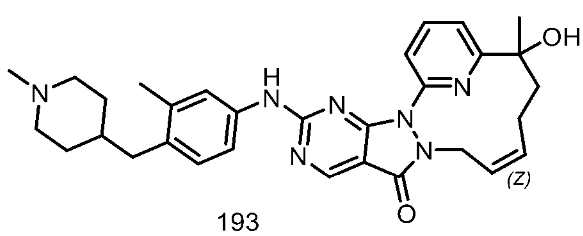

193

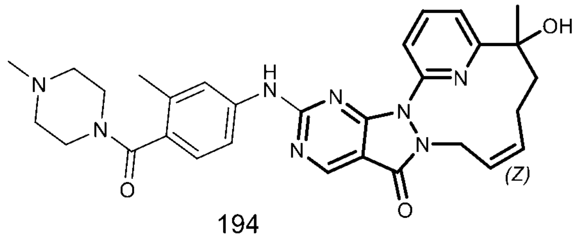

194

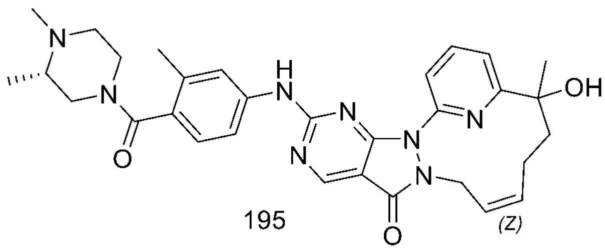

195

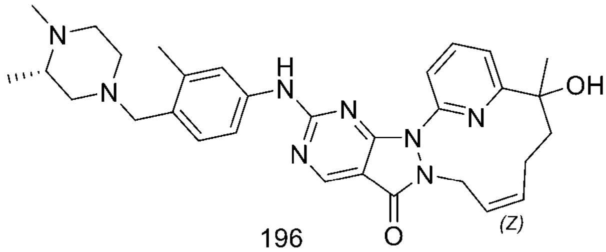

196

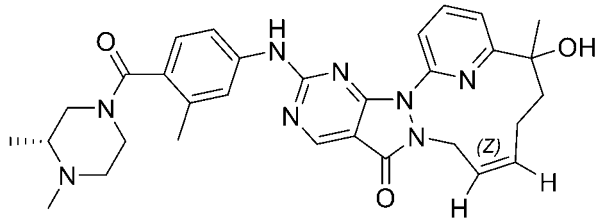

197

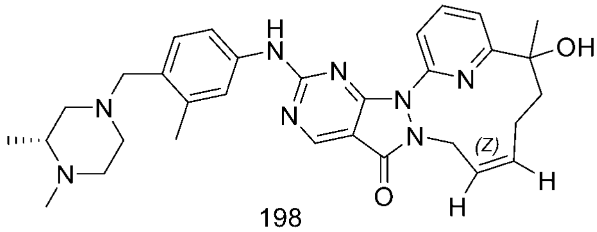

198

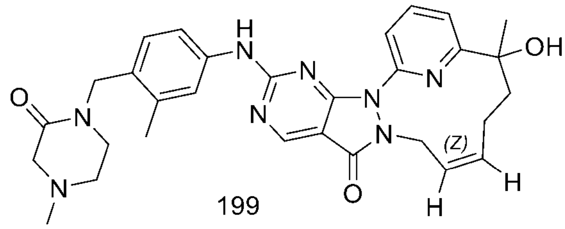

199

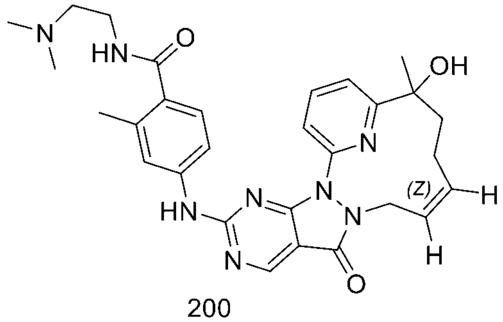

200

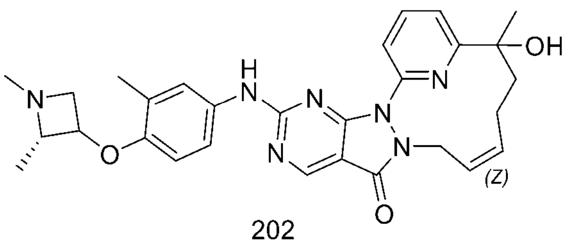

202

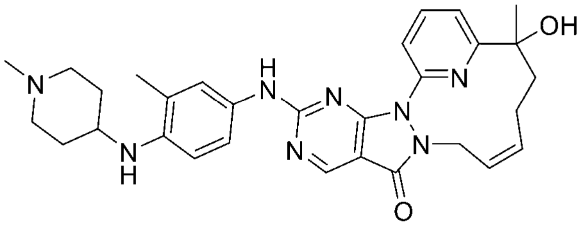

**203**

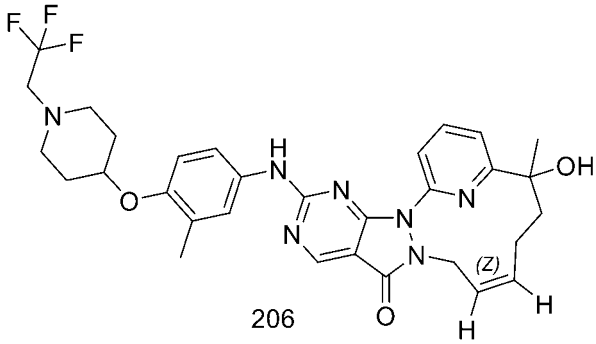

206

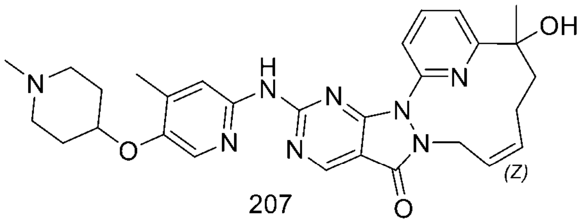

207

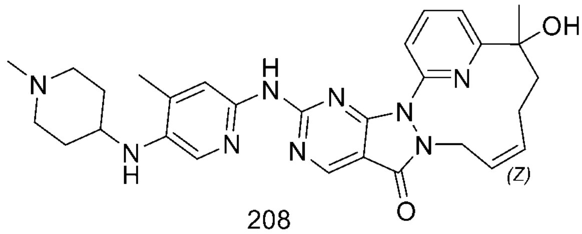

208

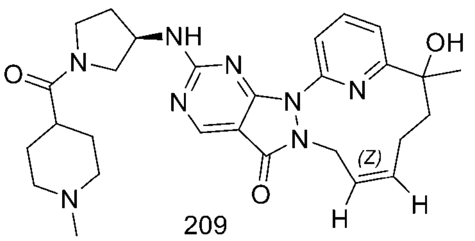

209

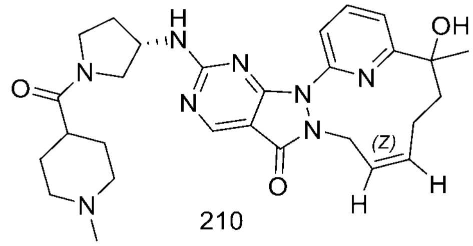

210

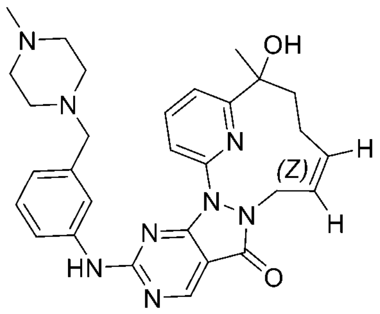

213

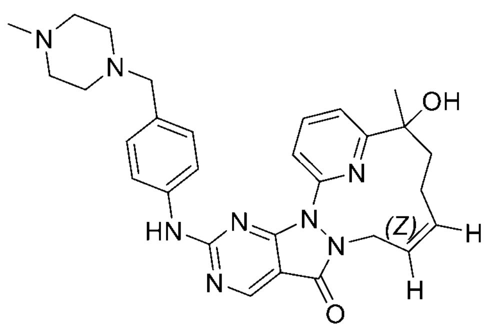

214

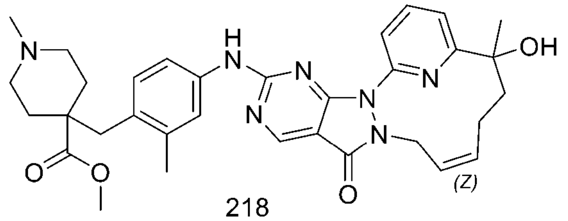

218

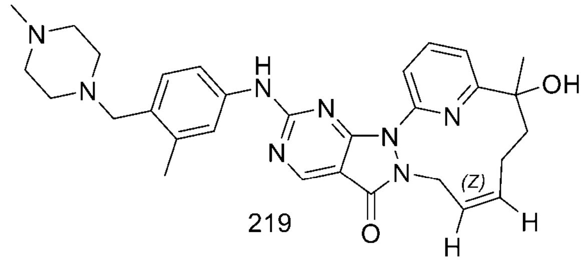

219

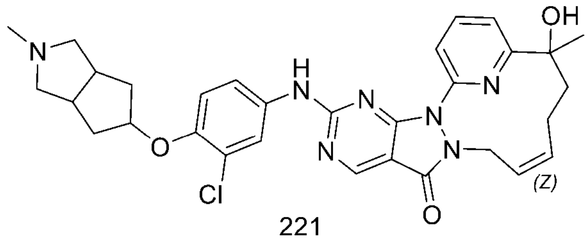

221

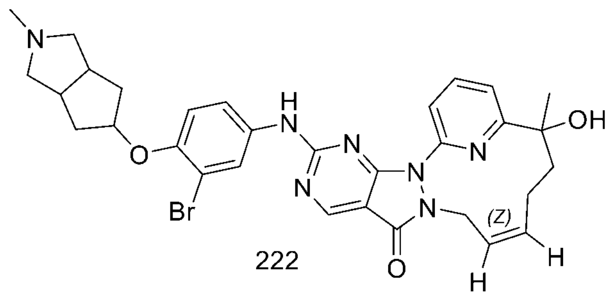

222

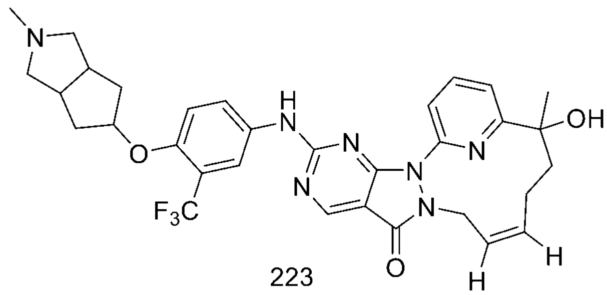

223

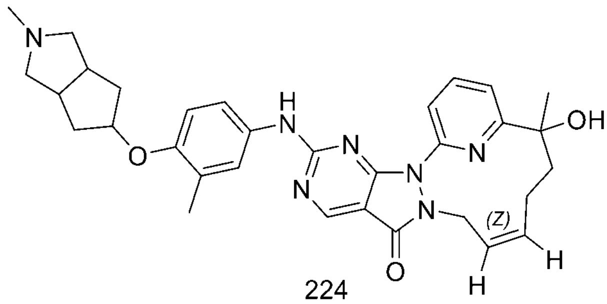

224

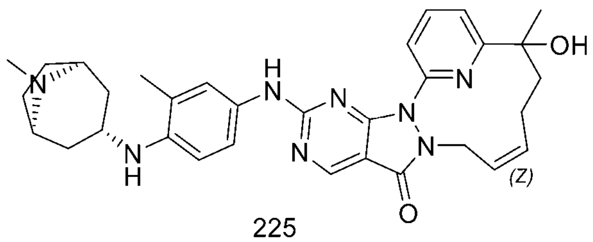

225

.

In one embodiment of the present disclosure, the compound, the pharmaceutically acceptable salt, the isotopically substituted compound, or the isomer thereof has one of the structures shown below:

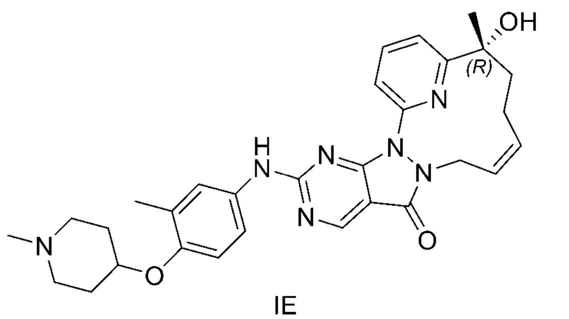

IE

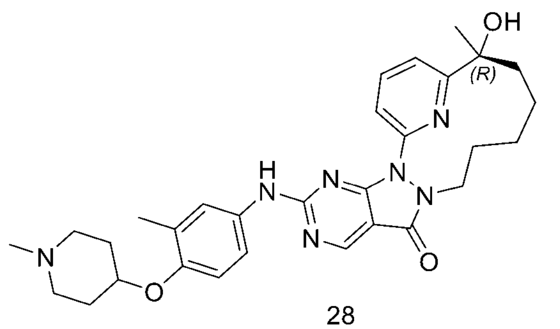

28

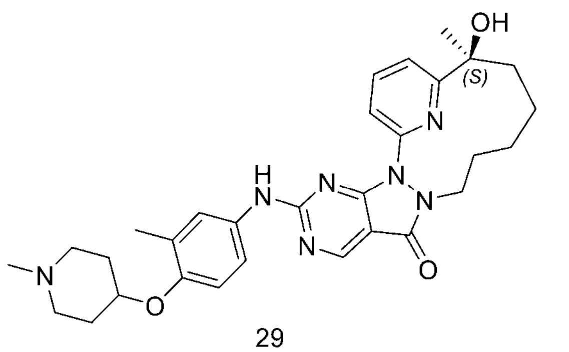

29

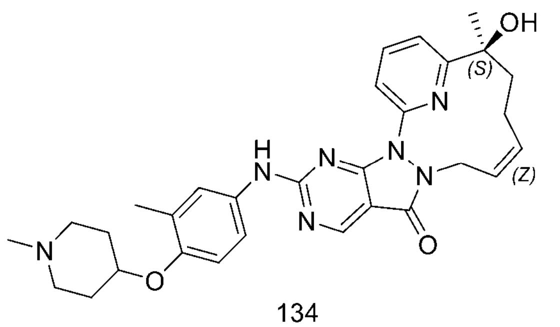

134

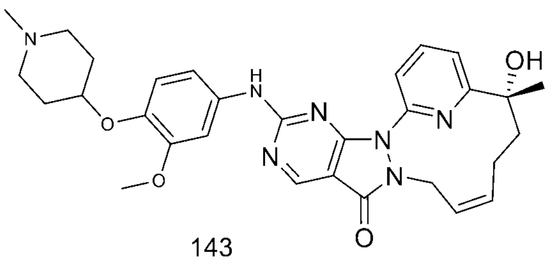

143

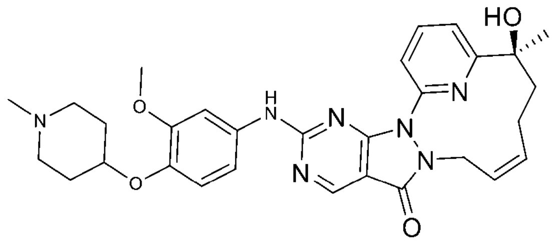

144

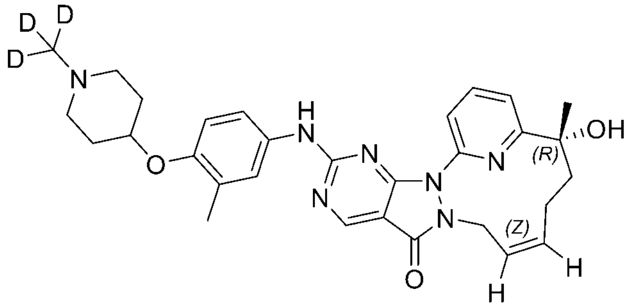

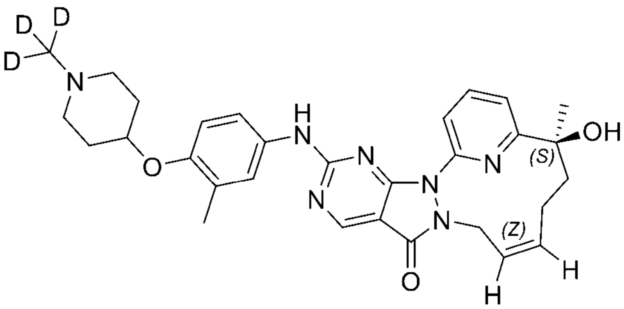

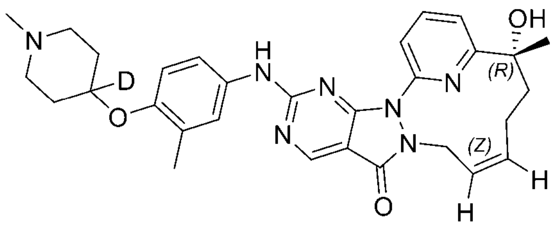

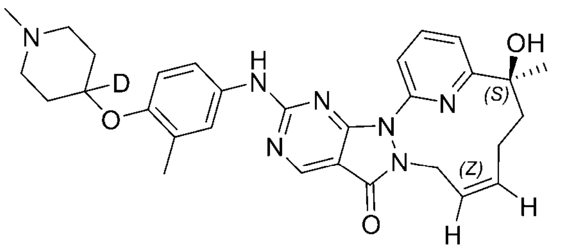

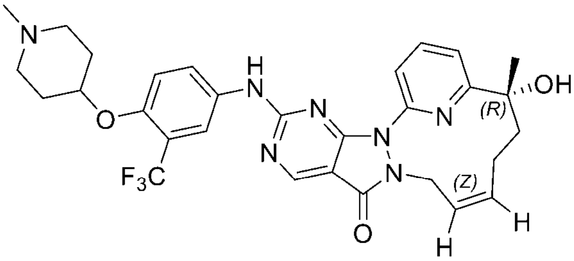

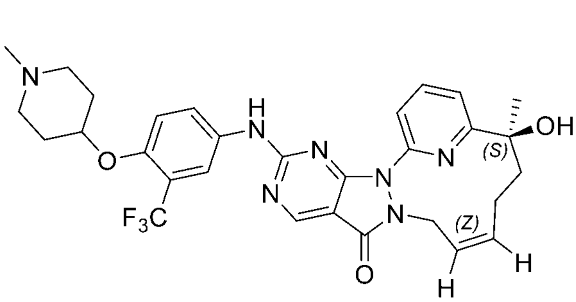

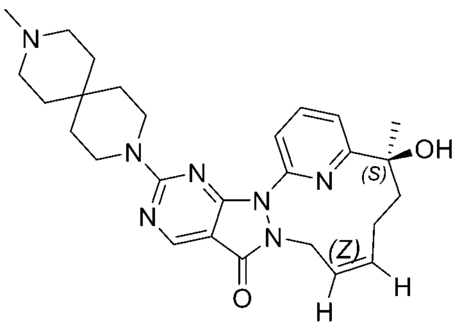

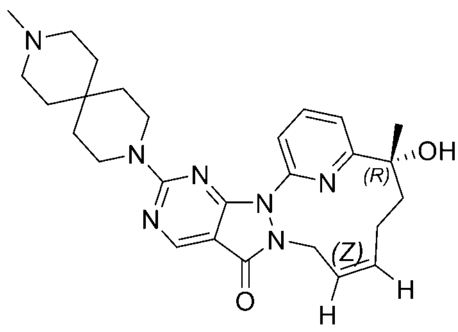

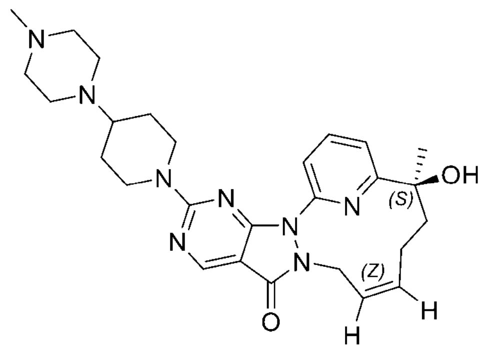

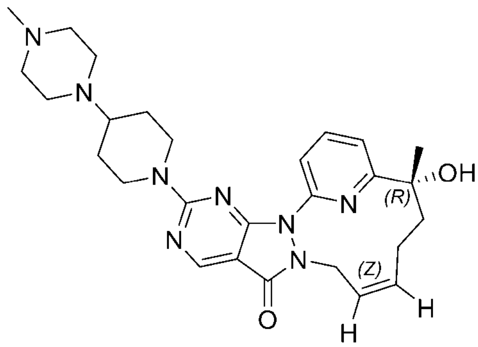

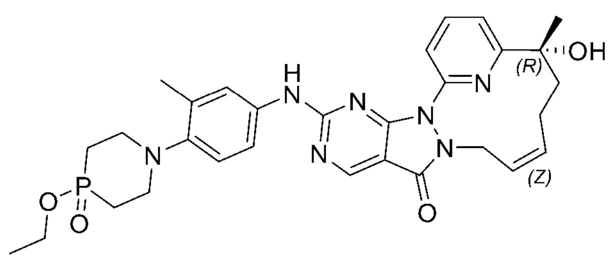

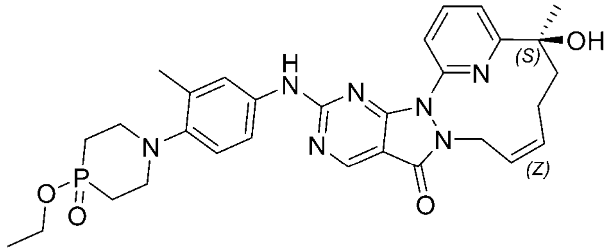

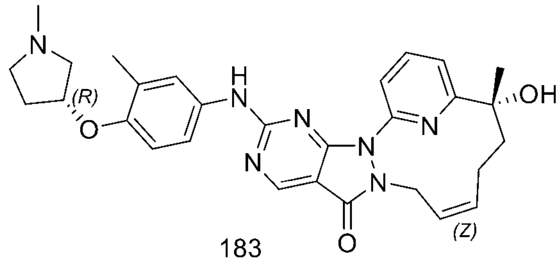

183

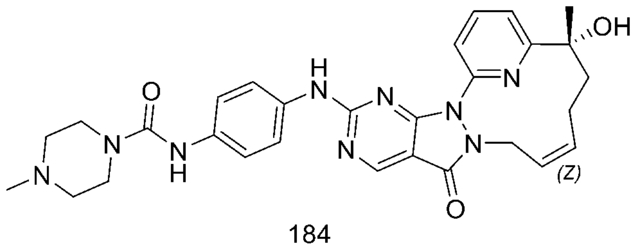

184

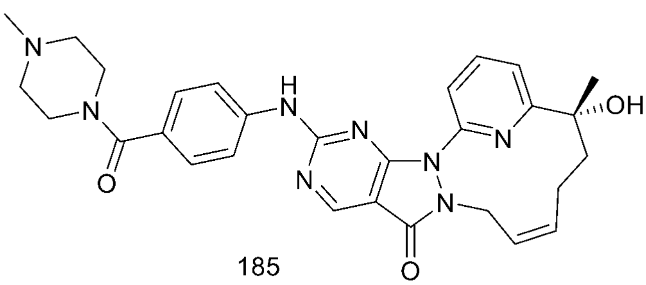

185

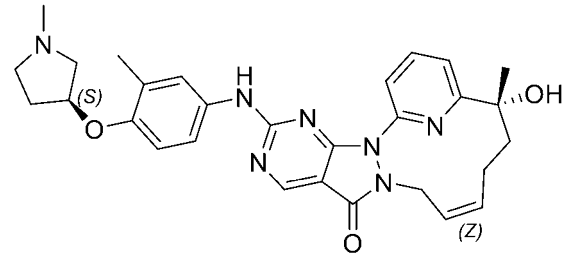

186

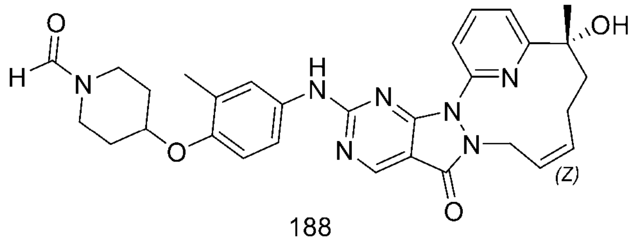

188

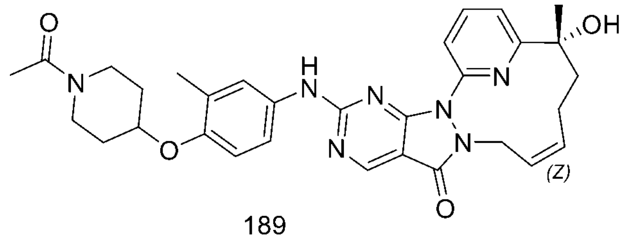

189

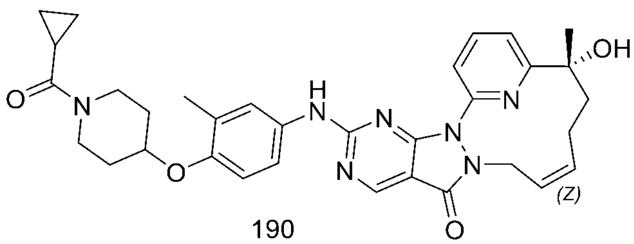

190

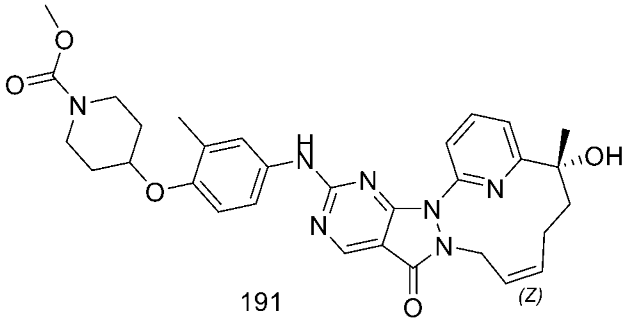

191

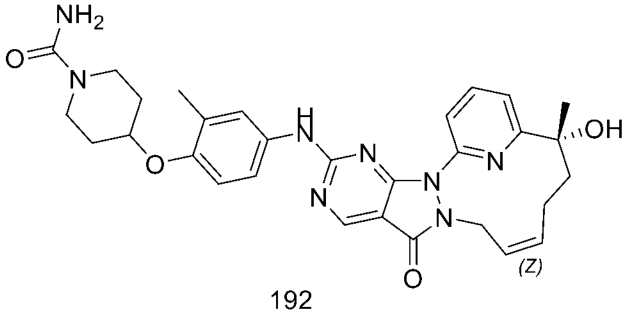

192

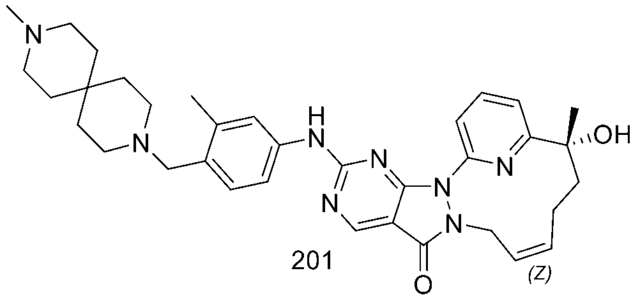

201

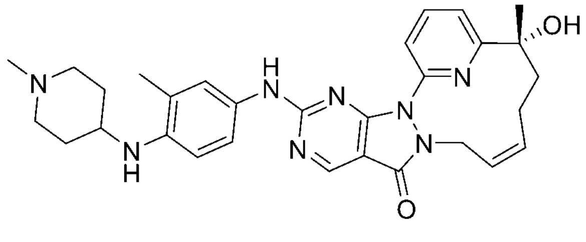

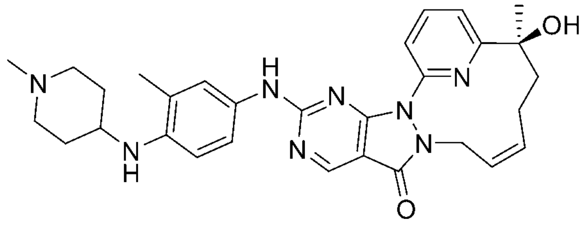

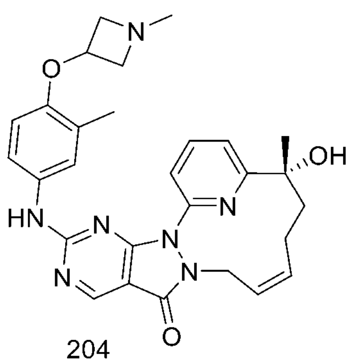

204

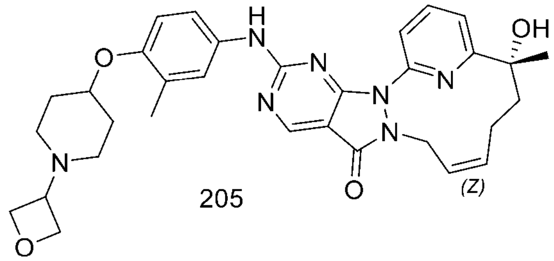

205

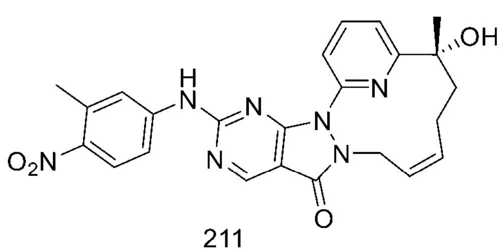

211

,

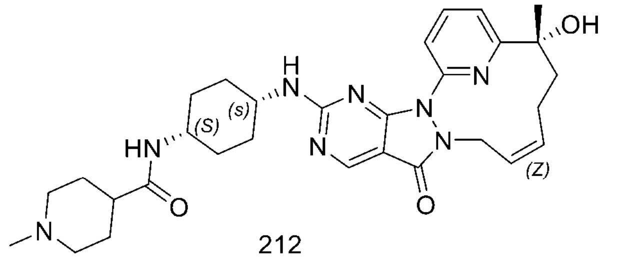

212

,

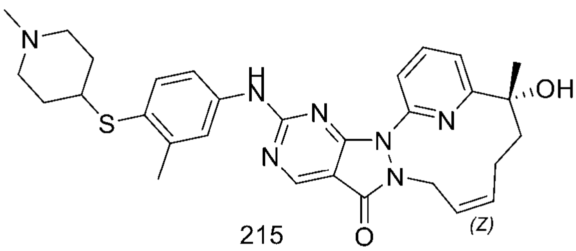

215

,

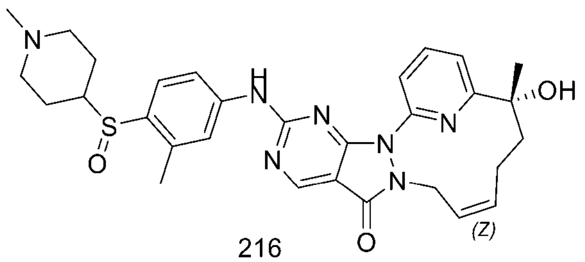

216

,

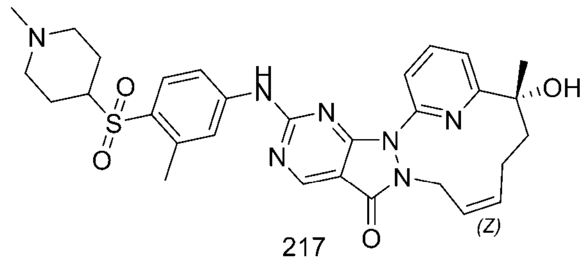

217

,

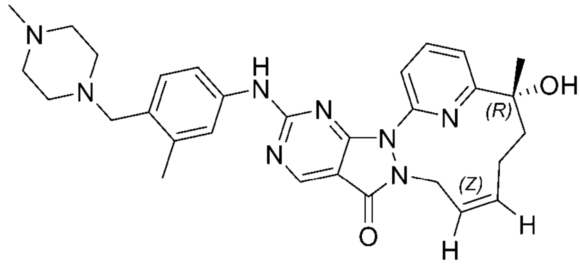

,

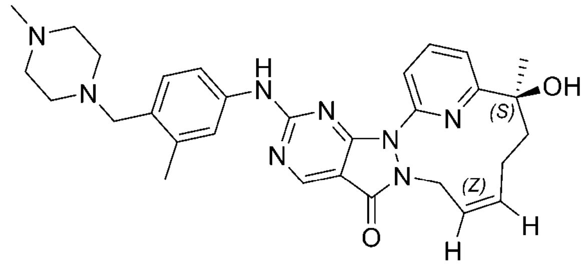

,

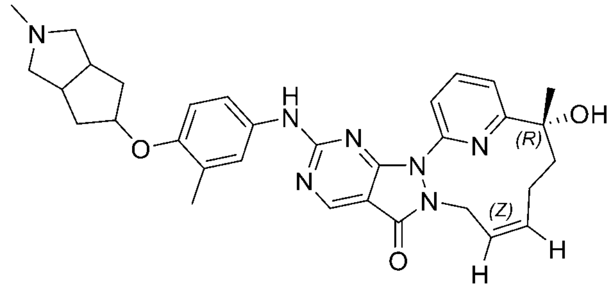

,

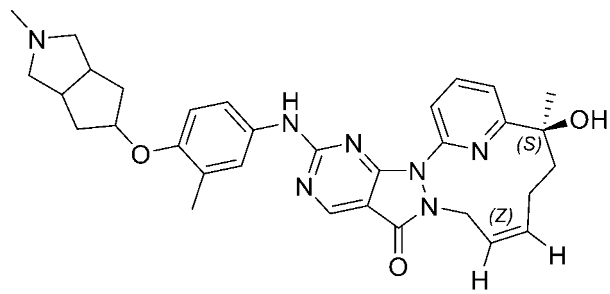

,

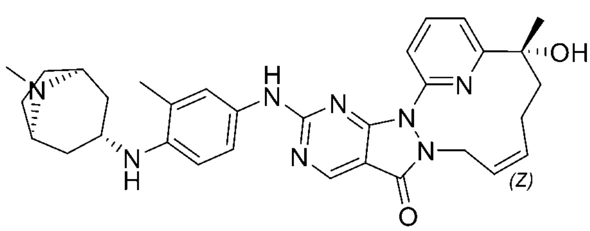

,

and

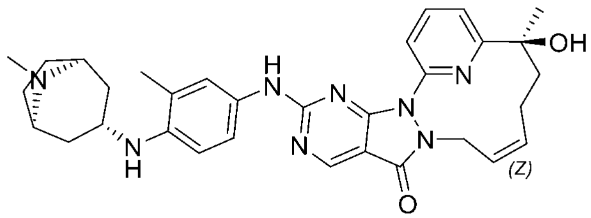

In certain embodiments of the present disclosure, the compound, the pharmaceutically acceptable salt, the isotopically substituted compound, or the isomer thereof has a structure of formula (IE):

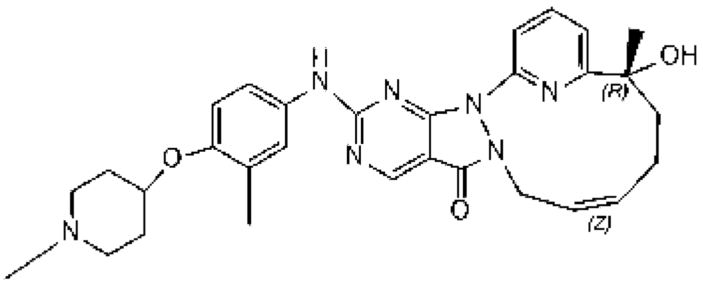

formula (IE).

The present disclosure further provides a polymorph of the compound represented by formula (IE):

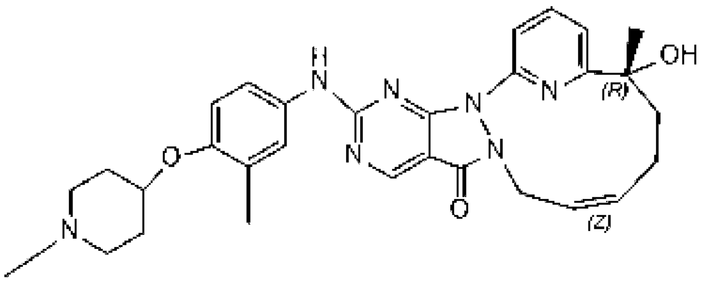

formula (IE).

According to an embodiment of the present disclosure, the polymorph includes, but is not limited to, a nonsolvate crystal form, a hydrate crystal form, or a metastable crystal form of compound IE;

Further, according to an embodiment of the present disclosure, the nonsolvate crystal form may be a crystal form A, B, or C as described below; the hydrate crystal form may be a crystal form D, E, F, or G as described below; the metastable crystal form may be a crystal form H, I, J, K, L, M, N, O, P, or Q as described below:

**Regarding the crystal form A:**

The present disclosure provides a crystal form A of compound (IE), wherein an X-ray powder diffraction pattern of the crystal form comprises peaks at diffraction angles (20) of 5.10±0.2°, 15.72±0.2°, 15.25±0.2°, and 20.53±0.2°.

According to the present disclosure, the crystal form A preferably further comprises peaks at diffraction angles (20) of 23.66±0.2°, 23.92±0.2°, 17.47±0.2°, 20.14±0.2°, and 24.44±0.2°.

According to the present disclosure, the crystal form A more preferably further comprises peaks at diffraction angles (2θ) of 26.66±0.2°, 17.22±0.2°, 26.92±0.2°, 16.48±0.2°, 10.20±0.2°, and 21.97±0.2°.

Preferably, the X-ray powder diffraction pattern of the crystal form A has the diffraction angles (2θ) shown in Table 1, wherein the 2θ angles have a margin of error of ±0.20°:

Table 1

| 2θ (°) | Intensity% | 2θ (°) | Intensity% |
|---|---|---|---|
| 5.10 | 100 | 24.45 | 6.1 |
| 7.91 | 0.3 | 25.47 | 0.3 |
| 8.57 | 0.3 | 25.66 | 0.4 |
| 10.21 | 2.0 | 26.32 | 0.7 |
| 12.53 | 0.7 | 26.67 | 4.3 |
| 13.40 | 0.5 | 26.92 | 2.8 |
| 13.74 | 0.4 | 27.66 | 0.7 |
| 13.95 | 0.7 | 28.19 | 1.5 |
| 14.65 | 1.3 | 28.48 | 0.3 |
| 15.25 | 14.2 | 28.81 | 0.4 |
| 15.72 | 14.5 | 29.63 | 0.3 |
| 16.48 | 2.5 | 30.31 | 1.7 |
| 17.22 | 3.5 | 30.72 | 1.6 |
| 17.47 | 7.0 | 31.37 | 0.9 |
| 18.04 | 0.5 | 32.78 | 0.4 |
| 19.48 | 0.9 | 33.25 | 0.7 |
| 20.14 | 7.0 | 34.39 | 0.4 |
| 20.53 | 10.5 | 35.39 | 0.6 |
| 21.31 | 1.9 | 35.61 | 0.6 |
| 21.97 | 2.0 | 36.89 | 0.3 |
| 22.83 | 1.9 | 38.05 | 0.3 |
| 23.67 | 9.8 | 38.63 | 0.2 |
| 23.92 | 8.8 | 39.14 | 0.6 |

Preferably, the crystal form A has the X-ray powder diffraction intensities shown in Table 1. Preferably, the crystal form A has an X-ray powder diffraction pattern substantially as shown in FIG. 1.

Preferably, according to DSC analysis, an endothermic peak appears when the crystal form A is heated to a peak temperature of about 180.86 °C.

Preferably, the crystal form A has a DSC profile substantially as shown in FIG. 2.

Preferably, the crystal form A has a TGA profile substantially as shown in FIG. 3.

**Regarding the crystal form B:**

The present disclosure provides a crystal form B of compound (IE), wherein an X-ray powder diffraction pattern of the crystal form comprises peaks at diffraction angles (2θ) of 10.33±0.2°, 5.18±0.2°, 13.69±0.2°, and 18.72±0.2°.

According to the present disclosure, the crystal form B preferably further comprises peaks at diffraction angles (2θ) of 8.96±0.2°, 11.73±0.2°, 25.89±0.2°, 17.10±0.2°, and 23.20±0.2°. According to the present disclosure, the crystal form B more preferably further comprises peaks at diffraction angles (2θ) of 26.13±0.2°, 24.92±0.2°, 22.77±0.2°, 20.85 ±0.2°, 17.95±0.2°, and 36.45±0.2°.

According to the present disclosure, preferably, the X-ray powder diffraction pattern of the crystal form B has the diffraction angles (20) shown in Table 2, wherein the 20 angles have a margin of error of ±0.20°:

Table 2

| 2θ (°) | Intensity% | 2θ (°) | Intensity% |
|---|---|---|---|
| 5.18 | 72.2 | 24.92 | 4.9 |

(continued)

| 2θ (°) | Intensity% | 2θ (°) | Intensity% |
|---|---|---|---|
| 8.96 | 47.2 | 25.46 | 0.6 |
| 10.33 | 100 | 25.89 | 6.2 |
| 11.73 | 8 | 26.13 | 5.3 |
| 13.69 | 52.5 | 27.59 | 2.3 |
| 17.10 | 6.1 | 28.01 | 0.8 |
| 17.43 | 1.5 | 29.30 | 2.4 |
| 17.95 | 3 | 31.35 | 0.8 |
| 18.72 | 47.5 | 32.90 | 0.6 |
| 20.85 | 3.4 | 33.70 | 2.2 |
| 21.35 | 0.8 | 35.98 | 1.4 |
| 21.74 | 2 | 36.45 | 2.5 |
| 22.77 | 4.4 | 36.80 | 0.8 |
| 23.20 | 5.7 | 37.91 | 1.1 |
| 23.79 | 0.6 | 38.91 | 0.5 |

Preferably, the crystal form B has the X-ray powder diffraction intensities shown in Table 2. Preferably, the crystal form B has an X-ray powder diffraction pattern substantially as shown in FIG. 4.

Preferably, according to DSC analysis, an endothermic peak appears when the crystal form B is heated to a peak temperature of about 179.71 °C.

Preferably, the crystal form B has a DSC profile substantially as shown in FIG. 5.

Preferably, the crystal form B has a TGA profile substantially as shown in FIG. 6.

**Regarding the crystal form C:**

The present disclosure provides a crystal form C of compound (IE), wherein an X-ray powder diffraction pattern of the crystal form comprises peaks at diffraction angles (2θ) of 5.39±0.2°, 12.05±0.2°, 9.70±0.2°, and 18.00±0.2°.

According to the present disclosure, the crystal form C preferably further comprises peaks at diffraction angles (20) of 23.06±0.2°, 21.21±0.2°, 17.30±0.2°, 17.14±0.2°, and 25.09±0.2°. According to the present disclosure, the crystal form C more preferably further comprises peaks at diffraction angles (2θ) of 22.39±0.2°, 19.54±0.2°, 13.48±0.2°, 27.27±0.2°, 10.68±0.2°, and 5.98±0.2°.

According to the present disclosure, preferably, the X-ray powder diffraction pattern of the crystal form C has the diffraction angles (20) shown in Table 3, wherein the 2θ angles have a margin of error of ±0.20°:

Table 3

| 2θ (°) | Intensity% | 2θ (°) | Intensity% |
|---|---|---|---|
| 5.39 | 100 | 21.21 | 7.6 |
| 5.98 | 2.5 | 21.66 | 0.5 |
| 7.58 | 1.5 | 21.86 | 1 |
| 9.70 | 10.8 | 22.39 | 4.2 |
| 10.68 | 2.7 | 23.06 | 8.9 |
| 11.03 | 0.9 | 23.67 | 0.3 |
| 12.06 | 17.8 | 24.29 | 0.3 |
| 12.64 | 0.2 | 25.09 | 5.7 |
| 13.48 | 3.9 | 25.48 | 2.3 |
| 14.03 | 2.2 | 26.80 | 0.6 |

(continued)

| 2θ (°) | Intensity% | 2θ (°) | Intensity% |
|---|---|---|---|
| 14.51 | 0.4 | 27.27 | 3.4 |
| 15.02 | 1.1 | 28.33 | 0.3 |
| 15.23 | 1.3 | 28.86 | 0.3 |
| 16.30 | 2.2 | 29.53 | 1.7 |
| 17.14 | 5.8 | 30.04 | 0.3 |
| 17.30 | 6.2 | 31.86 | 0.3 |
| 18.00 | 10.7 | 32.10 | 0.3 |
| 18.94 | 1.7 | 33.87 | 0.2 |
| 19.54 | 4.1 | 36.08 | 0.2 |
| 20.28 | 1.9 | | |

Preferably, the crystal form C has the X-ray powder diffraction intensities shown in Table 3. Preferably, the crystal form C has an X-ray powder diffraction pattern substantially as shown in FIG. 7.

Preferably, according to DSC analysis, an endothermic peak appears when the crystal form C is heated to a peak temperature of about 173.43 °C.

Preferably, the crystal form C has a DSC profile substantially as shown in FIG. 8.

Preferably, the crystal form C has a TGA profile substantially as shown in FIG. 9.

**Regarding the hydrate crystal form D:**

The present disclosure provides a hydrate crystal form D of compound (IE), wherein an X-ray powder diffraction pattern of the crystal form comprises peaks at diffraction angles (2θ) of 5.20±0.2°, 15.74±0.2°, 22.81±0.2°, and 18.39 ±0.2°.

According to the present disclosure, the crystal form D preferably further comprises peaks at diffraction angles (2θ) of 23.12±0.2°, 7.81±0.2°, 16.78±0.2°, 5.89±0.2°, and 10.43±0.2°. According to the present disclosure, the crystal form D more preferably further comprises peaks at diffraction angles (2θ) of 25.44±0.2°, 13.08±0.2°, 15.06±0.2°, 18.96 ±0.2°, 21.04±0.2°, and 21.95±0.2°.

According to the present disclosure, preferably, the X-ray powder diffraction pattern of the crystal form D has the diffraction angles (2θ) shown in Table 4, wherein the 2θ angles have a margin of error of ±0.20°:

Table 4

| 2θ (°) | Intensity% | 2θ (°) | Intensity% |
|---|---|---|---|
| 5.20 | 100 | 23.12 | 1.8 |
| 5.89 | 0.8 | 23.79 | 0.5 |
| 7.81 | 1.4 | 24.96 | 0.2 |
| 10.43 | 0.6 | 25.44 | 0.6 |
| 13.08 | 0.5 | 27.15 | 0.5 |
| 15.06 | 0.5 | 27.84 | 0.3 |
| 15.74 | 4.6 | 29.09 | 0.3 |
| 16.78 | 1.4 | 29.53 | 0.2 |
| 18.04 | 0.1 | 31.85 | 0.4 |
| 18.39 | 1.8 | 33.21 | 0.1 |
| 18.78 | 0.3 | 34.56 | 0.2 |
| 18.96 | 0.5 | 35.77 | 0.2 |
| 20.12 | 0.2 | 36.84 | 0.2 |

(continued)

| 2θ (°) | Intensity% | 2θ (°) | Intensity% |
|---|---|---|---|
| 21.04 | 0.5 | 38.06 | 0.2 |
| 21.95 | 0.5 | 39.33 | 0.2 |
| 22.81 | 3.4 | | |

Preferably, the crystal form D has the X-ray powder diffraction intensities shown in Table 4. Preferably, the crystal form D has an X-ray powder diffraction pattern substantially as shown in FIG. 10.

Preferably, according to DSC analysis, endothermic peaks appear when the crystal form D is heated to peak temperatures of about 157.79 °C and 181.65 °C.

Preferably, the crystal form D has a DSC profile substantially as shown in FIG. 11. Preferably, the crystal form D has a TGA profile substantially as shown in FIG. 12.

**Regarding the hydrate crystal form E:**

The present disclosure provides a hydrate crystal form E of compound (IE), wherein an X-ray powder diffraction pattern of the crystal form comprises peaks at diffraction angles (2θ) of 4.54±0.2°, 13.72±0.2°, 9.12±0.2°, and 18.35 ±0.2°.

According to the present disclosure, the crystal form E preferably further comprises peaks at diffraction angles (2θ) of 15.88±0.2°, 23.00±0.2°, 5.24±0.2°, 23.88±0.2°, and 17.20±0.2°.

According to the present disclosure, the crystal form E more preferably further comprises peaks at diffraction angles (2θ) of 21.72±0.2°, 20.42±0.2°, 23.49±0.2°, 25.01±0.2°, 25.29±0.2°, and 9.72±0.2°.

According to the present disclosure, preferably, the X-ray powder diffraction pattern of the crystal form E has the diffraction angles (2θ) shown in Table 5, wherein the 2θ angles have a margin of error of ±0.20°:

Table 5

| 2θ (°) | Intensity% | 2θ (°) | Intensity% |
|---|---|---|---|
| 4.54 | 100 | 21.72 | 0.4 |
| 5.24 | 0.5 | 22.60 | 0.2 |
| 9.12 | 1.9 | 23.01 | 0.8 |
| 9.72 | 0.2 | 23.49 | 0.3 |
| 13.72 | 2.3 | 23.88 | 0.5 |
| 14.83 | 0.2 | 25.01 | 0.3 |
| 15.88 | 0.9 | 25.29 | 0.3 |
| 17.20 | 0.4 | 26.11 | 0.2 |
| 17.61 | 0.2 | 26.79 | 0.2 |
| 18.35 | 1 | 27.68 | 0.1 |
| 19.73 | 0.1 | 30.08 | 0.2 |
| 20.15 | 0.1 | 37.83 | 0.1 |
| 20.42 | 0.3 | 38.36 | 0.2 |
| 21.06 | 0.2 | | |

Preferably, the crystal form E has the X-ray powder diffraction intensities shown in Table 5. Preferably, the crystal form E has an X-ray powder diffraction pattern substantially as shown in FIG. 13.

Preferably, according to DSC analysis, endothermic peaks appear when the crystal form E is heated to peak temperatures of about 69.77 °C and 181.95 °C.

Preferably, the crystal form E has a DSC profile substantially as shown in FIG. 14. Preferably, the crystal form E has a TGA profile substantially as shown in FIG. 15.

**Regarding the hydrate crystal form F:**

The present disclosure provides a hydrate crystal form F of compound (IE), wherein an X-ray powder diffraction pattern of the crystal form comprises peaks at diffraction angles (2θ) of 9.08±0.2°, 18.27±0.2°, 6.25±0.2°, and 13.73 ±0.2°.
According to the present disclosure, the crystal form F preferably further comprises peaks at diffraction angles (20) of 7.52±0.2°, 5.12±0.2°, 10.25±0.2°, 10.50±0.2°, and 15.16±0.2°. According to the present disclosure, the crystal form F more preferably further comprises peaks at diffraction angles (20) of 15.00±0.2°, 16.60±0.2°, 19.04±0.2°, 21.46 ±0.2°, 27.60±0.2°, and 11.71±0.2°.
According to the present disclosure, preferably, the X-ray powder diffraction pattern of the crystal form F has the diffraction angles (20) shown in Table 6, wherein the 2θ angles have a margin of error of ±0.20°:

Table 6

| 2θ (°) | Intensity% | 2θ (°) | Intensity% |
|---|---|---|---|
| 5.12 | 8.0 | 18.27 | 30.4 |
| 6.25 | 24.1 | 19.04 | 3.1 |
| 7.52 | 14.5 | 20.81 | 0.6 |
| 9.08 | 100.0 | 21.46 | 1.5 |
| 10.25 | 7.8 | 22.65 | 0.7 |
| 10.50 | 6.8 | 23.61 | 0.8 |
| 11.71 | 1.1 | 24.51 | 0.8 |
| 13.73 | 20.1 | 27.60 | 1.2 |
| 15.00 | 3.6 | 29.24 | 0.8 |
| 15.16 | 5.0 | 29.47 | 1.0 |
| 16.60 | 3.5 | 32.44 | 0.6 |
| 17.17 | 0.8 | | |

Preferably, the crystal form F has the X-ray powder diffraction intensities shown in Table 6. Preferably, the crystal form F has an X-ray powder diffraction pattern substantially as shown in FIG. 16.
Preferably, according to DSC analysis, endothermic peaks appear when the crystal form F is heated to peak temperatures of about 101.36 °C and 177.98 °C, and an exothermic peak appears at about 146.72 °C.
Preferably, the crystal form F has a DSC profile substantially as shown in FIG. 17. Preferably, the crystal form F has a TGA profile substantially as shown in FIG. 18.

**Regarding the hydrate crystal form G:**

The present disclosure provides a hydrate crystal form G of compound (IE), wherein an X-ray powder diffraction pattern of the crystal form comprises peaks at diffraction angles (2θ) of 9.06±0.2°, 14.98±0.2°, 15.76±0.2°, and 18.16 ±0.2°.
According to the present disclosure, the crystal form G preferably further comprises peaks at diffraction angles (2θ) of 12.04±0.2°, 20.96±0.2°, 24.12±0.2°, 9.89±0.2°, and 28.54±0.2°. According to the present disclosure, the crystal form G more preferably further comprises peaks at diffraction angles (2θ) of 7.93±0.2°, 6.03±0.2°, 25.73±0.2°, 27.70 ±0.2°, 21.78+0.2°, and 24.51±0.2°.
According to the present disclosure, preferably, the X-ray powder diffraction pattern of the crystal form G has the diffraction angles (20) shown in Table 7, wherein the 2θ angles have a margin of error of ±0.20°:

Table 7

| 2θ ( ° ) | Intensity% | 2θ ( ° ) | Intensity% |
|---|---|---|---|
| 6.04 | 4.5 | 27.70 | 4 |
| 7.93 | 5.7 | 28.54 | 6.5 |

(continued)

| 2θ ( ° ) | Intensity% | 2θ ( ° ) | Intensity% |
|---|---|---|---|
| 9.06 | 100 | 29.86 | 0.9 |
| 9.90 | 6.6 | 30.33 | 0.6 |
| 12.04 | 35 | 30.86 | 1.6 |
| 14.98 | 65.7 | 31.23 | 1 |
| 15.76 | 48.5 | 31.79 | 0.9 |
| 18.16 | 47.8 | 32.46 | 0.8 |
| 19.17 | 1.7 | 33.35 | 1.1 |
| 20.16 | 1.3 | 34.09 | 1.2 |
| 20.96 | 22.1 | 34.91 | 2.5 |
| 21.78 | 3 | 35.84 | 0.9 |
| 22.64 | 1.5 | 36.74 | 1.4 |
| 24.12 | 13.8 | 37.38 | 1.4 |
| 24.51 | 2.9 | 39.62 | 2.3 |
| 25.73 | 4.4 | | |

Preferably, the crystal form G has the X-ray powder diffraction intensities shown in Table 7. Preferably, the crystal form G has an X-ray powder diffraction pattern substantially as shown in FIG. 19.

Preferably, according to DSC analysis, an endothermic peak appears when the crystal form G is heated to a peak temperature of about 126.74 °C.

Preferably, the crystal form G has a DSC profile substantially as shown in FIG. 20. Preferably, the crystal form G has a TGA profile substantially as shown in FIG. 21.

**Regarding the metastable crystal form H:**

The present disclosure provides a metastable crystal form H of compound (IE), wherein an X-ray powder diffraction pattern of the crystal form comprises peaks at diffraction angles (20) of 6.02±0.2°, 3.96±0.2°, 12.66±0.2°, and 9.35±0.2°.

According to the present disclosure, the crystal form H preferably further comprises peaks at diffraction angles (20) of 15.49±0.2°, 12.06±0.2°, 4.42±0.2°, 11.38±0.2°, and 18.31±0.2°.

According to the present disclosure, the crystal form H more preferably further comprises peaks at diffraction angles (2θ) of 6.68±0.2°, 13.87±0.2°, 9.88±0.2°, 19.05±0.2°, 17.92±0.2°, and 8.94±0.2°.

According to the present disclosure, preferably, the X-ray powder diffraction pattern of the crystal form H has the diffraction angles (2θ) shown in Table 8, wherein the 2θ angles have a margin of error of ±0.20°:

Table 8

| 2θ (°) | Intensity% | 2θ (°) | Intensity% |
|---|---|---|---|
| 3.96 | 83.5 | 17.92 | 8.7 |
| 4.42 | 12.5 | 18.31 | 10.1 |
| 6.02 | 100.0 | 19.05 | 8.8 |
| 6.68 | 10.0 | 20.09 | 7.7 |
| 8.94 | 8.6 | 20.96 | 3.2 |
| 9.35 | 16.3 | 21.20 | 4.5 |
| 9.88 | 9.6 | 22.02 | 4.9 |
| 10.36 | 4.7 | 22.57 | 3.5 |
| 10.70 | 3.5 | 23.05 | 6.8 |

(continued)

| 2θ (°) | Intensity% | 2θ (°) | Intensity% |
|---|---|---|---|
| 11.38 | 12.5 | 24.09 | 1.9 |
| 12.06 | 15.1 | 24.68 | 5.9 |
| 12.66 | 29.1 | 25.43 | 4.7 |
| 13.13 | 5.5 | 26.05 | 2.5 |
| 13.87 | 9.8 | 26.26 | 3.1 |
| 14.56 | 3.3 | 27.33 | 1.7 |
| 14.85 | 2.4 | 29.41 | 1.4 |
| 15.49 | 15.6 | 31.44 | 1.6 |
| 15.76 | 3.1 | 33.99 | 1.1 |
| 16.31 | 8.0 | 35.32 | 1.1 |
| 16.66 | 5.6 | | |

Preferably, the crystal form H has the X-ray powder diffraction intensities shown in Table 8. Preferably, the crystal form H has an X-ray powder diffraction pattern substantially as shown in FIG. 22.

Preferably, according to DSC analysis, endothermic peaks appear when the crystal form H is heated to peak temperatures of about 61.06 °C and 151.59 °C.

Preferably, the crystal form H has a DSC profile substantially as shown in FIG. 23. Preferably, the crystal form H has a TGA profile substantially as shown in FIG. 24.

**Regarding the metastable crystal form I:**

The present disclosure provides a metastable crystal form I of compound (IE), wherein an X-ray powder diffraction pattern of the crystal form comprises peaks at diffraction angles (2θ) of 4.93±0.2°, 4.37±0.2°, 16.01±0.2°, and 7.45±0.2°.

According to the present disclosure, the crystal form I preferably further comprises peaks at diffraction angles (20) of 6.45±0.2°, 22.79±0.2°, 17.57±0.2°, 17.98±0.2°, and 15.14±0.2°. According to the present disclosure, the crystal form I more preferably further comprises peaks at diffraction angles (20) of 22.48±0.2°, 16.65±0.2°, 10.00±0.2°, 12.59 ±0.2°, 20.05±0.2°, and 25.54±0.2°.

According to the present disclosure, preferably, the X-ray powder diffraction pattern of the crystal form I has the diffraction angles (2θ) shown in Table 9, wherein the 2θ angles have a margin of error of ±0.20°:

Table 9

| 2θ (°) | Intensity% | 2θ (°) | Intensity% |
|---|---|---|---|
| 4.37 | 19.9 | 16.65 | 1.1 |
| 4.93 | 100 | 17.57 | 1.3 |
| 6.45 | 2.3 | 17.98 | 1.3 |
| 7.45 | 2.7 | 20.05 | 0.8 |
| 10.00 | 0.9 | 22.48 | 1.2 |
| 12.59 | 0.8 | 22.79 | 1.5 |
| 14.37 | 0.3 | 25.54 | 0.4 |
| 15.14 | 1.2 | 26.96 | 0.4 |
| 16.01 | 3.3 | | |

Preferably, the crystal form I has the X-ray powder diffraction intensities shown in Table 9. Preferably, the crystal form I has an X-ray powder diffraction pattern substantially as shown in FIG. 25.

**Regarding the metastable crystal form J:**

The present disclosure provides a metastable crystal form J of compound (IE), wherein an X-ray powder diffraction pattern of the crystal form comprises peaks at diffraction angles (2θ) of 5.78±0.2°, 8.06±0.2°, 12.04±0.2°, and 16.19±0.2°.

According to the present disclosure, the crystal form J preferably further comprises peaks at diffraction angles (20) of 17.14±0.2°, 14.55±0.2°, 10.35±0.2°, 11.59±0.2°, and 19.77±0.2°. According to the present disclosure, the crystal form J more preferably further comprises peaks at diffraction angles (20) of 7.25±0.2°, 20.81±0.2°, 26.22±0.2°, 20.57±0.2°, 24.25±0.2°, and 22.62±0.2°.

According to the present disclosure, preferably, the X-ray powder diffraction pattern of the crystal form J has the diffraction angles (20) shown in Table 10, wherein the 2θ angles have a margin of error of ±0.20°:

Table 10

| 2θ (°) | Intensity% | 2θ (°) | Intensity% |
|---|---|---|---|
| 5.78 | 100.0 | 23.32 | 2.5 |
| 7.25 | 4.1 | 23.88 | 1.0 |
| 8.06 | 65.3 | 24.25 | 2.6 |
| 10.35 | 11.9 | 25.23 | 0.7 |
| 11.59 | 10.9 | 26.22 | 3.2 |
| 12.04 | 64.0 | 26.84 | 0.9 |
| 13.79 | 1.5 | 27.51 | 1.2 |
| 14.24 | 1.5 | 27.80 | 0.9 |
| 14.55 | 13.0 | 28.42 | 2.4 |
| 15.18 | 0.8 | 29.30 | 1.4 |
| 16.19 | 38.0 | 30.20 | 1.4 |
| 16.73 | 2.3 | 32.02 | 0.6 |
| 17.14 | 19.4 | 32.73 | 2.2 |
| 19.77 | 6.2 | 33.30 | 0.4 |
| 20.57 | 2.8 | 34.73 | 0.9 |
| 20.81 | 3.6 | 35.89 | 0.6 |
| 21.92 | 1.5 | 36.10 | 2.0 |
| 22.06 | 1.6 | 38.54 | 1.2 |
| 22.62 | 2.5 | | |

Preferably, the crystal form J has the X-ray powder diffraction intensities shown in Table 10. Preferably, the crystal form J has an X-ray powder diffraction pattern substantially as shown in FIG. 26.

**Regarding the metastable crystal form K:**

The present disclosure provides a metastable crystal form K of compound (IE), wherein an X-ray powder diffraction pattern of the crystal form comprises peaks at diffraction angles (2θ) of 4.05±0.2°, 4.89±0.2°, 4.44±0.2°, and 5.92±0.2°.

According to the present disclosure, the crystal form K preferably further comprises peaks at diffraction angles (20) of 12.53±0.2°, 7.87±0.2°, 16.76±0.2°, 8.83±0.2°, and 5.55±0.2°. According to the present disclosure, the crystal form K more preferably further comprises peaks at diffraction angles (20) of 14.59±0.2°, 9.84±0.2°, 13.38±0.2°, 20.10 ±0.2°, 14.08±0.2°, and 15.76±0.2°.

According to the present disclosure, preferably, the X-ray powder diffraction pattern of the crystal form K has the diffraction angles (2θ) shown in Table 11, wherein the 2θ angles have a margin of error of ±0.20°:

Table 11

| 2θ (°) | Intensity% | 2θ (°) | Intensity% |
|---|---|---|---|
| 4.05 | 100 | 14.08 | 5 |
| 4.44 | 47.5 | 14.59 | 7.5 |
| 4.89 | 88.3 | 15.10 | 3.3 |
| 5.55 | 9.3 | 15.51 | 3.1 |
| 5.92 | 21.7 | 15.76 | 5 |
| 6.86 | 3.7 | 16.76 | 11.8 |
| 6.86 | 3.7 | 17.75 | 3.1 |
| 7.29 | 4.7 | 18.72 | 2.1 |
| 7.87 | 11.8 | 18.88 | 3.3 |
| 8.83 | 9.5 | 19.76 | 3.4 |
| 9.84 | 5.4 | 20.10 | 5.4 |
| 10.08 | 3.4 | 20.40 | 3.1 |
| 10.27 | 2.3 | 22.42 | 2.8 |
| 10.76 | 4.9 | 23.24 | 4.6 |
| 12.53 | 18.2 | 26.63 | 2.2 |
| 13.38 | 5.4 | 34.50 | 1.2 |

Preferably, the crystal form K has the X-ray powder diffraction intensities shown in Table 11. Preferably, the crystal form K has an X-ray powder diffraction pattern substantially as shown in FIG. 27.

**Regarding the metastable crystal form L:**

The present disclosure provides a metastable crystal form L of compound (IE), wherein an X-ray powder diffraction pattern of the crystal form comprises peaks at diffraction angles (2θ) of 8.67±0.2°, 15.29±0.2°, 14.75±0.2°, and 11.76±0.2°.

According to the present disclosure, the crystal form L preferably further comprises peaks at diffraction angles (2θ) of 17.44±0.2°, 9.02±0.2°, 26.63±0.2°, 20.65±0.2°, and 7.89±0.2°. According to the present disclosure, the crystal form L more preferably further comprises peaks at diffraction angles (2θ) of 17.89±0.2°, 9.53±0.2°, 23.98±0.2°, 19.19 ±0.2°, 21.18±0.2°, and 13.64±0.2°.

According to the present disclosure, preferably, the X-ray powder diffraction pattern of the crystal form L has the diffraction angles (2θ) shown in Table 12, wherein the 2θ angles have a margin of error of ±0.20°:

Table 12

| 2θ (°) | Intensity% | 2θ (°) | Intensity% |
|---|---|---|---|
| 7.89 | 9.7 | 24.37 | 1.1 |
| 8.67 | 100 | 24.93 | 0.5 |
| 9.02 | 16.3 | 25.99 | 0.8 |
| 9.53 | 7.6 | 26.34 | 1 |
| 11.76 | 39.7 | 26.63 | 11 |
| 12.66 | 2 | 27.51 | 1.4 |
| 13.64 | 2.5 | 27.94 | 0.4 |
| 14.75 | 47.2 | 28.25 | 0.3 |
| 15.29 | 53.7 | 29.47 | 0.5 |
| 15.90 | 1.7 | 29.81 | 1.4 |

(continued)

| 2θ (°) | Intensity% | 2θ (°) | Intensity% |
|---|---|---|---|
| 17.44 | 24.6 | 31.08 | 1.3 |
| 17.89 | 7.7 | 31.87 | 0.7 |
| 18.14 | 0.9 | 32.70 | 0.5 |
| 19.19 | 3.7 | 33.20 | 0.7 |
| 19.50 | 0.6 | 34.87 | 1 |
| 20.65 | 9.8 | 35.65 | 0.3 |
| 21.18 | 3.7 | 35.94 | 0.3 |
| 21.74 | 0.5 | 36.31 | 0.4 |
| 22.23 | 1.8 | 36.98 | 0.3 |
| 22.95 | 0.4 | 38.86 | 1.1 |
| 23.69 | 0.9 | 39.49 | 0.5 |
| 23.98 | 6 | | |

Preferably, the crystal form L has the X-ray powder diffraction intensities shown in Table 12. Preferably, the crystal form L has an X-ray powder diffraction pattern substantially as shown in FIG. 28.

**Regarding the metastable crystal form M:**

The present disclosure provides a metastable crystal form M of compound (IE), wherein an X-ray powder diffraction pattern of the crystal form comprises peaks at diffraction angles (2θ) of 8.26±0.2°, 14.79±0.2°, 17.07±0.2°, and 14.57±0.2°.

According to the present disclosure, the crystal form M preferably further comprises peaks at diffraction angles (2θ) of 11.50±0.2°, 21.18±0.2°, 16.60±0.2°, 20.53±0.2°, and 25.33±0.2°. According to the present disclosure, the crystal form M more preferably further comprises peaks at diffraction angles (20) of 18.43±0.2°, 25.01±0.2°, 7.99±0.2°, 8.98±0.2°, 13.04±0.2°, and 24.12±0.2°.

According to the present disclosure, preferably, the X-ray powder diffraction pattern of the crystal form M has the diffraction angles (2θ) shown in Table 13, wherein the 2θ angles have a margin of error of ±0.20°:

Table 13

| 2θ (°) | Intensity% | 2θ (°) | Intensity% |
|---|---|---|---|
| 5.74 | 1.6 | 21.49 | 0.9 |
| 7.99 | 6.3 | 22.12 | 0.7 |
| 8.26 | 100.0 | 24.12 | 4.2 |
| 8.98 | 6.1 | 24.49 | 1.2 |
| 11.50 | 22.5 | 25.01 | 7.8 |
| 12.70 | 1.5 | 25.33 | 11.2 |
| 13.04 | 5.4 | 26.13 | 2.9 |
| 14.57 | 27.7 | 27.66 | 0.6 |
| 14.79 | 65.8 | 28.15 | 0.6 |
| 15.99 | 1.1 | 29.40 | 1.1 |
| 16.60 | 13.7 | 29.81 | 0.5 |
| 17.07 | 51.5 | 30.39 | 1 |
| 17.79 | 0.8 | 32.23 | 1.3 |
| 18.02 | 1.2 | 33.01 | 0.4 |

(continued)

| 2θ (°) | Intensity% | 2θ (°) | Intensity% |
|---|---|---|---|
| 18.43 | 8.4 | 33.55 | 2.5 |
| 19.38 | 0.5 | 34.70 | 0.3 |
| 20.32 | 2.6 | 35.77 | 1 |
| 20.53 | 11.2 | 37.43 | 0.4 |
| 21.18 | 17.2 | 38.28 | 1.3 |

Preferably, the crystal form M has the X-ray powder diffraction intensities shown in Table 13.

Preferably, the crystal form M has an X-ray powder diffraction pattern substantially as shown in FIG. 29.

**Regarding the metastable crystal form N:**

The present disclosure provides a metastable crystal form N of compound (IE), wherein an X-ray powder diffraction pattern of the crystal form comprises peaks at diffraction angles (20) of 4.48±0.2°, 5.02±0.2°, 5.55±0.2°, and 11.61±0.2°.

According to the present disclosure, the crystal form N preferably further comprises peaks at diffraction angles (20) of 3.14±0.2°, 14.70±0.2°, 11.42±0.2°, 7.31±0.2°, and 13.69±0.2°. According to the present disclosure, the crystal form N more preferably further comprises peaks at diffraction angles (20) of 13.34±0.2°, 9.57±0.2°, 15.71±0.2°, 15.10 ±0.2°, 12.43±0.2°, and 22.05±0.2°.

According to the present disclosure, preferably, the X-ray powder diffraction pattern of the crystal form N has the diffraction angles (20) shown in Table 14, wherein the 20 angles have a margin of error of ±0.20°:

Table 14

| 2θ (°) | Intensity% | 20 (°) | Intensity% |
|---|---|---|---|
| 3.14 | 7.3 | 13.34 | 4.5 |
| 4.48 | 100 | 13.69 | 4.7 |
| 5.02 | 22.3 | 14.70 | 6.3 |
| 5.55 | 19.6 | 15.10 | 3.9 |
| 7.31 | 5.2 | 15.71 | 4.2 |
| 8.24 | 3.1 | 17.16 | 2.2 |
| 9.57 | 4.2 | 18.13 | 2.2 |
| 10.78 | 2 | 20.29 | 3 |
| 11.42 | 6.1 | 20.29 | 3 |
| 11.61 | 8 | 22.05 | 3.3 |
| 12.43 | 3.7 | | |

Preferably, the crystal form N has the X-ray powder diffraction intensities shown in Table 14. Preferably, the crystal form N has an X-ray powder diffraction pattern substantially as shown in FIG. 30.

**Regarding the metastable crystal form O:**

The present disclosure provides a metastable crystal form O of compound (IE), wherein an X-ray powder diffraction pattern of the crystal form comprises peaks at diffraction angles (2θ) of 4.54±0.2°, 3.37±0.2°, 5.96±0.2°, and 12.21±0.2°.

According to the present disclosure, the crystal form O preferably further comprises peaks at diffraction angles (20) of 7.87±0.2°, 13.89±0.2°, 16.84±0.2°, 21.24±0.2°, and 32.21±0.2°. According to the present disclosure, preferably, the X-ray powder diffraction pattern of the crystal form O has the diffraction angles (2θ) shown in Table 15, wherein the 2θ angles have a margin of error of ±0.20°:

Table 15

| 2θ (°) | Intensity% | 2θ (°) | Intensity% |
|---|---|---|---|
| 3.37 | 15.4 | 13.89 | 2.9 |
| 4.54 | 100 | 16.84 | 2.7 |
| 5.96 | 14.4 | 21.24 | 2.4 |
| 7.87 | 4.4 | 32.21 | 1.3 |
| 12.21 | 5.4 | | |

Preferably, the crystal form O has the X-ray powder diffraction intensities shown in Table 15. Preferably, the crystal form O has an X-ray powder diffraction pattern substantially as shown in FIG. 31.

**Regarding the metastable crystal form P:**

The present disclosure provides a metastable crystal form P of compound (IE), wherein an X-ray powder diffraction pattern of the crystal form comprises peaks at diffraction angles (2θ) of 4.81±0.2°, 4.29±0.2°, 6.08±0.2°, and 14.65±0.2°.

According to the present disclosure, the crystal form P preferably further comprises peaks at diffraction angles (2θ) of 12.33±0.2°, 7.89±0.2°, 3.41±0.2°, 17.94±0.2°, and 8.81±0.2°. According to the present disclosure, the crystal form P more preferably further comprises peaks at diffraction angles (2θ) of 17.57±0.2°, 7.25±0.2°, 13.37±0.2°, 19.60 ±0.2°, and 20.23±0.2°. According to the present disclosure, preferably, the X-ray powder diffraction pattern of the crystal form P has the diffraction angles (20) shown in Table 16, wherein the 2θ angles have a margin of error of ±0.20°:

Table 16

| 2θ (°) | Intensity% | 2θ (°) | Intensity% |
|---|---|---|---|
| 3.41 | 4.8 | 12.33 | 6.5 |
| 4.29 | 71.9 | 13.37 | 2.2 |
| 4.81 | 100 | 14.65 | 8.3 |
| 6.08 | 15.9 | 17.57 | 3.1 |
| 7.25 | 2.2 | 17.94 | 3.6 |
| 7.89 | 5.4 | 19.60 | 2.2 |
| 8.81 | 3.3 | 20.23 | 2.1 |

Preferably, the crystal form P has the X-ray powder diffraction intensities shown in Table 16. Preferably, the crystal form P has an X-ray powder diffraction pattern substantially as shown in FIG. 32.

**Regarding the metastable crystal form Q:**

The present disclosure provides a metastable crystal form Q of compound IE, wherein an X-ray powder diffraction pattern of the crystal form comprises peaks at diffraction angles (2θ) of 4.96±0.2°, 7.42±0.2°, 14.82±0.2°, and 21.77±0.2°.

According to the present disclosure, the crystal form Q preferably further comprises peaks at diffraction angles (2θ) of 16.75±0.2°, 17.29±0.2°, 15.21±0.2°, 25.67±0.2°, and 24.53±0.2°. According to the present disclosure, the crystal form Q more preferably further comprises peaks at diffraction angles (20) of 9.86±0.2°, 15.76±0.2°, 17.56±0.2°, 22.92±0.2°, 23.67±0.2°, and 22.44±0.2°.

According to the present disclosure, preferably, the X-ray powder diffraction pattern of the crystal form Q has the diffraction angles (2θ) shown in Table 17, wherein the 2θ angles have a margin of error of ±0.20°:

Table 17

| 2θ (°) | Intensity% | 2θ (°) | Intensity% |
|---|---|---|---|
| 4.96 | 100 | 20.21 | 0.6 |

(continued)

| 2θ (°) | Intensity% | 2θ (°) | Intensity% |
|---|---|---|---|
| 7.42 | 9.9 | 20.41 | 0.5 |
| 9.86 | 1.4 | 21.77 | 4.4 |
| 12.34 | 0.6 | 22.44 | 0.9 |
| 13.00 | 0.1 | 22.92 | 1 |
| 14.82 | 4.6 | 23.67 | 1 |
| 15.21 | 1.8 | 24.22 | 0.4 |
| 15.76 | 1.3 | 24.53 | 1.5 |
| 15.96 | 0.3 | 24.78 | 0.4 |
| 16.75 | 1.9 | 25.67 | 1.8 |
| 17.29 | 1.9 | 26.81 | 0.2 |
| 17.56 | 1 | 27.29 | 0.1 |
| 18.43 | 0.6 | 27.57 | 0.2 |
| 19.87 | 0.2 | 29.05 | 0.7 |

Preferably, the crystal form Q has the X-ray powder diffraction intensities shown in Table 17. Preferably, the crystal form Q has an X-ray powder diffraction pattern substantially as shown in FIG. 33.

In a second aspect, the present disclosure provides a preparation method for the aforementioned polymorphs of compound (IE), comprising

step 1: dissolving or dispersing compound IE in a solvent; and

step 2: stirring at 0-50 °C for crystallization; or adding an anti-solvent to a clear solution of the compound for precipitation; or slowly volatilizing a clear solution of the compound. According to an embodiment of the present disclosure, the compound IE is preferably the metastable crystal form H of compound IE.

As a further preferred embodiment, the solvent is water, an organic solvent, or a mixed solvent of water with the organic solvent, wherein the organic solvent is selected from alcohols, chloroalkanes, ketones, ethers, cyclic ethers, esters, alkanes, cycloalkanes, benzenes, amides, sulfoxides, and mixtures thereof; preferably, the organic solvent is selected from methanol, ethanol, n-propanol, isopropanol, n-butanol, trifluoroethanol, acetonitrile, acetone, methyl ethyl ketone, methyl isobutyl ketone, 1,4-dioxane, tetrahydrofuran, 2-methyltetrahydrofuran, *N,N*-dimethylformamide, *N*,*N*-dimethylacetamide, *N*-methylpyrrolidone, dimethyl sulfoxide, ethyl acetate, isopropyl acetate, dichloromethane, trichloromethane, trichloroethane, carbon tetrachloride, methyl *tert*-butyl ether, cyclopentyl methyl ether, 2-methoxyethyl ether, isopropyl ether, diethyl ether, *n*-heptane, *n*-hexane, isooctane, pentane, cyclohexane, cyclopentane, methylcyclohexane, benzene, toluene, xylene, and mixtures thereof.

In a third aspect, the present disclosure provides a pharmaceutical composition comprising at least one of the aforementioned polymorphs of compound (IE) and a pharmaceutically acceptable carrier.

In a fourth aspect, the present disclosure provides use of the aforementioned polymorphs of compound (IE) for the manufacturing of a medicament for the prevention and/or treatment of diseases associated with the WEE1 and/or Yes target(s), including conditions such as tumors, inflammations, autoimmune diseases (e.g., lupus erythematosus and psoriasis), etc.

The present disclosure further provides a method for treating a disease, comprising administering to a subject in need thereof a therapeutically effective amount of at least one of the aforementioned polymorphs of compound (IE) or the pharmaceutical composition. According to an embodiment of the present disclosure, the disease is selected from diseases associated with the WEE1 and/or Yes target(s), including conditions such as tumors, inflammations, autoimmune diseases (e.g., lupus erythematosus and psoriasis), etc.

In some embodiments of the present disclosure, the compound, the pharmaceutically acceptable salt, the solvate, the enantiomer, the isotopically substituted compound, and the various crystal forms thereof described above are selected from new compounds of the structures disclosed in the examples of the present disclosure and new salt forms and crystal forms thereof.

The present disclosure further provides a pharmaceutical composition comprising a therapeutically effective amount of at least one of the compound represented by formula (I), the pharmaceutically acceptable salt, the solvate, the

enantiomer, the isotopically substituted compound, and the various crystal forms thereof.

According to an embodiment of the present disclosure, the pharmaceutical composition is formulated for oral, injection, rectal, nasal, pulmonary, topical, buccal and sublingual, vaginal, parenteral, subcutaneous, intramuscular, intravenous, intradermal, intrathecal, or epidural administration.

According to an embodiment of the present disclosure, the pharmaceutical composition is preferably administered orally.

The oral dosage form is not particularly limited, and any oral dosage form well known in the art can be used, preferably including tablets, capsules, suspensions, or oral solutions and other oral dosage forms known in the art.

According to an embodiment of the present disclosure, the pharmaceutical composition may further comprise a pharmaceutically acceptable auxiliary material, including but not limited to, at least one of the following auxiliary materials: a filler, a disintegrant, an adhesive, a lubricant, a surfactant, a flavoring agent, a wetting agent, a pH regulator, a solubilizer or a cosolvent, and a tonicity adjusting agent. Those skilled in the art can easily determine how to select the corresponding auxiliary materials and their corresponding amounts according to the needs of specific dosage forms.

According to an embodiment of the present disclosure, the pharmaceutical composition may further comprise one or more additional therapeutic agents.

Another object of the present disclosure is to provide use of the compounds described above for the manufacturing of a medicament for the prevention and/or treatment of diseases associated with the WEE1 or Yes target, including conditions such as tumors, inflammations, autoimmune diseases (e.g., lupus erythematosus and psoriasis), etc.

The present disclosure further provides use of the compounds represented by formulae (I)-(IE), the pharmaceutically acceptable salts, the solvates, the enantiomers, the isotopically substituted compounds, and the various salt forms and crystal forms thereof, and the pharmaceutical composition for the prevention and/or treatment of diseases associated with the WEE1 and/or Yes signaling pathway(s). The diseases associated with the WEE1 and/or Yes signaling pathway(s) include conditions such as tumors, inflammations, autoimmune diseases (e.g., lupus erythematosus and psoriasis), etc.

The present disclosure further provides a method for preventing and/or treating diseases associated with the WEE1 and/or Yes signaling pathway(s), comprising administering to a patient a prophylactically or therapeutically effective amount of at least one of the compound represented by formula (I), the pharmaceutically acceptable salt, the solvate, the enantiomer, and the isotopically substituted compound thereof, or a prophylactically or therapeutically effective amount of the pharmaceutical composition described above. The diseases associated with the WEE1 or Yes signaling pathway are defined as described above.

In some embodiments, the patient is a mammal, preferably a human.

## BRIEF DESCRIPTION OF THE DRAWINGS

FIG. 1 shows an X-ray powder diffraction pattern of the crystal form A of compound IE of the present disclosure. The abscissa represents 20 values (degrees), and the ordinate represents peak intensity.

FIG. 2 shows a DSC profile of the crystal form A of compound IE of the present disclosure. The abscissa represents temperature (°C), and the ordinate represents heat flow (mW).

FIG. 3 shows a TGA profile of the crystal form A of compound IE of the present disclosure. The abscissa represents temperature (°C), and the ordinate represents weight (%).

FIG. 4 shows an X-ray powder diffraction pattern of the crystal form B of compound IE of the present disclosure. The abscissa represents 2θ values (degrees), and the ordinate represents peak intensity.

FIG. 5 shows a DSC profile of the crystal form B of compound IE of the present disclosure. The abscissa represents temperature (°C), and the ordinate represents heat flow (mW).

FIG. 6 shows a TGA profile of the crystal form B of compound IE of the present disclosure. The abscissa represents temperature (°C), and the ordinate represents weight (%).

FIG. 7 shows an X-ray powder diffraction pattern of the crystal form C of compound IE of the present disclosure. The abscissa represents 20 values (degrees), and the ordinate represents peak intensity.

FIG. 8 shows a DSC profile of the crystal form C of compound IE of the present disclosure. The abscissa represents temperature (°C), and the ordinate represents heat flow (mW).

FIG. 9 shows a TGA profile of the crystal form C of compound IE of the present disclosure. The abscissa represents temperature (°C), and the ordinate represents weight (%).

FIG. 10 shows an X-ray powder diffraction pattern of the hydrate crystal form D of compound IE of the present disclosure. The abscissa represents 20 values (degrees), and the ordinate represents peak intensity.

FIG. 11 shows a DSC profile of the hydrate crystal form D of compound IE of the present disclosure. The abscissa represents temperature (°C), and the ordinate represents heat flow (mW).

FIG. 12 shows a TGA profile of the hydrate crystal form D of compound IE of the present disclosure. The abscissa represents temperature (°C), and the ordinate represents weight (%).

FIG. 13 shows an X-ray powder diffraction pattern of the hydrate crystal form E of compound IE of the present disclosure. The abscissa represents 20 values (degrees), and the ordinate represents peak intensity.

FIG. 14 shows a DSC profile of the hydrate crystal form E of compound IE of the present disclosure. The abscissa represents temperature (°C), and the ordinate represents heat flow (mW).

FIG. 15 shows a TGA profile of the hydrate crystal form E of compound IE of the present disclosure. The abscissa represents temperature (°C), and the ordinate represents weight (%).

FIG. 16 shows an X-ray powder diffraction pattern of the hydrate crystal form F of compound IE of the present disclosure. The abscissa represents 20 values (degrees), and the ordinate represents peak intensity.

FIG. 17 shows a DSC profile of the hydrate crystal form F of compound IE of the present disclosure. The abscissa represents temperature (°C), and the ordinate represents heat flow (mW).

FIG. 18 shows a TGA profile of the hydrate crystal form F of compound IE of the present disclosure. The abscissa represents temperature (°C), and the ordinate represents weight (%).

FIG. 19 shows an X-ray powder diffraction pattern of the hydrate crystal form G of compound IE of the present disclosure. The abscissa represents 20 values (degrees), and the ordinate represents peak intensity.

FIG. 20 shows a DSC profile of the hydrate crystal form G of compound IE of the present disclosure. The abscissa represents temperature (°C), and the ordinate represents heat flow (mW).

FIG. 21 shows a TGA profile of the hydrate crystal form G of compound IE of the present disclosure. The abscissa represents temperature (°C), and the ordinate represents weight (%).

FIG. 22 shows an X-ray powder diffraction pattern of the metastable crystal form H of compound IE of the present disclosure. The abscissa represents 20 values (degrees), and the ordinate represents peak intensity.

FIG. 23 shows a DSC profile of the metastable crystal form H of compound IE of the present disclosure. The abscissa represents temperature (°C), and the ordinate represents heat flow (mW).

FIG. 24 shows a TGA profile of the metastable crystal form H of compound IE of the present disclosure. The abscissa represents temperature (°C), and the ordinate represents weight (%).

FIG. 25 shows an X-ray powder diffraction pattern of the metastable crystal form I of compound IE of the present disclosure. The abscissa represents 20 values (degrees), and the ordinate represents peak intensity.

FIG. 26 shows an X-ray powder diffraction pattern of the metastable crystal form J of compound IE of the present disclosure. The abscissa represents 20 values (degrees), and the ordinate represents peak intensity.

FIG. 27 shows an X-ray powder diffraction pattern of the metastable crystal form K of compound IE of the present disclosure. The abscissa represents 2θ values (degrees), and the ordinate represents peak intensity.

FIG. 28 shows an X-ray powder diffraction pattern of the metastable crystal form L of compound IE of the present disclosure. The abscissa represents 20 values (degrees), and the ordinate represents peak intensity.

FIG. 29 shows an X-ray powder diffraction pattern of the metastable crystal form M of compound IE of the present disclosure. The abscissa represents 20 values (degrees), and the ordinate represents peak intensity.

FIG. 30 shows an X-ray powder diffraction pattern of the metastable crystal form N of compound IE of the present disclosure. The abscissa represents 2θ values (degrees), and the ordinate represents peak intensity.

FIG. 31 shows an X-ray powder diffraction pattern of the metastable crystal form O of compound IE of the present disclosure. The abscissa represents 20 values (degrees), and the ordinate represents peak intensity.

FIG. 32 shows an X-ray powder diffraction pattern of the metastable crystal form P of compound IE of the present disclosure. The abscissa represents 20 values (degrees), and the ordinate represents peak intensity.

FIG. 33 shows an X-ray powder diffraction pattern of the metastable crystal form Q of compound IE of the present disclosure. The abscissa represents 20 values (degrees), and the ordinate represents peak intensity.

FIG. 34 shows a DVS profile of the crystal form A of compound IE of the present disclosure. The abscissa represents relative humidity (%), and the ordinate represents weight change (%).

FIG. 35 shows a DVS profile of the crystal form B of compound IE of the present disclosure. The abscissa represents relative humidity (%), and the ordinate represents weight change (%).

FIG. 36 shows a DVS profile of the crystal form C of compound IE of the present disclosure. The abscissa represents relative humidity (%), and the ordinate represents weight change (%).

FIG. 37 shows a DVS profile of the crystal form D of compound IE of the present disclosure. The abscissa represents relative humidity (%), and the ordinate represents weight change (%).

FIG. 38 shows the single-crystal analysis result of compound IE of the present disclosure.

## Definitions and Description:

$C_{1-10}$ is selected from $C_1$, $C_2$, $C_3$, $C_4$, $C_5$, $C_6$, $C_7$, $C_8$, $C_9$, and $C_{10}$; $C_{2-10}$ is selected from $C_2$, $C_3$, $C_4$, $C_5$, $C_6$, $C_7$, $C_8$, $C_9$, and $C_{10}$; $C_{3-10}$ is selected from $C_3$, $C_4$, $C_5$, $C_6$, $C_7$, $C_8$, $C_9$, and $C_{10}$.

It should be understood that when one, two, or more are described herein, "more" shall mean an integer greater than 2, e.g., an integer greater than or equal to 3, e.g., 3, 4, 5, 6, 7, 8, 9, or 10. The term "halogen" refers to fluorine, chlorine, bromine, and iodine.

The term "alkyl" refers to a linear or branched saturated monovalent hydrocarbyl group having 1-12 carbon atoms, preferably "$C_{1-8}$ alkyl" or "$C_{1-6}$ alkyl". "$C_{1-8}$ alkyl" refers to a linear and branched alkyl group having 1, 2, 3, 4, 5, 6, 7, or 8 carbon atoms. "$C_{1-6}$ alkyl" refers to a linear and branched alkyl group having 1, 2, 3, 4, 5, or 6 carbon atoms. The alkyl is, for example, methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or the like, or an isomer thereof.

The term "alkenyl" should be understood to refer to a linear or branched monovalent hydrocarbyl group containing one or more double bonds and having 2-12 carbon atoms, preferably "$C_{2-10}$ alkenyl" or "$C_{2-6}$ alkenyl". "$C_{2-10}$ alkenyl" should be understood to preferably refer to a linear or branched monovalent hydrocarbyl group containing one or more double bonds and having 2, 3, 4, 5, 6, 7, 8, 9, or 10 carbon atoms. "$C_{2-6}$ alkenyl" should be understood to preferably refer to a linear or branched monovalent hydrocarbyl group containing one or more double bonds and having 2, 3, 4, 5, or 6 carbon atoms. It should be understood that in the case that the alkenyl contains more than one double bond, the double bonds can be separated from one another or conjugated. The alkenyl is, for example, ethenyl, allyl, (*E*)-2-methylethenyl, (*Z*)-2-methylethenyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, or 1-isopropylvinyl.

The term "haloalkyl" refers to an alkyl group substituted with one, two, or more halogens, wherein the halogen and the alkyl group are as defined above. For example, "$C_{1-6}$ haloalkyl" means a $C_{1-6}$ group substituted with one, two, or more halogens, wherein the halogen and the $C_{1-6}$ group are as defined above.

The term "deuterated alkyl" refers to an alkyl group substituted with one, two, or more deuterium atoms, wherein the alkyl group is as defined above. For example, "deuterated $C_{1-6}$ alkyl" means a $C_{1-6}$ group substituted with one, two, or more deuterium atoms, wherein the $C_{1-6}$ group is as defined above.

The term "alkylene" refers to a divalent alkyl, wherein the alkyl is as defined above. The alkylene is preferably an alkylene group having 1 to 6 carbon atoms (i.e., $C_{1-6}$ alkylene).

The term "alkenylene" refers to a divalent alkenyl, wherein the alkenyl is as defined above. The alkenylene is preferably an alkenylene group having 2 to 6 carbon atoms (i.e., $C_{2-6}$ alkenylene). The term "alkynyl" should be understood to refer to a linear or branched monovalent hydrocarbyl group containing one or more triple bonds and having 2-12 carbon atoms, preferably "$C_{2-10}$ alkynyl" or $C_{2-6}$ alkynyl. The term "$C_{2-10}$ alkynyl" should be understood to preferably refer to a linear or branched monovalent hydrocarbyl group containing one or more triple bonds and having 2, 3, 4, 5, 6, 7, 8, 9, or 10 carbon atoms, for example, "$C_{2-8}$ alkynyl" has 2, 3, 4, 5, 6, 7, or 8 carbon atoms, "$C_{2-6}$ alkynyl" has 2, 3, 4, 5, or 6 carbon atoms, or "$C_{2-3}$ alkynyl" has 2 or 3 carbon atoms. The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

As used herein, the term "one or more" means one or more than one, e.g., 1, 2, 3, 4, 5, or more. The term "alicyclic", "carbocycle (group)", or "cycloalkyl" refers to a saturated or partially unsaturated monocyclic or polycyclic cyclic hydrocarbyl group, wherein the carbocycle may contain 3 to 20 carbon atoms, preferably 3 to 12 (e.g. 3, 4, 5, 6, 7, 8, 9, 10, 11, or 12) carbon atoms, and more preferably 3 to 6 carbon atoms. The carbocycle may be monocyclic or polycyclic, and may be a saturated cycloalkyl group or may be a cycloalkenyl or cycloalkynyl group optionally containing one, two, or more double and/or triple bonds on its ring. In the case that the carbocycle has multiple rings, these rings may form spiro-ring, fused-ring, and bridged-ring structures. For example, non-limiting examples of a monocyclic carbocycle include cyclopropyl, cyclobutyl, cyclopentyl, cyclopentenyl, cyclohexyl, cyclohexenyl, cyclohexadienyl, cycloheptyl, cycloheptatrienyl, cyclooctyl, cyclooctatetraenyl, etc.; non-limiting examples of a polycyclic carbocycle include decalinyl or isobornyl.

The term "cycloalkyl" should be understood to refer to a saturated monovalent monocyclic or bicyclic (e.g., fused-ring, bridged-ring, or spiro-ring) hydrocarbon ring or tricyclic alkane having 3-12 carbon atoms, preferably "$C_{3-10}$

cycloalkyl", and more preferably "$C_{3-6}$ cycloalky". The term "$C_{3-10}$ cycloalkyl" should be understood to refer to a saturated monovalent monocyclic or bicyclic (e.g., bridged-ring or spiro-ring) hydrocarbon ring or tricyclic alkane having 3, 4, 5, 6, 7, 8, 9, or 10 carbon atoms. The $C_{3-10}$ cycloalkyl may be monocyclic hydrocarbyl such as cyclopropyl, cyclobutyl, cyclopentyl, cyclohexyl, cycloheptyl, cyclooctyl, cyclononyl, or cyclodecyl, or bicyclic hydrocarbyl such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, or 2,6-diazaspiro[3,4]octyl, or tricyclic hydrocarbyl such as adamantyl.

The term "aryl" or "aromatic ring" should be understood to preferably refer to an aromatic or partially aromatic monovalent monocyclic, bicyclic (e.g., fused-ring, bridged-ring, or spiro-ring), or tricyclic hydrocarbon ring having 6-20 carbon atoms, and may be an aromatic monocyclic ring or a fused aromatic polycyclic ring, preferably "$C_{6-14}$ aryl". The term "$C_{6-14}$ aryl" should be understood to preferably refer to an aromatic or partially aromatic monovalent monocyclic, bicyclic, or tricyclic hydrocarbon ring having 6, 7, 8, 9, 10, 11, 12, 13, or 14 carbon atoms ("$C_{6-14}$ aryl"), in particular a ring having 6 carbon atoms ("$C_6$ aryl"), such as phenyl or biphenyl, a ring having 9 carbon atoms ("$C_9$ aryl"), such as indanyl or indenyl, a ring having 10 carbon atoms ("$C_{10}$ aryl"), such as tetrahydronaphthyl, dihydronaphthyl, or naphthyl, a ring having 13 carbon atoms ("$C_{13}$ aryl"), such as fluorenyl, or a ring having 14 carbon atoms ("$C_{14}$ aryl"), such as anthryl. When the $C_{6-20}$ aryl is substituted, it may be monosubstituted or polysubstituted. In addition, the substitution site is not limited, and the substitution may be, for example, ortho-substitution, para-substitution, or meta-substitution.

The term "spiro-ring" refers to a ring system in which two rings share one ring-forming atom, and may contain the aforementioned alicyclic, heterocyclic, aromatic, or heteroaromatic ring. The term "fused-ring" refers to a ring system in which two rings share two ring-forming atoms, and may contain the aforementioned alicyclic, heterocyclic, aromatic, or heteroaromatic ring. The term "bridged-ring" refers to a ring system in which two rings share three or more ring-forming atoms, and may contain the aforementioned alicyclic, heterocyclic, aromatic, or heteroaromatic ring. The term "heterocyclic (group)" refers to a saturated or partially unsaturated monocyclic or polycyclic hydrocarbyl containing 3 to 20 ring atoms, wherein one or more of the ring atoms are heteroatoms or radical groups selected from N, O, NH, S(O), and $S(O)_2$, but exclude a cyclic moiety of -O-O-, -O-S-, or -S-S-, and the remaining ring atoms are carbon atoms. Preferably, it contains 3 to 12 ring atoms wherein 1-4 (e.g., 1, 2, 3, and 4) of the ring atoms are heteroatoms; more preferably, it contains 3 to 6 (e.g., 3, 4, 5, or 6) ring atoms. The heterocyclyl may be attached to the remainder of the molecule via any one of the carbon atoms or a nitrogen atom (if present) or an oxygen or sulfur atom (particularly where an onium salt is formed). The heterocyclyl may include a fused or bridged ring and/or a spiro ring. Non-limiting examples of a monocyclic heterocyclyl group include azetidinyl, oxetanyl, pyrrolidinyl, imidazolidinyl, tetrahydrofuranyl, tetrahydrothienyl, dihydroimidazolyl, dihydrofuranyl, dihydropyrazolyl, dihydropyrrolyl, dioxolyl, tetrahydropyranyl, pyrrolinyl, piperidyl, piperazinyl, morpholinyl, thiomorpholinyl, dithianyl, trithianyl, homopiperazinyl, diazepanyl, etc.; preferably piperidyl or pyrrolidinyl. Polycyclic heterocyclyl groups include spiro-ring, fused-ring, and bridged-ring heterocyclyl groups and may also be benzo-fused heterocyclyl groups such as dihydroisoquinolyl. The heterocyclyl may be bicyclic, and its non-limiting examples include hexahydrocyclopenta[c]pyrrol-2(1*H*)-yl and hexahydropyrrolo[1,2-*a*]pyrazin-2(1*H'*)-yl. Heterocyclyl may also be partially unsaturated, i.e., it may contain one or more double bonds, and its non-limiting examples include dihydrofuranyl, dihydropyranyl, 2,5-dihydro-1*H*-pyrrolyl, 4*H*-[1,3,4]thiadiazinyl, 4,5-dihydrooxazolyl, or 4*H*-[1,4]thiazinyl.

The term "heterocyclyl" refers to a saturated or unsaturated non-aromatic ring or ring system. For example, it is a 4-, 5-, 6-, or 7-membered monocyclic, 7-, 8-, 9-, 10-, 11-, or 12-membered bicyclic (e.g., fused-ring, bridged-ring, or spiro-ring), or 10-, 11-, 12-, 13-, 14-, or 15-membered tricyclic ring system, and contains at least one, e.g., 1, 2, 3, 4, 5, or more heteroatoms selected from O, S, and N, wherein N and S may also be optionally oxidized to various oxidation states to form nitrogen oxides,-S(O)-, or $-S(O)_2-$. Preferably, the heterocyclyl may be selected from "3- to 10-membered heterocyclyl". The term "3- to 10-membered heterocyclyl" refers to a saturated or unsaturated non-aromatic ring or ring system and contains at least one heteroatom selected from O, S, and N. The heterocyclyl may be attached to the remainder of the molecule via any one of the carbon atoms or a nitrogen atom (if present). The heterocyclyl may include a fused or bridged ring as well as a spiro ring. In particular, the heterocyclyl may include, but are not limited to, 4-membered rings such as azetidinyl and oxetanyl; 5-membered rings such as tetrahydrofuranyl, dioxolyl, pyrrolidinyl, imidazolidinyl, pyrazolidinyl, and pyrrolinyl; or 6-membered rings such as tetrahydropyranyl, piperidyl, morpholinyl, dithianyl, thiomorpholinyl, piperazinyl, and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[c]pyrrol-2(1H)-yl ring, or a 5,6-membered bicyclic ring such as a hexahydropyrrolo[1,2-*a*]pyrazin-2(1*H*)-yl ring. The heterocyclyl may be partially unsaturated, i.e., it may contain one or more double bonds, for example, but not limited to, dihydrofuranyl, dihydropyranyl, 2,5-dihydro-1*H*-pyrrolyl, 4*H*-[1,3,4]thiadiazinyl, 1,2,3,5-tetrahydrooxazolyl, or 4*H*-[1,4]thiazinyl, or it may be benzo-fused, for example, but not limited to, dihydroisoquinolyl. When the 3- to 20-membered heterocyclyl is connected to another group to form a compound of the present disclosure, it may be a carbon atom on the 3- to 20-membered heterocyclyl that is connected to the group, or it may be a heteroatom on the 3- to 20-membered heterocyclyl that is connected to the group. For example, when the 3- to 20-membered heterocyclyl is piperazinyl, it may be a nitrogen atom on piperazinyl that is connected to the group. Alternatively, when the 3- to 20-membered heterocyclyl is

piperidyl, it may be the nitrogen atom on piperidyl or the carbon atom in the para position that is connected to the group. The heterocyclyl may be optionally substituted or unsubstituted, and when it is substituted, the substituent is preferably one or more independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, and carboxylate group. As used herein, the term "heteroaryl/heteroaromatic ring" refers to a heteroaromatic system comprising 1 to 4 heteroatoms and 5 to 20 ring atoms, wherein the heteroatom is selected from oxygen, sulfur, nitrogen, and phosphorus. Preferably, the heteroaryl is 5- to 10-membered (e.g. 5-, 6-, 7-, 8-, 9-, or 10-membered), more preferably, 5- or 6-membered. Non-limiting examples of a heteroaryl group include, but are not limited to, thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl, etc., and benzo derivatives thereof, such as benzofuranyl, benzothienyl, benzoxazolyl, benzoisoxazolyl, benzimidazolyl, benzotriazolyl, indazolyl, indolyl, and isoindolyl; or pyridinyl, pyridazinyl, pyrimidinyl, pyrazinyl, triazinyl, etc., and benzo derivatives thereof, such as quinolyl, quinazolinyl, and isoquinolyl; or azocinyl, indolizinyl, purinyl, etc., and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, and/or phenoxazinyl, etc.

The heteroaryl/heteroaromatic ring may be optionally substituted or unsubstituted, and when it is substituted, the substituent is preferably one, two, or more groups independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, and carboxylate group.

Unless otherwise stated, the heterocyclyl, heteroaryl, or heteroaromatic ring includes all possible isomeric forms thereof, e.g., position isomers thereof. Thus, for some illustrative non-limiting examples, forms that involving substitutions at or bonding to other groups at one, two, or more of positions 1, 2, 3, 4, 5, 6, 7, 8, 9, 10, 11, 12, etc. (if present) may be included, including pyridin-2-yl, pyridinylene-2-yl, pyridin-3-yl, pyridinylene-3-yl, pyridin-4-yl, and pyridinylene-4-yl; thienyl or thienylene, including thien-2-yl, thien-2-ylene, thien-3-yl, and thien-3-ylene; and pyrazol-1-yl, pyrazol-3-yl, pyrazol-4-yl, and pyrazol-5-yl.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having a particular substituent disclosed herein and a relatively nontoxic acid or base. When the compounds of the present disclosure contain a relatively acidic functional group, a base addition salt can be obtained by contacting the neutral form of such a compound with a sufficient amount of a base in a pure solution or a suitable inert solvent. A pharmaceutically acceptable base addition salt includes sodium, potassium, calcium, ammonium, organic amine, or magnesium salt, or a similar salt. When the compounds of the present disclosure contain a relatively basic functional group, an acid addition salt can be obtained by contacting the neutral form of such a compound with a sufficient amount of an acid in a pure solution or a suitable inert solvent. Examples of a pharmaceutically acceptable acid addition salt include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, phosphorous acid, etc.; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid, and the like; salts of amino acids (e.g., arginine and the like) and salts of organic acids such as glucuronic acid and the like (see Berge et al., "Pharmaceutical Salts", Journal of Pharmaceutical Science, 66:1-19 (1977)). Certain specific compounds of the present disclosure contain both basic and acidic functional groups that allow the compounds to be converted into either a base or acid addition salt.

Preferably, the neutral form of the compound is regenerated by contacting the salt with a base or acid and isolating the parent compound in a conventional manner. The parent form of the compound differs from its various salt forms in certain physical properties, such as solubility in a polar solvent.

As used herein, "pharmaceutically acceptable salt" is a derivative of the compound of the present disclosure, wherein the parent compound is modified by salt formation with an acid or with a base. Examples of a pharmaceutically acceptable salt include, but are not limited to, an inorganic or organic acid salt of a base such as amine, an alkali metal or organic salt of an acid group such as carboxylic acid, etc. A pharmaceutically acceptable salt includes a conventional non-toxic salt such as Na salt, potassium salt, amine salt, quaternary ammonium salt of the parent compound, etc. Conventional non-toxic salts include, but are not limited to, those derived from inorganic or organic acids, and inorganic or organic bases, wherein the inorganic or organic acids are selected from 2-acetoxybenzoic acid, 2-hydroxyethanesulfonic acid, acetic acid, ascorbic acid, benzenesulfonic acid, benzoic acid, bicarbonate, carbonic acid, citric acid, edetic acid, ethanedisulfonic acid, ethanesulfonic acid, fumaric acid, glucoheptose, gluconic acid, glutamic acid, glycolic acid, hydrobromic acid, hydrochloric acid, hydroiodic acid, hydroxyl, hydroxynaphthalene, isethionic acid, lactic acid, lactose,

dodecylsulfonic acid, maleic acid, malic acid, mandelic acid, methanesulfonic acid, nitric acid, oxalic acid, embonic acid, pantothenic acid, phenylacetic acid, phosphoric acid, polygalacturonic acid, propionic acid, salicylic acid, stearic acid, succinic acid, sulfamic acid, *p*-aminobenzenesulfonic acid, sulfuric acid, tannin, tartaric acid, *p*-toluenesulfonic acid, etc.; the inorganic and organic bases are selected from hydroxides of Na, potassium, magnesium, calcium, etc., and amines, diethylamine, triethylamine, ethanolamine, etc.

The pharmaceutically acceptable salts of the present disclosure can be synthesized from a parent compound having an acidic or basic group by conventional chemical methods. In general, such salts are prepared by the following method: reacting the free acid or base form of the compound with a stoichiometric amount of the appropriate base or acid in water or an organic solvent or a mixture thereof. Generally, nonaqueous media such as an ether, ethyl acetate, ethanol, isopropanol, or acetonitrile, etc., are preferred.

In addition to the salt forms, the compounds provided by the present disclosure also have prodrug forms. Prodrugs of the compounds described herein readily undergo chemical changes under physiological conditions to convert to the compounds of the present disclosure. In addition, prodrugs can be converted to the compounds of the present disclosure in an *in vivo* environment by chemical or biochemical methods.

Certain compounds of the present disclosure may exist in non-solvated forms or solvated forms, including hydrated forms. In general, the solvated forms are comparable to the non-solvated forms, and both are encompassed within the scope of the present disclosure. Certain compounds of the present disclosure may exist in a polymorphic or amorphous form.

As used herein, the term "solvate" refers to an association formed from one or more solvent molecules and the compound of the present disclosure. The solvent for forming the solvate includes, but are not limited to, water, isopropanol, ethanol, methanol, dimethyl sulfoxide, ethyl acetate, acetic acid, and aminoethanol. Thus, the term "hydrate" refers to an association in which the solvent molecule is water.

Unless otherwise stated, the following terms used in the specification and the claims have the following meanings. A particular phrase or term, unless otherwise specifically defined, should not be considered uncertain or unclear, but construed according to its common meaning. When referring to a trade name, it is intended to refer to its corresponding commercial product or its active ingredient.

"Pharmaceutical composition" refers to contain one or more of the compounds described herein or a physiologically/pharmaceutically acceptable salt or a mixture of prodrug thereof and other chemical component, as well as other component such as a physiological/pharmaceutically acceptable carrier and excipient. The pharmaceutical composition aims to facilitate administration to an organism, promote absorption of the active ingredient, and thereby exert biological activity.

As used herein, "polymorph" refers to crystal forms having the same chemical composition but different spatial arrangements of the molecules, atoms, and/or ions that make up the crystal. Despite the same chemical composition, polymorphs differ in packing and geometric arrangement and may exhibit different physical properties such as melting point, shape, color, density, hardness, deformability, stability, solubility, dissolution rate, and the like. Two polymorphs may be either monotropic or enantiotropic, depending on their temperature-stability relationships. For a monotropic system, the relative stability between the two solid phases remains unchanged upon temperature changes. In contrast, in an enantiotropic system, there is a transition temperature at which the stability of the two phases switches ((Theory and Origin of Polymorphism in "Polymorphism in Pharmaceutical Solids" (1999) ISBN:)-8247-0237). The phenomenon of a compound existing in different crystal structures is referred to as pharmaceutical polymorphism.

The various crystal structures of the present disclosure can be distinguished from each other using various analytical techniques known to those of ordinary skill in the art. Such techniques include, but are not limited to, X-ray powder diffraction (XRPD), differential scanning calorimetry (DSC), and/or thermogravimetric analysis (TGA).

As used herein, the term "room temperature" or "RT" refers to an ambient temperature of 20 °C to 25 °C (68 °F-77 °F).

The term "essentially the same" as used herein regarding X-ray diffraction peak positions means considering the typical variability in peak positions and intensities. For example, those skilled in the art will appreciate that the peak position (2θ) measurement may vary depending on the XRPD instrument; in some cases, this variation can be as much as 0.2°. Furthermore, those skilled in the art will appreciate that factors such as the XRPD sample preparation method, the XRPD instrument, the crystallinity of the sample, the amount of the sample, and the preferred crystal orientation will lead to changes in the relative peak intensities in the XRPD diffraction pattern of the sample.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art. Preferred embodiments include but are not limited to the examples of the present disclosure.

The chemical reactions of the specific embodiments of the present disclosure are carried out in a suitable solvent that must be suitable for the chemical changes in the present disclosure and the reagents and materials required. In order to obtain the compounds of the present disclosure, it is sometimes necessary for those skilled in the art to modify or select a

synthesis procedure or a reaction scheme based on the existing embodiments.

The present disclosure is described in detail below through examples; however, these examples are not intended to limit the present disclosure in any way.

All solvents used in the present disclosure are commercially available and can be used without further purification.

Unless otherwise specified, all reactions of the present disclosure are carried out under continuous magnetic stirring in dry solvents, with temperatures expressed in degrees Celsius (°C).

Certain compounds of the present disclosure may have an asymmetric carbon atom (optical center) or double bond. Racemates, diastereoisomers, geometric isomers, and individual isomers are all included within the scope of the present disclosure.

The illustration of the racemic, ambiscalemic and scalemic, or enantiomerically pure compounds herein is from Maehr, J. Chem. Ed. 1985, 62:114-120. Unless otherwise stated, the absolute configuration of a stereogenic center is represented by a wedged bond and a dashed bond. When the compounds described herein contain olefinic double bonds or other geometric asymmetric centers, they include *E* and *Z* geometric isomers unless otherwise specified. Likewise, all tautomeric forms are included within the scope of the present disclosure.

The compounds of the present disclosure may be in the form of a specific geometric isomer or stereoisomer. All such compounds are contemplated herein, including *cis* and *trans* isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present disclosure. Substituents such as alkyl may have an additional asymmetric carbon atom. All these isomers and mixtures thereof are encompassed within the scope of the present disclosure. Optically active (*R*)- and (*S*)-isomers and *D* and *L* isomers can be prepared by chiral synthesis or chiral reagents or other conventional techniques. If one enantiomer of a certain compound of the present disclosure is desired, the desired pure enantiomer can be prepared by asymmetric synthesis or derivatization using a chiral auxiliary, wherein the resulting diastereoisomeric mixture is separated and the auxiliary group is cleaved. Alternatively, when the molecule contains a basic functional group (such as amino) or an acidic functional group (such as carboxyl), the compound reacts with an appropriate optically active acid or base to form a salt of the diastereoisomer, which is then subjected to resolution of diastereoisomers through fractional crystallization or chromatography known in the art to acquire the pure enantiomer. Furthermore, the enantiomer and the diastereoisomer are generally isolated through chromatography using a chiral stationary phase, optionally in combination with chemical derivatization (e.g., generating carbamate from amines).

The compounds of the present disclosure may contain an unnatural proportion of atomic isotope at one or more of the atoms that constitute the compounds. For example, the compounds may be labeled with a radioisotope, such as tritium ($^{3}H$), iodine-125 ($^{125}I$), or C-14 ($^{14}C$). All isotopic variations of the compounds disclosed herein, whether radioactive or not, are encompassed within the scope of the present disclosure.

The term "pharmaceutically acceptable carrier" refers to any formulation or carrier medium capable of delivering an effective amount of the active substance of the present disclosure without interfering with the biological activity of the active substance and having no toxic and side effects on the host or patient. Representative carriers include water, oils, vegetables and minerals, cream bases, lotion bases, ointment bases, and the like. Such bases include suspending agents, thickeners, transdermal enhancers, and the like. Their formulations are well known to those skilled in the cosmetic or topical pharmaceutical field. For additional information on vectors, reference may be made to Remington: The Science and Practice of Pharmacy, 21st Ed., Lippincott, Williams & Wilkins (2005), the contents of which are incorporated herein by reference.

When any variable (e.g., R) occurs more than once in the constitution or structure of a compound, the variable is independently defined in each case. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound. When a bond of a substituent can be cross-linked to two atoms on a ring, the substituent can be bonded to any atom on the ring. When the substituents listed are not indicated by which atom they are attached to the compounds included in the general chemical structural formula but not specifically mentioned, such substituents may be bonded through any atom thereof. A combination of a substituent and/or a variant thereof is permissible only if the combination can result in a stable compound.

The term "halo" or "halogen" refers to fluorine, chlorine, bromine, and iodine.

The present disclosure will now be further described with examples. The following examples are given for illustrative purposes only and are not intended to limit the scope of the present disclosure. The compounds of the present disclosure can be prepared by many methods known in the art of organic synthesis. Examples of the present disclosure may be synthesized using the methods described below, as well as synthetic methods known in the art of organic synthetic chemistry, or improved methods based thereon. Preferred methods include, but are not limited to, the methods described below.

Unless otherwise specified, all solvents used in the present disclosure were commercially available and were

used without further purification. The reactions were generally carried out under an inert atmosphere of nitrogen using an anhydrous solvent. The nuclear magnetic resonance analyses were carried out on a Bruker-Avance-400 (400 MHz) spectrometer, and chemical shifts are reported in δ (ppm) form. The mass spectrometry analyses were carried out using an Agilent 1200 series (plus 6110 /and 1956A) LC/MS or Shimadzu MS (DAD: SPD-M20A(LC)) and a Shimadzu Micromass 2020 detector. The mass spectrometer was equipped with an electrospray ionization (ESI) source operating in either positive or negative mode. The present disclosure employs the following abbreviations: aq stands for water; DCM stands for dichloromethane; PE stands for petroleum ether; DMF stands for *N*,*N*-dimethylformamide; DMSO stands for dimethyl sulfoxide; EtOAc stands for ethyl acetate; EtOH stands for ethanol; MeOH stands for methanol; Cbz stands for benzyloxycarbonyl, an amine protecting group; Boc stands for *tert*-butyloxycarbonyl, an amine protecting group; HOAc stands for acetic acid; $NaBH(OAc)_3$ stands for sodium triacetoxyborohydride; r.t stands for room temperature; THF stands for tetrahydrofuran; $Boc_2O$ stands for di-*tert*-butyl dicarbonate; TFA stands for trifluoroacetic acid; DIPEA stands for diisopropylethylamine; $Pd(dppf)Cl_2$ stands for [1,1'-bis(diphenylphosphino)ferrocene]palladium(II) dichloride; $POCl_3$ stands for phosphorus oxychloride; NaH stands for sodium hydride; LAH stands for lithium aluminum hydride; $Pd(OAc)_2$ stands for palladium(II) acetate; $Pd_2(dba)_3$ stands for tris(dibenzylideneacetone)dipalladium(0); $Pd(PPh_3)_4$ stands for tetrakis(triphenylphosphine)palladium(0); $Et_3SiH$ stands for triethylsilane; $PPh_3$ stands for triphenylphosphine; Xantphos stands for 4,5-bis(diphenylphosphino)-9,9-dimethylxanthene; $MeSO_3H$ stands for methanesulfonic acid; Xphos stands for 2-dicyclohexylphosphino-2',4',6'-triisopropylbiphenyl; Lawesson reagent refers to 2,4-bis(4-methoxyphenyl)-1,3-dithia-2,4-diphosphetane-2,4-disulfide; NBS stands for N-bromosuccinimide; t-BuOK stands for potassium tert-butoxide; DIPEA is diisopropylethylamine; $SOCl_2$ is thionyl chloride; $CS_2$ is carbon disulfide; TsOH is 4-toluenesulfonic acid; MTBE is methyl *tert*-butyl ether; i-PrOH is 2-propanol; DAST is diethylaminosulfur trifluoride; DIAD is diisopropyl azodicarboxylate; DEAD is diethyl azodicarboxylate; DBAD is di-*tert*-butyl azodicarboxylate; TES is triethylsilane; LDA is lithium diisopropylamide; NBS is *N*-bromosuccinimide; NIS is *N*-iodosuccinimide; NCS is *N*-chlorosuccinimide; DMP is DMP reagent; DMF-DMA is 1,1-dimethoxy-*N*,*N*-dimethylmethylamine; TMP is 2,2,6,6-tetramethylpiperidine; NMO is *N*-methylmorpholine *N*-oxide; TBSC is *tert*-butyldimethylsilyl chloride; SEMC is 2-(trimethylsilyl)ethoxymethyl chloride; NFSI is *N*-fluorobenzenesulfonamide; AIBN is azobisisobutyronitrile; EDCI is 1-ethyl-3-(3-dimethylaminopropyl)carbodiimide; HOBT is hydroxybenzotriazole; TBAF is tetra-*n*-butylammonium fluoride; HATU is 1-[bis(dimethylamino)methylene]-1*H*-1,2,3-triazolo[4,5-*b*]pyridinium 3-oxide hexafluorophosphate; Xphos is 2-dicyclohexylphosphino-2',4',6'-triisopropylbiphenyl; cataCXium A is di(1-adamantyl)-*n*-butylphosphine; DPPP is 1,3-bis(diphenylphosphino)propane; DPPF is 1,1'-bis(diphenylphosphino)ferrocene; HMTA is 1,3,5,7-tetraazaadamantane; PMBC is *p*-methoxybenzyl chloride; HEPES is 4-(2-hydroxyethyl)-1-piperazineethanesulfonic acid; EGTA is ethylene glycol bis(2-aminoethyl ether)-*N,N,N',N'*-tetraacetic acid.

A compound may be named manually or using ChemDraw®; if it is purchased commercially, the supplier's catalog name may be used. TLC or LC-MS is usually used to determine whether a reaction is completed.

## DETAILED DESCRIPTION

In order to illustrate the present disclosure in more detail, the following examples are given; however, the scope of the present disclosure is not limited to these examples.

### Example 1: Synthesis of (*R*,*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound IE)

#### 1) Synthesis of 2-(6-bromopyridin-2-yl)hex-5-en-2-ol

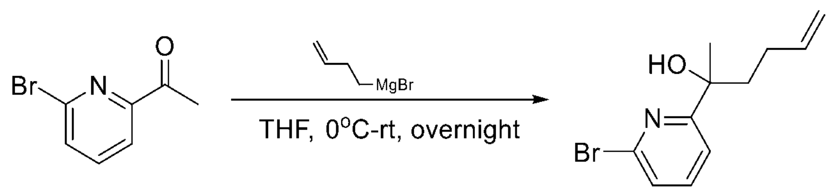


But-3-en-1-methylmagnesium bromide (150 mL, 75.0 mmol) was added to a solution of 1-(6-bromopyridin-2-yl) ethan-1-one (10.0 g, 50.0 mmol) in tetrahydrofuran (100 mL) at 0 °C. The resulting mixed reaction solution was stirred at room temperature overnight under an argon atmosphere. The mixed reaction solution was quenched with a saturated aqueous ammonium chloride solution (200 mL) and extracted with ethyl acetate (3 × 200 mL) at 0 °C. The combined

organic phases were washed with saturated brine (1 × 100 mL), dried over anhydrous sodium sulfate, filtered, and concentrated under reduced pressure. The residue was purified by silica gel column chromatography (ethyl acetate:petroleum ether = 0%-30%) to give 2-(6-bromopyridin-2-yl)hex-5-en-2-ol (6.0 g, yield: 47%), LC-MS m/z: 256 [M + H]$^+$.

**2) Synthesis of 2-allyl-1-(6-(2-hydroxyhex-5-en-2-yl)pyridin-2-yl)-6-(methylthio)-1,2-dihydro-3*H*-pyrazolo[3,4-*d*]pyrimidin-3-one**

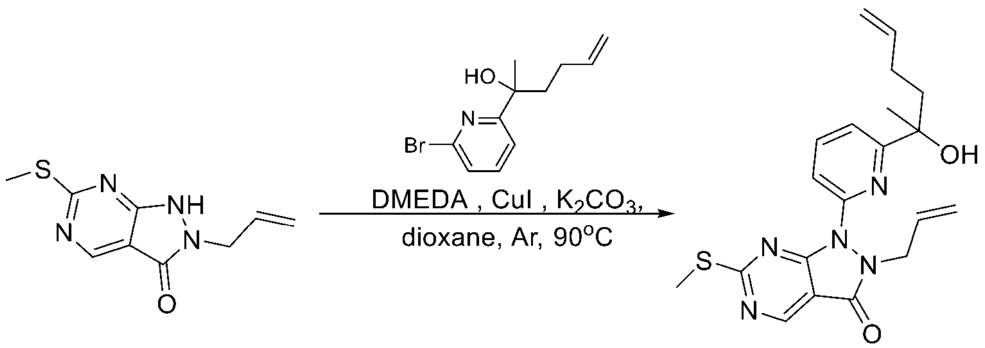


A solution of 2-allyl-6-(methylthio)-1,2-dihydro-3*H*-pyrazolo[3,4-*d*]pyrimidin-3-one (5.00 g, 22.5 mmol), 2-(6-bromopyridin-2-yl)hex-5-en-2-ol (5.75 g, 22.4 mmol), *N,N*-dimethylethylenediamine (2.18 g, 24.7 mmol), copper(I) iodide (4.25 g, 22.3 mmol), and potassium carbonate (4.34 g, 31.4 mmol) in dioxane (60 mL) was heated to 90 °C and stirred overnight under an argon atmosphere. After cooling, the resulting reaction solution was diluted with water (100 mL) and extracted with ethyl acetate (3 × 100 mL). The combined organic phases were washed with saturated brine (1 × 100 mL), dried over anhydrous sodium sulfate, filtered, and then concentrated under reduced pressure. The residue was purified by silica gel column chromatography (ethyl acetate:petroleum ether = 0%-50%) to give 2-allyl-1-(6-(2-hydroxyhex-5-en-2-yl)pyridin-2-yl)-6-(methylthio)-1,2-dihydro-3*H*-pyrazolo[3,4-*d*]pyrimidin-3-one (5.25 g, yield: 58.7%), LCMS m/z: 398 [M + H]$^+$.

**3) Synthesis of (*Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one**

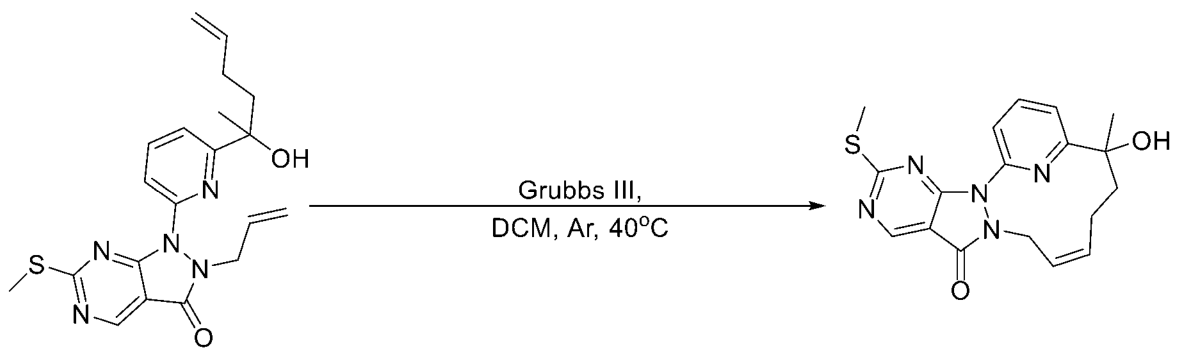


A solution of 2-allyl-1-(6-(2-hydroxyhex-5-en-2-yl)pyridin-2-yl)-6-(methylthio)-1,2-dihydro-3H pyrazolo[3,4-*d*]pyrimidin-3-one (5.25 g, 13.2 mmol) and Grubbs III (1.17 g, 1.32 mmol) in dichloromethane (1.6 L) was heated to 40 °C and stirred overnight under an argon atmosphere. After cooling, the mixed reaction solution was concentrated under pressure. The residue was purified by silica gel column chromatography (petroleum ether:ethyl acetate = 20%-65%) to give (*Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one (510 mg, yield: 10.5%), and 2-allyl-1-(6-(2-hydroxyhex-5-en-2-yl)pyridin-2-yl)-6-(methylthio)-1,2-dihydro-3*H*-pyrazolo[3,4-*d*]pyrimidin-3-one (4.0 g, yield: 76.1%) was recovered, LC-MS m/z: 370 [M + H]$^+$.

**4) Synthesis of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one**

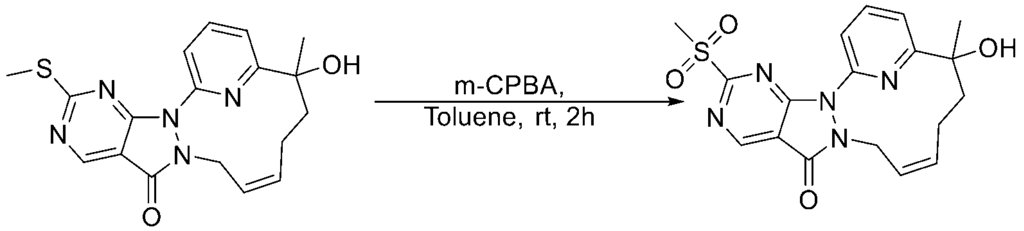


A solution of (*Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one (510 mg, 1.38 mmol) and *m*-chloroperoxybenzoic acid (500 mg, 2.46 mmol) in toluene (50 mL) was stirred at room temperature for 3 h. The resulting reaction solution was concentrated under reduced pressure to give a crude product, which was used directly in the next reaction without further purification. LC-MS *m/z:* 402 $[M + H]^+$;

**5) Synthesis of 1-methyl-4-(2-methyl-4-nitrophenoxy)piperidine**

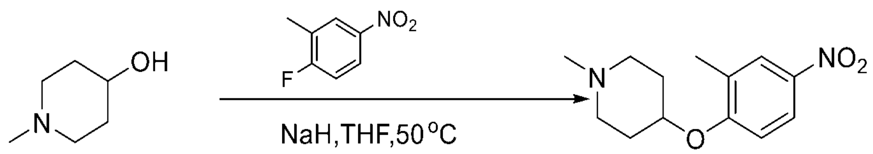


Sodium hydride (3.47 g, 86.82 mmol, content: 60%) was added in batches to a solution of 1-methylpiperidin-4-ol (5 g, 43.41 mmol) in tetrahydrofuran (50 mL) at 0 °C. The resulting mixed reaction solution was stirred at room temperature for 0.5 h. Then, 1-fluoro-2-methyl-4-nitrobenzene (7.4 g, 47.7 mmol) was added in batches to the above mixed reaction solution. The resulting mixed reaction solution was heated to 50 °C and stirred for 17 h. The reaction solution was quenched with a saturated aqueous ammonium chloride solution (50 mL) and extracted with dichloromethane (1 × 50 mL) at 0 °C. The combined organic phases were washed with saturated brine (1 × 50 mL), dried over anhydrous sodium sulfate, filtered, and concentrated under reduced pressure. The residue was purified by silica gel column chromatography (methanol:dichloromethane = 0%-10%) to give 1-methyl-4-(2-methyl-4-nitrophenoxy)piperidine (6.5 g, yield: 59.82%), LC-MS m/z: 251 $[M + H]^+$.

**6) Synthesis of 3-methyl-4-((1-methylpiperidin-4-yl)oxy)aniline**

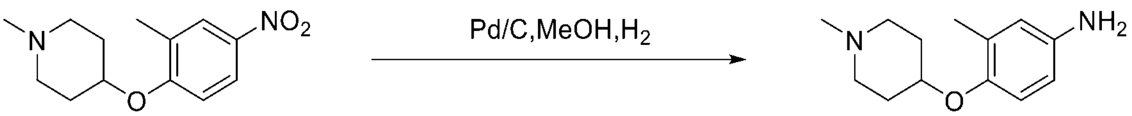


A solution of 1-methyl-4-(2-methyl-4-nitrophenoxy)piperidine (3.7 g, 14.7 mmol) and palladium on carbon (2.6 g, content: 10%) in methanol (40 mL) was stirred at 25 °C overnight under a hydrogen atmosphere. The resulting mixed reaction solution was filtered, and the filter cake was washed with methanol (3 × 20 mL). The filtrate was collected and concentrated under reduced pressure to give 3-methyl-4-((1-methylpiperidin-4-yl)oxy)aniline (3 g, yield: 92.1%), LC-MS m/z: 221 $[M + H]^+$.

**7) Synthesis of (*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one**

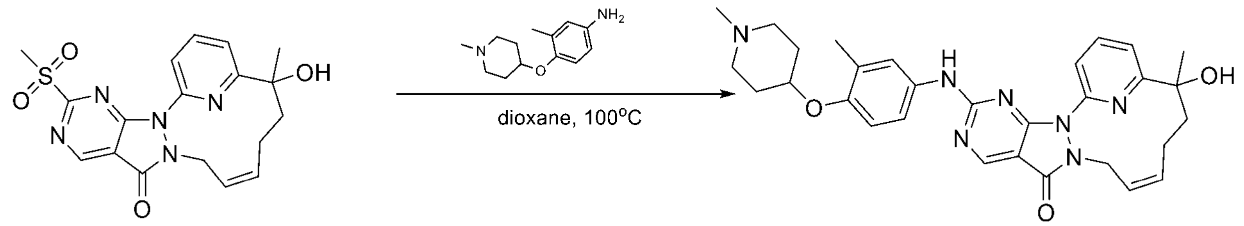


A solution of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (500 mg, 1.24 mmol) and 3-methyl-4-((1-methylpiperidin-4-yl)oxy)aniline (328.3 mg, 1.49 mmol) in dioxane (50 mL) was heated to 100 °C and stirred overnight. After cooling, the mixed reaction solution was concentrated under reduced pressure. The residue was purified by silica gel column chromatography (methanol:dichloromethane = 0%-10%) to give (*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (490 mg, yield: 73.0%), LC-MS m/z: 542 [M + H]$^+$.

**8) Synthesis of (*R*,*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

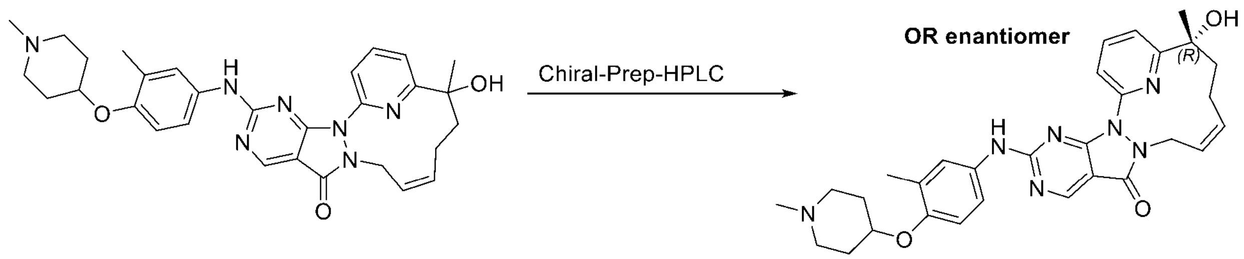


(*Z*)-12-Hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (490 mg, 0.91 mmol) was purified by preparative supercritical fluid chromatography (chromatography conditions: instrument: SHIMADZU LC-20AP; column: DAICELCHIRALPAK®IC, 250 × 40 mm 10 μm; mobile phase A: *n*-hexane; mobile phase B: ethanol containing 0.1% ammonia/methanol (7 M); gradient: 60% to 60% B in 24 min; flow rate: 80 mL/min; column temperature: 20 °C; detection wavelength: 254 nm) to give (*R*,*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (160 mg, yield: 32.6%) (compound IE), LC-MS m/z: 542 [M + H]$^+$.

**Confirmation of the absolute configuration of compound IE (single-crystal growth):**

Approximately 5 mg of the metastable crystal form H of compound IE was dissolved in 1.2 mL of isopropyl acetate, and the mixture was filtered into a 3 mL vial. The vial was then pierced and placed into a 20 mL vial containing 3 mL of *n*-pentane, and the 20 mL vial was left to stand at 25 °C for crystallization. Through gas-liquid diffusion in the isopropyl acetate and *n*-pentane system, a plate-like crystal was obtained, and the product was analyzed and characterized by a single-crystal X-ray diffraction pattern (FIG. 38). Through crystal structure analysis, the single crystal was found to belong to the triclinic crystal system, with a space group of P1, and the following unit cell parameters: a = 8.2644(3) Å, b = 8.2701(3) Å, c = 45.8129(16) Å; $\alpha$ = 94.562(2)°, $\beta$ = 94.343(2)°, $\gamma$ = 94.807(2)°. Furthermore, it was confirmed that compound IE has one chiral atom, the absolute configuration is *R*, and the double bond within the ring has a *cis* configuration.

$^1$H NMR (400 MHz, MeOD-d4): $\delta$ 8.78 (s, 1H), 7.94 (t, J = 8.0 Hz, 1H), 7.81 (d, J = 8.0 Hz, 1H), 7.58 (d, J = 8.0 Hz, 1H), 7.55 (s, 1H), 7.34 (dd, J = 8.0, 4.0 Hz, 1H), 6.87 (d, J = 12.0 Hz, 1H), 5.53 - 5.41 (m, 2H), 4.57 (d, J = 8.0 Hz, 2H), 4.40 (s, 1H), 2.73 (s, 2H), 2.44 (s, 2H), 2.34 (s, 3H), 2.20 (s, 3H), 2.19 - 2.16 (m, 1H), 2.16-2.08 (m, 1H), 2.06 - 1.96 (m, 3H), 1.92 - 1.79 (m, 3H), 1.67 (s, 3H).

**Example 2: Synthesis of (*S,Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound 134)**

**OR enantiomer**

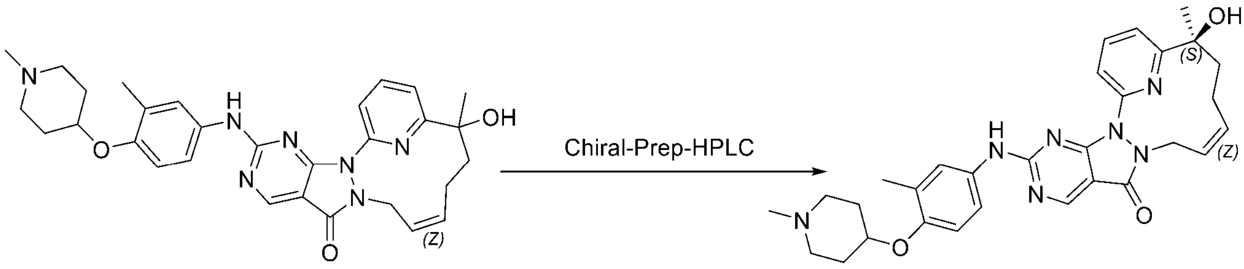


The racemate (490 mg, 0.91 mmol) was purified by preparative supercritical fluid chromatography (chromatography conditions: system: SHIMADZU LC-20AP; column: DAICELCHIRALPAK®IC, 250 × 40 mm 10 μm; mobile phase A: *n*-hexane; mobile phase B: ethanol (+ 0.1% 7.0 M ammonia in MeOH); A:B = 40:60; detection wavelength: 254 nm; flow rate: 80 mL/min; column temperature: RT; column pressure: 100 bar) to give (*S*,*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or the corresponding isomer (150 mg, 30.6%), LC-MS *m/z:* 542 $[M + H]^+$;

$^1$H NMR (400 MHz, MeOD-d4): δ 8.78 (d, *J* = 2.8 Hz, 1H), 7.94 (td, *J* = 8.0, 2.0 Hz, 1H), 7.80 (dd, *J* = 8.0, 2.4 Hz, 1H), 7.58 (d, *J* = 7.8 Hz, 2H), 7.34 (d, *J* = 8.8 Hz, 1H), 6.87 (dd, *J* = 8.8, 2.8 Hz, 1H), 5.54 - 5.37 (m, 2H), 4.57 (d, *J* = 6.2 Hz, 2H), 4.40 (s, 1H), 2.75 (s, 2H), 2.46 (s, 2H), 2.35 (d, *J* = 3.2 Hz, 3H), 2.20 (s, 3H), 2.16 (s, 1H), 2.00 (d, *J* = 9.8 Hz, 3H), 1.98 - 1.77 (m, 4H), 1.68 (s, 3H).

**Example 3: Synthesis of (*R*)-12-hydroxy-12-methyl-2-((3-methyl-4-((1-methylpiperidin-4-yl)oxy)phenyl)amino)-7,8,9,10,11,12-hexahydro-5*H*-13,17-(azeno)pyrimido[4',5':3,4]pyrazolo[1,2-*a*][1,2]diazacyclotridecin-5-one(Compound 28)**

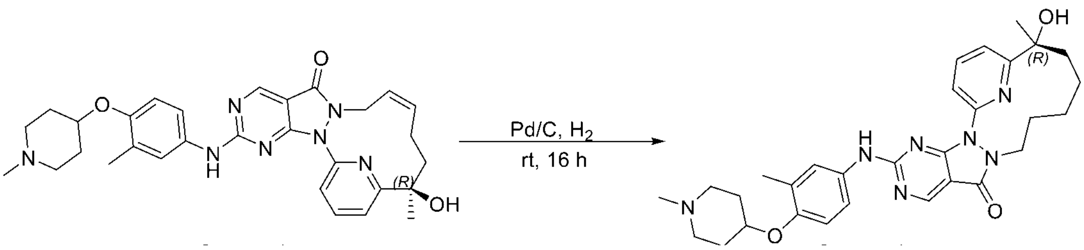


A solution of (*R*,*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (50 mg, 0.092 mmol) and palladium on carbon (10 mg) in methanol (2 mL) was stirred at room temperature for 16 h under a hydrogen atmosphere. The resulting mixed reaction solution was filtered, and the filter cake was washed with methanol (3 × 10 mL). The collected filtrate was concentrated under reduced pressure. The residue was purified using a C18 column (acetonitrile:purified water containing 0.1% $NH_4HCO_3$ = 5%-95% elution) to give (*R*)-12-hydroxy-12-methyl-2-((3-methyl-4-((1-methylpiperidin-4-yl)oxy)phenyl)amino)-7,8,9,10,11,12-hexahydro-5*H*-13,17-(azeno)pyrimido[4',5':3,4]pyrazolo[1,2-*a*][1,2]diazacyclotridecin-5-one (34 mg, yield: 67%), LC-MS m/z: 544 $[M + H]^+$;

$^1$H NMR (400 MHz, MeOD-d4):δ 8.78 (s, 1H), 8.00 (t, J = 8.0 Hz, 1H), 7.93 (d, J = 8.0 Hz, 1H), 7.67 (d, J = 8.0 Hz, 1H), 7.66 (s, 1H), 7.34 (d, J = 4.0 Hz, 1H), 6.90 (d, J = 8.0 Hz, 1H), 4.41 (s, 1H), 3.88-3.84 (m, 1H), 3.31-3.30(m,1H), 2.72(s,2H), 2.43-2.39(m,3H), 2.32(s,4H), 2.23(s,1H), 2.14-2.11(m,3H), 2.01-1.98(m,2H), 1.93-1.90(m,1H), 1.89-1.86(m,2H), 1.51(s,6H), 1.29(s,1H).

**Example 4: Synthesis of (*S*)-12-hydroxy-12-methyl-2-((3-methyl-4-((1-methylpiperidin-4-yl)oxy)phenyl)amino)-7,8,9,10,11,12-hexahydro-5*H*-13,17-(azeno)pyrimido[4',5':3,4]pyrazolo[1,2-*a*][1,2]diazacyclotridecin-5-one (Compound 29)**

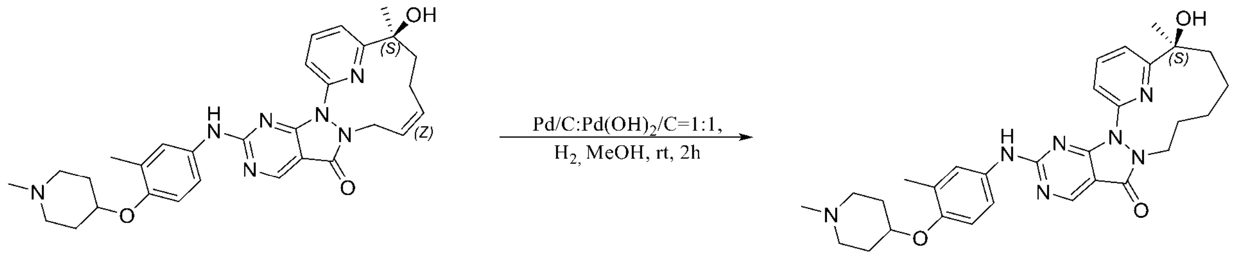


A solution of (*S,Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (10.0 mg, 0.02 mmol), palladium hydroxide/carbon (5 mg), and Pd/C (5 mg) in methanol (1 mL) was stirred at room temperature for 2 h under a hydrogen atmosphere. The mixed reaction solution was filtered, and the filter cake was washed with methanol (3 × 5 mL). The collected filtrate was concentrated under reduced pressure. The residue was purified by C18 column chromatography (acetonitrile:purified water containing 0.1% $NH_4HCO_3$ = 5%-95% elution) to give (*S*)-12-hydroxy-12-methyl-2-((3-methyl-4-((1-methylpiperidin-4-yl)oxy)phenyl)amino)-7,8,9,10,11,12-hexahydro-5*H*-13,17-(azeno)pyrimido[4',5':3,4]pyrazolo[1,2-*a*][1,2]diazacyclotridecin-5-one (8 mg, yield: 79.7%), LC-MS m/z: 544 $[M + H]^+$;

$^1H$ NMR (400 MHz, MeOD-d4):δ 8.79 (s, 1H), 8.01 (t, J = 8.0 Hz, 1H), 7.94 (d, J = 8.0 Hz, 1H), 7.68 (d, J = 8.0 Hz, 1H), 7.66 (s, 1H), 7.38 (s, 1H), 6.92 (d, J = 8.0 Hz, 1H), 4.86 (s, 1H), 3.88-3.84 (m, 1H), 3.31-3.30 (m,1H), 3.11-2.98 (m, 2H), 2.78-2.58 (m, 2H), 2.48 (s, 3H), 2.25-2.19 (m, 2H), 2.06 (s,3H), 2.20-1.93 (m, 6H), 1.51(s,5H), 1.29 (s,2H).

**Example 5: Synthesis of (*Z*)-2-((4-((1,4-dimethylpiperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 89)**

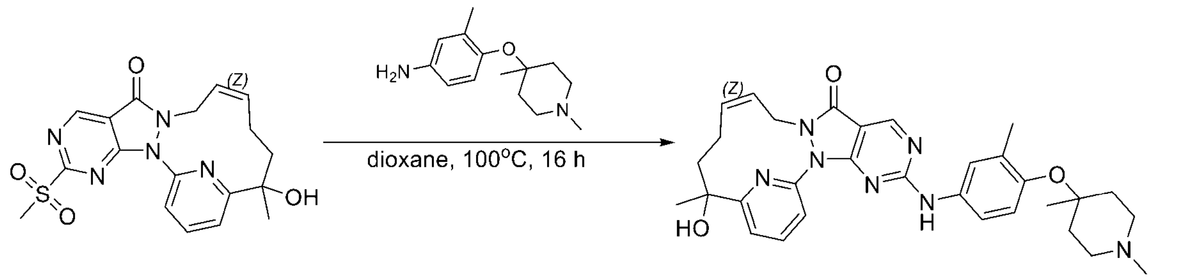


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (50 mg, 0.12 mmol) and 4-((1,4-dimethylpiperidin-4-yl)oxy)-3-methylaniline (35 mg, 0.15 mmol) in 1,4-dioxane (5 mL) was heated to 100 °C and stirred overnight. After cooling, the resulting mixed reaction solution was concentrated under reduced pressure. The residue was purified by silica gel column chromatography (methanol:dichloromethane = 0%-10% elution) to give (*Z*)-2-((4-((1,4-dimethylpiperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*] [1,2]diazacyclotridecin-5-one (43 mg, yield: 62.2%), LC-MS m/z: 556 $[M + H]^+$;

$^1H$ NMR (400 MHz, MeOD) δ 8.82 (s, 1H), 7.95 (t, J = 8.0 Hz, 1H), 7.82 (d, J = 8.0 Hz, 1H), 7.64 (s, 1H), 7.59 (d, J = 7.6 Hz, 1H), 7.35 (d, J = 10.8 Hz, 1H), 6.94 (d, J = 8.8 Hz, 1H), 5.52 - 5.39 (m, 2H), 4.61 (d, J = 6.4 Hz, 2H), 3.09 (s, 2H), 2.67 (s, 3H), 2.26 (d, J = 14.8 Hz, 6H), 2.20 - 2.08 (m, 2H), 2.04 - 1.83 (m, 5H), 1.68 (s, 3H), 1.27 (s, 3H).

**Example 6: Synthesis of (*Z*)-12-hydroxy-2-((2-methoxy-4-((1-methylpiperidin-4-yl)oxy)phenyl)amino)-12-methyl-7,10,12-tetrahydro-5*H*-13,17-(azeno)pyrimido[4',5':3,4]pyrazolo[1,2-*a*][1,2]diazacyclotridecin-5-one (Compound 90)**

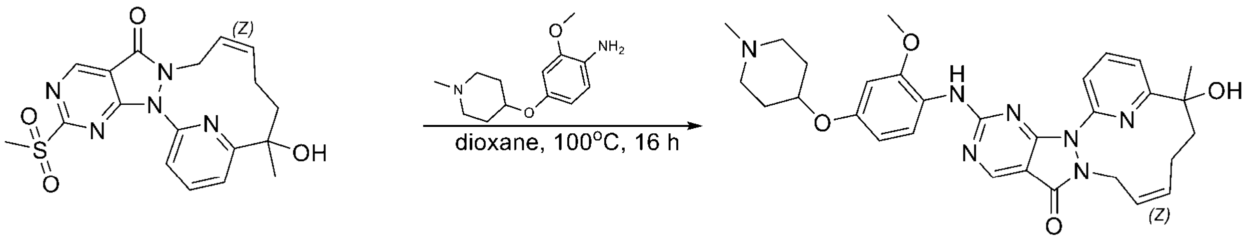


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (50 mg, 0.12 mmol) and 2-methoxy-4-((1-methylpiperidin-4-yl) oxy)aniline (35 mg, 0.15 mmol) in 1,4-dioxane (5 mL) was heated to 100 °C and stirred overnight. After cooling, the mixed reaction solution was concentrated under reduced pressure. The residue was purified by silica gel column chromatography (methanol:dichloromethane = 0%-10% elution) to give (*Z*)-12-hydroxy-2-((2-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*] [1,2]diazacyclotridecin-5-one (45 mg, yield: 64.8%), LC-MS m/z: 558 $[M + H]^+$;

$^1$H NMR (400 MHz, MeOD): δ 8.80 (s, 1H), 7.94 (t, J = 8.0 Hz, 2H), 7.74 (d, J = 8.0 Hz, 1H), 7.59 (d, J = 7.6 Hz, 1H), 6.64 (d, J = 2.4 Hz, 1H), 6.54 (d, J = 9.2 Hz, 1H), 5.45 (dd, J = 17.2, 9.2 Hz, 2H), 4.61 (d, J = 7.2 Hz, 2H), 4.43 (s, 1H), 3.86 (s, 3H), 2.79-2.72 (m, 2H), 2.48-2.43 (m, 2H), 2.34 (s, 3H), 2.24-2.16 (m, 2H), 2.04-1.98 (m, 3H), 1.90-1.82 (m, 3H), 1.68 (s, 3H).

**Example 7: Synthesis of 2-((3-methyl-4-((1-methylpiperidin-4-yl)oxy)phenyl)amino)-10,11-dihydro-5*H*,7*H*-13,17-(azeno)pyrimido[4',5':3,4]pyrazolo[1,2-*g*][1]thia[2,7,8]triazacyclotridecin-5-one 12,12-dioxide (Compound 130)**

**1) Synthesis of *N*-allyl-6-(2-allyl-6-(methylthio)-3-oxo-2,3-dihydro-1*H*-pyrazolo[3,4-*d*]pyrimidin-1-yl)pyridine-2-sulfonamide**

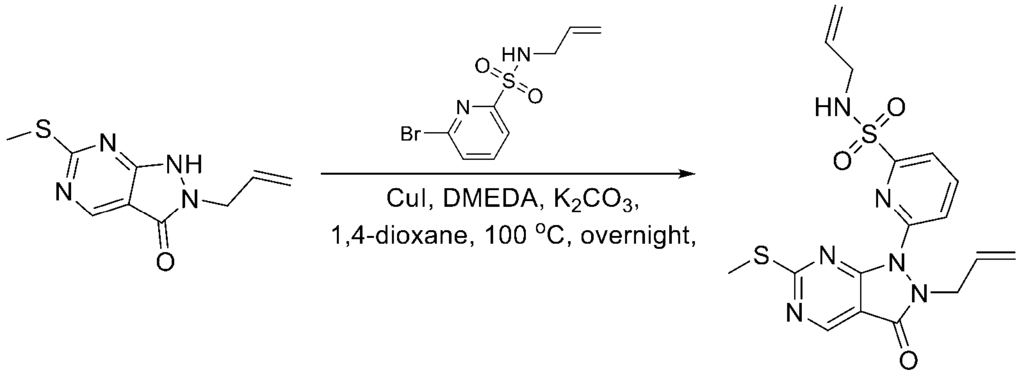


*N*,*N'*-Dimethylethylenediamine (87 mg, 0.99 mmol), copper(I) iodide (171 mg, 0.9 mmol), and potassium carbonate (311 mg, 2.25 mmol) were added in batches to a solution of 2-allyl-6-(methylthio)-1,2-dihydro-3*H*-pyrazolo [3,4-*d*]pyrimidin-3-one (200 mg, 0.9 mmol) in 1,4-dioxane (2 mL) at room temperature under a nitrogen atmosphere. The resulting mixed reaction solution was heated to 100 °C and stirred overnight. The mixed reaction solution was diluted with water, extracted with ethyl acetate (3 × 5 mL), dried over anhydrous sodium sulfate, filtered, and concentrated under reduced pressure. The residue was purified by silica gel column chromatography (methanol:dichloromethane = 10%-20% elution) to give *N*-allyl-6-(2-allyl-6-(methylthio)-3-oxo-2,3-dihydro-1*H*-pyrazolo[3,4-*d*]pyrimidin-1-yl)pyridine-2-sulfonamide (100 mg, yield: 26.5%), LC-MS m/z: 419 $[M + H]^+$.

**2) Synthesis of 2-(methylthio)-10,11-dihydro-5*H*,7*H*-13,17-(azeno)pyrimido[4',5':3,4]pyrazolo[1,2-*g*][1]thia [2,7,8]triazacyclotridecin-5-one 12,12-dioxide**

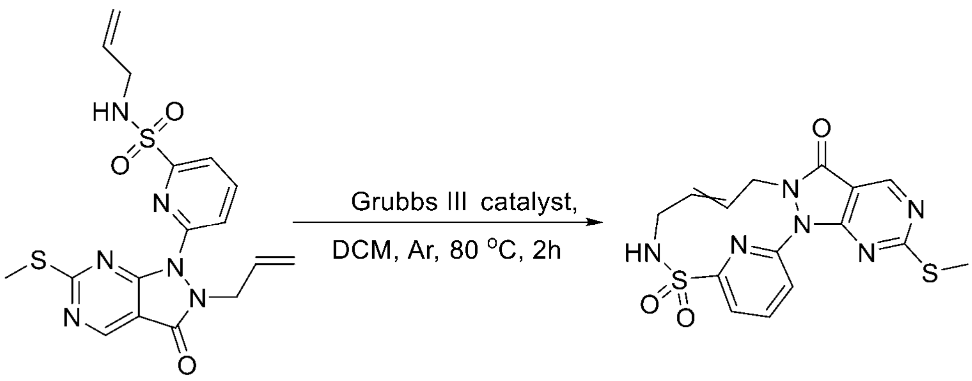


Dichloro[1,3-bis(2,4,6-trimethylphenyl)-2-imidazolidinylidene](benzylidene)bis(3-bromopyridine)ruthenium(II) (10.3 mg, 0.012 mL) was added to a solution of *N*-allyl-6-(2-allyl-6-(methylthio)-3-oxo-2,3-dihydro-1*H*-pyrazolo[3,4-*d*] pyrimidin-1-yl)pyridine-2-sulfonamide (47 mg, 0.112 mmol) in dichloromethane (2 mL) at room temperature under a nitrogen atmosphere. The resulting mixed reaction solution was heated to 80 °C and stirred for 2 h under an argon atmosphere. The mixed reaction solution was diluted with water, extracted with dichloromethane (3 × 5 mL), dried over anhydrous sodium sulfate, filtered, and concentrated under reduced pressure. The residue was purified by silica gel column chromatography (MeOH:dichloromethane = 10%-20% elution) to give 2-(methylthio)-10,11-dihydro-5*H*,7*H*-13,17-(azeno)pyrimido[4',5':3,4]pyrazolo[1,2-g][1]thia[2,7,8]triazacyclotridecin-5-one 12,12-dioxide (40 mg, yield: 91.2%), LC-MS m/z: 391 $[M + H]^+$.

**3) Synthesis of 2-((3-methyl-4-((1-methylpiperidin-4-yl)oxy)phenyl)amino)-10,11-dihydro-5*H*,7*H*-13,17-(azeno) pyrimido[4',5':3,4]pyrazolo[1,2-*g*][1]thia[2,7,8]triazacyclotridecin-5-one 12,12-dioxide**

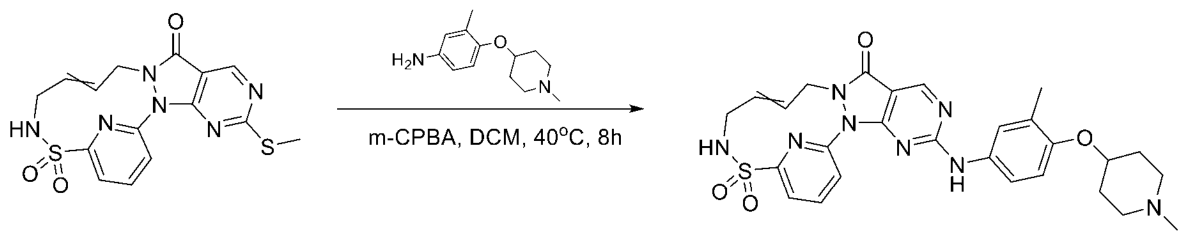


3-Methyl-4-((1-methylpiperidin-4-yl)oxy)aniline (30 mg, 0.135 mmol) and *m*-chloroperoxybenzoic acid (26.5 mg, 0.154 mmol) were added in batches to a solution of 2-(methylthio)-10,11-dihydro-5*H*,7*H*-13,17-(azeno)pyrimido[4',5':3,4] pyrazolo[1,2-g][1]thia[2,7,8]triazacyclotridecin-5-one 12,12-dioxide (30 mg, 0.077 mmol) in dichloromethane (2 mL) at room temperature. The resulting mixed reaction solution was heated to 40 °C and stirred for 8 h. The mixed reaction solution was diluted with water, extracted with ethyl acetate (3 × 5 mL), dried over anhydrous sodium sulfate, and concentrated under reduced pressure. The residue was purified by C18 column chromatography (acetonitrile:purified water containing 0.1% $NH_3 \cdot H_2O$ = 5%-95% elution) to give 2-((3-methyl-4-((1-methylpiperidin-4-yl)oxy)phenyl)amino)-10,11-dihydro-5*H*,7*H*-13,17-(azeno)pyrimido[4',5':3,4]pyrazolo[1,2-g][1]thia[2,7,8]triazacyclotridecin-5-one 12,12-dioxide (6 mg, yield: 13.9%), LC-MS m/z: 563 $[M + H]^+$;

$^1H$ NMR (400 MHz, MeOD) δ 8.80 (s, 1H), 8.16 (d, J = 6.4 Hz, 2H), 7.90 (d, J = 8.3 Hz, 1H), 7.46 - 7.60 (m, 1H), 7.38 (s, 1H), 6.94 (d, J = 8.8 Hz, 1H), 6.04 - 6.14 (m, 1H), 5.07 (s, 3H), 4.50 - 4.68 (m, 1H), 3.89 - 4.00 (m, 1H), 3.77 - 3.87 (m, 1H), 3.57 - 3.64 (m, 1H), 3.18 - 3.23 (m, 1H), 2.99 - 3.09 (m, 1H), 2.75 (s, 3H), 2.26 (s, 3H), 2.07 - 2.19 (m, 3H), 2.03 (s, 2H).

**Example 8: Synthesis of 12-hydroxy-12-methyl-2-((3-methyl-4-((1-methylpiperidin-4-yl)oxy)phenyl)amino)-7,8,9,10,11,12-hexahydro-5*H*-13,17-(azeno)pyrimido[4',5':3,4]pyrazolo[1,2-*a*][1,2]diazacyclotridecin-5-one (Compound 1A)**

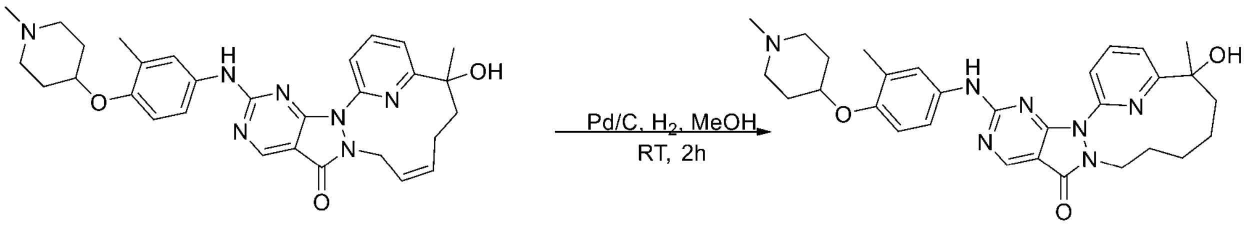


A solution of (*Z*)-12-hydroxy-12-methyl-2-((3-methyl-4-((1-methylpiperidin-4-yl)oxy)phenyl)amino)-7,10,11,12-tetrahydro-5*H*-13,17-(azeno)pyrimido[4',5':3,4]pyrazolo[1,2-a][1,2]diazacyclotridecin-5-one (50 mg, 0.092 mmol) and palladium on carbon (10 mg) in methanol (2 mL) was stirred at room temperature for 2 h under a hydrogen atmosphere. The mixed reaction solution was filtered, and the filter cake was washed with methanol (3 × 10 mL). The collected filtrate was concentrated under reduced pressure. The residue was purified using a C18 column (acetonitrile:purified water containing 0.1% $NH_4HCO_3$ = 5%-95%) to give 12-hydroxy-12-methyl-2-((3-methyl-4-((1-methylpiperidin-4-yl)oxy)phenyl)amino)-7,8,9,10,11,12-hexahydro-5*H*-13,17-(azeno)pyrimido[4',5':3,4]pyrazolo[1,2-*a*][1,2]diazacyclotridecin-5-one (30 mg, yield: 60%), LC-MS *m/z:* 544 $[M + H]^+$;

$^1$H NMR (400 MHz, MeOD-*d*4): δ 8.78 (s, 1H), 8.00 (t, *J* = 8.0 Hz, 1H), 7.93 (d, *J* = 8.0 Hz, 1H), 7.67 (d, *J* = 8.0 Hz, 1H), 7.62 (s, 1H), 7.34(dd, *J* = 8.0 Hz, 1H), 6.90 (d, *J* = 8.0 Hz, 1H), 4.43 (s, 1H),3.84-3.80 (m, 1H), 3.31 (t, *J* = 4.0 Hz, 1H), 2.78 (s, 2H), 2.50 (s, 2H), 2.37 (s, 3H), 2.32-2.31 (m, 2H), 2.23-2.21 (m, 3H), 2.02-1.98 (m, 2H), 1.89-1.84 (m, 3H), 1.51 (s, 6H), 1.36-1.34(m, 2H).

**Example 9: Synthesis of (*Z*)-12-hydroxy-12-methyl-2-((4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 140)**

**1) Synthesis of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one**

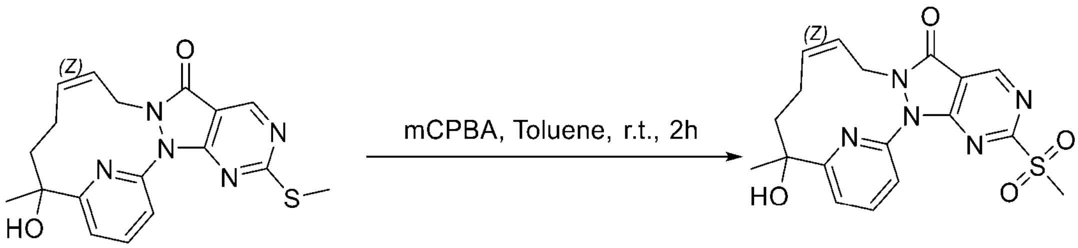


A solution of (Z)-12-hydroxy-12-methyl-2-(methylthio)-7,10,11,12-tetrahydro-5H-13,17-(azeno)pyrimido [4',5':3,4]pyrazolo[1,2-a][1,2]diazacyclotridecin-5-one (100 mg, 0.27 mmol) and *m*-chloroperoxybenzoic acid (234 mg, 1.36 mmol) in toluene (2 mL) was stirred at room temperature for 2 h. The resulting mixed reaction solution was concentrated under reduced pressure to give a crude product, which was used directly in the next step without further purification, LC-MS m/z: 402 $[M + H]^+$.

**2) Synthesis of (Z)-12-hydroxy-12-methyl-2-((4-((1-methylpiperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one**

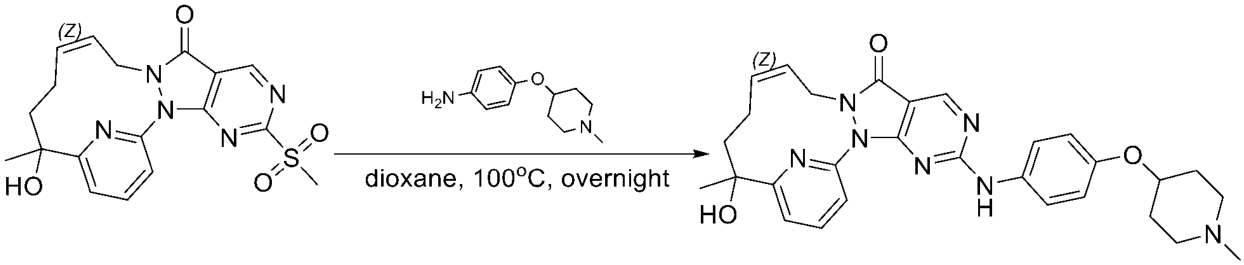


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100.0 mg, 0.25 mmol) and 4-((1-methylpiperidin-4-yl)oxy)aniline (76 mg, 0.37 mmol) in 1,4-dioxane (2 mL) was heated to 100 °C and stirred overnight. The resulting mixed reaction solution was concentrated under reduced pressure. The residue was purified by C18 column chromatography (acetonitrile:purified water containing 0.1% formic acid = 5%-95% elution) to give (Z)-12-hydroxy-12-methyl-2-((4-((1-methylpiperidin-4-yl) oxy)phenyl)amino)-7,10,11,12-tetrahydro-5*H*-13,17-(azeno)pyrimido[4',5':3,4]pyrazolo[1,2-*a*][1,2]diazacyclotridecin-5-one (20 mg, yield: 15.2%), LC-MS *m/z:* 528 [M + H]$^+$;

$^1$H NMR (400 MHz, MeOD-d4):δ 8.81 (s, 1H), 7.95 (t, J = 8.0 Hz, 1H), 7.79 (d, J = 12.0 Hz, 1H), 7.57 (t, J = 8.0 Hz, 3H), 6.91 (d, J = 8.0 Hz, 2H), 5.50-5.40 (m, 2H), 4.62-4.58 (m, 2H), 4.42-4.37 (m, 1H), 2.78-2.72 (m, 2H), 2.46-2.39 (m, 2H), 2.33 (s, 3H), 2.24-2.15 (m, 2H), 2.03-1.97 (m, 3H), 1.92-1.87(m,3H), 1.88(s,3H).

**Example 10: Synthesis of (*Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-a][1,2]diazacyclotridecin-5-one(Compound 141)**

**1) Synthesis of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one**

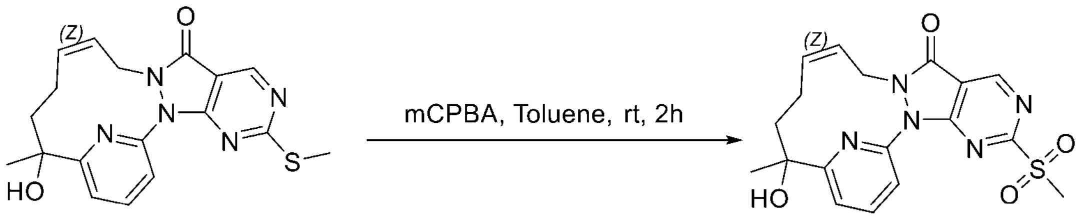


A solution of (Z)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one (100 mg, 0.27 mmol) and m-chloroperoxybenzoic acid (234 mg, 1.36 mmol) in toluene (2 mL) was stirred at room temperature for 2 h. The resulting mixed reaction solution was concentrated under reduced pressure to give a crude product, which was used directly in the next step without further purification, LC-MS m/z: 402 [M + H]$^+$.

**2) Synthesis of (*Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*][1,2]diazacyclotridecin-5-one**

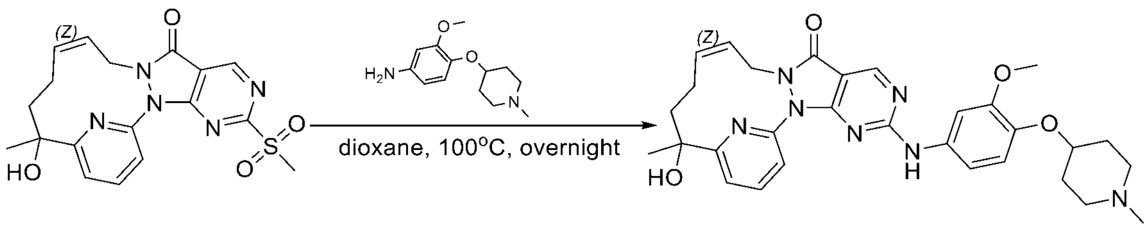


A solution of (*Z*)-12-hydroxy-12-methyl-2-(methylsulfonyl)-7,10,11,12-tetrahydro-5*H*-13,17-(azeno)pyrimido

[4',5':3,4]pyrazolo[1,2-*a*][1,2]diazacyclotridecin-5-one (100.0 mg, 0.25 mmol) and 3-methoxy-4-((1-methylpiperidin-4-yl)oxy)aniline (88.3 mg, 0.37 mmol) in 1,4-dioxane (2 mL) was heated to 100 °C and stirred overnight. The resulting mixed reaction solution was concentrated under reduced pressure. The residue was purified by C18 reverse phase column chromatography (acetonitrile:purified water containing 0.1% formic acid = 5%-95% elution) to give (*Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*][1,2]diazacyclotridecin-5-one (20 mg, yield: 14.4%), LC-MS m/z: 558 [M + H]+;

$^1H$ NMR (400 MHz, MeOD-d4):δ 8.83 (s, 1H), 7.93 (t, J = 8.0 Hz, 1H), 7.76 (d, J = 8.0 Hz, 1H), 7.61 (d, J = 8.0 Hz, 1H), 7.49 (d, J = 4.0 Hz, 1H), 7.15-7.10 (m, 1H), 6.93 (d, J = 8.0 Hz, 1H), 5.50-5.30 (m, 2H), 4.64 (d, J = 8.0 Hz, 2H), 4.27 (s, 1H), 3.71 (s, 3H), 2.82 (s, 2H), 2.46-2.32 (m, 5H), 2.30-2.10 (m, 2H), 2.01-1.92(m, 3H), 1.91-1.79(m,3 H), 1.69(s, 3H).

**Example 11: Synthesis of (*R,Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*][1,2]diazacyclotridecin-5-one (Compound 143)**

**1) Synthesis of (*R,Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one**

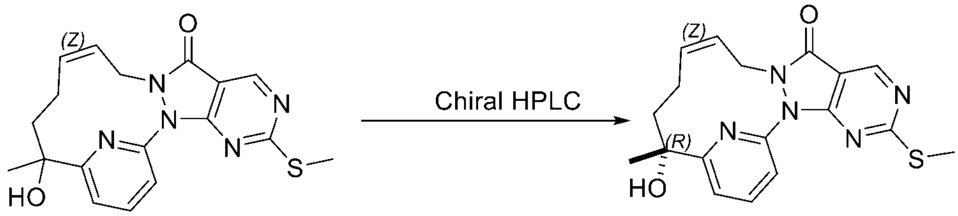


The racemate (300 mg, 0.81 mmol) was separated and purified by preparative supercritical fluid chromatography (chromatography conditions: system: Waters SFC 150; column: DAICELCHIRALCEL®AS, 250 × 25 mm 10 μm; mobile phase A: supercritical $CO_2$; mobile phase B: MeOH (+ 0.1% 7.0 mol/L ammonia in MEOH); A:B = 75:25; detection wavelength: 214 nm; flow rate: 100 mL/min; column temperature: RT; column pressure: 100 bar; injection volume: 4.5 mL) to give (*R*,*Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one (130 mg, yield: 43%).

**2) Synthesis of (R,Z)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one**

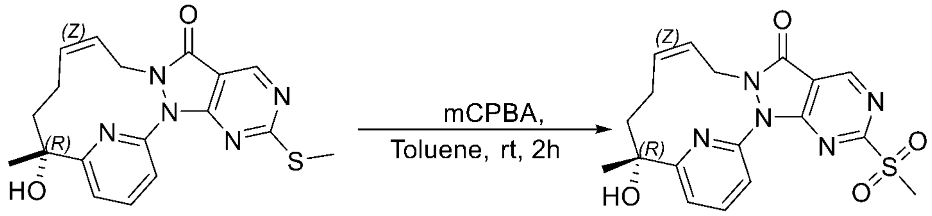


A solution of (*R*,*Z*)-12-hydroxy-12-methyl-2-(methylthio)-7,10,11,12-tetrahydro-5H-13,17-(azeno)pyrimido[4',5':3,4]pyrazolo[1,2-*a*][1,2]diazacyclotridecin-5-one (100 mg, 0.27 mmol) and *m*-chloroperoxybenzoic acid (234 mg, 1.36 mmol) in toluene (2 mL) was stirred at room temperature for 2 h. The resulting mixed reaction solution was concentrated under reduced pressure to give a crude product, which was used directly in the next step without further purification. LC-MS *m/z:* 402 [M + H]+;

**3) Synthesis of (*R,Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*][1,2]diazacyclotridecin-5-one**

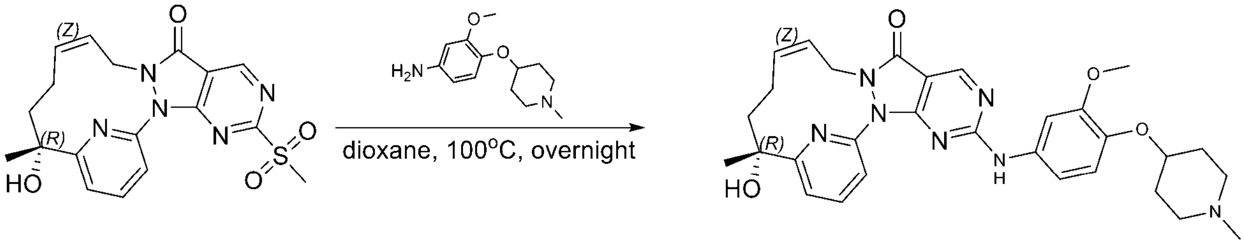


A solution of (*R*,*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol) and 3-methoxy-4-((1-methylpiperidin-4-yl) oxy)aniline (88 mg, 0.37 mmol) in 1,4-dioxane (2 mL) was heated to 100 °C and stirred overnight. The resulting mixed reaction solution was concentrated under reduced pressure. The residue was purified by C18 column chromatography (acetonitrile:purified water containing 0.1% formic acid = 5%-95% elution) to give (*R*,*Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo [1,2-*a*][1,2]diazacyclotridecin-5-one (20 mg, yield: 14.4%), LC-MS m/z: 558 $[M + H]^+$;

$^1$H NMR (400 MHz, MeOD-d4):δ 8.83 (s, 1H), 7.93 (t, J = 8.0 Hz, 1H), 7.76 (d, J = 8.0 Hz, 1H), 7.61 (d, J = 8.0 Hz, 1H), 7.48 (d, J = 4.0 Hz, 1H), 7.15-7.10 (m, 1H), 6.93 (d, J = 8.0 Hz, 1H), 5.45-5.30 (m, 2H), 4.64 (d, *J* = 8.0 Hz, 2H), 4.26 (s, 1H), 3.71 (s, 3H), 2.81 (s, 2H), 2.45-2.32 (m, 5H), 2.30-2.10 (m, 2H), 2.00-1.92(m,3H), 1.99-1.77(m,3H), 1.69(s,3H).

**Example 12: Synthesis of (*S,Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*][1,2]diazacyclotridecin-5-one (Compound 144)**

**1) Synthesis of (*S*,*Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one**

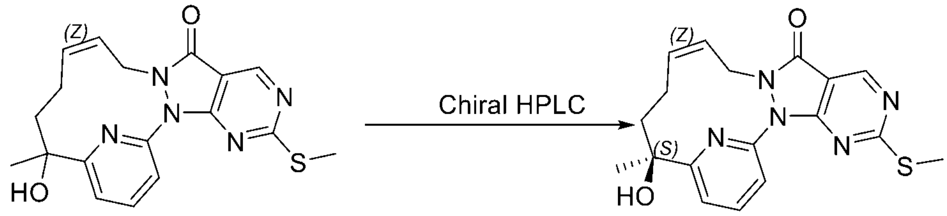


The racemate (300 mg, 0.81 mmol) was separated and purified by preparative supercritical fluid chromatography (chromatography conditions: system: Waters SFC 150; column: DAICELCHIRALCEL®AS, 250 × 25 mm 10 μm; mobile phase A: supercritical $CO_2$; mobile phase B: MeOH (+ 0.1% 7.0 mol/L ammonia in MEOH); A:B = 75:25; detection wavelength: 214 nm; flow rate: 100 mL/min; column temperature: RT; column pressure: 100 bar; injection volume: 4.5 mL) to give (*S*,*Z*)-12-hydroxy-12-methyl-2-(methylthio)-7,10,11,12-tetrahydro-5*H*-13,17-(azeno)pyrimido[4',5':3,4]pyrazolo [1,2-*a*][1,2]diazacyclotridecin-5-one (150 mg, yield: 50%).

**2) Synthesis of (*S*,*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one**

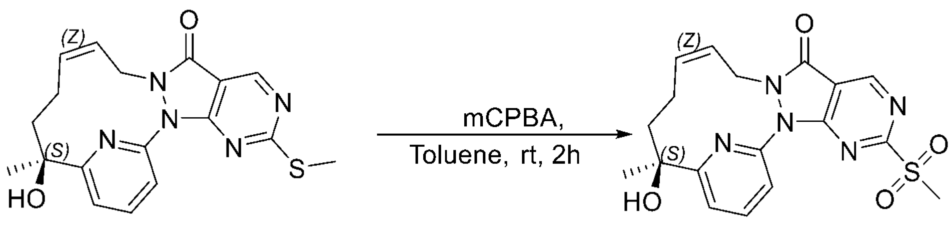


A solution of (S,Z)-12-hydroxy-12-methyl-2-(methylthio)-7,10,11,12-tetrahydro-5H-13,17-(azeno)pyrimido

[4',5':3,4]pyrazolo[1,2-a][1,2]diazacyclotridecin-5-one (100 mg, 0.27 mmol) and m-chloroperoxybenzoic acid (234 mg, 1.36 mmol) in toluene (2 mL) was stirred at room temperature for 2 h. The resulting mixed reaction solution was concentrated under reduced pressure to give a crude product, which was used directly in the next step without further purification. LC-MS *m/z:* 402 [M + H]$^+$;

**3) Synthesis of (*S*,*Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo[1,2-*a*][1,2]diazacyclotridecin-5-one**

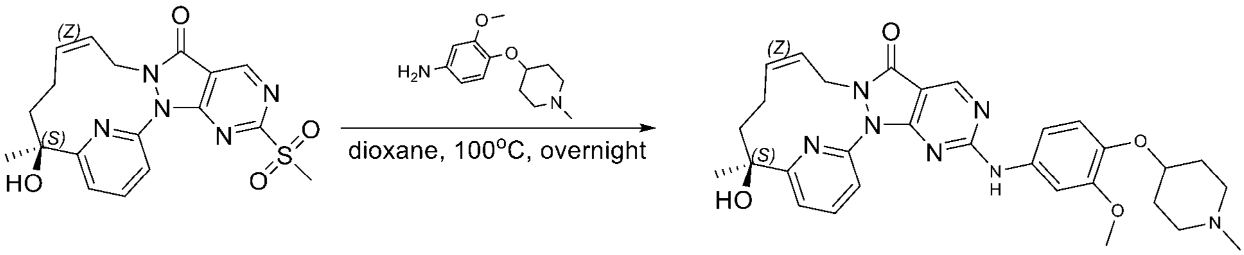


A solution of (*S*,*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol) and 3-methoxy-4-((1-methylpiperidin-4-yl) oxy)aniline (88 mg, 0.37 mmol) in 1,4-dioxane (2 mL) was heated to 100 °C and stirred overnight. The resulting mixed reaction solution was concentrated under reduced pressure. The residue was purified by C18 column chromatography (acetonitrile:purified water containing 0.1% formic acid = 5%-95% elution) to give (*S*,*Z*)-12-hydroxy-2-((3-methoxy-4-((1-methylpiperidin-4-yl)oxy)phenyl)amino)-12-methyl-7,10,11,12-tetrahydro-5*H*-13,17-(azeno)pyrimido[4',5':3,4]pyrazolo [1,2-*a*][1,2]diazacyclotridecin-5-one (20 mg, yield: 14.4%), LC-MS m/z: 558 [M + H]$^+$;

$^1$H NMR (400 MHz, MeOD-d4):$\delta$ 8.83 (s, 1H), 7.93 (t, J = 8.0 Hz, 1H), 7.76 (d, J = 8.0 Hz, 1H), 7.61 (d, J = 8.0 Hz, 1H), 7.48 (d, J = 4.0 Hz, 1H), 7.15-7.10 (m, 1H), 6.93 (d, J = 8.0 Hz, 1H), 5.44-5.30 (m, 2H), 4.64 (d, J = 8.0 Hz, 2H), 4.27 (s, 1H), 3.71 (s, 3H), 2.82 (s, 2H), 2.49-2.34 (m, 5H), 2.30-2.08 (m, 2H), 2.02-1.92(m,3H), 1.91-1.79(m,3H), 1.69(s,3H).

**Example 13: Synthesis of 3a-hydroxy-14-((3-methyl-4-((1-methylpiperidin-4-yl)oxy)phenyl)amino)-3,3a,4,5,6,9-hexahydro-2*H*,11*H*-1,17-(epiethane[1,2]diylidene)cyclopenta[*e*]pyrimido[4',5':3,4]pyrazolo[1,2-*a*][1,2,4]triazacyclododecin-11-one (Compound 146)**

**1) Synthesis of 2-bromo-7-(pent-4-en-1-yl)-6,7-dihydro-5*H*-cyclopenta[*b*]pyridin-7-ol**

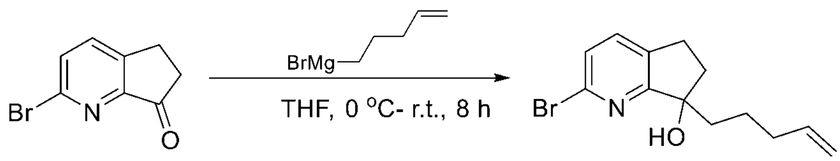


Pent-4-en-1-methylmagnesium bromide (5 mL, 25.0 mmol, 0.5 M in tetrahydrofuran) was added dropwise to a solution of 2-bromo-5,6-dihydro-7*H*-cyclopenta[*b*]pyridin-7-one (1.0 g, 4.74 mmol) in tetrahydrofuran (10 mL) at 0 °C and under an argon atmosphere. The resulting mixed reaction solution was stirred overnight at room temperature under an argon atmosphere. The mixed reaction solution was quenched with a saturated aqueous ammonium chloride solution (20 mL) and extracted with ethyl acetate (3 × 30 mL) at 0 °C. The combined organic phases were washed with saturated brine (1 × 20 mL), dried over anhydrous sodium sulfate, filtered, and concentrated under reduced pressure. The residue was purified by silica gel column chromatography (ethyl acetate:petroleum ether = 0%-30% elution) to give 2-bromo-7-(pent-4-en-1-yl)-6,7-dihydro-5*H*-cyclopenta[*b*]pyridin-7-ol (360 mg, yield: 27%), LC-MS m/z: 282 [M + H]$^+$.

**2) Synthesis of 2-allyl-1-(7-hydroxy-7-(pent-4-en-1-yl)-6,7-dihydro-5*H*-cyclopenta[*b*]pyridin-2-yl)-6-(methylthio)-1,2-dihydro-3*H*-pyrazolo[3,4-*d*]pyrimidin-3-one**

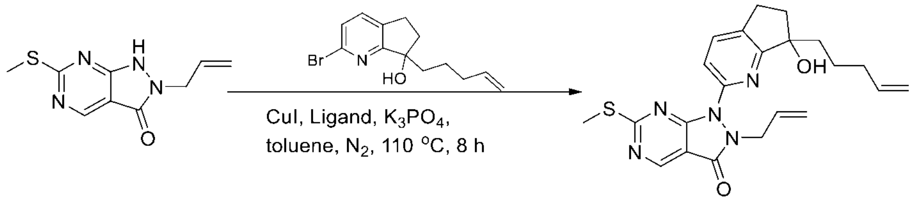


A solution of 2-allyl-6-(methylthio)-1,2-dihydro-3H-pyrazolo[3,4-*d*]pyrimidin-3-one (211 mg, 0.95 mmol), 2-bromo-7-(pent-4-en-1-yl)-6,7-dihydro-5*H*-cyclopenta[*b*]pyridin-7-ol (320 mg, 1.14 mmol), copper(I) iodide (90 mg, 0.47 mmol), (1*R*,2*R*)-*N*,*N*-dimethyl-1,2-cyclohexanediamine (54 mg, 0.38 mmol), and potassium phosphate (604 mg, 2.85 mmol) in toluene (8 mL) was heated to 110 °C and stirred for 8 h under a nitrogen atmosphere. The resulting mixed reaction solution was concentrated under reduced pressure. The residue was purified by C18 column chromatography (acetonitrile:purified water containing 0.1 % $NH_3 \cdot H_2O$ = 5%-95% elution) to give 2-allyl-1-(7-hydroxy-7-(pent-4-en-1-yl)-6,7-dihydro-5*H*-cyclopenta[b]pyridin-2-yl)-6-(methylthio)-1,2-dihydro-3*H*-pyrazolo[3,4-*d*]pyrimidin-3-one (234 mg, yield: 58.1%), LC-MS m/z: 424 [M + H]$^+$.

**3) Synthesis of 3a-hydroxy-14-(methylthio)-3,3a,4,5,6,9-hexahydro-2*H*,11*H*-1,17-(epiethane[1,2]diylidene)cyclopenta[e]pyrimido[4',5':3,4]pyrazolo[1,2-*a*][1,2,4]triazacyclododecin-11-one**

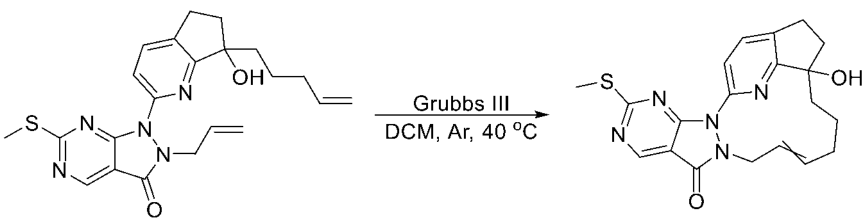


A solution of 2-allyl-1-(7-hydroxy-7-(pent-4-en-1-yl)-6,7-dihydro-5*H*-cyclopenta[*b*]pyridin-2-yl)-6-(methylthio)-1,2-dihydro-3*H*-pyrazolo[3,4-*d*]pyrimidin-3-one (234 mg, 0.55 mmol) and Grubbs III (98 mg, 0.11 mmol) in dichloromethane (100 mL) was heated to 40 °C and stirred overnight under an argon atmosphere. After cooling, the mixed reaction solution was concentrated under reduced pressure. The residue was purified by silica gel column chromatography (ethyl acetate:petroleum ether = 20%-65% elution) to give 3a-hydroxy-14-(methylthio)-3,3a,4,5,6,9-hexahydro-2*H*,11*H*-1,17-(epiethane[1,2]diylidene)cyclopenta[e]pyrimido[4',5':3,4]pyrazolo[1,2-a][1,2,4]triazacyclododecin-11-one (23 mg, yield: 10.5%), LC-MS m/z: 396 [M + H]$^+$.

**4) Synthesis of 3a-hydroxy-14-(methylsulfonyl)-3,3a,4,5,6,9-hexahydro-2*H*,11*H*-1,17-(epiethane[1,2]diylidene) cyclopenta[*e*]pyrimido[4',5':3,4]pyrazolo[1,2-*a*][1,2,4]triazacyclododecin-11-one**

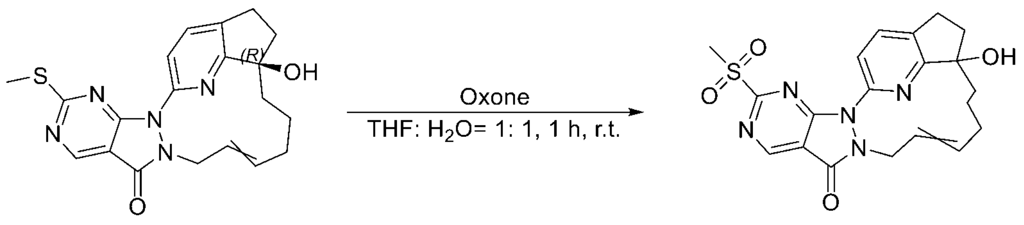


A mixed solution of 3a-hydroxy-14-(methylthio)-3,3a,4,5,6,9-hexahydro-2*H*,11*H*-1,17-(epiethane[1,2]diylidene) cyclopenta[*e*]pyrimido[4',5':3,4]pyrazolo[1,2-a][1,2,4]triazacyclododecin-11-one (50 mg, 0.13 mmol) and Oxone (87.6 mg, 0.25 mmol) in tetrahydrofuran and water (2 mL, v:v =1:1) was stirred at room temperature for 1 h. The resulting mixed reaction solution was concentrated under reduced pressure to give a crude product, which was used directly in the next step without further purification. LC-MS *m/z:* 428 [M + H]$^+$;

**5) Synthesis of 3a-hydroxy-14-((3-methyl-4-((1-methylpiperidin-4-yl)oxy)phenyl)amino)-3,3a,4,5,6,9-hexahydro-2*H*,11*H*-1,17-(epiethane[1,2]diylidene)cyclopenta[*e*]pyrimido [4',5':3,4]pyrazolo [1,2-*a*][1,2,4]triazacyclododecin-11-one**

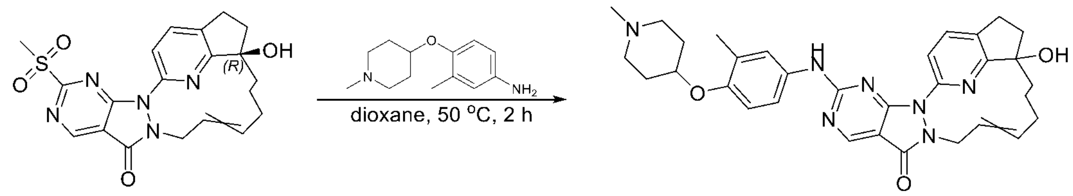


A solution of 3a-hydroxy-14-(methylsulfonyl)-3,3a,4,5,6,9-hexahydro-2*H*,11*H*-1,17-(epiethane[1,2]diylidene) cyclopenta[*e*]pyrimido[4',5':3,4]pyrazolo[1,2-*a*][1,2,4]triazacyclododecin-11-one (45 mg, 0.11 mmol) and 3-methyl-4-((1-methylpiperidin-4-yl)oxy)aniline (75 mg, 0.34 mmol) in 1,4-dioxane (1 mL) was heated to 50 °C and stirred for 2 h. After cooling, the mixed reaction solution was concentrated under reduced pressure. The residue was purified by C18 column chromatography (acetonitrile:purified water containing 0.1% formic acid = 5%-95% elution) to give 3a-hydroxy-14-((3-methyl-4-((1-methylpiperidin-4-yl)oxy)phenyl)amino)-3,3a,4,5,6,9-hexahydro-2*H*,11*H*-1,17-(epiethane [1,2]diylidene)cyclopenta[*e*]pyrimido[4',5':3,4]pyrazolo[1,2-*a*][1,2,4]triazacyclododecin-11-one (27 mg, yield: 45.2%), LC-MS m/z: 568 $[M + H]^+$;

1H NMR (400 MHz, MeOD-d4) δ 8.77 (s, 1H), 7.93 (d, J = 8.1 Hz, 1H), 7.72 (d, J = 8.0 Hz, 1H), 7.56 (s, 1H), 7.40 - 7.32 (m, 1H), 6.89 (dd, J = 16.3, 8.9 Hz, 1H), 5.93 - 5.79 (m, 1H), 5.53 (t, J = 9.5 Hz, 1H), 4.57 - 4.41 (m, 1H), 4.27 (dd, J = 14.9, 4.4 Hz, 1H), 4.11 (dd, J = 14.8, 10.9 Hz, 1H), 3.61 (d, J = 12.4 Hz, 1H), 3.50 - 3.41 (m, 2H), 3.19 - 3.10 (m, 2H), 2.99 - 2.91 (m, 5H), 2.50 - 2.35 (m, 3H), 2.27 (s, 4H), 2.18 (s, 1H), 2.15 - 1.98 (m, 3H), 1.83 (t, J = 13.4 Hz, 4H), 1.67 - 1.55 (m, 2H).

**Example 14: Synthesis of (*R,Z*)-12-hydroxy-12-methyl-2-((3-methyl-4-((1-(methyl-$d_3$)piperidin-4-yl)oxy)phenyl) amino)-7,10,11,12-tetrahydro-5*H*-13,17-(azeno)pyrimido[4',5':3,4]pyrazolo[1,2-*a*][1,2]diazacyclotridecin-5-one or Enantiomer and (*S,Z*)-12-hydroxy-12-methyl-2-((3-methyl-4-((1-(methyl-$d_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compounds 171A and B)**

**1) Synthesis of *tert*-butyl 4-(2-methyl-4-nitrophenoxy)piperidine-1-carboxylate**

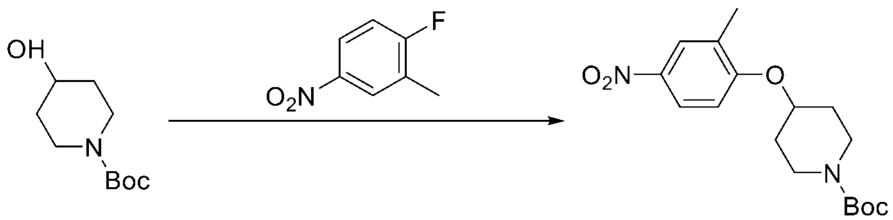

Sodium hydride (796 mg, 19.9 mmol, content: 60%) was added to a solution of *tert*-butyl 4-hydroxypiperidine-1-carboxylate (2000 mg, 9.95 mmol) in tetrahydrofuran (20 mL), and the mixture was stirred at room temperature for 1 h. Then, 1-fluoro-2-methyl-4-nitrobenzene (1850.7 mg, 11.94 mmol) was added in batches at room temperature. The resulting mixed reaction solution was stirred overnight at 50 °C. After cooling, the mixed reaction solution was quenched with methanol (50 mL) and concentrated under reduced pressure. The residue was purified by C18 reverse phase column chromatography ($CH_3CN$:$H_2O$ (0.1% $NH_4HCO_3$) = 5%-95% elution) to give *tert*-butyl 4-(2-methyl-4-nitrophenoxy) piperidine-1-carboxylate (1100 mg, yield: 32.9%), LC-MS *m/z:* 337 $[M + H]^+$.

**2) Synthesis of 4-(2-methyl-4-nitrophenoxy)piperidine**

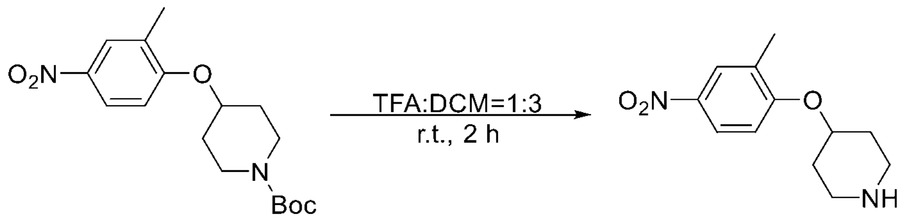


2,2,2-Trifluoroacetic acid (1 mL) was added dropwise to a solution of *tert*-butyl 4-(2-methyl-4-nitrophenoxy) piperidine-1-carboxylate (1100 mg, 3.27 mmol) in dichloromethane (3 mL) at room temperature. The resulting mixed reaction solution was stirred at room temperature for 1 h. The resulting mixed reaction solution was concentrated under reduced pressure. The residue was purified by C18 reverse phase column chromatography ($CH_3CN:H_2O$ (0.1% $NH_4HCO_3$) = 5%-95% elution) to give 4-(2-methyl-4-nitrophenoxy)piperidine (659 mg, yield: 85.3%), LC-MS *m/z:* 237 $[M + H]^+$.

**3) Synthesis of 1-(methyl-$d_3$)-4-(2-methyl-4-nitrophenoxy)piperidine**

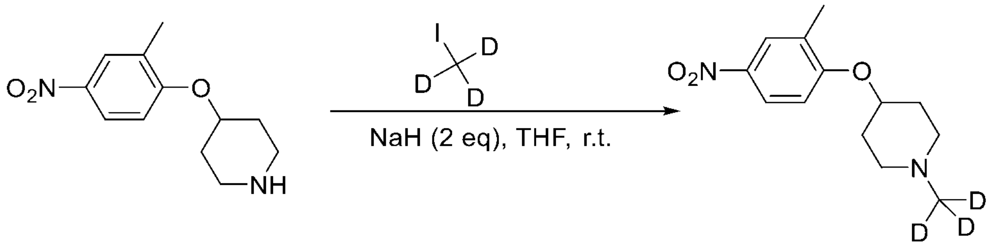


Sodium hydride (223.2 mg, 5.58 mmol, content: 60%) was added in batches to a solution of 4-(2-methyl-4-nitrophenoxy)piperidine (659 mg, 2.79 mmol) in tetrahydrofuran (10 mL) at 0 °C. The resulting mixed reaction solution was stirred at room temperature for 1 h. Iodomethane-$d_3$ (485 mg, 3.35 mmol) was added in batches to the above mixed reaction solution at room temperature. The resulting mixed reaction solution was heated to 50 °C and stirred overnight. After cooling, the mixed reaction solution was quenched with methanol (20 mL) and concentrated under reduced pressure at 0°C. The residue was purified by C18 reverse phase column chromatography ($CH_3CN:H_2O$ (0.1% $NH_4HCO_3$) = 5%-95% elution) to give 1-(methyl-$d_3$)-4-(2-methyl-4-nitrophenoxy)piperidine (301 mg, yield: 42.6%), LC-MS m/z: 254 $[M + H]^+$.

**4) Synthesis of 3-methyl-4-((1-(methyl-$d_3$)piperidin-4-yl)oxy)aniline**

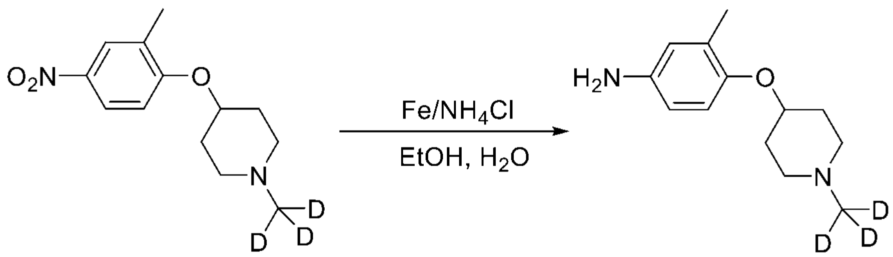


A solution of 1-(methyl-$d_3$)-4-(2-methyl-4-nitrophenoxy)piperidine (301 mg, 1.19 mmol), Fe (333 mg, 5.95 mmol) in saturated aqueous ammonium chloride solution (1 mL) and ethanol (4 mL) was heated to 70 °C and stirred overnight. After cooling, the mixed reaction solution was filtered, and the filter cake was washed with methanol (3 × 10 mL). The filtrate was collected and concentrated under reduced pressure to give a crude product, which was used directly in the next step without further purification. LC-MS m/z: 224 $[M + H]^+$;

**5) Synthesis of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one**

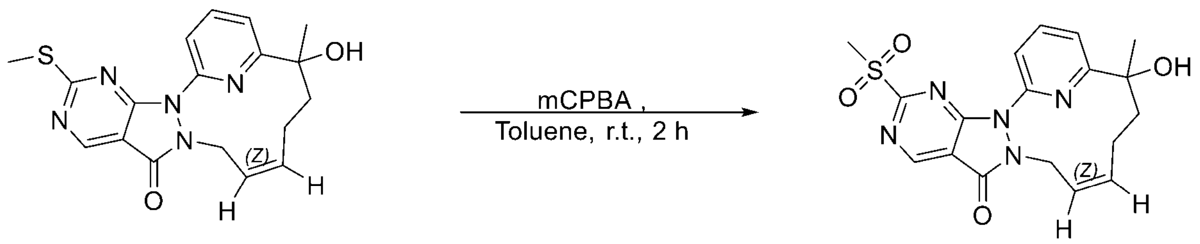


A solution of (*Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one (510 mg, 1.38 mmol) and 3-chloroperoxybenzoic acid (500 mg, 2.46 mmol) in toluene (50 mL) was stirred at room temperature for 3 h. The resulting mixed reaction solution was concentrated under reduced pressure to give a crude product, which was used directly in the next step without further purification. LC-MS *m/z:* 402 $[M + H]^+$;

**6) Synthesis of (*Z*)-12-hydroxy-12-methyl-2-((3-methyl-4-((1-(methyl-*d*$_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one**

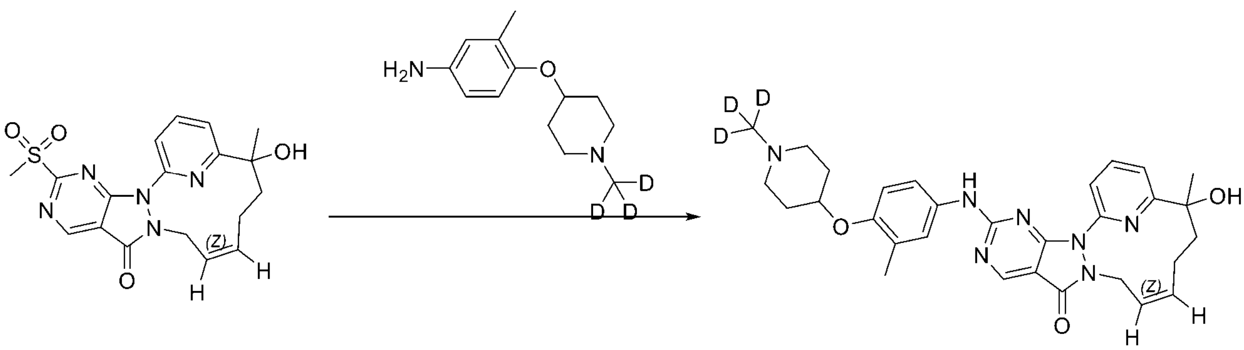

A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (400 mg, 0.99 mmol) and 3-methyl-4-((1-(methyl-*d*$_3$)piperidin-4-yl)oxy)aniline (266 mg, 1.19 mol) in 1,4-dioxane (20 mL) was heated to 100 °C and stirred overnight. After cooling, the mixed reaction solution was filtered and concentrated under reduced pressure. The residue was purified by C18 reverse phase column chromatography ($CH_3CN:H_2O$ (0.1% $NH_4HCO_3$) = 5%-95% elution) to give (*Z*)-12-hydroxy-12-methyl-2-((3-methyl-4-((1-(methyl-*d*$_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (201 mg, yield: 37.3%), LC-MS *m/z:* 545 $[M + H]^+$.

**7) Synthesis of (*R,Z*)-12-hydroxy-12-methyl-2-((3-methyl-4-((1-(methyl-*d*$_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer and (*S,Z*)-12-hydroxy-12-methyl-2-((3-methyl-4-((1-(methyl-d$_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

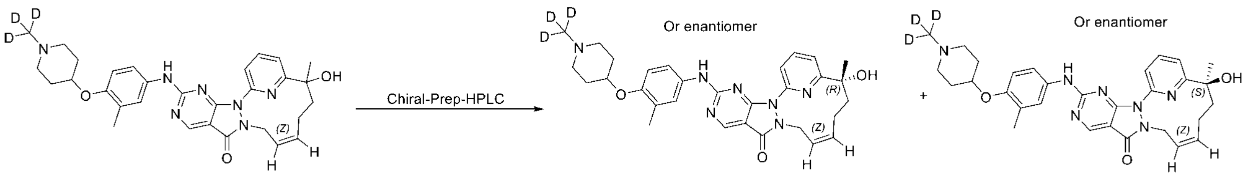


Compound 171A Compound 171B

($Z$)-12-Hydroxy-12-methyl-2-((3-methyl-4-((1-(methyl-$d_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (201 mg, 0.37 mmol) was purified by preparative chiral high performance liquid chromatography (chromatography conditions: system: Waters SFC 150; column model: DAICELCHIRALCEL®AD; column: 250 × 25 mm 10 μm; mobile phase A: supercritical $CO_2$; mobile phase B: EtOH (+ 0.1% 7.0 mol/L ammonia in MeOH); A:B = 65:35; detection wavelength: 214 nm; flow rate: 100 mL/min; column temperature: room temperature; column pressure: 100 bar) to give two isomers: **compound 171A** and **compound 171B: Compound 171A:** Peak 1 **(peak time: 2.389 min):** ($R$,$Z$)-12-hydroxy-12-methyl-2-((3-methyl-4-((1-(methyl-$d_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (80 mg, yield: 40%), LC-MS *m/z:* 545 $[M + H]^+$;

$^1H$ NMR (400 MHz, MeOD): δ 8.79 (s, 1H), , 7.95 (t, J = 7.9 Hz, 1H), 7.81 (d, J = 8.0 Hz, 1H), 7.59 (d, J = 7.7 Hz, 2H), 7.35 (dd, J = 9.0, 2.4 Hz, 1H), 6.88 (d, J = 8.8 Hz, 1H), 5.48 (dd, J = 13.4, 6.7 Hz, 2H), 4.59 (d, J = 6.8 Hz, 2H), 4.43 (s, 1H), 2.82 (s, 2H), 2.55 (s, 2H), 2.22 (s, 3H), 2.03 (s, 4H), 1.89 (s, 4H), 1.68 (s, 3H).

**Compound 171B:** Peak 2 **(peak time: 3.537 min):** (S,Z)-12-hydroxy-12-methyl-2-((3-methyl-4-((1-(methyl-$d_3$)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (80 mg, yield: 40%), LC-MS *m/z:* 545 $[M + H]^+$;

$^1H$ NMR (400 MHz, MeOD): δ 8.79 (s, 1H), 7.95 (t, J = 7.9 Hz, 1H), 7.81 (d, J = 8.1 Hz, 1H), 7.58 (d, J = 7.7 Hz, 2H), 7.35 (dd, J = 8.7, 2.5 Hz, 1H), 6.88 (d, J = 8.8 Hz, 1H), 5.61 - 5.38 (m, 2H), 4.58 (d, J = 6.9 Hz, 2H), 4.43 (s, 1H), 2.81 (s, 2H), 2.53 (s, 2H), 2.21 (s, 3H), 2.06 (dd, J = 33.8, 24.0 Hz, 4H), 1.93 (d, J = 29.8 Hz, 4H), 1.68 (s, 3H).

**Example 15: Synthesis of (*R*,*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer and (*S*,*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compounds 172A and B)**

**1) Synthesis of 1-methylpiperidin-4-*d*-4-ol**

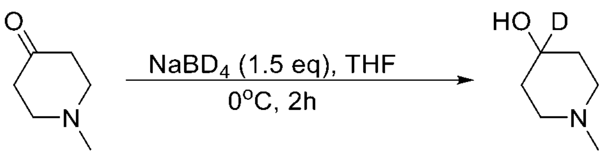


$NaBD_4$ (557.5 mg, 13.3 mmol) was added in batches to a solution of 1-methylpiperidin-4-one (1.0 g, 8.85 mmol) in tetrahydrofuran (20 mL) at 0 °C. The resulting mixed reaction solution was stirred overnight at room temperature. The resulting mixed reaction solution was concentrated under reduced pressure to give a crude product, which was used directly in the next step without further purification. LC-MS *m/z:* 117 $[M + H]^+$;

**2) Synthesis of 1-methyl-4-(2-methyl-4-nitrophenoxy)piperidine-4-*d***

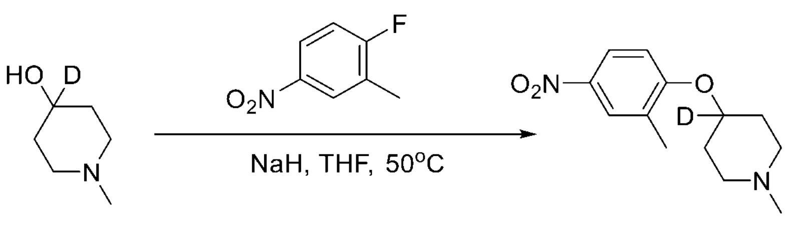

Sodium hydride (690 mg, 17.24 mmol, content: 60%) was added in batches to a solution of 1-methylpiperidin-4-d-4-ol (1 g, 8.62 mmol) in tetrahydrofuran (20 mL) at 0 °C, and the mixture was stirred at 0°C for 1 h. 1-Fluoro-2-methyl-4-nitrobenzene (1.6 g, 10.3 mmol) was added in batches to the above mixed reaction solution at 0 °C. The resulting mixed reaction solution was heated to 50 °C and stirred overnight. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give 1-methyl-4-(2-methyl-4-nitrophenoxy)piperidine-4-d (600 mg, yield: 27.7%), LC-MS *m/z:* 252 $[M + H]^+$.

**3) Synthesis of 3-methyl-4-((1-methylpiperidin-4-yl-4-d)oxy)aniline**

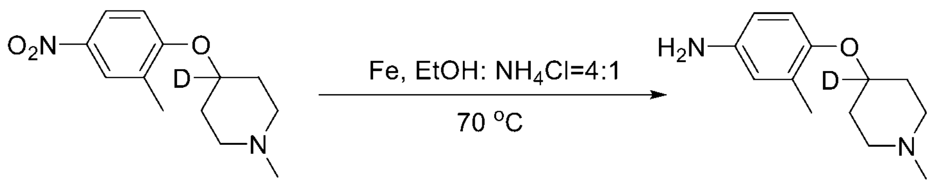


A solution of 1-methyl-4-(2-methyl-4-nitrophenoxy)piperidine-4-*d* (600 mg, 2.39 mmol) and Fe (669.3 mg, 11.95 mmol) in aqueous ammonium chloride solution (1 mL) and ethanol (4 mL) was heated to 70 °C and stirred for 2 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m/z:* 222 $[M + H]^+$;

**4) Synthesis of (*Z*)-12-hydroxy-12-methyl-2-((3-methyl-4-((1-methylpiperidin-4-yl-4-d)oxy)phenyl)amino)-7,10,11,12-tetrahydro-5*H*-13,17-(azeno)pyrimido[4',5':3,4]pyrazolo[1,2-*a*][1,2]diazacyclotridecin-5-one**

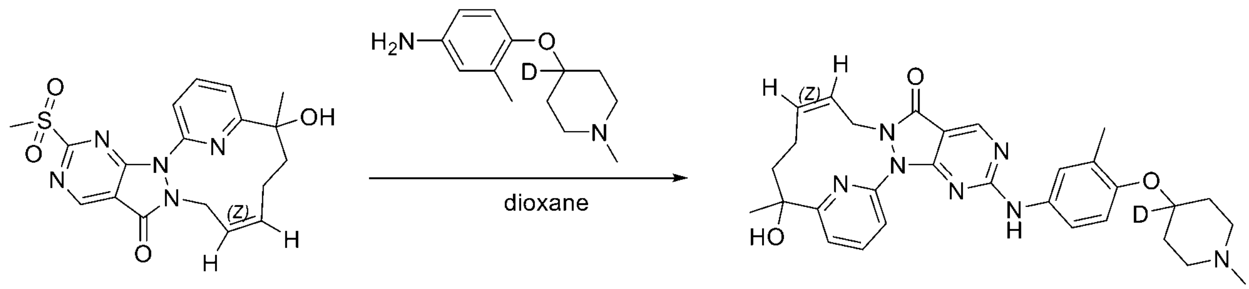


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (400 mg, 0.99 mmol) and 3-methyl-4-((1-methylpiperidin-4-yl-4-d)oxy)aniline (266 mg, 1.19 mol) in 1,4-dioxane (20 mL) was heated to 100 °C and stirred overnight. After cooling, the mixed reaction solution was concentrated under reduced pressure. The residue was purified by C18 reverse phase column chromatography ($CH_3CN$:$H_2O$ (0.1% $NH_4HCO_3$) = 5%-95% elution) to give (*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4] pyrazolo[1,2-*a*][1,2]diazacyclotridecin-5-one (201 mg, yield: 37.3%), LC-MS *m/z:* 543 $[M + H]^+$.

**5) Synthesis of (*R*,*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer and (*S*,*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

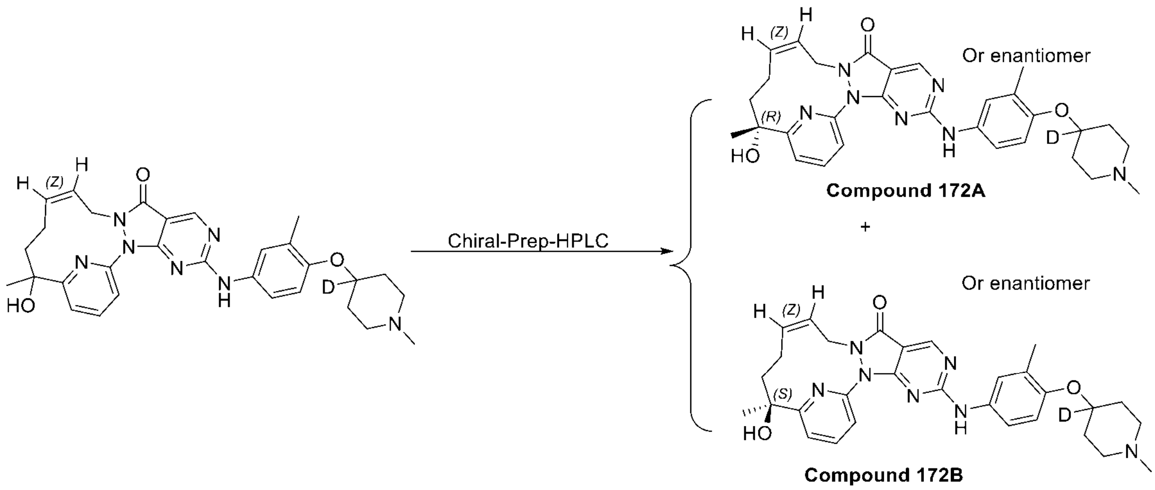


Compound 172A

+

Compound 172B

(*Z*)-12-Hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (201 mg, 0.37 mmol) was purified by preparative chiral high performance liquid chromatography (chromatography conditions: system: Waters SFC 150; column model: DAICELCHIRALCEL®AD; column size: 250 × 25 mm 10 μm; mobile phase A: supercritical $CO_2$; mobile phase B: ETOH (+ 0.1% 7.0 mol/L ammonia in MEOH); A:B = 60:40; detection wavelength: 214 nm; flow rate: 100 mL/min; column temperature: RT; column pressure: 100 bar) to give two isomers: **compound 172A** and **compound 172B:**

**Compound 172A: Peak 1 (peak time: 2.338 min):** (*R*,*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (80 mg, yield: 40%), LC-MS *m/z:* 543 [M + H]$^+$;
$^1$H NMR (400 MHz, MeOD): δ 8.80 (s, 1H), 7.94 (d, J = 8.0 Hz, 1H), 7.82 (d, J = 8.0 Hz, 1H), 7.59 (d, J = 8.0 Hz, 2H), 7.35 (dd, J = 8.6, 2.6 Hz, 1H), 6.88 (d, J = 8.7 Hz, 1H), 5.52 - 5.43 (m, 2H), 4.59 (d, J = 6.4 Hz, 2H), 2.73 (s, 2H), 2.45 (s, 2H), 2.34 (s, 3H), 2.21 (s, 3H), 1.99 (s, 4H), 1.87 (s, 4H), 1.68 (s, 3H).

**Compound 172B: Peak 2 (peak time: 3.527 min):** (*S*,*Z*)-12-hydroxy-12-methyl-2-((3-methyl-4-((1-methylpiperidin-4-yl-4-*d*)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (80 mg, yield: 40%), LC-MS *m/z:* 543 [M + H]$^+$;

$^1$H NMR (400 MHz, MeOD): δ 8.80 (s, 1H), 7.94 (d, J = 7.9 Hz, 1H), 7.82 (d, J = 8.0 Hz, 1H), 7.59 (d, J = 7.7 Hz, 2H), 7.35 (dd, J = 8.6, 2.6 Hz, 1H), 6.88 (d, J = 8.7 Hz, 1H), 5.52 - 5.43 (m, 2H), 4.59 (d, J = 6.7 Hz, 2H), 2.73 (s, 2H), 2.45 (s, 2H), 2.34 (s, 3H), 2.21 (s, 3H), 1.99 (s, 4H), 1.87 (s, 4H), 1.68 (s, 3H).

**Example 16: Synthesis of (*R*,*Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer and (*S*,*Z*)-12-hydroxy-12-methyl-2-((4-((1-methylpiperidin-4-yl)oxy)-3-(trifluoromethyl)phenyl)amino)-7,10,11,12-tetrahydro-5H-13,17-(azeno)pyrimido[4',5':3,4]pyrazolo[1,2-*a*][1,2]diazacyclotridecin-5-one or Enantiomer (Compounds 173A and B)**

**1) Synthesis of 1-methyl-4-(4-nitro-2-(trifluoromethyl)phenoxy)piperidine**

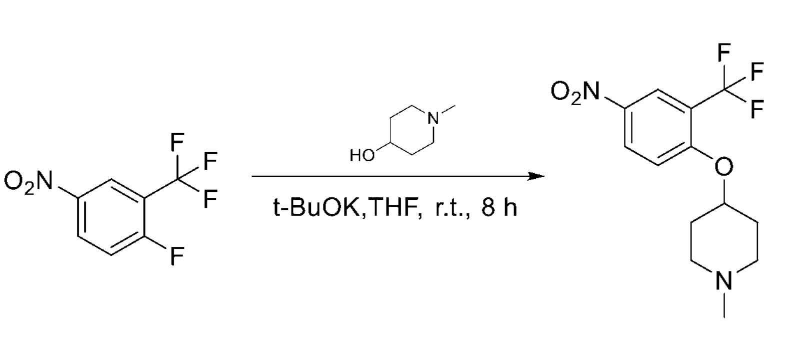

Potassium *tert*-butoxide (5.37 g, 47.82 mmol) was added to a solution of 1-methylpiperidin-4-ol (3.03 g, 26.30 mmol) in tetrahydrofuran (50 mL) at 0 °C. 1-Fluoro-4-nitro-2-(trifluoromethyl)benzene (5 g, 23.91 mmol) was added to the above mixed reaction solution at 0 °C. The resulting mixed reaction solution was stirred at room temperature for 8 h. The mixed reaction solution was concentrated under reduced pressure. The residue was purified by silica gel column chromatography (ethyl acetate/petroleum ether = 0%-55% elution) to give 1-methyl-4-(4-nitro-2-(trifluoromethyl)phenoxy)piperidine (5.2 g, yield: 71.5%), LC-MS *m/z:* 305 $[M + H]^+$.

**2) Synthesis of 4-((1-methylpiperidin-4-yl)oxy)-3-(trifluoromethyl)aniline**

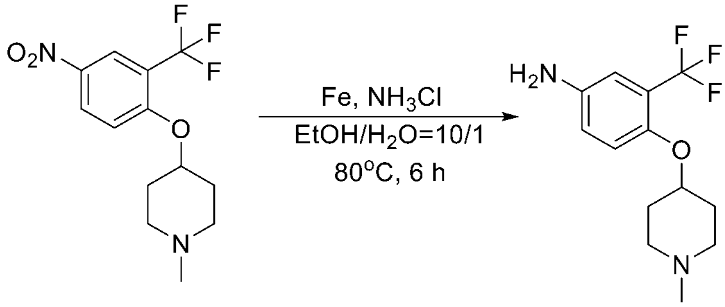


A solution of 1-methyl-4-(4-nitro-2-(trifluoromethyl)phenoxy)piperidine (3 g, 9.86 mmol), Fe (2.75 g, 49.3 mmol), and ammonium chloride (5.27 g, 98.6 mmol) in ethanol (30 mL) and water (3 mL) was heated to 80 °C and stirred for 6 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m*/*z*: 275 $[M + H]^+$;

**3) Synthesis of (*Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one**

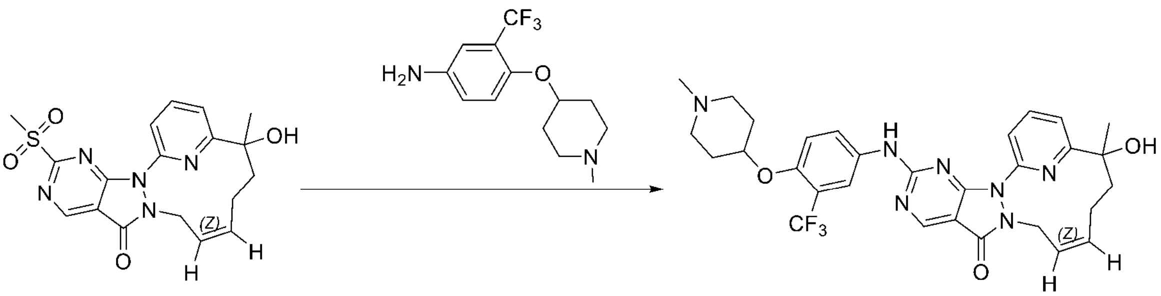

A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (217 mg, 0.54 mmol) and 4-((1-methylpiperidin-4-yl)oxy)-3-(trifluoromethyl)aniline (178.2 mg, 0.65 mmol) in 1,4-dioxane (10 mL) was heated to 100 °C and stirred overnight. After cooling, the filtrate was concentrated under reduced pressure. The residue was purified by C18 reverse phase column chromatography ($CH_3CN$:$H_2O$ (0.1% $NH_4HCO_3$) = 5%-95% elution) to give (*Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one (96 mg, yield: 29.8%), LC-MS *m/z:* 596 $[M + H]^+$.

**4) Synthesis of (*R,Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer and (*S,Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

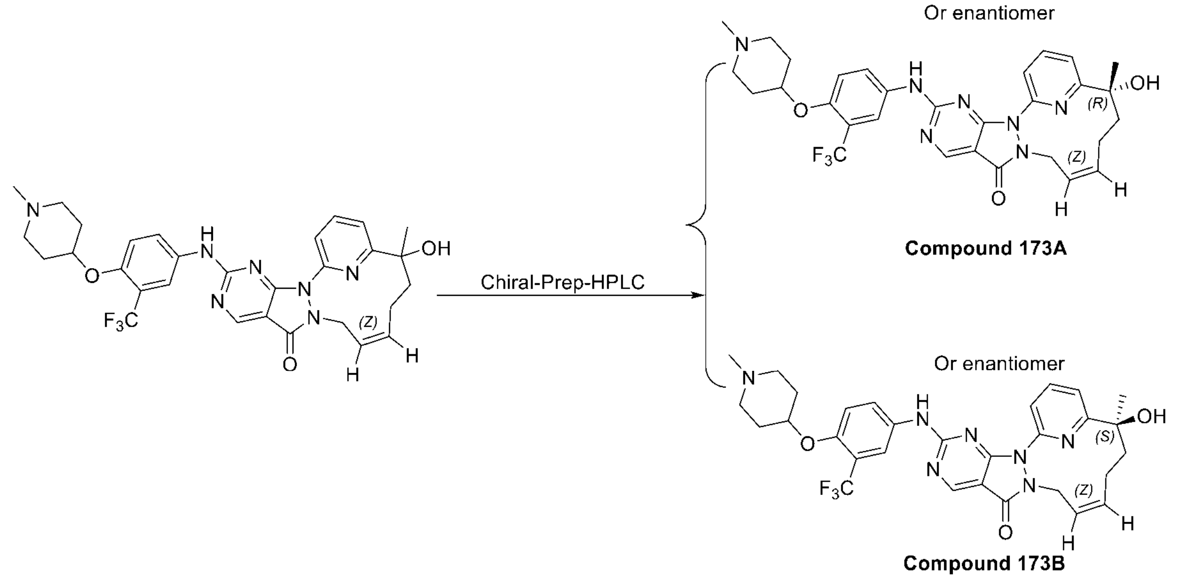


Compound 173A

Compound 173B

(*Z*)-12-Hydroxy-12-methyl-2-((4-((1-methylpiperidin-4-yl)oxy)-3-(trifluoromethyl)phenyl)amino)-7,10,11,12-tetrahydro-5*H*-13,17-**(azeno)pyrimido[4',5':3,4]pyrazolo[1,2-*a*][1,2]diazacyclotridecin-5-one** (96 mg, 0.16 mmol) was purified by preparative chiral high performance liquid chromatography (chromatography conditions: system: SHIMADZU LC-20AP; column model: DAICELCHIRALPAK®IE; column size: 250 × 25 mm 10 μm; mobile phase A: n-hexane; mobile phase B: EtOH (+ 0.1% 7.0 mol/L ammonia in EtOH); A:B = 50:50; detection wavelength: 214 nm; flow rate: 40 mL/min; column temp: RT) to give two isomers: **compound 173A** and **compound 173B:**

**Compound 173A:** Peak 1 **(peak time: 17.883 min):** (*R*,*Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (40 mg, yield: 41.6%), LC-MS *m/z:* 596 [M + H]$^+$;
$^1$H NMR (400 MHz, MeOD): δ 8.87 (s, 1H), 8.21 (s, 1H), 7.95 (t, J = 8.0 Hz, 1H), 7.78 (d, J = 8.0 Hz, 1H), 7.68 (s, 1H), 7.63 (d, J = 8.0 Hz, 1H), 7.19 (d, J = 8.0 Hz, 1H), 5.51-5.47 (m, 2H), 4.70-4.67 (m, 1H), 4.63 (d, J = 6.4 Hz, 2H), 2.86-2.82 (m, 2H), 2.69-2.65 (m, 2H), 2.46 (s, 3H), 2.21-2.16 (m, 2H), 2.07-1.98 (m, 6H), 1.71 (s, 3H).

**Compound 173B:** Peak 2 (peak time: after peak 1): (*S,Z*)-12-hydroxy-12-methyl-2-((4-((1-methylpiperidin-4-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (40 mg, yield: 41.6%), LC-MS m/z: 596 [M + H]$^+$;

$^1$H NMR (400 MHz, MeOD): δ 8.87 (s, 1H), 8.21 (s, 1H), 7.95 (t, J = 7.9 Hz, 1H), 7.78 (d, J = 7.8 Hz, 1H), 7.68 (s, 1H), 7.63 (d, J = 7.6 Hz, 1H), 7.20 (d, J = 8.9 Hz, 1H), 5.49 (dd, J = 12.4, 6.3 Hz, 2H), 4.69 (s, 1H), 4.64 (d, J = 7.0 Hz, 2H), 2.84 (s, 2H), 2.69 (s, 2H), 2.46 (s, 3H), 2.18 (s, 2H), 2.05 (s, 3H), 1.97 (d, J = 18.4 Hz, 3H), 1.71 (s, 3H).

**Example 17: Synthesis of (*Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 174)**

**1) Synthesis of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one**

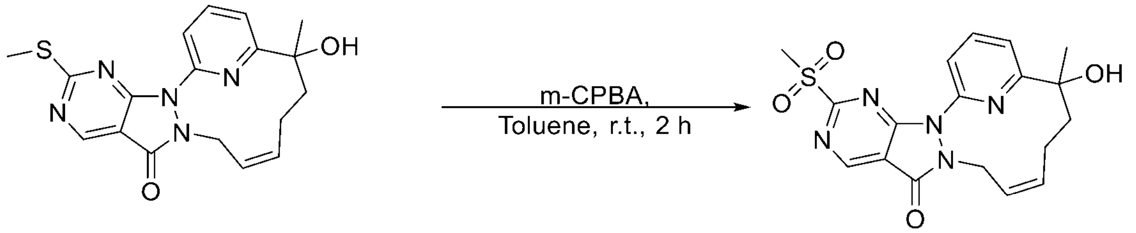

A solution of (*Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one (510 mg, 1.38 mmol) and 3-chloroperoxybenzoic acid (500 mg, 2.46 mmol) in toluene (50 mL) was stirred at room temperature for 3 h. The resulting mixed reaction solution was concentrated under reduced pressure to give a crude product, which was used directly in the next step without further purification. LC-MS *m*/*z:* 402 [M + H]+;

**2) Synthesis of (*Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one**

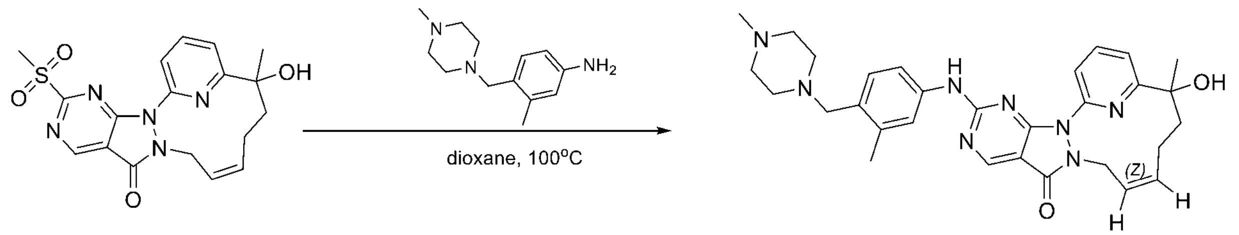


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (500 mg, 1.24 mmol) and 3-methyl-4-((4-methylpiperazin-1-yl) methyl)aniline (328.3 mg, 1.49 mmol) in 1,4-dioxane (50 mL) was heated to 100 °C and stirred overnight. After cooling, the mixed reaction solution was filtered and concentrated under reduced pressure. The residue was purified by silica gel column chromatography (MeOH:DCM = 0%-10% elution) to give (*Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2] diazacyclotridecin-5-one (490 mg, yield: 73.0%), LC-MS m/z: 541 [M + H]+;
$^1$H NMR (400 MHz, MeOD-d4) δ 8.84 (s, 1H), 7.98 (t, *J* = 8.0 Hz, 1H), 7.85 (d, *J* = 8.0 Hz, 1H), 7.68 - 7.58 (m, 2H), 7.41 (d, *J* = 6.8 Hz, 1H), 7.17 (d, *J* = 8.4 Hz, 1H), 5.48 (d, *J* = 6.4 Hz, 2H), 4.62 (d, *J* = 6.4 Hz, 2H), 3.48 (s, 2H), 2.51 (s, 7H), 2.36 (s, 3H), 2.29 (s, 3H), 2.25 - 2.11 (m, 2H), 2.00 (s, 1H), 1.93 (s, 1H), 1.69 (s, 3H).

**Example 18: Synthesis of (*Z*)-12-hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 175)**

**1) Synthesis of *tert*-butyl 5-(2-methyl-4-nitrophenoxy)hexahydrocyclopenta[*c*]pyrrole-2(1*H*)-carboxylate**

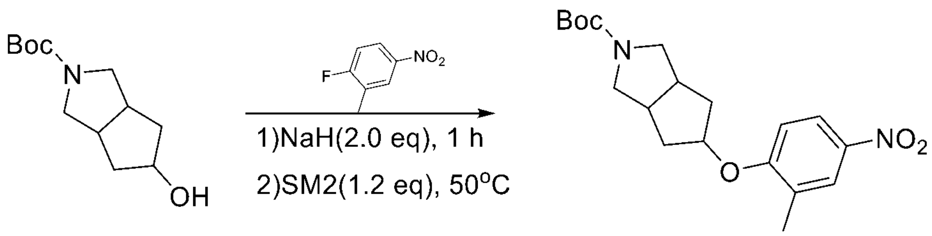


A solution of *tert*-butyl 5-hydroxyhexahydrocyclopenta[*c*]pyrrole-2(1*H*)-carboxylate (400 mg, 1.76 mmol) and sodium hydride (141 mg, 3.52 mmol, content: 60%) in tetrahydrofuran (4.0 mL) was stirred at room temperature for 1 h. Then, 1-fluoro-2-methyl-4-nitrobenzene (409 mg, 2.64 mmol) was added to the above mixed reaction solution, and the mixed reaction solution was heated to 50 °C and stirred for 2 h. After cooling, the mixed reaction solution was quenched with methanol (4 mL) at 0 °C. The mixed reaction solution was filtered and concentrated under reduced pressure. The residue was purified by C18 reverse phase column chromatography (MeCN:$H_2O$ (0.1% $NH_3.H_2O$) = 5%-95% elution) to give *tert*-butyl 5-(2-methyl-4-nitrophenoxy)hexahydrocyclopenta[*c*]pyrrole-2(1*H*)-carboxylate (450 mg, yield: 70.56%), LC-MS *m*/*z:* 363 [M + H]+.

**2) Synthesis of 5-(2-methyl-4-nitrophenoxy)octahydrocyclopenta[*c*]pyrrole**

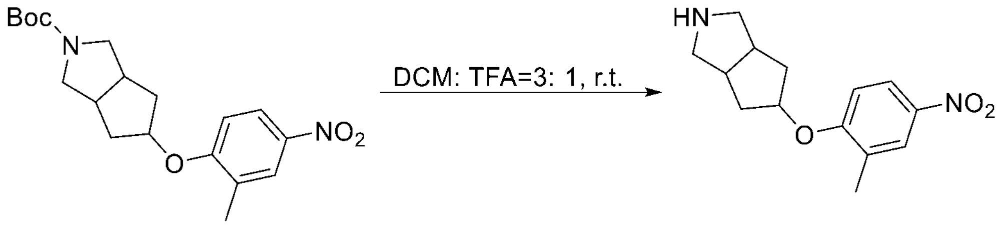


2,2,2-Trifluoroacetic acid (0.5 mL) was added dropwise to a solution of *tert*-butyl 5-(2-methyl-4-nitrophenoxy) hexahydrocyclopenta[*c*]pyrrole-2(1*H*)-carboxylate (450 mg, 1.24 mmol) in dichloromethane (1.5 mL) at room temperature. The resulting mixed reaction solution was stirred at room temperature for 1 h. The resulting mixed reaction solution was concentrated under reduced pressure. The residue was purified by C18 reverse phase column chromatography (MeCN:$H_2O$ (0.1% FA) = 5%-95% elution) to give 5-(2-methyl-4-nitrophenoxy)octahydrocyclopenta[c]pyrrole (300 mg, yield: 92.13%), LC-MS *m*/*z*: 263 $[M + H]^+$.

**3) Synthesis of 2-methyl-5-(2-methyl-4-nitrophenoxy)octahydrocyclopenta[*c*]pyrrole**

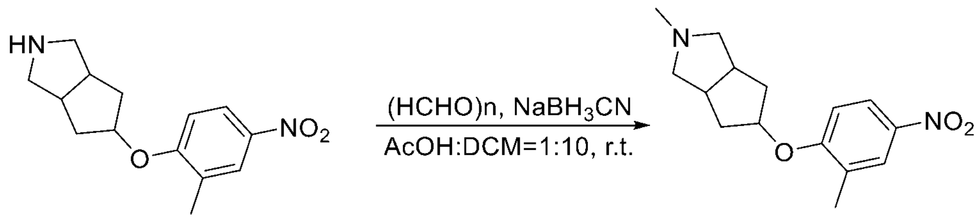


A solution of 5-(2-methyl-4-nitrophenoxy)octahydrocyclopenta[c]pyrrole (300 mg, 1.14 mmol), paraformaldehyde (73 mg, 2.29 mmol), and sodium cyanoborohydride (144 mg, 2.29 mmol) in acetic acid (0.3 mL) and dichloromethane (3 mL) was stirred at room temperature for 0.5 h. The mixed reaction solution was quenched with water (3 mL) at room temperature and concentrated under reduced pressure. The residue was purified by C18 reverse phase column chromatography (MeCN:$H_2O$ (0.1% FA) = 5%-95% elution) to give 2-methyl-5-(2-methyl-4-nitrophenoxy)octahydrocyclopenta[c]pyrrole (200 mg, yield: 63.13%), LC-MS *m*/*z*: 277 $[M + H]^+$.

**4) Synthesis of 3-methyl-4-((2-methyloctahydrocyclopenta[*c*]pyrrol-5-yl)oxy)aniline**

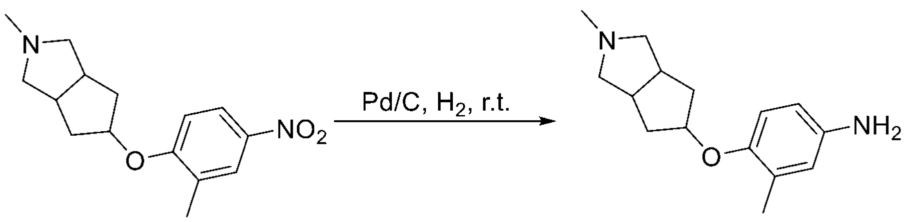


A solution of 2-methyl-5-(2-methyl-4-nitrophenoxy)octahydrocyclopenta[c]pyrrole (200 mg, 0.72 mmol) and Pd/C (200 mg) in methanol (5.0 mL) was stirred at room temperature for 4 h under a hydrogen atmosphere. The mixed reaction solution was filtered, the filter cake was washed with methanol (3 × 5 mL), and the filtrate was collected and concentrated under reduced pressure. The residue was purified by C18 reverse phase column chromatography (MeCN:$H_2O$ (0.1% $NH_3.H_2O$) = 5%-95% elution) to give 3-methyl-4-((2-methyloctahydrocyclopenta[*c*]pyrrol-5-yl)oxy) aniline (100 mg, yield: 56.18%), LC-MS m/z: 247 $[M + H]^+$.

**5) Synthesis of (*Z*)-12-hydroxy-12-methyl-2-((3-methyl-4-((2-methyloctahydrocyclopenta[*c*]pyrrol-5-yl)oxy) phenyl)amino)-7,10,11,12-tetrahydro-5*H*-13,17-(azeno)pyrimido[4',5':3,4]pyrazolo[1,2-*a*][1,2]diazacyclotridecin-5-one**

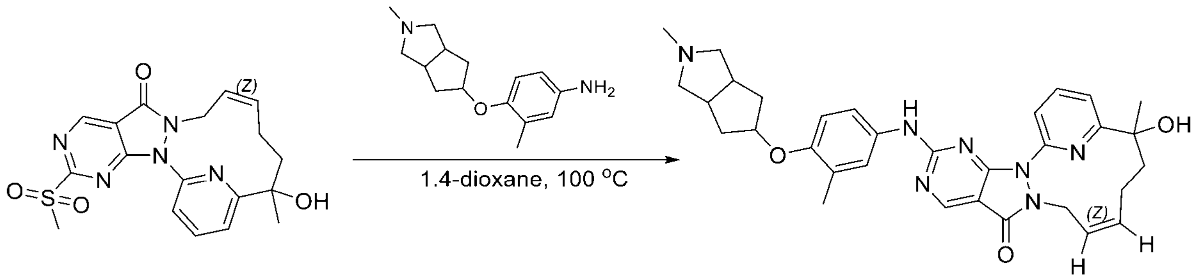


A solution of (*Z*)-12-hydroxy-12-methyl-2-(methylsulfonyl)-7,10,11,12-tetrahydro-5*H*-13,17-**(azeno)pyrimido [4',5':3,4]pyrazolo[1,2-*a*][1,2]diazacyclotridecin-5-one** (108 mg, 0.27 mmol) and 3-methyl-4-((2-methyloctahydrocyclopenta[c]pyrrol-5-yl)oxy)aniline (100 mg, 0.40 mmol) in 1,4-dioxane (2.0 mL) was heated to 100 °C and stirred for 16 h. After cooling, the mixed reaction solution was filtered and concentrated under reduced pressure. The residue was purified by C18 reverse phase column chromatography (MeCN:$H_2O$ (0.1% $NH_3.H_2O$) = 5%-95% elution) to give (*Z*)-12-hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (60 mg, yield: 39.14%), LC-MS *m*/*z:* 568 [M + H]$^+$;

$^1$H NMR (400 MHz, MeOD-d4):δ 8.80 (s, 1H), 7.98-7.92 (m, 1H), 7.82 (d, J=8.0 Hz, 1H), 7.62-7.53 (m, 2H), 7.42-7.38 (m, 1H), 6.90 (d, J=8.0 Hz, 1H), 5.53-5.39 (m, 2H), 4.60 (d, J=8.0 Hz, 2H), 3.61 (s, 1H), 3.20-3.07 (m, 4H), 2.87 (s, 3H), 2.32-2.10 (m, 8H), 2.08-1.96 (m, 3H), 1.95-1.84 (m, 2H), 1.68 (s, 3H).

**Example 19: Synthesis of (*S,Z*)-12-hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one or Enantiomer and (*R*,*Z*)-12-hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one or Enantiomer (Compounds 176A and B)**

**1) Synthesis of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one**

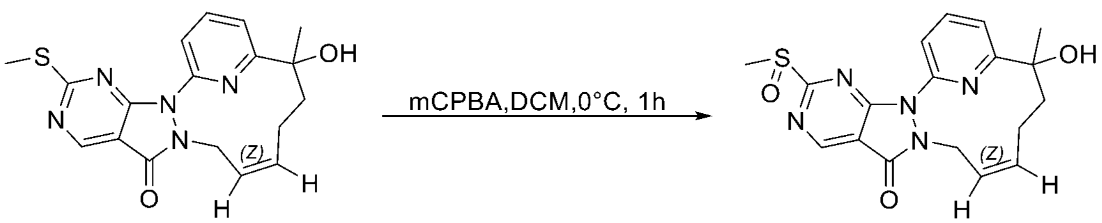


3-Chloroperoxybenzoic acid (141 mg, 0.81 mmol) was added in batches to a solution of (*Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one (200 mg, 0.54 mmol) in dichloromethane (2 mL) at 0 °C. The resulting mixed reaction solution was stirred at 0 °C for 1 h. The mixed reaction solution was concentrated under reduced pressure to give a crude product, which was used directly in the next step without further purification. LC-MS *m*/*z:* 386 [M + H]$^+$;

**2) Synthesis report of (*Z*)-12-hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one**

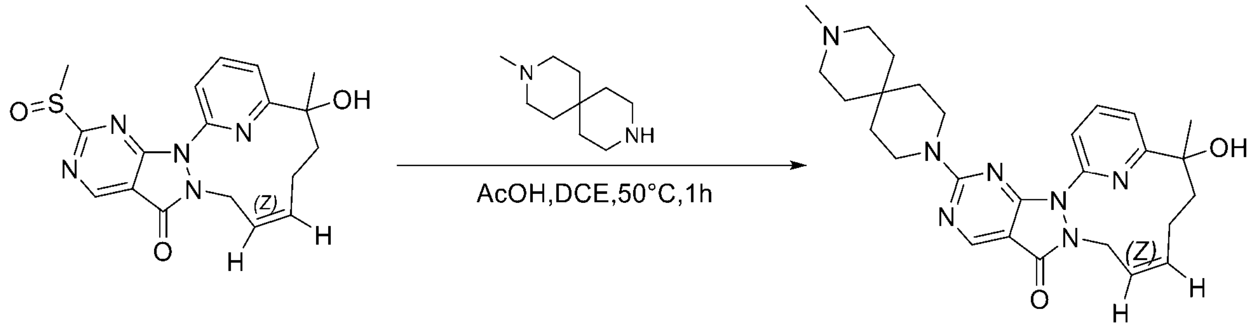


A solution of (*Z*)-12-hydroxy-12-methyl-2-(methylsulfinyl)-7,10,11,12-tetrahydro-5*H*-13,17-(azeno)pyrimido[4',5':3,4]pyrazolo[1,2-*a*][1,2]diazacyclotridecin-5-one (200 mg, 0.52 mmol), 3-methyl-3,9-diazaspiro[5.5]undecane (130.1 mg, 0.78 mmol), and acetic acid (187.2 mg, 3.12 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give (*Z*)-12-hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one (200 mg, yield: 78.7%), LC-MS *m*/*z*: 490 [M + H]$^+$.

**3) Synthesis of (*S*,*Z*)-12-hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one or enantiomer and (*R*,*Z*)-12-hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one or enantiomer**

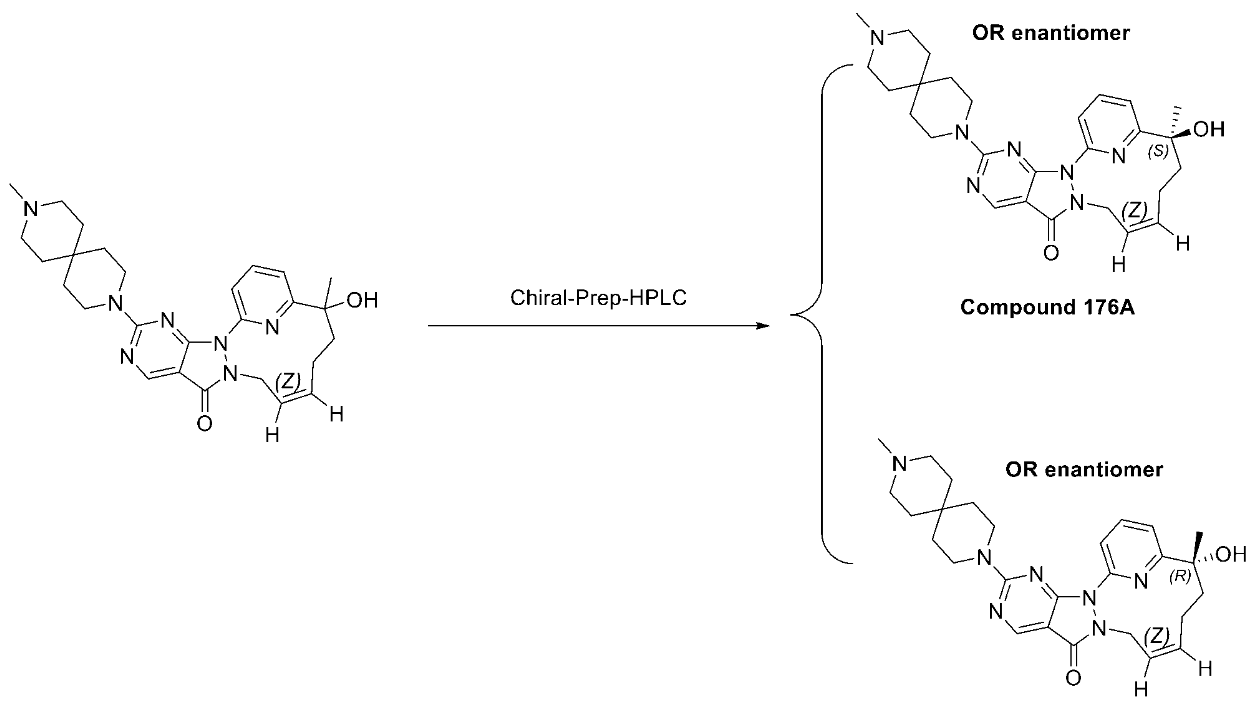


OR enantiomer

**Compound 176A**

OR enantiomer

**Compound 176B**

(*Z*)-12-Hydroxy-12-methyl-2-(9-methyl-3,9-diazaspiro[5.5]undecan-3-yl)-7,10,11,12-tetrahydro-5*H*-13,17-(azeno)pyrimido[4',5':3,4]pyrazolo[1,2-*a*][1,2]diazacyclotridecin-5-one (200 mg, 0.41 mmol) was purified by preparative chiral high performance liquid chromatography (chromatography conditions: system: Waters SFC 150; column: DAICELCHIRALCEL®AD 250 × 30 mm 10 μm; mobile phase A: supercritical $CO_2$; mobile phase B: MeOH (+ 0.1% 7.0 mol/L ammonia in MEOH); A:B: 50:50; detection wavelength: 214 nm; flow rate: 120 mL/min; column temperature: RT; column pressure: 100 bar) to give two isomers: **compound 176A** and **compound 176B:**

**Compound 176A: Peak 1 (peak time: 2.796 min): (*S,Z*)-12-hydroxy-12-methyl-2-(9-methyl-3,9-diazaspiro[5.5] undecan-3-yl)-7,10,11,12-tetrahydro-5*H*-13,17-(azeno)pyrimido[4',5':3,4]pyrazolo[1,2-*a*][1,2]diazacyclotridecin-5-one or enantiomer** (52.92 mg, yield: 29.5%), LC-MS *m/z:* 490 [M + H]$^+$;
$^1$H NMR (400 MHz, MeOD-d4): δ 8.74 (s, 1H), 7.94 (t, *J* = 7.6 Hz, 1H), 7.73 (d, *J* = 8.4 Hz, 1H), 7.55 (d, *J* = 7.6 Hz, 1H), 5.53-5.40 (m, 2H), 4.53 (d, *J* = 6.8 Hz, 2H), 3.92 (s, 4H), 2.59 (s, 4H), 2.38 (s, 3H), 2.27-2.08 (m, 2H), 2.02-1.86 (m, 2H), 1.67(s, 7H), 1.61-1.52 (m, 4H).

**Compound 176B: Peak 2 (peak time: 6.63 min):** (*R,Z*)-12-hydroxy-12-methyl-2-(9-methyl-3,9-diazaspiro[5.5] undecan-3-yl)-7,10,11,12-tetrahydro-5*H*-13,17-(azeno)pyrimido[4',5':3,4]pyrazolo[1,2-*a*][1,2]diazacyclotridecin-5-one or enantiomer (28.94 mg, yield: 14.4%), LC-MS m/z: 490 [M + H]$^+$;

$^1$H NMR (400 MHz, MeOD-d4): δ 8.74 (s, 1H), 7.94 (t, *J* = 8.0 Hz, 1H), 7.73 (d, *J* = 8.0 Hz, 1H), 7.55 (d, *J* = 7.6 Hz, 1H), 5.53-5.40 (m, 2H), 4.53 (d, *J* = 6.8 Hz, 2H), 3.91 (s, 4H), 2.51 (s, 4H), 2.32 (d, *J* = 10.0 Hz, 3H), 2.24-2.07 (m, 2H), 2.02-1.86 (m, 2H), 1.67(s, 3H), 1.64(s, 4H), 1.55 (s, 4H).

**Example 20: Synthesis of (*S,Z*)-12-hydroxy-12-methyl-2-(4-(4-methylpiperazin-1-yl)piperidin-1-yl)-7,10,11,12-tetrahydro-5*H*-13,17-(azeno)pyrimido[4',5':3,4]pyrazolo[1,2-*a*][1,2]diazacyclotridecin-5-one or Enantiomer and (*R,Z*)-12-hydroxy-12-methyl-2-(4-(4-methylpiperazin-1-yl)piperidin-1-yl)-7,10,11,12-tetrahydro-5*H* 13,17-(azeno) pyrimido[4',5':3,4]pyrazolo[1,2-*a*][1,2]diazacyclotridecin-5-one or Enantiomer (Compounds 177A and B)**

**1) Synthesis of (*Z*)-12-hydroxy-12-methyl-2-(4-(4-methylpiperazin-1-yl)piperidin-1-yl)-7,10,11,12-tetrahydro-5*H*-13,17-(azeno)pyrimido[4',5':3,4]pyrazolo[1,2-*a*][1,2]diazacyclotridecin-5-one**

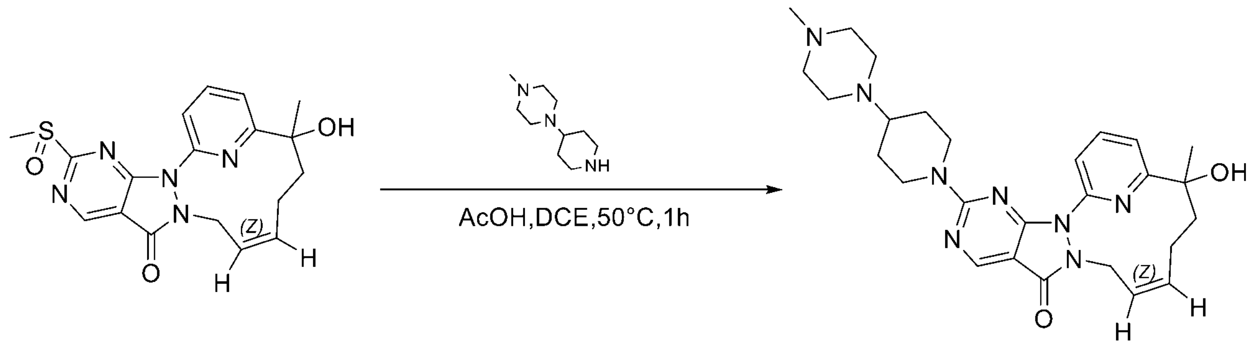


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (200 mg, 0.52 mmol), 1-methyl-4-(piperidin-4-yl)piperazine (142.8 mg, 0.78 mmol), and ethanol (187.2 mg, 3.12 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN$:$H_2O$ (0.1% $NH_3$.$H_2O$) = 5%-95% elution) to give (*Z*)-12-hydroxy-12-methyl-2-(4-(4-methylpiperazin-1-yl)piperidin-1-yl)-7,10,11,12-tetrahydro-5*H*-13,17-(azeno)pyrimido [4',5':3,4]pyrazolo[1,2-*a*][1,2]diazacyclotridecin-5-one (210 mg, yield: 80.2%), LC-MS *m/z:* 505 [M + H]$^+$.

**2) Synthesis of (*S,Z*)-12-hydroxy-12-methyl-2-(4-(4-methylpiperazin-1-yl)piperidin-1-yl)-7,10,11,12-tetrahydro-5*H*-13,17-(azeno)pyrimido[4',5':3,4]pyrazolo[1,2-*a*][1,2]diazacyclotridecin-5-one or enantiomer and (*R,Z*)-12-hydroxy-12-methyl-2-(4-(4-methylpiperazin-1-yl)piperidin-1-yl)-7,10,11,12-tetrahydro-5*H*-13,17-(azeno) pyrimido[4',5':3,4]pyrazolo[1,2-*a*][1,2]diazacyclotridecin-5-one or enantiomer**

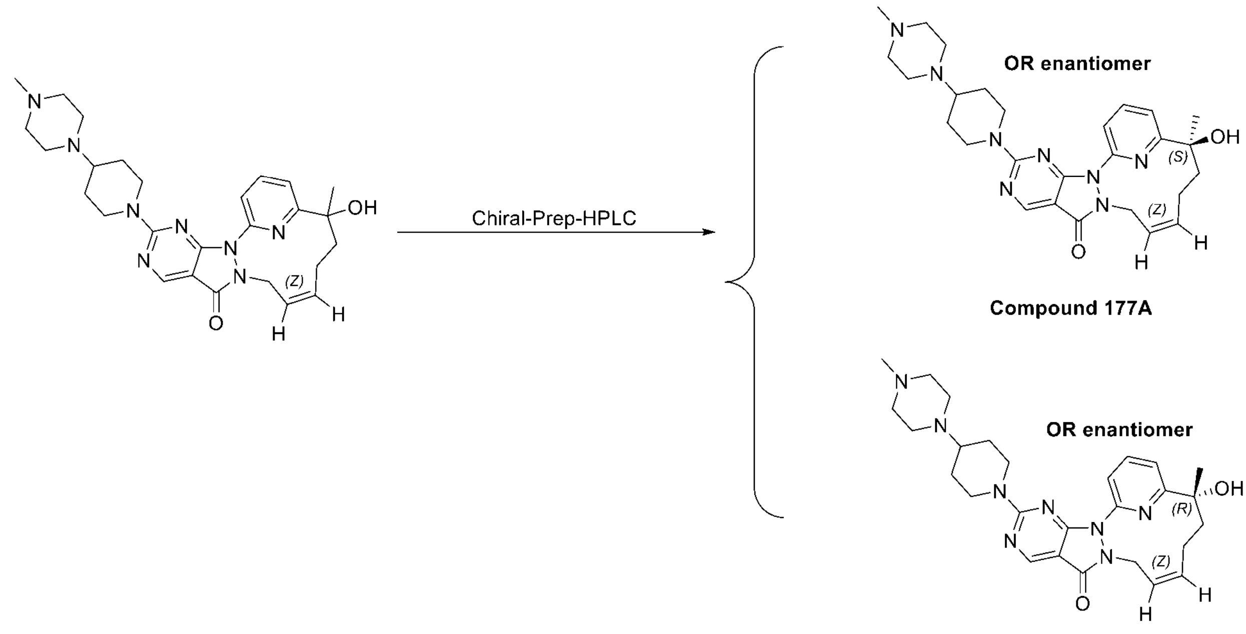


Compound 177A

Compound 177B

(*Z*)-12-Hydroxy-12-methyl-2-(4-(4-methylpiperazin-1-yl)piperidin-1-yl)-7,10,11,12-tetrahydro-5*H*-13,17-(azeno)pyrimido[4',5':3,4]pyrazolo[1,2-*a*][1,2]diazacyclotridecin-5-one (210 mg, 0.42 mmol) was purified by preparative chiral high performance liquid chromatography (chromatography conditions: system: Waters SFC 150; column: DAICELCHIRALCEL®AD 250 × 30 mm 10 μm; mobile phase A: supercritical $CO_2$; mobile phase B: MeOH (+ 0.1% 7.0 mol/L ammonia in MeOH); A:B: 50:50; detection wavelength: 214 nm; flow rate: 140 mL/min; column temperature: RT; column pressure: 100 bar) to give two isomers: **compound 177A** and **compound 177B:**

**Compound 177A:** Peak 1 **(peak time: 1.556 min):** (*S,Z*)-12-hydroxy-12-methyl-2-(4-(4-methylpiperazin-1-yl)piperidin-1-yl)-7,10,11,12-tetrahydro-5*H*-13,17-(azeno)pyrimido[4',5':3,4]pyrazolo[1,2-*a*][1,2]diazacyclotridecin-5-one or enantiomer (41.63 mg, yield: 19.8%), LC-MS *m/z:* 505 $[M + H]^+$;
$^1$H NMR (400 MHz, MeOD-*d*4): δ 8.75 (s, 1H), 7.94 (t, *J* = 8.0 Hz, 1H), 7.73 (d, *J* = 8.0 Hz, 1H), 7.55 (d, *J* = 8.0 Hz, 1H), 5.54-5.39 (m, 2H), 4.94 (s, 2H), 4.54 (d, *J* = 6.8 Hz, 2H), 2.99 (t, *J* = 12.4 Hz, 2H), 2.80-2.34 (m, 9H), 2.28 (s, 3H), 2.22-2.07 (m, 2H), 2.04-1.94 (m, 3H), 1.93-1.84 (m, 1H), 1.67 (s, 3H), 1.52-1.37 (m, 2H).

**Compound 177B: Peak 2 (peak time: 5.082 min):** (*R,Z*)-12-hydroxy-12-methyl-2-(4-(4-methylpiperazin-1-yl)piperidin-1-yl)-7,10,11,12-tetrahydro-5H-13,17-(azeno)pyrimido[4',5':3,4]pyrazolo[1,2-*a*][1,2]diazacyclotridecin-5-one or enantiomer (66.71 mg, yield: 31.7%), LC-MS *m/z:* 505 $[M + H]^+$;

$^1$H NMR (400 MHz, MeOD-*d*4): δ 8.76 (s, 1H), 7.94 (t, *J* = 8.0 Hz, 1H), 7.73 (d, *J* = 8.0 Hz, 1H), 7.55 (d, *J* = 7.6 Hz, 1H), 5.54-5.39 (m, 2H), 4.94 (s, 2H), 4.54 (d, *J* = 6.4 Hz, 2H), 2.99 (t, *J* = 12.0 Hz, 2H), 2.80-2.39 (m, 9H), 2.28 (s, 3H), 2.22-2.07 (m, 2H), 2.04-1.94 (m, 3H), 1.93-1.84 (m, 1H), 1.67 (s, 3H), 1.52-1.37 (m, 2H).

**Example 21: Synthesis of (*Z*)-12-hydroxy-12-methyl-2-((4-morpholinophenyl)amino)-7,10,11,12-tetrahydro-5*H*-13,17-(azeno)pyrimido[4',5':3,4]pyrazolo[1,2-*a*][1,2]diazacyclotridecin-5-one (Compound 178)**

**1) Synthesis of (*Z*)-12-hydroxy-12-methyl-2-(methylsulfinyl)-7,10,11,12-tetrahydro-5*H*-13,17-(azeno)pyrimido[4',5':3,4]pyrazolo[1,2-*a*][1,2]diazacyclotridecin-5-one**

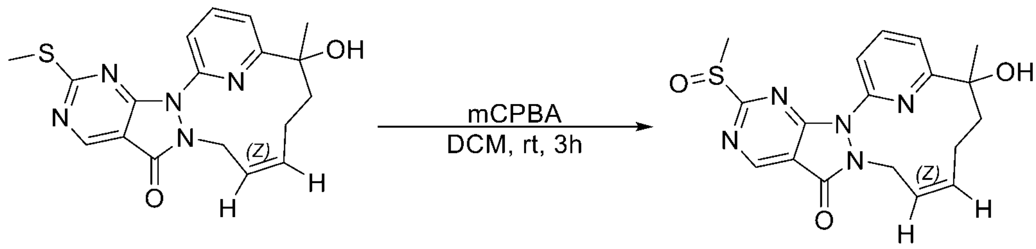


A solution of (*Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one (200 mg, 0.54 mmol) and 3-chloroperoxybenzoic acid (186 mg, 1.08 mmol) in dichloromethane (5 mL) was stirred at room temperature for 3 h. The resulting mixed reaction solution was concentrated under reduced pressure to give a crude product, which was used directly in the next step without further purification. LC-MS *m*/*z:* 386 $[M + H]^+$;

**2) Synthesis of (*Z*)-12-hydroxy-12-methyl-2-((4-morpholinophenyl)amino)-7,10,11,12-tetrahydro-5*H*-13,17-(azeno)pyrimido[4',5':3,4]pyrazolo[1,2-*a*][1,2]diazacyclotridecin-5-one**

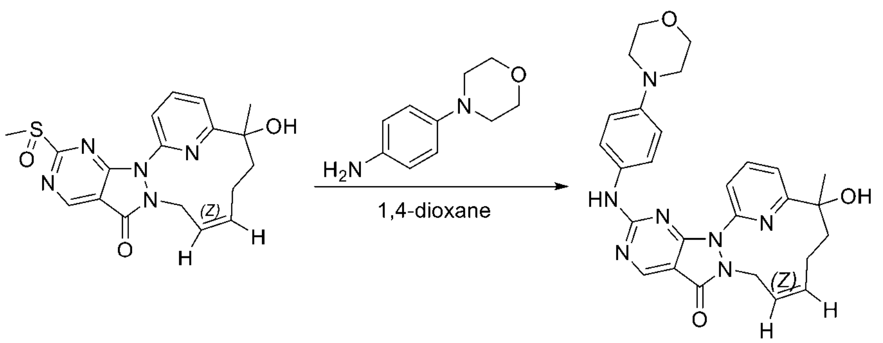


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (200 mg, 0.54 mmol) and 4-morpholinoaniline (115 mg, 0.65 mmol) in 1,4-dioxane (5 mL) was heated to 100 °C and stirred overnight. After cooling, the mixed reaction solution was filtered and concentrated under reduced pressure. The residue was purified by C18 reverse phase column chromatography ($CH_3CN:H_2O$ (0.1% $NH_4HCO_3$) = 5%-95% elution) to give (*Z*)-12-hydroxy-12-methyl-2-((4-morpholinophenyl)amino)-7,10,11,12-tetrahydro-5H-13,17-(azeno)pyrimido[4',5':3,4]pyrazolo[1,2-*a*][1,2]diazacyclotridecin-5-one (102 mg, yield: 39.4%), LC-MS *m*/*z:* 500 $[M + H]^+$;
$^1H$ NMR (400 MHz, $CDCl_3$): δ 8.85 (s, 1H), 7.89-7.80 (m, 2H), 7.50 (d, J = 8.0 Hz, 2H), 7.27-7.74 (m, 2H), 6.91 (d, J = 8.0 Hz, 2H), 5.75-5.71 (m, 1H), 4.50-4.30 (m, 2H), 3.89-3.84 (m, 4H), 3.20-3.09 (m, 4H), 2.22 - 1.91 (m, 3H), 1.80-1.70 (m, 1H), 1.68 (s, 3H).

**Example 22: Synthesis of (*Z*)-2-((4-(4-ethoxy-4-oxido-1,4-azaphosphinan-1-yl)-3-fluorophenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 180)**

**1) Synthesis of 4-ethoxy-1-(2-fluoro-4-nitrophenyl)-1,4-azaphosphinane-4-oxide**

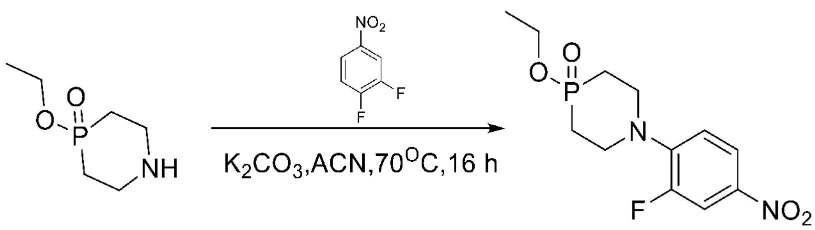

A solution of 1,2-difluoro-4-nitrobenzene (150 mg, 0.94 mmol), potassium carbonate (394 mg, 2.82 mmol), and 4-ethoxy-1,4-azaphosphinane-4-oxide (199 mg, 1.2 mmol) in acetonitrile (3 mL) was heated to 70 °C and stirred for 1 h. The resulting mixed reaction solution was concentrated under reduced pressure. The residue was purified by silica gel column chromatography (methanol:dichloromethane = 0%-10% elution) to give 4-ethoxy-1-(2-fluoro-4-nitrophenyl)-1,4-azaphosphinane-4-oxide (147 mg, yield: 52%), LC-MS *m/z:* 303 $[M + H]^+$.

**2) Synthesis of 1-(4-amino-2-fluorophenyl)-4-ethoxy-1,4-azaphosphinane-4-oxide**

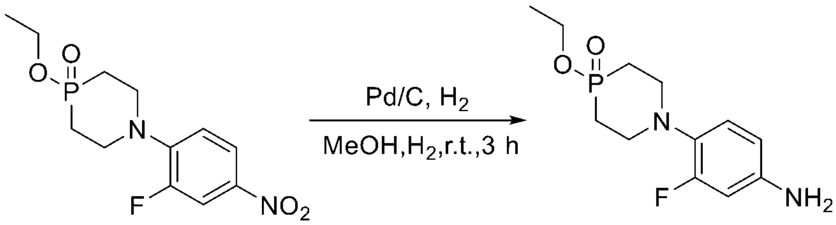


A solution of 4-ethoxy-1-(2-fluoro-4-nitrophenyl)-1,4-azaphosphinane-4-oxide (127 mg, 0.42 mmol) and Pd/C (60 mg) in methanol (3 mL) was stirred at room temperature for 3 h under a hydrogen atmosphere (1 atm). The mixed reaction solution was filtered, and the filter cake was washed with methanol (3 × 20 mL). The filtrate was collected and concentrated under pressure to give 1-(4-amino-2-fluorophenyl)-4-ethoxy-1,4-azaphosphinane-4-oxide (90 mg, yield: 78%), LC-MS *m/z*: 273 $[M + H]^+$.

**3) Synthesis of (*Z*)-2-((4-(4-ethoxy-4-oxido-1,4-azaphosphinan-1-yl)-3-fluorophenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

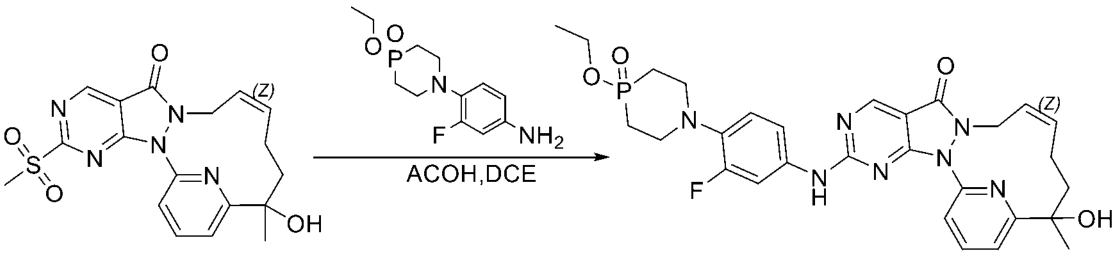


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (80 mg, 0.20 mmol), 1-(4-amino-2-fluorophenyl)-4-ethoxy-1,4-azaphosphinane-4-oxide (81 mg, 0.30 mmol), and acetic acid (72 mg, 1.2 mmol) in 1,2-dichloroethane (2 mL) was heated to 50 °C and stirred for 16 h. The resulting mixed reaction solution was concentrated under reduced pressure. The residue was purified by C18 reverse phase column chromatography ($CH_3CN:H_2O$ (0.1% FA) = 30%-70% elution) to give (*Z*)-2-((4-(4-ethoxy-4-oxido-1,4-azaphosphinan-1-yl)-3-fluorophenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (25 mg, yield: 21%), LC-MS *m/z:* 594 $[M + H]^+$;

$^1H$ NMR (400 MHz, MeOD-d4) δ 8.82 (s, 1H), 7.97 (t, J = 7.9 Hz, 1H), 7.81 - 7.71 (m, 2H), 7.61 (d, J = 7.6 Hz, 1H), 7.25 (dd, J = 8.6, 1.7 Hz, 1H), 7.03 (t, J = 9.2 Hz, 1H), 5.51 - 5.37 (m, 2H), 4.61 (d, J = 6.8 Hz, 2H), 4.15 (p, J = 7.1 Hz, 2H), 3.60 - 3.46 (m, 2H), 3.30 - 3.23 (m, 2H), 2.11 (dd, J = 22.3, 9.7 Hz, 6H), 2.04 - 1.86 (m, 2H), 1.69 (s, 3H), 1.39 (t, J = 7.0 Hz, 3H).

**Example 23: Synthesis of (*Z*)-2-((4-(4-ethoxy-4-oxido-1,4-azaphosphinan-1-yl)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 181)**

**1) Synthesis of 4-ethoxy-1-(4-nitrophenyl)-1,4-azaphosphinane-4-oxide**

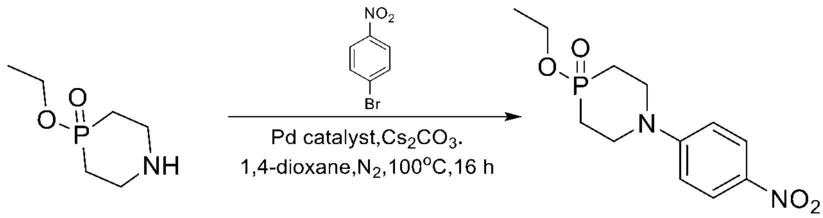


A solution of 1-bromo-4-nitrobenzene (300 mg, 1.49 mmol), 4-ethoxy-1,4-azaphosphinane-4-oxide (364 mg, 2.24 mmol), (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (125 mg, 0.15 mmol), and cesium carbonate (1450 mg, 4.47 mmol) in 1,4-dioxane (5 mL) was heated to 100 °C and stirred overnight under a nitrogen atmosphere. After cooling, the mixed reaction solution was concentrated under reduced pressure. The residue was purified by silica gel column chromatography (methanol:dichloromethane = 0%-10% elution) to give 4-ethoxy-1-(4-nitrophenyl)-1,4-azaphosphinane-4-oxide (409 mg, yield: 96%), LC-MS *m*/*z:* 285 [M + H]$^+$.

**2) Synthesis of 1-(4-aminophenyl)-4-ethoxy-1,4-azaphosphinane-4-oxide**

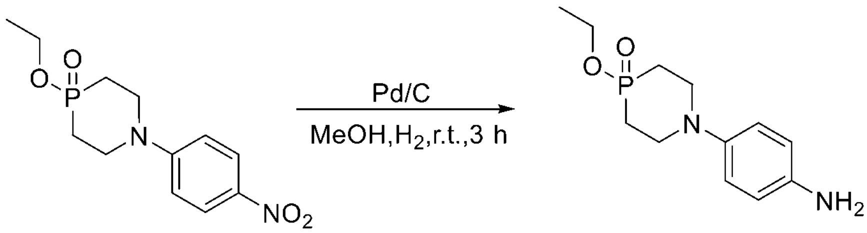


A solution of 4-ethoxy-1-(4-nitrophenyl)-1,4-azaphosphinane-4-oxide (300 mg, 1.05 mmol) and Pd/C (150 mg) in methanol (6 mL) was stirred at room temperature for 3 h under a hydrogen atmosphere (1 atm). The mixed reaction solution was filtered, and the filter cake was washed with methanol (3 × 20 mL). The filtrate was collected and concentrated under reduced pressure to give 1-(4-aminophenyl)-4-ethoxy-1,4-azaphosphinane-4-oxide (247 mg, yield: 92%), LC-MS *m*/*z:* 255 [M + H]$^+$.

**3) Synthesis of (*Z*)-2-((4-(4-ethoxy-4-oxido-1,4-azaphosphinan-1-yl)phenyl)amino)-12-hydroxy-12-methyl-7,10,11,12-tetrahydro-5H-13,17-(azeno)pyrimido[4',5':3,4]pyrazolo[1,2-*a*][1,2]diazacyclotridecin-5-one**

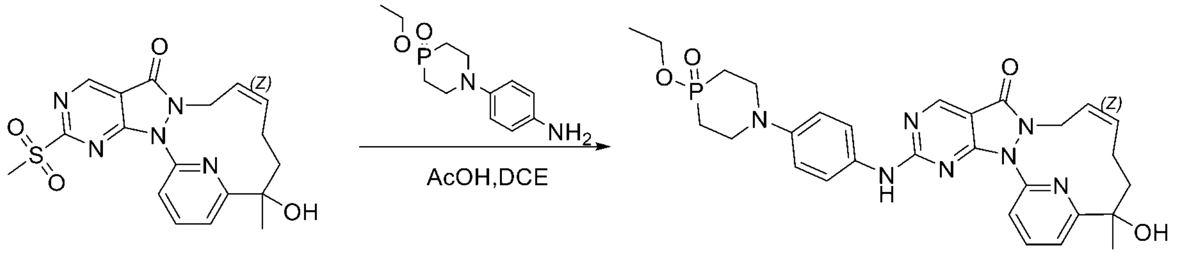


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol), 1-(4-aminophenyl)-4-ethoxy-1,4-azaphosphinane-4-oxide (95 mg, 0.38 mmol), and acetic acid (90 mg, 1.50 mmol) in 1,2-dichloroethane (3 mL) was heated to 50 °C and stirred for 16 h. The resulting mixed reaction solution was concentrated under reduced pressure. The residue was purified by C18 reverse phase column chromatography ($CH_3CN$:$H_2O$ (0.1% $NH_4HCO_3$) = 30%-70% elution) to give (*Z*)-2-((4-(4-ethoxy-4-oxido-1,4-azaphosphinan-1-yl)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (35 mg, yield: 24%), LC-MS *m*/*z:* 576 [M + H]$^+$;

$^1$H NMR (400 MHz, MeOD-d4) δ 8.78 (s, 1H), 7.94 (t, J = 7.9 Hz, 1H), 7.78 (d, J = 8.0 Hz, 1H), 7.55 (dd, J = 10.9, 8.4

Hz, 3H), 6.95 (d, J = 9.1 Hz, 2H), 5.52 - 5.38 (m, 2H), 4.58 (d, J = 6.8 Hz, 2H), 4.14 (dq, J = 14.2, 7.1 Hz, 2H), 4.03 - 3.88 (m, 2H), 3.54 - 3.43 (m, 2H), 2.17 (s, 2H), 2.03 - 1.84 (m, 6H), 1.67 (s, 3H), 1.38 (t, J = 7.0 Hz, 3H).

**Example 24: Synthesis of (*R*,*Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one and (*S*,*Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compounds 182A and B)**

**1) Synthesis of 4-ethoxy-1-(2-methyl-4-nitrophenyl)-1,4-azaphosphinane-4-oxide**

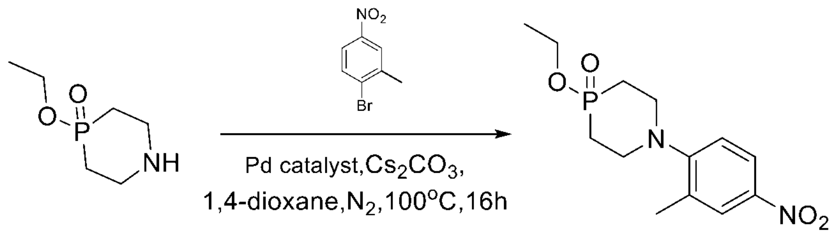


A solution of 1-bromo-2-methyl-4-nitrobenzene (300 mg, 1.39 mmol), 4-ethoxy-1,4-azaphosphinane 4-oxide (340 mg, 2.08 mmol), (SP-4-1)-[1,3-bis[2,6-bis(1-ethylpropyl)phenyl]-4,5-dichloro-1,3-dihydro-2*H*-imidazol-2-ylidene] dichloro(2-methylpyridine)palladium (117 mg, 0.14 mmol), and cesium carbonate (1350 mg, 4.17 mmol) in 1,4-dioxane (5 mL) was heated to 100 °C and stirred overnight under a nitrogen atmosphere. After cooling, the mixed reaction solution was concentrated under reduced pressure. The residue was purified by silica gel column chromatography (methanol:dichloromethane = 0%-20% elution) to give 4-ethoxy-1-(2-methyl-4-nitrophenyl)-1,4-azaphosphinane-4-oxide (400 mg, yield: 96%), LC-MS *m/z:* 299 [M + H]$^+$.

**2) Synthesis of 1-(4-amino-2-methylphenyl)-4-ethoxy-1,4-azaphosphinane-4-oxide**

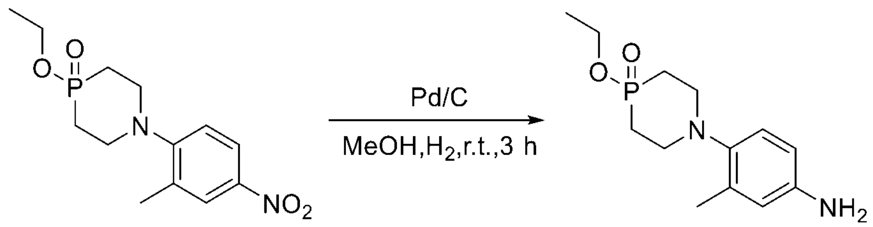


A solution of 4-ethoxy-1-(2-methyl-4-nitrophenyl)-1,4-azaphosphinane-4-oxide (400 mg, 1.33 mol) and Pd/C (200 mg) in methanol (6 mL) was stirred at room temperature for 3 h under a hydrogen atmosphere. The mixed reaction solution was filtered, the filter cake was washed with methanol (3 × 20 mL), and the filtrate was collected and concentrated under reduced pressure to give 1-(4-amino-2-methylphenyl)-4-ethoxy-1,4-azaphosphinane-4-oxide (334 mg, yield: 92%), LC-MS *m/z:* 269 [M + H]$^+$.

**3) Synthesis of (*Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

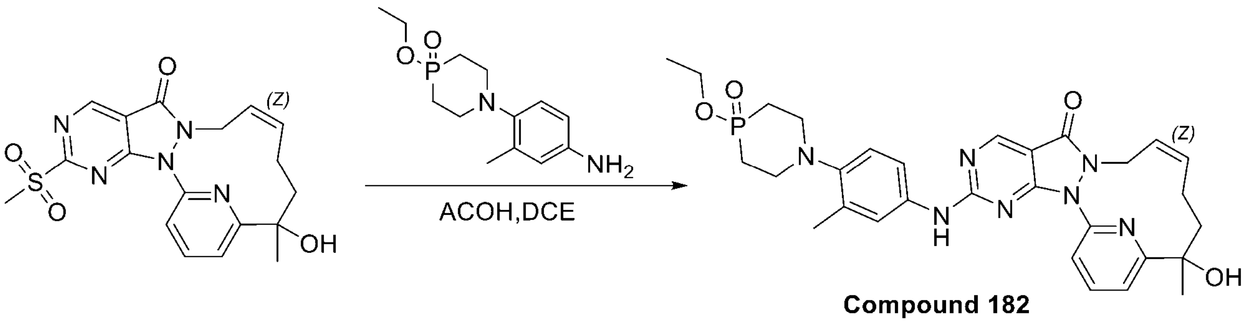


Compound 182

A solution of (Z)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol), 1-(4-amino-2-methylphenyl)-4-ethoxy-1,4-azaphosphinane-4-oxide (100 mg, 0.37 mmol), and acetic acid (90 mg, 1.50 mmol) in 1,2-dichloroethane (3 mL) was heated to 50 °C and stirred for 16 h. The resulting mixed reaction solution was concentrated under reduced pressure. The residue was purified by C18 reverse phase column chromatography ($CH_3CN$:$H_2O$ (0.1% FA) = 30%-70% elution) to give (*Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (35 mg, yield: 24%), LC-MS *m/z:* 590 $[M + H]^+$;
$^1$H NMR (400 MHz, MeOD-d4) δ 8.79 (s, 1H), 7.97 (t, J = 7.9 Hz, 1H), 7.81 (d, J = 8.0 Hz, 1H), 7.61 (t, J = 8.9 Hz, 2H), 7.38 (dd, J = 8.6, 2.4 Hz, 1H), 7.02 (d, J = 8.6 Hz, 1H), 5.53 - 5.41 (m, 2H), 4.58 (d, J = 6.8 Hz, 2H), 4.20 - 4.11 (m, 2H), 3.29 - 3.19 (m, 2H), 3.18 - 3.09 (m, 2H), 2.31 (s, 3H), 2.21 - 2.05 (m, 6H), 1.99 (dd, J = 16.8, 8.5 Hz, 1H), 1.93 - 1.86 (m, 1H), 1.68 (s, 3H), 1.40 (t, J = 7.0 Hz, 3H).

**4) Synthesis of (*R,Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer and (*S,Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer**

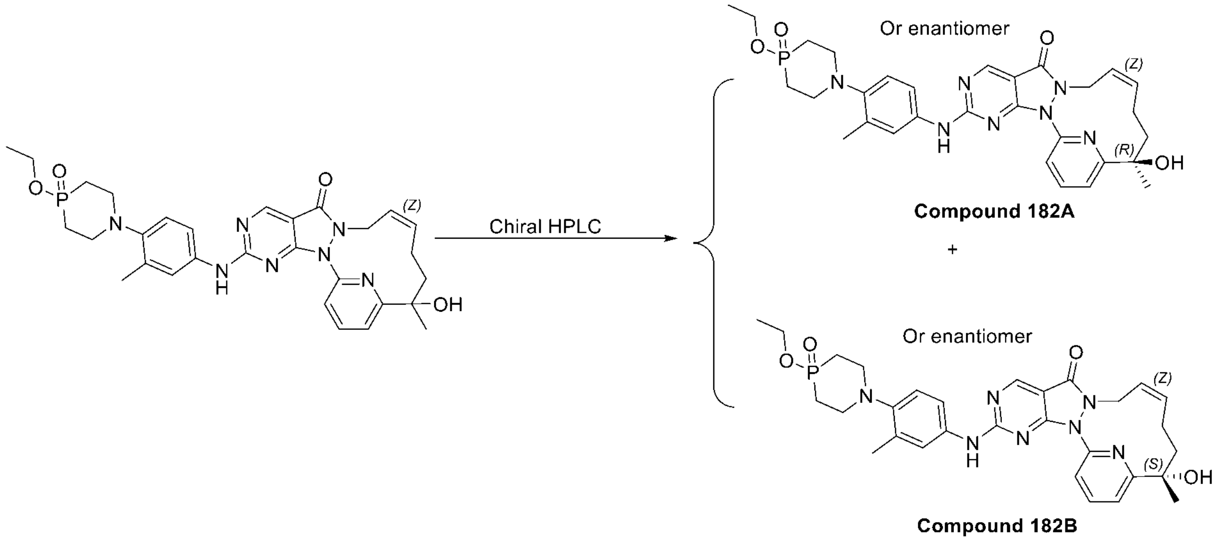


Compound 182A

Compound 182B

(*Z*)-2-((4-(4-Ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (20 mg, 0.03 mmol) was purified by preparative chiral high performance liquid chromatography (chromatography conditions: system: Waters SFC 80; column: DAICELCHIRALPAK®AD 250 × 30 mm 10 μm; mobile phase A: supercritical $CO_2$; mobile phase B: EtOH (+ 0.1% 7.0 mol/L ammonia in MeOH); gradient: A:B: 50:50; detection wavelength: 214 nm; flow rate: 70 mL/min; column temperature: room temperature; column pressure: 100 bar) to give two isomers: **compound 182A** and **compound 182B:**

**Compound 182A:** Peak 1 **(peak time: 3.817 min):** (*R,Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer (3 mg, yield: 15%).

$^1$H NMR (400 MHz, MeOD) δ 8.81 (s, 1H), 7.98 (t, J = 7.8 Hz, 1H), 7.83 (d, J = 8.1 Hz, 1H), 7.61 (t, J = 10.3 Hz, 2H), 7.40 (d, J = 6.0 Hz, 1H), 7.03 (d, J = 8.6 Hz, 1H), 5.48 (dd, J = 13.5, 6.8 Hz, 2H), 4.60 (d, J = 6.6 Hz, 2H), 4.19 - 4.14 (m, 2H), 3.23 (s, 2H), 3.18 - 3.09 (m, 2H), 2.32 (s, 3H), 2.14 - 2.04 (m, 6H), 2.00 (s, 1H), 1.89 (s, 1H), 1.68 (s, 3H), 1.40 (t, J = 7.0 Hz, 3H).

**Compound 182B:** Peak 2 **(peak time: 5.219 min):** (*S,Z*)-2-((4-(4-ethoxy-4-oxido-1,4-azaphosphinan-1-yl)-3-methylphenyl)amino)-12-hydroxy-12-methyl-7,10,11,12-tetrahydro-5*H*-13,17-(azeno)pyrimido [4',5':3,4]pyrazolo[1,2-*a*][1,2]diazacyclotridecin-5-one or enantiomer (2.5 mg, yield: 12.5%).

$^1$H NMR (400 MHz, MeOD) δ 8.81 (s, 1H), 7.98 (t, J = 7.9 Hz, 1H), 7.83 (d, J = 8.1 Hz, 1H), 7.61 (t, J = 10.3 Hz, 2H), 7.40 (d, J = 8.6 Hz, 1H), 7.03 (d, J = 8.6 Hz, 1H), 5.53 - 5.40 (m, 2H), 4.60 (d, J = 6.8 Hz, 2H), 4.16 (dd, J = 14.5, 7.3 Hz, 2H), 3.23 (s, 2H), 3.16 (dd, J = 13.1, 4.8 Hz, 2H), 2.32 (s, 3H), 2.20 - 2.06 (m, 6H), 2.01 (d, J = 13.1 Hz, 1H), 1.90 (s, 1H), 1.68 (s, 3H), 1.40 (t, J = 7.0 Hz, 3H).

**Example 25: Synthesis of (*R,Z*)-12-hydroxy-12-methyl-2-((3-methyl-4-(((*R*)-1-methylpyrrolidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound 183)**

**1) Synthesis of (*R*)-1-methyl-3-(2-methyl-4-nitrophenoxy)pyrrolidine**

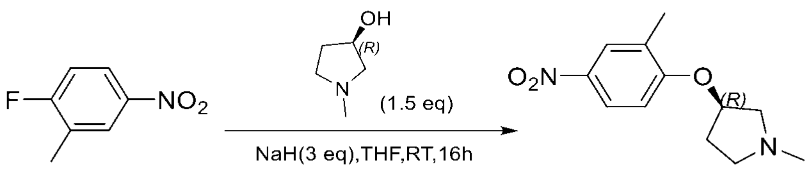


Sodium hydride (0.775 g, 19.35 mmol, content: 60%) was added to a solution of (*R*)-1-methylpyrrolidin-3-ol (0.98 g, 9.68 mmol) in tetrahydrofuran (10 mL) at 0 °C, and the mixture was stirred at 0 °C for 1 h. Then, 1-fluoro-2-methyl-4-nitrobenzene (1 g, 6.45 mmol) was added to the above mixture. The resulting mixed reaction solution was stirred overnight at room temperature. The mixed reaction solution was quenched with a saturated aqueous ammonium chloride solution (50 mL) and extracted with ethyl acetate (3 × 100 mL) at 0 °C. The combined organic phases were washed with saturated brine (1 × 50 mL), dried over anhydrous sodium sulfate, filtered, and concentrated under reduced pressure. The residue was purified by silica gel column chromatography (MeOH:DCM = 3%-8% elution) to give (*R*)-1-methyl-3-(2-methyl-4-nitrophenoxy)pyrrolidine (240 mg, yield: 15.8%), LC-MS *m/z:* 237 [M + H]$^+$.

**2) Synthesis of (*R*)-3-methyl-4-((1-methylpyrrolidin-3-yl)oxy)aniline**

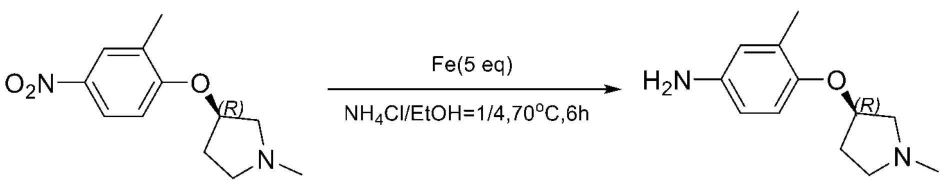


A saturated aqueous ammonium chloride solution (0.5 mL) was added to a solution of (*R*)-1-methyl-3-(2-methyl-4-nitrophenoxy)pyrrolidine (230 mg, 0.97 mmol) and Fe (273 mg, 4.87 mmol) in ethanol (2 mL) at room temperature. The resulting mixed reaction solution was heated to 70 °C and stirred for 6 h. After cooling, the mixed reaction solution was diluted with water and extracted with ethyl acetate (3 × 50 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 0%-10% elution) to give (*R*)-3-methyl-4-((1-methylpyrrolidin-3-yl)oxy)aniline (140 mg, yield: 69.7%), LC-MS *m/z*: 207 [M + H]$^+$.

**3) Synthesis of (*R,Z*)-12-hydroxy-12-methyl-2-((3-methyl-4-(((*R*)-1-methylpyrrolidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

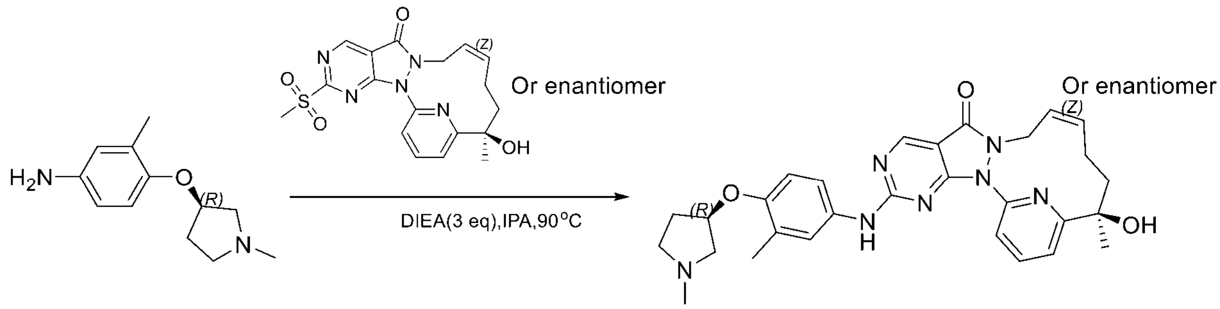


*N,N*-Diisopropylethylamine (225 mg, 1.75 mmol) was added to a solution of (*R*)-3-methyl-4-((1-methylpyrrolidin-3-yl)oxy)aniline (120 mg, 0.58 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (280 mg, 0.70 mmol) in isopropanol (2 mL). The resulting mixed reaction solution was heated to 90 °C and stirred for 6 h. The resulting mixed reaction solution was extracted with ethyl acetate (3 × 50 mL). The combined organic phases were washed with saturated brine (1 × 10 mL), dried over anhydrous sodium sulfate, filtered, and concentrated under reduced pressure. The residue was purified by C18 reverse phase column chromatography (MeCN/$H_2O$ (0.1% FA) = 30%-60% elution) to give (*R,Z*)-12-hydroxy-12-methyl-2-((3-methyl-4-(((*R*)-1-methylpyrrolidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (70 mg, yield: 22.8%), LC-MS *m/z:* 528 $[M + H]^+$;

$^1$H NMR (400 MHz, MeOD-$d_4$): 8.78 (s, 1H), 7.94 (t, J = 8.0 Hz, 1H), 7.81 (d, *J* = 8.0 Hz, 1H), 7.58 (d, J = 8.0 Hz, 2H), 7.33 (d, J = 8.0 Hz,1H), 6.75 (d, J = 8.0 Hz, 1H),5.53-5.44 (m, 2H),4.88(s, 1H),4.58 (d, J = 4.0 Hz,2H),2.94-2.90(m,1H),2.85(t, J=8.0 Hz,2H),2.58-2.52(m, 1H),2.41(s, 3H),2.39-2.32(m, 1H),2.19-2.13(m, 5H), 2.02-1.89(m, 3H), 1.68(s, 3H).

**Example 26: Synthesis of (*R, Z*)-N-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)phenyl)-4-methylpiperazine-1-carboxamide or Enantiomer (Compound 184)**

**1) Synthesis of 4-methyl-*N*-(4-nitrophenyl)piperazine-1-carboxamide**

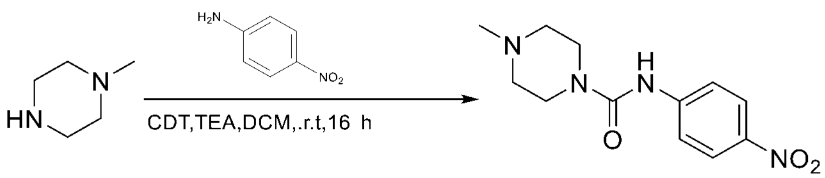


A solution of 4-nitroaniline (0.3 g, 2.17 mmol), *N,N'*-carbonylbis(1,2,4-triazole) (0.535 g, 3.26 mmol), and triethylamine (0.65 g, 6.52 mmol) in dichloromethane (4 mL) was stirred at room temperature for 1 h under an argon atmosphere. Then, 1-methylpiperazine (0.326 g, 3.26 mmol) was added to the above mixed reaction solution. The resulting mixed reaction solution was stirred overnight at room temperature. The mixed reaction solution was diluted with water and extracted with ethyl acetate (3 × 100 mL). The combined organic phases were washed with saturated brine (1 × 50 mL), dried over anhydrous sodium sulfate, filtered, and concentrated under reduced pressure. The residue was purified by silica gel column chromatography (MeOH:DCM = 8%-10% elution) to give 4-methyl-*N*-(4-nitrophenyl)piperazine-1-carboxamide (500 mg, yield: 87%), LC-MS *m/z*: 265 $[M + H]^+$;

**2) Synthesis of *N*-(4-aminophenyl)-4-methylpiperazine-1-carboxamide**

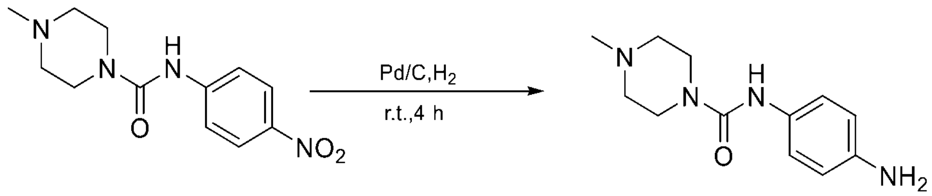


A solution of 4-methyl-*N*-(4-nitrophenyl)piperazine-1-carboxamide (300 mg, 1.15 mmol) and Pd/C (181 mg, 1.70 mmol) in methanol (3 mL) was stirred at room temperature for 1 h under a hydrogen atmosphere. The mixed reaction solution was filtered, and the filter cake was washed with methanol. The filtrate was collected and concentrated under reduced pressure to give *N*-(4-aminophenyl)-4-methylpiperazine-1-carboxamide (200 mg, yield: 75.2%), LC-MS *m/z*: 235 $[M + H]^+$;

**3) Synthesis of (*R, Z*)-*N*-(4-((12-hydroxy-12-methyl-5-oxo-7,10,11,12-tetrahydro-5*H*-13,17-(azeno)pyrimido [4',5':3,4]pyrazolo[1,2-*a*][1,2]diazacyclotridecin-2-yl)amino)phenyl)-4-methylpiperazine-1-carboxamide or enantiomer**

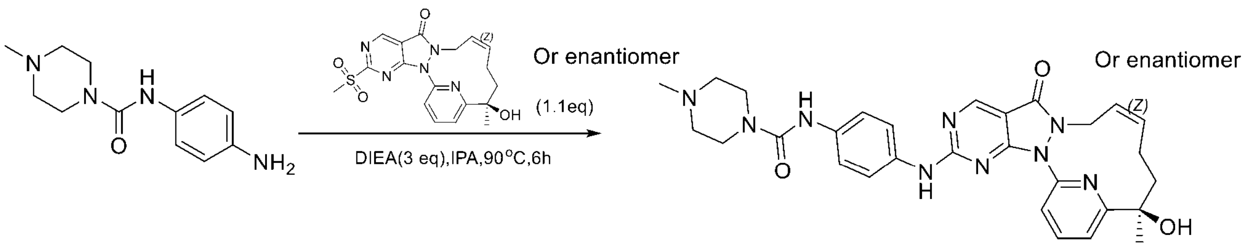


*N,N*-Diisopropylethylamine (720 mg, 2.56 mmol) was added to a solution of *N*-(4-aminophenyl)-4-methylpiperazine-1-carboxamide (200 mg, 0.85 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (378 mg, 0.94 mmol) in isopropanol (2 mL). The resulting mixed reaction solution was heated to 90 °C and stirred for 6 h. The mixed reaction solution was diluted with water and extracted with ethyl acetate (3 × 50 mL), and the combined organic phases were washed with saturated brine (1 × 10 mL), dried over anhydrous sodium sulfate, filtered, and concentrated under reduced pressure. The residue was purified by C18 reverse phase column chromatography (MeCN:$H_2O$ (0.1% FA) = 30%-60% elution) to give (*R, Z*)-*N*-(4-((12-hydroxy-12-methyl-5-oxo-7,10,11,12-tetrahydro-5*H*-13,17-(azeno)pyrimido[4',5':3,4]*pyrazolo*[1,2-*a*][1,2] diazacyclotridecin-2-yl)amino)phenyl)-4-methylpiperazine-1-carboxamide or enantiomer (150 mg, yield: 31.6%), LC-MS *m/z:* 556 $[M + H]^+$;

$^1$H NMR (400 MHz, MeOD-d4): 8.82 (s, 1H), 7.96 (t, J = 8.0 Hz, 1H), 7.80 (d, J = 8.0 Hz, 1H), 7.60 (t, J = 8.0 Hz, 3H), 7.31 (d, J = 8.0 Hz, 2H), 5.48-5.44 (m, 2H), 4.605 (d, J = 4.0 Hz, 2H), 3.64-3.58 (m, 4H),2.68-2.65 (m, 4H),2.48 (s, 3H),2.25-2.14 (m, 2H),1.99-1.92 (m, 2H),1.68 (s, 3H).

**Example 27: Synthesis of (*R,Z*)-12-hydroxy-12-methyl-2-((4-(4-methylpiperazine-1-carbonyl)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound 185)**

**1) Synthesis of (4-methylpiperazin-1-yl)(4-nitrophenyl)methanone**

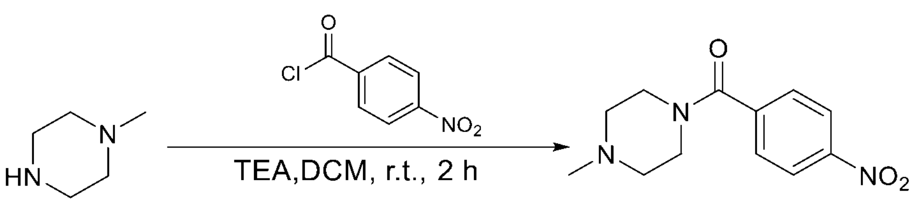


A solution of 1-methylpiperazine (2 g, 19.97 mmol), 4-nitrobenzoyl chloride (4.45 g, 23.96 mmol), and triethylamine (6.06 g, 59.91 mmol) in dichloromethane (20 mL) was stirred at room temperature for 2 h. The resulting mixed reaction solution was concentrated under reduced pressure. The residue was dissolved in *N,N*-dimethylformamide (10 mL), and the solution was purified by C18 reverse phase column chromatography (ACN:$H_2O$ = 2%-5% elution) to give (4-methylpiperazin-1-yl)(4-nitrophenyl)methanone (4 g, yield: 80.4%), LC-MS *m/z:* 250 [M + H]$^+$;

**2) Synthesis of (4-aminophenyl)(4-methylpiperazin-1-yl)methanone**

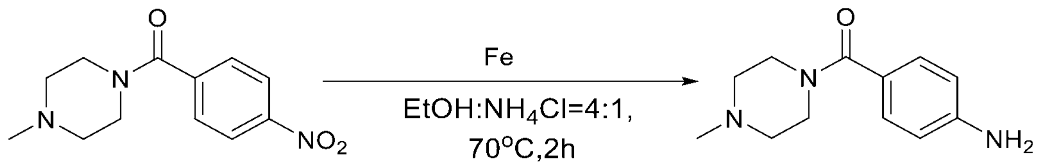


A solution of (4-methylpiperazin-1-yl)(4-nitrophenyl)methanone (4 g, 16.05 mmol) and Fe (4.48 g, 80.25 mmol) in ethanol (32 mL) and aqueous ammonium chloride solution (8 mL) was heated to 70 °C and stirred for 2 h. After cooling, the mixed reaction solution was filtered, and the filter cake was washed with methanol. The filtrate was collected and concentrated under reduced pressure to give (4-aminophenyl)(4-methylpiperazin-1-yl)methanone (1.2 g, yield: 34.1%), LC-MS *m/z:* 220 [M + H]$^+$;

**3) Synthesis of (*R,Z*)-12-hydroxy-12-methyl-2-((4-(4-methylpiperazine-1-carbonyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

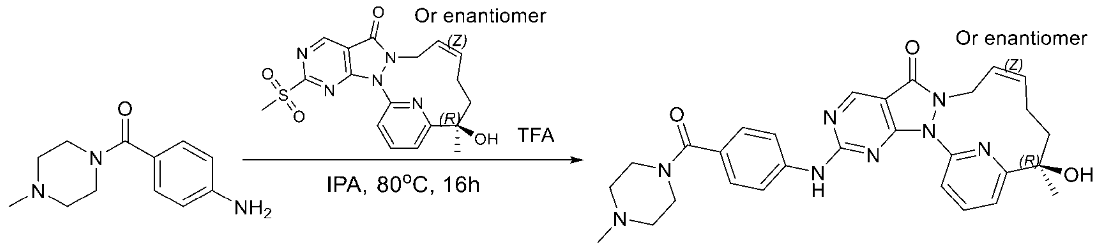


A solution of (*R,Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one or enantiomer (100 mg, 0.25 mmol), (4-aminophenyl)(4-methylpiperazin-1-yl)methanone (82.23 mg, 0.38 mmol), and trifluoroacetic acid (0.043 g, 0.38 mmol) in isopropanol (1.5 mL) was heated to 100 °C and stirred for 16 h. After cooling, the resulting mixed reaction solution was concentrated under reduced pressure. The residue was dissolved in *N,N*-dimethylformamide (1 mL), and the solution was purified by C18 reverse phase column chromatography (acetonitrile:purified water = 35%-50% elution) to give (*R,Z*)-12-hydroxy-12-methyl-2-((4-(4-methylpiperazine-1-carbonyl)phenyl)amino)-7,10,11,12-tetrahydro-5*H*-13,17-(azeno)pyrimido [4',5':3,4]pyrazolo[1,2-*a*][1,2]diazacyclotridecin-5-one or enantiomer (60 mg, yield: 44.6%), LC-MS *m/z:* 541 [M + H]$^+$;

$^1$H NMR (400 MHz, MeOD-d4) δ 8.89 (s, 1H), 7.99 (t, J = 7.9 Hz, 1H), 7.82 (dd, J = 13.6, 8.3 Hz, 3H), 7.62 (d, J = 7.6 Hz, 1H), 7.40 (d, J = 8.6 Hz, 2H), 5.44 (dd, J = 14.2, 7.1 Hz, 2H), 4.64 (d, J = 6.7 Hz, 2H), 3.66 (s, 4H), 2.50 (s, 4H), 2.34 (s, 3H), 2.17 (s, 2H), 2.06 - 1.85 (m, 2H), 1.69 (s, 3H).

**Example 28: Synthesis of (*R,Z*)-12-hydroxy-12-methyl-2-((3-methyl-4-(((*S*)-1-methylpyrrolidin-3-yl)oxy)phenyl) amino)-7,10,11,12-tetrahydro-5*H*-13,17-(azeno)pyrimido[4',5':3,4]pyrazolo[1,2-*a*][1,2]diazacyclotridecin-5-one or Isomer (Compound 186)**

**1) Synthesis of (*S*)-1-methyl-3-(2-methyl-4-nitrophenoxy)pyrrolidine**

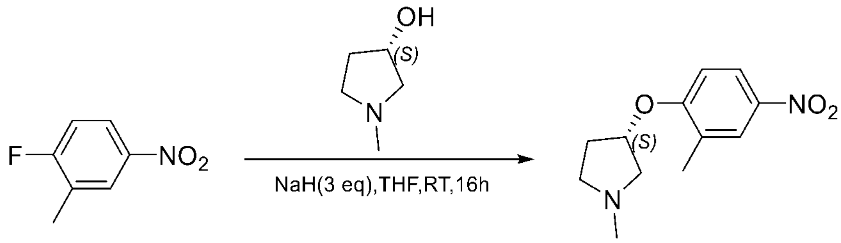


(*S*)-1-Methylpyrrolidin-3-ol (0.98 g, 9.68 mmol) was added to a solution of sodium hydride (0.465 g, 19.35 mmol, 60% content) in tetrahydrofuran (10 mL), and the mixture was stirred at 0 °C for 1 h. Then, 1-fluoro-2-methyl-4-nitrobenzene (1 g, 6.45 mmol) was added to the above mixed reaction solution, and the mixed reaction solution was stirred at room temperature overnight. At 0 °C, the mixed reaction solution was quenched with a saturated aqueous ammonium chloride solution (50 mL) and extracted with ethyl acetate (3 × 100 mL). The combined organic phases were washed with saturated brine (1 × 50 mL), dried over anhydrous sodium sulfate, filtered, and concentrated under reduced pressure. The residue was purified by silica gel column chromatography (methanol:dichloromethane = 3%-8% elution) to give (S)-1-methyl-3-(2-methyl-4-nitrophenoxy)pyrrolidine (740 mg, yield: 47.1%), LC-MS *m/z*: 237$[M + H]^+$;

**2) Synthesis of (*S*)-3-methyl-4-((1-methylpyrrolidin-3-yl)oxy)aniline**

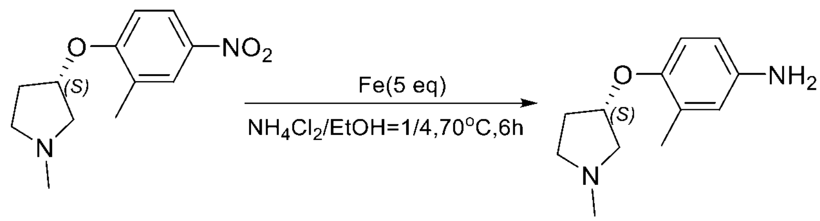


A solution of (*S*)-1-methyl-3-(2-methyl-4-nitrophenoxy)pyrrolidine (730 mg, 3.09 mmol) and Fe (866 mg, 15.47 mmol) in ethanol (6 mL) and saturated aqueous ammonium chloride solution (1.5 mL) was heated to 70 °C and stirred for 6 h. After cooling, the mixed reaction solution was diluted with water and extracted with ethyl acetate (3 × 50 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 0%-10% elution) to give (S)-3-methyl-4-((1-methylpyrrolidin-3-yl)oxy)aniline (400 mg, yield: 62.5%), LC-MS *m/z*: 207$[M + H]^+$;

**3) Synthesis of (*R,Z*)-12-hydroxy-12-methyl-2-((3-methyl-4-(((*S*)-1-methylpyrrolidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or isomer**

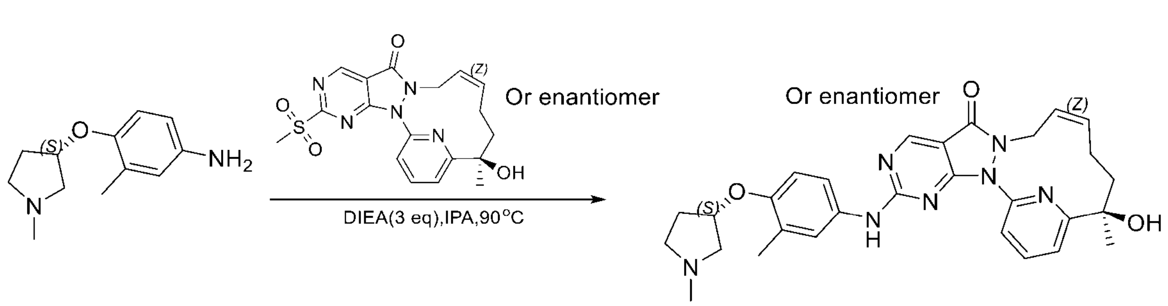


*N,N*-Diisopropylethylamine (282 mg, 2.18 mmol) was added to a solution of (*S*)-3-methyl-4-((1-methylpyrrolidin-3-yl)oxy)aniline (150 mg, 0.73 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one or isomer (351 mg, 0.87 mmol) in isopropanol (2 mL). The resulting mixed reaction solution was heated to 90 °C and stirred for 6 h. The mixed reaction

solution was diluted with water and extracted with ethyl acetate (3 × 50 mL). The combined organic phases were washed with saturated brine (1 × 10 mL), dried over anhydrous sodium sulfate, filtered, and concentrated under reduced pressure. The residue was purified by C18 reverse phase column chromatography (ACN/$H_2O$ (0.1% FA) = 30%-60% elution) to give (*R,Z*)-12-hydroxy-12-methyl-2-((3-methyl-4-(((*S*)-1-methylpyrrolidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or isomer (90 mg, yield: 23.5%), LC-MS *m*/*z:* 528[M + H]+;

$^1$H NMR (400 MHz, MeOD-d4): 8.80 (s, 1H), 7.95 (t, J = 8.0 Hz, 1H), 7.82 (d, J = 12.0 Hz, 1H), 7.59 (d, J = 8.0 Hz, 2H), 7.36 (d, J = 8.0 Hz, 1H), 6.77 (d, J = 8.0 Hz,1H),5.50-5.45 (m, 2H),4.94 (s, 1H),4.59 (d, J = 4.0 Hz, 2H), 3.05-3.02 (m, 3H), 2.73-2.71(m, 1H), 2.30 (s, 3H), 2.45-2.38 (m, 1H), 2.23-2.14 (m, 5H), 2.07-1.89 (m, 3H), 1.68 (s, 3H).

**Example 29: Synthesis of (*Z*)-12-hydroxy-12-methyl-2-((3-methyl-4-((2-methyl-2-azaspiro[3.3]heptan-6-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 187)**

**1) Synthesis of *tert*-butyl 6-((2-methyl-4-nitrophenyl)amino)-2-azaspiro[3.3]heptane-2-carboxylate**

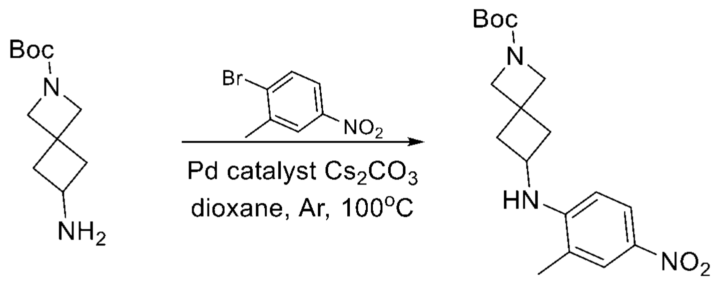


A solution of *tert*-butyl 6-amino-2-azaspiro[3.3]heptane-2-carboxylate (200 mg, 0.94 mmol), 1-bromo-2-methyl-4-nitrobenzene (305 mg, 1.41 mmol), (SP-4-1)-[1,3-bis[2,6-bis (1-ethylpropyl) phenyl]-4,5-dichloro-1,3-dihydro-2*H*-imidazol-2-ylidene]dichloro(2-methylpyridine)palladium (79 mg, 0.09 mmol), and cesium carbonate (921 mg, 2.82 mmol) in 1,4-dioxane (2.0 mL) was heated to 100 °C and stirred for 16 h under an argon atmosphere. After cooling, the mixed reaction solution was filtered and concentrated under reduced pressure. The residue was purified by C18 reverse phase column chromatography (MeCN:$H_2O$ (0.1% $NH_3.H_2O$) = 5%-95% elution) to give *tert*-butyl 6-((2-methyl-4-nitrophenyl)amino)-2-azaspiro[3.3]heptane-2-carboxylate (150 mg, yield: 45.87%), LC-MS *m*/*z*: 348[M + H]+;

**2) Synthesis of *N*-(2-methyl-4-nitrophenyl)-2-azaspiro[3.3]heptan-6-amine**

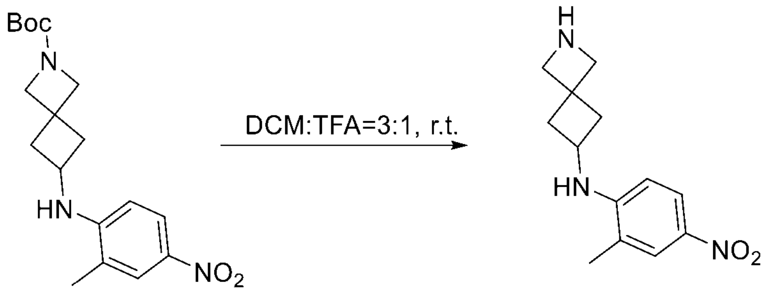


2,2,2-Trifluoroacetic acid (0.5 mL) was added dropwise to a solution of *tert*-butyl 6-((2-methyl-4-nitrophenyl)amino)-2-azaspiro[3.3]heptane-2-carboxylate (150 mg, 0.43 mmol) in dichloromethane (1.5 mL) at room temperature. The resulting mixed reaction solution was stirred at room temperature for 1 h. The resulting mixed reaction solution was concentrated under reduced pressure. The residue was purified by C18 reverse phase column chromatography (MeCN/$H_2O$ (0.1% FA) = 5%-95% elution) to give N-(2-methyl-4-nitrophenyl)-2-azaspiro[3.3]heptan-6-amine (80 mg, yield: 74.93%), LC-MS *m/z:* 248[M + H]+;

**3) Synthesis of 2-methyl-*N*-(2-methyl-4-nitrophenyl)-2-azaspiro[3.3]heptan-6-amine**

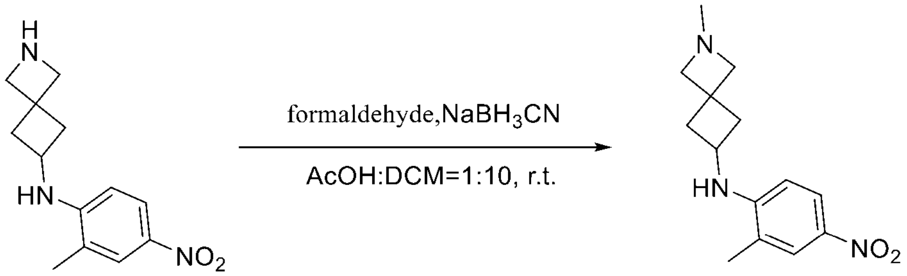


A solution of *N*-(2-methyl-4-nitrophenyl)-2-azaspiro[3.3]heptan-6-amine (80 mg, 0.32 mmol), paraformaldehyde (20 mg, 0.64 mmol), and $NaBH_3CN$ (41 mg, 0.65 mmol) in acetic acid (0.1 mL) and dichloromethane (10 mL) was stirred at room temperature for 0.5 h. The mixed reaction solution was filtered and concentrated under reduced pressure. The residue was purified by C18 reverse phase column chromatography (MeCN:$H_2O$ (0.1% FA) = 5%-95% elution) to give 2-methyl-*N*-(2-methyl-4-nitrophenyl)-2-azaspiro[3.3]heptan-6-amine (40 mg, yield: 47.32%), LC-MS *m/z*: 262[M + H]$^+$;

**4) Synthesis of 2-methyl-*N*$^1$-(2-methyl-2-azaspiro[3.3]heptan-6-yl)benzene-1,4-diamine**

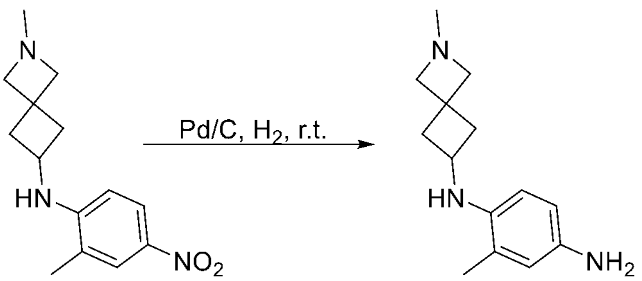


A solution of 2-methyl-*N*-(2-methyl-4-nitrophenyl)-2-azaspiro[3.3]heptan-6-amine (40 mg, 0.15 mmol) and Pd/C (40 mg) in methanol (2.0 mL) was stirred at room temperature for 4 h under a hydrogen atmosphere. The mixed reaction solution was filtered, and the filter cake was washed with methanol (3 × 5 mL). The filtrate was collected and concentrated under reduced pressure. The residue was purified by C18 reverse phase column chromatography (MeCN:$H_2O$ (0.1% $NH_3 \cdot H_2O$) = 5%-95% elution) to give 2-methyl-*N*$^1$-(2-methyl-2-azaspiro[3.3]heptan-6-yl)benzene-1,4-diamine (20 mg, yield: 56.48%), LC-MS m/z: 232 [M + H]$^+$;

**5) Synthesis of (*Z*)-12-hydroxy-12-methyl-2-((3-methyl-4-((2-methyl-2-azaspiro[3.3]heptan-6-yl)amino)phenyl) amino)-7,10,11,12-tetrahydro-5*H*-13,17-(azeno)pyrimido[4',5':3,4]pyrazolo[1,2-*a*][1,2]diazacyclotridecin-5-one**

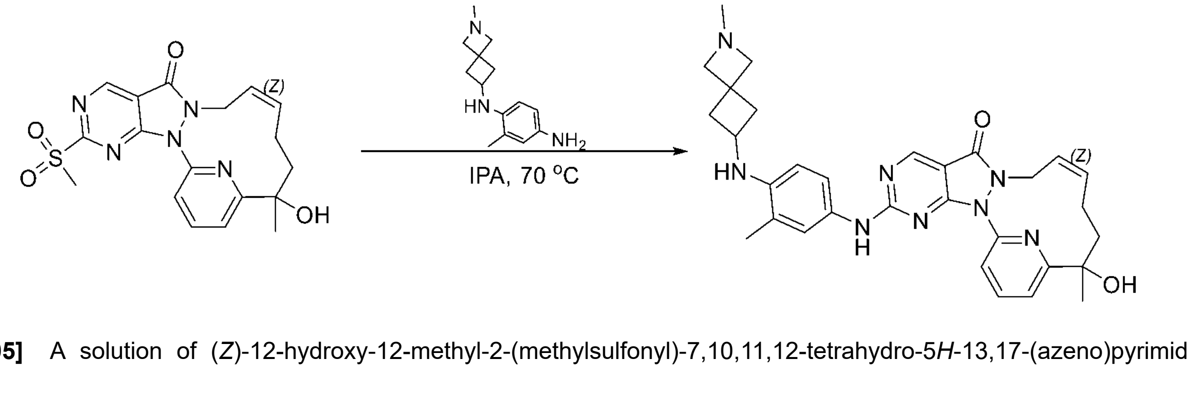


A solution of (*Z*)-12-hydroxy-12-methyl-2-(methylsulfonyl)-7,10,11,12-tetrahydro-5*H*-13,17-(azeno)pyrimido

[4',5':3,4]pyrazolo[1,2-*a*][1,2]diazacyclotridecin-5-one (21 mg, 0.052 mmol) and 2-methyl-$N^1$-(2-methyl-2-azaspiro[3.3]heptan-6-yl)benzene-1,4-diamine (20 mg, 0.09 mmol) in isopropanol (1.0 mL) was heated to 70 °C and stirred for 2 h. After cooling, the mixed reaction solution was filtered and concentrated under reduced pressure. The residue was purified by C18 reverse phase column chromatography (MeCN:$H_2O$ (0.1% $NH_3.H_2O$) = 5%-95% elution) to give (*Z*)-12-hydroxy-12-methyl-2-((3-methyl-4-((2-methyl-2-azaspiro[3.3]heptan-6-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (5 mg, yield: 17.30%), LC-MS *m/z:* 553[M + H]$^+$; $^1$H NMR (400 MHz, MeOD) δ 8.75 (d, J = 16.0 Hz, 1H), 7.96-7.78 (m, 1H), 7.70 (s, 1H), 7.57-7.42 (m, 1H), 6.70-6.42 (m, 3H), 5.62-5.41 (m, 2H), 4.59-4.43 (m, 2H), 4.09-3.88 (m, 3H), 3.29-3.18 (m, 3H), 3.03-2.84 (m, 2H), 2.24-2.09 (m, 3H), 2.03-1.94 (m, 3H), 1.94-1.76 (m, 3H), 1.69-1.52 (m, 5H).

**Example 30: Synthesis of (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenoxy)piperidine-1-carbaldehyde or Enantiomer (Compound 188)**

**1) Synthesis of *tert*-butyl 4-(2-methyl-4-nitrophenoxy)piperidine-1-carboxylate**

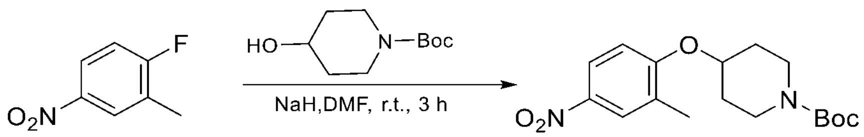


At 0 °C, sodium hydride (1.858 g, 77.419 mmol) was added in batches to a solution of *tert*-butyl 4-hydroxypiperidine-1-carboxylate (6.225 g, 30.968 mmol) in *N,N*-dimethylformamide (30 mL), and the resulting mixed reaction solution was stirred at room temperature for 30 min. Then, a solution of 1-fluoro-2-methyl-4-nitrobenzene (4 g, 25.806 mmol) in *N,N*-dimethylformamide (20 mL) was added dropwise to the above mixed reaction solution. The resulting mixed reaction solution was stirred at room temperature for 3 h. After cooling, the mixed reaction solution was diluted with water and extracted with ethyl acetate (6 × 300 mL). The combined organic phases were washed with saturated brine (3 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give *tert*-butyl 4-(2-methyl-4-nitrophenoxy)piperidine-1-carboxylate (2.8 g, yield: 32.3%), LC-MS *m/z:* 337 [M + H]$^+$;

**2) Synthesis of 4-(2-methyl-4-nitrophenoxy)piperidine**

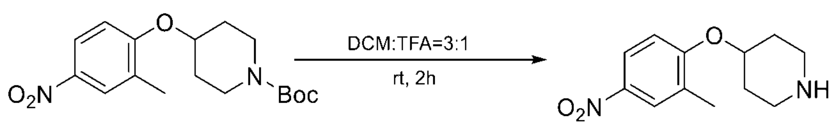


A solution of *tert*-butyl 4-(2-methyl-4-nitrophenoxy)piperidine-1-carboxylate (2.800 g, 8.333 mmol) in dichloromethane (21 mL) and 2,2,2-trifluoroacetic acid (7 mL) was stirred at room temperature for 2 h. The mixed reaction solution was diluted with water and extracted with ethyl acetate (5 × 60 mL). The combined organic phases were washed with saturated brine (3 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 4-(2-methyl-4-nitrophenoxy)piperidine (1.5 g, yield: 76.3%), LC-MS *m/z:* 237 [M + H]$^+$;

**3) Synthesis of 4-(2-methyl-4-nitrophenoxy)piperidine-1-carbaldehyde**

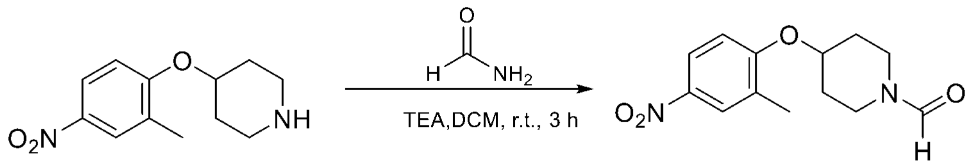


Triethylamine (154.068 mg, 1.525 mmol) was added to a solution of 4-(2-methyl-4-nitrophenoxy)piperidine (120 mg, 0.508 mmol) and formamide (27.45 mg, 0.610 mmol) in dichloromethane (2 mL) at room temperature. The resulting mixed reaction solution was stirred at room temperature for 3 h. The mixed reaction solution was diluted with water and extracted with ethyl acetate (3 × 80 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 4-(2-methyl-4-nitrophenoxy)piperidine-1-carbaldehyde (86 mg, yield: 64.1%), LC-MS *m/z*: 265 $[M + H]^+$;

**4) Synthesis of 4-(4-amino-2-methylphenoxy)piperidine-1-carbaldehyde**

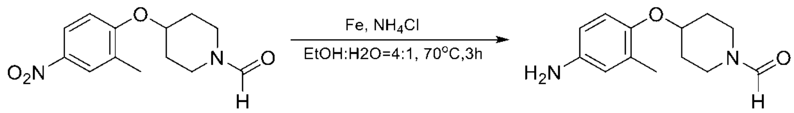


A solution of 4-(2-methyl-4-nitrophenoxy)piperidine-1-carbaldehyde (86 mg, 3.06 mmol), $NH_4Cl$ (51.795 mmol, 0.977 mmol), and Fe (91.212 mg, 1.629 mmol) in ethanol (2 mL) and water (0.5 mL) was heated to 70 °C and stirred for 3 h. The mixed reaction solution was diluted with water and extracted with ethyl acetate (3 × 80 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 4-(4-amino-2-methylphenoxy)piperidine-1-carbaldehyde (54 mg, yield: 70.8%), LC-MS *m/z*: 235 $[M + H]^+$;

**5) Synthesis of (*R,Z*)-4-(4-((12-hydroxy-12-methyl-5-oxo-7,10,11,12-tetrahydro-5*H*- 13,17-(azeno)pyrimido [4',5':3,4]pyrazolo[1,2-*a*][1,2]diazacyclotridecin-2-yl)amino)-2-methylphenoxy)piperidine-1-carbaldehyde or enantiomer**

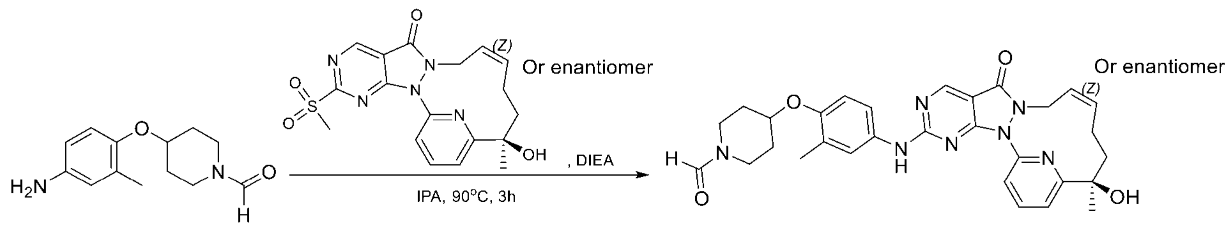


*N,N*-Diisopropylethylamine (52.115 mg, 0.404 mmol) was added to a solution of 4-(4-amino-2-methylphenoxy) piperidine-1-carbaldehyde (38.07 mg, 0.162 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one or enantiomer (54 mg, 0.135 mmol) in isopropanol (3 mL) at room temperature. The resulting mixed reaction solution was heated to 90 °C and stirred for 3 h. After cooling, the mixed reaction solution was diluted with water and extracted with ethyl acetate (3 × 50 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenoxy)piperidine-1-carbaldehyde or enantiomer (20 mg, yield: 26.8%), LC-MS *m/z*: 556 $[M + H]^+$;
$^1$H NMR (400 MHz, MeOD-*d4*): δ 8.80 (s, 1H), 8.04 (s, 1H), 7.96 (t, *J* = 7.8 Hz, 1H), 7.82 (d, *J* = 8.4 Hz, 1H), 7.60 (d, *J* = 8 Hz, 1H), 7.37 (d, *J* = 8.8 Hz, 1H), 6.93 (d, *J* = 8.8 Hz, 1H), 5.50-5.42 (m, 2H), 4.60 (d, *J* = 6.4 Hz, 2H), 3.72-3.65 (m, 2H), 3.58-3.54 (m, 1H), 3.48-3.41 (m, 2H), 2.23 (s, 3H), 2.19-2.13 (m, 1H), 2.06-1.73 (m, 7H), 1.68 (s, 3H).

**Example 31: Synthesis of (*R,Z*)-2-((4-((1-acetylpiperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or Enantiomer (Compound 189)**

**1) Synthesis of 1-(4-(2-methyl-4-nitrophenoxy)piperidin-1-yl)ethan-1-one**

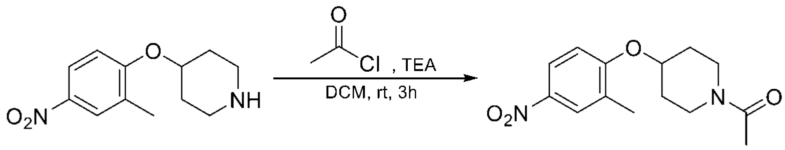


Triethylamine (398 mg, 3.941 mmol) was added to a solution of 4-(2-methyl-4-nitrophenoxy)piperidine (310 mg, 1.314 mmol) and acetyl chloride (122.949 mg, 1.576 mmol) in dichloromethane (4 mL) at room temperature. The resulting mixed reaction solution was stirred at room temperature for 3 h. The mixed reaction solution was diluted with water and extracted with ethyl acetate (5 × 20 mL). The combined organic phases were washed with saturated brine (3 × 10 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 1-(4-(2-methyl-4-nitrophenoxy)piperidin-1-yl)ethan-1-one (186 mg, yield: 50.9%), LC-MS *m/z*: 279 $[M + H]^+$;

**2) Synthesis of 1-(4-(4-amino-2-methylphenoxy)piperidin-1-yl)ethan-1-one**

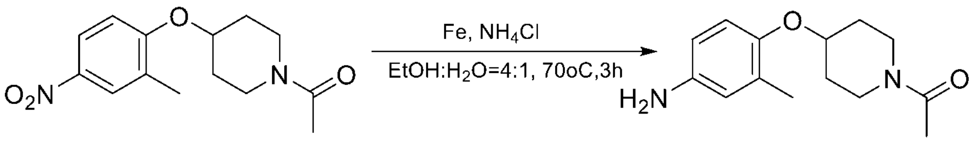


A solution of 1-(4-(2-methyl-4-nitrophenoxy)piperidin-1-yl)ethan-1-one (186 mg, 0.6699 mmol), Fe (18.338 mg, 3.345 mmol), and ammonium chloride (70.921 mg, 1.338 mmol) in ethanol (4 mL) and water (1 mL) was heated to 70 °C and stirred for 3 h. The mixed reaction solution was diluted with water and extracted with ethyl acetate (4 × 30 mL). The combined organic phases were washed with saturated brine (4 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 1-(4-(4-amino-2-methylphenoxy)piperidin-1-yl)ethan-1-one (66 mg, yield: 39.8%), LC-MS *m/z:* 249 $[M + H]^+$;

**3) Synthesis of (*R,Z*)-2-((4-((1-acetylpiperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer**

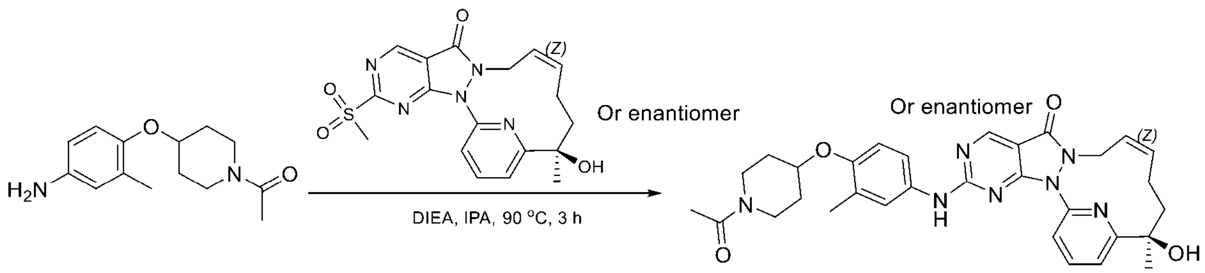


*N,N*-Diisopropylethylamine (48.254 mg, 0.374 mmol) was added to a solution of 1-(4-(4-amino-2-methylphenoxy)piperidin-1-yl)ethan-1-one (37.107 mg, 0.150 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one or enantiomer (50 mg, 0.125 mmol) in isopropanol (2 mL) at room temperature. The resulting mixed reaction solution was heated to 90 °C and stirred for 3 h. After cooling, the mixed reaction solution was diluted with water and extracted with ethyl acetate (3 × 50 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (*R,Z*)-2-((4-((1-acetylpiperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer (18 mg, yield: 25.3%), LC-MS *m/z:* 570 [M + H]$^+$;
$^1$H NMR (400 MHz, MeOD-*d*4): δ 8.80-8.77 (m, 1H), 7.78-7.93 (m, 1H), 7.82-7.79 (m, 1H), 7.61-7.55 (m, 2H), 7.38-7.33 (m, 1H), 6.94-6.89 (m, 1H), 5.52-5.40 (m, 2H), 4.59 (s, 3H), 3.79-3.72 (m, 2H), 3.63-3.57 (m, 1H), 3.54-3.48 (m, 1H), 2.24-2.22 (m, 4H), 2.13 (s, 4H), 2.04-1.89 (m, 4H), 1.86-1.74 (m, 2H), 1.68 (s, 3H).

**Example 32: Synthesis of (*R,Z*)-2-((4-((1-(cyclopropanecarbonyl)piperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or Enantiomer (Compound 190)**

**1) Synthesis of cyclopropyl(4-(2-methyl-4-nitrophenoxy)piperidin-1-yl)methanone**

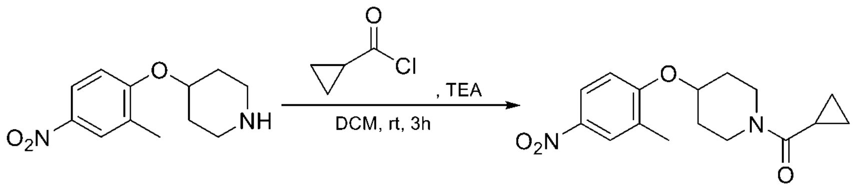


Triethylamine (38.008 mg, 3.941 mmol) was added to a solution of 4-(2-methyl-4-nitrophenoxy)piperidine (310 mg, 1.314 mmol) and cyclopropanecarbonyl chloride (163.932 mg, 1.576 mmol) in dichloromethane (3 mL) at room temperature. The resulting mixed reaction solution was stirred at room temperature for 3 h. The mixed reaction solution was diluted with water and extracted with ethyl acetate (3 × 30 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give cyclopropyl(4-(2-methyl-4-nitrophenoxy)piperidin-1-yl)methanone (154 mg, yield: 38.6%), LC-MS *m/z:* 305 [M + H]$^+$;

**2) Synthesis of (4-(4-amino-2-methylphenoxy)piperidin-1-yl)(cyclopropyl)methanone**

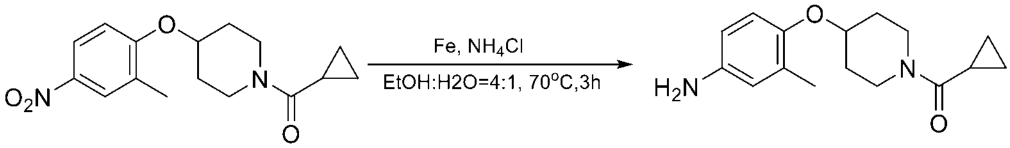


A solution of cyclopropyl(4-(2-methyl-4-nitrophenoxy)piperidin-1-yl)methanone (154 mg, 0.507 mmol), Fe (141.842 mg, 2.533 mmol), and ammonium chloride (80.546 mg, 1.520 mmol) in ethanol (4 mL) and water (1 mL) was heated to 70 °C and stirred for 3 h. The mixed reaction solution was diluted with water and extracted with ethyl acetate (3 × 80 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (4-(4-amino-2-methylphenoxy)piperidin-1-yl)(cyclopropyl)methanone (66 mg, yield: 47.5%), LC-MS *m/z:* 275 [M + H]$^+$;

**3) Synthesis of (*R,Z*)-2-((4-((1-(cyclopropanecarbonyl)piperidin-4-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer**

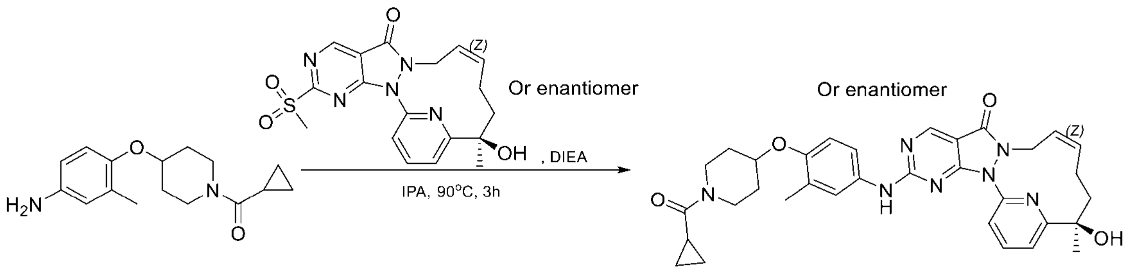


*N,N*-Diisopropylethylamine (24.2 mg, 0.1875 mmol) was added to a solution of (4-(4-amino-2-methylphenoxy) piperidin-1-yl)(cyclopropyl)methanone (49.197 mg, 0.180 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one or enantiomer (60 mg, 0.180 mmol) in isopropanol (3 mL) at room temperature. The resulting mixed reaction solution was heated to 90 °C and stirred for 3 h. After cooling, the mixed reaction solution was diluted with water and extracted with ethyl acetate (3 × 50 mL). The combined organic phases were washed with saturated brine (1 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (*R,Z*)-2-((4-((1-(cyclopropanecarbonyl) piperidin-4-yl)oxy)-3-methylphenyl)amino)-12-hydroxy-12-methyl-7,10,11,12-tetrahydro-5*H*-13,17-(azeno)pyrimido [4',5':3,4]pyrazolo[1,2-*a*][1,2]diazacyclotridecin-5-one or enantiomer (23 mg, yield: 25.8%), LC-MS *m/z*: 596 $[M + H]^+$; $^1H$ NMR (400 MHz, MeOD-d4): δ 8.7 (s, 1H), 7.95 (t, *J* = 8 Hz, 1H), 7.81 (d, *J* = 8 Hz, 1H), 7.59 (d, *J* = 7.2 Hz, 2H), 7.37-7.34 (m, 1H), 6.92 (d, *J* = 8.8 Hz, 1H), 5.53-5.43 (m, 2H), 4.64-4.58 (m, 3H), 3.99 (s, 1H), 3.77 (s, 2H), 3.60 (s, 1H), 2.23 (s, 3H), 2.16 (s, 1H), 2.03-1.96 (m, 3H), 1.93-1.86 (m, 2H), 1.75 (s, 1H), 1.68 (s, 2H), 0.90-0.80 (m, 4H).

**Example 33: Synthesis of methyl (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenoxy)piperidine-1-carboxylate or Enantiomer (Compound 191)**

**1) Synthesis of methyl 4-(2-methyl-4-nitrophenoxy)piperidine-1-carboxylate**

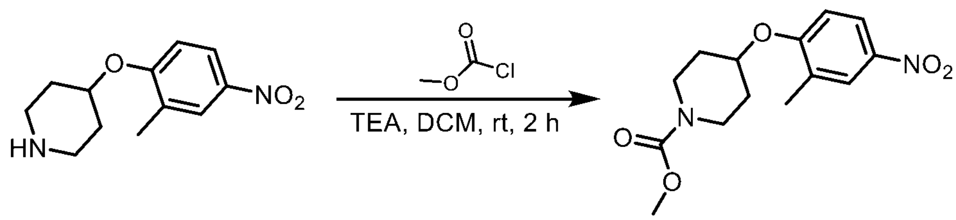


Methyl chloroformate (96 mg, 1.02 mmol) was added to a solution of 4-(2-methyl-4-nitrophenoxy)piperidine (200 mg, 0.85 mmol) and triethylamine (260 mg, 2.55 mmol) in dichloromethane (3 mL) at 0 °C. The resulting mixed reaction solution was stirred at room temperature for 1 h. The mixed reaction solution was quenched with methanol at room temperature. The resulting mixed reaction solution was concentrated under reduced pressure to give a crude product, which was used directly in the next step without further purification. LC-MS *m/z:* 295 $[M + H]^+$;

**2) Synthesis of methyl 4-(4-amino-2-methylphenoxy)piperidine-1-carboxylate**

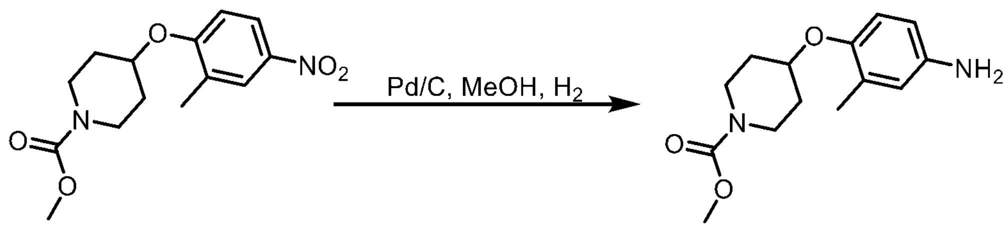

A solution of methyl 4-(2-methyl-4-nitrophenoxy)piperidine-1-carboxylate (200 mg, 0.68 mmol) and Pd/C (140 mg, 1.36 mmol) in methanol (3 mL) was stirred at room temperature for 1 h under a hydrogen atmosphere. The mixed reaction solution was filtered, and the filter cake was washed with methanol. The filtrate was collected and concentrated under reduced pressure. The residue was purified by silica gel column chromatography (EA:PE = 0%-60% elution) to give methyl 4-(4-amino-2-methylphenoxy)piperidine-1-carboxylate (120 mg, yield: 66.8%), LC-MS m/z: 265 [M + H]$^+$;

**3) Synthesis of methyl (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenoxy)piperidine-1-carboxylate or enantiomer**

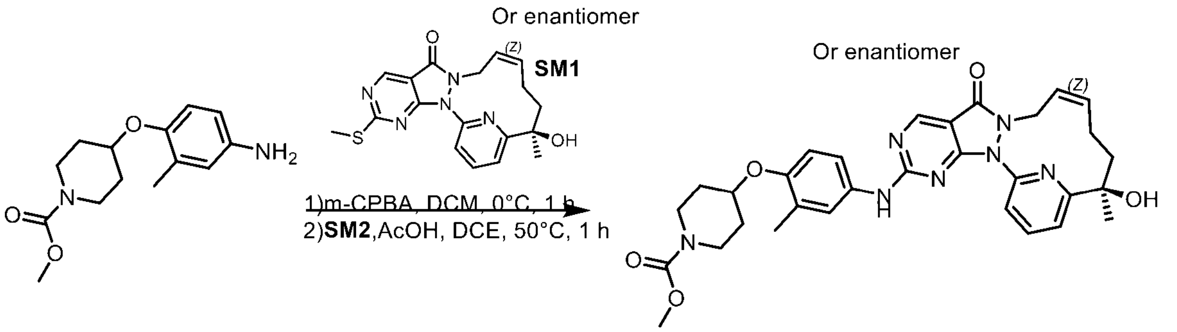


A solution of (*R,Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one or enantiomer (110 mg, 0.27 mmol) and 3-chloroperoxybenzoic acid (70 mg, 0.41 mmol) in dichloromethane (1 mL) was stirred at 0 °C for 1 h. Then, a solution of methyl 4-(4-amino-2-methylphenoxy)piperidine-1-carboxylate (110 mg, 0.41 mmol) and acetic acid (97 mg, 1.62 mmol) in 1,2-dichloroethane (1 mL) was added dropwise to the above mixed reaction solution. The resulting mixed reaction solution was heated to 50 °C and stirred for 1 h. The mixed reaction solution was concentrated under reduced pressure, and the residue was purified by C18 reverse phase column chromatography (MeCN:$H_2O$ = 0-80% elution) to give methyl (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl) amino)-2-methylphenoxy)piperidine-1-carboxylate or enantiomer (70 mg, yield: 44.2%), LC-MS *m/z:* 586 [M + H]$^+$; $^1$H NMR (400 MHz, MeOD-d4):δ 8.77 (s, 1H), 7.94 (t, J = 8.0 Hz, 1H), 7.80 (d, J = 8.0 Hz, 1H), 7.58 (d, J = 7.6 Hz, 2H), 7.34 (dd, J = 8.8, 2.4 Hz, 1H), 6.88 (d, J = 8.8 Hz, 1H), 5.53 - 5.40 (m, 2H), 4.57 (d, J = 7.2 Hz, 2H), 4.55 - 4.50 (m, 1H), 3.73 (d, J = 3.6 Hz, 1H), 3.70 (s, 3H), 3.69 - 3.66 (m, 1H), 3.48 - 3.40 (m, 2H), 2.20 (s, 3H), 2.16 (s, 2H), 2.02 - 1.86 (m, 4H), 1.73 (dd, J = 13.2, 4.0 Hz, 2H), 1.67 (s, 3H).

**Example 34: Synthesis of (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenoxy)piperidine-1-carboxamide or Enantiomer (Compound 192)**

**1) Synthesis of 4-(2-methyl-4-nitrophenoxy)piperidine-1-carboxamide**

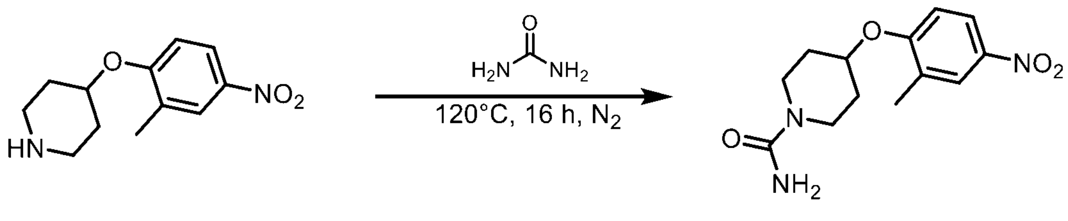


A mixed reaction solution of 4-(2-methyl-4-nitrophenoxy)piperidine (200 mg, 0.85 mmol) and urea (51 mg, 0.85 mol) was heated to 120 °C and stirred for 16 h under a nitrogen atmosphere. The resulting mixed reaction solution was purified by C18 reverse phase column chromatography (MeCN:$H_2O$ = 0%-60% elution) to give 4-(2-methyl-4-nitrophenoxy)piperidine-1-carboxamide (100 mg, yield: 42.3%), LC-MS *m/z:* 280[M + H]$^+$;

**2) Synthesis of 4-(4-amino-2-methylphenoxy)piperidine-1-carboxamide**

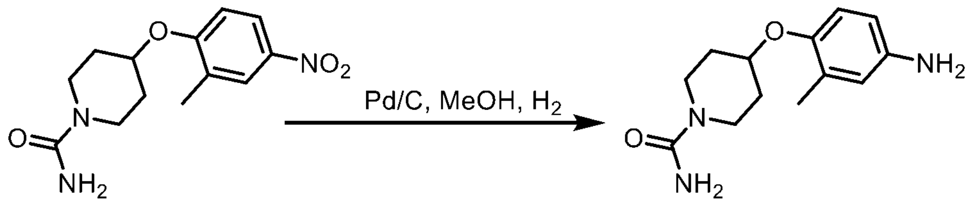


A solution of 4-(2-methyl-4-nitrophenoxy)piperidine-1-carboxamide (100 mg, 0.36 mmol) and Pd/C (77 mg, 0.72 mmol) in methanol (3 mL) was stirred at room temperature for 1 h under a hydrogen atmosphere. The mixed reaction solution was filtered, and the filter cake was washed with methanol. The filtrate was collected and concentrated under reduced pressure to give a crude product, which was used directly in the next step without further purification. LC-MS *m/z:* 250 $[M + H]^+$;

**3) Synthesis of (*R*,*Z*)-4-(4-((12-hydroxy-12-methyl-5-oxo-7,10,11,12-tetrahydro-5*H*-13,17-(azeno)pyrimido [4',5':3,4]pyrazolo[1,2-*a*][1,2]diazacyclotridecin-2-yl)amino)-2-methylphenoxy)piperidine-1-carboxamide or enantiomer**

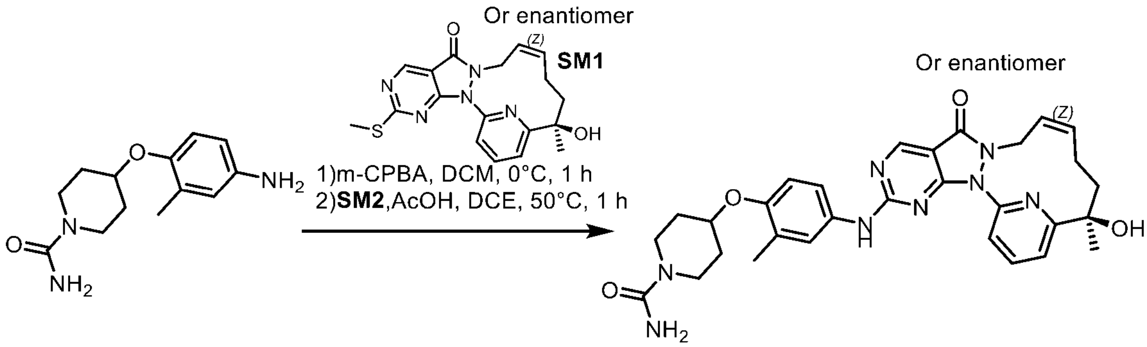


A solution of (*R,Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one or enantiomer (100 mg, 0.27 mmol) and 3-chloroperoxybenzoic acid (70 mg, 0.41 mmol) in dichloromethane (1 mL) was stirred at 0 °C for 1 h. Then, a solution of 4-(4-amino-2-methylphenoxy)piperidine-1-carboxamide (100 mg, 0.41 mmol) and acetic acid (97 mg, 1.62 mmol) in 1,2-dichloroethane (1 mL) was added. The resulting mixed reaction solution was heated to 50 °C and stirred for 1 h. The residue was purified by C18 column chromatography (MeCN:$H_2O$ = 0-80%) to give (*R,Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenoxy)piperidine-1-carboxamide or enantiomer (25 mg, yield: 17.6%), LC-MS *m/z*: 571 $[M + H]^+$;
$^1$H NMR (400 MHz, MeOD-d4):δ 8.79 (s, 1H), 7.95 (t, J = 7.6 Hz, 1H), 7.82 (d, J = 8.0 Hz, 1H), 7.58 (d, J = 7.6 Hz, 2H), 7.35 (dd, J = 8.8, 2.5 Hz, 1H), 6.90 (d, J = 8.8 Hz, 1H), 5.54 - 5.40 (m, 2H), 4.57 (dd, J = 12.0, 5.0 Hz, 3H), 3.70 - 3.63 (m, 2H), 3.40 - 3.35 (m, 2H), 2.22 (s, 5H), 2.02 - 1.84 (m, 4H), 1.80 - 1.65 (m, 5H).

**Example 35: Synthesis of (*Z*)-12-hydroxy-12-methyl-2-((3-methyl-4-((1-methylpiperidin-4-yl)methyl)phenyl) amino)-7,10,11,12-tetrahydro-5*H*-13,17-(azeno)pyrimido[4',5':3,4]pyrazolo[1,2-*a*][1,2]diazacyclotridecin-5-one (Compound 193)**

**1) Synthesis of *tert*-butyl 4-(2-methyl-4-nitrobenzyl)piperidine-1-carboxylate**

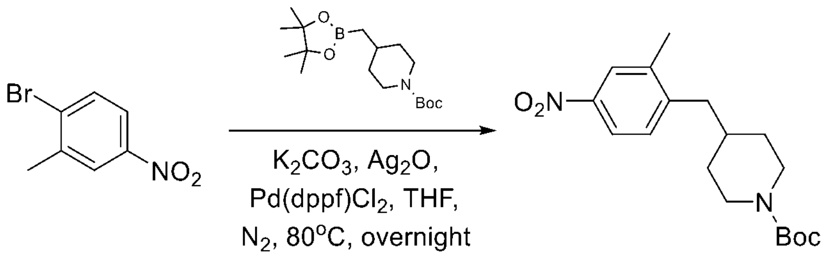


A solution of bromo-2-methyl-4-nitrobenzene (200 mg, 0.93 mmol), *tert-butyl* 4-((4,4,5,5-tetramethyl-1,3,2-dioxaborolan-2-yl)methyl)piperidine-1-carboxylate (604.5 mg, 1.86 mmol), potassium carbonate (385 mg, 2.79 mmol), and silver oxide (539.4 mg, 2.325 mmol) and pd(dppf)$Cl_2$ (68.1 mg, 0.093 mmol) in tetrahydrofuran (2 mL) was heated to 80 °C and stirred overnight under a nitrogen atmosphere. After cooling, the resulting mixed reaction solution was concentrated under reduced pressure. The residue was purified by C18 reverse phase column chromatography ($CH_3CN$:$H_2O$ (0.1% FA) = 5%-95% elution) to give *tert*-butyl 4-(2-methyl-4-nitrobenzyl)piperidine-1-carboxylate (120 mg, yield: 38.6%), LC-MS *m/z:* 335 [M + H]$^+$;

**2) Synthesis of 4-(2-methyl-4-nitrobenzyl)piperidine**

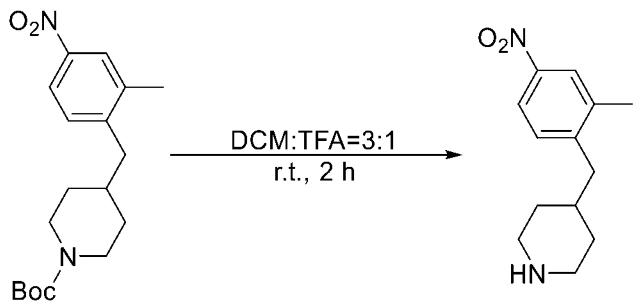


2,2,2-Trifluoroacetic acid (0.3 mL) was added dropwise to a solution of *tert*-butyl 4-(2-methyl-4-nitrobenzyl) piperidine-1-carboxylate (120 mg, 0.36 mmol) in dichloromethane (0.9 mL) at room temperature. The resulting mixed reaction solution was stirred at room temperature for 1 h. The resulting mixed reaction solution was concentrated under reduced pressure. The residue was purified by C18 reverse phase column chromatography ($CH_3CN$:$H_2O$ (0.1% FA) = 5%-95% elution) to give 4-(2-methyl-4-nitrobenzyl)piperidine (60 mg, yield: 71.4%), LC-MS *m/z*: 235 [M + H]$^+$;

**3) Synthesis of 1-methyl-4-(2-methyl-4-nitrobenzyl)piperidine**

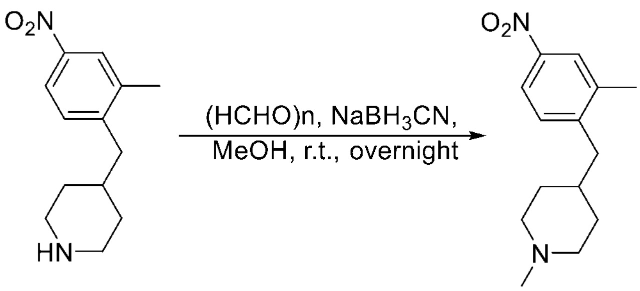


A solution of 4-(2-methyl-4-nitrobenzyl)piperidine (60 mg, 0.26 mmol), sodium cyanoborohydride (32.76 mg, 0.52 mmol), and paraformaldehyde (11.7 mg, 0.39 mmol) in methanol (2 mL) was stirred at room temperature overnight. The resulting mixed reaction solution was concentrated under reduced pressure. The residue was purified by C18 reverse phase column chromatography ($CH_3CN$:$H_2O$ (0.1% FA) = 5%-95% elution) to give 1-methyl-4-(2-methyl-4-nitrobenzyl) piperidine (60 mg, yield: 94.4%), LC-MS *m/z*: 249 [M + H]$^+$;

**4) Synthesis of 3-methyl-4-((1-methylpiperidin-4-yl)methyl)aniline**

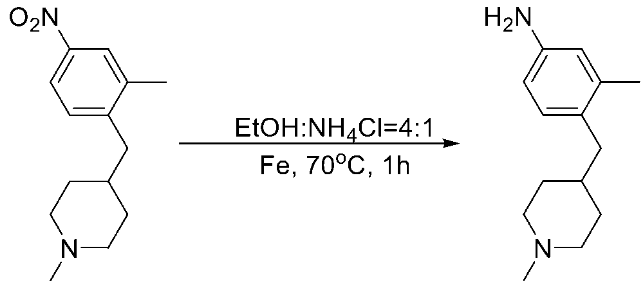


A solution of 1-methyl-4-(2-methyl-4-nitrobenzyl)piperidine (60 mg, 0.24 mmol) and Fe (67.74 mg, 1.21 mmol) in ethanol (0.8 mL) and saturated aqueous ammonium chloride solution (0.2 mL) was heated to 70 °C and stirred for 2 h under a nitrogen atmosphere. After cooling, the mixed reaction solution was filtered, and the filter cake was washed with methanol (3 × 15 mL). The filtrate was collected and concentrated under reduced pressure to give a crude product, which was used directly in the next step without further purification. LC-MS *m/z:* 219 [M + H]$^+$;

**5) Synthesis of (*Z*)-12-hydroxy-12-methyl-2-((3-methyl-4-((1-methylpiperidin-4-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one**

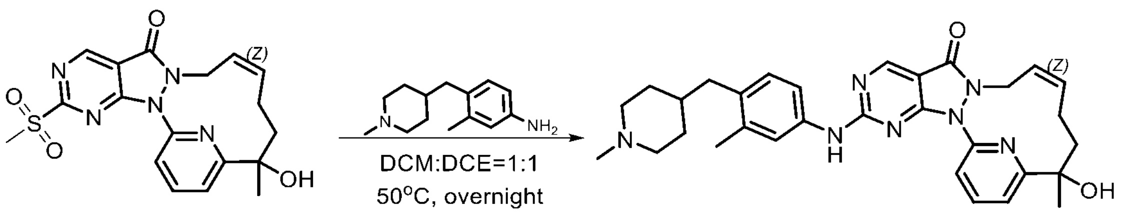


A solution of (Z)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol) and 3-methyl-4-((1-methylpiperidin-4-yl) methyl)aniline (81.75 mg, 0.375 mmol) in dichloromethane (1.5 mL) and 1,2-dichloromethane (1.5 mL) was heated to 50 °C and stirred overnight. After cooling, the mixed reaction solution was concentrated under reduced pressure. The residue was purified by C18 reverse phase column chromatography ($CH_3CN:H_2O$ (0.1% FA) = 5%-95% elution) to give (Z)-12-hydroxy-12-methyl-2-((3-methyl-4-((1-methylpiperidin-4-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (5 mg, yield: 3.7%), LC-MS *m/z:* 540 [M + H]$^+$;

$^1$H NMR (400 MHz, MeOD) δ 8.82 (s, 1H), 7.95 (d, *J* = 7.8 Hz, 1H), 7.84 (d, *J* = 7.6 Hz, 1H), 7.60 (d, *J* = 7.2 Hz, 2H), 7.37 (d, *J* = 8.2 Hz, 1H), 7.04 (s, 1H), 5.56 - 5.39 (m, 2H), 4.61 (d, *J* = 6.7 Hz, 2H), 2.97 (d, *J* = 11.9 Hz, 2H), 2.56 (d, *J* = 7.1 Hz, 2H), 2.36 (s, 3H), 2.30 (s, 3H), 2.15 (s, 4H), 2.00 (s, 1H), 1.93 (s, 1H), 1.73 (s, 1H), 1.69 (s, 1H), 1.68 (s, 3H), 1.57 (s, 1H), 1.38 (d, *J* = 12.4 Hz, 2H).

**Example 36: Synthesis of (*Z*)-12-hydroxy-12-methyl-2-((3-methyl-4-(4-methylpiperazine-1-carbonyl)phenyl) amino)-7,10,11,12-tetrahydro-5*H*-13,17-(azeno)pyrimido[4',5':3,4]pyrazolo[1,2-*a*][1,2]diazacyclotridecin-5-one (Compound 194)**

**1) Synthesis of (2-methyl-4-nitrophenyl)(4-methylpiperazin-1-yl)methanone**

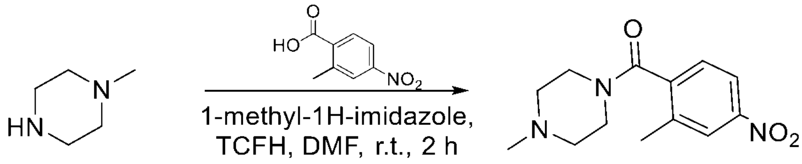


A solution of 1-methylpiperazine (1000 mg, 10 mmol), 2-methyl-4-nitrobenzoic acid (2715 mg, 15 mmol), 1-methyl-1*H*-imidazole (3280 mg, 40 mmol), and *N,N,N',N'*-tetramethylchloroformamidinium hexafluorophosphate (5620 mg, 20 mmol) in *N,N*-dimethylformamide (20 mL) was stirred at room temperature overnight. The resulting mixed reaction solution was concentrated under reduced pressure. The residue was purified by C18 reverse phase column chromatography ($CH_3CN$:$H_2O$ (0.1% FA) = 5%-95% elution) to give (2-methyl-4-nitrophenyl)(4-methylpiperazin-1-yl)methanone (1100 mg, yield: 41.8%), LC-MS *m/z:* 264 [M + H]$^+$;

**2) Synthesis of (4-amino-2-methylphenyl)(4-methylpiperazin-1-yl)methanone**

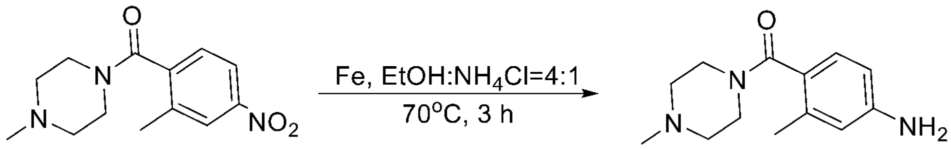


A solution of (2-methyl-4-nitrophenyl)(4-methylpiperazin-1-yl)methanone (500 mg, 1.9 mmol) and Fe (532.3 mg, 9.5 mmol) in ethanol (4 mL) and saturated aqueous ammonium chloride solution (1 mL) was heated to 70 °C and stirred for 2 h under a nitrogen atmosphere. After cooling, the mixed reaction solution was filtered, and the filter cake was washed with methanol (3 × 15 mL). The filtrate was collected and concentrated under reduced pressure to give a crude product, which was used directly in the next step without further purification. LC-MS *m/z:* 234 [M + H]$^+$;

**3) Synthesis of (*Z*)-12-hydroxy-12-methyl-2-((3-methyl-4-(4-methylpiperazine-1-carbonyl)phenyl)amino)-7,10,11,12-tetrahydro-5*H*-13,17-(azeno)pyrimido[4',5':3,4]pyrazolo[1,2-*a*][1,2]diazacyclotridecin-5-one**

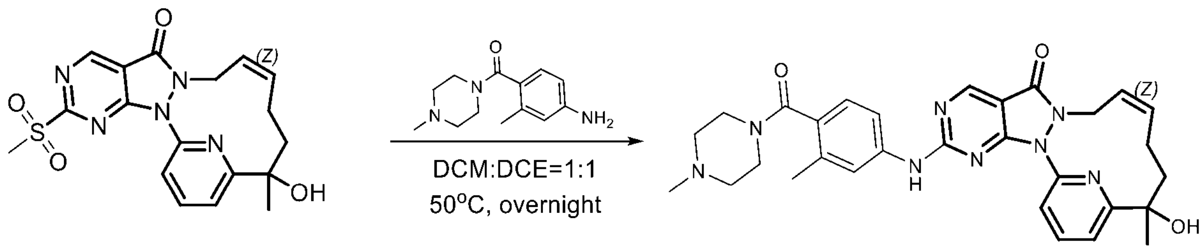


A solution of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (100 mg, 0.25 mmol) and (4-amino-2-methylphenyl)(4-methylpiperazin-1-yl)methanone (87.4 mg, 0.375 mmol) in dichloromethane (1.5 mL) and 1,2-dichloroethane (1.5 mL) was heated to 50 °C and stirred overnight. After cooling, the mixed reaction solution was filtered and concentrated. The residue was purified by C18 reverse phase column chromatography ($CH_3CN$:$H_2O$ (0.1% FA) = 5%-95% elution) to give (*Z*)-12-hydroxy-12-methyl-2-((3-methyl-4-(4-methylpiperazine-1-carbonyl)phenyl)amino)-7,10,11,12-tetrahydro-5*H*-13,17-(azeno)pyrimido[4',5':3,4]pyrazolo[1,2-*a*][1,2]diazacyclotridecin-5-one (0.9 mg, yield: 0.65%), LC-MS *m/z:* 555 [M + H]$^+$;
$^1$H NMR (400 MHz, MeOD) δ 8.87 (s, 1H), 8.00 (s, 1H), 7.85 - 7.78 (m, 2H), 7.62 (d, *J* = 7.8 Hz, 1H), 7.56 (d, *J* = 7.6 Hz, 1H), 7.14 (d, *J* = 8.4 Hz, 1H), 5.51 - 5.40 (m, 2H), 4.63 (d, *J* = 6.6 Hz, 2H), 4.58 (s, 2H), 3.81 (s, 2H), 2.53 (s, 2H), 2.37 (s, 2H), 2.32 (s, 3H), 2.28 (s, 3H), 2.17 (s, 2H), 2.00 (s, 1H), 1.89 (s, 1H), 1.69 (s, 3H).

**Example 37: Synthesis of (*Z*)-2-((4-((*S*)-3,4-dimethylpiperazine-1-carbonyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 195)**

**1) Synthesis of (*S*)-(3,4-dimethylpiperazin-1-yl)(2-methyl-4-nitrophenyl)methanone**

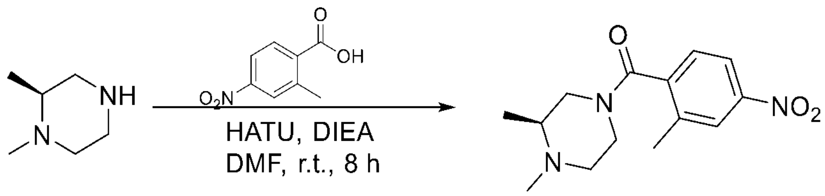


A solution of (*S*)-1,2-dimethylpiperazine (300 mg, 2.63 mmol), 2-methyl-4-nitrobenzoic acid (714.0 mg, 3.94 mmol), *O*-(7-azabenzotriazol-1-yl)-*N,N,N',N'*-tetramethyluronium hexafluorophosphate (1.5 g, 3.95 mmol), and *N,N*-diisopropylethylamine (1.7 g, 13.18 mmol) in *N,N*-dimethylformamide (3 mL) was stirred at room temperature for 8 h. The mixed reaction solution was purified by C18 reverse phase column chromatography ($CH_3CN$:$H_2O$ (0.1% $NH_3$.$H_2O$) = 5%-95% elution) to give (S)-(3,4-dimethylpiperazin-1-yl)(2-methyl-4-nitrophenyl)methanone (300 mg, yield: 41.15%), LC-MS *m/z*: 278 $[M + H]^+$;

**2) Synthesis of (*S*)-(4-amino-2-methylphenyl)(3,4-dimethylpiperazin-1-yl)methanone**

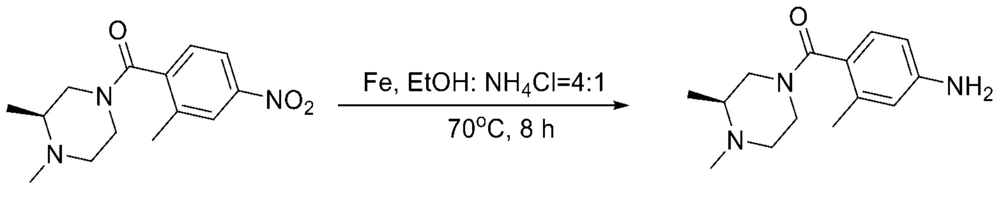


A solution of (*S*)-(3,4-dimethylpiperazin-1-yl)(2-methyl-4-nitrophenyl)methanone (300 mg, 1.08 mmol) and Fe (303.24 mg, 5.41 mmol) in saturated aqueous ammonium chloride solution (1 mL) and ethanol (4 mL) was heated to 70 °C and stirred for 8 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m/z*: 248 $[M + H]^+$;

**3) Synthesis of (*Z*)-2-((4-(((*S*)-3,4-dimethylpiperazine-1-carbonyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

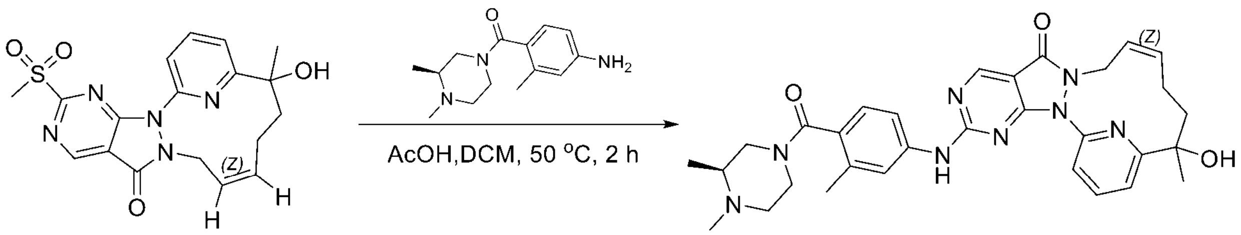


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (300 mg, 0.75 mmol), (*S*)-(4-amino-2-methylphenyl)(3,4-dimethylpiperazin-1-yl)methanone (277 mg, 1.12 mmol), and acetic acid (269 mg, 4.48 mmol) in 1,2-dichloroethane (5 mL) was heated to 50 °C and stirred for 2 h. After cooling, the mixed reaction solution was concentrated under reduced pressure. The residue was purified by C18 reverse phase column chromatography ($CH_3CN$:$H_2O$ (0.1% $NH_3$.$H_2O$) = 5%-95% elution) to give (*Z*)-2-((4-((*S*)-3,4-dimethylpiperazine-1-carbonyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (10 mg, yield: 2.35%), LC-MS *m/z:* 569 $[M + H]^+$;
$^1$H NMR (400 MHz, MeOD-*d*4): δ 8.89 - 8.84 (m, 1H), 8.01 - 7.96 (m, 1H), 7.84 - 7.78 (m, 2H), 7.62 - 7.54 (m, 2H), 7.14 (d, *J* =

8.0 Hz, 1H), 5.51 - 5.38 (m, 2H), 4.67-4.60 (m, 2H), 4.49 - 4.42 (m, 1H), 3.48 - 3.36 (m, 1H), 3.28-3.13 (m, 1H), 3.05 - 2.81 (m, 2H), 2.41 - 2.16 (m, 10H), 2.02-1.86 (m, 2H), 1.68 (s, 3H), 1.22-0.98 (m, 3H).

**Example 38: Synthesis of (*Z*)-2-((4-(((S)-3,4-dimethylpiperazin-1-yl)methyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 196)**

**1) Synthesis of (*S*)-1,2-dimethyl-4-(2-methyl-4-nitrobenzyl)piperazine**

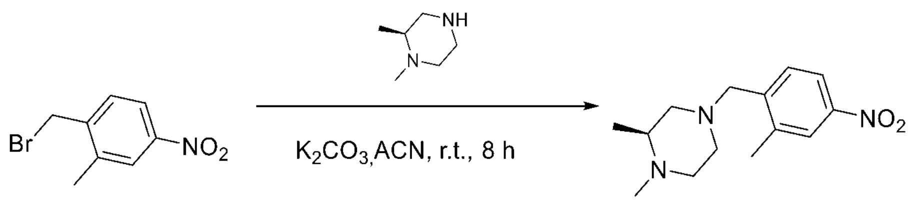


A solution of 1-(bromomethyl)-2-methyl-4-nitrobenzene (300 mg, 1.3 mmol), (S)-1,2-dimethylpiperazine (148.7 mg, 1.3 mol), and potassium carbonate (540 mg, 3.91 mmol) in acetonitrile (3 mL) was stirred at room temperature for 8 h. The resulting mixed reaction solution was concentrated under reduced pressure. The residue was purified by C18 reverse phase column chromatography ($CH_3CN$:$H_2O$ (0.1% $NH_3$.$H_2O$) = 5%-95% elution) to give (*S*)-1,2-dimethyl-4-(2-methyl-4-nitrobenzyl)piperazine (200 mg, yield: 58.3%), LC-MS m/z: 264 [M + H]$^+$;

**2) Synthesis of (*S*)-4-((3,4-dimethylpiperazin-1-yl)methyl)-3-methylaniline**

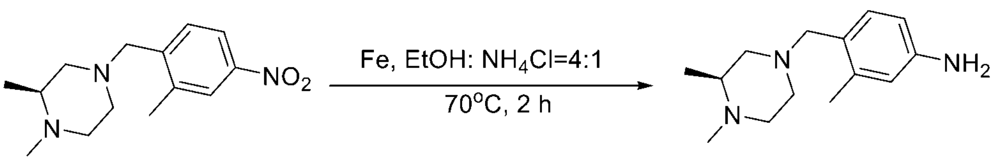


A solution of (*S*)-1,2-dimethyl-4-(2-methyl-4-nitrobenzyl)piperazine (200 mg, 0.76 mmol) and Fe (212.0 mg, 3.78 mmol) in aqueous ammonium chloride solution (1 mL) and ethanol (4 mL) was heated to 70 °C and stirred for 2 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m/z:* 234 [M + H]$^+$;

**3) Synthesis of (*Z*)-2-((4-(((*S*)-3,4-dimethylpiperazin-1-yl)methyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

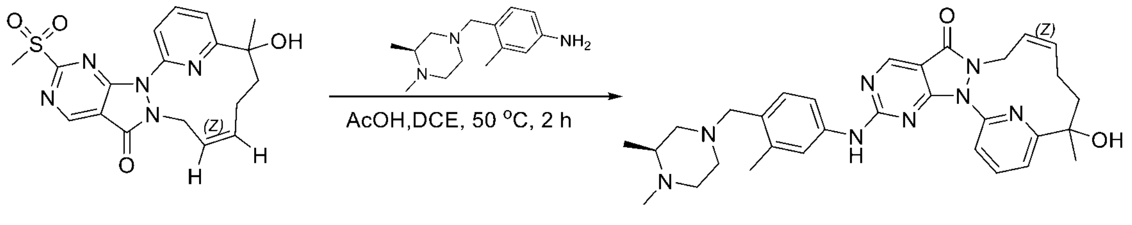


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol), (*S*)-4-((3,4-dimethylpiperazin-1-yl) methyl)-3-methylaniline (87.2 mg, 0.37 mmol), and acetic acid (89.7 mg, 1.50 mmol) in 1,2-dichloroethane (2 mL) was heated to 50 °C and stirred for 2 h. After cooling, the mixed reaction solution was filtered, and the filtrate was concentrated under reduced pressure. The residue was purified by C18 reverse phase column chromatography

($CH_3CN:H_2O$ (0.1% $NH_3.H_2O$) = 5%-95% elution) to give (*Z*)-2-((4-(((*S*)-3,4-dimethylpiperazin-1-yl)methyl)-3-methylphenyl)amino)-12-hydroxy-12-methyl-7,10,11,12-tetrahydro-5*H*-13, 17-(azeno)pyrimido[4',5':3,4]pyrazolo[1,2-*a*][1,2]diazacyclotridecin-5-one (35 mg, yield: 25.4%), LC-MS *m/z:* 555 [M + H]$^+$;
$^1$H NMR (400 MHz, MeOD-d4): δ 8.81 (s, 1H), 7.96 (t, *J* = 8.0 Hz, 1H), 7.82 (d, *J* = 8.0 Hz, 1H), 7.66 - 7.56 (m, 2H), 7.41-7.38 (m, 1H), 7.15 (d, *J* = 8.0Hz, 1H), 5.53 - 5.40 (m, 2H), 4.59 (d, *J* = 8.0 Hz, 2H), 3.49-3.42 (m, 2H), 2.87 (d, *J* = 12.0 Hz, 1H), 2.78 (t, *J* = 12.0 Hz, 2H), 2.47 - 2.20 (m, 11H), 2.03-1.95 (m, 2H), 1.94 - 1.88 (m, 1H), 1.68 (s, 3H), 1.09 (d, *J* = 8.0 Hz, 3H).

**Example 39: Synthesis of (*Z*)-2-((4-((*R*)-3,4-dimethylpiperazine-1-carbonyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 197)**

**1) Synthesis of (*R*)-(3,4-dimethylpiperazin-1-yl)(2-methyl-4-nitrophenyl)methanone**

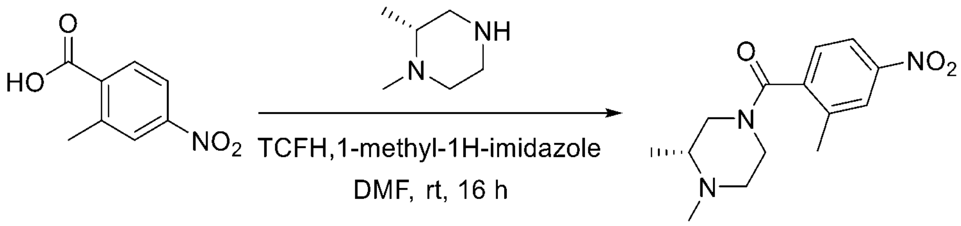


A solution of 2-methyl-4-nitrobenzoic acid (400.0 mg, 2.21 mmol), (*R*)-1,2-dimethylpiperazine (378 mg, 3.31 mmol), *N,N,N,N*-tetramethylchloroformamidinium hexafluorophosphate (1.2 g, 4.42 mmol), and 1-methyl-1*H*-imidazole (725 mg, 8.84 mmol) in *N,N*-dimethylformamide (2.0 mL) was stirred at room temperature for 16 h. LCMS analysis showed that the reaction was complete. After cooling, the resulting mixed reaction solution was concentrated under reduced pressure. The residue was purified by C18 reverse phase column chromatography (MeCN:$H_2O$ (0.1% $NH_3.H_2O$) = 5%-95% elution) to give (*R*)-(3,4-dimethylpiperazin-1-yl)(2-methyl-4-nitrophenyl)methanone (610 mg, yield: 99.6%), LC-MS *m*/*z*: 278 [M + H]$^+$;

**2) Synthesis of (*R*)-(4-amino-2-methylphenyl)(3,4-dimethylpiperazin-1-yl)methanone**

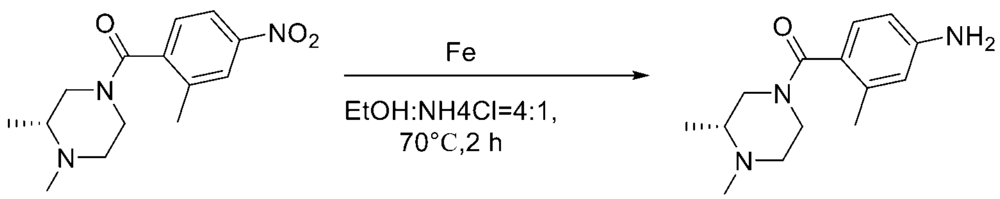


A solution of (*R*)-(3,4-dimethylpiperazin-1-yl)(2-methyl-4-nitrophenyl)methanone (610 mg, 2.2 mmol) and Fe (617 mg, 11 mmol) in saturated ammonium chloride solution (0.5 mL) and ethanol (2 mL) was heated to 70 °C and stirred for 2 h. LCMS analysis showed that the reaction was complete. After cooling, the mixed reaction solution was filtered, and the filter cake was washed with dichloromethane (3 × 10 mL). The filtrate was collected and concentrated under reduced pressure to give a crude product, which was used directly in the next step without further purification. LC-MS *m/z:* 248 [M + H]$^+$;

**3) Synthesis of (*Z*)-2-((4-(((*R*)-3,4-dimethylpiperazine-1-carbonyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

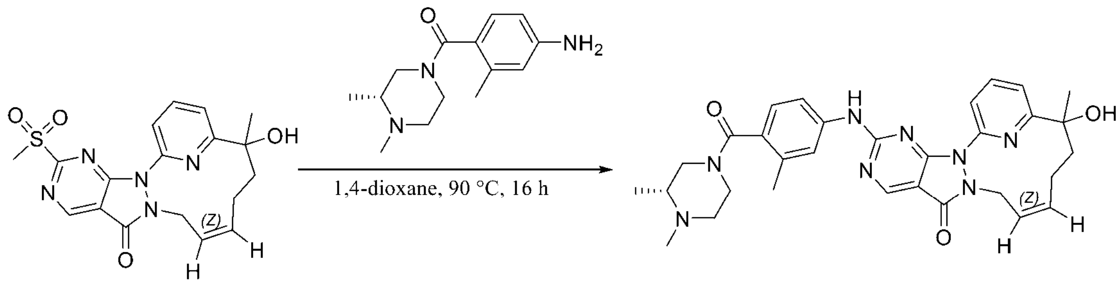


BIOT-005-7054

A solution of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (110 mg, 0.274 mmol) and (*R*)-(4-amino-2-methylphenyl)(3,4-dimethylpiperazin-1-yl)methanone (169 mg, 0.686 mmol) in 1,4-dioxane (2 mL) was heated to 90 °C and stirred for 16 h. LCMS analysis showed that the reaction was complete. After cooling, the resulting mixed reaction solution was concentrated under reduced pressure. The residue was purified by C18 reverse phase column chromatography (MeCN:$H_2O$ (0.1% $NH_3.H_2O$) = 5%-95% elution) to give (*Z*)-2-((4-((*R*)-3,4-dimethylpiperazine-1-carbonyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (10 mg, yield: 6.4%), LC-MS *m/z:* 569 $[M + H]^+$;

$^1$H NMR (400 MHz, MeOD-d4) δ 8.88 (s, 1H), 8.23 (s, 1H), 7.99 (t, *J* = 8.0 Hz, 1H), 7.86 - 7.77 (m, 2H), 7.60 (dd, *J* = 16.0, 8.0 Hz, 2H), 7.16 (d, *J* = 8.0 Hz, 1H), 5.46 (dd, *J* = 12.0, 8.0 Hz, 2H), 4.87 (s, 1H), 4.83 (s, 1H), 4.64 (d, *J* = 8.0 Hz, 2H), 4.48 (s, 1H), 3.32 (s, 1H), 3.29 - 3.26 (m, 1H), 2.93 (s, 2H), 2.49 (d, *J* = 16.0 Hz, 4H), 2.28 (s, 3H), 2.17 (s, 1H), 1.99 (d, *J* = 8.0 Hz, 1H), 1.94 - 1.85 (m, 1H), 1.69 (s, 3H), 1.26 (s, 1H), 1.03 (s, 1H).

**Example 40: Synthesis of (*Z*)-2-((4-(((*R*)-3,4-dimethylpiperazin-1-yl)methyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 198)**

**1) Synthesis of (*R*)-1,2-dimethyl-4-(2-methyl-4-nitrobenzyl)piperazine**

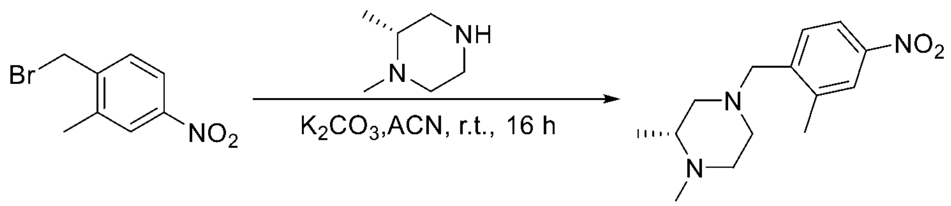


A solution of 1-(bromomethyl)-2-methyl-4-nitrobenzene (300.0 mg, 1.304 mmol), (R)-1,2-dimethylpiperazine (149 mg, 1.3004 mmol), and potassium carbonate (540 mg, 3.913 mmol) in acetonitrile (2.0 mL) was stirred at room temperature for 16 h. LCMS analysis showed that the reaction was complete. After cooling, the resulting mixed reaction solution was concentrated under reduced pressure. The residue was purified by C18 reverse phase column chromatography (MeCN:$H_2O$ (0.1% $NH_3.H_2O$) = 5%-95% elution) to give (*R*)-1,2-dimethyl-4-(2-methyl-4-nitrobenzyl)piperazine (300 mg, yield: 87.4%), LC-MS *m/z*: 264 $[M + H]^+$;

**2) Synthesis of (*R*)-4-((3,4-dimethylpiperazin-1-yl)methyl)-3-methylaniline**

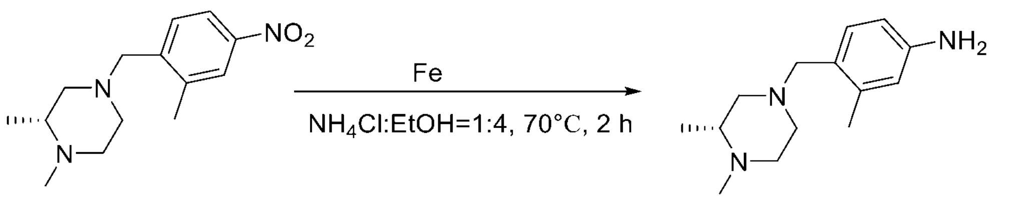

A solution of (*R*)-1,2-dimethyl-4-(2-methyl-4-nitrobenzyl)piperazine (300 mg, 1.14 mmol) and Fe (319 mg, 5.70 mmol) in saturated ammonium chloride solution (0.5 mL) and ethanol (2 mL) was heated to 70 °C and stirred for 2 h. LCMS analysis showed that the reaction was complete. After cooling, the mixed reaction solution was filtered, and the filter cake was washed with dichloromethane (3 × 10 mL). The filtrate was collected and concentrated under reduced pressure to give a crude product, which was used directly in the next step without further purification. LC-MS *m/z:* 234 [M + H]$^+$;

**3) Synthesis of (*Z*)-2-((4-(((*R*)-3,4-dimethylpiperazin-1-yl)methyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

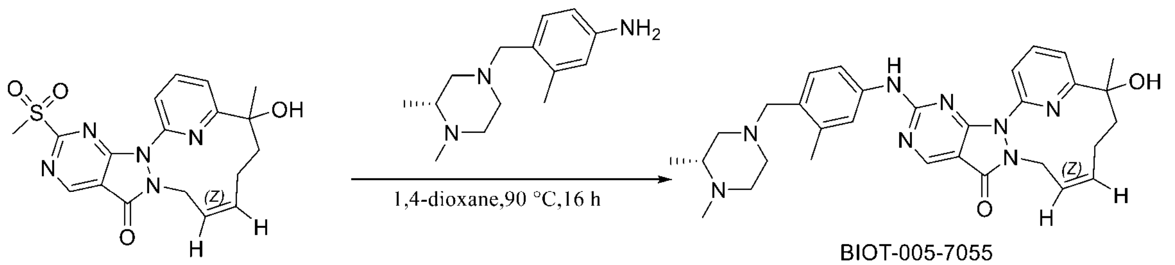


BIOT-005-7055

A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (40 mg, 0.108 mmol) and (*R*)-4-((3,4-dimethylpiperazin-1-yl) methyl)-3-methylaniline (30 mg, 0.128 mmol) in 1,4-dioxane (2 mL) was heated to 90 °C and stirred for 16 h. LCMS analysis showed that the reaction was complete. After cooling, the mixed reaction solution was concentrated under reduced pressure. The residue was purified by C18 reverse phase column chromatography (CAN:$H_2O$ (0.1% $NH_3.H_2O$) = 5%-95% elution) to give (*Z*)-2-((4-(((*R*)-3,4-dimethylpiperazin-1-yl)methyl)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (10 mg, yield: 18.1%), LC-MS *m/z:* 555 [M + H]$^+$;

$^1$H NMR (400 MHz, MeOD-d4) δ 8.84 (s, 1H), 7.97 (t, *J* = 7.9 Hz, 1H), 7.84 (d, *J* = 7.7 Hz, 1H), 7.67 - 7.57 (m, 2H), 7.41 (d, *J* = 8.4 Hz, 1H), 7.16 (d, *J* = 8.2 Hz, 1H), 5.55 - 5.37 (m, 2H), 4.87 (s, 1H), 4.83 - 4.80 (m, 1H), 4.61 (d, *J* = 6.9 Hz, 2H), 3.46 (s, 2H), 2.91 - 2.70 (m, 4H), 2.36 (s, 6H), 2.20 - 2.14 (m, 1H), 1.97 (d, *J* = 26.6 Hz, 4H), 1.69 (s, 3H), 1.08 (d, *J* = 6.3 Hz, 3H).

**Example 41: Synthesis of (Z)-12-hydroxy-12-methyl-2-((3-methyl-4-((4-methyl-2-oxopiperazin-1-yl)methyl)phenyl)amino)-7,10,11,12-tetrahydro-5H-13,17-(azeno)pyrimido[4',5':3,4]pyrazolo[1,2-*a*][1,2]diazacyclotridecin-5-one (Compound 199)**

**1) Synthesis of 4-methyl-1-(2-methyl-4-nitrobenzyl)piperazin-2-one**

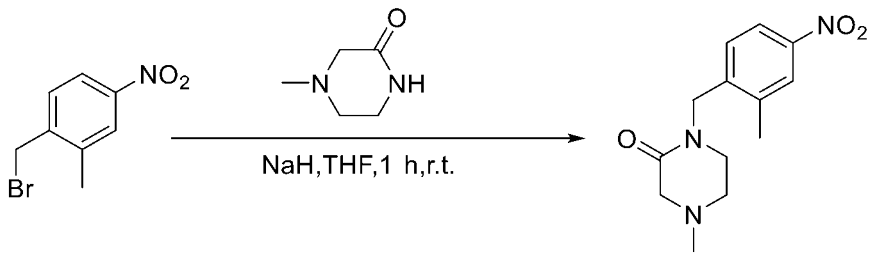


Sodium hydride (175 mg, 4.39 mmol, content: 60%) was added in batches to a solution of 4-methylpiperazin-2-one (300 mg, 2.63 mmol) in tetrahydrofuran (10 mL) at 0 °C. The resulting mixed reaction solution was stirred at room temperature for 1 h. 1-(Bromomethyl)-2-methyl-4-nitrobenzene (500 mg, 2.18 mmol) was added to the above mixed reaction solution at room temperature. The resulting mixed reaction solution was stirred overnight at room temperature. The mixed reaction solution was quenched with water (20 mL) and extracted with ethyl acetate (3 × 20 mL) at 0 °C. The combined organic phases were washed with saturated brine (1 × 20 mL), dried over anhydrous sodium sulfate, filtered, and concentrated under reduced pressure. The residue was purified by C18 reverse phase column chromatography ($CH_3CN$:$H_2O$ (0.1% $NH_3.H_2O$) = 5%-95% elution) to give 4-methyl-1-(2-methyl-4-nitrobenzyl)piperazin-2-one (50 mg,

yield: 8.7%), LC-MS m/z: 264 [M + H]+;

**2) Synthesis of 1-(4-amino-2-methylbenzyl)-4-methylpiperazin-2-one**

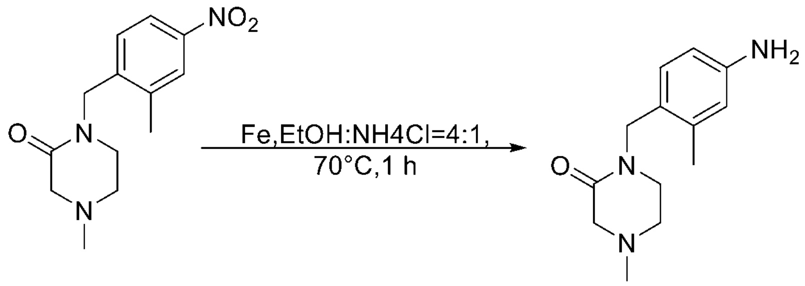


A solution of 4-methyl-1-(2-methyl-4-nitrobenzyl)piperazin-2-one (50 mg, 0.19 mmol) and Fe (53 mg, 0.95 mmol) in aqueous ammonium chloride solution (0.2 mL) and ethanol (0.8 mL) was heated to 70 °C and stirred for 2 h. After cooling, the mixed reaction solution was filtered. The filter cake was washed with methanol (3 × 10 mL). The filtrate was collected and concentrated under reduced pressure to give a crude product, which was used directly in the next step without further purification. LC-MS *m/z:* 234 [M + H]+;

**3) Synthesis of (Z)-12-hydroxy-12-methyl-2-((3-methyl-4-((4-methyl-2-oxopiperazin-1-yl)methyl)phenyl)amino)-7,10,11,12-tetrahydro-5H-13,17-(azeno)pyrimido[4',5':3,4]pyrazolo[1,2-*a*][1,2]diazacyclotridecin-5-one**

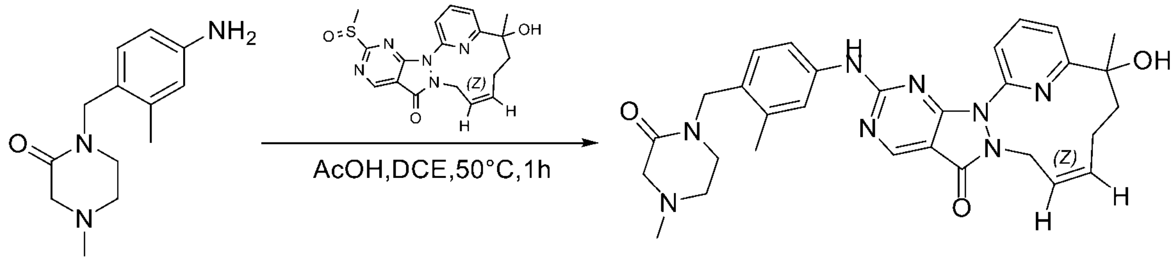


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (70 mg, 0.18 mmol), 1-(4-amino-2-methylbenzyl)-4-methylpiperazin-2-one (50 mg, 0.21 mmol), and acetic acid (64.8 mg, 1.08 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN:H_2O$ (0.1% $NH_3.H_2O$) = 5%-95% elution) to give (*Z*)-12-hydroxy-12-methyl-2-((3-methyl-4-((4-methyl-2-oxopiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (30 mg, yield: 31%), LC-MS *m/z:* 555 [M + H]+;

$^1$H NMR (400 MHz, MeOD-d4): δ 8.84 (s, 1H), 7.98 (t, *J* = 8.0 Hz, 1H), 7.83 (d, *J* = 8.0 Hz, 1H), 7.69 (s, 1H), 7.61 (d, *J* = 7.6 Hz, 1H), 7.47-7.42 (m, 1H), 7.11 (d, *J* = 8.4 Hz, 1H), 5.53-5.40 (m, 2H), 4.64 (s, 2H), 4.62 (d, *J* = 6.4 Hz, 2H), 3.22 (t, *J* = 5.6 Hz, 2H), 3.18 (s, 2H), 2.68 (t, *J* = 5.2 Hz, 2H), 2.35 (s, 3H), 2.28 (s, 3H), 2.23-2.10 (m, 2H), 2.03-1.96 (m, 1H), 1.93-1.85 (m, 1H), 1.69(s, 3H).

**Example 42: Synthesis of (*Z*)-*N*-(2-(dimethylamino)ethyl)-4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylbenzamide (Compound 200)**

**1) Synthesis of *N*-(2-(dimethylamino)ethyl)-2-methyl-4-nitrobenzamide**

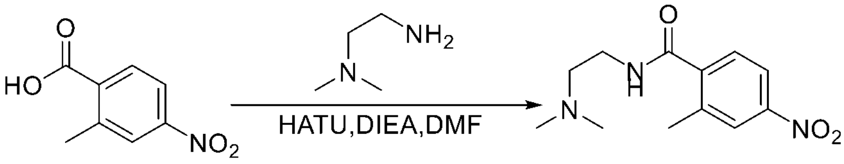


A solution of 2-methyl-4-nitrobenzoic acid (100 mg, 0.55 mmol), *N*[1],*N*[1]-dimethylethane-1,2-diamine (58.3 mg, 0.66 mmol), HATU (313.5 mg, 0.83 mmol), and DIEA (213 mg, 1.65 mmol) in *N,N*-dimethylformamide (2 mL) was stirred at room temperature for 2 h. The mixed reaction solution was purified by C18 reverse phase column chromatography ($CH_3CN$:$H_2O$ (0.1% $NH_3.H_2O$) = 5%-95% elution) to give *N*-(2-(dimethylamino)ethyl)-2-methyl-4-nitrobenzamide (100 mg, yield: 71.8%), LC-MS *m/z:* 252 $[M + H]^+$;

**2) Synthesis of 4-amino-*N*-(2-(dimethylamino)ethyl)-2-methylbenzamide**

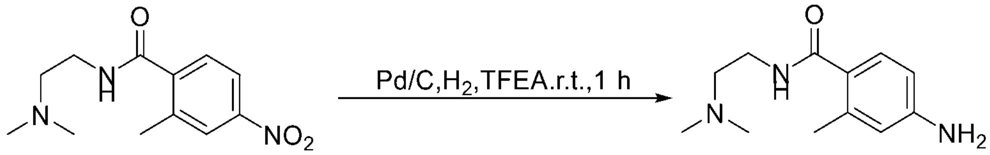


A solution of *N*-(2-(dimethylamino)ethyl)-2-methyl-4-nitrobenzamide (100 mg, 0.40 mmol) and Pd/C (50 mg, 10%) in 2,2,2-trifluoroethanol (2 mL) was stirred at room temperature for 1 h under a hydrogen atmosphere. The mixed reaction solution was filtered, and the filter cake was washed with 2,2,2-trifluoroethanol (3 × 10 mL). The filtrate was collected and concentrated under reduced pressure to give a crude product, which was used directly in the next step without further purification. LC-MS *m/z*: 222 $[M + H]^+$;

**3) Synthesis of (*Z*)-*N*-(2-(dimethylamino)ethyl)-4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylbenzamide**

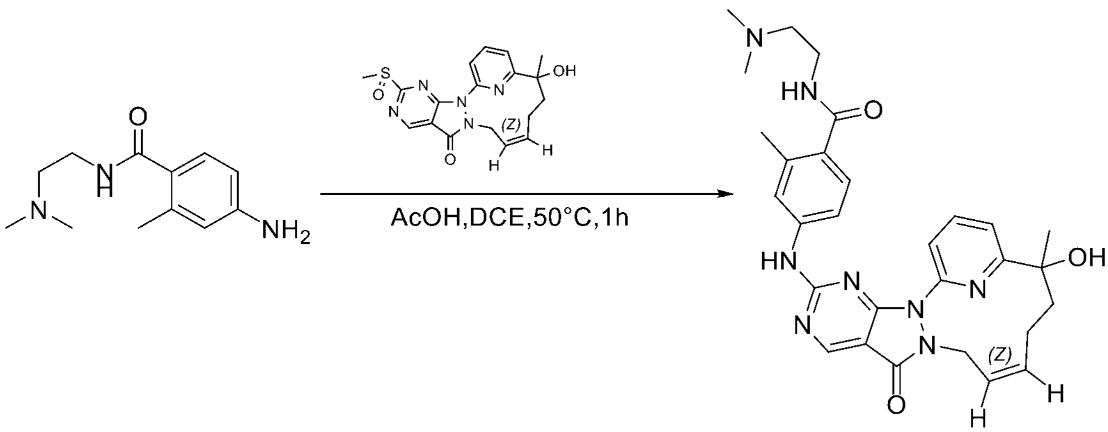


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (80 mg, 0.21 mmol), 4-amino-N-(2-(dimethylamino)ethyl)-2-methylbenzamide (55.7 mg, 0.25 mmol), and acetic acid (90 mg, 1.5 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give (*Z*)-*N*-(2-(dimethylamino)ethyl)-4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylbenzamide (6.5 mg, yield: 5.8%), LC-MS *m/z:* 543 $[M + H]^+$;

$^1$H NMR (400 MHz, MeOD-d4): δ 8.90-8.85 (m, 1H), 8.00 (t, *J* = 8.4 Hz, 1H), 7.84 (d, *J* = 7.6 Hz, 1H), 7.74 (s, 1H), 7.62 (d, *J* = 8.0 Hz, 1H), 7.57-7.51 (m, 1H), 7.37 (d, *J* = 8.4 Hz, 1H), 5.53-5.40 (m, 2H),4.63 (d, *J* = 6.0 Hz, 2H), 3.50 (t, *J* = 6.8 Hz, 2H),

2.65-2.56 (m, 2H), 2.41 (s, 3H), 2.34 (s, 6H), 2.29-2.20 (m, 1H), 2.19-2.10 (m, 1H), 2.05-1.96 (m, 1H), 1.94-1.83 (m, 1H), 1.69 (s, 3H).

**Example 43: Synthesis of (*R*,*Z*)-12-hydroxy-12-methyl-2-((3-methyl-4-((9-methyl-3,9-diazaspiro[5.5]undecan-3-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound 201)**

**1) Synthesis of 3-methyl-9-(2-methyl-4-nitrobenzyl)-3,9-diazaspiro[5.5]undecane**

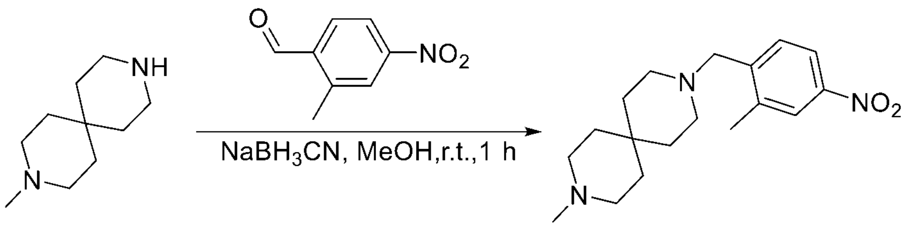


A solution of 3-methyl-3,9-diazaspiro[5.5]undecane (0.2 g, 1.19 mmol), 2-methyl-4-nitrobenzaldehyde (0.20 g, 1.19 mmol), and sodium cyanoborohydride (0.075 g, 1.19 mol) in methanol (2 mL) was stirred at room temperature for 1 h. The resulting mixed reaction solution was concentrated under reduced pressure, and the residue was purified by silica gel column chromatography (ethyl acetate:petroleum ether = 3:1 elution) to give 3-methyl-9-(2-methyl-4-nitrobenzyl)-3,9-diazaspiro[5.5]undecane (0.2 g, yield: 53.01%), LCMS m/z: 318 $[M + H]^+$.

**2) Synthesis of 3-methyl-4-((9-methyl-3,9-diazaspiro[5.5]undecan-3-yl)methyl)aniline**

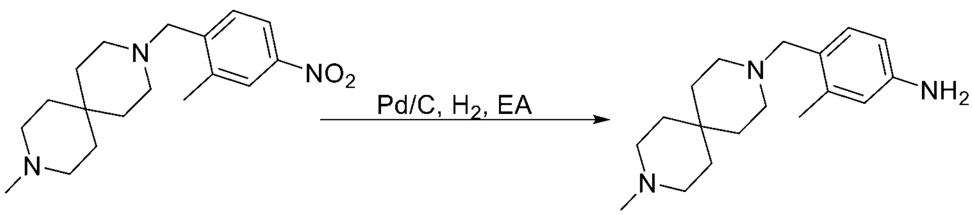


A solution of 3-methyl-9-(2-methyl-4-nitrobenzyl)-3,9-diazaspiro[5.5]undecane (0.05 g, 0.16 mmol) and Pd/C (0.017 g) in ethyl acetate (0.5 mL) were stirred at room temperature for 1 h under a hydrogen atmosphere. The mixed reaction solution was filtered, and the filter cake was washed with ethyl acetate (0.5 mL × 3). The filtrate was collected and concentrated under reduced pressure to give a crude product (0.03 g), which was used directly in the next step. LCMS *m/z:* 288 $[M + H]^+$.

**3) Synthesis of (*R,Z*)-12-hydroxy-12-methyl-2-((3-methyl-4-((9-methyl-3,9-diazaspiro[5.5]undecan-3-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

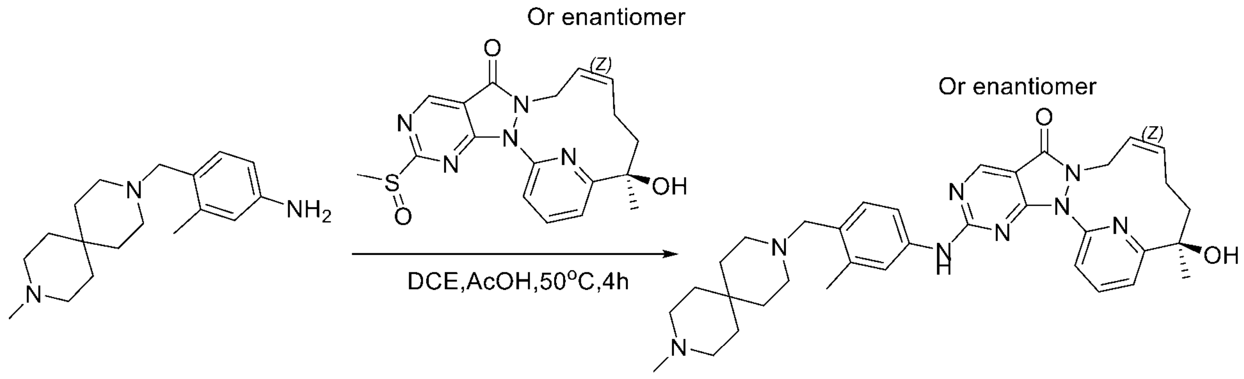


A solution of (12*R*,Z)-12-hydroxy-12-methyl-2-(methylsulfinyl)-7,10,11,12-tetrahydro-5*H*-13,17-(azeno)pyrimido[4',5':3,4]pyrazolo[1,2-*a*][1,2]diazacyclotridecin-5-one or enantiomer (0.1 g, 0.26 mmol), 3-methyl-4-((9-methyl-3,9-diazaspiro[5.5]undecan-3-yl)methyl)aniline (0.090 g, 0.31 mmol), and acetic acid (18.6 mg, 0.31 mmol) in 1,2-dichloroethane (1 mL) was heated to 50 °C and stirred for 2 h. The mixed reaction solution was concentrated under reduced pressure, and the residue was purified by silica gel column chromatography (methanol:dichloromethane = 0%-10% elution) to give (*R,Z*)-12-hydroxy-12-methyl-2-((3-methyl-4-((9-methyl-3,9-diazaspiro[5.5]undecan-3-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (0.02 g, yield: 12.66%), LCMS m/z: 609 [M + H]$^+$;
$^1$H NMR (400 MHz, MeOH-d4): δ 8.86 (s, 1H), 8.52 (s, 1H), 7.98 (t, *J* = 7.6 Hz, 1H), 7.84 (d, J = 7.6 Hz, 1H), 7.72 (s, 1H), 7.62 (d, *J* = 7.6 Hz, 1H), 7.51 (d, *J* = 7.6 Hz, 1H), 7.27 (d, *J* = 8.4 Hz, 1H), 5.49 - 5.40 (m, 2H), 4.62 (s, 2H), 3.84 (s, 2H), 3.09 (s, 4H), 2.82 (s, 4H), 2.76 (s, 3H), 2.40 (s, 3H), 2.17 (s, 2H), 2.01 (s, 1H), 1.90 (s, 1H), 1.76 (s, 4H), 1.69 (s, 7H).

**Example 44: Synthesis of (*Z*)-2-((4-(((2*S*)-1,2-dimethylazetidin-3-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 202)**

**1) Synthesis of *tert*-butyl (2*S*)-2-methyl-3-(2-methyl-4-nitrophenoxy)azetidine-1-carboxylate**

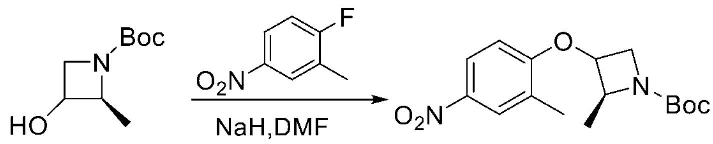


Sodium hydride (125 mg, 3.13 mmol) was added in batches to a solution of *tert*-butyl (2*S*)-3-hydroxy-2-methylazetidine-1-carboxylate (482 mg, 2.57 mmol) in *N,N*-dimethylformamide (5 mL) at 0 °C. The resulting mixed reaction solution was stirred at room temperature for 0.5 h. Then, 1-fluoro-2-methyl-4-nitrobenzene (400 mg, 2.57 mmol) was added to the above mixed reaction solution at 0 °C. The resulting mixed reaction solution was stirred at 0 °C for 3 h. At 0 °C, the mixed reaction solution was quenched with a saturated aqueous ammonium chloride solution (50 mL) and extracted with ethyl acetate (3 × 200 mL). The combined organic phases were washed with saturated brine (1 × 100 mL), dried over anhydrous sodium sulfate, filtered, and concentrated under reduced pressure. The residue was purified by silica gel column chromatography (ethyl acetate:petroleum ether = 0%-30% elution) to give *tert*-butyl (2*S*)-2-methyl-3-(2-methyl-4-nitrophenoxy)azetidine-1-carboxylate (450 mg, yield: 54%), LC-MS *m/z:* 323 [M + H]$^+$;

**2) Synthesis of (2*S*)-2-methyl-3-(2-methyl-4-nitrophenoxy)azetidine**

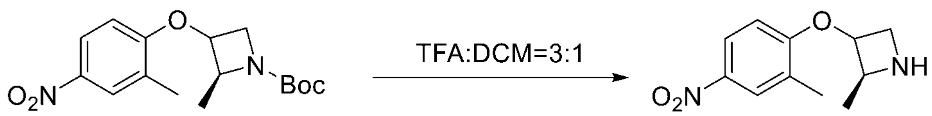


A solution of *tert*-butyl (2*S*)-2-methyl-3-(2-methyl-4-nitrophenoxy)azetidine-1-carboxylate (467 mg, 1.45 mmol)

in dichloromethane (3 mL) and 2,2,2-trifluoroacetic acid (1 mL) was stirred at room temperature for 3 h. The resulting mixed reaction solution was concentrated under reduced pressure. The residue was purified by silica gel column chromatography (methanol:dichloromethane = 0%-10% elution) to give (2*S*)-2-methyl-3-(2-methyl-4-nitrophenoxy)azetidine (300 mg, yield: 93%), LC-MS *m/z:* 223 [M + H]$^+$;

**3) Synthesis of (2*S*)-1,2-dimethyl-3-(2-methyl-4-nitrophenoxy)azetidine**

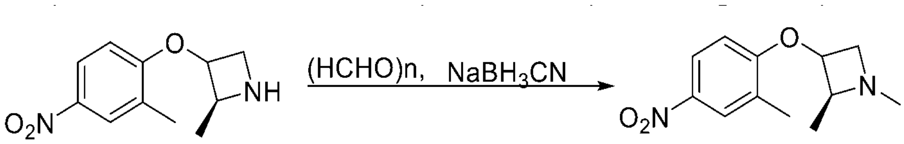


A solution of (2*S*)-2-methyl-3-(2-methyl-4-nitrophenoxy)azetidine (322 mg, 1.45 mmol), $NaBH_3CN$ (182 mg, 2.9 mmol), and paraformaldehyde (43 mg, 1.45 mmol) in acetic acid (0.3 mL) and dichloromethane (3 mL) was stirred at room temperature for 1 h. The resulting mixed reaction solution was concentrated under reduced pressure. The residue was purified by silica gel column chromatography (methanol:dichloromethane = 0%-10% elution) to give (2S)-1,2-dimethyl-3-(2-methyl-4-nitrophenoxy)azetidine (282 mg, yield: 82%), LC-MS *m*/*z*: 237 [M + H]$^+$;

**4) Synthesis of 4-(((2*S*)-1,2-dimethylazetidin-3-yl)oxy)-3-methylaniline**

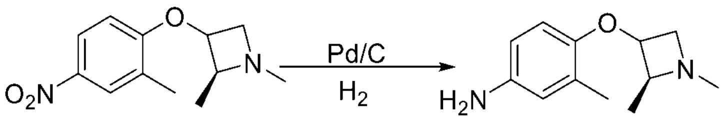


A solution of (2*S*)-1,2-dimethyl-3-(2-methyl-4-nitrophenoxy)azetidine (272 mg, 1.15 mmol) and Pd/C (140 mg) in methanol (3 mL) was stirred at room temperature for 3 h under a hydrogen atmosphere. The mixed reaction solution was filtered, and the filter cake was washed with methanol (3 × 20 mL). The filtrate was collected and concentrated under reduced pressure to give 4-(((2*S*)-1,2-dimethylazetidin-3-yl)oxy)-3-methylaniline (222 mg, yield: 93.4%), LC-MS *m/z:* 207 [M + H]$^+$;

**5) Synthesis of (*Z*)-2-((4-(((2*S*)-1,2-dimethylazetidin-3-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

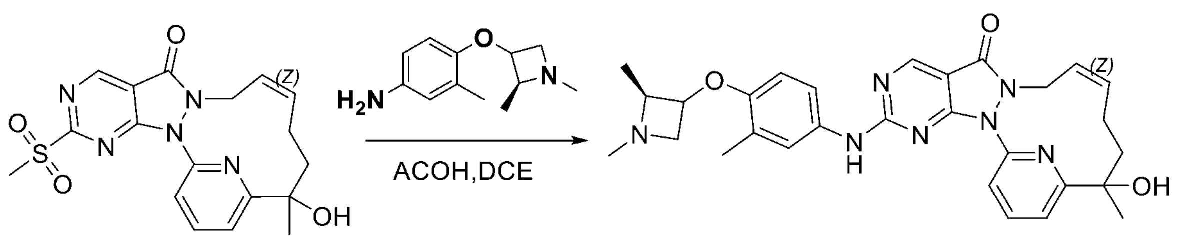


A solution of (Z)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol), 4-(((2*S*)-1,2-dimethylazetidin-3-yl)oxy)-3-methylaniline (77 mg, 0.38 mmol), and acetic acid (90 mg, 1.5 mmol) in 1,2-dichloroethane (2 mL) was heated to 50 °C and stirred for 16 h. The resulting mixed reaction solution was concentrated under reduced pressure. The residue was purified by C18 reverse phase column chromatography ($CH_3CN:H_2O$ (0.1% FA) = 30%-70% elution) to give (*Z*)-2-((4-(((2*S*)-1,2-dimethylazetidin-3-yl)oxy)-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (35 mg, yield: 26.6%), LC-MS *m/z:* 528 [M + H]$^+$;
$^1$H NMR (400 MHz, MeOD-d4) δ 8.79 (s, 1H), 7.95 (t, J = 7.9 Hz, 1H), 7.79 (d, J = 8.0 Hz, 1H), 7.60 (t, J = 7.4 Hz, 2H), 7.42 - 7.35 (m, 1H), 6.61 (d, J = 8.8 Hz, 1H), 5.53 - 5.38 (m, 2H), 5.10 (td, J = 5.9, 2.9 Hz, 1H), 4.71 (dd, J = 13.3, 6.6 Hz, 1H), 4.58

(d, J = 7.0 Hz, 2H), 4.30 (dd, J = 11.4, 5.8 Hz, 1H), 4.10 (dd, J = 11.4, 2.7 Hz, 1H), 2.90 (s, 3H), 2.29 (s, 3H), 2.25 - 2.10 (m, 2H), 1.99 (dd, J = 16.8, 8.6 Hz, 1H), 1.90 (d, J = 8.6 Hz, 1H), 1.68 (s, 3H), 1.55 (d, J = 6.9 Hz, 3H).

**Example 45: Synthesis of (*R,Z*)-12-hydroxy-12-methyl-2-((3-methyl-4-((1-methylpiperidin-4-yl)amino)phenyl) amino)-7,10,11,12-tetrahydro-5*H*-13,17-(azeno)pyrimido[4',5':3,4]pyrazolo[1,2-*a*][1,2]diazacyclotridecin-5-one and (*S,Z*)-12-hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (Compounds 203A and B)**

**1) Synthesis of *tert*-butyl 4-((2-methyl-4-nitrophenyl)amino)piperidine-1-carboxylate**

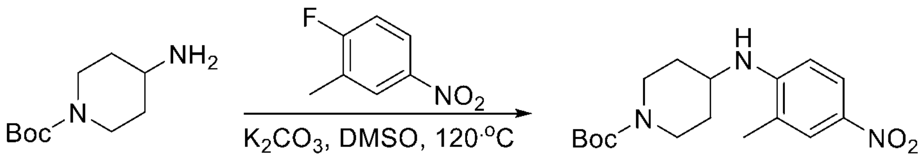


Potassium carbonate (2070 mg, 15.00 mmol) was added to a solution of *tert*-butyl 4-aminopiperidine-1-carboxylate (1000 mg, 5.00 mmol) and 1-fluoro-2-methyl-4-nitrobenzene (930 mg, 6.00 mmol) in dimethyl sulfoxide (10 mL) at room temperature. The resulting mixed reaction solution was heated to 120 °C and stirred for 8 h. After cooling, the mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give *tert*-butyl 4-((2-methyl-4-nitrophenyl)amino)piperidine-1-carboxylate (900 mg, yield: 53.6%), LC-MS *m/z:* 336 [M + H]$^+$;

**2) Synthesis of *N*-(2-methyl-4-nitrophenyl)piperidin-4-amine**

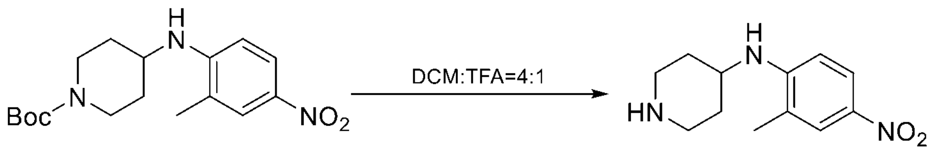


A solution of *tert*-butyl 4-((2-methyl-4-nitrophenyl)amino)piperidine-1-carboxylate (900 mg, 2.69 mmol) in dichloromethane (8 mL) and 2,2,2-trifluoroacetic acid (2 mL) was stirred at room temperature for 8 h. The mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give *N*-(2-methyl-4-nitrophenyl)piperidin-4-amine (562 mg, yield: 88.9%), LC-MS *m/z:* 236 [M + H]$^+$;

**3) Synthesis of 1-methyl-*N*-(2-methyl-4-nitrophenyl)piperidin-4-amine**

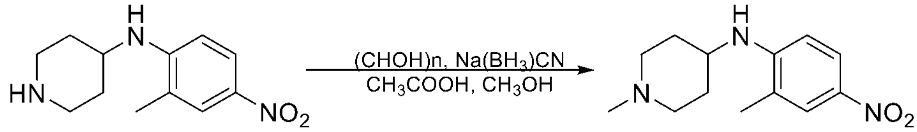


Paraformaldehyde (231 mg, 7.69 mmol) was added in batches to a solution of *N*-(2-methyl-4-nitrophenyl) piperidin-4-amine (800 mg, 3.285 mmol) and acetic acid (462 mg, 7.69 mmol) in methanol (8 mL) at 0 °C, and the mixture was stirred at room temperature for 1 h. Then, sodium cyanoborohydride (485 mg, 7.69 mmol) was added to the above mixed reaction solution, and the mixture was stirred at room temperature for another 1 h. The mixed reaction solution was

diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 1-methyl-*N*-(2-methyl-4-nitrophenyl)piperidin-4-amine (700 mg, yield: 82.6%), LC-MS *m/z*: 250 $[M + H]^+$;

**4) Synthesis of 2-methyl-*N*1-(1-methylpiperidin-4-yl)benzene-1,4-diamine**

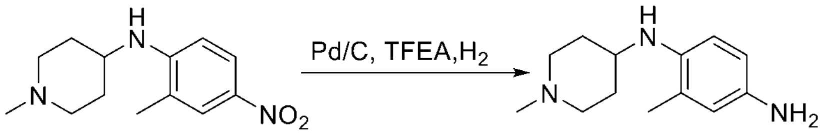


A solution of methyl-*N*-(2-methyl-4-nitrophenyl)piperidin-4-amine (700 mg, 2.81 mmol) and palladium hydroxide (787 mg, 5.62 mmol) in 2,2,2-trifluoroacetic acid (7 mL) was stirred at room temperature for 2 h under a hydrogen atmosphere. The mixed reaction solution was filtered, and the filter cake was washed with methanol. The filtrate was collected and concentrated under reduced pressure to give 2-methyl-*N*1-(1-methylpiperidin-4-yl)benzene-1,4-diamine (600 mg, yield: 97.4%), LC-MS *m/z:* 220 $[M + H]^+$;

**5) Synthesis of (*Z*)-12-hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one**

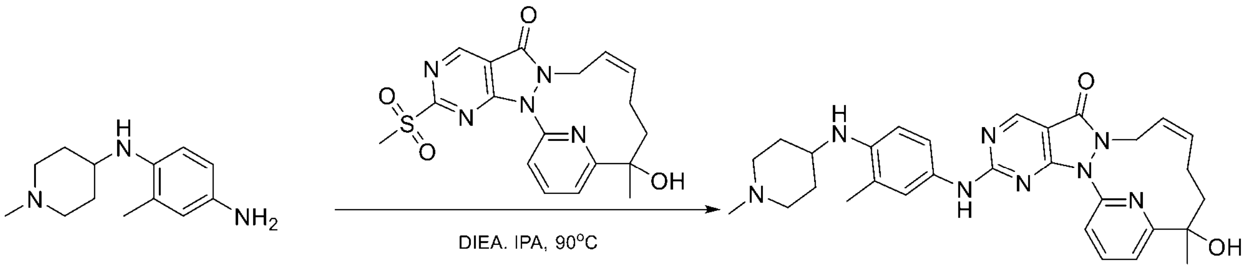


**Compound 203**

N,N-Diisopropylethylamine (177 mg, 1.37 mmol) was added to a solution of 2-methyl-*N*1-(1-methylpiperidin-4-yl) benzene-1,4-diamine (100 mg, 0.46 mmol) and (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (275 mg, 0.68 mmol) in isopropanol (2 mL) at room temperature. The resulting mixed reaction solution was heated to 90 °C and stirred for 2 h. After cooling, the mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (*Z*)-12-hydroxy-12-methyl-2-((3-methyl-4-((1-methylpiperidin-4-yl)amino)phenyl) amino)-7,10,11,12-tetrahydro-5*H*-13,17-(azeno)pyrimido[4',5':3,4]pyrazolo[1,2-*a*][1,2]diazacyclotridecin-5-one (60 mg, yield: 24.3%), LC-MS *m/z:* 541 $[M + H]^+$;
$^1$H NMR (400 MHz, MeOD-d4) δ 8.75 (s, 1H), 7.92 (t, J = 7.9 Hz, 1H), 7.82 (d, J = 8.0 Hz, 1H), 7.57 (d, J = 7.6 Hz, 1H), 7.44 (s, 1H), 7.25 (dd, J = 8.6, 2.5 Hz, 1H), 6.63 (d, J = 8.7 Hz, 1H), 5.56 - 5.40 (m, 2H), 4.56 (d, J = 6.9 Hz, 2H), 3.37 (d, J = 10.0 Hz, 1H), 2.92 (d, J = 11.6 Hz, 2H), 2.35 (s, 3H), 2.32 - 2.13 (m, 7H), 2.07 (d, J = 11.8 Hz, 2H), 2.01 - 1.94 (m, 1H), 1.93 - 1.86 (m, 1H), 1.67 (s, 3H), 1.56 (dd, J = 21.4, 10.1 Hz, 2H).

**6) Synthesis of (*R,Z*)-12-hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer or (*S,Z*)-12-hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

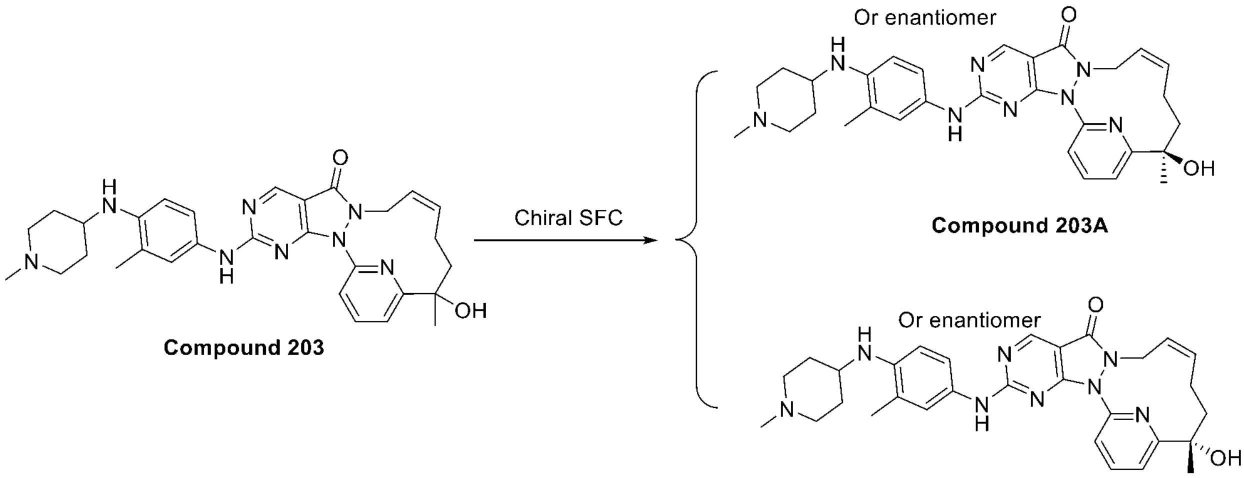


Compound 203

Compound 203A

Compound 203B

(*Z*)-12-Hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (118 mg, 0.22 mmol) was separated and purified by preparative chiral high performance liquid chromatography (system: SHIMADZU LC-20AP; Column name: DAICELCHIRALPAK®IG; 250 × 25 mm 10 μm; mobile phase A: *n*-hexane; mobile phase B: EtOH (+ 0.1% 7.0 mol/L ammonia in MeOH); A:B: = 50:50; detection wavelength: 254 nm; flow rate: 60; column temperature: RT; column pressure: 100 bar) to give two isomers: **compound 203A** and **compound 203B:**

> **Compound 203A: Peak 1 (peak time: 18.865 min);** (*R,Z*)-12-hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (20 mg, yield: 17%), LC-MS *m/z:* 541 [M + H]$^+$;
> $^1$H NMR (400 MHz, MeOD-d4) δ 8.74 (s, 1H), 7.92 (t, J = 7.9 Hz, 1H), 7.81 (d, J = 8.0 Hz, 1H), 7.56 (d, J = 7.6 Hz, 1H), 7.44 (s, 1H), 7.24 (dd, J = 8.6, 2.4 Hz, 1H), 6.63 (d, J = 8.7 Hz, 1H), 5.54 - 5.41 (m, 2H), 4.55 (d, J = 6.8 Hz, 2H), 3.42 - 3.33 (m, 1H), 2.94 (d, J = 11.7 Hz, 2H), 2.36 (s, 3H), 2.31 (t, J = 11.8 Hz, 2H), 2.22 - 2.16 (m, 1H), 2.15 - 2.04 (m, 6H), 2.02 - 1.94 (m, 1H), 1.93 - 1.85 (m, 1H), 1.67 (s, 3H), 1.56 (dd, J = 21.6, 10.1 Hz, 2H).

**Compound 203B, peak 2 (peak time: 26.148 min); (*S,Z*)-12-hydroxy-12-methyl-2-((3-methyl-4-((1-methylpiperidin-4-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer** (20 mg, yield: 17%)), LC-MS *m/z*: 541 [M + H]$^+$;
$^1$H NMR (400 MHz, MeOD-d4) δ 8.74 (s, 1H), 7.92 (t, J = 7.9 Hz, 1H), 7.81 (d, J = 8.1 Hz, 1H), 7.56 (d, J = 7.6 Hz, 1H), 7.44 (s, 1H), 7.24 (dd, J = 8.6, 2.4 Hz, 1H), 6.63 (d, J = 8.7 Hz, 1H), 5.55 - 5.40 (m, 2H), 4.55 (d, J = 6.8 Hz, 2H), 3.42 - 3.36 (m, 1H), 2.93 (d, J = 11.7 Hz, 2H), 2.35 (s, 3H), 2.29 (t, J = 11.1 Hz, 2H), 2.19 (dd, J = 9.6, 5.3 Hz, 1H), 2.16 - 2.03 (m, 6H), 2.02 - 1.85 (m, 2H), 1.67 (s, 3H), 1.62 - 1.50 (m, 2H).

**Example 46: (*R,Z*)-12-hydroxy-12-methyl-2-((3-methyl-4-((1-methylazetidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (compound 204)**

**1) Synthesis of *tert*-butyl 3-(2-methyl-4-nitrophenoxy)azetidine-1-carboxylate**

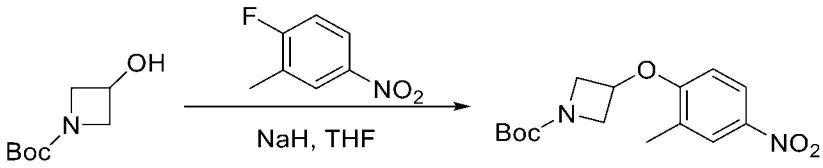


Sodium hydride (1110 mg, 46.24 mmol) was added in batches to a solution of *tert*-butyl 3-hydroxyazetidine-1-carboxylate (4000 mg, 23.14 mmol) in tetrahydrofuran (40 mL) at 0 °C, and the resulting mixed reaction solution was stirred at room temperature for 50 min. Then, a solution of 1-fluoro-2-methyl-4-nitrobenzene (5376 mg, 34.68 mmol) in tetrahydrofuran (10 mL) was added dropwise to the above mixed reaction solution. The resulting mixed reaction solution was heated to 90 °C and stirred for 8 h. After cooling, the mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give *tert*-butyl 3-(2-methyl-4-nitrophenoxy)azetidine-1-carboxylate (3800 g, yield: 53%), LC-MS *m/z*: 309 $[M + H]^+$;

**2) Synthesis of 3-(2-methyl-4-nitrophenoxy)azetidine**

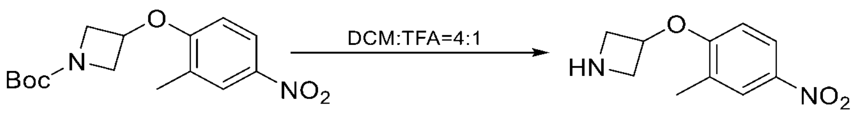


A solution of *tert*-butyl 3-(2-methyl-4-nitrophenoxy)azetidine-1-carboxylate (3800 mg, 12.34 mmol) in dichloromethane (32 mL) and 2,2,2-trifluoroacetic acid (8 mL) was stirred at room temperature for 8 h. The mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 3-(2-methyl-4-nitrophenoxy)azetidine (2300 mg, yield: 89.5%), LC-MS *m/z:* 209 $[M + H]^+$;

**3) Synthesis of 1-methyl-3-(2-methyl-4-nitrophenoxy)azetidine**

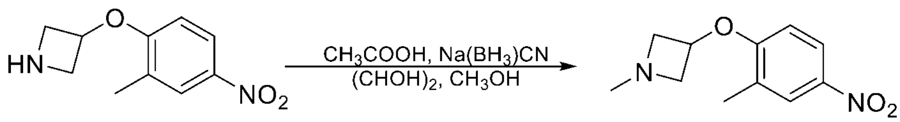


Paraformaldehyde (981 mg, 32.69 mmol) was added in batches to a solution of 3-(2-methyl-4-nitrophenoxy) azetidine (3400 mg, 16.35 mmol) and acetic acid (1471 mg, 24.52 mmol) in methanol (30 mL) at 0 °C. The resulting mixed reaction solution was stirred at room temperature for 1 h. Then, sodium cyanoborohydride (3089 mg, 49.04 mmol) was added in batches. The resulting mixed reaction solution was stirred at room temperature for 1 h. The mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 1-methyl-3-(2-methyl-4-nitrophenoxy)azetidine (3200 mg, yield: 88%), LC-MS *m/z:* 223 $[M + H]^+$;

**4) Synthesis of 3-methyl-4-((1-methylazetidin-3-yl)oxy)aniline**

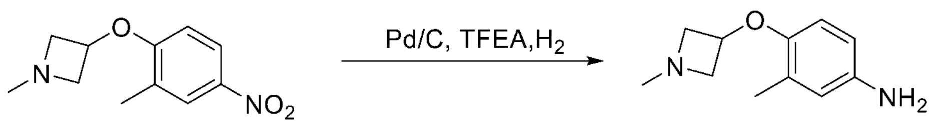

A solution of 1-methyl-3-(2-methyl-4-nitrophenoxy)azetidine (3200 mg, 14.41 mmol) and palladium hydroxide (4036 mg, 28.83 mmol) in 2,2,2-trifluoroethanol (30 mL) was stirred at room temperature for 8 h under a hydrogen atmosphere. The mixed reaction solution was filtered, and the filter cake was washed with methanol. The filtrate was collected and concentrated under reduced pressure to give 3-methyl-4-((1-methylazetidin-3-yl)oxy)aniline (2596 mg, yield: 93.75%), LC-MS *m/z:* 193 [M + H]$^+$;

**5) Synthesis of (*R,Z*)-12-hydroxy-12-methyl-2-((3-methyl-4-((1-methylazetidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

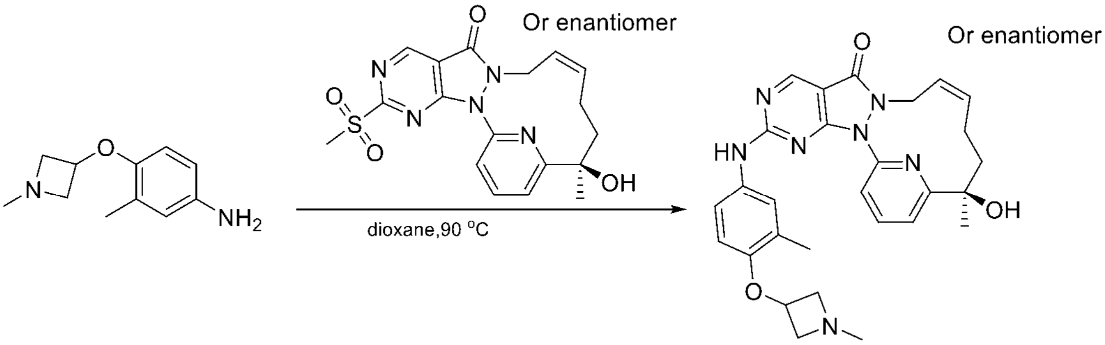


A solution of 3-methyl-4-((1-methylazetidin-3-yl)oxy)aniline (100 mg, 0.52 mmol) and (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (251 mg, 0.63 mmol) in 1,4-dioxane (2 mL) was heated to 90 °C and stirred for 2 h. After cooling, the mixed reaction solution was diluted with water and extracted with ethyl acetate (3 × 20 mL). The combined organic phases were washed with saturated brine (1 × 30 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (*R,Z*)-12-hydroxy-12-methyl-2-((3-methyl-4-((1-methylazetidin-3-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (60 mg, yield: 22.5%), LC-MS *m/z:* 514 [M + H]$^+$;

$^1$H NMR (400 MHz, MeOD-d4) δ 8.77 (s, 1H), 7.94 (t, *J* = 7.9 Hz, 1H), 7.78 (d, *J* = 8.0 Hz, 1H), 7.59 (d, *J* = 7.7 Hz, 2H), 7.35 (d, *J* = 7.7 Hz, 1H), 6.56 (d, *J* = 8.7 Hz, 1H), 5.54 - 5.37 (m, 2H), 5.04 - 4.94 (m, 1H), 4.57 (d, *J* = 6.9 Hz, 2H), 4.44 - 4.34 (m, 2H), 3.98 - 3.88 (m, 2H), 2.84 (s, 3H), 2.24 (s, 3H), 2.18 (d, *J* = 18.5 Hz, 2H), 2.02-1.93 (m, 1H), 1.91 (d, *J* = 11.6 Hz, 1H), 1.68 (s, 3H).

**Example 47: Synthesis of (*R,Z*)-12-hydroxy-12-methyl-2-((3-methyl-4-((1-(oxetan-3-yl)piperidin-4-yl)oxy)phenyl)amino)-7,10,11,12-tetrahydro-5H-13,17-(azeno)pyrimido[4',5':3,4]pyrazolo[1,2-*a*][1,2]diazacyclotridecin-5-one or Enantiomer (Compound 205)**

**1) Synthesis of 4-(2-methyl-4-nitrophenoxy)-1-(oxetan-3-yl)piperidine**

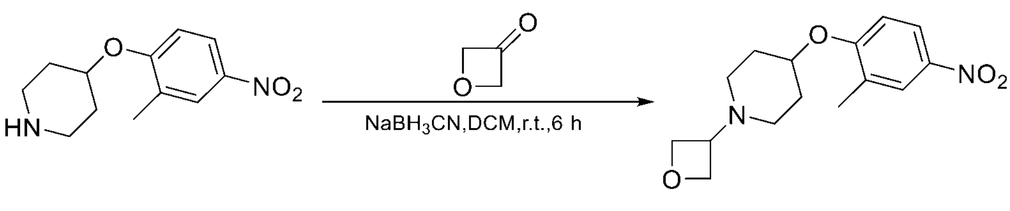


Sodium cyanoborohydride (214 mg, 3.39 mmol) was added to a solution of 4-(2-methyl-4-nitrophenoxy) piperidine (200 mg, 0.85 mmol) and oxetan-3-one (92 mg, 1.27 mmol) in dichloromethane (4 mL) at room temperature. The resulting mixed reaction solution was stirred at room temperature for 6 h. The mixed reaction solution was diluted with water and extracted with ethyl acetate (3 × 50 mL). The combined organic phases were washed with saturated brine (1 × 20 mL), dried over anhydrous sodium sulfate, filtered, and concentrated under reduced pressure. The residue was purified

by silica gel column chromatography (ethyl acetate:petroleum ether = 40%-70% elution) to give 4-(2-methyl-4-nitrophenoxy)-1-(oxetan-3-yl)piperidine (130 mg, yield: 52.5%), LC-MS *m/z:* 293 $[M + H]^+$;

**2) Synthesis of 3-methyl-4-((1-(oxetan-3-yl)piperidin-4-yl)oxy)aniline**

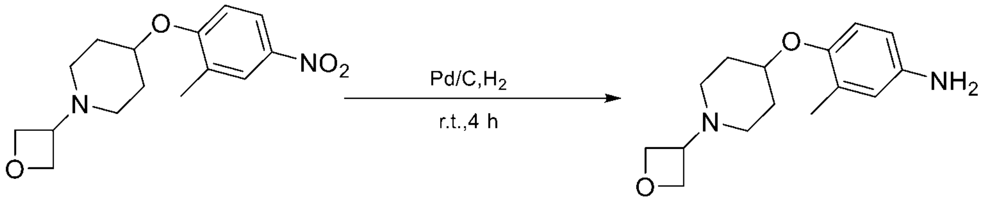


A solution of 4-(2-methyl-4-nitrophenoxy)-1-(oxetan-3-yl)piperidine (120 mg, 0.41 mmol) and Pd/C (65 mg, 0.62 mmol) in methanol (2 mL) were stirred at room temperature for 1 h under a hydrogen atmosphere. The mixed reaction solution was filtered, and the filter cake was washed with methanol. The filtrate was collected and concentrated under reduced pressure to give 3-methyl-4-((1-(oxetan-3-yl)piperidin-4-yl)oxy)aniline (90 mg, yield: 83.6%), LC-MS *m/z:* 263 $[M + H]^+$;

**3) Synthesis of (*R,Z*)-12-hydroxy-12-methyl-2-((3-methyl-4-((1-(oxetan-3-yl)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

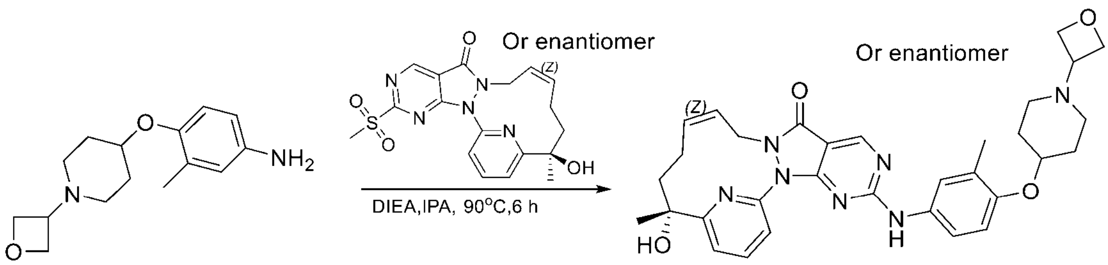


A solution of 3-methyl-4-((1-(oxetan-3-yl)piperidin-4-yl)oxy)aniline (80 mg, 0.31 mmol), *(R,Z)-12-hydroxy-12-methyl-2-(methylsulfonyl)-7,10,11,12-tetrahydro-5H-13,17*-(azeno)pyrimido[4',5':3,4]pyrazolo[1,2-*a*][1,2]diazacyclotridecin-5-one or enantiomer (147 mg, 0.37 mmol), and *N,N*-isopropylethylamine (118 mg, 0.92 mmol) in isopropanol (1.5 mL) was heated to 90 °C and stirred for 6 h. The resulting mixed reaction solution was diluted with water and extracted with ethyl acetate (3 × 50 mL). The combined organic phases were washed with saturated brine (1 × 10 mL), dried over anhydrous sodium sulfate, filtered, and concentrated under reduced pressure. The residue was purified by C18 reverse phase column chromatography (MeCN:$H_2O$ (0.1% FA) = 30%-60% elution) to give (***R***,*Z*)-12-hydroxy-12-methyl-2-((3-methyl-4-((1-(oxetan-3-yl)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (40 mg, yield: 22.5%), LC-MS *m/z:* 584$[M + H]^+$; $^1$H NMR (400 MHz, MeOD-$d_4$): 8.80 (s, 1H), 7.96 (t, J = 4.0 Hz, 1H), 7.81(d, J = 8 Hz, 1H), 7.59 (d, J = 8.0 Hz, 2H), 7.42-7.39 (m, 1H), 6.93 (d, J = 8 Hz,1H),5.52-5.42 (m, 2H),4.92-4.88 (m, 6H), 4.83-4.79 (m, 3H),4.60 (d, J = 8 Hz, 2H),4.49 (s, 1H), 3.32(s, 1H), 3.24(s, 1H),2.25 (s, 3H),2.17-1.9 (m, 6H),1.68 (s, 3H).

**Example 48: Synthesis of (*Z*)-12-hydroxy-12-methyl-2-((3-methyl-4-((1-(2,2,2-trifluoroethyl)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 206)**

**1) Synthesis of 4-(2-methyl-4-nitrophenoxy)-1-(2,2,2-trifluoroethyl)piperidine**

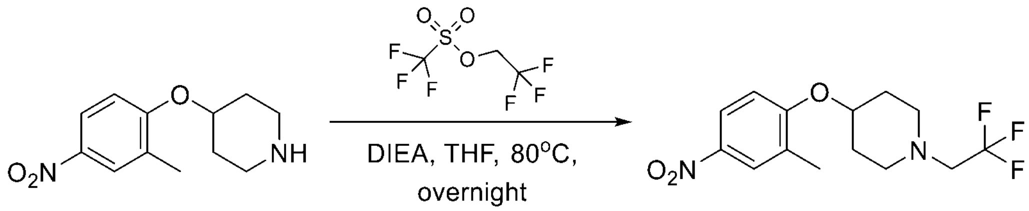


A solution of 4-(2-methyl-4-nitrophenoxy)piperidine (500 mg, 2.12 mmol), 2,2,2-trifluoroethyl trifluoromethanesulfonate (738 mg, 3.18 mmol) and *N,N*-diisopropylethylamine (822 mg, 6.36 mmol) in tetrahydrofuran (10 mL) was heated to 80 °C and stirred overnight. After cooling, the resulting mixed reaction solution was concentrated under reduced pressure. The residue was purified by C18 reverse phase column chromatography ($CH_3CN$:$H_2O$ (0.1% $NH_3 \cdot H_2O$) = 5%-95% elution) to give 4-(2-methyl-4-nitrophenoxy)-1-(2,2,2-trifluoroethyl)piperidine (400 mg, yield: 59.0%), LC-MS m/z: 319 $[M + H]^+$;

**2) Synthesis of 3-methyl-4-((1-(2,2,2-trifluoroethyl)piperidin-4-yl)oxy)aniline**

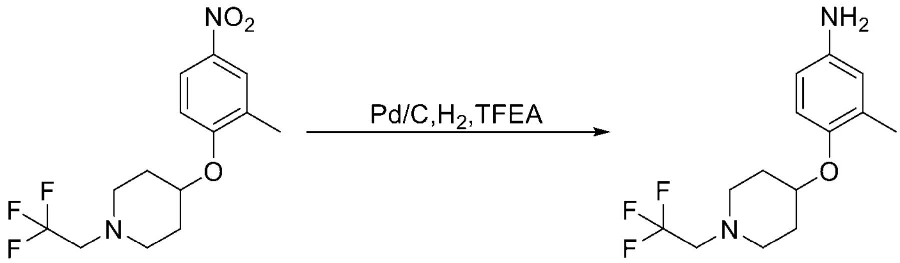


A solution of 4-(2-methyl-4-nitrophenoxy)-1-(2,2,2-trifluoroethyl)piperidine (200 mg, 0.63 mmol) and Pd/C (100 mg) in 2,2,2-trifluoroethanol (2.0 mL) was stirred at room temperature for 2 h. The mixed reaction solution was filtered, and the filter cake was washed with 2,2,2-trifluoroethanol (3 × 10 mL). The filtrate was collected and concentrated under reduced pressure to give a crude product, which was used directly in the next step without further purification. LC-MS *m/z*: 289 $[M + H]^+$;

**3) Synthesis of (*Z*)-12-hydroxy-12-methyl-2-((3-methyl-4-((1-(2,2,2-trifluoroethyl)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one**

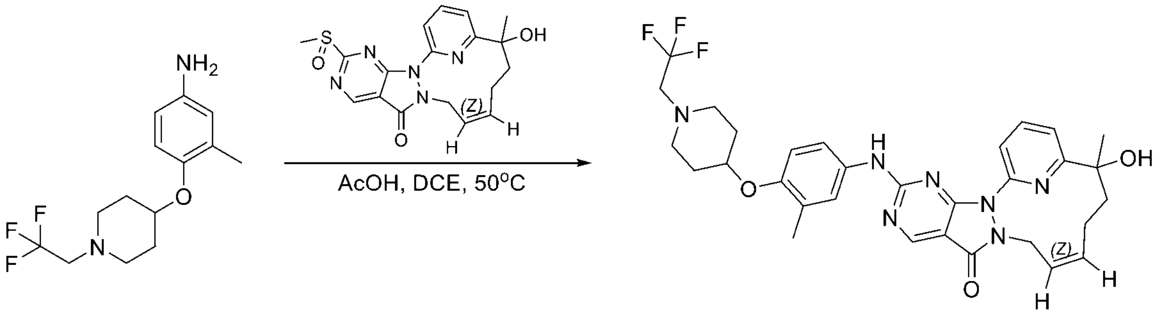


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (80 mg, 0.21 mmol), 3-methyl-4-((1-(2,2,2-trifluoroethyl)piperidin-4-yl)oxy)aniline (72 mg, 0.25 mmol), and acetic acid (90 mg, 1.5 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give (*Z*)-12-hydroxy-12-methyl-2-((3-methyl-4-((1-(2,2,2-trifluoroethyl)piperidin-4-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (40 mg, yield: 31.6%), LC-MS *m/z:* 610 $[M + H]^+$;

$^1$H NMR (400 MHz, MeOD-d4): δ 8.78 (s, 1H), 7.94 (t, *J* = 8.0 Hz, 1H), 7.80 (d, *J* = 8.0 Hz, 1H), 7.58 (d, *J* = 7.6 Hz, 2H),7.33 (dd, *J* = 8.8, 2.4Hz, 1H),6.86 (d, *J* = 8.8 Hz, 1H), 5.53-5.41 (m, 2H), 4.57 (d, *J* = 6.8 Hz, 2H), 4.43-4.35 (m, 1H), 3.22-3.11 (m, 2H), 3.02-2.92 (m, 2H),2.74-2.64 (m, 2H), 2.21 (s, 3H), 2.15-1.79 (m, 8H), 1.68 (s, 3H).

**Example 49: Synthesis of (*Z*)-12-hydroxy-12-methyl-2-((4-methyl-5-((1-methylpiperidin-4-yl)oxy)pyridin-2-yl) amino)-7,10,11,12-tetrahydro-5H-13,17-(azeno)pyrimido[4',5':3,4]pyrazolo[1,2-*a*][1,2]diazacyclotridecin-5-one (Compound 207)**

**1) Synthesis of 4-methyl-5-((1-methylpiperidin-4-yl)oxy)-2-nitropyridine**

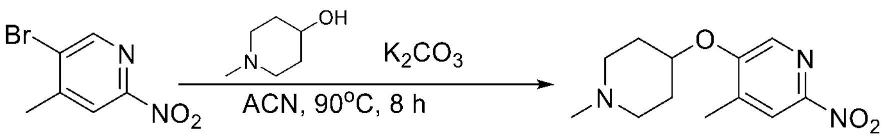


A solution of 5-bromo-4-methyl-2-nitropyridine (500.0 mg, 2.30 mmol), 1-methylpiperidin-4-ol (265.0 mg, 2.3 mmol), and potassium carbonate (954.0 mg, 6.91 mmol) in acetonitrile (5 mL) was heated to 90 °C and stirred for 8 h. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give 4-methyl-5-((1-methylpiperidin-4-yl)oxy)-2-nitropyridine (300 mg, yield: 51.2%), LC-MS *m*/*z:* 252 [M + H]$^+$;

**2) Synthesis of 4-methyl-5-((1-methylpiperidin-4-yl)oxy)pyridin-2-amine**

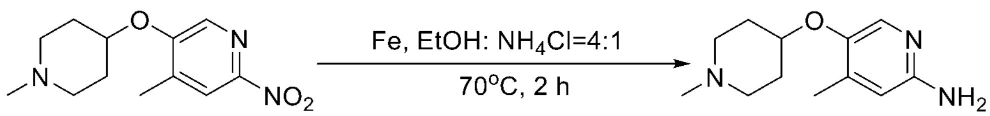


A solution of 4-methyl-5-((1-methylpiperidin-4-yl)oxy)-2-nitropyridine (300.0 mg, 1.20 mmol) and Fe (335.0 mg, 5.98 mmol) in aqueous ammonium chloride solution (1 mL) and ethanol (4 mL) was heated to 70 °C and stirred for 2 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m*/*z:* 222 [M + H]$^+$;

**3) Synthesis of (*Z*)-12-hydroxy-12-methyl-2-((4-methyl-5-((1-methylpiperidin-4-yl)oxy)pyridin-2-yl)amino)-7,10,11,12-tetrahydro-5H-13,17-(azeno)pyrimido[4',5':3,4]pyrazolo[1,2-*a*][1,2]diazacyclotridecin-5-one**

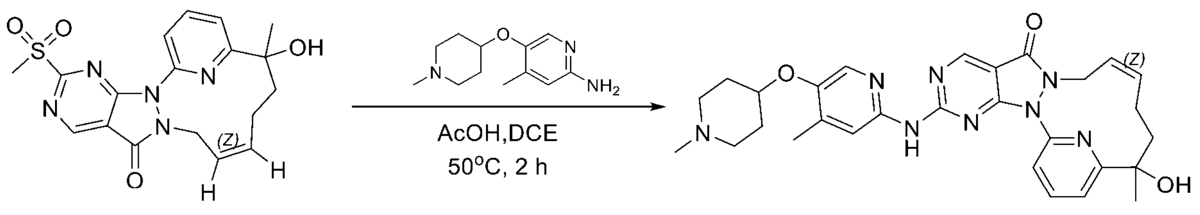


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol), 4-methyl-5-((1-methylpiperidin-4-yl)oxy) pyridin-2-amine (82.66 mg, 0.37 mmol), and acetic acid (89.8 mg, 1.50 mmol) in 1,2-dichloroethane (2 mL) was heated to 50 °C and stirred for 2 h. After cooling, the mixed reaction solution was filtered and concentrated under reduced pressure. The residue was purified by C18 reverse phase column chromatography ($CH_3CN$:$H_2O$ (0.1% $NH_3.H_2O$) = 5%-95% elution) to give (*Z*)-12-hydroxy-12-methyl-2-((4-methyl-5-((1-methylpiperidin-4-yl)oxy)pyridin-2-yl)amino)-7,10,11,12-tetrahydro-5*H*-13,17-(azeno)pyrimido[4',5':3,4]pyrazolo[1,2-*a*][1,2]diazacyclotridecin-5-one (10 mg,

yield: 7.4%), LC-MS *m/z:* 543 [M + H]+;
$^1$H NMR (400 MHz, MeOD-*d*4): δ 8.78 (s, 1H), 8.52 (s, 1H), 8.10 (s, 1H), 7.82 (s, 1H), 7.54 (d, *J* = 8.0 Hz, 1H), 6.76 (s, 1H), 5.51 - 5.36 (m, 2H), 5.20 (s, 1H), 4.58 (d, *J* = 8.0 Hz, 2H), 3.28-3.2 (m, 2H), 3.09 (s, 2H), 2.75 (s, 3H), 2.23 (s, 3H), 2.22 - 2.12 (m, 4H), 2.10-2.00 (m, 2H), 1.99 - 1.83 (m, 2H), 1.66 (s, 3H).

**Example 50: Synthesis of (*Z*)-12-hydroxy-12-methyl-2-((4-methyl-5-((1-methylpiperidin-4-yl)amino)pyridin-2-yl)amino)-7,10,11,12-tetrahydro-5*H*-13,17-(azeno)pyrimido[4',5':3,4]pyrazolo[1,2-*a*][1,2]diazacyclotridecin-5-one (Compound 208)**

**1) Synthesis of 4-methyl-*N*-(1-methylpiperidin-4-yl)-6-nitropyridin-3-amine**

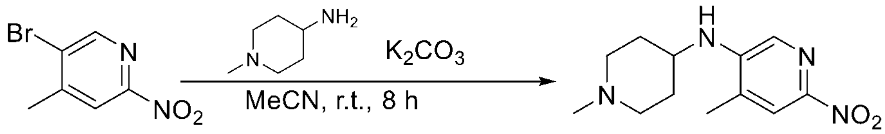


A solution of 5-bromo-4-methyl-2-nitropyridine (400 mg, 1.84 mmol), 1-methylpiperidin-4-amine (210.0 mg, 1.84 mmol), and potassium carbonate (763.0 mg, 5.53 mmol) in acetonitrile (4 mL) was stirred at room temperature for 8 h. The resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give 4-methyl-*N*-(1-methylpiperidin-4-yl)-6-nitropyridin-3-amine (300 mg, yield: 64.2%), LC-MS *m/z:* 251 [M + H]+;

**2) Synthesis of 4-methyl-*N*5-(1-methylpiperidin-4-yl)pyridine-2,5-diamine**

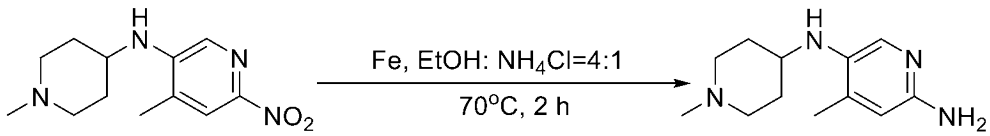


A solution of 4-methyl-*N*-(1-methylpiperidin-4-yl)-6-nitropyridin-3-amine (190 mg, 0.76 mmol), and Fe (336.0 mg, 3.78 mmol) in aqueous ammonium chloride solution (1 mL) and ethanol (4 mL) was heated to 70 °C and stirred for 2 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m/z:* 221 [M + H]+;

**3) Synthesis of (*Z*)-12-hydroxy-12-methyl-2-((4-methyl-5-((1-methylpiperidin-4-yl)amino)pyridin-2-yl)amino)-7,10,11,12-tetrahydro-5*H*-13,17-(azeno)pyrimido[4',5':3,4]pyrazolo[1,2-*a*][1,2]diazacyclotridecin-5-one**

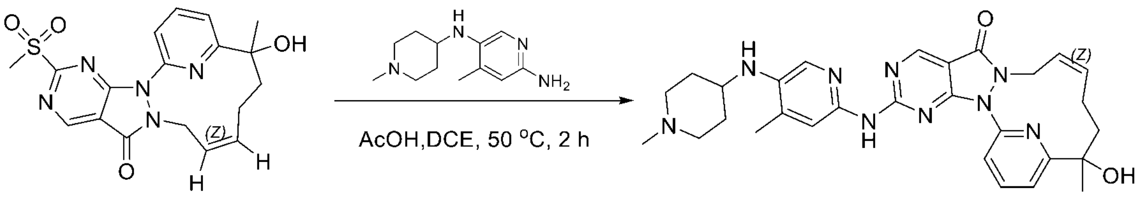


A solution of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (300 mg, 0.75 mmol), 4-methyl-*N*5-(1-methylpiperidin-4-yl)pyridine-2,5-diamine (247 mg, 1.12 mmol), and acetic acid (269.32 mg, 4.49 mmol) in 1,2-dichloroethane (3 mL) was heated to 50 °C and stirred for 2 h. After cooling, the mixed reaction solution was filtered and concentrated under reduced pressure. The residue was purified by C18 reverse phase column chromatography ($CH_3CN$:$H_2O$ (0.1% $NH_3.H_2O$) = 5%-95% elution) to give (*Z*)-12-hydroxy-12-methyl-2-((4-methyl-5-((1-methylpiperidin-4-yl)amino)pyridin-2-yl)amino)-7,10,11,12-tetrahydro-5*H*-13,17-(azeno)pyrimido[4',5':3,4]pyrazolo[1,2-*a*][1,2]diazacyclotridecin-5-one (20 mg, yield: 4.9%), LC-MS *m/z:* 542 [M + H]$^+$;

$^1$H NMR (400 MHz, MeOD-d4): δ 8.75 (s, 1H), 8.01 - 7.44 (m, 4H), 6.48 (s, 1H), 5.43 (s, 2H), 4.57 (s, 2H), 3.94 (s, 1H), 3.48 (s, 2H), 3.14 (s, 2H), 2.85 (s, 3H), 2.23-2.22 (m, 3H), 2.14-2.19 (m, 4H), 1.94 (s, 1H), 1.90-1.70 (m, 3H), 1.65 (s, 3H).

**Example 51: Synthesis of (*Z*)-12-hydroxy-12-methyl-2-(((*R*)-1-(1-methylpiperidine-4-carbonyl)pyrrolidin-3-yl) amino)-7,10,11,12-tetrahydro-5*H*-13,17-(azeno)pyrimido[4',5':3,4]pyrazolo[1,2-*a*][1,2]diazacyclotridecin-5-one (Compound 209)**

**1) Synthesis of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one**

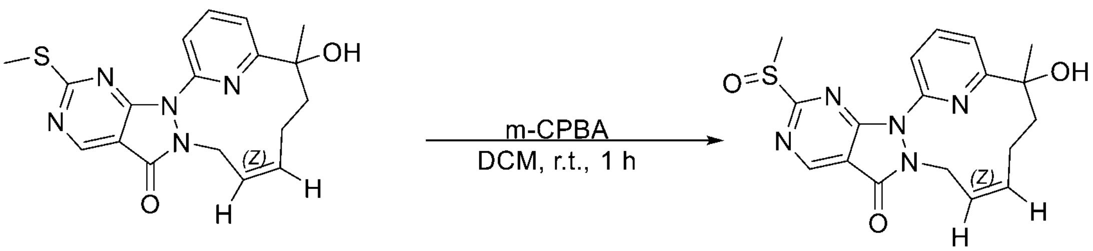


A solution of (*Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one (200 mg, 0.54 mmol) and 3-chloroperoxybenzoic acid (186 mg, 1.08 mmol) in dichloromethane (5 mL) was stirred at room temperature for 3 h. The resulting mixed reaction solution was concentrated under reduced pressure to give a crude product, which was used directly in the next step without further purification. LC-MS *m/z:* 386 [M + H]$^+$;

**2) Synthesis of *tert*-butyl (3*R*)-3-(((*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)pyrrolidine-1-carboxylate**

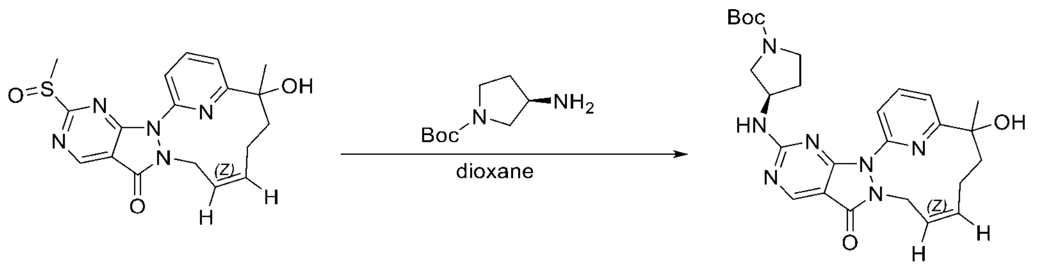


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (200 mg, 0.52 mmol) and *tert*-butyl (*R*)-3-aminopyrrolidine-1-carboxylate (120.5 mg, 0.65 mmol) in 1,4-dioxane (5 mL) was heated to 100 °C and stirred overnight. After cooling, the mixed reaction solution was concentrated under reduced pressure, and the residue was purified by C18 reverse phase column chromatography ($CH_3CN$:$H_2O$ (0.1% $NH_4HCO_3$) = 5%-95% elution) to give *tert*-butyl (3*R*)-3-(((*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl) amino)pyrrolidine-1-carboxylate (162 mg, yield: 62%), LC-MS *m/z*: 508[M + H]$^+$;

**3) Synthesis of (*Z*)-12-hydroxy-12-methyl-2-(((R)-pyrrolidin-3-yl)amino)-7,10,11,12-tetrahydro-5*H*-13,17-(azeno) pyrimido[4',5':3,4]pyrazolo[1,2-*a*][1,2]diazacyclotridecin-5-one**

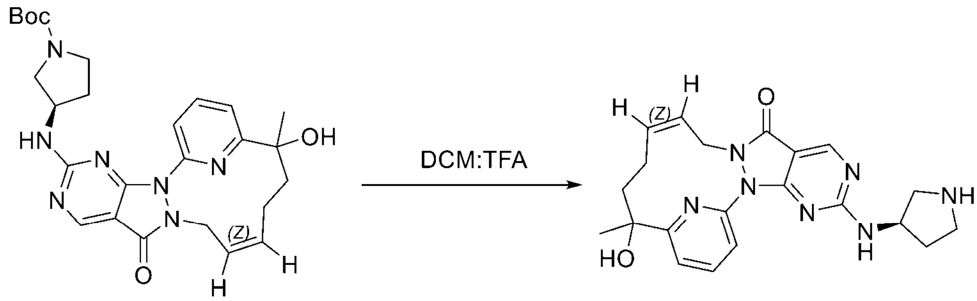


2,2,2-Trifluoroacetic acid (0.3 mL) was added dropwise to a solution of *tert*-butyl (3*R*)-3-(((*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl) amino)pyrrolidine-1-carboxylate (162 mg, 0.32 mmol) in dichloromethane (0.9 mL) at room temperature. The resulting mixed reaction solution was stirred at room temperature for 0.5 h. The resulting mixed reaction solution was concentrated under reduced pressure. The residue was purified by C18 reverse phase column chromatography ($CH_3CN:H_2O$ (0.1% $NH_4HCO_3$) = 5%-95% elution) to give (*Z*)-12-hydroxy-12-methyl-2-(((*R*)-pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one (120 mg, yield: 92.1%), LC-MS *m*/*z:* 408 $[M + H]^+$;

**4) Synthesis of (*Z*)-12-hydroxy-12-methyl-2-(((*R*)-1-(1-methylpiperidine-4-carbonyl)pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one**

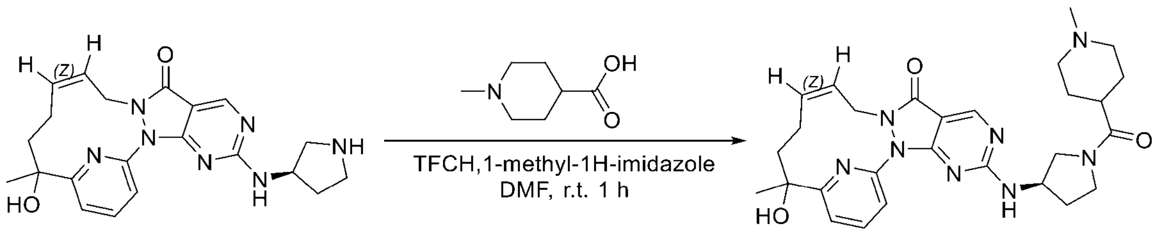


A solution of (*Z*)-12-hydroxy-12-methyl-2-(((*R*)-pyrrolidin-3-yl)amino)-7,10,11,12-tetrahydro-5*H*-13,17-(azeno) pyrimido[4',5':3,4]pyrazolo[1,2-*a*][1,2]diazacyclotridecin-5-one (120 mg, 0.29 mmol), 1-methyl-1*H*-imidazole (118.9 mg, 1.45 mmol), *N,N,N',N'*-tetramethylchloroformamidinium hexafluorophosphate (162.4 mg, 0.58 mmol), and 1-methyl-1*H*-piperidine-4-carboxylic acid (49.8 mg, 0.35 mmol) in *N,N*-dimethylformamide (2 mL) was stirred at room temperature for 1 h. The mixed reaction solution was purified by C18 reverse column chromatography ($CH_3CN:H_2O$ (0.1% $NH_4HCO_3$) = 5%-95% elution) to give (*Z*)-12-hydroxy-12-methyl-2-(((*R*)-1-(1-methylpiperidine-4-carbonyl)pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one (71 mg, yield: 45.0%), LC-MS *m*/*z:* 533 $[M + H]^+$;
$^1H$ NMR (400 MHz, MeOD): δ 8.73 (s, 1H), 7.93 (s, 1H), 7.76 (s, 1H), 7.57 (d, J = 6.6 Hz, 1H), 5.51 - 5.33 (m, 2H), 4.58 (s, 2H), 4.01 - 3.39 (m, 7H), 3.04 - 2.75 (m, 6H), 2.25 (d, J = 73.5 Hz, 4H), 1.98 (dd, J = 29.8, 10.9 Hz, 6H), 1.67 (s, 3H).

**Example 52: Synthesis of (*Z*)-12-hydroxy-12-methyl-2-(((*S*)-1-(1-methylpiperidine-4-carbonyl)pyrrolidin-3-yl) amino)-7,10,11,12-tetrahydro-5*H*-13,17-(azeno)pyrimido[4',5':3,4]pyrazolo[1,2-*a*][1,2]diazacyclotridecin-5-one (Compound 210)**

**1) Synthesis of (Z)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one**

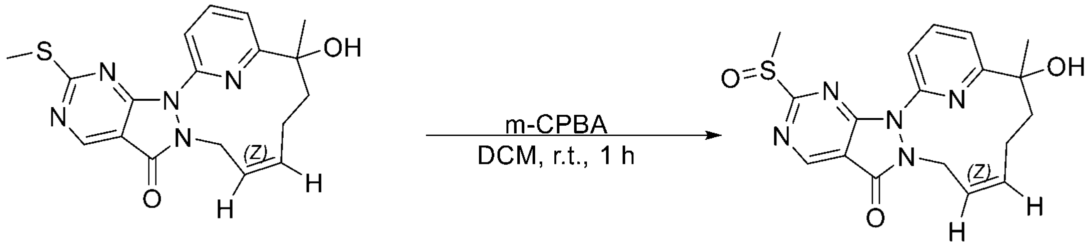


A solution of (*Z*)-12-hydroxy-12-methyl-2-(methylthio)-7,10,11,12-tetrahydro-5*H*-13,17-(azeno)pyrimido [4',5':3,4]pyrazolo[1,2-*a*][1,2]diazacyclotridecin-5-one (200 mg, 0.54 mmol) and 3-chloroperoxybenzoic acid (186 mg, 1.08 mmol) in dichloromethane (5 mL) was stirred at room temperature for 3 h. The resulting mixed reaction solution was concentrated under reduced pressure to give a crude product, which was used directly in the next step without further purification. LC-MS *m/z:* 386 [M + H]$^+$;

**2) Synthesis of *tert*-butyl (3*S*)-3-(((*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)pyrrolidine-1-carboxylate**

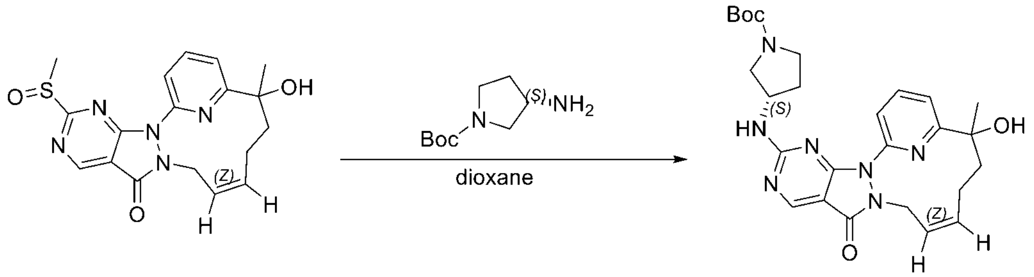


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (200 mg, 0.52 mmol) and *tert-butyl* (*S*)-3-aminopyrrolidine-1-carboxylate (120.5 mg, 0.65 mmol) in 1,4-dioxane (5 mL) was heated to 100 °C and stirred overnight. After cooling, the mixed reaction solution was concentrated under reduced pressure, and the residue was purified by C18 reverse phase column chromatography ($CH_3CN:H_2O$ (0.1% $NH_4HCO_3$) = 5%-95% elution) to give *tert*-butyl (3*S*)-3-(((*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl) amino)pyrrolidine-1-carboxylate (162 mg, yield: 62%), LC-MS *m/z:* 508 [M + H]$^+$;

**3) Synthesis of (*Z*)-12-hydroxy-12-methyl-2-(((*S*)-pyrrolidin-3-yl)amino)-7,10,11,12-tetrahydro-5*H*-13,17-(azeno) pyrimido[4',5':3,4]pyrazolo[1,2-*a*][1,2]diazacyclotridecin-5-one**

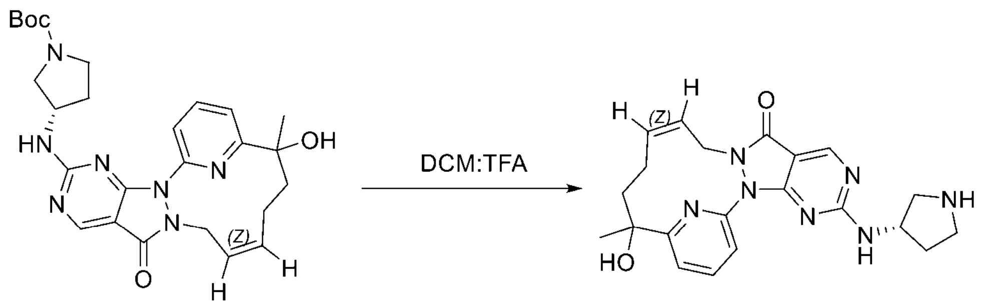


2,2,2- Trifluoroacetic acid (0.3 mL) was added dropwise to a solution of *tert*-butyl (3*S*)-3-(((*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl) amino)pyrrolidine-1-carboxylate (162 mg, 0.32 mmol) in dichloromethane (0.9 mL) at room temperature. The resulting mixed reaction solution was stirred at room temperature for 0.5 h. The resulting mixed reaction solution was concentrated

under reduced pressure. The residue was purified by C18 reverse phase column chromatography ($CH_3CN:H_2O$ (0.1% $NH_4HCO_3$) = 5%-95% elution) to give (*Z*)-12-hydroxy-12-methyl-2-(((*S*)-pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one (120 mg, yield: 92.1%), LC-MS *m*/*z*: 408 [M + H]$^+$;

**4) Synthesis of (*Z*)-12-hydroxy-12-methyl-2-(((*S*)-1-(1-methylpiperidine-4-carbonyl)pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one**

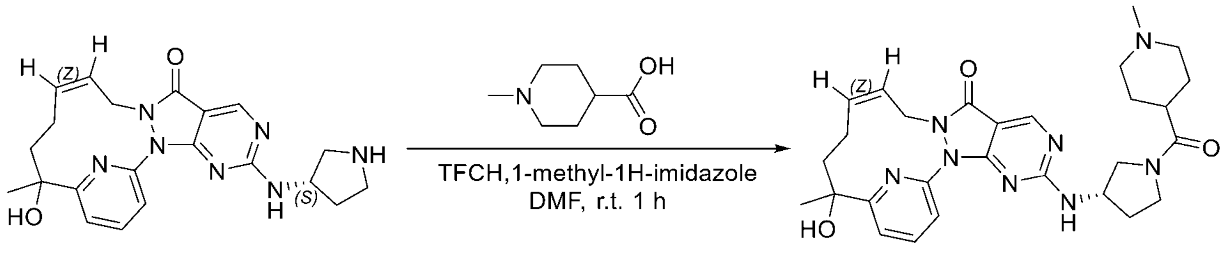


A solution of (*Z*)-12-hydroxy-12-methyl-2-(((*S*)-pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one (120 mg, 0.29 mmol), 1-methyl-1*H*-imidazole (118.9 mg, 1.45 mmol), *N,N,N,N*-tetramethylchloroformamidinium hexafluorophosphate (162.4 mg, 0.58 mmol), and 1-methyl-1*H*-piperidine-4-carboxylic acid (49.8 mg, 0.35 mmol) in N,N-dimethylformamide (2 mL) was stirred at room temperature for 1 h. The mixed reaction solution was purified by C18 reverse column chromatography ($CH_3CN:H_2O$ (0.1% $NH_4HCO_3$) = 5%-95% elution) to give (*Z*)-12-hydroxy-12-methyl-2-(((*S*)-1-(1-methylpiperidine-4-carbonyl)pyrrolidin-3-yl)amino)-7,10,11,12-tetrahydro-5*H*-13,17-(azeno)pyrimido[4',5':3,4]pyrazolo[1,2-*a*][1,2]diazacyclotridecin-5-one (56 mg, yield: 35.2%), LC-MS *m*/*z:* 533 [M + H]$^+$;
$^1$H NMR (400 MHz, MeOD): δ 8.73 (s, 1H), 7.93 (s, 1H), 7.76 (dd, J = 8.1, 4.5 Hz, 1H), 7.56 (d, J = 7.7 Hz, 1H), 5.43 (dd, J = 17.7, 9.2 Hz, 2H), 4.58 (s, 2H), 3.95 (s, 1H), 3.75 (d, J = 7.1 Hz, 2H), 3.66 - 3.47 (m, 2H), 3.45 - 3.35 (m, 2H), 2.81 (dt, J = 34.9, 11.3 Hz, 6H), 2.41 - 2.08 (m, 4H), 2.07 - 1.82 (m, 6H), 1.67 (s, 3H).

**Example 53: Synthesis of (*S*)-*N*-(4-(((*R,Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenyl)-1-methylpyrrolidine-3-carboxamide (Compound 211)**

**1) Synthesis of (*R,Z*)-12-hydroxy-12-methyl-2-((3-methyl-4-nitrophenyl)amino)-7,10,11,12-tetrahydro-5*H*-13,17-(azeno)pyrimido[4',5':3,4]pyrazolo[1,2-*a*][1,2]diazacyclotridecin-5-one or isomer**

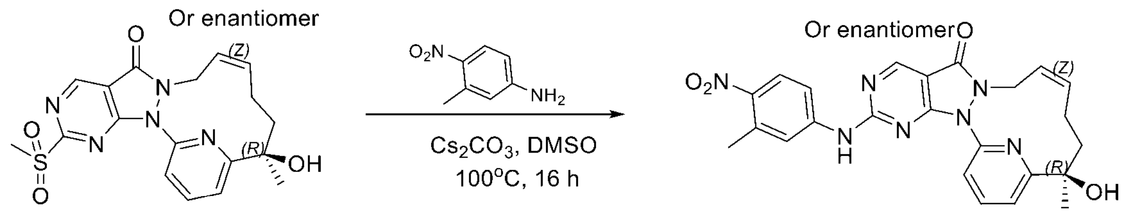


A solution of (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one or enantiomer (0.8 g, 1.99 mmol), 3-methyl-4-nitroaniline (0.61 g, 3.98 mmol), and cesium carbonate (1.95 g, 5.97 mmol) in dimethyl sulfoxide (10 mL) was heated to 100 °C and stirred for 16 h. After cooling, the mixed reaction solution was filtered, and the filter cake was washed with methanol. The filtrate was collected and concentrated under reduced pressure. The residue was dissolved in methanol (5 mL), and the solution was purified by C18 reverse phase column chromatography ($MeCN:H_2O$ = 65%-80% elution) to give (*R,Z*)-12-hydroxy-12-methyl-2-((3-methyl-4-nitrophenyl)amino)-7,10,11,12-tetrahydro-5*H*-13,17-(azeno)pyrimido[4',5':3,4]pyrazolo[1,2-*a*][1,2]diazacyclotridecin-5-one or isomer (0.28 g, yield: 29.7%), LC-MS *m*/*z:* 474 [M + H]$^+$;

**2) Synthesis of (*R,Z*)-2-((4-amino-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer**

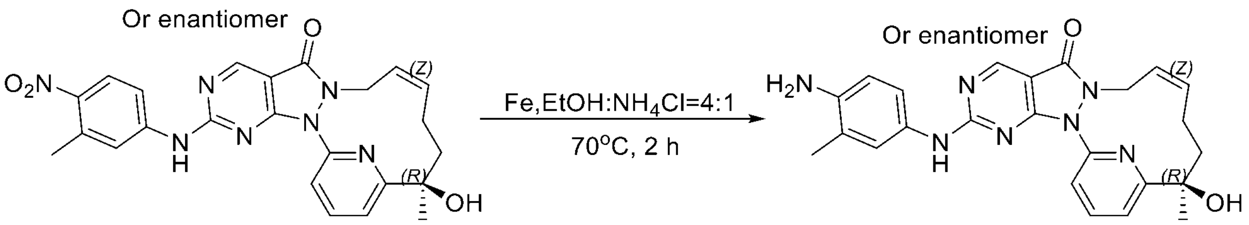


A solution of (*R*,*Z*)-12-hydroxy-12-methyl-2-((3-methyl-4-nitrophenyl)amino)-7,10,11,12-tetrahydro-5*H*-13,17-(azeno)pyrimido[4',5':3,4]pyrazolo[1,2-*a*][1,2]diazacyclotridecin-5-one or isomer (280 mg, 0.59 mmol) and Fe (164.76 mg, 2.95 mmol) in ethanol (2.4 mL) and aqueous ammonium chloride solution (0.6 mL) was heated to 70 °C and stirred for 2 h. After cooling, the mixed reaction solution was filtered, and the filter cake was washed with methanol. The filtrate was collected and concentrated under reduced pressure to give (*R*,*Z*)-2-((4-amino-3-methylphenyl) amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer (150 mg, yield: 57.2%), LC-MS *m*/*z*: 444[M + H]$^+$;

**3) Synthesis of (*S*)-*N*-(4-((((*R*,*Z*)-12-hydroxy-12-methyl-5-oxo-7,10,11,12-tetrahydro-5*H*-13,17-(azeno)pyrimido [4',5':3,4]pyrazolo[1,2-*a*][1,2]diazacyclotridecin-2-yl)amino)-2-methylphenyl)-1-methylpyrrolidine-3-carboxamide**

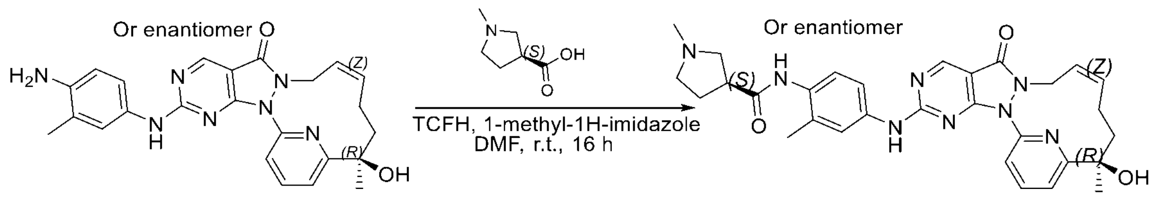


A solution of (*R*,*Z*)-2-((4-amino-3-methylphenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer (50 mg, 0.11 mmol), (3S)-1-methylpyrrolidine-3-carboxylic acid (28.42 mg, 0.22 mmol), *N,N,N',N'*-tetramethylchloroformamidinium hexafluorophosphate (61.73 mg, 0.22 mmol), and 1-methyl-1H-imidazole (36.12 mg, 0.44 mmol) in *N,N*-dimethylformamide (1 mL) was stirred at room temperature for 16 h. The resulting mixed reaction solution was purified by C18 reverse phase column chromatography (ACN:$H_2O$ = 15%-30% elution) to give (*S*)-*N*-(4-(((*R*,*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylphenyl)-1-methylpyrrolidine-3-carboxamide (35 mg, yield: 56.0%), LC-MS *m*/*z*: 555 [M + H]$^+$;
$^1$H NMR (400 MHz, MeOD-d4) δ 8.83 (s, 1H), 7.96 (t, J = 7.9 Hz, 1H), 7.82 (d, J = 8.0 Hz, 1H), 7.71 (s, 1H), 7.60 (d, J = 7.7 Hz, 1H), 7.47 (dd, J = 8.6, 2.1 Hz, 1H), 7.26 (d, J = 8.6 Hz, 1H), 5.54 - 5.38 (m, 2H), 4.60 (d, J = 6.8 Hz, 2H), 3.39 (dd, J = 16.9, 7.2 Hz, 3H), 3.21 (t, J = 7.1 Hz, 2H), 2.79 (s, 3H), 2.45 (td, J = 15.6, 7.7 Hz, 1H), 2.35 - 2.09 (m, 6H), 2.00 (t, J = 11.0 Hz, 1H), 1.94 - 1.85 (m, 1H), 1.68 (s, 3H).

**Example 54: Synthesis of *N*-((1*S*,4*s*)-4-(((*R*,*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)cyclohexyl)-1-methylpiperidine-4-carboxamide or enantiomer (Compound 212)**

**1) Synthesis of (*R,Z*)-2-(((1*s*,4*S*)-4-aminocyclohexyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer**

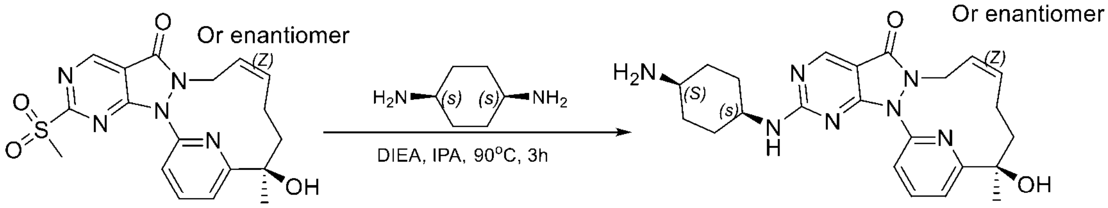


N,N-Diisopropylethylamine (125 mg, 0.973 mmol) was added to a solution of (*R,Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one or enantiomer (130 mg, 0.324 mmol) and (1*s*,4*s*)-cyclohexane-1,4-diamine (44 mg, 0.389 mmol) in isopropanol (3 mL) at room temperature. The resulting mixed reaction solution was heated to 90 °C and stirred for 3 h. After cooling, the mixed reaction solution was diluted with water and extracted with ethyl acetate (3 × 50 mL). The combined organic phases were washed with saturated brine (3 × 30 mL), dried over anhydrous sodium sulfate, filtered, and concentrated under reduced pressure. The residue was purified by C18 reverse phase column chromatography (ACN (0.1%$NH_4HCO_3$):water (0.1% $NH_4HCO_3$) = 60%-80% elution) to give (*R,Z*)-2-(((1*s*,4*S*)-4-aminocyclohexyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer (70 mg, yield: 49.6%), LC-MS *m/z:* 436 [M + H]$^+$;

**2) Synthesis of *N*-((1*S*,4*s*)-4-(((*R,Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)cyclohexyl)-1-methylpiperidine-4-carboxamide or enantiomer**

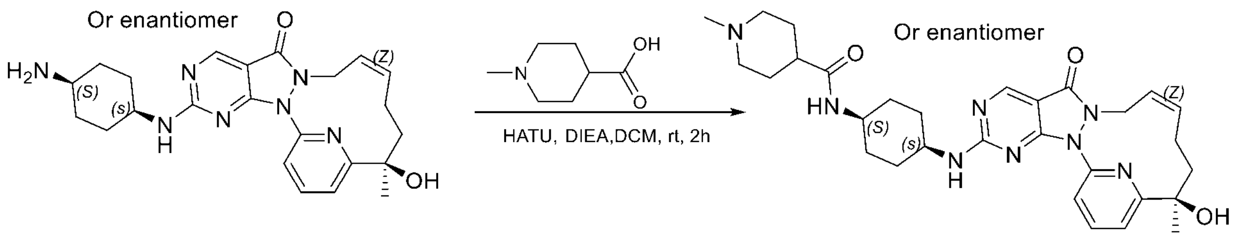


*O*-(7-Azabenzotriazol-1-yl)-*N,N,N',N'*-tetramethyluronium hexafluorophosphate (122 mg, 0.322 mmol) and *N,N*-diisopropylethylamine (62 mg, 0.483 mmol) were added to a solution of (*R,Z*)-2-(((1*s*,4*S*)-4-aminocyclohexyl) amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one or enantiomer (70 mg, 0.161 mmol) and 1-methylpiperidine-4-carboxylic acid (28 mg, 0.193 mmol) in dichloromethane (2 mL) at room temperature. The resulting mixed reaction solution was stirred at room temperature for 2 h. The mixed reaction solution was diluted with water and extracted with ethyl acetate (4 × 80 mL). The combined organic phases were washed with saturated brine (2 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give *N*-((1*S*,4*s*)-4-(((*R*,*Z*)-12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)cyclohexyl)-1-methylpiperidine-4-carboxamide or enantiomer (20 mg, yield: 22.2%), LC-MS *m/z*: 561 [M + H]$^+$;
$^1$H NMR (400 MHz, MeOD-d4): δ 8.78 (s, 1H), 8.01-7.90 (m, 1H), 7.73 (d, *J* = 8 Hz, 1H), 7.63 (d, *J* = 6.8 Hz, 1H), 5.46-5.30 (m, 2H), 4.66-4.56 (m, 2H), 4.06-3.97 (m, 1H), 3.81 (s, 1H), 3.55 (d, *J* = 12 Hz, 2H), 3.40-3.34 (m, 1H), 3.07-2.94 (m, 2H), 2.90-2.83 (m, 3H), 2.57-2.46 (m, 1H), 2.26-2.10 (m, 2H), 2.05-1.96 (m, 3H), 1.93 (s, 2H), 1.90-1.79 (m, 5H), 1.74-1.70 (m, 2H), 1.68 (s, 3H).

**Example 55: Synthesis of (*Z*)-12-hydroxy-12-methyl-2-((3-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 213)**

**1) Synthesis of (*Z*)-12-hydroxy-12-methyl-2-((3-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one**

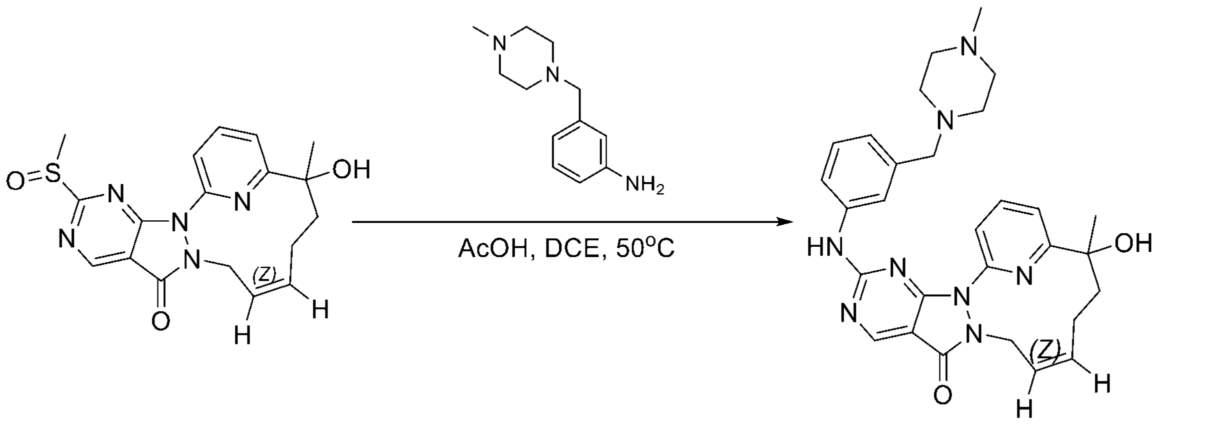


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (80 mg, 0.21 mmol), 3-((4-methylpiperazin-1-yl)methyl)aniline (51.3 mg, 0.25 mmol), and acetic acid (90 mg, 1.5 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give (*Z*)-12-hydroxy-12-methyl-2-((3-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (32.0 mg, yield: 29.2%), LC-MS *m/z:* 527 $[M + H]^+$;

$^1$H NMR (400 MHz, MeOD-d4): δ 8.87-8.85 (m, 1H), 7.98 (t, *J* = 8.0 Hz, 1H), 7.81 (d, *J* = 8.0 Hz, 1H), 7.76 (s, 1H), 7.63-7.56 (m, 2H), 7.29 (t, *J* = 8.0 Hz, 1H), 7.06 (d, *J* = 7.6 Hz, 1H), 5.51-5.36 (m, 2H),4.63 (d, *J* = 6.4 Hz, 2H), 3.57 (s, 2H), 2.90-2.51 (m, 8H), 2.48 (s, 3H), 2.33-2.10 (m, 2H), 2.06-1.85 (m, 2H), 1.69 (s, 3H).

**Example 56: Synthesis of (*Z*)-12-hydroxy-12-methyl-2-((4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 214)**

**1) Synthesis of (*Z*)-12-hydroxy-12-methyl-2-((4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one**

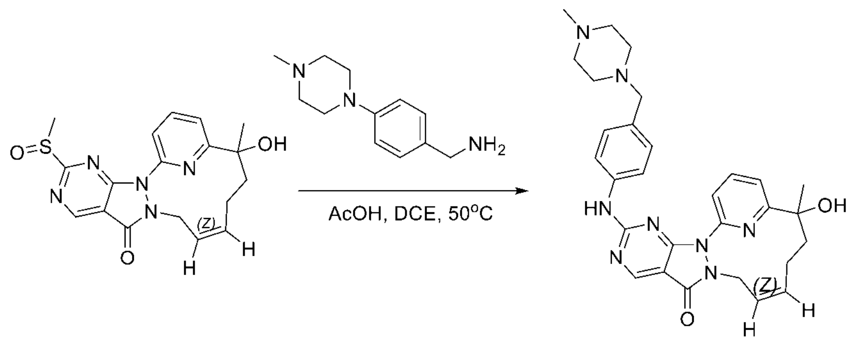


A solution of (*Z*)-12-hydroxy-12-methyl-2-(methylsulfinyl)-7,10,11,12-tetrahydro-5*H*-13,17-(azeno)pyrimido [4',5':3,4]pyrazolo[1,2-*a*][1,2]diazacyclotridecin-5-one (80 mg, 0.21 mmol), (4-(4-methylpiperazin-1-yl)phenyl)methanamine (51.3 mg, 0.25 mmol), and acetic acid (90 mg, 1.5 mmol) in 1,2-dichloroethane (2 mL) was heated to 50 °C and stirred for 1 h. After cooling, the resulting mixed reaction solution was concentrated under reduced pressure. The residue was purified by C18 reverse phase column chromatography ($CH_3CN$:$H_2O$ (0.1% $NH_3.H_2O$) = 5%-95% elution) to give (*Z*)-12-hydroxy-12-methyl-2-((4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (36.5 mg, yield: 33.5%), LC-MS *m/z:* 527 $[M + H]^+$;

$^1$H NMR (400 MHz, MeOD-$d_4$): δ 8.69 (s, 1H), 7.88 (t, *J* = 8.0 Hz, 1H), 7.54 (d, *J* = 7.6 Hz, 2H), 7.23 (d, *J* = 8.4 Hz, 2H), 6.92 (d, *J* = 8.8 Hz, 2H), 5.51-5.33 (m, 2H), 4.56 (d, *J* = 6.8 Hz, 2H), 4.48 (s, 2H), 3.20-3.12 (m, 4H), 2.65-2.57 (m, 4H), 2.34 (s, 3H), 2.27-2.07(m, 2H), 2.02-1.82 (m, 2H), 1.67 (s, 3H).

**Example 57: Synthesis of (*R,Z*)-12-hydroxy-12-methyl-2-((3-methyl-4-((1-methylpiperidin-4-yl)thio)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 215)**

**1) Synthesis of *tert*-butyl 4-((2-methyl-4-nitrophenyl)thio)piperidine-1-carboxylate**

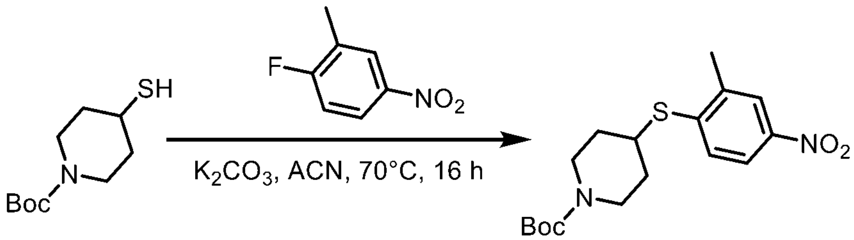


A solution of *tert-butyl* 4-mercaptopiperidine-1-carboxylate (1 g, 4.60 mmol), 1-fluoro-2-methyl-4-nitrobenzene (0.71 g, 4.6 mmol), and potassium carbonate (0.95 g, 6.90 mmol) in acetonitrile (10 mL) was heated to 70 °C and stirred for 16 h. The resulting mixed reaction solution was concentrated under reduced pressure, and the residue was purified by silica gel column chromatography (ethyl acetate:petroleum ether = 0%-15% elution) to give *tert*-butyl 4-((2-methyl-4-nitrophenyl)thio)piperidine-1-carboxylate (910 mg, yield: 55.4%), LC-MS *m/z:* 353 [M + H]$^+$;

**2) Synthesis of 4-((2-methyl-4-nitrophenyl)thio)piperidine**

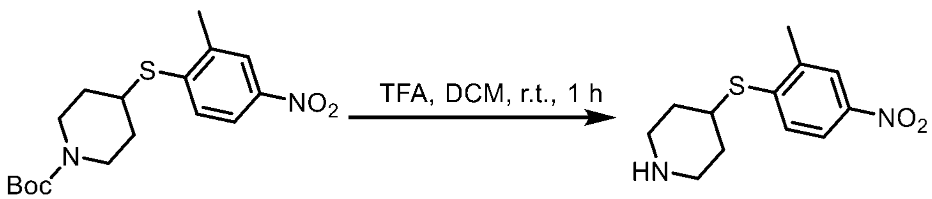


A solution of *tert*-butyl 4-((2-methyl-4-nitrophenyl)thio)piperidine-1-carboxylate (900 mg, 2.55 mmol) in dichloromethane (9 mL) and 2,2,2-trifluoroacetic acid (3 mL) was stirred at room temperature for 1 h. The resulting mixed reaction solution was concentrated under reduced pressure to give a crude product, which was used directly in the next step without further purification. LC-MS *m/z:* 253 H]$^+$;

**3) Synthesis of 1-methyl-4-((2-methyl-4-nitrophenyl)thio)piperidine**

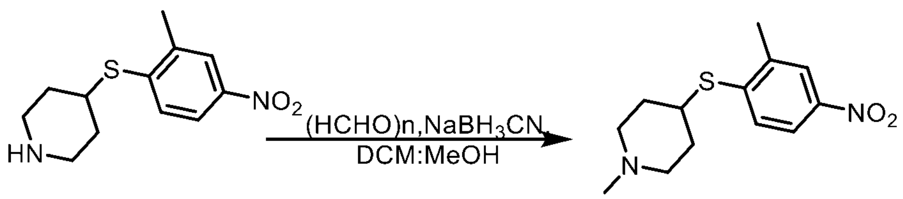


A solution of 4-((2-methyl-4-nitrophenyl)thio)piperidine (900 mg, 3.57 mmol), paraformaldehyde (160 mg, 5.33 mmol), and sodium cyanoborohydride (1120 mg, 17.82 mmol) in methanol (6 mL) and dichloromethane (2 mL) was stirred at room temperature for 16 h. The resulting mixed reaction solution was concentrated under reduced pressure. The residue was purified by C18 reverse phase column chromatography (ACN:$H_2O$ = 0%-50% elution) to give 1-methyl-4-((2-methyl-4-nitrophenyl)thio)piperidine (900 mg, yield: 94.7%), LC-MS m/z: 267 [M + H]$^+$;

**4) Synthesis of 3-methyl-4-((1-methylpiperidin-4-yl)thio)aniline**

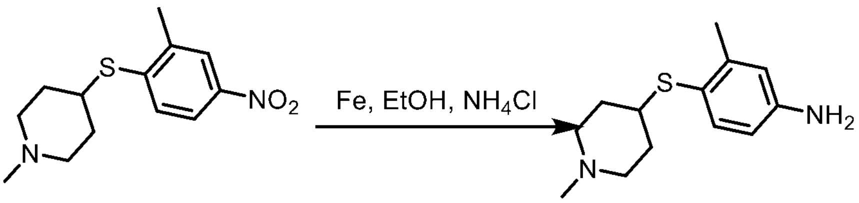


A solution of 1-methyl-4-((2-methyl-4-nitrophenyl)thio)piperidine (300 mg, 1.13 mmol) and Fe (320 mg, 5.65 mmol) in ethanol (4 mL) and aqueous ammonium chloride solution (1 mL) was heated to 70 °C and stirred for 2 h. The mixed reaction solution was filtered, and the filter cake was washed with methanol. The filtrate was collected and concentrated under reduced pressure to give a crude product, which was used directly in the next step without further purification. LC-MS *m/z:* 237 $[M + H]^+$;

**5) Synthesis of (*R,Z*)-12-hydroxy-12-methyl-2-((3-methyl-4-((1-methylpiperidin-4-yl)thio)phenyl)amino)-7,10,11,12-tetrahydro-5H-13,17-(azeno)pyrimido[4',5':3,4]pyrazolo[1,2-*a*][1,2]diazacyclotridecin-5-one**

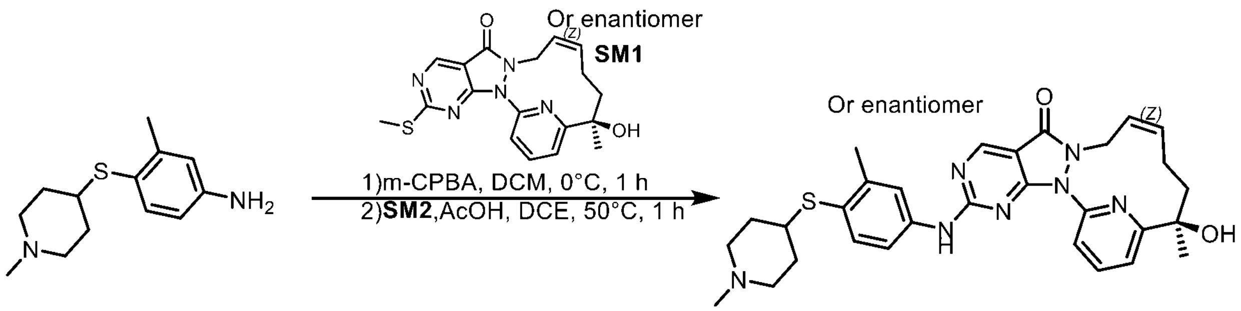


A solution of (*R,Z*)-12-hydroxy-12-methyl-2-(methylthio)-7,10,11,12-tetrahydro-5*H*-13,17-(azeno)pyrimido[4',5':3,4]pyrazolo[1,2-*a*][1,2]diazacyclotridecin-5-one or enantiomer (200 mg, 0.54 mmol) and 3-chloroperoxybenzoic acid (140 mg, 0.81 mmol) in dichloromethane (2 mL) was stirred at 0 °C for 1 h. Then, a solution of 3-methyl-4-((1-methylpiperidin-4-yl)thio)aniline (190 mg, 0.81 mmol) and acetic acid (190 mg, 3.24 mmol) in 1,2-dichloroethane (1 mL) was added to the above mixed reaction solution. The resulting mixed reaction solution was heated to 50 °C and stirred for 16 h. The mixed reaction solution was concentrated under reduced pressure, and the residue was purified by C18 column chromatography (ACN:$H_2O$ = 0%-70% elution) to give (*R,Z*)-12-hydroxy-12-methyl-2-((3-methyl-4-((1-methylpiperidin-4-yl)thio)phenyl)amino)-7,10,11,12-tetrahydro-5*H*-13,17-(azeno)pyrimido[4',5':3,4]pyrazolo[1,2-*a*][1,2]diazacyclotridecin-5-one (60 mg, yield: 44.7%), LC-MS *m/z:* 558 $[M + H]^+$;
$^1H$ NMR (400 MHz, MeOD-d4):δ 8.83 (s, 1H), 7.97 (t, J = 8.0 Hz, 1H), 7.81 (d, J = 8.0 Hz, 1H), 7.75 (s, 1H), 7.61 (d, J = 7.6 Hz, 1H), 7.43 (dd, J = 8.4, 2.0 Hz, 1H), 7.36 (d, J = 8.4 Hz, 1H), 5.52 - 5.39 (m, 2H), 4.60 (d, J = 6.8 Hz, 2H), 3.02 (s, 1H), 2.88 (d, J = 12.0 Hz, 2H), 2.41 (s, 3H), 2.30 (s, 3H), 2.20 (s, 4H), 2.03 - 1.87 (m, 4H), 1.69 (s, 3H), 1.67 - 1.58 (m, 2H).

**Example 58: Synthesis of (12*R,Z*)-12-hydroxy-12-methyl-2-((3-methyl-4-((1-methylpiperidin-4-yl)sulfinyl)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound 216)**

**1) Synthesis of (12*R,Z*)-12-hydroxy-12-methyl-2-((3-methyl-4-((1-methylpiperidin-4-yl)sulfinyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

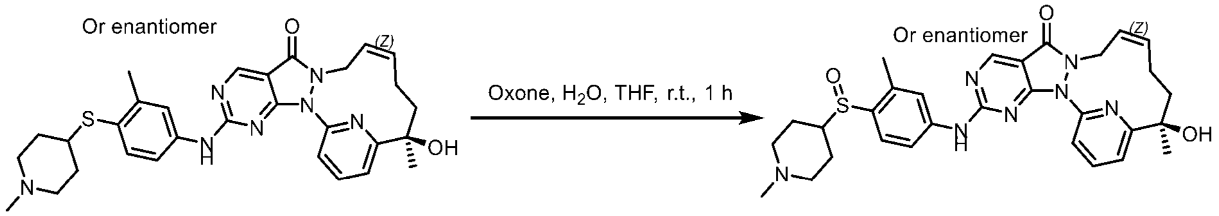


A solution of (*R,Z*)-12-hydroxy-12-methyl-2-((3-methyl-4-((1-methylpiperidin-4-yl)thio)phenyl)amino)-7,10,11,12-tetrahydro-5*H*-13,17-(azeno)pyrimido[4',5':3,4]pyrazolo[1,2-*a*][1,2]diazacyclotridecin-5-one (50 mg, 0.090 mmol) and oxone (66 mg, 0.11 mmol) in tetrahydrofuran (0.5 mL) and water (0.5 mL) was stirred at room temperature for 1 h. The mixed reaction solution was concentrated under reduced pressure, and the residue was purified by C18 reverse phase column chromatography (MeCN:$H_2O$ = 0%-70% elution) to give (12*R,Z*)-12-hydroxy-12-methyl-2-((3-methyl-4-((1-methylpiperidin-4-yl)sulfinyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (20 mg, yield: 38.88%), LC-MS *m/z:* 574 [M + H]$^+$; $^1$H NMR (400 MHz, MeOD-*d*4): δ 8.90 (s, 1H), 8.02 (t, J = 8.0 Hz, 1H), 7.88 (d, J = 5.6 Hz, 1H), 7.83 (d, J = 8.0 Hz, 1H), 7.78 (t, J = 7.6 Hz, 1H), 7.68 (d, J = 8.8 Hz, 1H), 7.64 (d, J = 7.6 Hz, 1H), 5.51 - 5.38 (m, 2H), 4.65 (d, J = 6.8 Hz, 2H), 3.04 - 2.89 (m, 2H), 2.82 (s, 1H), 2.41 (s, 3H), 2.28 (s, 3H), 2.11 (s, 4H), 2.00 (t, J = 11.2 Hz, 1H), 1.94 - 1.88 (m, 1H), 1.84 (dd, J = 9.2, 3.8 Hz, 2H), 1.75 (s, 2H), 1.69 (s, 3H).

**Example 59: Synthesis of (*R,Z*)-12-hydroxy-12-methyl-2-((3-methyl-4-((1-methylpiperidin-4-yl)sulfonyl)phenyl)amino)-7,10,11,12-tetrahydro-5*H*-13,17-(azeno)pyrimido[4',5':3,4]pyrazolo[1,2-*a*][1,2]diazacyclotridecin-5-one or Enantiomer (Compound 217)**

**1) Synthesis of (*R,Z*)-12-hydroxy-12-methyl-2-((3-methyl-4-((1-methylpiperidin-4-yl)sulfonyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

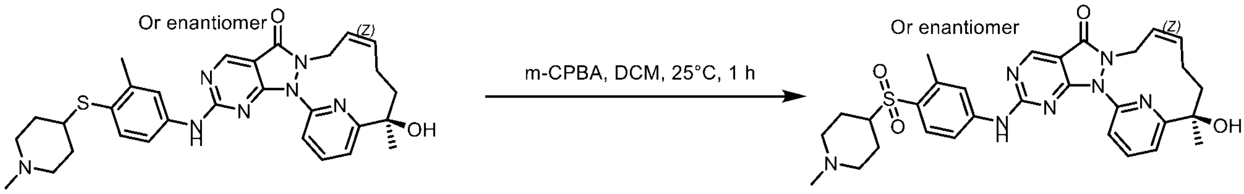


A solution of (*R,Z*)-12-hydroxy-12-methyl-2-((3-methyl-4-((1-methylpiperidin-4-yl)thio)phenyl)amino)-7,10,12-tetrahydro-5*H*-13,17-(azeno)pyrimido[4',5':3,4]pyrazolo[1,2-*a*][1,2]diazacyclotridecin-5-one (30 mg, 0.054 mmol) and 3-chloroperoxybenzoic acid (19 mg, 0.11 mmol) in dichloromethane (1 mL) was stirred at room temperature for 1 h. The mixed reaction solution was concentrated under reduced pressure, and the residue was purified by C18 reverse phase column chromatography (MeCN:$H_2O$ = 0%-70% elution) to give (*R,Z*)-12-hydroxy-12-methyl-2-((3-methyl-4-((1-methylpiperidin-4-yl)sulfonyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (10 mg, yield: 31.52%), LC-MS *m/z:* 590 [M + H]$^+$;

$^1$H NMR (400 MHz, MeOD-*d*4): δ 8.86 (s, 1H), 8.00 (dd, J = 11.6, 4.2 Hz, 1H), 7.86 (d, J = 9.6 Hz, 1H), 7.81 - 7.74 (m, 2H), 7.68 (d, J = 8.8 Hz, 1H), 7.63 (d, J = 7.6 Hz, 1H), 5.43 (qd, J = 17.6, 10.4 Hz, 2H), 4.63 (d, J = 6.8 Hz, 2H), 3.62 (t, J = 11.6 Hz, 2H), 3.57 - 3.45 (m, 2H), 3.38 (d, J = 13.2 Hz, 3H), 3.05 (dd, J = 12.4, 9.6 Hz, 1H), 2.48 - 2.35 (m, 5H), 2.18 (d, J = 18.4 Hz, 3H), 1.99 (dd, J = 16.4, 8.4 Hz, 1H), 1.91 (d, J = 11.6 Hz, 1H), 1.79 (d, J = 14.0 Hz, 1H), 1.69 (s, 3H).

**Example 60: Synthesis of methyl (*Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylbenzyl)-1-methylpiperidine-4-carboxylate (Compound 218)**

**1) Synthesis of methyl 1-methyl-4-(2-methyl-4-nitrobenzyl)piperidine-4-carboxylate**

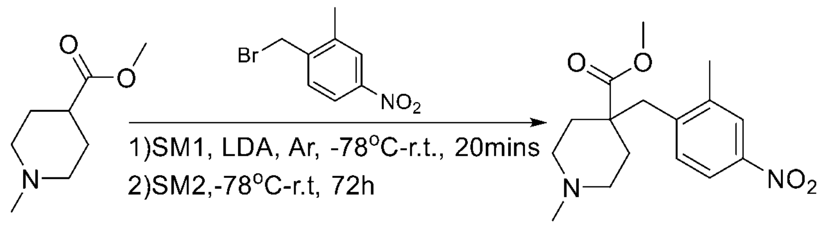


LDA (2.4 mL, 4.78 mmol, 2 M in tetrahydrofuran) was added dropwise to a solution of methyl 1-methylpiperidine-4-carboxylate (500 mg, 3.18 mmol) in tetrahydrofuran (10 mL) under an argon atmosphere at -78 °C, and the mixture was slowly warmed to room temperature over 20 min. The mixed reaction solution was again cooled to -78 °C, and a solution of 1-(bromomethyl)-2-methyl-4-nitrobenzene (878 mg, 3.82 mmol) in tetrahydrofuran (10 mL) was added to the mixed reaction solution using an injection pump over 20 min. The resulting mixed reaction solution was stirred at -78 °C for 1 h. At room temperature, the mixed reaction solution was quenched with saturated ammonium bicarbonate (5 mL) and extracted with ethyl acetate (3 × 10 mL). The combined organic phases were washed with saturated brine (1 × 30 mL), dried over anhydrous sodium sulfate, filtered, and concentrated under reduced pressure. The residue was purified by C18 reverse phase column chromatography (MeCN:$H_2O$ (0.1% $NH_3.H_2O$) = 5%-95% elution) to give methyl 1-methyl-4-(2-methyl-4-nitrobenzyl)piperidine-4-carboxylate (200 mg, yield: 20.52%), LC-MS *m/z:* 307 $[M + H]^+$.

**2) Synthesis of methyl 4-(4-amino-2-methylbenzyl)-1-methylpiperidine-4-carboxylate**

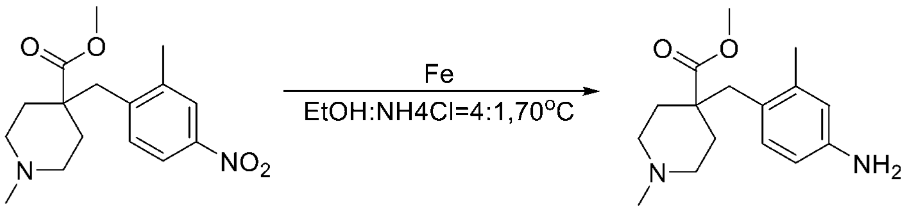


A solution of methyl 1-methyl-4-(2-methyl-4-nitrobenzyl)piperidine-4-carboxylate (200 mg, 0.65 mmol) and Fe (183 mg, 3.27 mmol) in ethanol (1.6 mL) and aqueous ammonium chloride solution (0.4 mL) was heated to 70 °C and stirred for 2 h. The mixed reaction solution was concentrated under reduced pressure, and the filter cake was washed with methanol (3 × 5 mL). The filtrate was collected and concentrated under reduced pressure. The residue was purified by C18 reverse phase column chromatography (MeCN:$H_2O$ (0.1% $NH_3.H_2O$) = 5%-95% elution) to give methyl 4-(4-amino-2-methylbenzyl)-1-methylpiperidine-4-carboxylate (120 mg, yield: 66.67%), LC-MS *m/z:* 277 $[M + H]^+$.

**3) Synthesis of methyl (*Z*)-4-(4-((12-hydroxy-12-methyl-5-oxo-7,10,11,12-tetrahydro-5*H*-13,17-(azeno)pyrimido [4',5':3,4]pyrazolo[1,2-*a*][1,2]diazacyclotridecin-2-yl)amino)-2-methylbenzyl)-1-methylpiperidine-4-carboxylate**

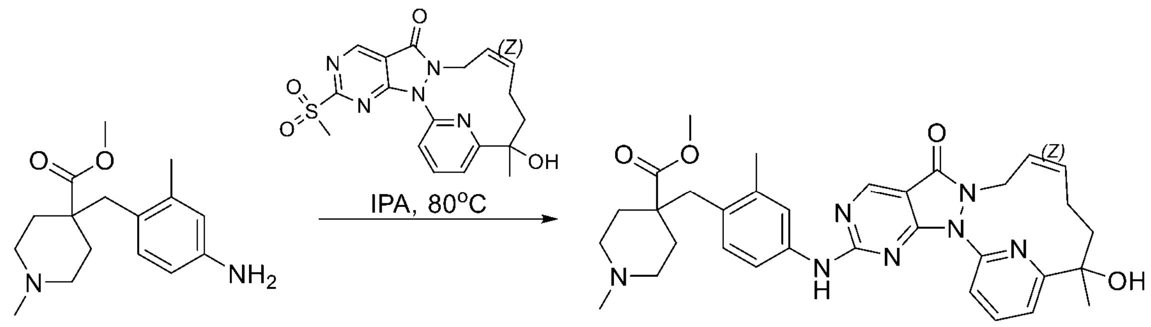


A solution of methyl 4-(4-amino-2-methylbenzyl)-1-methylpiperidine-4-carboxylate (120 mg, 0.43 mmol) and (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (262 mg, 0.65 mmol) in isopropanol (2.0 mL) was heated to 80 °C and stirred for 16 h. After cooling, the mixed reaction solution was filtered and concentrated under reduced pressure. The residue was purified by

C18 reverse phase column chromatography (MeCN:$H_2O$ (0.1% $NH_3.H_2O$) = 5%-95% elution) to give methyl (*Z*)-4-(4-((12-hydroxy-12-methyl-5-oxo-7,10,11,12-tetrahydro-5*H*-13,17-(azeno)pyrimido[4',5':3,4]pyrazolo[1,2-*a*][1,2]diazacyclotridecin-2-yl)amino)-2-methylbenzyl)-1-methylpiperidine-4-carboxylate (20 mg, yield: 7.70%), LC-MS *m*/*z*: 598 $[M + H]^+$; $^1H$ NMR (400 MHz, MeOD-*d*4): δ 8.84 (s, 1H), 7.98 (t, *J* = 8.0 Hz, 1H), 7.85-7.82 (m, 1H), 7.63-7.59 (m, 2H), 7.40-7.35 (m, 1H), 6.95 (t, J=8.0Hz, 1H), 5.52-5.40 (m, 2H), 4.64-4.59 (m, 2H), 3.64 (s, 3H), 2.89-2.82 (m, 4H), 2.29 (s, 3H), 2.27 (s, 3H), 2.24-2.18 (m, 3H), 2.14-2.05 (m, 2H), 2.04-1.96 (m, 1H), 1.94-1.85 (m, 1H), 1.69 (s, 3H), 1.68-1.60 (m, 2H).

**Example 61: Synthesis of (*R,Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one and (*S,Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (Compounds 219A and B)**

**1) Synthesis of (*Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one**

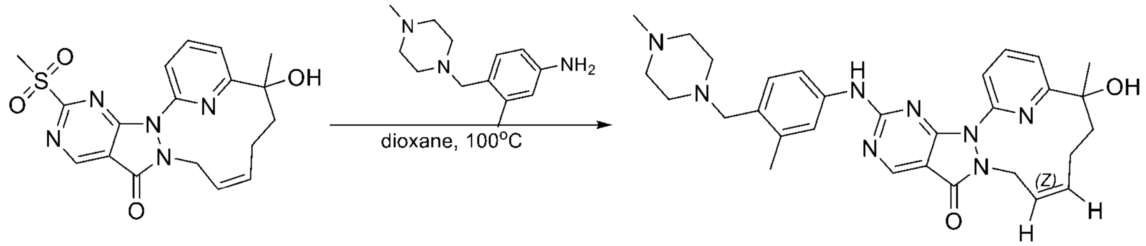


A solution of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (500 mg, 1.24 mmol) and 3-methyl-4-((4-methylpiperazin-1-yl)methyl)aniline (328.3 mg, 1.49 mmol) in 1,4-dioxane (50 mL) was heated to 100 °C and stirred overnight. After cooling, the mixed reaction solution was filtered and concentrated under reduced pressure. The residue was purified by silica gel column chromatography (methanol:dichloromethane = 0%-10% elution) to give (*Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (490 mg, yield: 73.0%), LC-MS *m*/*z*: 541 $[M + H]^+$.

**2) Synthesis of (*R,Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer and (*S,Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

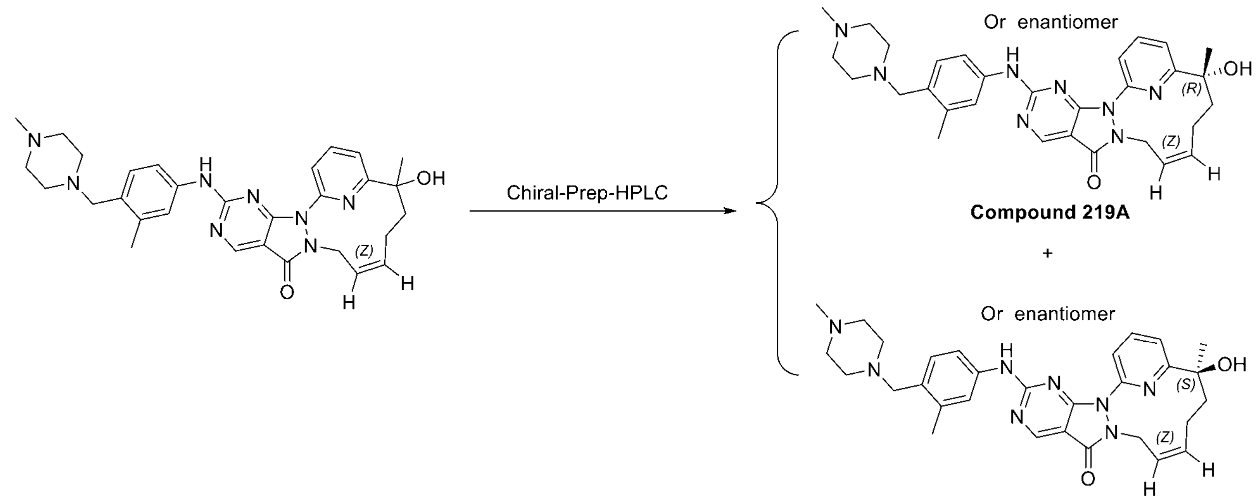


**Compound 219A**

+

**Compound 219B**

(*Z*)-12-Hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one (16 mg, 0.03 mmol) was separated and purified by preparative chiral high performance liquid chromatography (chromatography conditions: column: DAICELCHIRALPAK®IC, 250 × 40 mm 10 μm; mobile phase A: supercritical $CO_2$; mobile phase B: methanol (+ 0.1% 7.0 mol/L ammonia in MeOH); gradient: 54% B in 7.57 min; flow rate: 120 mL/min; column temperature: 25 °C; detection wavelength: 214 nm) to give two isomers: **compound 219A** and **compound 219B:**

**Compound 219A:** Peak 1 **(peak time: 2.239 min):** (*R,Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (3 mg, yield: 18.75%), LC-MS *m/z:* 541 $[M + H]^+$;
$^1H$ NMR (400 MHz, MeOD-d4) δ 8.84 (s, 1H), 7.98 (s, 1H), 7.85 (d, *J* = 8.0 Hz, 1H), 7.66 - 7.59 (m, 2H), 7.41 (s, 1H), 7.17 (d, *J* = 8.0 Hz, 1H), 5.47 (s, 2H), 4.63 (s, 2H), 3.49 (s, 2H), 2.56 (s, 7H), 2.35 (d, *J* = 8.0 Hz, 6H), 2.24 - 2.13 (m, 2H), 2.02 - 1.96 (m, 1H), 1.91 (s, 1H), 1.69 (s, 3H).

**Compound 219B:** Peak 2 **(peak time: 4.073 min):** (*S,Z*)-12-hydroxy-12-methyl-2-((3-methyl-4-((4-methylpiperazin-1-yl)methyl)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer (3 mg, yield: 18.75%), LC-MS *m/z:* 541 $[M + H]^+$;
$^1H$ NMR (400 MHz, MeOD-d4) δ 8.84 (s, 1H), 7.98 (t, *J* = 8.0 Hz, 1H), 7.85 (d, *J* = 8.4 Hz, 1H), 7.66 - 7.58 (m, 2H), 7.42 (d, *J* = 8.8 Hz, 1H), 7.17 (d, *J* = 8.4 Hz, 1H), 5.49 - 5.42 (m, 2H), 4.62 (d, *J* = 6.8 Hz, 2H), 3.49 (s, 2H), 2.55 (s, 7H), 2.35 (d, *J* = 11.6 Hz, 6H), 2.17 (s, 2H), 2.00 (s, 1H), 1.90 (s, 1H), 1.69 (s, 3H).

### Example 62: Synthesis of (*Z*)-2-((3-chloro-4-((2-methyloctahydrocyclopenta[*c*]pyrrol-5-yl)oxy)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 221)

#### 1) Synthesis of *tert-butyl* 5-(2-chloro-4-nitrophenoxy)hexahydrocyclopenta[*c*]pyrrole-2(1*H*)-carboxylate

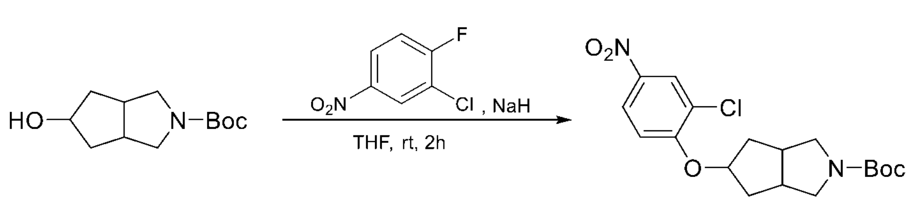


Sodium hydride (137 mg, 5.714 mmol) was added in batches to a solution of *tert*-butyl 5-hydroxyhexahydrocyclopenta[c]pyrrole-2(1*H*)-carboxylate (778 mg, 3.43 mmol) in tetrahydrofuran (3 mL) at 0 °C, and the resulting mixed reaction solution was stirred at room temperature for 30 min. Then, a solution of 2-chloro-1-fluoro-4-nitrobenzene (500 mg, 2.81 mmol) in tetrahydrofuran (3 mL) was added dropwise to the above mixture. The resulting mixed reaction solution was

stirred at room temperature for 2 h. After cooling, the mixed reaction solution was diluted with water and extracted with ethyl acetate (6 × 50 mL). The combined organic phases were washed with saturated brine (3 × 20 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give *tert*-butyl 5-(2-chloro-4-nitrophenoxy)hexahydrocyclopenta[*c*]pyrrole-2(1*H*)-carboxylate (860 mg, yield: 78.8%), LC-MS *m/z:* 383 $[M + H]^+$.

**2) Synthesis of 5-(2-chloro-4-nitrophenoxy)octahydrocyclopenta[c]pyrrole**

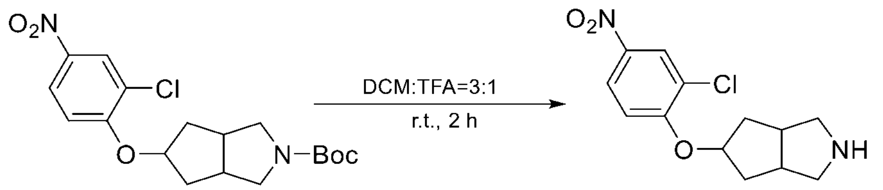


A solution of *tert*-butyl 5-(2-chloro-4-nitrophenoxy)hexahydrocyclopenta[*c*]pyrrole-2(1*H*)-carboxylate (860 mg, 2.251 mmol) in dichloromethane (6 mL) and 2,2,2-trifluoroacetic acid (2 mL) was stirred at room temperature for 2 h. The mixed reaction solution was diluted with water and extracted with ethyl acetate (5 × 40 mL). The combined organic phases were washed with saturated brine (5 × 10 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 5-(2-chloro-4-nitrophenoxy)octahydrocyclopenta[*c*]pyrrole (460 mg, yield: 72.5%), LC-MS *m/z*: 283 $[M + H]^+$.

**3) Synthesis of 5-(2-chloro-4-nitrophenoxy)-2-methyloctahydrocyclopenta[*c*]pyrrole**

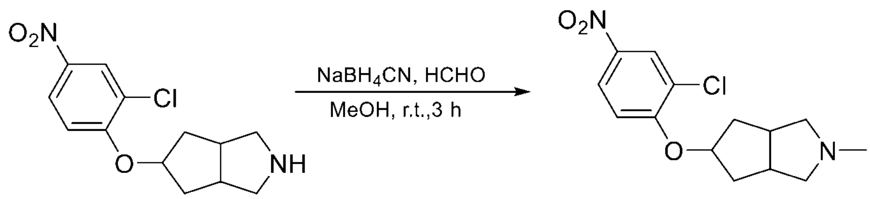


Paraformaldehyde (98 mg, 3.262 mmol) was added to a mixture in a solution of 5-(2-chloro-4-nitrophenoxy) octahydrocyclopenta[c]pyrrole (460 mg, 1.631 mmol) and sodium cyanoborohydride (123 mg, 1.957 mmol) in methanol (4 mL) at room temperature. The resulting mixed reaction solution was stirred at room temperature for 3 h. The mixed reaction solution was diluted with water and extracted with ethyl acetate (3 × 60 mL). The combined organic phases were washed with saturated brine (3 × 10 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 5-(2-chloro-4-nitrophenoxy)-2-methyloctahydrocyclopenta[*c*]pyrrole (310 mg, yield: 64.2%), LC-MS *m/z*: 297 $[M + H]^+$.

**4) Synthesis of 3-chloro-4-((2-methyloctahydrocyclopenta[c]pyrrol-5-yl)oxy)aniline**

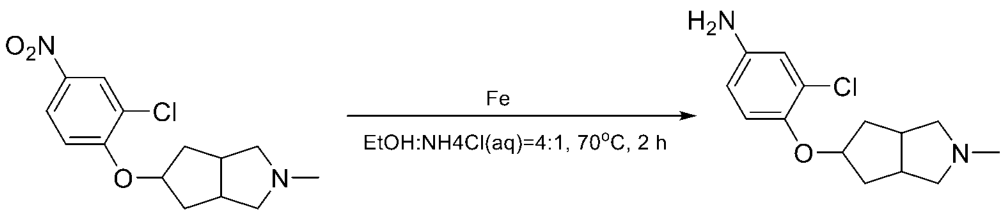


A solution of 5-(2-chloro-4-nitrophenoxy)-2-methyloctahydrocyclopenta[*c*]pyrrole (310 mg, 1.047 mmol), $NH_4Cl$

(167 mg, 3.142 mmol) and Fe (293 mg, 5.236 mmol) in ethanol (4 mL) and water (1 mL) was heated to 70 °C and stirred for 2 h. The mixed reaction solution was diluted with water and extracted with ethyl acetate (3 × 50 mL). The combined organic phases were washed with saturated brine (3 × 10 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give 3-chloro-4-((2-methyloctahydrocyclopenta[*c*]pyrrol-5-yl)oxy)aniline (98 mg, yield: 35.2%), LC-MS *m*/*z:* 267 [M + H]$^+$.

**5) Synthesis of (*Z*)-2-((3-chloro-4-((2-methyloctahydrocyclopenta[*c*]pyrrol-5-yl)oxy)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

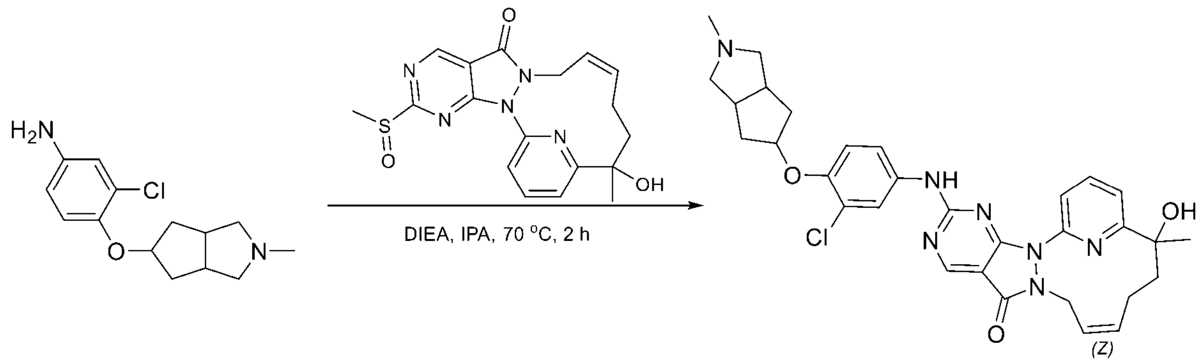


*N,N*-Diisopropylethylamine (52.12 mg, 0.40 mmol) was added to a solution of 3-chloro-4-((2-methyloctahydrocyclopenta[*c*]pyrrol-5-yl)oxy)aniline (41 mg, 0.156 mmol) and (*Z*)-12-hydroxy-12-methyl-2-(methylsulfinyl)-7,10,11,12-tetrahydro-5*H*-13,17-(azeno)pyrimido[4',5':3,4]pyrazolo[1,2-*a*][1,2]diazacyclotridecin-5-one (50 mg, 0.130 mmol) in isopropanol (2 mL) at room temperature. The resulting mixed reaction solution was heated to 70 °C and stirred for 2 h. After cooling, the resulting mixed reaction solution was diluted with water and extracted with ethyl acetate (3 × 50 mL). The combined organic phases were washed with saturated brine (3 × 10 mL), dried over anhydrous sodium sulfate, filtered, and concentrated under reduced pressure. The residue was purified by C18 reverse phase column chromatography (ACN (0.1% FA):water (0.1% FA) = 25%-65% elution) to give (*Z*)-2-((3-chloro-4-((2-methyloctahydrocyclopenta[*c*]pyrrol-5-yl)oxy)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (10 mg, yield: 13.1%), LC-MS *m*/*z:* 588 [M + H]$^+$;
$^1$H NMR (400 MHz, MeOD-d4): δ 8.83 (s, 1H), 8.13 (s, 1H), 8.00 (t, *J* = 7.6 Hz, 1H), 7.81 (d, *J* = 7.6 Hz, 1H), 7.60 (d, *J* = 7.6 Hz, 1H), 7.42 (d, *J* = 9.2 Hz, 1H), 7.11 (t, *J* = 10.8 Hz, 1H), 5.54-5.44 (m, 2H), 5.16 (s, 1H), 4.61 (d, *J* = 6.8 Hz, 2H), 3.87 (s, 1H), 3.65 (d, *J* = 10.8 Hz, 1H), 3.47-3.39 (m, 1H), 3.13 (s, 3H), 2.97 (s, 3H), 2.18-2.07 (m, 4H), 2.03-1.98 (m, 1H), 1.94-1.90 (m, 1H), 1.68 (s, 3H), 1.34-1.28 (m, 1H).

**Example 63: Synthesis of (*Z*)-2-((3-bromo-4-((2-methyloctahydrocyclopenta[*c*]pyrrol-5-yl)oxy)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (Compound 222)**

**1) Synthesis of *tert*-butyl 5-(2-bromo-4-nitrophenoxy)hexahydrocyclopenta[*c*]pyrrole-2(1*H*)-carboxylate**

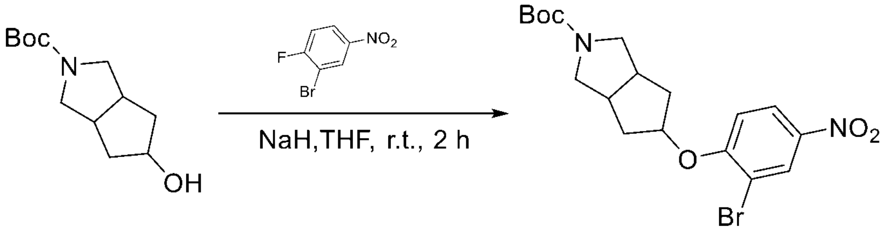


Sodium hydride (210.0 mg, 8.75 mmol) was added to a solution of *tert*-butyl 5-hydroxyhexahydrocyclopenta[*c*]

pyrrole-2(1*H*)-carboxylate (1 g, 4.41 mmol) in tetrahydrofuran (10 mL) at 0 °C, and the mixture was stirred at 0 °C for 1 h. 2-Bromo-1-fluoro-4-nitrobenzene (1.45 g, 6.59 mmol) was added to the above mixed reaction solution at 0 °C. The resulting mixed reaction solution was stirred at room temperature for 4 h, and the resulting mixed reaction solution was concentrated under reduced pressure. The residue was purified by C18 reverse phase column chromatography ($CH_3CN:H_2O$ (0.1% $NH_3.H_2O$) = 5%-95% elution) to give *tert*-butyl 5-(2-bromo-4-nitrophenoxy)hexahydrocyclopenta[*c*]pyrrole-2(1*H*)-carboxylate (1700 mg, yield: 90.6%), LC-MS *m/z:* 427 [M + H]$^+$.

**2) Synthesis of 5-(2-bromo-4-nitrophenoxy)octahydrocyclopenta[*c*]pyrrole**

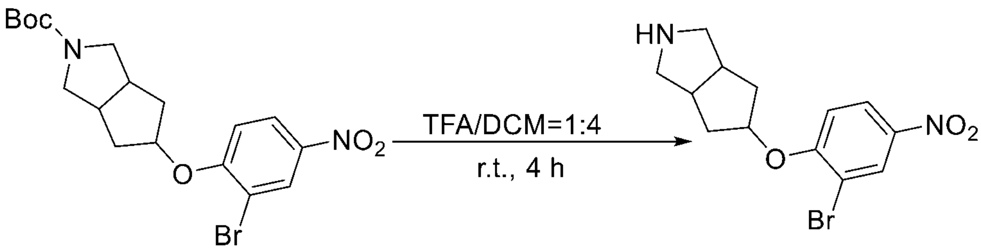


2,2,2-Trifluoroacetic acid (2 mL) was added dropwise to a solution of *tert*-butyl 5-(2-bromo-4-nitrophenoxy) hexahydrocyclopenta[*c*]pyrrole-2(1*H*)-carboxylate (1700 mg, 3.99 mmol) in dichloromethane (8 mL) at room temperature. The resulting mixed reaction solution was stirred at room temperature for 4 h. The resulting mixed reaction solution was concentrated under reduced pressure. The residue was purified by C18 reverse phase column chromatography ($CH_3CN:H_2O$ (0.1% $NH_3.H_2O$) = 5%-95% elution) to give 5-(2-bromo-4-nitrophenoxy)octahydrocyclopenta[c]pyrrole (1000 mg, yield: 76.7%), LC-MS *m*/*z*: 327 [M + H]$^+$.

**3) Synthesis of 5-(2-bromo-4-nitrophenoxy)-2-methyloctahydrocyclopenta[*c*]pyrrole**

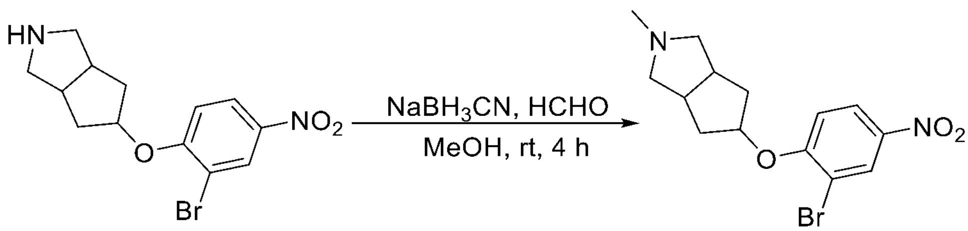


Sodium cyanoborohydride (386.0 mg, 6.13 mmol) was added to a solution of 5-(2-bromo-4-nitrophenoxy) octahydrocyclopenta[*c*]pyrrole (1 g, 3.10 mmol) and paraformaldehyde (276.0 mg, 9.20 mmol) in methanol (10 mL) at room temperature. The resulting mixed reaction solution was stirred at room temperature for 4 h. The resulting mixed reaction solution was concentrated under reduced pressure. The residue was purified by C18 reverse phase column chromatography ($CH_3CN:H_2O$ (0.1% $NH_3.H_2O$) = 5%-95% elution) to give 5-(2-bromo-4-nitrophenoxy)-2-methyloctahydrocyclopenta[c]pyrrole (500 mg, yield: 47.8%), LC-MS *m*/*z*: 341 [M + H]$^+$.

**4) Synthesis of 3-bromo-4-((2-methyloctahydrocyclopenta[c]pyrrol-5-yl)oxy)aniline**

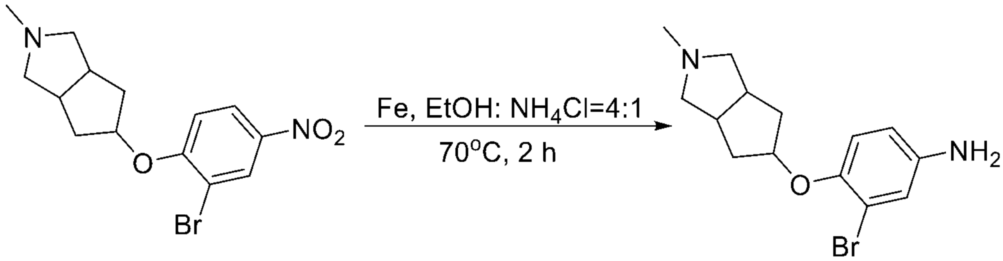


A solution of 5-(2-bromo-4-nitrophenoxy)-2-methyloctahydrocyclopenta[*c*]pyrrole (500 mg, 1.47 mmol) and Fe (410.0 mg, 7.32 mmol) in aqueous ammonium chloride solution (1 mL) and ethanol (4 mL) was heated to 70 °C and stirred for 2 h. After cooling, the mixed reaction solution was filtered, and the filter cake was washed with methanol (3 × 50 mL). The filtrate was collected and concentrated under reduced pressure to give a crude product, which was used directly in the next step without further purification. LC-MS *m/z*: 311 [M + H]$^+$;

**5) Synthesis of (*Z*)-2-((3-bromo-4-((2-methyloctahydrocyclopenta[*c*]pyrrol-5-yl)oxy)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one**

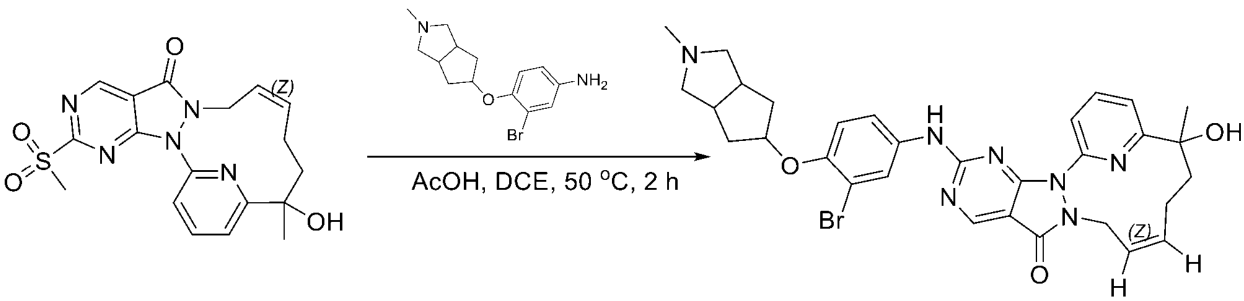


A solution of (Z)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol), 3-bromo-4-((2-methyloctahydrocyclopenta[*c*]pyrrol-5-yl)oxy)aniline (116.33 mg, 0.37 mmol), and acetic acid (89.77 mg, 1.49 mmol) in 1,2-dichloroethane (3 mL) was heated to 50 °C and stirred for 2 h. After cooling, the mixed reaction solution was filtered and concentrated under reduced pressure. The residue was purified by C18 reverse phase column chromatography ($CH_3CN$:$H_2O$ (0.1% $NH_3.H_2O$) = 5%-95% elution) to give (*Z*)-2-((3-bromo-4-((2-methyloctahydrocyclopenta[*c*]pyrrol-5-yl)oxy)phenyl)amino)-12-hydroxy-12-methyl-7,10,11,12-tetrahydro-5*H*-13,17-(azeno)pyrimido[4',5':3,4]pyrazolo[1,2-*a*][1,2]diazacyclotridecin-5-one (5 mg, yield: 3.18%), LC-MS *m/z:* 632 [M + H]$^+$;
$^1$H NMR (400 MHz, MeOD-d4):δ 8.81 (s, 1H), 8.54 (s, 1H), 8.03 (t, *J* = 8.0 Hz, 1H), 7.81 (d, *J = 8.0* Hz, 1H), 7.59 (d, *J* = 8.0 Hz, 1H), 7.41 (d, *J* = 8.0 Hz, 1H), 7.04 (d, *J* = 8.0 Hz, 1H), 5.56 - 5.42 (m, 2H), 5.05 (s, 1H), 4.58 (d, *J* = 8.0 Hz, 2H), 3.61 - 3.52 (m, 2H), 3.20-3.01 (m, 4H), 2.87 (s, 3H), 2.23 - 2.06 (m, 6H), 2.04 - 1.85 (m, 2H), 1.68 (s, 3H).

**Example 64: Synthesis of (*Z*)-12-hydroxy-12-methyl-2-((4-((2-methyloctahydrocyclopenta[*c*]pyrrol-5-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one (Compound 223)**

**1) Synthesis of *tert-butyl* 5-(4-nitro-2-(trifluoromethyl)phenoxy)hexahydrocyclopenta[*c*]pyrrole-2(1*H*)-carboxylate**

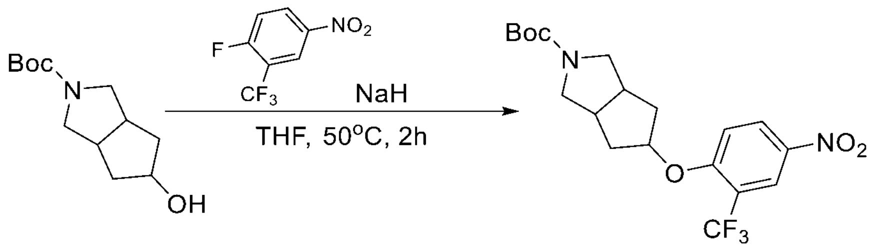


Sodium hydride (110 mg, 4.4 mmol) was added in batches to a solution of *tert*-butyl 5-hydroxyhexahydrocyclopenta[*c*]pyrrole-2(1*H*)-carboxylate (500 mg, 2.20 mmol) in tetrahydrofuran (10 mL) at 0 °C, and the mixture was stirred at room temperature for 1 h. 1-Fluoro-4-nitro-2-(trifluoromethyl)benzene (690 mg, 3.30 mmol) was added to the above mixed reaction solution at room temperature. The resulting mixed reaction solution was heated to 50 °C and stirred for 2 h. After cooling, the resulting mixed reaction solution was concentrated under reduced pressure. The residue was dissolved in methanol (10 mL), and the solution was purified by C18 reverse phase column chromatography (ACN:$H_2O$ = 75%-90% elution) to give *tert-butyl* 5-(4-nitro-2-(trifluoromethyl)phenoxy)hexahydrocyclopenta[*c*]pyrrole-2(1*H*)-carboxylate (415 mg, yield: 45.3%), LC-MS *m/z*: 417 [M + H]$^+$.

**2) Synthesis of 5-(4-nitro-2-(trifluoromethyl)phenoxy)octahydrocyclopenta[*c*]pyrrole**

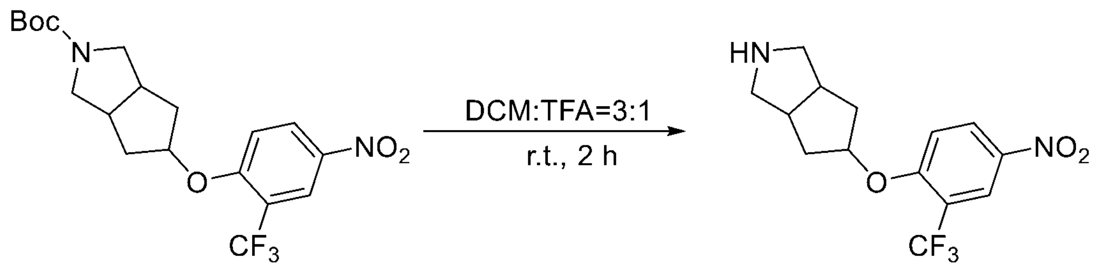


A solution of *tert*-butyl 5-(4-nitro-2-(trifluoromethyl)phenoxy)hexahydrocyclopenta[c]pyrrole-2(1H)-carboxylate (400 mg, 0.96 mmol) in dichloromethane (4.5 mL) and 2,2,2-trifluoroacetic acid (1.5 mL) was stirred at room temperature for 2 h. The resulting mixed reaction solution was concentrated under reduced pressure. The residue was purified by C18 reverse phase column chromatography (MeCN:$H_2O$ = 75%-90% elution) to give 5-(4-nitro-2-(trifluoromethyl)phenoxy) octahydrocyclopenta[c]pyrrole (300 mg, yield: 98.7%), LC-MS *m/z:* 317 [M + H]$^+$.

**3) Synthesis of 2-methyl-5-(4-nitro-2-(trifluoromethyl)phenoxy)octahydrocyclopenta[*c*]pyrrole**

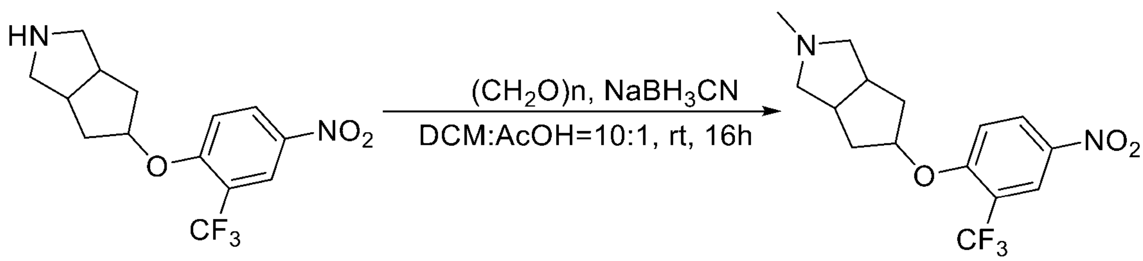


Paraformaldehyde (85 mg, 2.85 mmol) was added to a solution of 5-(4-nitro-2-(trifluoromethyl)phenoxy)octahydrocyclopenta[*c*]pyrrole (300 mg, 0.95 mmol) and sodium cyanoborohydride (119.40 mg, 1.9 mmol) in dichloromethane (3 mL) and acetic acid (0.3 mL) at room temperature. The resulting mixed reaction solution was stirred at room temperature for 2 h. The resulting mixed reaction solution was concentrated under reduced pressure. The residue was dissolved in *N,N*-dimethylformamide (3 mL), and the solution was purified by C18 reverse phase column chromatography (MeCN:$H_2O$ = 25%-40% elution) to give 2-methyl-5-(4-nitro-2-(trifluoromethyl)phenoxy)octahydrocyclopenta[*c*]pyrrole (300 mg, yield: 95.7%), LC-MS *m/z:* 331 [M + H]$^+$.

**4) Synthesis of 4-((2-methyloctahydrocyclopenta[*c*]pyrrol-5-yl)oxy)-3-(trifluoromethyl)aniline**

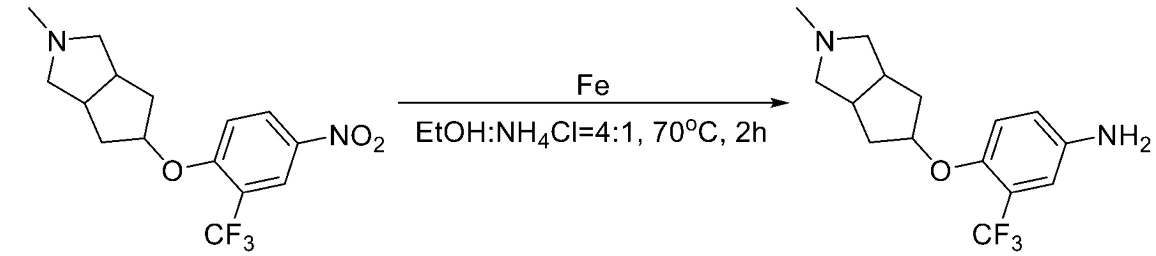


A solution of 2-methyl-5-(4-nitro-2-(trifluoromethyl)phenoxy)octahydrocyclopenta[*c*]pyrrole (300 mg, 0.91 mmol) and Fe (254.31 mg, 4.55 mmol) in ethanol (2.4 mL) and aqueous ammonium chloride solution (0.6 mL) was heated to 70 °C and stirred for 2 h. The resulting mixed reaction solution was filtered, and the filter cake was washed with methanol. The filtrate was collected and concentrated under reduced pressure to give 4-((2-methyloctahydrocyclopenta [*c*]pyrrol-5-yl)oxy)-3-(trifluoromethyl)aniline (250 mg, yield: 91.7%), LC-MS *m*/*z:* 301 $[M + H]^+$.

**5) Synthesis of (*Z*)-12-hydroxy-12-methyl-2-((4-((2-methyloctahydrocyclopenta[*c*]pyrrol-5-yl)oxy)-3-(trifluoromethyl)phenyl)amino)-7,10,11,12-tetrahydro-5*H*-13,17-(azeno)pyrimido[4',5':3,4]pyrazolo[1,2-*a*][1,2]diazacyclotridecin-5-one**

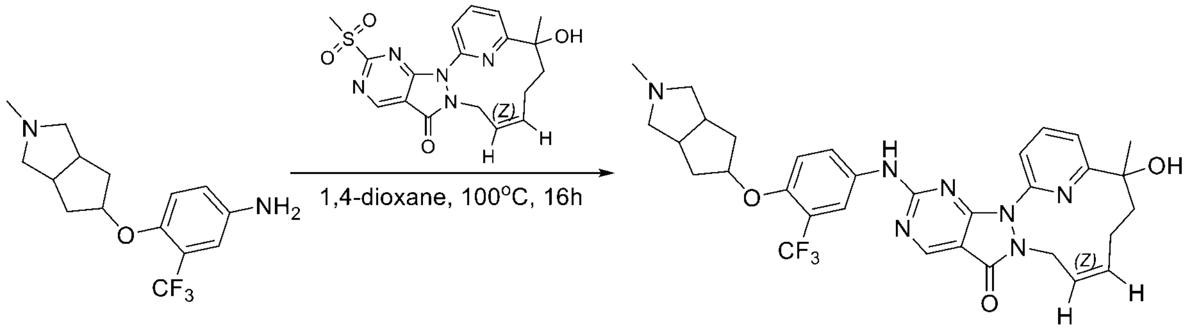


A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (100 mg, 0.25 mmol) and 4-((2-methyloctahydrocyclopenta[*c*] pyrrol-5-yl)oxy)-3-(trifluoromethyl)aniline (225.24 mg, 0.75 mmol) in 1,4-dioxane (1 mL) was heated to 100 °C and stirred for 16 h. After cooling, the resulting mixed reaction solution was concentrated under reduced pressure. The residue was dissolved in *N,N*-dimethylformamide (2 mL), and the solution was purified by C18 reverse phase column chromatography ($MeCN:H_2O$ = 50%-65% elution) to give (*Z*)-12-hydroxy-12-methyl-2-((4-((2-methyloctahydrocyclopenta[*c*]pyrrol-5-yl) oxy)-3-(trifluoromethyl)phenyl)amino)-7,10,11,12-tetrahydro-5*H*-13,17-(azeno)pyrimido[4',5':3,4]pyrazolo[1,2-*a*][1,2] diazacyclotridecin-5-one (45 mg, yield: 29.1%), LC-MS *m*/*z:* 622 $[M + H]^+$;
$^1H$ NMR (400 MHz, MeOD-d4) δ 8.84 (s, 1H), 8.17 (s, 1H), 7.92 (t, J = 7.9 Hz, 1H), 7.75 (d, J = 8.0 Hz, 1H), 7.65 (s, 1H), 7.60 (d, J = 7.7 Hz, 1H), 7.14 (d, J = 9.1 Hz, 1H), 5.46 (dd, J = 13.5, 6.9 Hz, 2H), 4.92 (s, 1H), 4.60 (d, J = 6.7 Hz, 2H), 3.04 (d, J = 7.8 Hz, 2H), 2.88 (s, 2H), 2.56 (d, J = 8.2 Hz, 2H), 2.44 (s, 3H), 2.18 (dd, J = 12.6, 6.9 Hz, 4H), 2.03 - 1.95 (m, 1H), 1.91 (d, J = 13.6 Hz, 3H), 1.68 (s, 3H).

**Example 65: Synthesis of (12*R,Z*)-12-hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one and**

**(12*S*,*Z*)-12-hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (224A and B)**

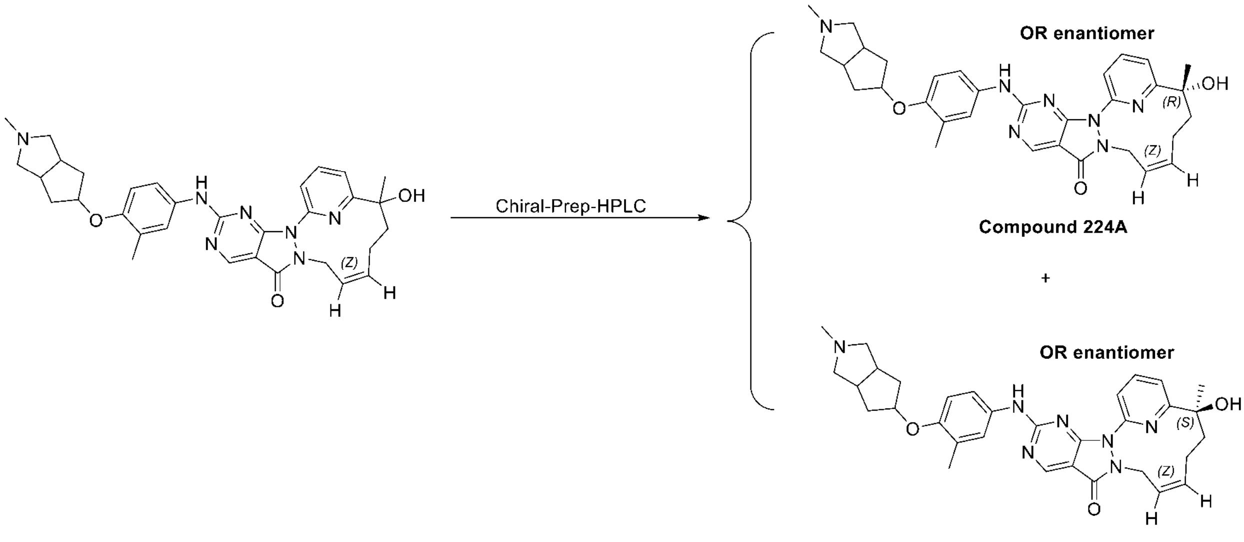


Compound 224B

(*Z*)-12-Hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido[4',5':3,4]pyrazolo[1,2-*a*][1,2]diazacyclotridecin-5-one (60 mg, 0.11 mmol) was purified by preparative chiral high performance liquid chromatography (chromatography conditions: column: DAICELCHIRALPAK®IC, 250 × 40 mm 10 μm; mobile phase A: MeCN; mobile phase B: EtOH (+ 0.2% 7.0 mol/L ammonia in MeOH); gradient: A:B (70:30) in 17.5 min; flow rate: 35 mL/min; column temperature: room temperature) to give two isomers: **compound 224A** and **compound 224B:**

**Compound 224A:** Peak 1 **(peak time: 8.274 min); (12*R*,*Z*)-12-hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido [4',5':3,4]pyrazolo[1,2-*a*][1,2]diazacyclotridecin-5-one or enantiomer** (20 mg, yield: 33.33%).
$^1$H NMR (400 MHz, MeOD) δ 8.80 (s, 1H), 7.95 (t, J = 8.0 Hz, 1H), 7.82 (d, J = 8.0 Hz, 1H), 7.61-7.51 (m, 2H), 7.38-7.34 (m, 2H), 7.36 (d, J = 9.0 Hz, 1H), 6.86 (d, J = 8.0 Hz, 1H), 4.83-4.80 (m, 2H), 4.62-4.57 (m, 2H), 3.00 (s, 2H), 2.85 (s, 2H), 2.55 (s, 2H), 2.44 (s, 3H), 2.21 (s, 3H), 2.19-2.10 (m, 4H), 2.04-1.96 (m, 1H), 1.92-1.83 (m, 3H), 1.68 (s, 3H).

**Compound 224B:** Peak 1 **(peak time: 11.270 min); (12*S*,*Z*)-12-hydroxy-12-methyl-2-((3-methyl-4-((2-methyloctahydrocyclopenta[*c*]pyrrol-5-yl)oxy)phenyl)amino)-7,10,11,12-tetrahydro-5*H*-13,17-(azeno)pyrimido [4',5':3,4]pyrazolo[1,2-*a*][1,2]diazacyclotridecin-5-one or enantiomer** (22 mg, yield: 36.67%).
$^1$H NMR (400 MHz, MeOD) δ 8.80 (s, 1H), 7.95 (t, J = 8.0 Hz, 1H), 7.82 (d, J = 8.0 Hz, 1H), 7.61-7.51 (m, 2H), 7.38-7.34 (m, 2H), 7.36 (d, J = 9.0 Hz, 1H), 6.86 (d, J = 8.0 Hz, 1H), 4.83-4.80 (m, 2H), 4.62-4.57 (m, 2H), 3.00 (s, 2H), 2.85 (s, 2H), 2.55 (s, 2H), 2.44 (s, 3H), 2.21 (s, 3H), 2.19-2.10 (m, 4H), 2.04-1.96 (m, 1H), 1.92-1.83 (m, 3H), 1.68 (s, 3H).

**Example 66: Synthesis of (*R*,*Z*)-12-hydroxy-12-methyl-2-((3-methyl-4-(((1*R*,3*s*,5*S*)-8-methyl-8-azabicyclo[3.2.1] octan-3-yl)amino)phenyl)amino)-7,10,11,12-tetrahydro-5*H*-13,17-(azeno)pyrimido[4',5':3,4]pyrazolo[1,2-*a*][1,2] diazacyclotridecin-5-one and (*S*,*Z*)-12-hydroxy-12-methyl-2-((3-methyl-4-(((1*R*,3*s*,5*S*)-8-methyl-8-azabicyclo [3.2.1]octan-3-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (Compounds 225A and B)**

**1) Synthesis of *tert*-butyl (1*R*,3s,5*S*)-3-((2-methyl-4-nitrophenyl)amino)-8-azabicyclo[3.2.1]octane-8-carboxylate**

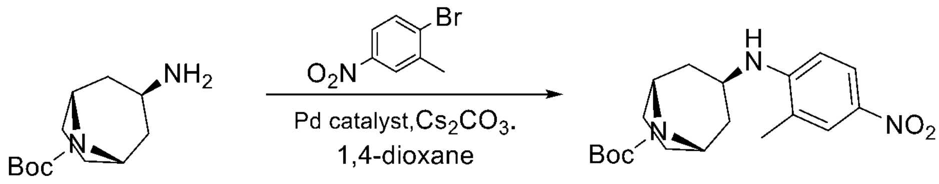


A solution of 1-bromo-2-methyl-4-nitrobenzene (1.00 g, 4.63 mmol), *tert*-butyl (1*R*,3*s*,5*S*)-3-amino-8-azabicyclo [3.2.1]octane-8-carboxylate (1.57 g, 6.94 mmol), (SP-4-1)-[1,3-bis[2,6-bis(1-ethylpropyl)phenyl]-4,5-dichloro-1,3-dihydro-2*H*-imidazol-2-ylidene]dichloro(2-methylpyridine)palladium (389 mg, 0.46 mmol), and cesium carbonate (4.51 g, 13.89 mmol) in 1,4-dioxane (20 mL) was heated to 100 °C and stirred overnight under a nitrogen atmosphere. After cooling, the mixed reaction solution was concentrated under reduced pressure. The residue was purified by silica gel column chromatography (ethyl acetate:petroleum ether = 10%-20% elution) to give *tert*-butyl (1*R*,3*s*,5*S*)-3-((2-methyl-4-nitrophenyl)amino)-8-azabicyclo[3.2.1]octane-8-carboxylate (1.50 g, yield: 89%), LC-MS *m/z:* 362 $[M + H]^+$.

**2) Synthesis of (1*R*,3*s*,5*S*)-*N*-(2-methyl-4-nitrophenyl)-8-azabicyclo[3.2.1]octan-3-amine**

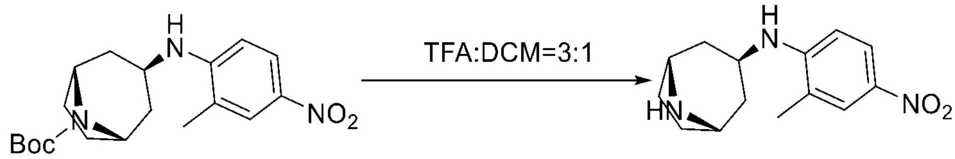


A solution of *tert*-butyl (1*R*,3*s*,5*S*)-3-((2-methyl-4-nitrophenyl)amino)-8-azabicyclo[3.2.1]octane-8-carboxylate (1.50 g, 4.16 mmol) in dichloromethane (20 mL) and 2,2,2-trifluoroacetic acid (6 mL) was stirred at room temperature for 3 h. The resulting mixed reaction solution was concentrated under reduced pressure. The residue was purified by silica gel column chromatography (methanol:dichloromethane = 0%-10% elution) to give (1*R*,3*s*,5*S*)-*N*-(2-methyl-4-nitrophenyl)-8-azabicyclo[3.2.1]octan-3-amine (997 mg, yield: 92%), LC-MS *m/z:* 262 $[M + H]^+$.

**3) Synthesis of (1*R*,3*s*,5*S*)-8-methyl-*N*-(2-methyl-4-nitrophenyl)-8-azabicyclo[3.2.1]octan-3-amine**

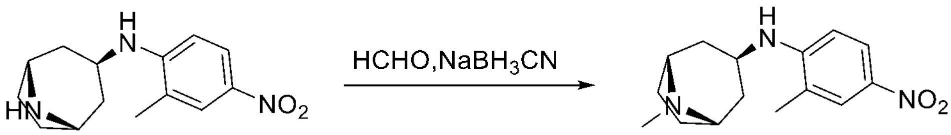


A solution of (1*R*,3*s*,5*S*)-*N*-(2-methyl-4-nitrophenyl)-8-azabicyclo[3.2.1]octan-3-amine (997 mg, 3.82 mmol), sodium cyanoborohydride (480 mg, 7.64 mmol), and formaldehyde (229 mg, 7.64 mmol) in acetic acid (0.3 mL) and dichloromethane (3 mL) was stirred at room temperature for 1 h. The resulting mixed reaction solution was concentrated under reduced pressure. The residue was purified by silica gel column chromatography (methanol:dichloromethane = 0%-10% elution) to give (1*R*,3*s*,5*S*)-8-methyl-*N*-(2-methyl-4-nitrophenyl)-8-azabicyclo[3.2.1]octan-3-amine (980 mg, yield: 93%), LC-MS *m*/*z*: 276 $[M + H]^+$.

**4) Synthesis of 2-methyl-$N^1$-((1*R*,3*s*,5*S*)-8-methyl-8-azabicyclo[3.2.1]octan-3-yl)benzene-1,4-diamine**

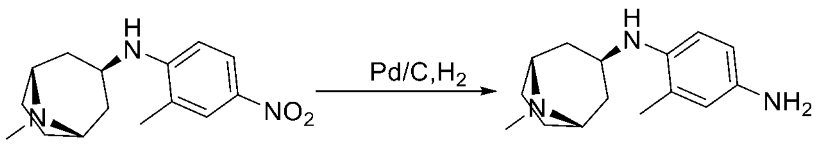


A solution of (1*R*,3*s*,5*S*)-8-Methyl-*N*-(2-methyl-4-nitrophenyl)-8-azabicyclo[3.2.1]octan-3-amine (100 mg, 0.36

mmol) and Pd/C (50 mg) in methanol (3 mL) was stirred at room temperature for 3 h under a hydrogen atmosphere (1 atm). The mixed reaction solution was filtered, and the filter cake was washed with methanol (3 × 20 mL). The filtrate was collected and concentrated under reduced pressure to give 2-methyl-N1-((1*R*,3*s*,5*S*)-8-methyl-8-azabicyclo[3.2.1]octan-3-yl)benzene-1,4-diamine (77 mg, yield: 86%), LC-MS *m*/*z*: 246 [M + H]+.

**5) Synthesis of (*Z*)-12-hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one**

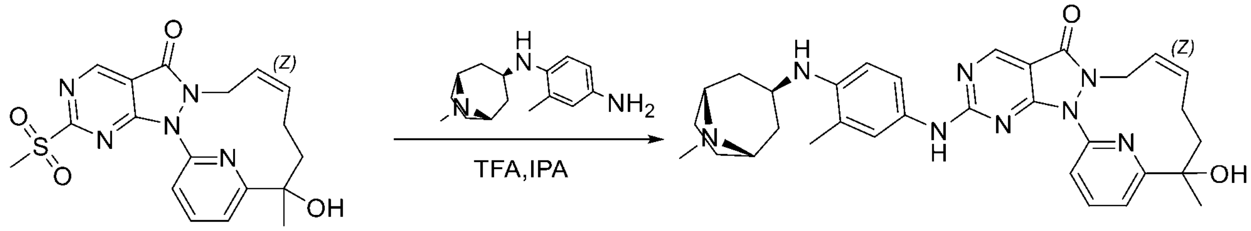


**Compound 225**

A solution of (*Z*)-12-hydroxy-12-methyl-2-(methylsulfonyl)-7,10,11,12-tetrahydro-5*H*-13,17-(azeno)pyrimido[4',5':3,4]pyrazolo[1,2-*a*][1,2]diazacyclotridecin-5-one (80 mg, 0.20 mmol), 2-methyl-N1-((1*R*,3*s*,5*S*)-8-methyl-8-azabicyclo[3.2.1]octan-3-yl)benzene-1,4-diamine (73 mg, 0.30 mmol), and 2,2,2,-trifluoroacetic acid (228 mg, 2.00 mmol) in isopropanol (2 mL) was heated to 80 °C and stirred for 16 h. The resulting mixed reaction solution was concentrated under reduced pressure. The residue was purified by C18 reverse phase column chromatography ($CH_3CN$:$H_2O$ (0.1% FA) = 30%-70% elution) to give (*Z*)-12-hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (23 mg, yield: 20%), LC-MS *m/z:* 567 [M + H]+;
$^1$H NMR (400 MHz, MeOD-d4) δ 8.78 (s, 1H), 7.94 (t, J = 7.9 Hz, 1H), 7.81 (d, J = 8.0 Hz, 1H), 7.59 (d, J = 7.1 Hz, 1H), 7.49 (s, 1H), 7.32 (s, 1H), 6.79 (s, 1H), 5.53 - 5.38 (m, 2H), 4.59 (d, J = 6.0 Hz, 2H), 3.98 (s, 3H), 2.82 (s, 3H), 2.37 (t, J = 13.5 Hz, 3H), 2.19 (dd, J = 13.4, 7.3 Hz, 7H), 1.99 (dd, J = 16.9, 8.3 Hz, 2H), 1.93 - 1.78 (m, 3H), 1.68 (s, 3H).

**6) Synthesis of (*R,Z*)-12-hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer and (*S,Z*)-12-hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one or enantiomer**

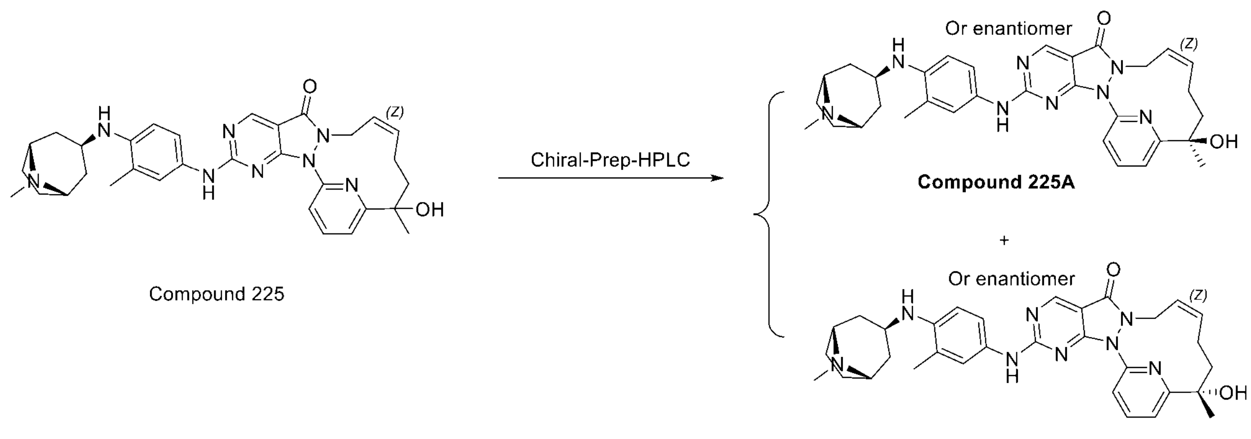


Compound 225

**Compound 225A**

+

**Compound 225B**

(*Z*)-12-Hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido[4',5':3,4]pyrazolo[1,2-*a*][1,2]diazacyclotridecin-5-one (15 mg, 0.03 mmol) was purified by preparative chiral high performance liquid chromatography (system: Waters SFC 80; column: DAICELCHIRALPAK®AD 250 × 30 mm 10 μm; mobile phase A: supercritical $CO_2$; mobile phase B: EtOH (+ 0.1% 7.0 mol/L ammonia in MEOH); A:B: 45:55; detection wavelength: 214 nm; flow rate: 70 mL/min; column temperature RT; column pressure 100 bar) to give two isomers: **compound 225A** and **compound 225B:**

**Compound 225A:** Peak 1 (**peak time: 3.558 min**): (*R*,*Z*)-12-hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido [4',5':3,4]pyrazolo[1,2-*a*][1,2]diazacyclotridecin-5-one or enantiomer (2.5 mg, yield: 16%).
$^1$H NMR (400 MHz, MeOD-$d_4$) δ 8.77 (s, 1H), 7.94 (t, J = 8.0 Hz, 1H), 7.82 (d, J = 8.0 Hz, 1H), 7.55 (t, J = 21.4 Hz, 2H), 7.29 (s, 1H), 6.75 (d, J = 8.6 Hz, 1H), 5.52 - 5.39 (m, 2H), 4.59 (d, J = 6.8 Hz, 2H), 3.97 (s, 3H), 2.82 (s, 3H), 2.37 (s, 3H), 2.25 - 2.11 (m, 7H), 2.00 (s, 1H), 1.83 (dd, J = 36.8, 23.6 Hz, 3H), 1.68 (s, 3H).

**Compound 225B:** Peak 2 **(peak time: 6.910 min):** (*S,Z*)-12-hydroxy-12-methyl-2-((3-methyl-4-(((1*R*,3*s*,5*S*)-8-methyl-8-azabicyclo[3.2.1]octan-3-yl)amino)phenyl)amino)-7,10,11,12-tetrahydro-5*H*-13,17-(azeno)pyrimido[4',5':3,4] pyrazolo[1,2-*a*][1,2]diazacyclotridecin-5-one or enantiomer (2.0 mg, yield: 13.3%).

$^1$H NMR (400 MHz, MeOD-$d_4$) δ 8.77 (s, 1H), 7.94 (t, J = 7.9 Hz, 1H), 7.82 (d, J = 8.1 Hz, 1H), 7.55 (t, J = 22.5 Hz, 2H), 7.29 (s, 1H), 6.76 (d, J = 8.2 Hz, 1H), 5.53 - 5.39 (m, 2H), 4.59 (d, J = 7.1 Hz, 2H), 3.97 (s, 3H), 2.82 (s, 3H), 2.36 (d, J = 11.3 Hz, 3H), 2.25 - 2.13 (m, 7H), 2.00 (s, 1H), 1.82 (dd, J = 32.9, 19.8 Hz, 3H), 1.68 (s, 3H).

### Example 67: Study of Representative Crystal Forms of Compound IE

Considering the ultra-broad-spectrum and ultra-high anti-tumor activity of compound IE, the polymorphs of compound IE and their preparation methods were further investigated in the present disclosure, and a series of new crystal forms were obtained. These crystal forms have the advantages of having good stability and good flowability and being easily pulverized, which make them more suitable for clinical formulation development. The preparation methods provided by the present disclosure have the following advantages: the processes are simple and easy to implement, and the reaction conditions are mild. In addition, it is not necessary to perform purification several times, and the operations are safe and environmentally friendly, which is conducive to the industrial production of the polymorphs. The crystal forms can meet the demands of clinical pharmaceutical formulation development and have very important clinical application value, and they are expected to be developed into completely new WEE1 and Yes dual-target small molecule inhibitors soon. The crystal forms and their preparation methods are specifically described as follows:
The structures of the compounds were determined by nuclear magnetic resonance (NMR). The NMR shifts (δ) are given in parts per million (ppm). The NMR analyses were performed using a Bruker avance-400MHz NMR spectrometer with deuterated dimethyl sulfoxide (DMSO-d6) or deuterated methanol (MeOD-d4) as the solvent and tetramethylsilane (TMS) as the internal standard, and the chemical shifts are given in $10^{-6}$ ppm.

The HPLC steps were performed using an Agilent 1260 high performance liquid chromatograph or an equivalent high performance liquid chromatograph (with a Sunfire C18 150 × 4.6 m chromatography column or an equivalent chromatography column).

The polymorphs of compound IE were characterized by their X-ray powder diffraction patterns. The X-ray powder diffraction patterns of the salts were collected on a Bruker D8 Advance powder diffractometer operating in reflection mode using Cu Kα radiation. The instrument used Cu Kα radiation (40 kV, 40 mA) and operated at room temperature using an SSD160-2 detector. The 2θ scan range was from 3° to 40°, and the scan speed was 0.1 s/step. The diffraction patterns were analyzed using DIFFRAC.MEA.CENTER software.

XRPD samples were prepared by placing a sample on a monocrystalline silicon wafer and pressing the sample powder with a glass slide or an equivalent to ensure that the sample had a flat surface and an appropriate height. The sample holder was then placed into the Bruker D8 Advance instrument, and the X-ray powder diffraction pattern was collected using the instrumental parameters described above. Measurement differences associated with such X-ray powder diffraction analysis results result from a variety of factors including: (a) errors in sample preparation (e.g., sample height), (b) instrument errors, (c) calibration errors, (d) operator errors (including those errors present when determining the peak positions), and (e) the nature of the material (e.g., preferred orientation errors). Calibration errors and sample height errors often result in a shift of all the peaks in the same direction. In general, this correction factor will bring the measured peak positions into agreement with the expected peak positions and may be in the range of the expected 2θ value ± 0.2°.

The experimental method for characterizing a crystal form of an acid salt or a base salt of compound IE by differential scanning calorimetry (DSC) was as follows: A small amount of a polymorph of the crystalline compound IE was taken and placed into an aluminum crucible that is compatible with the instrument and can be sealed with a lid. After the sample was loaded, the crucible was sealed with an aluminum plate and then sent into the instrument for analysis. In this

patent, the instrument model used in the differential scanning calorimetry analysis was METTLER TOLEDO DSC 3, and scan parameters were set as follows: a nitrogen atmosphere was used, and the temperature ramp rate was 10.0 k/min.

The experimental method for characterizing a polymorph of compound IE by thermogravimetric analysis (TGA) was as follows: A small amount of a powder of the polymorph of compound IE was placed into an aluminum oxide crucible that is compatible with the instrument. After the sample was loaded, the crucible was sent into the instrument for analysis. In this application, the instrument model used in the thermogravimetric analysis was METTLER TOLEDO TGA 2, and scan parameters were set as follows: a nitrogen atmosphere was used, and the temperature ramp rate was 10.0 k/min.

The experimental method for characterizing an acid salt or a base salt of compound IE by dynamic vapor sorption (DVS) was as follows: A small amount of a polymorph powder of compound IE was placed into a precise sample tray that is compatible with the instrument. After the sample was loaded, the sample tray was sent into the instrument for analysis. In this patent, the instrument model used in the dynamic vapor sorption analysis was Intrinsic PLUS; the experimental parameter was set as follows: a constant temperature of 25 °C was set; the mass percent change rate per unit time (dm/dt) = 0.02%/min was used as a criterion for determining that a balance is achieved; the program humidity change cycle was set as follows: the initial relative humidity was 0%, and the endpoint relative humidity was 90%.

**Example 67-1: Synthesis of (*R,Z*)-12-hydroxy-12-methyl-2-((3-methyl-4-((1-methylpiperidin-4-yl)oxy)phenyl) amino)-7,10,11,12-tetrahydro-5H-13,17-(azeno)pyrimido[4',5':3,4]pyrazolo[1,2-a] [1,2]diazacyclotridecin-5-one (Metastable Crystal Form H of Compound of Formula IE)**

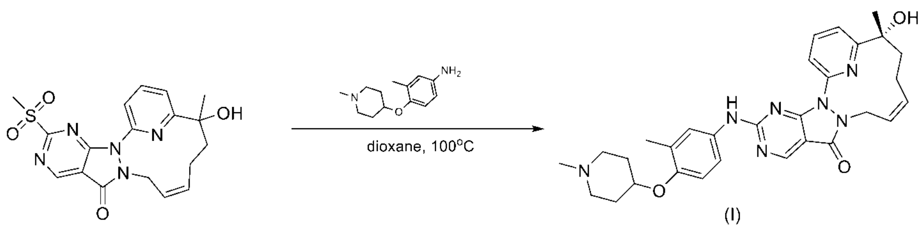


(I)

A solution of (*Z*)-12-hydroxy-12-methyl-2-(methylsulfonyl)-7,10,11,12-tetrahydro-5*H*-13,17-(azeno)pyrimido [4',5':3,4]pyrazolo[1,2-*a*][1,2]diazacyclotridecin-5-one (500 mg, 1.24 mmol) and 3-methyl-4-((1-methylpiperidin-4-yl) oxy)aniline (328.3 mg, 1.49 mmol) in dioxane (50 mL) was heated to 100 °C and stirred overnight. After cooling, the mixed reaction solution was concentrated under reduced pressure. The residue was purified by silica gel column chromatography (methanol:dichloromethane = 0%-10%) to give a crude product, and the crude product was purified by preparative supercritical fluid chromatography (chromatography conditions: instrument: SHIMADZU LC-20AP; column: DAICELCHIRALPAK®IC, 250 × 40 mm 10 μm; mobile phase A: *n*-hexane; mobile phase B: ethanol containing 0.1% ammonia/methanol (7 M)) to give (*R,Z*)-12-hydroxy-12-methyl-2-((3-methyl-4-((1-methylpiperidin-4-yl)oxy)phenyl) amino)-7,10,11,12-tetrahydro-5*H*-13,17-(azeno)pyrimido[4',5':3,4]pyrazolo[1,2-*a*][1,2]diazacyclotridecin-5-one (160 mg, yield: 19.0%), which is the metastable crystal form H of compound IE. The product was characterized by XRPD (FIG. 22), DSC (FIG. 23), and TGA (FIG. 24) analyses. $^1$H NMR (400 MHz, MeOD-*d*4) : δ 8.78 (s, 1H), 7.94 (t, *J* = 8.0 Hz, 1H), 7.81 (d, *J* = 8.0 Hz, 1H), 7.58 (d, *J* = 8.0 Hz, 1H), 7.55 (s, 1H), 7.34 (dd, *J* = 8.0, 4.0 Hz, 1H), 6.87 (d, *J* = 12.0 Hz, 1H), 5.53 - 5.41 (m, 2H), 4.57 (d, *J* = 8.0 Hz, 2H), 4.40 (s, 1H), 2.73 (s, 2H), 2.44 (s, 2H), 2.34 (s, 3H), 2.20 (s, 3H), 2.19 - 2.16 (m, 1H), 2.16-2.08 (m, 1H), 2.06 - 1.96 (m, 3H), 1.92 - 1.79 (m, 3H), 1.67 (s, 3H).

**Example 67-2: Preparation of Crystal Form A of Compound IE**

200 mg of the metastable crystal form H of compound IE was added to 5 mL of ACN/$H_2O$ (4:1, v:v). The mixture was stirred at 25 °C for 4 days and subjected to suction filtration, and the filter cake was blow-dried at 50 °C to give the crystal form A of compound IE. The product was characterized by XRPD (FIG. 1), DSC (FIG. 2), and TGA (FIG. 3) analyses.

**Example 67-3: Preparation of Crystal Form B of Compound IE**

200 mg of the metastable crystal form H of compound IE was added to 12 mL of IPAc/MTBE (18:5, v:v). The sample was dissolved by sonication at 50 °C, and the solution was filtered. The filtrate was stirred at 5 °C for 16 h and subjected to suction filtration, and the filter cake was blow-dried at 50 °C to give the crystal form B of the compound. The product was characterized by XRPD (FIG. 4), DSC (FIG. 5), and TGA (FIG. 6) analyses.

**Example 67-4: Preparation of Crystal Form C of Compound IE**

200 mg of the metastable crystal form H of compound IE was added to 12 mL of EA. The mixture was stirred at 25 °C for 16 h and subjected to suction filtration, and the filter cake was blow-dried at 50 °C to give the crystal form C of compound IE. The product was characterized by XRPD (FIG. 7), DSC (FIG. 8), and TGA (FIG. 9) analyses.

**Example 67-5: Preparation of Hydrate Crystal Form D of Compound IE**

200 mg of the metastable crystal form H of compound IE was added to 5 mL of acetone. The mixture was stirred at 25 °C for 4 days and subjected to suction filtration, and the filter cake was blow-dried at 50 °C to give the crystal form D of compound IE. The product was characterized by XRPD (FIG. 10), DSC (FIG. 11), and TGA (FIG. 12) analyses.

**Example 67-6: Preparation of Hydrate Crystal Form E of Compound IE**

200 mg of the metastable crystal form H of compound IE was added to 7 mL of $H_2O$/IPA (4:1, v:v). The mixture was stirred at 50 °C for 1 day and subjected to suction filtration, and the filter cake was added to 7 mL of $H_2O$/IPA (4:1, v:v). The mixture was stirred at 50 °C for 3 h and then subjected to suction filtration, and the filter cake was dried at room temperature for 3 days to give the crystal form E of compound IE. The product was characterized by XRPD (FIG. 13), DSC (FIG. 14), and TGA (FIG. 15) analyses.

**Example 67-7: Preparation of Hydrate Crystal Form F of Compound IE**

200 mg of the metastable crystal form H of compound IE was added to 5.1 mL of MeOH/$H_2O$ (2:1, v:v). The sample was dissolved by sonication at 50 °C, and the solution was filtered. The filtrate was stirred at 5 °C for 16 h and subjected to suction filtration, and the filter cake was dried *in vacuo* at room temperature to give the crystal form F of compound IE. The product was characterized by XRPD (FIG. 16), DSC (FIG. 17), and TGA (FIG. 18) analyses.

**Example 67-8: Preparation of Hydrate Crystal Form G of Compound IE**

200 mg of the metastable crystal form H of compound IE was added to 2 mL of $H_2O$/THF (2:1, v:v). After the mixture was stirred at room temperature for 10 min, another 3 mL of $H_2O$/THF (2:1, v:v) was added. The mixture was stirred at room temperature for 16 h and filtered, and the filter cake was dried at room temperature for 16 h and then blow-dried at 50 °C for 1 h to give the crystal form G of compound IE. The product was characterized by XRPD (FIG. 19), DSC (FIG. 20), and TGA (FIG. 21) analyses.

**Example 67-9: Preparation of Metastable Crystal Form I of Compound IE**

20 mg of the metastable crystal form H of compound IE was weighed out and added to 0.5 mL of $H_2O$/TFE (5:1, v:v). The mixture was stirred at room temperature for 4 days and centrifuged to give the metastable crystal form I of compound IE. The product was characterized by XRPD (FIG. 25) analysis. After drying *in vacuo* at room temperature for 16 h, the product was transformed into the crystal form D.

**Example 67-10: Preparation of Metastable Crystal Form J of Compound IE**

20 mg of the metastable crystal form H of compound IE was added to 1.5 mL of MeOH/$H_2O$ (2:1, v:v). The sample was dissolved by sonication at 50 °C, and the solution was filtered. The filtrate was stirred at 5 °C for 16 h and then centrifuged to give the metastable crystal form J of compound IE. The product was characterized by XRPD (FIG. 26) analysis. After drying *in vacuo* at room temperature for 16 h, the product was transformed into the crystal form F.

**Example 67-11: Preparation of Metastable Crystal Form K of Compound IE**

20 mg of the metastable crystal form H of compound IE was added to 0.6 mL of EtOAc/CPME (1:5, v:v). The sample was dissolved by sonication at 50 °C, and the solution was filtered. The filtrate was stirred at 5 °C for 16 h and centrifuged to give the metastable crystal form K of compound IE. The product was characterized by XRPD (FIG. 27) analysis. After stirring in acetonitrile for 3 h, the metastable crystal form K was transformed into the crystal form D.

**Example 67-12: Preparation of Metastable Crystal Form L of Compound IE**

200 mg of the metastable crystal form H of compound IE was added to 2 mL of $H_2O$/THF (2:1, v:v). After the mixture was stirred at room temperature for 15 min, another 3 mL of $H_2O$/THF (2:1, v:v) was added. The mixture was stirred at room temperature for 16 h and filtered to give the metastable crystal form L of compound IE. The product was characterized by XRPD (FIG. 28) analysis. After blow-drying at 50 °C for 1 h, the metastable crystal form L was completely transformed into the crystal form G.

**Example 67-13: Preparation of Metastable Crystal Form M of Compound IE**

200 mg of the metastable crystal form H of compound IE was added to 5 mL of 1,4-dioxane/$H_2O$ (6:5, v:v). The sample was dissolved by sonication at 50 °C, and the solution was filtered. The filtrate was stirred at 5 °C for 16 h and filtered to give the metastable crystal form M of compound IE. The product was characterized by XRPD (FIG. 29) analysis. After drying *in vacuo* at room temperature for 3 h, the metastable crystal form M was transformed into the crystal form G.

**Example 67-14: Preparation of Metastable Crystal Form N of Compound IE**

20 mg of the metastable crystal form H of compound IE was added to 0.5 mL of MTBE. The mixture was stirred at room temperature for 4 days and centrifuged to give the metastable crystal form N of compound IE. The product was characterized by XRPD (FIG. 30) analysis. After stirring in acetonitrile for 3 h, the metastable crystal form N was transformed into the crystal form D.

**Example 67-15: Preparation of Metastable Crystal Form O of Compound IE**

20 mg of the metastable crystal form H of compound IE was added to 0.5 mL of 2-BtOH/toluene (1:20, v:v). The mixture was stirred at room temperature for 4 days and centrifuged to give the metastable crystal form O of compound IE. The product was characterized by XRPD (FIG. 31) analysis. After stirring in acetonitrile for 3 h, the metastable crystal form O was transformed into the crystal form D.

**Example 67-16: Preparation of Metastable Crystal Form P of Compound IE**

20 mg of the metastable crystal form H of compound IE was added to 0.5 mL of CPME/toluene (1:1, v:v). The mixture was stirred at 5 °C for 4 days and centrifuged to give the metastable crystal form P of compound IE. The product was characterized by XRPD (FIG. 32) analysis. After stirring in acetonitrile for 3 h, the metastable crystal form P was transformed into the crystal form D.

**Example 67-17: Preparation of Metastable Crystal Form Q of Compound IE**

The hydrate crystal form D was heated to 150 °C to give the metastable crystal form Q. The product was characterized by XRPD (FIG. 33) analysis. After cooling to room temperature, the metastable crystal form Q was transformedback to the hydrate crystal form D.

**Biological Assays**

**Test Example 1: WEE1 Protein Kinase Inhibitory Activity Assay**

The influence of the compound MRANK-106 of the present disclosure on the kinase activity of Weel was assessed using an *in vitro* kinase reaction system.

1. Experimental materials

(1) Reagents and consumables

The reagents and consumables used in this test example are shown in Table 2-1.

Table 2-1. Information about reagents and consumables

| Reagent/consumable | Brand | Cat. No. |
|---|---|---|
| White 384-well MicroPlate | PerkinElmer | 6007290 |
| 5X Enzymatic buffer | Cisbio | 62EZBFDD |
| ATP | Invitrogen | PV3227 |
| DTT | Sigma | D5545 |
| $MgCl_2$ | Sigma | M8266 |
| ADP-GLO Kit | Promega | V9102 |
| Weel | Invitrogen | PV3817 |

(2) Instruments and devices

The instruments and devices used in this test example are shown in Table 2-2.

Table 2-2. Instruments and devices

| Instrument | Brand | Model |
|---|---|---|
| Microplate reader | Molecular Devices | SpectraMax L |
| Microplate centrifuge | Monad | PlatePro 3100 |
| Analytical balance | Mettlertoledo | XS105DU |

2. Experimental method

(1) Reagent preparation

① 1× kinase buffer: A 5× kinase buffer was diluted with $ddH_2O$ to make a 1× kinase buffer. $MgCl_2$ and other reagents also was needed to be added to the kinase, as specifically shown in Table 2-3 below.

Table 2-3. The reagent components added to the 1× kinase buffer

| Kinase name | 1 mL of the 1× kinase buffer contained: |
|---|---|
| Wee1 | 50 mM $MgCl_2$, and 1 mM DTT |

② 2.5× ATP working solution: The specific concentration of ATP is shown in Table 2.

③ 5× enzyme working solution: The optimization of the enzyme concentration had been completed in previous work, and the concentration of the reagent used in the screening is shown in Table 2-4. A 5× enzyme working solution of the enzyme was formulated using the 1× kinase buffer.

④ Compound formulation: A 10 mM mother liquor was formulated using DMSO, aliquoted, and then stored at -20 °C. Immediately before use, the mother liquor was thawed, diluted with DMSO to 1 mM, serially diluted 4-fold to 10 concentration points, and then 40-fold diluted with the 1× kinase buffer to give a 2.5× working solution.

Table 2-4. The experimental conditions for the kinase activity assay

| | Enzyme amount (ng/well) | ATP (μM) | Reaction time (h) | Reaction temperature (°C) |
|---|---|---|---|---|
| Weel | 10 | 10 | 3 | 37 |

(2) Experimental procedures

After the above reagents were formulated and equilibrated to room temperature, 4 μL of the diluted solution of the compound to be tested and 2 μL of the formulated enzyme solution were added to reaction wells. After shaking and centrifugation, the plate was incubated at room temperature for 15 min, and 4 μL of the ATP solution was then added to start a kinase reaction. After 3 h of reaction at 37 °C, 10 μL of an ADP-GLO reagent was added. After thorough mixing, the mixture was left to react at room temperature for 40 min, and 20 μL of a detection reagent was then added. After 30 min of reaction at room temperature, the luminescence signals were detected using a SpectraMax L instrument. Two independent duplicate assays were performed. The inhibition rate of each well was calculated based on all activity wells and background signal wells, with an average calculated for duplicate wells. Meanwhile, the inhibition rate was plotted using the professional graphing and analysis software PRISM 9.0, and the $IC_{50}$ was calculated.

(3) Data analysis

The inhibition rates of the compounds to be tested against the kinase activity of Wee1 were calculated using the following formula:

$$\text{Inhibition rate\%} = (Lum_{vehicle} - Lum_{compound})/(Lum_{vehicle} - Lum_{blank}) \times 100\%$$

$Lum_{compound}$: the luminescence of the sample treatment groups
$Lum_{vehicle}$: the luminescence of the solvent control group
$Lum_{blank}$: the luminescence of the blank group

3. Experimental results

The results of the inhibitory activity assay of the test samples against the kinase are shown in Table 2-5 below.

Table 2-5. The inhibitory activity of the representative compounds against the Wee1 kinase

| Compound No. | IC50 (nM) |
|---|---|
| Zn-c3 | 5.6 |
| Compound IE | 7.8 |
| Compound 29 | 67 |
| Compound 134 | 21 |
| Compound 89 | 66.8 |
| Compound 171B | 5.4 |
| Compound 172A | 6.5 |
| Compound 181 | 33.2 |
| Compound 182 | 30.1 |
| Compound 183 | 16.1 |
| Compound 186 | 18.7 |
| Compound 202 | 16.4 |
| Compound 203A | 13.2 |
| Compound 219A | 13.1 |
| Compound 223 | 18.7 |
| Compound 224A | 12.8 |
| Compound 225A | 20.4 |

**Test Example 2: YES1 Protein Kinase Inhibitory Activity Assay**

In this experiment, the capability of the compound MRANK-106 of the present disclosure to inhibit kinase activity was assessed by an HTRF method using a drug screening system based on the YES1 kinase.

1. Experimental materials

(1) Reagents and consumables

The reagents and consumables used in this test example are shown in Table 3-1.

Table 3-1. Information about reagents and consumables

| Name | Cat. No. | Brand |
|---|---|---|
| HTRF KinEASE TK kit | 62TK0PEJ | PerkinElmer |
| MgCl2 | M1028 | Sigma |
| DTT | HY-15917 | MCE |
| DMSO | D4540 | Sigma |
| ATP | V915B | Promega |
| YES1 | 08-175 | Carna |
| SU6656 | HY-B0789 | MCE |
| 96 Well Plates | 249944 | Nunc |
| 384-well plate | 784075 | Greiner |
| 384-well PP 2.0 Microplate, Echo® Qualified ® | PP-0200 | LABCYTE |

(2) Instruments and devices

The instruments and devices used in this test example are shown in Table 3-2.

Table 3-2. Instruments and devices

| Instrument | Brand | Model |
|---|---|---|
| Microplate reader | Molecular Devices | SpectraMax L |
| Microplate centrifuge | Monad | PlatePro 3100 |
| Analytical balance | Mettlertoledo | XS105DU |

2. Experimental method

(1) Experimental system

The enzymatic activity conditions and HTRF system formulation are shown in Table 3-3; the experimental conditions for the kinase activity assay are shown in Table 3-4.

Table 3-3. HTRF system formulation

| Name | Storage concentration | Working concentration |
|---|---|---|
| 5× Buffer | 5× | 1× |
| MgCl2 | 1 M | 5 mM |
| DTT | 1 M | 1 mM |
| ddH2O | N/A | N/A |

Table 3-4. The experimental conditions for the kinase activity assay

| | Enzyme amount (ng/well) | ATP (μM) | Reaction time (h) | Reaction temperature (°C) |
|---|---|---|---|---|
| YES1 | 10 | 10 | 3 | 37 |

(2) Compound formulation

The test compound MRANK-106 was dissolved in DMSO, and the solution was vortexed and shaken for thorough mixing. The solution was serially diluted 3-fold with DMSO, with the initial concentration being 503 nM.

(3) Detection steps

① A 2× ATP/substrate solution and a 2× kinase solution were formulated using a kinase reaction buffer.

② 50 nL of the compound dilution was transferred to a 384 detection plate using Echo 655. After centrifugation, 2.5 μL of a 2× kinase solution was added to the 384 detection plate. The plate was centrifuged at 1000 rpm for 1 min and incubated at 25 °C for 10 min.

② 2.5 μL of the 2× substrate and ATP solution was added to the 384 detection plate, and the plate was centrifuged at 1000 rpm for 1 min and incubated at 25 °C for 60 min.

④ A 2× XL665 and antibody detection reagent was formulated using a detection buffer.

⑤ 5 μL of a kinase detection reagent was added to the detection plate, and the plate was incubated at 25 °C. The plate was centrifuged at 1000 rpm for 1 min and incubated at 25 °C for 1 h.

⑥ The fluorescence signals at 620 nm (Cryptate) and 665 nm (XL665) were read using a BMG high-throughput drug-screening multi-mode microplate reader.

(4) Data analysis

With the reading of the negative control set as an inhibition rate of 0% and the reading of the positive control set as an inhibition rate of 100%, the inhibition rate of each test solution was calculated using the following formula:

$$\text{Inhibition rate of the compound}=\left[\frac{\overline{\text{Data}}_{\text{negative control}}-\text{Data}_{\text{Compound}}}{\overline{\text{Data}}_{\text{negative control}}-\overline{\text{Data}}_{\text{positive control}}}\right]*100$$

$\text{Data}_{\text{positive}}$ control: the average of the positive control well values

$\text{Data}_{\text{negative}}$ control : the average of the negative control well values

The $IC_{50}$ (half maximal inhibitory concentration) of the compound was obtained using the following nonlinear fitting formulas:

$$Y = \text{Bottom} + (\text{Top} - \text{Bottom}) / (1 + 10\text{^}((\text{LogIC50} - X) * \text{HillSlope}))$$

X: the log value of the compound concentration

Y: the inhibition rate (% inh) of the compound

The Z' factor calculation equation:

$$Z' = 1 - 3(\text{SDmin} + \text{SDmax}) / (\text{AVEmax} - \text{AVEmin})$$

where:

Min is the positive control Data value, and Max is the negative control DMSO Data value;

SD stands for standard deviation, and AVE stands for average.

3. Experimental results

The results of the inhibitory activity assay of the compounds of the present disclosure against the Yes kinase are shown in Table 3-5 below.

Table 3-5. The inhibitory activity of MRANK-106 against the YES1 kinase

| Compound No. | IC50 (nM) |
|---|---|
| SU6656 | 43.0 |
| Znc3 | > 500 |
| Compound IE | 3.05 |

(continued)

| Compound No. | IC50 (nM) |
|---|---|
| Compound 134 | 68.9 |
| Compound 89 | 18.5 |
| Compound 171B | 346.2 |
| Compound 172A | 5.8 |
| Compound 180 | 52.5 |
| Compound 181 | 117.4 |
| Compound 182 | 19.9 |
| Compound 185 | 129.0 |
| Compound 199 | 59.1 |
| Compound 202 | 18.8 |
| Compound 206 | 130.5 |
| Compound 223 | 21.8 |

It can be seen from the above assay results that most of the representative compounds of the present disclosure, such as compounds IE, 134, 89, 172A, 180, 182, 199, 202, 223, etc., simultaneously and strongly inhibited the Weel and Yes targets, indicating that they are Yes and Wee1 dual-target inhibitors.

**Test Example 3: Tumor Cell Proliferation Inhibitory Activity Assay of Representative Compounds**

**Human lung cancer A427 cells are cancer cells reported to be sensitive to wee1 inhibitors. In this assay, the anti-cancer effects of the compounds of the present disclosure were verified by assessing the activity of the test substances in inhibiting A427 cell proliferation.**

**1. Experimental materials**

| Cell name | Culture method |
|---|---|
| A427 (ATCC) | EMEM+10%FBS |
| **Reagent and consumable** | |
| CCK-8 kit (Bioscience, Targetmol) | |

**2. Experimental method**

**Cell culture**

A427 cells were cultured according to the cell culture instructions, and cells in the logarithmic growth phase were collected, counted, resuspended in a complete culture medium, and inoculated into a 96-well plate at a cell concentration of 100 μL/3000 cells/well. The cells were incubated in an incubator at 37 °C with 100% relative humidity and 5% $CO_2$ for 24 h.

**Formulation of compound to be tested**

The compounds to be tested were dissolved in DMSO and diluted 4-fold with a culture medium from 10 μM to 10 concentration gradients.

**Compound incubation**

The culture medium waste was pipetted off, and 100 μL of the formulated culture medium of the compound to be tested was added to each well. The cells were incubated in an incubator at 37 °C with 100% relative humidity and 5% $CO_2$ for 72 h.

**CCK-8 cell proliferation assessment**

The plate was taken out, the CCK-8 reagent was added at 10 μL/well, and the plate was incubated in an incubator at 37 °C for 2-4 h. After gentle shaking, the absorbance was detected at 450 nm to 650 nm, and the inhibition rates were calculated.

**Data analysis and results:**

The cell growth inhibition rates of the drugs were calculated using the following formula:

$$\text{Cell growth inhibition rate\%} = [(Ac - As)/(Ac - Ab)] \times 100\%$$

As: the OA of the samples (cells + CCK-8 + compounds to be tested)
Ac: the OA of the normal growth cell control (cells + CCK-8)
Ab: the OA of the blank control (culture medium + CCK-8)

IC50 curve fitting was performed using the software Graphpad Prism 6 and the calculation formula XY-analysis/Nonlinear regression(curve fit)/Dose response-Inhibition/log(inhibitor)vs. response-Variable slope(four parameters), and the IC50 values were calculated.

**Table 1. Data analysis and results for some representative compounds**

| Compound No. | $IC_{50}$ (uM) |
|---|---|
| Debio 0123 | 1.609 |
| ZN-c3 | 0.129 |
| Compound IE | 0.013 |
| Compound 28 | 0.152 |
| Compound 89 | 0.219 |
| Compound 143 | 0.065 |
| Compound 172A | 0.015 |
| Compound 182 | 0.160 |
| Compound 202 | 0.078 |
| Compound 203A | 0.071 |
| Compound 205 | 0.163 |
| Compound 214 | 0.144 |

It can be seen from the above results that most of the compounds of the present disclosure exhibited higher activity than the structurally known reference compound Debio 0123 or ZN-c3 in inhibiting cancer cell proliferation, and the anti-cancer activity of some representative compounds, such as IE, 143, 172A, 202, 203A, etc., was even several times to several dozen times higher.

**Test Example 4: *In Vivo* Efficacy Study of Representative Compounds Against A427 Tumors**

A427 human lung cancer cells were subcultured and expanded in an MEM culture medium containing 10% FBS. BALB/c nude mice aged 6-8 weeks were taken and each subcutaneously injected with $5 \times 10^6$ cells to establish a tumor-bearing model. The tumor volume was monitored, and when the tumor volume reached about 150 $mm^3$, the animals were

randomized into 5 groups according to body weight and tumor volume. The animals were given, by oral gavage, the vehicle or compounds to be tested (ZN-c3 and IE) once daily for a total of 28 days. During the period of administration, the tumor volume and the animal body weight were measured twice a week, and the animals' states were observed daily. At the endpoint, the animals were euthanized with carbon dioxide, and the subcutaneous tumors were isolated and weighed.

The data calculation formulae are shown below:
The tumor volume (TV) was calculated using the formula $1/2 \times axb^2$, where a and b represent the length and width measurements of the tumor, respectively.

The tumor growth inhibition rate ($\%TGI_{TV}$) was calculated using the formula $(1 - TV_T/TV_C) \times 100\%$, where TVc represents the average tumor volume of the negative control group, and $TV_T$ represents the average tumor volume of the treatment group.

The relative tumor volume (RTV) was calculated using the formula $Vt/V_0$, where $V_0$ represents the tumor volume at the time of grouping, and Vt represents the tumor volume at each measurement.

Data analysis and results are shown in Table 1.

Table 1. A427 relative tumor volumes and tumor inhibition rates:

| **Administration regimen** | **Relative tumor volume Day 28** | **Relative tumor inhibition rate (%) Day 28** |
|---|---|---|
| Group 1 Vehicle, PO, QD×28 | 6.26 | - |
| Group 2 ZN-c3, 80mg/kg, PO, QD×28 | 3.12 | 48.93 |
| Group 3 ZN-c3, 30mg/kg, PO, QD×28 | 5.03 | 16.17 |
| Group 4 compound IE, 80/60 mg/kg, PO, QD × 28 | 0.02 | 99.75 |
| Group 5 compound IE, 30 mg/kg, PO, QD × 28 | 2.41 | 60.81 |

It can be seen from the above results that the representative compound IE of the present disclosure exhibited excellent inhibitory activity against tumors *in vivo* and exhibited higher activity than the reference molecule ZN-c3 in inhibiting tumor growth in the A427 tumor model.

**Test Example *5: In Vivo* Efficacy Study of Representative Compounds Against SW620 Tumors**

SW620 human colon cancer cells were subcultured and expanded in an L-15 culture medium containing 10% FBS. BALB/c nude mice aged 6-8 weeks were taken and each subcutaneously injected with $1 \times 10^6$ cells to establish a tumor-bearing model. The tumor volume was monitored, and when the tumor volume reached about 150 $mm^3$, the animals were randomized into 5 groups according to body weight and tumor volume. The animals were given, by oral gavage, the vehicle or compounds to be tested (ZN-c3 and compound IE) once daily for a total of 21 days. During the period of administration, the tumor volume and the animal body weight were measured twice a week, and the animals' states were observed daily. At the endpoint, the animals were euthanized with carbon dioxide, and the subcutaneous tumors were isolated and weighed.

The data calculation formulae are shown below:
The tumor volume (TV) was calculated using the formula $1/2 \times axb^2$, where a and b represent the length and width measurements of the tumor, respectively.

The tumor growth inhibition rate ($\%TGI_{TV}$) was calculated using the formula $(1 - TV_T/TV_C) \times 100\%$, where TVc represents the average tumor volume of the negative control group, and $TV_T$ represents the average tumor volume of the treatment group.

The relative tumor volume (RTV) was calculated using the formula $Vt/V_0$, where $V_0$ represents the tumor volume at the time of grouping, and Vt represents the tumor volume at each measurement.

Data analysis and results are shown in Table 2.

Table 2. SW620 relative tumor volumes and tumor inhibition rates:

| **Administration regimen** | **Relative tumor volume Day 21** | **Relative tumor inhibition rate (%) Day 21** |
|---|---|---|
| Group 1 Vehicle, PO, QD×21 | 11.83 | - |
| Group 2 ZN-c3, 80mg/kg, PO, QD×21 | 7.94 | 36.78 |
| Group 3 ZN-c3, 30mg/kg, PO, QD×21 | 8.57 | 28.24 |

(continued)

| Administration regimen | Relative tumor volume Day 21 | Relative tumor inhibition rate (%) Day 21 |
|---|---|---|
| Group 4 compound IE, 80 mg/kg, PO, QD × 21 | 2.57 | 78.38 |
| Group 5 compound IE, 30 mg/kg, PO, QD × 21 | 7.31 | 38.18 |

It can be seen from the above results that the representative compound IE of the present disclosure exhibited excellent inhibitory activity against tumors *in vivo* and exhibited higher activity than the reference molecule ZN-c3 in inhibiting tumor growth in the SW620 tumor model.

**Test Example 6: *In Vivo* Efficacy Study of Representative Compounds Against LoVo Tumors**

Human colon cancer LoVo cells were subcultured and expanded in an F12K culture medium containing 10% FBS. BALB/c nude mice aged 6-8 weeks were taken and each subcutaneously injected with $2 \times 10^6$ cells to establish a tumor-bearing model. The tumor volume was monitored, and when the tumor volume reached about 150 $mm^3$, the animals were randomized into 5 groups according to body weight and tumor volume. The animals were given, by oral gavage, the vehicle or compounds to be tested (ZN-c3 and IE) once daily for a total of 28 days. During the period of administration, the tumor volume and the animal body weight were measured twice a week, and the animals' states were observed daily. At the endpoint, the animals were euthanized with carbon dioxide, and the subcutaneous tumors were isolated and weighed.
The data calculation formulae are shown below:
The tumor volume (TV) was calculated using the formula $1/2 \times axb^2$, where a and b represent the length and width measurements of the tumor, respectively.
The tumor growth inhibition rate ($\%TGI_{TV}$) was calculated using the formula $(1 - TV_T/TV_C) \times 100\%$, where $TV_C$ represents the average tumor volume of the negative control group, and $TV_T$ represents the average tumor volume of the treatment group.
The relative tumor volume (RTV) was calculated using the formula $Vt/V_0$, where $V_0$ represents the tumor volume at the time of grouping, and Vt represents the tumor volume at each measurement.
Data analysis and results are shown in Table 3.

Table 3. LoVo relative tumor volumes and tumor inhibition rates:

| Administration regimen | Relative tumor volume Day 28 | Relative tumor inhibition (%) Day 28 |
|---|---|---|
| Vehicle, PO, QD×28 | 5.75 | - |
| ZN-c3, 80mg/kg, PO, QD × 28 | 2.41 | 57.98 |
| Compound IE, 80 mg/kg, PO, QD × 28 | 0.17 | 97.12 |
| Compound IE, 40 mg/kg, PO, QD × 28 | 0.63 | 89.12 |
| Compound IE, 20 mg/kg, PO, QD × 28 | 2.28 | 60.90 |

It can be seen from the above results that the representative dual-target compound IE of the present disclosure exhibited better inhibitory activity against tumors *in vivo* than the single-target Weel selective reference molecule ZN-c3: compound IE exhibited significantly better activity than ZN-c3 in inhibiting tumor growth in the LoVo tumor model.

**Test Example 7: *In Vivo* Efficacy Study of Representative Compounds Against OVCAR-3 Tumors**

Human ovarian cancer OVCAR-3 cells were subcultured and expanded in an RPMI-1640 culture medium containing 20% FBS and 0.01 mg/mL bovine insulin. BALB/c nude mice aged 6-8 weeks were taken and each subcutaneously injected with $5 \times 10^6$ cells to establish a tumor-bearing model. The tumor volume was monitored, and when the tumor volume reached about 150 $mm^3$, the animals were randomized into 5 groups according to body weight and tumor volume. The animals were given, by oral gavage, the vehicle or compounds to be tested (ZN-c3 and IE) once daily for a total of 28 days. During the period of administration, the tumor volume and the animal body weight were measured twice a week, and the animals' states were observed daily. At the endpoint, the animals were euthanized with carbon dioxide, and the subcutaneous tumors were isolated and weighed.
The data calculation formulae are shown below:
The tumor volume (TV) was calculated using the formula $1/2 \times axb^2$, where a and b represent the length and width

measurements of the tumor, respectively.

The tumor growth inhibition rate ($\%TGI_{TV}$) was calculated using the formula ($1 - TV_T/TV_C$) × 100%, where TVc represents the average tumor volume of the negative control group, and $TV_T$ represents the average tumor volume of the treatment group.

The relative tumor volume (RTV) was calculated using the formula $Vt/V_0$, where $V_0$ represents the tumor volume at the time of grouping, and Vt represents the tumor volume at each measurement.

Data analysis and results are shown in Table 4.

Table 4. OVCAR-3 relative tumor volumes and tumor inhibition rates:

| Administration regimen | Relative tumor volume Day 28 | Relative tumor inhibition (%) Day 28 |
|---|---|---|
| Vehicle, PO, QD×28 | 5.2 | - |
| AZD-1775, 80mg/kg, PO, QD×28 | 2.18 | 58.28 |
| ZN-c3, 100mg/kg, PO, QD×28 | 2.01 | 61.10 |
| Compound IE, 80/60 mg/kg, PO, QD × 28 | 0.38 | 92.69 |
| Compound IE, 40 mg/kg, PO, QD × 28 | 0.94 | 81.11 |
| Compound IE, 20 mg/kg, PO, QD × 28 | 3.16 | 40.51 |

It can be seen from the above results that the representative dual-target compound IE of the present disclosure exhibited better inhibitory activity against tumors *in vivo* than the single-target Weel selective reference molecule ZN-c3: compound IE exhibited significantly better activity than ZN-c3 in inhibiting tumor growth in the OVCAR-3 tumor model.

**Physical and Chemical Property Tests:**

**Test Example 8: Equilibrium Solubility Study of Representative Polymorphs of Compound (IE) in Different Vehicles**

The equilibrium solubility of the crystal forms A, B, C, and D of compound (IE) was tested in water ($H_2O$), fasted state simulated gastric fluid (FaSSGF), fasted state simulated intestinal fluid (FaSSIF), and fed state simulated intestinal fluid (FeSSIF). In the test, the solids were suspended in corresponding buffers to formulate suspensions (~10 mg/mL) and mixed at 37±2 °C. After 24 h, samples of the suspensions were taken. The supernatant was filtered, and the concentration was determined. The test results are shown in the table below:

| Solid form | Solution | Equilibrium solubility |
|---|---|---|
| Crystal form A of compound IE | FaSSGF | >10 |
| | FeSSIF | 0.27 |
| | FaSSIF | 1.73 |
| Crystal form B of compound IE | FaSSGF | >10 |
| | FeSSIF | 2.07 |
| | FaSSIF | 1.23 |
| Crystal form C of compound IE | FaSSGF | >10 |
| | FeSSIF | 1.299 |
| | FaSSIF | 0.710 |
| Crystal form D of compound IE | FaSSGF | 8.44 |
| | FeSSIF | 0.11 |
| | FaSSIF | 0.81 |

It can be seen from the above experimental results that the crystal forms A, B, C, and D of compound (IE) demonstrated relatively good solubility in the simulated physiological systems.

**Test Example 9: Solid-State Stability Study of Representative Polymorphs of Compound (IE)**

The crystal forms A, B, C, and D of compound (IE) were left to stand under long-term (25 °C/60% RH), accelerated (40 °C/75% RH), and high-temperature (60 °C, RH < 30%) conditions for 7 days, and the HPLC purity and crystal form changes were assessed to study their solid-state stability. The results are shown in the table below:

| Solid form | Initial purity (%) | 25 °C/60% RH, 7 days | | 40 °C/75% RH, 7 days | | 60 °C, 7 days | |
|---|---|---|---|---|---|---|---|
| | | Purity (%) | Crystal form | Purity (%) | Crystal form | Purity (%) | Crystal form |
| Crystal form A of compound IE | 98.08 | 98.01 | Crystal form A | 98.11 | Crystal form E | 98.06 | Crystal forms A+E |
| Crystal form B of compound IE | 97.97 | 98.01 | Crystal form B | 98.25 | Crystal form B | 98.24 | Crystal form B |
| Crystal form C of compound IE | 98.74 | 98.75 | Crystal form C | 98.73 | Crystal form C | 98.72 | Crystal form C |
| Crystal form D of compound IE | 97.79 | 97.82 | Crystal form D | 97.87 | Crystal form D | 97.74 | Crystal form D |

It can be seen from the purity results of the above experiment that standing under long-term conditions for 7 days, the crystal forms A, B, C, and D of compound (IE) all remained stable; standing under high-temperature and accelerated conditions for 7 days, the crystal forms B, C, and D also remained stable.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound represented by formula (I), a pharmaceutically acceptable salt, a solvate, an enantiomer, an isotopically substituted compound, or a polymorph thereof:

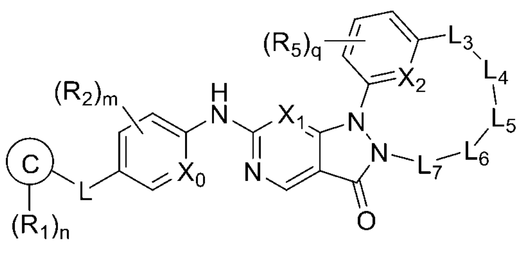

formula (I),

wherein

$X_0$, $X_1$, and $X_2$ are each independently selected from $-C(R_x)-$ and -N-;

$L_3$, $L_4$, $L_5$, $L_6$, and $L_7$ are identical or different and are independently absent, or selected from single bond, double bond, alkyne bond, $-C(R_{d1})(R_{d2})-$, $-C(R_{d1})(R_{d2})C(R_{d1})(R_{d2})-$, $-OC(R_{d1})(R_{d2})-$, $-C(R_{d1})(R_{d2})O-$, $-C(=O)N(R_{d3})-$, $-N(R_{d3})C(=O)-$, $-N(R_{d3})-$, $-C(=NR_{d3})-$, $-S(=O)_2N(R_{d3})-$, $-N(R_{d3})S(=O)_2-$, $-C(R_{d1})(R_{d2})N(R_{d3})-$, $-N(R_{d3})C(R_{d1})(R_{d2})-$, -O-, -S-, -C(=O)O-, -OC(=O)-, -C(=O)-, -C(=S)-, -N-, -CH-, -S(=O)-, and $-S(=O)_2-$;

L is absent or is selected from O, S, $CH_2$, C(O), S(O), $S(O)_2$, NH, C(O)NH, and NHC(O); ring C is heterocyclyl unsubstituted or substituted with $R_0$;

$R_0$ is independently selected from H, deuterium, halogen, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally

substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein hydrogen on the aryl, saturated or partially saturated cycloalkyl, or heterocycloalkyl is optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, =O, and saturated or partially saturated $C_{3-6}$ cycloalkyl, wherein the $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl;

$R_1$, $R_{d1}$, $R_{d2}$, and $R_x$ are independently selected from hydrogen, deuterium, halogen, cyano, oxo (=O), amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, wherein the $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or $R_{d1}$ and $R_{d2}$, or $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

$R_{d3}$ is optionally and independently selected from hydrogen, $NH_2$, $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, and $C_{3-10}$ heterocyclyl substituted with $C_{3-10}$ cycloalkyl or $C_{3-10}$ heterocycloalkyl, wherein the $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, or $C_{3-10}$ heterocyclyl substituted with $C_{3-10}$ cycloalkyl or $C_{3-10}$ heterocycloalkyl is further optionally substituted with one or more substituents selected from hydrogen, deuterium, halogen, oxo, CN, OH, and $C_{3-10}$ saturated or partially saturated cycloalkyl or heterocyclyl;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5; and

q is an integer optionally selected from 0, 1, 2, and 3.

**2.** The compound, the pharmaceutically acceptable salt, the solvate, the enantiomer, the isotopically substituted compound, or the polymorph thereof as claimed in claim 1, wherein:

$X_0$, $X_1$, and $X_2$ are each independently selected from $-C(R_x)-$ and -N-, wherein $R_x$ is selected from H and $C_{1-6}$ alkyl (e.g., methyl);

preferably, $X_0$ is -CH-;

preferably, $X_1$ is N;

preferably, $X_2$ is N.

3. The compound, the pharmaceutically acceptable salt, the solvate, the enantiomer, the isotopically substituted compound, or the polymorph thereof as claimed in claim 1 or 2, wherein:

L is selected from O, S, and NH;
preferably, $L_3$, $L_4$, $L_5$, $L_6$, and $L_7$ are identical or different and are independently absent, or selected from single bond, and the following groups unsubstituted or optionally substituted with one, two, or more $R_{d1}$: $C_{1-6}$ alkylene and $C_{2-6}$ alkenylene;
preferably, $L_3$, $L_4$, $L_5$, $L_6$, and $L_7$ are identical or different and are independently absent, or selected from single bond, and the following groups unsubstituted or optionally substituted with one, two, or more $R_{d1}$: $CH_2$, $CH_2CH_2$, $CH_2CH_2CH_2$, CH=CH, and $CH=CHCH_2$;
preferably, $L_3$ is $-C(R_{d1})(R_{d1})-$, preferably $-C(CH_3)(OH)-$;
preferably, $L_6$ is vinylene, preferably cis-vinylene;
preferably, $L_4$, $L_5$, and $L_7$ are identical or different and are independently $CH_2$;
preferably, each $R_{d1}$ is identical or different and is independently selected from H, OH, and $C_{1-6}$ alkyl (e.g., methyl).

4. The compound, the pharmaceutically acceptable salt, the solvate, the enantiomer, the isotopically substituted compound, or the polymorph thereof as claimed in any one of claims 1-3, wherein:
ring C is heterocyclyl unsubstituted or substituted with $R_0$, wherein the heterocyclyl may be selected from piperidyl (e.g.,

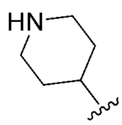

), piperazinyl (e.g.,

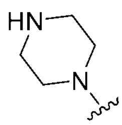

), morpholinyl (e.g.,

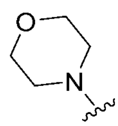

), azetidinyl (e.g.,

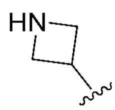

), tetrahydropyrrolidinyl (e.g.,

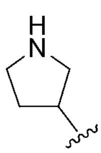 ), 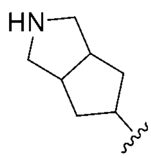 ,

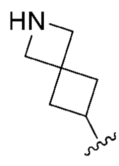, 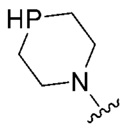, 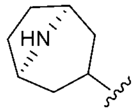, and 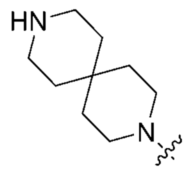;

preferably,

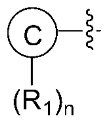

is selected from

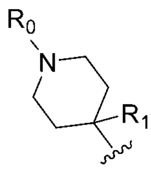, 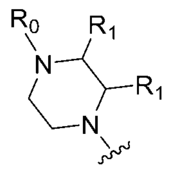, 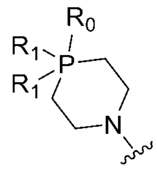, 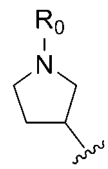,

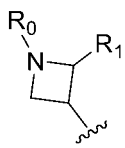, 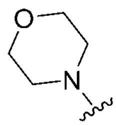, 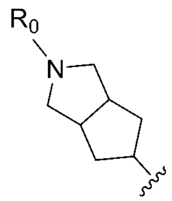, 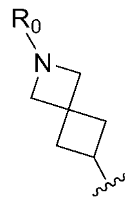, 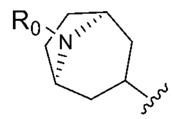, and 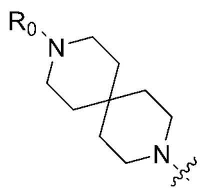;

preferably,

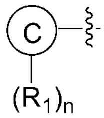

is selected from

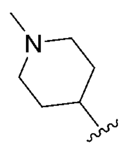, 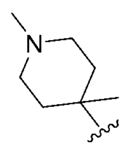, 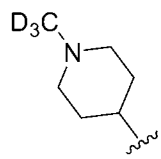, 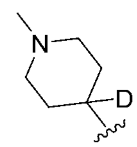,

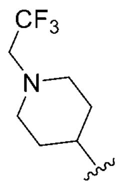, 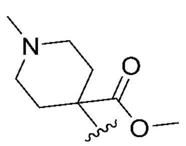, 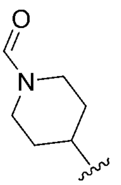, 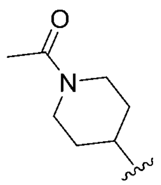, 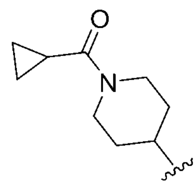, 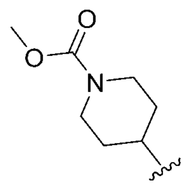,

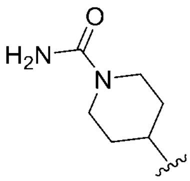, 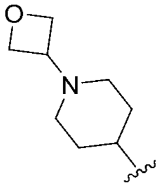, 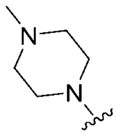, 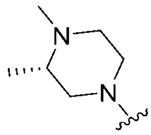, 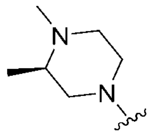, 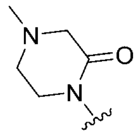,

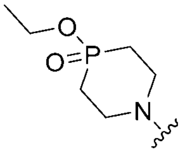, 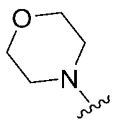, 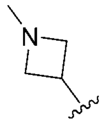, 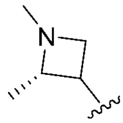, 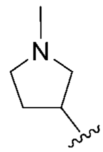, 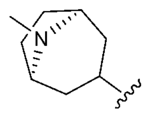, 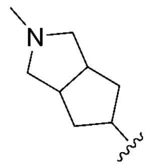,

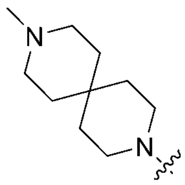, and 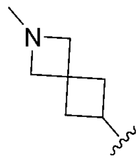.

**5.** The compound, the pharmaceutically acceptable salt, the solvate, the enantiomer, the isotopically substituted compound, or the polymorph thereof as claimed in any one of claims 1-4, wherein:

$R_0$ is selected from H, $C_{1-10}$ alkyl, $C_{1-10}$ alkoxy, deuterated $C_{1-10}$ alkyl, $C_{1-10}$ haloalkyl, HC(O)-, $NH_2C(O)$-, $C_{1-10}$ alkyl-C(O)-, $C_{1-10}$ alkoxy-C(O)-, $C_{3-8}$ cycloalkyl-C(O)-, and 3- to 8-membered heterocyclyl;
preferably, $R_0$ is selected from H, $C_{1-6}$ alkyl, $C_{1-6}$ alkoxy, deuterated $C_{1-6}$ alkyl, $C_{1-6}$ haloalkyl, HC(O)-, $NH_2C(O)$-, $C_{1-6}$ alkyl-C(O)-, $C_{1-6}$ alkoxy-C(O)-, $C_{3-6}$ cycloalkyl-C(O)-, and 3- to 6-membered heterocyclyl;
preferably, $R_0$ is selected from H, methyl, ethoxy, trideuterated methyl, $CF_3CH_2$-, HC(O)-, $CH_3C(O)$-,

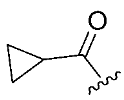, 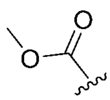, 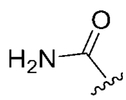, and 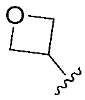;

preferably, each $R_1$ is identical or different and is independently selected from H, deuterium, oxo (=O), $C_{1-6}$ alkyl, $C_{1-6}$ alkoxy, $C_{1-6}$ alkoxy-C(O)-, $C_{1-6}$ haloalkyl, $C_{1-6}$ haloalkoxy, $C_{1-6}$ alkylamino, $C_{6-10}$ aryl, and 5- to 10-membered heteroaryl;
preferably, each $R_1$ is identical or different and is independently selected from H, deuterium, oxo (=O), $C_{1-6}$ alkyl, and $C_{1-6}$ alkoxy-C(O)-;
preferably, each $R_1$ is identical or different and is independently selected from H, methyl, deuterium, oxo (=O), and

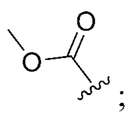;

preferably, each $R_2$ is identical or different and is independently selected from H, deuterium, oxo (=O), $C_{1-6}$ alkyl, $C_{1-6}$ alkoxy, $C_{1-6}$ alkoxy-C(O)-, $C_{1-6}$ haloalkyl, $C_{1-6}$ haloalkoxy, $C_{1-6}$ alkylamino, $C_{6-10}$ aryl, and 5- to 10-membered heteroaryl;
preferably, each $R_2$ is identical or different and is independently selected from H and $C_{1-6}$ alkyl (e.g., methyl);
preferably, each $R_5$ is identical or different and is independently selected from H, deuterium, oxo (=O), $C_{1-6}$ alkyl, $C_{1-6}$ alkoxy, $C_{1-6}$ alkoxy-C(O)-, $C_{1-6}$ haloalkyl, $C_{1-6}$ haloalkoxy, $C_{1-6}$ alkylamino, $C_{6-10}$ aryl, and 5- to 10-membered heteroaryl;
preferably, each $R_5$ is identical or different and is independently selected from H and $C_{1-6}$ alkyl (e.g., methyl).

**6.** The compound, the pharmaceutically acceptable salt, the solvate, the enantiomer, the isotopically substituted

compound, or the polymorph thereof as claimed in any one of claims 1-5, having a structure of formula (II):

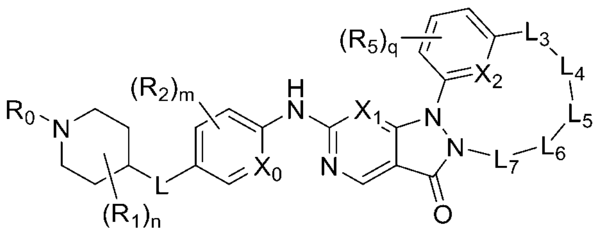

(II),

wherein L, $L_3$, $L_4$, $L_5$, $L_6$, $L_7$, $X_0$, $X_1$, $X_2$, $R_0$, $R_1$, $R_2$, $R_5$, m, n, and q are independently defined as described in any one of claims 1-5;
preferably, $X_0$, $X_1$, and $X_2$ are each independently selected from -C($R_x$)- and -N-;
$L_3$, $L_4$, $L_5$, $L_6$, and $L_7$ are independently absent, or selected from single bond, double bond,
alkyne bond, -C($R_{d1}$)($R_{d2}$)-, -C($R_{d1}$)($R_{d2}$)C($R_{d1}$)($R_{d2}$)-, -OC($R_{d1}$)($R_{d2}$)-, -C($R_{d1}$)($R_{d2}$)O-, - C(=O)N($R_{d3}$)-, -N($R_{d3}$) C(=O)-, -N($R_{d3}$)-, -C(=N$R_{d3}$)-, -S(=O)$_2$N($R_{d3}$)-, -N($R_{d3}$)S(=O)$_2$-, - C($R_{d1}$)($R_{d2}$)N($R_{d3}$)-, -N($R_{d3}$)C($R_{d1}$)($R_{d2}$)-, -O-, -S-, -C(=O)O-, -OC(=O)-, -C(=O)-, -C(=S)-, - N-, -CH-, -S(=O)-, and -S(=O)$_2$-;
L is optionally and independently selected from O, S, and NH;
$R_0$ is independently selected from H, deuterium, halogen, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;
each $R_2$ or each $R_5$ may be identical or different and is independently selected from hydrogen, deuterium, halogen, -CN, -OH, -SH, -$NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein hydrogen on the aryl, saturated or partially saturated cycloalkyl, or heterocycloalkyl is optionally substituted with a group selected from hydrogen, deuterium, halogen, -CN, -OH, $CF_3$, $C_{1-6}$ alkyl, $C_{1-6}$ alkoxy, -$NH_2$, - NH$C_{1-6}$ alkyl, -N($C_{1-6}$ alkyl)$_2$, =O, and saturated or partially saturated $C_{3-6}$ cycloalkyl, wherein the $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl;
$R_1$, $R_{d1}$, $R_{d2}$, and $R_x$ are independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, -$NH_2$, -NH$C_{1-6}$ alkyl, -N($C_{1-6}$ alkyl)$_2$, oxy, and saturated or partially saturated $C_{3-6}$ cycloalkyl, wherein the $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or $R_{d1}$ and $R_{d2}$, or $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen,

oxo, CN, $CF_3$, OH, $OCH_3$, and $OCH_2CH_3$;

$R_{d3}$ is optionally and independently selected from hydrogen, $NH_2$, $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, and $C_{3-10}$ heterocyclyl substituted with $C_{3-10}$ cycloalkyl or $C_{3-10}$ heterocycloalkyl; wherein the $C_{1-10}$ alkyl, $C_{2-10}$ alkynyl, $C_{1-10}$ alkoxy, $C_{1-10}$ alkylacyl, $C_{1-10}$ alkylsulfonyl, $C_{2-10}$ heteroalkyl, $C_{3-10}$ cycloalkyl, $C_{3-10}$ heterocycloalkyl, $C_{1-10}$ alkyl substituted with $C_{3-10}$ cyclohydrocarbyl, or $C_{3-10}$ heterocyclyl substituted with $C_{3-10}$ cycloalkyl or $C_{3-10}$ heterocycloalkyl is further optionally substituted with one or more substituents selected from hydrogen, deuterium, halogen, oxo, CN, OH, and $C_{3-10}$ saturated or partially saturated cycloalkyl or heterocyclyl;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5; and

q is an integer optionally selected from 0, 1, 2, and 3;

preferably, the compound, the pharmaceutically acceptable salt, the solvate, the enantiomer, the isotopically substituted compound, or the polymorph thereof has a structure of formula (IA):

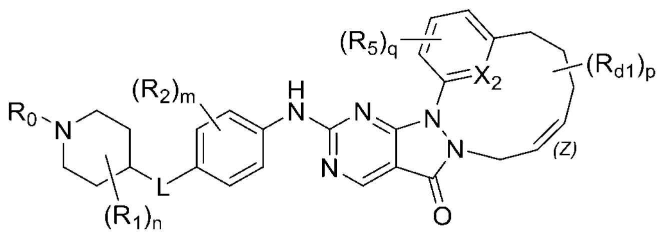

formula (IA),

wherein L, $X_2$, $R_0$, $R_1$, $R_2$, $R_5$, $R_{d1}$, m, n, and q are independently defined as described in any one of claims 1-5, and p is an integer optionally selected from 0, 1, 2, 3, and 4;

preferably, $X_2$ is independently selected from $-C(R_x)-$ and -N-;

L is optionally and independently selected from O, S, and NH;

$R_0$ is independently selected from H, deuterium, halogen, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein hydrogen on the aryl, saturated or partially saturated cycloalkyl, or heterocycloalkyl is optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, =O, and saturated or partially saturated $C_{3-6}$ cycloalkyl, wherein the $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl;

$R_1$, $R_{d1}$, and $R_x$ are independently selected from hydrogen, deuterium, halogen, cyano, amino, hydroxyl, $C_{1-10}$

alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, wherein the $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or optionally, $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5;

q is an integer optionally selected from 0, 1, 2, and 3; and

p is an integer optionally selected from 0, 1, 2, 3, and 4;

preferably, the compound, the pharmaceutically acceptable salt, the solvate, the enantiomer, the isotopically substituted compound, or the polymorph thereof has a structure of formula (IB):

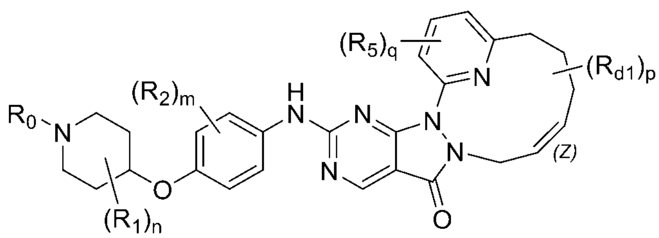

formula (IB),

wherein $R_0$, $R_1$, $R_2$, $R_5$, $R_{d1}$, m, n, and q are independently defined as described in any one of claims 1-5;

preferably, $R_0$ is independently selected from H, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ or each $R_5$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$ or any two $R_5$, together with the carbon atoms to which they are attached on the rings, form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ or $R_5$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN; or any two $R_2$ or any two $R_5$, together with the carbon atoms to which they are attached, form 5- to 6-membered heteroaryl, 3- to 8-membered saturated or partially saturated cycloalkyl, or 3- to 8-membered saturated or partially saturated heterocyclyl, wherein hydrogen on the aryl, saturated or partially saturated cycloalkyl, or heterocycloalkyl is optionally substituted with a group selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, =O, and saturated or partially saturated $C_{3-6}$ cycloalkyl, wherein the $C_{1-6}$ alkyl and $C_{1-6}$ alkoxy are optionally substituted with one or more groups selected from hydrogen, deuterium, halogen, oxo, CN, $CF_3$, OH, $OCH_3$, $OCH_2CH_3$, and saturated or

partially saturated $C_{3-6}$ cycloalkyl;

$R_1$ and $R_{d1}$ are independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl, wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, wherein the $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or optionally, $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4;

n is an integer optionally selected from 0, 1, 2, 3, 4, and 5;

q is an integer optionally selected from 0, 1, 2, and 3; and

p is an integer optionally selected from 0, 1, 2, 3, and 4;

preferably, the compound, the pharmaceutically acceptable salt, the solvate, the enantiomer, the isotopically substituted compound, or the polymorph thereof has a structure of formula (IC):

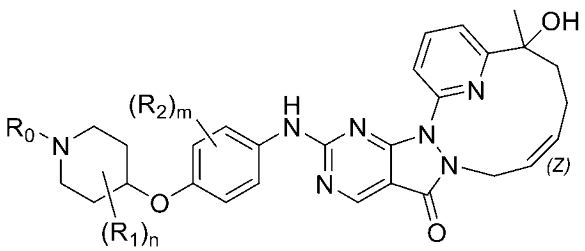

formula (IC),

wherein $R_0$, $R_1$, $R_2$, m, and n are independently defined as described in any one of claims 1-5; preferably, $R_0$ is independently selected from H, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

each $R_2$ may be identical or different and is independently selected from hydrogen, deuterium, halogen, -CN, -OH, -SH, $-NH_2$, -COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$, together with the carbon atoms to which they are attached on the ring form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

$R_1$ is independently selected from hydrogen, deuterium, halogen, cyano, amino, hydroxyl, $C_{1-10}$ alkyl, $C_{2-10}$ alkenyl, $C_{2-10}$ alkynyl, $C_{1-10}$ alkylamino, aryl, heteroaryl, *N,N*-di($C_{1-10}$ alkyl)amino, $C_{1-10}$ alkyloxy, $C_{1-10}$ alkylacyl, $C_{1-10}$ alkyloxy, $C_{1-10}$ alkylsulfonyl, $C_{1-10}$ alkylsulfinyl, $C_{3-10}$ cycloalkylamino, $C_{3-10}$ heterocycloalkylamino, $C_{3-10}$ cycloalkoxy, $C_{3-10}$ cycloalkylacyl, $C_{3-10}$ cycloalkoxyacetyl, $C_{3-10}$ cycloalkylsulfonyl, and $C_{3-10}$ cycloalkylsulfinyl,

wherein the alkyl, alkenyl, alkynyl, aryl, saturated or partially saturated cycloalkyl, and heterocycloalkyl are optionally substituted with one or more groups selected from hydrogen, deuterium, halogen, -CN, -OH, $CF_3$, $C_{1-6}$ alkyl, $C_{1-6}$ alkoxy, $-NH_2$, $-NHC_{1-6}$ alkyl, $-N(C_{1-6}$ alkyl$)_2$, oxy, and saturated or partially saturated $C_{3-6}$ cycloalkyl, wherein the $C_{1-6}$ alkyl and $C_{1-6}$ alkoxy are optionally further substituted with one or more groups selected from hydrogen, deuterium, halogen, oxo, CN, $CF_3$, OH, $OCH_3$, $OCH_2CH_3$, and saturated or partially saturated $C_{3-6}$ cycloalkyl; or optionally, $R_{d1}$ and $R_5$, together with the carbon atoms to which they are attached, may form 5- to 6-membered aryl or heteroaryl, 3- to 8-membered saturated or partially saturated cycloalkyl, or 3- to 8-membered saturated or partially saturated heterocyclyl, wherein the cycloalkyl and heterocycloalkyl are optionally substituted with one or more groups selected from hydrogen, deuterium, halogen, oxo, CN, $CF_3$, OH, $OCH_3$, and $OCH_2CH_3$;

the hetero represents any heteroatom optionally and independently selected from O, N, S, P, S=O, and $S(=O)_2$ and isotopes thereof;

the halogen is optionally and independently selected from F, Cl, Br, and I and isotopes thereof; m is an integer optionally selected from 0, 1, 2, 3, and 4; and

n is an integer optionally selected from 0, 1, 2, 3, and 4;

preferably, the compound, the pharmaceutically acceptable salt, the solvate, the enantiomer, the isotopically substituted compound, or the polymorph thereof has a structure of formula (ID):

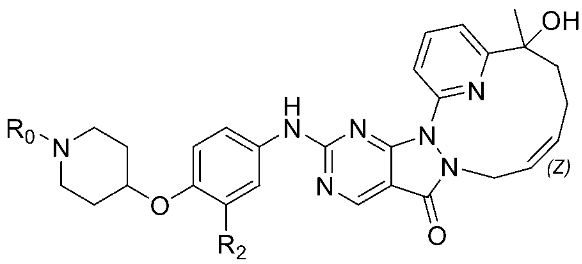

formula (ID),

wherein $R_0$ and $R_2$ are independently defined as described in any one of claims 1-5; preferably, $R_0$ is independently selected from H, alkyl, haloalkyl, deuterated alkyl, cycloalkyl, heterocyclyl, alkoxyalkyl, alkynyl, acetyl, methanesulfonyl, and phosphoryl; further, hydrogen(s) on $R_0$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

$R_2$ is independently selected from hydrogen, deuterium, halogen, -CN, -OH, -SH, $-NH_2$, - COOH, $C_{1-10}$ alkyl, $C_{2-10}$ alkenyl, $C_{2-10}$ alkynyl, $C_{1-10}$ alkoxy, $C_{2-10}$ heteroalkyl, $C_{3-10}$ saturated or partially saturated cycloalkyl, $C_{3-10}$ saturated or partially saturated heterocycloalkyl, $C_{1-10}$ alkyl substituted with $C_{3-10}$ cycloalkyl or $C_{3-10}$ heterocycloalkyl, $C_{2-10}$ heteroalkyl substituted with $C_{3-10}$ cycloalkyl, $C_{3-10}$ heterocyclyl, carboxyl or carboxyl surrogate substituted with $C_{1-10}$ alkyl, aryl, and heteroaryl; or any two $R_2$, together with the carbon atoms to which they are attached on the ring form a 3- to 8-membered monocyclic or polycyclic structure, wherein the monocyclic or polycyclic structure may be optionally selected from an aromatic ring structure, a heteroaromatic ring structure, an alicyclic structure, a heterocyclic structure, a fused-ring structure, a spiro-ring structure, and a bridged-ring structure, wherein the alicyclic structure, the heterocyclic structure, the fused-ring structure, the spiro-ring structure, or the bridged-ring structure may contain zero or more unsaturated alkene bonds; further, hydrogen(s) on $R_2$ is(are) optionally and optimally substituted with one or more substituents selected from H, deuterium, halogen, alkyl, alkenyl, alkynyl, cycloalkyl, heterocyclyl, $OCH_3$, carboxyl, OH, and CN;

the hetero represents any heteroatom optionally and independently selected from O, N, S, P, S=O, and $S(=O)_2$ and isotopes thereof; and

the halogen is optionally and independently selected from F, Cl, Br, and I and isotopes thereof;

preferably, the compound, the pharmaceutically acceptable salt, the solvate, the enantiomer, the isotopically substituted compound, or the polymorph thereof has a structure of formula (III):

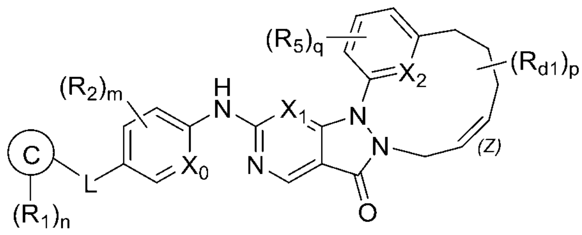

(III),

wherein ring C, L, $X_0$, $X_1$, $X_2$, $R_1$, $R_2$, $R_5$, $R_{d1}$, m, n, and q are independently defined as described in any one of claims 1-5;
preferably, the compound, the pharmaceutically acceptable salt, the solvate, the enantiomer, the isotopically substituted compound, or the polymorph thereof has a structure of formula (IIIA):

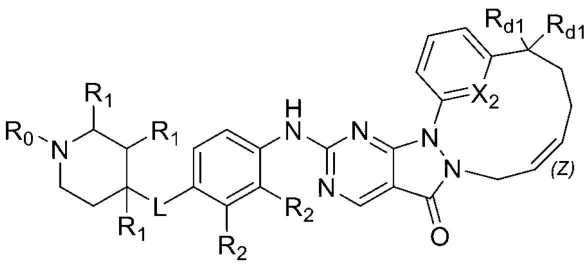

(IIIA),

wherein L, $X_2$, $R_0$, $R_1$, $R_2$, and $R_{d1}$ are independently defined as described in any one of claims 1-5;
preferably, the compound, the pharmaceutically acceptable salt, the solvate, the enantiomer, the isotopically substituted compound, or the polymorph thereof has a structure represented by formula (IE):

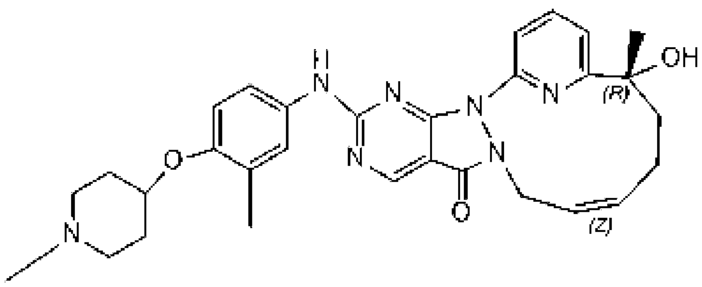

formula (IE).

7. The compound, the pharmaceutically acceptable salt, the solvate, the enantiomer, the isotopically substituted compound, or the polymorph thereof as claimed in any one of claims 1-6, having a structure selected from:

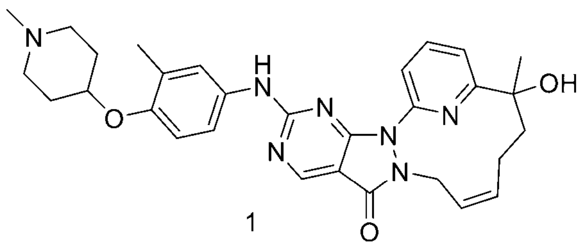

1 ,

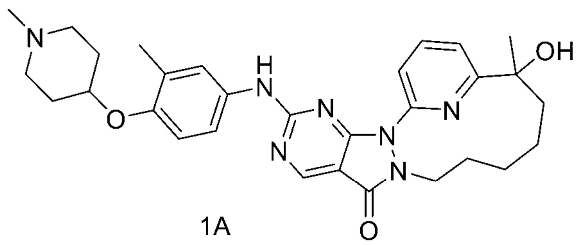

1A ,

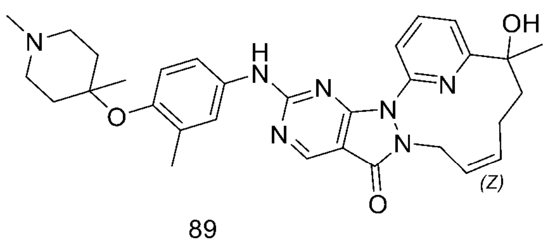

89 ,

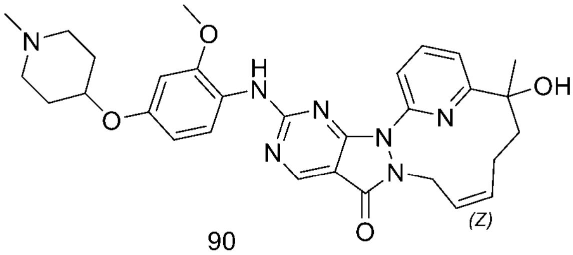

90 ,

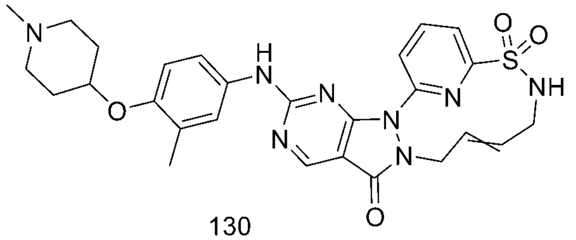

130 ,

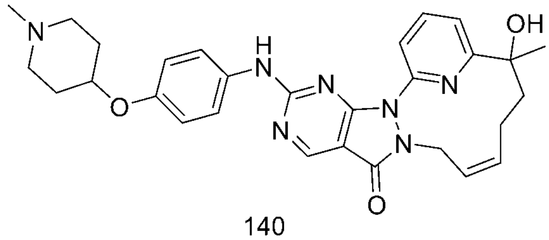

140 ,

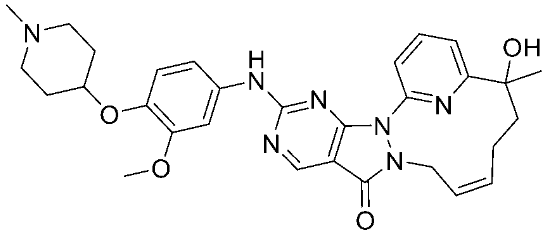

141 ,

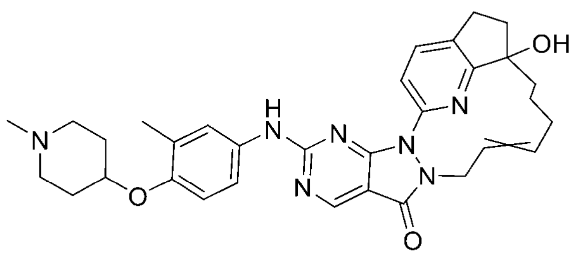

146 ,

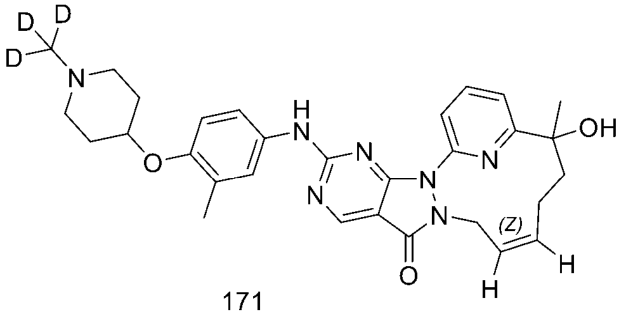

171 ,

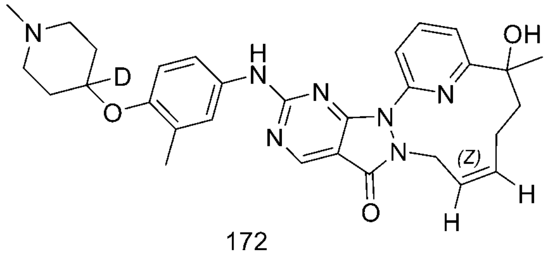

172 ,

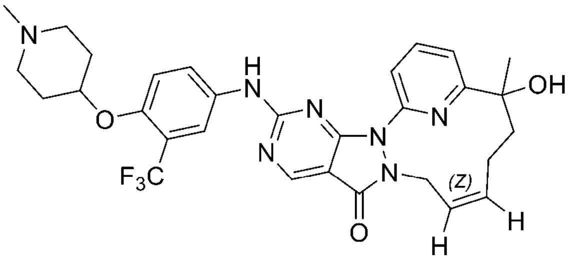

173 ,

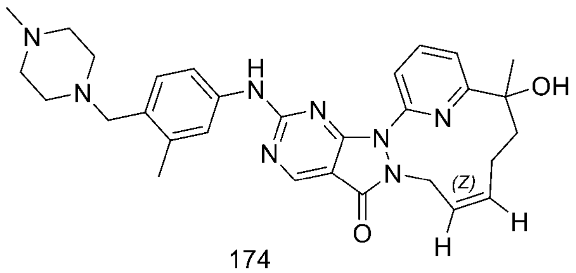

174

,

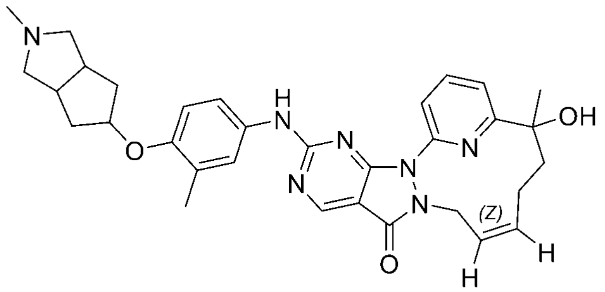

175

,

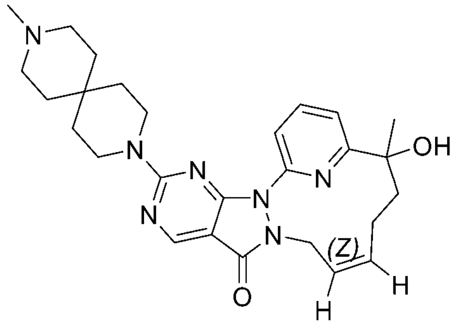

176

,

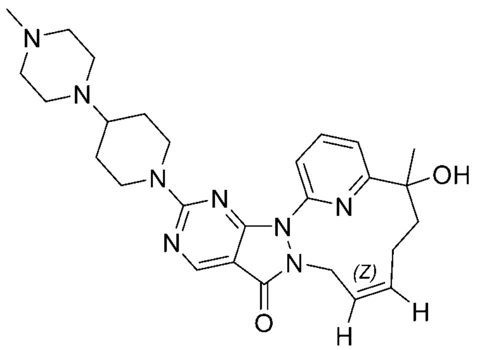

177

,

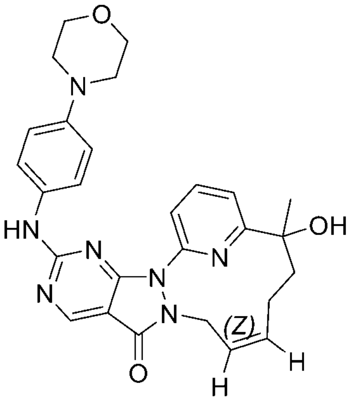

178

,

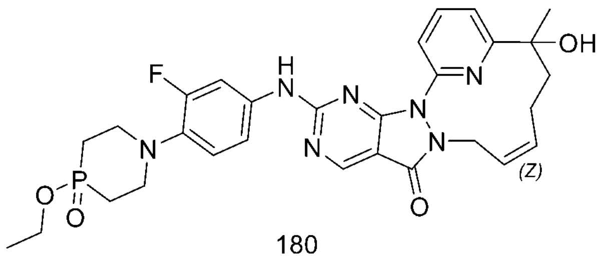

180

,

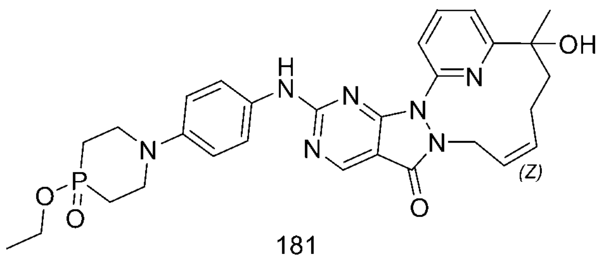

181

,

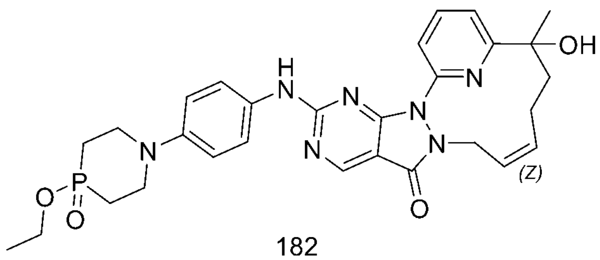

182

,

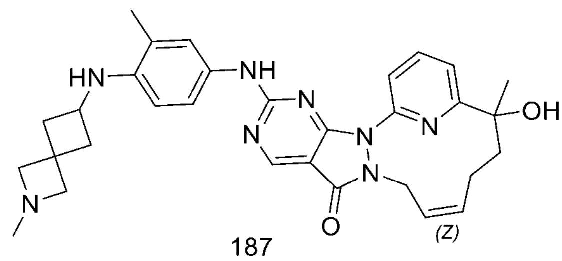

187

,

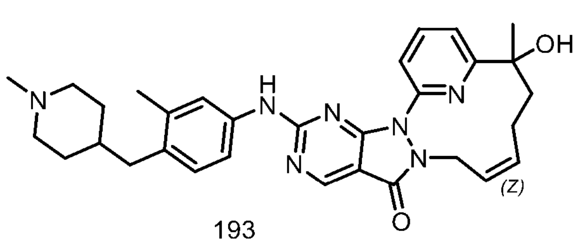

193

,

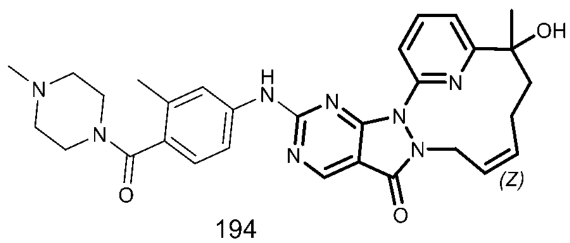

194

,

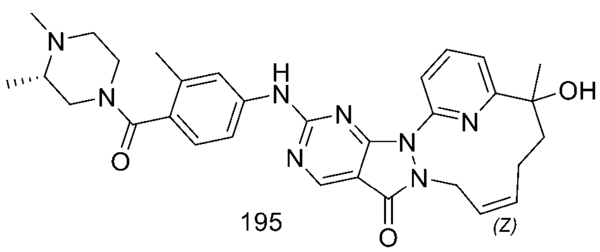

195

,

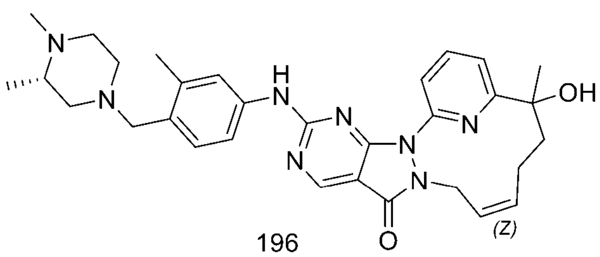

196

,

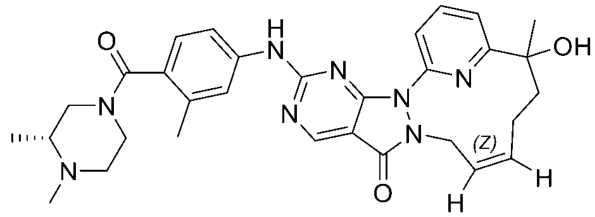

197

,

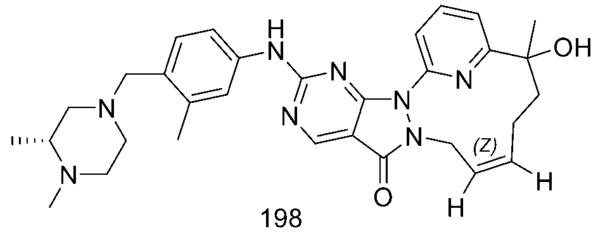

198

,

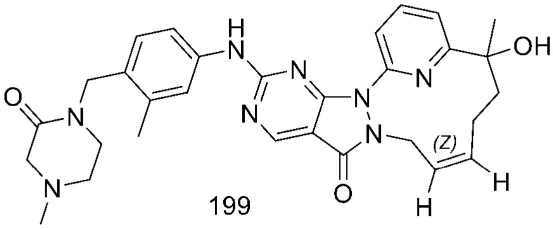

199

,

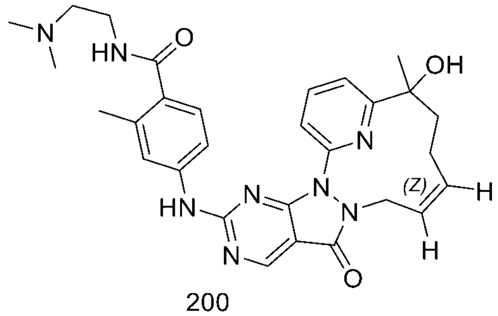

200

,

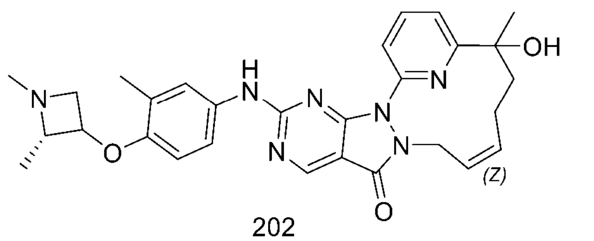

202

,

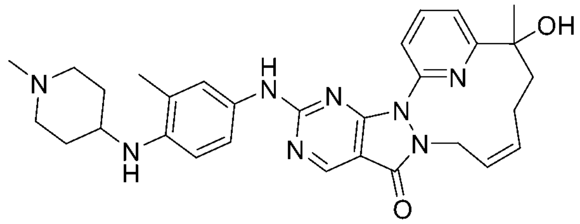

**203**

,

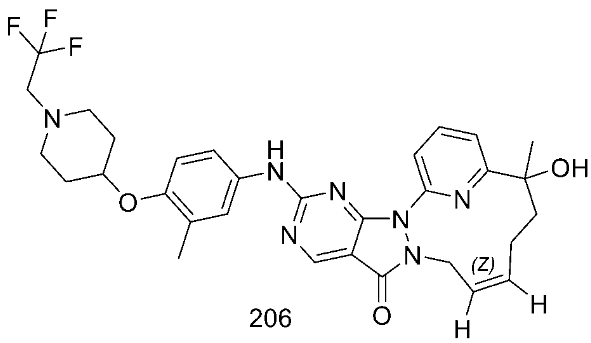

206

,

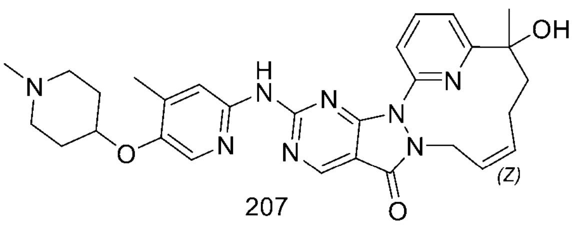
207
,

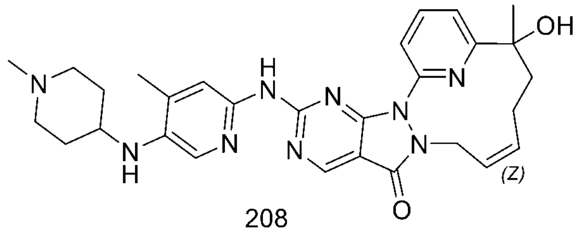
208
,

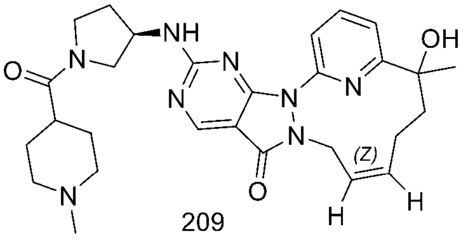
209
,

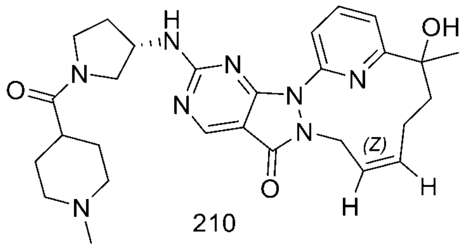
210
,

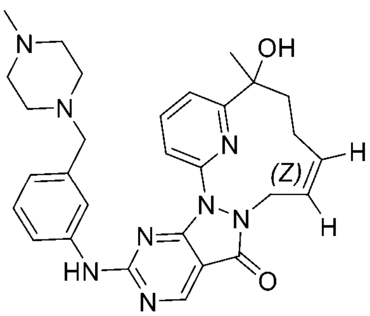
213
,

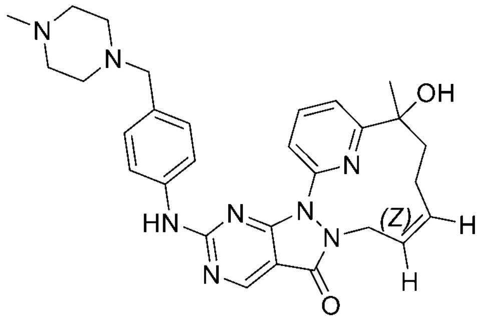
214
,

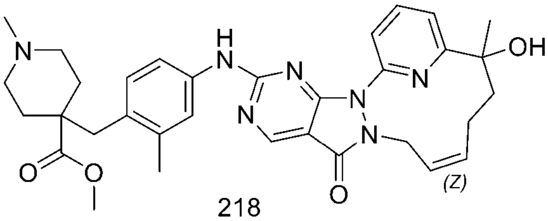
218
,

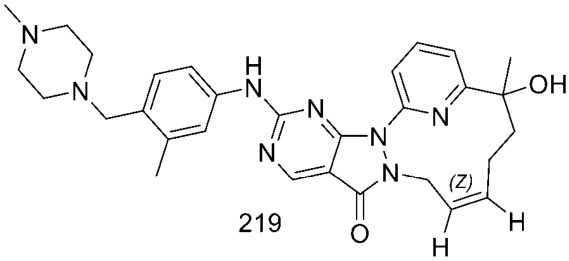
219
,

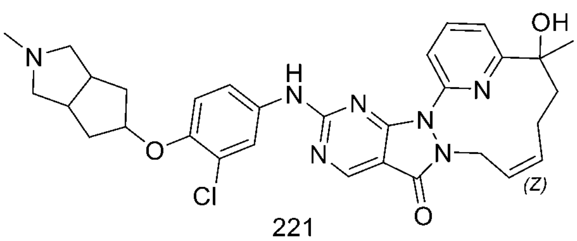
221
,

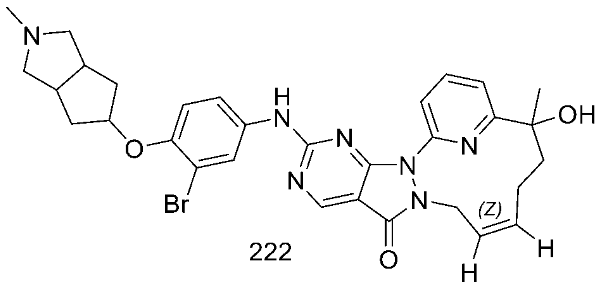

222

,

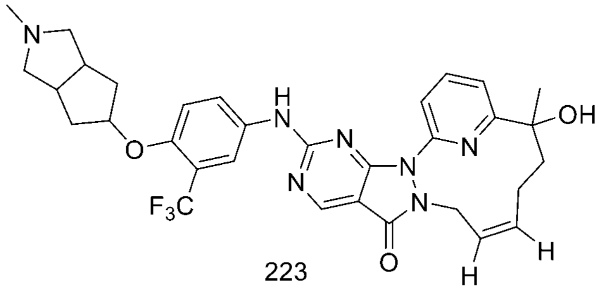

223

,

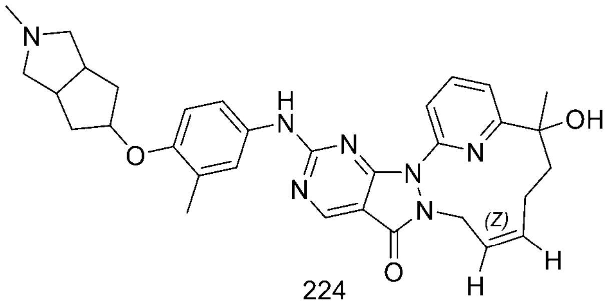

224

,

and

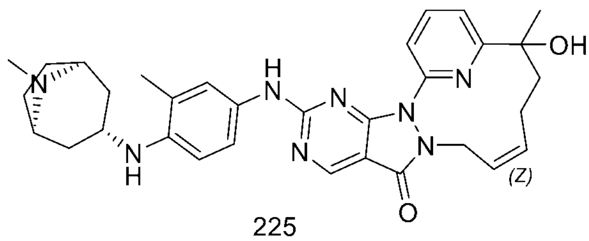

225

;

preferably, having a structure selected from:

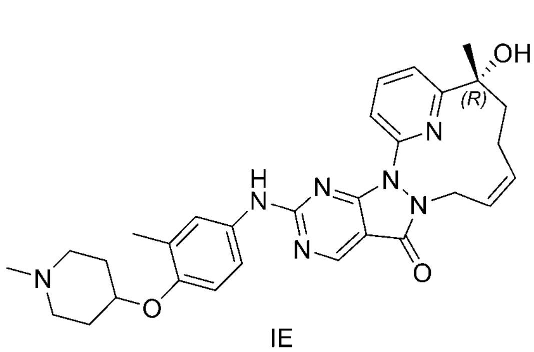

IE

,

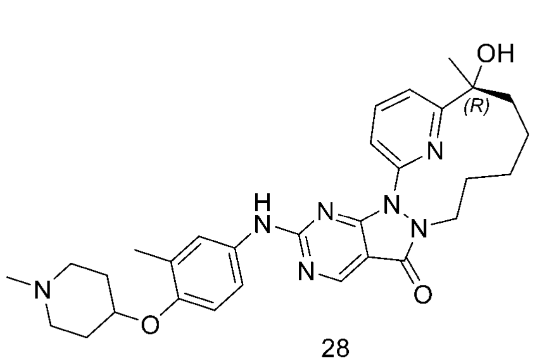

28

,

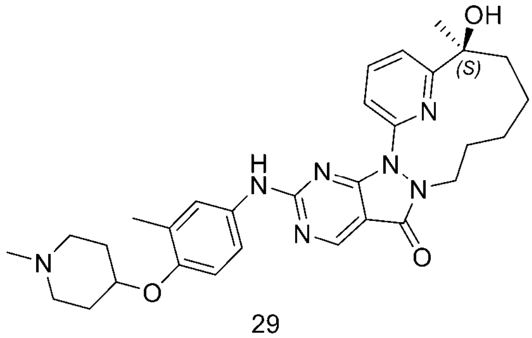

29

,

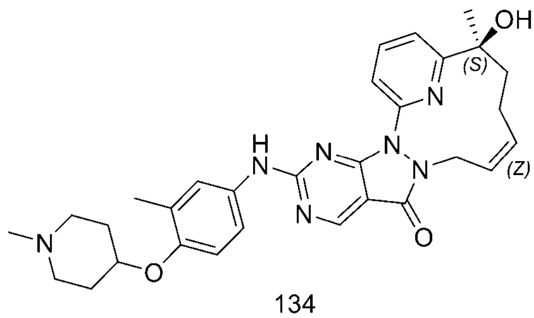

134

,

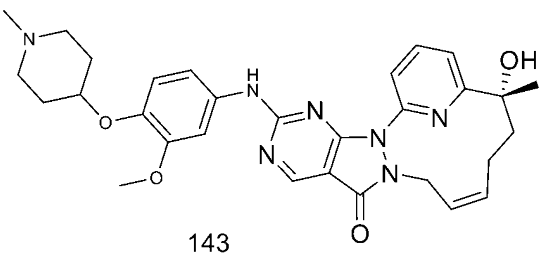

143

,

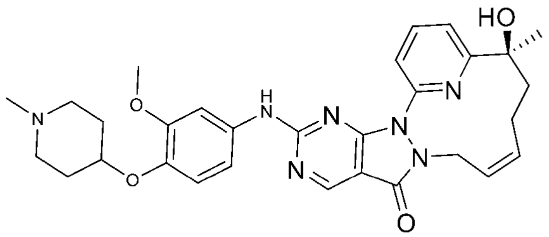

144

,

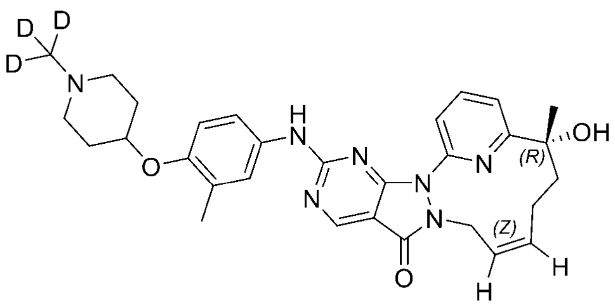

,

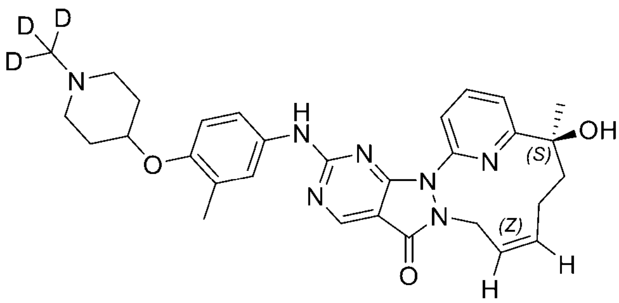

,

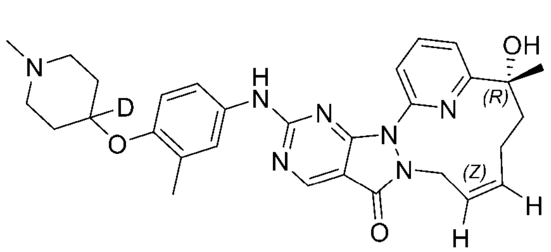

,

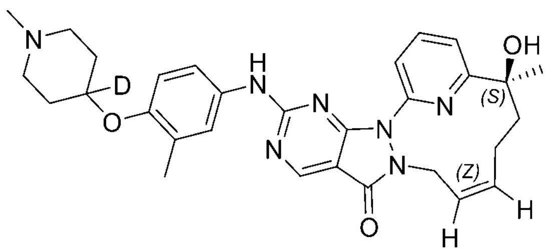

,

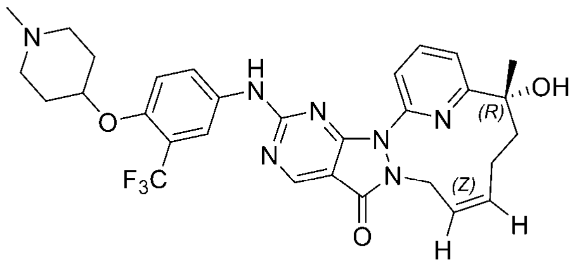

,

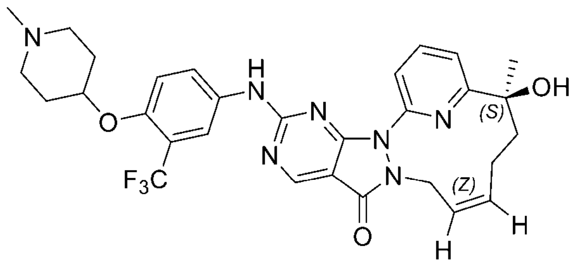

,

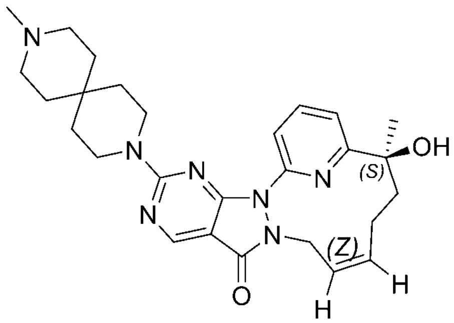

,

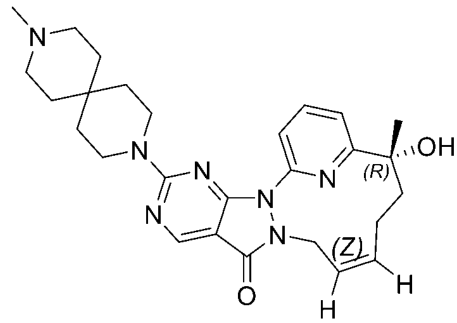

,

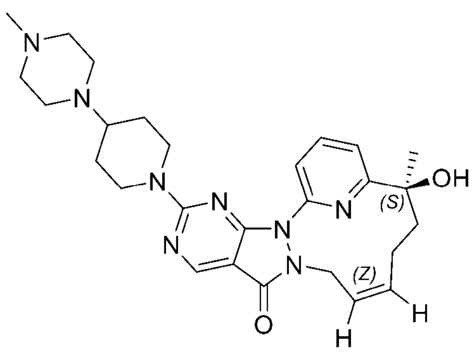

,

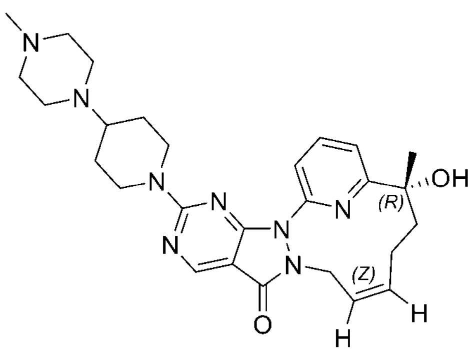

,

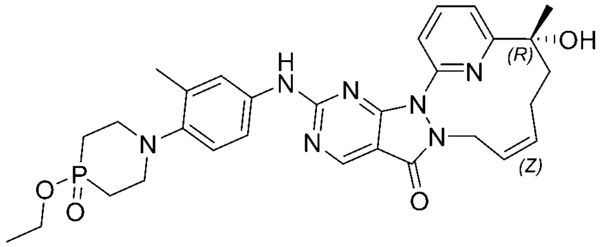

,

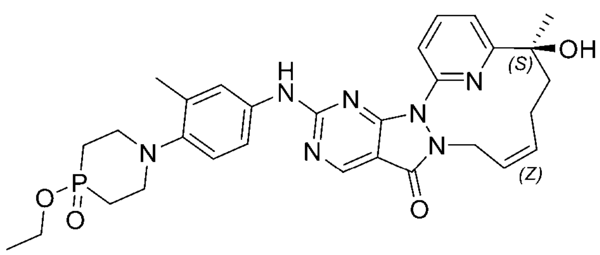

,

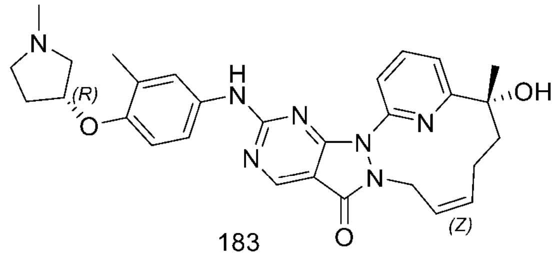

183

,

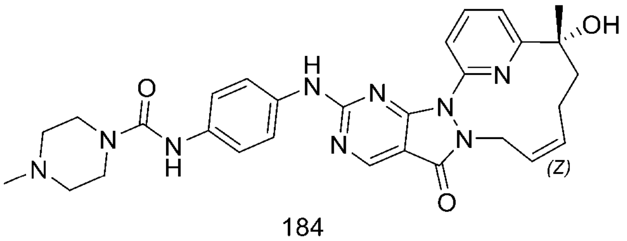

184

,

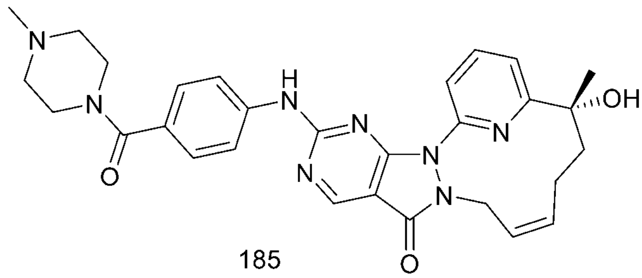

185

,

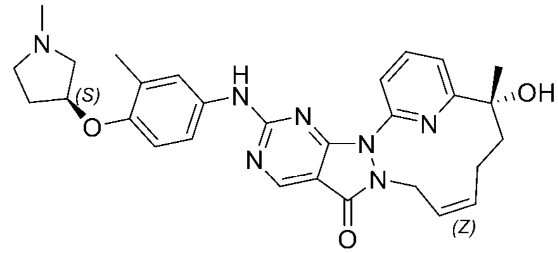

186

,

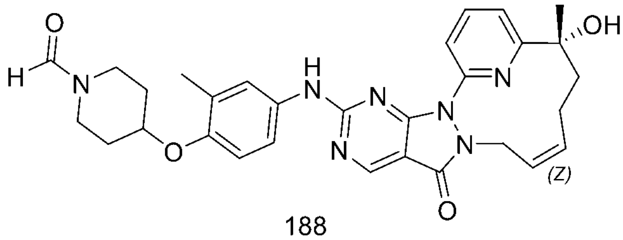

188

,

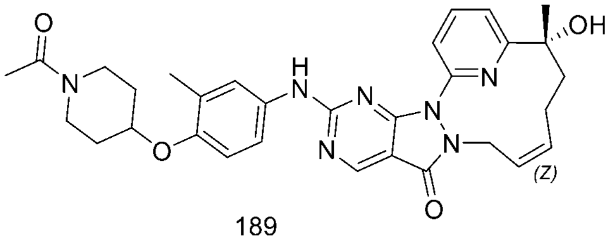

189

,

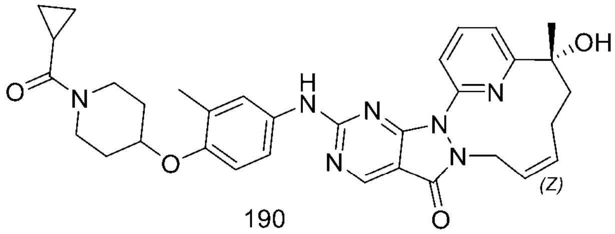

190

,

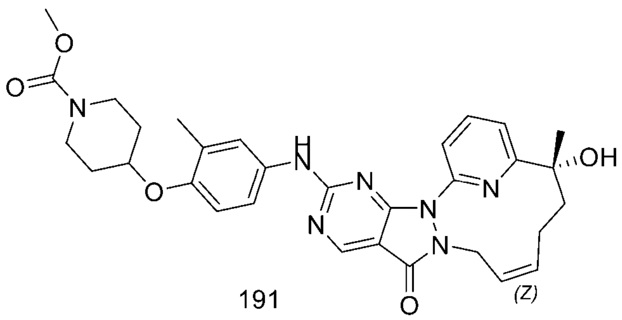

191

,

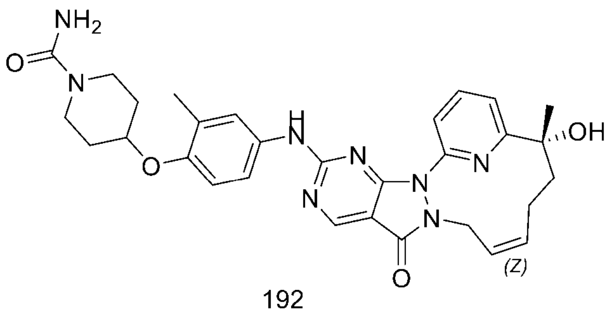

192

,

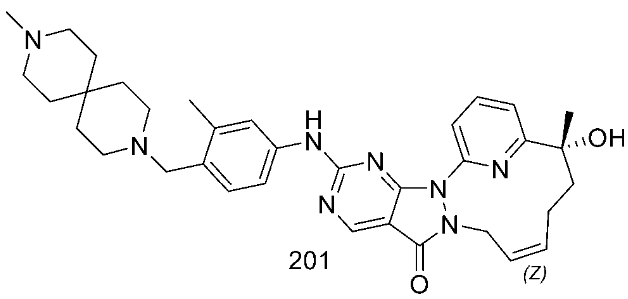

201

,

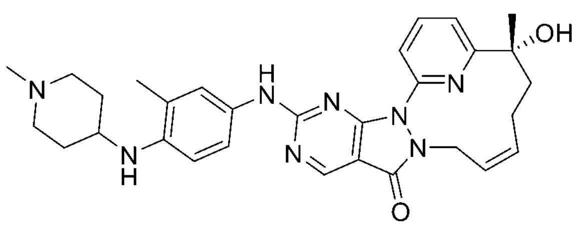

,

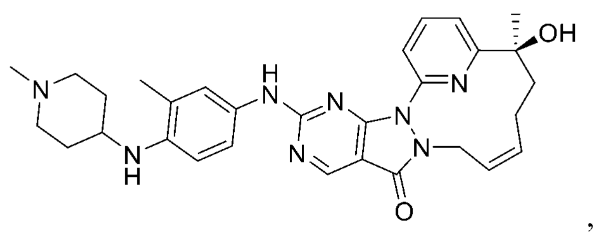

,

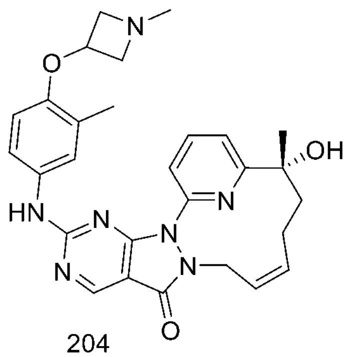
204

,

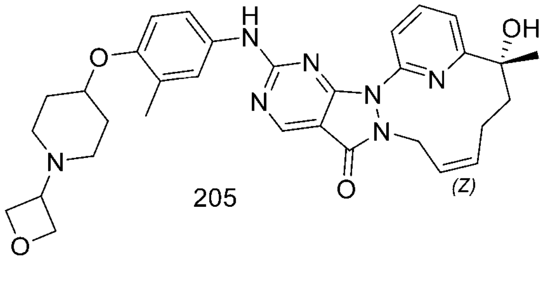
205

,

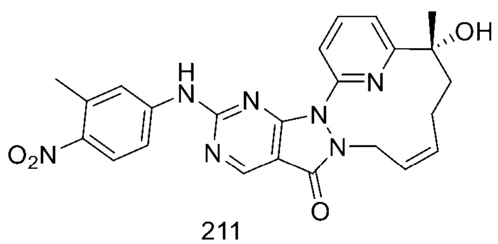
211

,

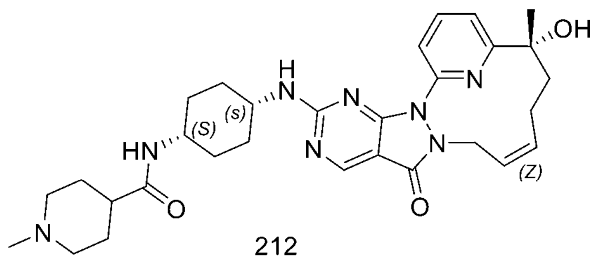
212

,

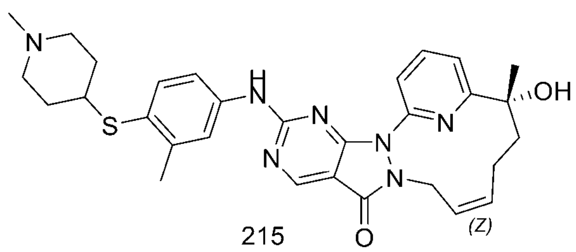
215

,

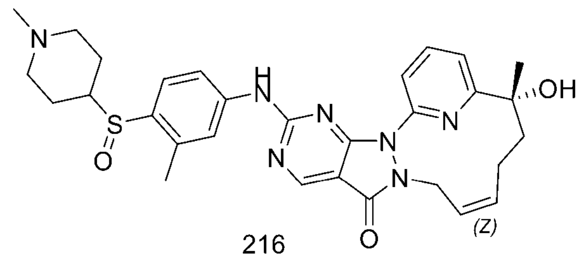
216

,

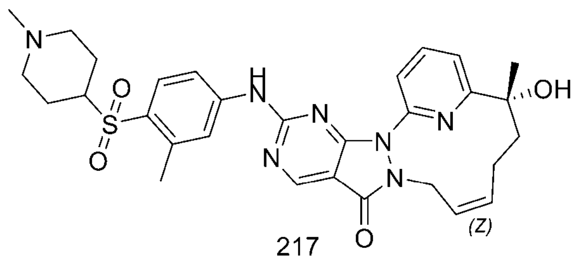
217

,

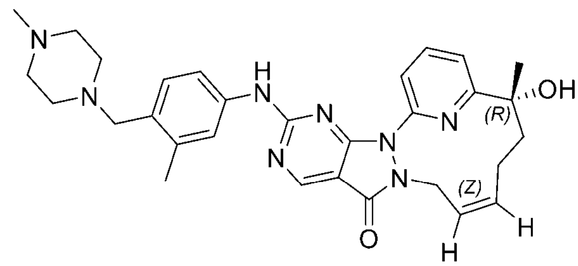

,

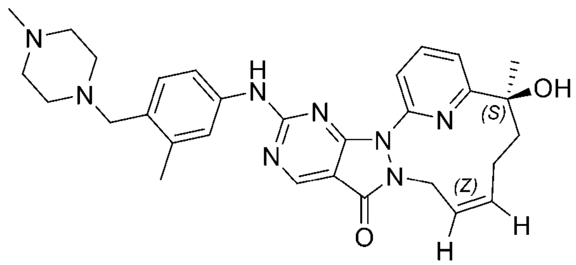

,

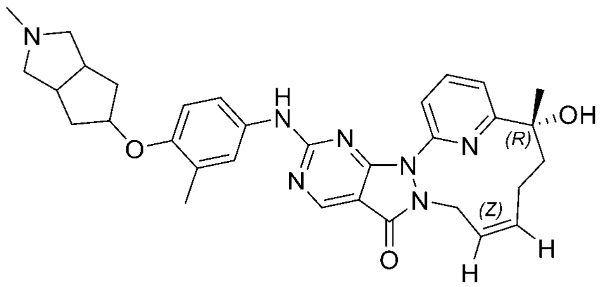

,

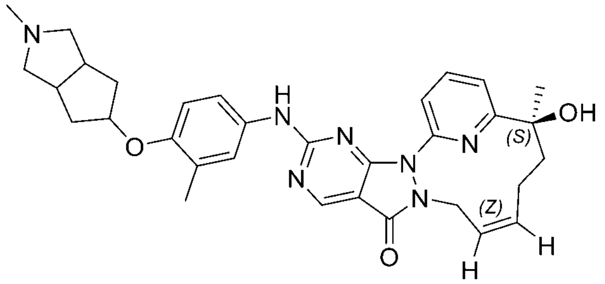

,

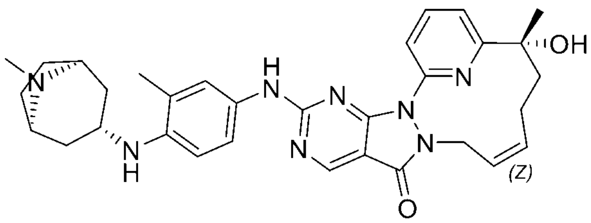

,

and

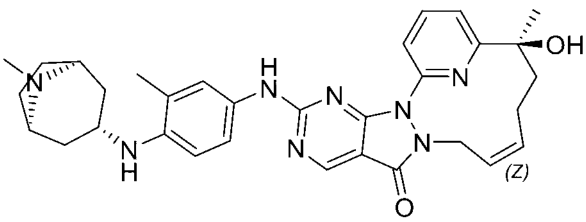

.

**8.** A polymorph of the compound represented by formula (IE):

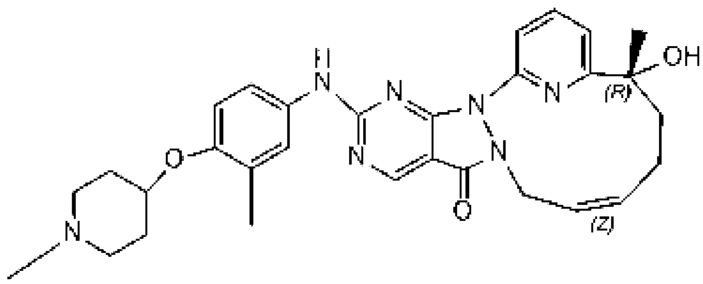

formula (IE),

wherein

the polymorph is selected from a nonsolvate crystal form, a hydrate crystal form, and a metastable crystal form of compound IE;
preferably, the nonsolvate crystal form may be a crystal form A, B, or C as described below; the hydrate crystal form may be a crystal form D, E, F, or G as described below; the metastable crystal form may be a crystal form H, I, J, K, L, M, N, O, P, or Q as described below;
regarding the crystal form A:

an X-ray powder diffraction pattern of the crystal form A comprises peaks at diffraction angles (2θ) of 5.10 ±0.2°, 15.72±0.2°, 15.25±0.2°, and 20.53±0.2°;
preferably, the crystal form A preferably further comprises peaks at diffraction angles (2θ) of 23.66±0.2°, 23.92±0.2°, 17.47±0.2°, 20.14±0.2°, and 24.44±0.2°;
preferably, the crystal form A more preferably further comprises peaks at diffraction angles (2θ) of 26.66 ±0.2°, 17.22±0.2°, 26.92±0.2°, 16.48±0.2°, 10.20±0.2°, and 21.97±0.2°;
preferably, the X-ray powder diffraction pattern of the crystal form A has the diffraction angles (2θ) shown in Table 1, wherein the 2θ angles have a margin of error of ±0.20°:

Table 1

| 2θ (°) | Intensity% | 2θ (°) | Intensity% |
|---|---|---|---|
| 5.10 | 100 | 24.45 | 6.1 |
| 7.91 | 0.3 | 25.47 | 0.3 |
| 8.57 | 0.3 | 25.66 | 0.4 |
| 10.21 | 2.0 | 26.32 | 0.7 |
| 12.53 | 0.7 | 26.67 | 4.3 |
| 13.40 | 0.5 | 26.92 | 2.8 |
| 13.74 | 0.4 | 27.66 | 0.7 |
| 13.95 | 0.7 | 28.19 | 1.5 |
| 14.65 | 1.3 | 28.48 | 0.3 |
| 15.25 | 14.2 | 28.81 | 0.4 |
| 15.72 | 14.5 | 29.63 | 0.3 |
| 16.48 | 2.5 | 30.31 | 1.7 |
| 17.22 | 3.5 | 30.72 | 1.6 |
| 17.47 | 7.0 | 31.37 | 0.9 |
| 18.04 | 0.5 | 32.78 | 0.4 |
| 19.48 | 0.9 | 33.25 | 0.7 |
| 20.14 | 7.0 | 34.39 | 0.4 |
| 20.53 | 10.5 | 35.39 | 0.6 |
| 21.31 | 1.9 | 35.61 | 0.6 |
| 21.97 | 2.0 | 36.89 | 0.3 |
| 22.83 | 1.9 | 38.05 | 0.3 |
| 23.67 | 9.8 | 38.63 | 0.2 |
| 23.92 | 8.8 | 39.14 | 0.6 |

preferably, the crystal form A has the X-ray powder diffraction intensities shown in Table 1;
preferably, the crystal form A has an X-ray powder diffraction pattern substantially as shown in FIG. 1;
preferably, according to DSC analysis, an endothermic peak appears when the crystal form A is heated to a peak temperature of about 180.86 °C;
preferably, the crystal form A has a DSC profile substantially as shown in FIG. 2;
preferably, the crystal form A has a TGA profile substantially as shown in FIG. 3;

regarding the crystal form B:

an X-ray powder diffraction pattern of the crystal form B comprises peaks at diffraction angles (2θ) of 10.33 ±0.2°, 5.18±0.2°, 13.69±0.2°, and 18.72±0.2°;
preferably, the crystal form B preferably further comprises peaks at diffraction angles (2θ) of 8.96±0.2°, 11.73 ±0.2°, 25.89±0.2°, 17.10±0.2°, and 23.20±0.2°;
preferably, the crystal form B more preferably further comprises peaks at diffraction angles (2θ) of 26.13 ±0.2°, 24.92±0.2°, 22.77±0.2°, 20.85±0.2°, 17.95±0.2°, and 36.45±0.2°;
preferably, the X-ray powder diffraction pattern of the crystal form B has the diffraction angles (2θ) shown in Table 2, wherein the 2θ angles have a margin of error of ±0.20°:

Table 2

| 2θ (°) | Intensity% | 2θ (°) | Intensity% |
|---|---|---|---|
| 5.18 | 72.2 | 24.92 | 4.9 |
| 8.96 | 47.2 | 25.46 | 0.6 |
| 10.33 | 100 | 25.89 | 6.2 |
| 11.73 | 8 | 26.13 | 5.3 |
| 13.69 | 52.5 | 27.59 | 2.3 |
| 17.10 | 6.1 | 28.01 | 0.8 |
| 17.43 | 1.5 | 29.30 | 2.4 |
| 17.95 | 3 | 31.35 | 0.8 |
| 18.72 | 47.5 | 32.90 | 0.6 |
| 20.85 | 3.4 | 33.70 | 2.2 |
| 21.35 | 0.8 | 35.98 | 1.4 |
| 21.74 | 2 | 36.45 | 2.5 |
| 22.77 | 4.4 | 36.80 | 0.8 |
| 23.20 | 5.7 | 37.91 | 1.1 |
| 23.79 | 0.6 | 38.91 | 0.5 |

preferably, the crystal form B has the X-ray powder diffraction intensities shown in Table 2;
preferably, the crystal form B has an X-ray powder diffraction pattern substantially as shown in FIG. 4;
preferably, according to DSC analysis, an endothermic peak appears when the crystal form B is heated to a peak temperature of about 179.71 °C;
preferably, the crystal form B has a DSC profile substantially as shown in FIG. 5;
preferably, the crystal form B has a TGA profile substantially as shown in FIG. 6;
regarding the crystal form C:

an X-ray powder diffraction pattern of the crystal form C comprises peaks at diffraction angles (2θ) of 5.39 ±0.2°, 12.05±0.2°, 9.70±0.2°, and 18.00±0.2°;
preferably, the crystal form C preferably further comprises peaks at diffraction angles (2θ) of 23.06±0.2°, 21.21±0.2°, 17.30±0.2°, 17.14±0.2°, and 25.09±0.2°;
preferably, the crystal form C more preferably further comprises peaks at diffraction angles (2θ) of 22.39 ±0.2°, 19.54±0.2°, 13.48±0.2°, 27.27±0.2°, 10.68±0.2°, and 5.98±0.2°;
preferably, the X-ray powder diffraction pattern of the crystal form C has the diffraction angles (2θ) shown in Table 3, wherein the 2θ angles have a margin of error of ±0.20°:

Table 3

| 2θ (°) | Intensity% | 2θ (°) | Intensity% |
|---|---|---|---|
| 5.39 | 100 | 21.21 | 7.6 |

(continued)

| 2θ (°) | Intensity% | 2θ (°) | Intensity% |
|---|---|---|---|
| 5.98 | 2.5 | 21.66 | 0.5 |
| 7.58 | 1.5 | 21.86 | 1 |
| 9.70 | 10.8 | 22.39 | 4.2 |
| 10.68 | 2.7 | 23.06 | 8.9 |
| 11.03 | 0.9 | 23.67 | 0.3 |
| 12.06 | 17.8 | 24.29 | 0.3 |
| 12.64 | 0.2 | 25.09 | 5.7 |
| 13.48 | 3.9 | 25.48 | 2.3 |
| 14.03 | 2.2 | 26.80 | 0.6 |
| 14.51 | 0.4 | 27.27 | 3.4 |
| 15.02 | 1.1 | 28.33 | 0.3 |
| 15.23 | 1.3 | 28.86 | 0.3 |
| 16.30 | 2.2 | 29.53 | 1.7 |
| 17.14 | 5.8 | 30.04 | 0.3 |
| 17.30 | 6.2 | 31.86 | 0.3 |
| 18.00 | 10.7 | 32.10 | 0.3 |
| 18.94 | 1.7 | 33.87 | 0.2 |
| 19.54 | 4.1 | 36.08 | 0.2 |
| 20.28 | 1.9 | | |

preferably, the crystal form C has the X-ray powder diffraction intensities shown in Table 3;
preferably, the crystal form C has an X-ray powder diffraction pattern substantially as shown in FIG. 7;
preferably, according to DSC analysis, an endothermic peak appears when the crystal form C is heated to a peak temperature of about 173.43 °C;
preferably, the crystal form C has a DSC profile substantially as shown in FIG. 8;
preferably, the crystal form C has a TGA profile substantially as shown in FIG. 9;
regarding the hydrate crystal form D:

an X-ray powder diffraction pattern of the crystal form D comprises peaks at diffraction angles (2θ) of 5.20±0.2°, 15.74±0.2°, 22.81±0.2°, and 18.39±0.2°;
preferably, the crystal form D preferably further comprises peaks at diffraction angles (2θ) of 23.12 ±0.2°, 7.81±0.2°, 16.78±0.2°, 5.89±0.2°, and 10.43±0.2°;
preferably, the crystal form D more preferably further comprises peaks at diffraction angles (2θ) of 25.44±0.2°, 13.08±0.2°, 15.06±0.2°, 18.96±0.2°, 21.04±0.2°, and 21.95±0.2°;
preferably, the X-ray powder diffraction pattern of the crystal form D has the diffraction angles (2θ) shown in Table 4, wherein the 2θ angles have a margin of error of ±0.20°:

Table 4

| 2θ (°) | Intensity% | 2θ (°) | Intensity% |
|---|---|---|---|
| 5.20 | 100 | 23.12 | 1.8 |
| 5.89 | 0.8 | 23.79 | 0.5 |
| 7.81 | 1.4 | 24.96 | 0.2 |
| 10.43 | 0.6 | 25.44 | 0.6 |
| 13.08 | 0.5 | 27.15 | 0.5 |

(continued)

| 2θ (°) | Intensity% | 2θ (°) | Intensity% |
|---|---|---|---|
| 15.06 | 0.5 | 27.84 | 0.3 |
| 15.74 | 4.6 | 29.09 | 0.3 |
| 16.78 | 1.4 | 29.53 | 0.2 |
| 18.04 | 0.1 | 31.85 | 0.4 |
| 18.39 | 1.8 | 33.21 | 0.1 |
| 18.78 | 0.3 | 34.56 | 0.2 |
| 18.96 | 0.5 | 35.77 | 0.2 |
| 20.12 | 0.2 | 36.84 | 0.2 |
| 21.04 | 0.5 | 38.06 | 0.2 |
| 21.95 | 0.5 | 39.33 | 0.2 |
| 22.81 | 3.4 | | |

preferably, the crystal form D has the X-ray powder diffraction intensities shown in Table 4;
preferably, the crystal form D has an X-ray powder diffraction pattern substantially as shown in FIG. 10;
preferably, according to DSC analysis, endothermic peaks appear when the crystal form D is heated to peak temperatures of about 157.79 °C and 181.65 °C;
preferably, the crystal form D has a DSC profile substantially as shown in FIG. 11;
preferably, the crystal form D has a TGA profile substantially as shown in FIG. 12;
regarding the hydrate crystal form E:

an X-ray powder diffraction pattern of the crystal form E comprises peaks at diffraction angles (2θ) of 4.54±0.2°, 13.72±0.2°, 9.12±0.2°, and 18.35±0.2°;
preferably, the crystal form E preferably further comprises peaks at diffraction angles (2θ) of 15.88±0.2°, 23.00±0.2°, 5.24±0.2°, 23.88±0.2°, and 17.20±0.2°;
preferably, the crystal form E more preferably further comprises peaks at diffraction angles (2θ) of 21.72±0.2°, 20.42±0.2°, 23.49±0.2°, 25.01±0.2°, 25.29±0.2°, and 9.72±0.2°;
preferably, the X-ray powder diffraction pattern of the crystal form E has the diffraction angles (2θ) shown in Table 5, wherein the 2θ angles have a margin of error of ±0.20°:

Table 5

| 2θ (°) | Intensity% | 2θ (°) | Intensity% |
|---|---|---|---|
| 4.54 | 100 | 21.72 | 0.4 |
| 5.24 | 0.5 | 22.60 | 0.2 |
| 9.12 | 1.9 | 23.01 | 0.8 |
| 9.72 | 0.2 | 23.49 | 0.3 |
| 13.72 | 2.3 | 23.88 | 0.5 |
| 14.83 | 0.2 | 25.01 | 0.3 |
| 15.88 | 0.9 | 25.29 | 0.3 |
| 17.20 | 0.4 | 26.11 | 0.2 |
| 17.61 | 0.2 | 26.79 | 0.2 |
| 18.35 | 1 | 27.68 | 0.1 |
| 19.73 | 0.1 | 30.08 | 0.2 |
| 20.15 | 0.1 | 37.83 | 0.1 |
| 20.42 | 0.3 | 38.36 | 0.2 |

(continued)

| 2θ (°) | Intensity% | 2θ (°) | Intensity% |
|---|---|---|---|
| 21.06 | 0.2 | | |

preferably, the crystal form E has the X-ray powder diffraction intensities shown in Table 5;
preferably, the crystal form E has an X-ray powder diffraction pattern substantially as shown in FIG. 13;
preferably, according to DSC analysis, endothermic peaks appear when the crystal form E is heated to peak temperatures of about 69.77 °C and 181.95 °C;
preferably, the crystal form E has a DSC profile substantially as shown in FIG. 14;
preferably, the crystal form E has a TGA profile substantially as shown in FIG. 15;
regarding the hydrate crystal form F:

an X-ray powder diffraction pattern of the crystal form F comprises peaks at diffraction angles (2θ) of 9.08±0.2°, 18.27±0.2°, 6.25±0.2°, and 13.73±0.2°;
preferably, the crystal form F preferably further comprises peaks at diffraction angles (2θ) of 7.52±0.2°, 5.12±0.2°, 10.25±0.2°, 10.50±0.2°, and 15.16±0.2°;
preferably, the crystal form F more preferably further comprises peaks at diffraction angles (2θ) of 15.00±0.2°, 16.60±0.2°, 19.04±0.2°, 21.46±0.2°, 27.60±0.2°, and 11.71±0.2°;
preferably, the X-ray powder diffraction pattern of the crystal form F has the diffraction angles (2θ) shown in Table 6, wherein the 2θ angles have a margin of error of ±0.20°:

Table 6

| 2θ (°) | Intensity% | 2θ (°) | Intensity% |
|---|---|---|---|
| 5.12 | 8.0 | 18.27 | 30.4 |
| 6.25 | 24.1 | 19.04 | 3.1 |
| 7.52 | 14.5 | 20.81 | 0.6 |
| 9.08 | 100.0 | 21.46 | 1.5 |
| 10.25 | 7.8 | 22.65 | 0.7 |
| 10.50 | 6.8 | 23.61 | 0.8 |
| 11.71 | 1.1 | 24.51 | 0.8 |
| 13.73 | 20.1 | 27.60 | 1.2 |
| 15.00 | 3.6 | 29.24 | 0.8 |
| 15.16 | 5.0 | 29.47 | 1.0 |
| 16.60 | 3.5 | 32.44 | 0.6 |
| 17.17 | 0.8 | | |

preferably, the crystal form F has the X-ray powder diffraction intensities shown in Table 6;
preferably, the crystal form F has an X-ray powder diffraction pattern substantially as shown in FIG. 16;
preferably, according to DSC analysis, endothermic peaks appear when the crystal form F is heated to peak temperatures of about 101.36 °C and 177.98 °C, and an exothermic peak appears at about 146.72 °C;
preferably, the crystal form F has a DSC profile substantially as shown in FIG. 17;
preferably, the crystal form F has a TGA profile substantially as shown in FIG. 18;
regarding the hydrate crystal form G:

an X-ray powder diffraction pattern of the crystal form G comprises peaks at diffraction angles (2θ) of 9.06±0.2°, 14.98±0.2°, 15.76±0.2°, and 18.16±0.2°;
according to the present disclosure, the crystal form G preferably further comprises peaks at diffraction angles (2θ) of 12.04±0.2°, 20.96±0.2°, 24.12±0.2°, 9.89±0.2°, and 28.54±0.2°;

according to the present disclosure, the crystal form G more preferably further comprises peaks at diffraction angles (2θ) of 7.93±0.2°, 6.03±0.2°, 25.73±0.2°, 27.70±0.2°, 21.78 ±0.2°, and 24.51±0.2°;

preferably, the X-ray powder diffraction pattern of the crystal form G has the diffraction angles (2θ) shown in Table 7, wherein the 2θ angles have a margin of error of ±0.20°:

Table 7

| 2θ (°) | Intensity% | 2θ ( ° ) | Intensity% |
|---|---|---|---|
| 6.04 | 4.5 | 27.70 | 4 |
| 7.93 | 5.7 | 28.54 | 6.5 |
| 9.06 | 100 | 29.86 | 0.9 |
| 9.90 | 6.6 | 30.33 | 0.6 |
| 12.04 | 35 | 30.86 | 1.6 |
| 14.98 | 65.7 | 31.23 | 1 |
| 15.76 | 48.5 | 31.79 | 0.9 |
| 18.16 | 47.8 | 32.46 | 0.8 |
| 19.17 | 1.7 | 33.35 | 1.1 |
| 20.16 | 1.3 | 34.09 | 1.2 |
| 20.96 | 22.1 | 34.91 | 2.5 |
| 21.78 | 3 | 35.84 | 0.9 |
| 22.64 | 1.5 | 36.74 | 1.4 |
| 24.12 | 13.8 | 37.38 | 1.4 |
| 24.51 | 2.9 | 39.62 | 2.3 |
| 25.73 | 4.4 | | |

preferably, the crystal form G has the X-ray powder diffraction intensities shown in Table 7;

preferably, the crystal form G has an X-ray powder diffraction pattern substantially as shown in FIG. 19;

preferably, according to DSC analysis, an endothermic peak appears when the crystal form G is heated to a peak temperature of about 126.74 °C;

preferably, the crystal form G has a DSC profile substantially as shown in FIG. 20;

preferably, the crystal form G has a TGA profile substantially as shown in FIG. 21;

regarding the metastable crystal form H:

an X-ray powder diffraction pattern of the metastable crystal form H comprises peaks at diffraction angles (2θ) of 6.02±0.2°, 3.96±0.2°, 12.66±0.2°, and 9.35±0.2°;

preferably, the crystal form H preferably further comprises peaks at diffraction angles (20) of 15.49±0.2°, 12.06±0.2°, 4.42±0.2°, 11.38±0.2°, and 18.31±0.2°;

preferably, the crystal form H more preferably further comprises peaks at diffraction angles (2θ) of 6.68±0.2°, 13.87±0.2°, 9.88±0.2°, 19.05±0.2°, 17.92±0.2°, and 8.94 ±0.2°;

preferably, the X-ray powder diffraction pattern of the crystal form H has the diffraction angles (2θ) shown in Table 8, wherein the 2θ angles have a margin of error of ±0.20°:

Table 8

| 2θ (°) | Intensity% | 2θ (°) | Intensity% |
|---|---|---|---|
| 3.96 | 83.5 | 17.92 | 8.7 |
| 4.42 | 12.5 | 18.31 | 10.1 |

(continued)

| 2θ (°) | Intensity% | 2θ (°) | Intensity% |
|---|---|---|---|
| 6.02 | 100.0 | 19.05 | 8.8 |
| 6.68 | 10.0 | 20.09 | 7.7 |
| 8.94 | 8.6 | 20.96 | 3.2 |
| 9.35 | 16.3 | 21.20 | 4.5 |
| 9.88 | 9.6 | 22.02 | 4.9 |
| 10.36 | 4.7 | 22.57 | 3.5 |
| 10.70 | 3.5 | 23.05 | 6.8 |
| 11.38 | 12.5 | 24.09 | 1.9 |
| 12.06 | 15.1 | 24.68 | 5.9 |
| 12.66 | 29.1 | 25.43 | 4.7 |
| 13.13 | 5.5 | 26.05 | 2.5 |
| 13.87 | 9.8 | 26.26 | 3.1 |
| 14.56 | 3.3 | 27.33 | 1.7 |
| 14.85 | 2.4 | 29.41 | 1.4 |
| 15.49 | 15.6 | 31.44 | 1.6 |
| 15.76 | 3.1 | 33.99 | 1.1 |
| 16.31 | 8.0 | 35.32 | 1.1 |
| 16.66 | 5.6 | | |

preferably, the crystal form H has the X-ray powder diffraction intensities shown in Table 8;
preferably, the crystal form H has an X-ray powder diffraction pattern substantially as shown in FIG. 22;
preferably, according to DSC analysis, endothermic peaks appear when the crystal form H is heated to peak temperatures of about 61.06 °C and 151.59 °C;
preferably, the crystal form H has a DSC profile substantially as shown in FIG. 23;
preferably, the crystal form H has a TGA profile substantially as shown in FIG. 24;
regarding the metastable crystal form I:

an X-ray powder diffraction pattern of the metastable crystal form I comprises peaks at diffraction angles (2θ) of 4.93±0.2°, 4.37±0.2°, 16.01±0.2°, and 7.45 ±0.2°;
preferably, the crystal form I preferably further comprises peaks at diffraction angles (2θ) of 6.45±0.2°, 22.79±0.2°, 17.57±0.2°, 17.98±0.2°, and 15.14±0.2°;
preferably, the crystal form I more preferably further comprises peaks at diffraction angles (2θ) of 22.48±0.2°, 16.65±0.2°, 10.00±0.2°, 12.59±0.2°, 20.05±0.2°, and 25.54±0.2°;
preferably, the X-ray powder diffraction pattern of the crystal form I has the diffraction angles (2θ) shown in Table 9, wherein the 2θ angles have a margin of error of ±0.20°:

Table 9

| 2θ (°) | Intensity% | 2θ (°) | Intensity% |
|---|---|---|---|
| 4.37 | 19.9 | 16.65 | 1.1 |
| 4.93 | 100 | 17.57 | 1.3 |
| 6.45 | 2.3 | 17.98 | 1.3 |

(continued)

| 2θ (°) | Intensity% | 2θ (°) | Intensity% |
|---|---|---|---|
| 7.45 | 2.7 | 20.05 | 0.8 |
| 10.00 | 0.9 | 22.48 | 1.2 |
| 12.59 | 0.8 | 22.79 | 1.5 |
| 14.37 | 0.3 | 25.54 | 0.4 |
| 15.14 | 1.2 | 26.96 | 0.4 |
| 16.01 | 3.3 | | |

preferably, the crystal form I has the X-ray powder diffraction intensities shown in Table 9;
preferably, the crystal form I has an X-ray powder diffraction pattern substantially as shown in FIG. 25;
regarding the metastable crystal form J:

an X-ray powder diffraction pattern of the metastable crystal form J comprises peaks at diffraction angles (2θ) of 5.78±0.2°, 8.06±0.2°, 12.04±0.2°, and 16.19±0.2°;
preferably, the crystal form J preferably further comprises peaks at diffraction angles (2θ) of 17.14±0.2°, 14.55±0.2°, 10.35±0.2°, 11.59±0.2°, and 19.77 ±0.2°;
preferably, the crystal form J more preferably further comprises peaks at diffraction angles (2θ) of 7.25±0.2°, 20.81±0.2°, 26.22±0.2°, 20.57±0.2°, 24.25±0.2°, and 22.62±0.2°;
preferably, the X-ray powder diffraction pattern of the crystal form J has the diffraction angles (2θ) shown in Table 10, wherein the 2θ angles have a margin of error of ±0.20°:

Table 10

| 2θ (°) | Intensity% | 2θ (°) | Intensity% |
|---|---|---|---|
| 5.78 | 100.0 | 23.32 | 2.5 |
| 7.25 | 4.1 | 23.88 | 1.0 |
| 8.06 | 65.3 | 24.25 | 2.6 |
| 10.35 | 11.9 | 25.23 | 0.7 |
| 11.59 | 10.9 | 26.22 | 3.2 |
| 12.04 | 64.0 | 26.84 | 0.9 |
| 13.79 | 1.5 | 27.51 | 1.2 |
| 14.24 | 1.5 | 27.80 | 0.9 |
| 14.55 | 13.0 | 28.42 | 2.4 |
| 15.18 | 0.8 | 29.30 | 1.4 |
| 16.19 | 38.0 | 30.20 | 1.4 |
| 16.73 | 2.3 | 32.02 | 0.6 |
| 17.14 | 19.4 | 32.73 | 2.2 |
| 19.77 | 6.2 | 33.30 | 0.4 |
| 20.57 | 2.8 | 34.73 | 0.9 |
| 20.81 | 3.6 | 35.89 | 0.6 |
| 21.92 | 1.5 | 36.10 | 2.0 |

(continued)

| 2θ (°) | Intensity% | 2θ (°) | Intensity% |
|---|---|---|---|
| 22.06 | 1.6 | 38.54 | 1.2 |
| 22.62 | 2.5 | | |

preferably, the crystal form J has the X-ray powder diffraction intensities shown in Table 10;
preferably, the crystal form J has an X-ray powder diffraction pattern substantially as shown in FIG. 26;
regarding the metastable crystal form K:

an X-ray powder diffraction pattern of the metastable crystal form K comprises peaks at diffraction angles (2θ) of 4.05±0.2°, 4.89±0.2°, 4.44±0.2°, and 5.92±0.2°;
preferably, the crystal form K preferably further comprises peaks at diffraction angles (2θ) of 12.53±0.2°, 7.87±0.2°, 16.76±0.2°, 8.83 ±0.2°, and 5.55±0.2°;
preferably, the crystal form K more preferably further comprises peaks at diffraction angles (2θ) of 14.59±0.2°, 9.84±0.2°, 13.38±0.2°, 20.10 ±0.2°, 14.08±0.2°, and 15.76±0.2°;
preferably, the X-ray powder diffraction pattern of the crystal form K has the diffraction angles (2θ) shown in Table 11, wherein the 2θ angles have a margin of error of ±0.20°:

Table 11

| 2θ (°) | Intensity% | 2θ (°) | Intensity% |
|---|---|---|---|
| 4.05 | 100 | 14.08 | 5 |
| 4.44 | 47.5 | 14.59 | 7.5 |
| 4.89 | 88.3 | 15.10 | 3.3 |
| 5.55 | 9.3 | 15.51 | 3.1 |
| 5.92 | 21.7 | 15.76 | 5 |
| 6.86 | 3.7 | 16.76 | 11.8 |
| 6.86 | 3.7 | 17.75 | 3.1 |
| 7.29 | 4.7 | 18.72 | 2.1 |
| 7.87 | 11.8 | 18.88 | 3.3 |
| 8.83 | 9.5 | 19.76 | 3.4 |
| 9.84 | 5.4 | 20.10 | 5.4 |
| 10.08 | 3.4 | 20.40 | 3.1 |
| 10.27 | 2.3 | 22.42 | 2.8 |
| 10.76 | 4.9 | 23.24 | 4.6 |
| 12.53 | 18.2 | 26.63 | 2.2 |
| 13.38 | 5.4 | 34.50 | 1.2 |

preferably, the crystal form K has the X-ray powder diffraction intensities shown in Table 11;
preferably, the crystal form K has an X-ray powder diffraction pattern substantially as shown in FIG. 27;
regarding the metastable crystal form L:

an X-ray powder diffraction pattern of the metastable crystal form L comprises peaks at diffraction angles (2θ) of 8.67±0.2°, 15.29±0.2°, 14.75±0.2°, and 11.76±0.2°;
preferably, the crystal form L preferably further comprises peaks at diffraction angles (2θ) of 17.44±0.2°, 9.02±0.2°, 26.63±0.2°, 20.65 ±0.2°, and 7.89±0.2°;
preferably, the crystal form L more preferably further comprises peaks at diffraction angles (2θ) of 17.89±0.2°, 9.53±0.2°, 23.98 ±0.2°, 19.19±0.2°, 21.18±0.2°, and 13.64±0.2°;
preferably, the X-ray powder diffraction pattern of the crystal form L has the diffraction angles (2θ) shown in Table 12, wherein the 2θ angles have a margin of error of ±0.20°:

Table 12

| 2θ (°) | Intensity% | 2θ (°) | Intensity% |
|---|---|---|---|
| 7.89 | 9.7 | 24.37 | 1.1 |
| 8.67 | 100 | 24.93 | 0.5 |
| 9.02 | 16.3 | 25.99 | 0.8 |
| 9.53 | 7.6 | 26.34 | 1 |
| 11.76 | 39.7 | 26.63 | 11 |
| 12.66 | 2 | 27.51 | 1.4 |
| 13.64 | 2.5 | 27.94 | 0.4 |
| 14.75 | 47.2 | 28.25 | 0.3 |
| 15.29 | 53.7 | 29.47 | 0.5 |
| 15.90 | 1.7 | 29.81 | 1.4 |
| 17.44 | 24.6 | 31.08 | 1.3 |
| 17.89 | 7.7 | 31.87 | 0.7 |
| 18.14 | 0.9 | 32.70 | 0.5 |
| 19.19 | 3.7 | 33.20 | 0.7 |
| 19.50 | 0.6 | 34.87 | 1 |
| 20.65 | 9.8 | 35.65 | 0.3 |
| 21.18 | 3.7 | 35.94 | 0.3 |
| 21.74 | 0.5 | 36.31 | 0.4 |
| 22.23 | 1.8 | 36.98 | 0.3 |
| 22.95 | 0.4 | 38.86 | 1.1 |
| 23.69 | 0.9 | 39.49 | 0.5 |
| 23.98 | 6 | | |

preferably, the crystal form L has the X-ray powder diffraction intensities shown in Table 12;
preferably, the crystal form L has an X-ray powder diffraction pattern substantially as shown in FIG. 28;
regarding the metastable crystal form M:

an X-ray powder diffraction pattern of the metastable crystal form M comprises peaks at diffraction angles (2θ) of 8.26±0.2°, 14.79±0.2°, 17.07±0.2°, and 14.57±0.2°;
preferably, the crystal form M preferably further comprises peaks at

diffraction angles (2θ) of 11.50±0.2°, 21.18±0.2°, 16.60±0.2°, 20.53±0.2°, and 25.33±0.2°;
preferably, the crystal form M more preferably further comprises peaks at diffraction angles (2θ) of 18.43±0.2°, 25.01±0.2°, 7.994+0.2°, 8.98±0.2°, 13.04±0.2°, and 24.12±0.2°;
preferably, the X-ray powder diffraction pattern of the crystal form M has the diffraction angles (2θ) shown in Table 13, wherein the 2θ angles have a margin of error of ±0.20°:

Table 13

| 2θ (°) | Intensity% | 2θ (°) | Intensity% |
|---|---|---|---|
| 5.74 | 1.6 | 21.49 | 0.9 |
| 7.99 | 6.3 | 22.12 | 0.7 |
| 8.26 | 100.0 | 24.12 | 4.2 |
| 8.98 | 6.1 | 24.49 | 1.2 |
| 11.50 | 22.5 | 25.01 | 7.8 |
| 12.70 | 1.5 | 25.33 | 11.2 |
| 13.04 | 5.4 | 26.13 | 2.9 |
| 14.57 | 27.7 | 27.66 | 0.6 |
| 14.79 | 65.8 | 28.15 | 0.6 |
| 15.99 | 1.1 | 29.40 | 1.1 |
| 16.60 | 13.7 | 29.81 | 0.5 |
| 17.07 | 51.5 | 30.39 | 1 |
| 17.79 | 0.8 | 32.23 | 1.3 |
| 18.02 | 1.2 | 33.01 | 0.4 |
| 18.43 | 8.4 | 33.55 | 2.5 |
| 19.38 | 0.5 | 34.70 | 0.3 |
| 20.32 | 2.6 | 35.77 | 1 |
| 20.53 | 11.2 | 37.43 | 0.4 |
| 21.18 | 17.2 | 38.28 | 1.3 |

preferably, the crystal form M has the X-ray powder diffraction intensities shown in Table 13;
preferably, the crystal form M has an X-ray powder diffraction pattern substantially as shown in FIG. 29;
regarding the metastable crystal form N:

an X-ray powder diffraction pattern of the metastable crystal form N comprises peaks at diffraction angles (2θ) of 4.48±0.2°, 5.02±0.2°, 5.55±0.2°, and 11.61±0.2°;
preferably, the crystal form N preferably further comprises peaks at diffraction angles (2θ) of 3.14±0.2°, 14.70±0.2°, 11.42±0.2°, 7.31±0.2°, and 13.69±0.2°;
preferably, the crystal form N more preferably further comprises peaks at diffraction angles (2θ) of 13.34±0.2°, 9.57±0.2°, 15.71 ±0.2°, 15.10±0.2°, 12.43±0.2°, and 22.05±0.2°;
preferably, the X-ray powder diffraction pattern of the crystal form N has the diffraction angles (2θ) shown in Table 14, wherein the 2θ angles have a margin of error of ±0.20°:

Table 14

| 2θ (°) | Intensity% | 2θ (°) | Intensity% |
|---|---|---|---|
| 3.14 | 7.3 | 13.34 | 4.5 |
| 4.48 | 100 | 13.69 | 4.7 |
| 5.02 | 22.3 | 14.70 | 6.3 |
| 5.55 | 19.6 | 15.10 | 3.9 |
| 7.31 | 5.2 | 15.71 | 4.2 |
| 8.24 | 3.1 | 17.16 | 2.2 |
| 9.57 | 4.2 | 18.13 | 2.2 |
| 10.78 | 2 | 20.29 | 3 |
| 11.42 | 6.1 | 20.29 | 3 |
| 11.61 | 8 | 22.05 | 3.3 |
| 12.43 | 3.7 | | |

preferably, the crystal form N has the X-ray powder diffraction intensities shown in Table 14;
preferably, the crystal form N has an X-ray powder diffraction pattern substantially as shown in FIG. 30;
regarding the metastable crystal form O:

an X-ray powder diffraction pattern of the metastable crystal form O comprises peaks at diffraction angles (2θ) of 4.54 ±0.2°, 3.37±0.2°, 5.96±0.2°, and 12.21±0.2°;
preferably, the crystal form O preferably further comprises peaks at diffraction angles (2θ) of 7.87±0.2°, 13.89±0.2°, 16.84±0.2°, 21.24±0.2°, and 32.21±0.2°;
preferably, the X-ray powder diffraction pattern of the crystal form O has the diffraction angles (2θ) shown in Table 14, wherein the 2θ angles have a margin of error of ±0.20°:

Table 15

| 2θ (°) | Intensity% | 2θ (°) | Intensity% |
|---|---|---|---|
| 3.37 | 15.4 | 13.89 | 2.9 |
| 4.54 | 100 | 16.84 | 2.7 |
| 5.96 | 14.4 | 21.24 | 2.4 |
| 7.87 | 4.4 | 32.21 | 1.3 |
| 12.21 | 5.4 | | |

preferably, the crystal form O has the X-ray powder diffraction intensities shown in Table 15;
preferably, the crystal form O has an X-ray powder diffraction pattern substantially as shown in FIG. 31;
regarding the metastable crystal form P:

an X-ray powder diffraction pattern of the metastable crystal form P comprises peaks at diffraction angles (2θ) of 4.81±0.2°, 4.29±0.2°, 6.08±0.2°, and 14.65 ±0.2°;
preferably, the crystal form P preferably further comprises peaks at diffraction angles (2θ) of 12.33±0.2°, 7.89±0.2°, 3.41±0.2°, 17.94±0.2°, and 8.81±0.2°;
preferably, the crystal form P more preferably further comprises peaks at diffraction angles (2θ) of 17.57 ±0.2°, 7.25±0.2°, 13.37±0.2°, 19.60±0.2°, and 20.23±0.2°;
preferably, the X-ray powder diffraction pattern of the crystal form P has the diffraction angles (2θ) shown in Table 14, wherein the 2θ angles have a margin of error of ±0.20°:

Table 16

| 2θ (°) | Intensity% | 2θ (°) | Intensity% |
|---|---|---|---|
| 3.41 | 4.8 | 12.33 | 6.5 |
| 4.29 | 71.9 | 13.37 | 2.2 |
| 4.81 | 100 | 14.65 | 8.3 |
| 6.08 | 15.9 | 17.57 | 3.1 |
| 7.25 | 2.2 | 17.94 | 3.6 |
| 7.89 | 5.4 | 19.60 | 2.2 |
| 8.81 | 3.3 | 20.23 | 2.1 |

preferably, the crystal form P has the X-ray powder diffraction intensities shown in Table 16;
preferably, the crystal form P has an X-ray powder diffraction pattern substantially as shown in FIG. 32;
regarding the metastable crystal form Q:

an X-ray powder diffraction pattern of the metastable crystal form Q comprises peaks at diffraction angles (2θ) of 4.96±0.2°, 7.42±0.2°, 14.82±0.2°, and 21.77±0.2°;
preferably, the crystal form Q preferably further comprises peaks at diffraction angles (2θ) of 16.75±0.2°, 17.29±0.2°, 15.21±0.2°, 25.67 ±0.2°, and 24.53±0.2°;
preferably, the crystal form Q more preferably further comprises peaks at diffraction angles (2θ) of 9.86±0.2°, 15.76±0.2°, 17.56±0.2°, 22.92 ±0.2°, 23.67±0.2°, and 22.44±0.2°;
preferably, the X-ray powder diffraction pattern of the crystal form Q has the diffraction angles (2θ) shown in Table 14, wherein the 2θ angles have a margin of error of ±0.20°:

Table 17

| 2θ (°) | Intensity% | 2θ (°) | Intensity% |
|---|---|---|---|
| 4.96 | 100 | 20.21 | 0.6 |
| 7.42 | 9.9 | 20.41 | 0.5 |
| 9.86 | 1.4 | 21.77 | 4.4 |
| 12.34 | 0.6 | 22.44 | 0.9 |
| 13.00 | 0.1 | 22.92 | 1 |

(continued)

| 2θ (°) | Intensity% | 2θ (°) | Intensity% |
|---|---|---|---|
| 14.82 | 4.6 | 23.67 | 1 |
| 15.21 | 1.8 | 24.22 | 0.4 |
| 15.76 | 1.3 | 24.53 | 1.5 |
| 15.96 | 0.3 | 24.78 | 0.4 |
| 16.75 | 1.9 | 25.67 | 1.8 |
| 17.29 | 1.9 | 26.81 | 0.2 |
| 17.56 | 1 | 27.29 | 0.1 |
| 18.43 | 0.6 | 27.57 | 0.2 |
| 19.87 | 0.2 | 29.05 | 0.7 |

preferably, the crystal form Q has the X-ray powder diffraction intensities shown in Table 17;
preferably, the crystal form Q has an X-ray powder diffraction pattern substantially as shown in FIG. 33.

**9.** A preparation method for the polymorph as claimed in claim 8, comprising the following steps:

step 1: dissolving or dispersing compound IE in a solvent; and
step 2: stirring at 0-50 °C for crystallization; or adding an anti-solvent to a clear solution of the compound for precipitation; or slowly volatilizing a clear solution of the compound;
wherein preferably, the compound IE is preferably the metastable crystal form H of compound IE;
preferably, the solvent is water, an organic solvent, or a mixed solvent of water with the organic solvent, wherein the organic solvent is selected from alcohols, chloroalkanes, ketones, ethers, cyclic ethers, esters, alkanes, cycloalkanes, benzenes, amides, sulfoxides, and mixtures thereof; preferably, the organic solvent is selected from methanol, ethanol, *n*-propanol, isopropanol, *n*-butanol, trifluoroethanol, acetonitrile, acetone, methyl ethyl ketone, methyl isobutyl ketone, 1,4-dioxane, tetrahydrofuran, 2-methyltetrahydrofuran, *N,N*-dimethylformamide, *N,N*-dimethylacetamide, *N*-methylpyrrolidone, dimethyl sulfoxide, ethyl acetate, isopropyl acetate, dichloromethane, trichloromethane, trichloroethane, carbon tetrachloride, methyl *tert*-butyl ether, cyclopentyl methyl ether, 2-methoxyethyl ether, isopropyl ether, diethyl ether, *n*-heptane, *n*-hexane, isooctane, pentane, cyclohexane, cyclopentane, methylcyclohexane, benzene, toluene, xylene, and mixtures thereof.

**10.** Use of the compound, the pharmaceutically acceptable salt, the solvate, the enantiomer, or the isotopically substituted compound thereof as claimed in any one of claims 1-7 or the polymorph as claimed in claim 8 for the manufacturing of a medicament for the prevention and/or treatment of diseases associated with the WEE1 and/or Yes target(s), wherein: preferably, the diseases include tumors, inflammations, and autoimmune diseases (e.g., lupus erythematosus and psoriasis).

**11.** A pharmaceutical composition, comprising the compound, the pharmaceutically acceptable salt, the solvate, the enantiomer, the isotopically substituted compound, or the polymorph thereof as claimed in any one of claims 1-7 or the polymorph as claimed in claim 8 as an active ingredient and an auxiliary ingredient that enables the pharmaceutical composition to be suitable for being formulated as different pharmaceutical dosage forms such as liquid formulations (including oral liquids, injectable liquids, eye drops, etc.), solid formulations (including tablets, capsules, pills, granules, etc.), wherein the pharmaceutical composition is capable of being used for the manufacturing of a medicament for the prevention and/or treatment of diseases associated with the WEE1 and/or Yes target(s);
preferably, the diseases include tumors, inflammations, and autoimmune diseases (e.g., lupus erythematosus and psoriasis).

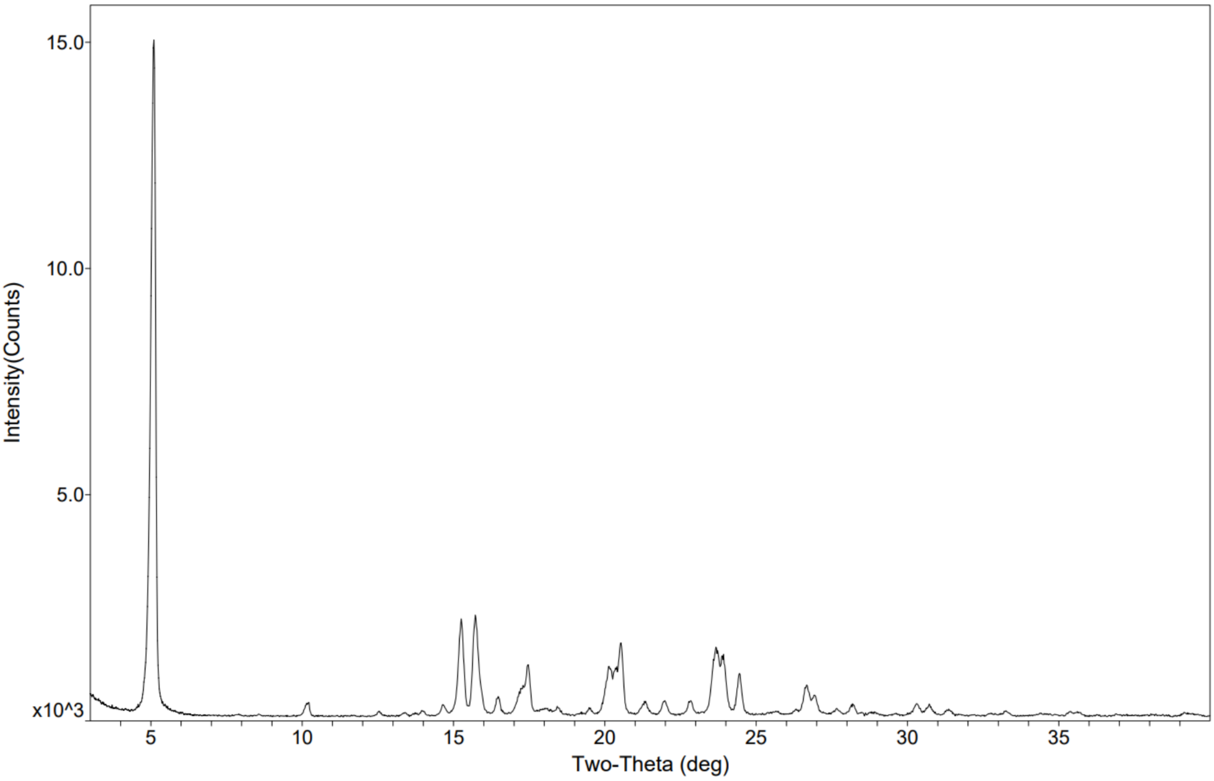
15.0
10.0
5.0
x10^3
Intensity(Counts)
5
10
15
20
25
30
35
Two-Theta (deg)

FIG. 1

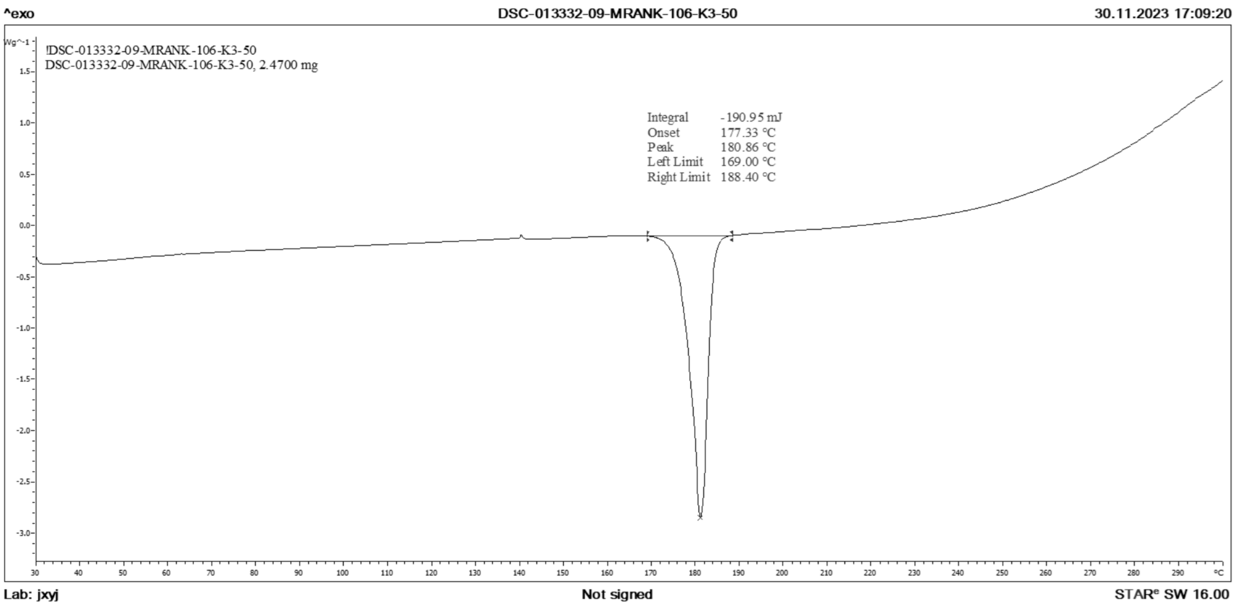
^exo
DSC-013332-09-MRANK-106-K3-50
30.11.2023 17:09:20
!DSC-013332-09-MRANK-106-K3-50
DSC-013332-09-MRANK-106-K3-50, 2.4700 mg
Integral -190.95 mJ
Onset 177.33 °C
Peak 180.86 °C
Left Limit 169.00 °C
Right Limit 188.40 °C
Lab: jxyj
Not signed
STARe SW 16.00

FIG. 2

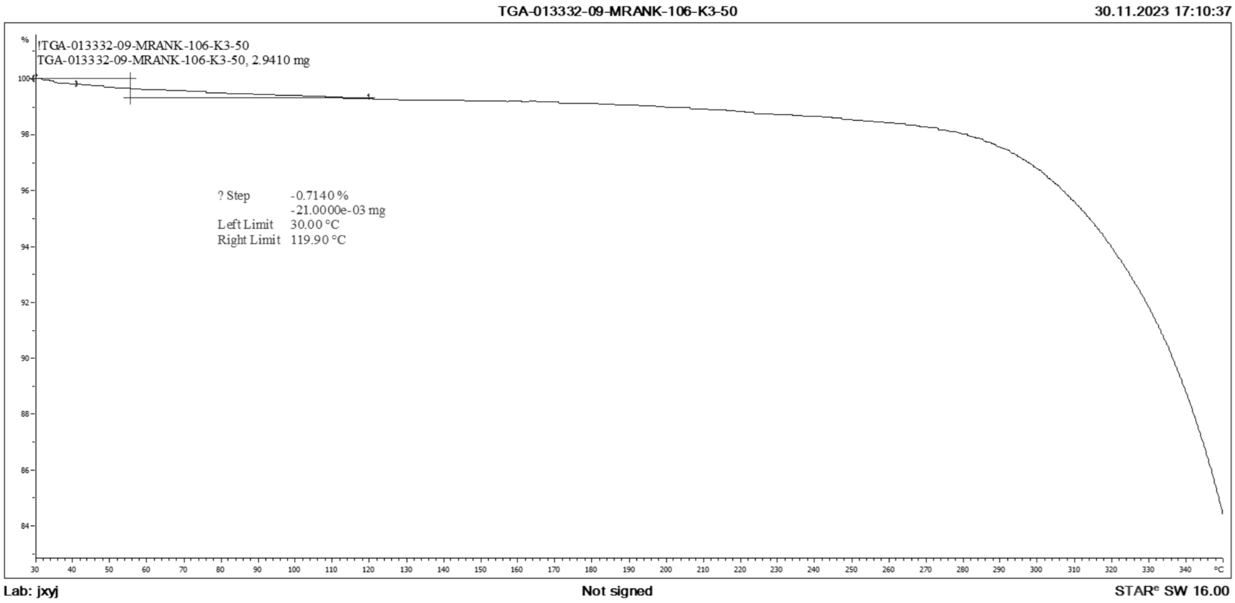
TGA-013332-09-MRANK-106-K3-50
30.11.2023 17:10:37
!TGA-013332-09-MRANK-106-K3-50
TGA-013332-09-MRANK-106-K3-50, 2.9410 mg
? Step -0.7140 %
-21.0000e-03 mg
Left Limit 30.00 °C
Right Limit 119.90 °C
Lab: jxyj
Not signed
STAR® SW 16.00

FIG. 3

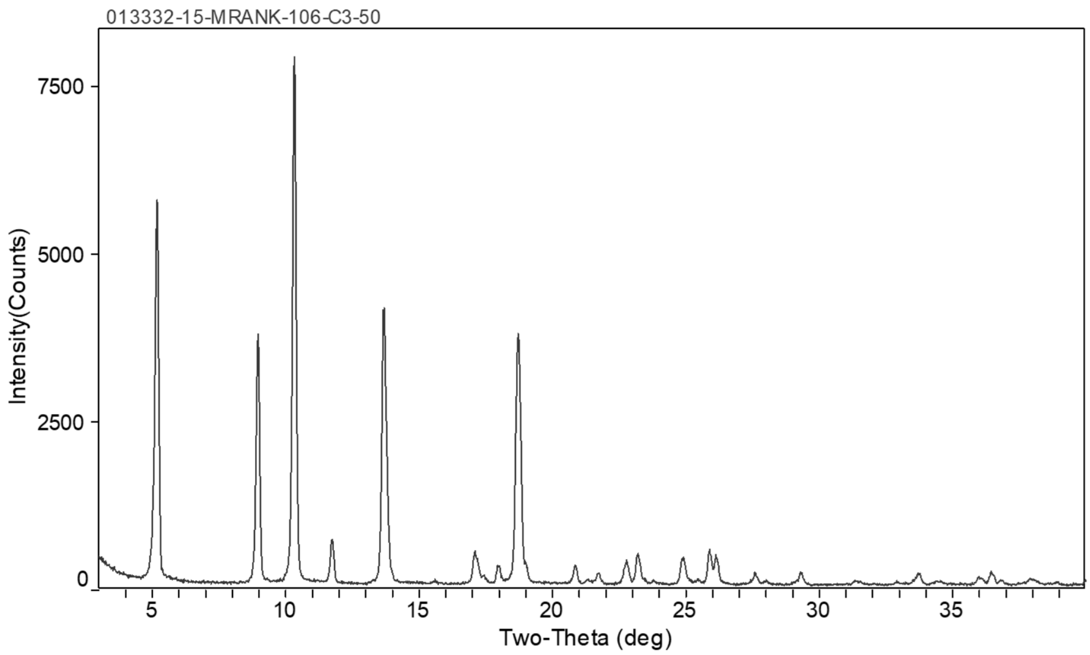
013332-15-MRANK-106-C3-50
Intensity(Counts)
Two-Theta (deg)

FIG. 4

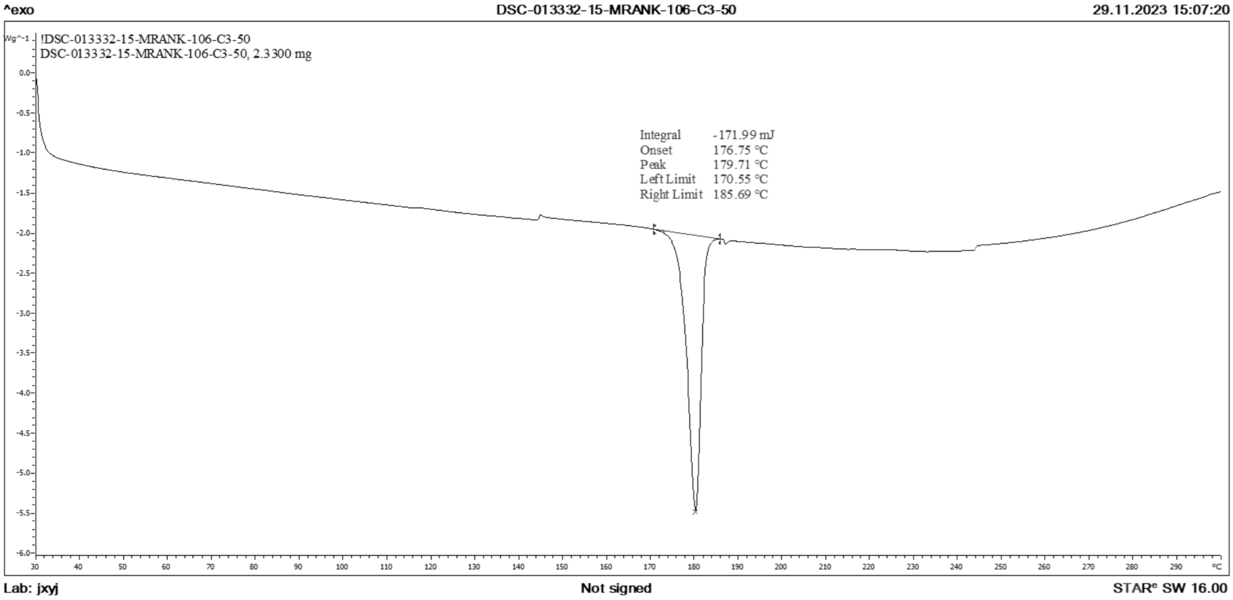
^exo
DSC-013332-15-MRANK-106-C3-50
29.11.2023 15:07:20
!DSC-013332-15-MRANK-106-C3-50
DSC-013332-15-MRANK-106-C3-50, 2.3300 mg
Integral -171.99 mJ
Onset 176.75 °C
Peak 179.71 °C
Left Limit 170.55 °C
Right Limit 185.69 °C
Lab: jxyj
Not signed
STARe SW 16.00

FIG. 5

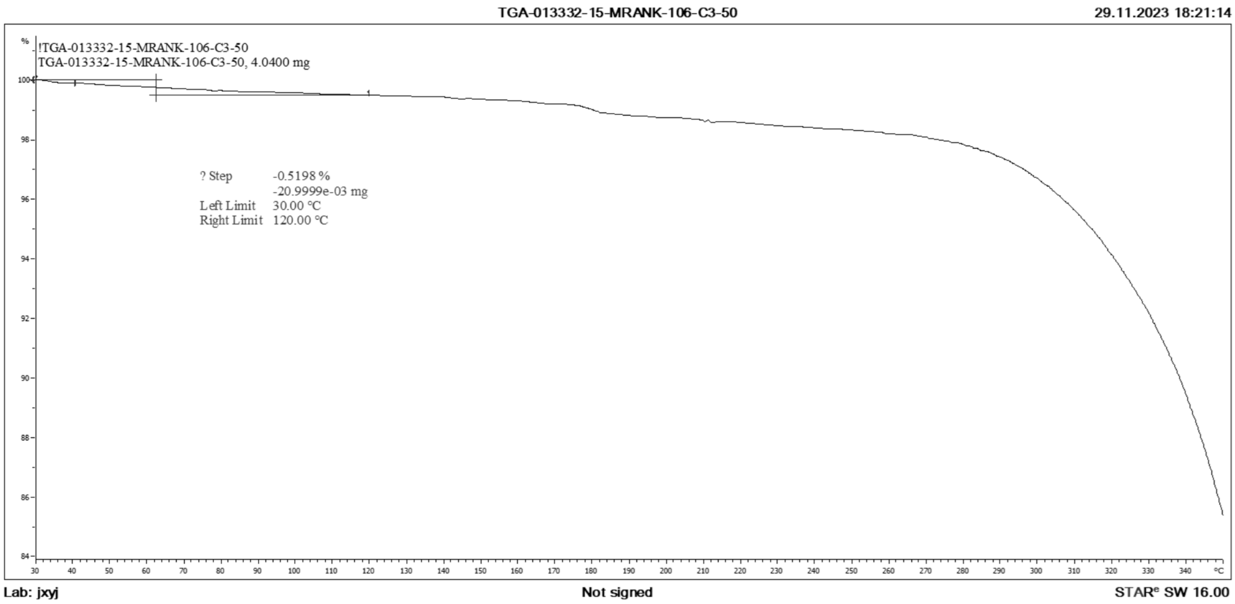
TGA-013332-15-MRANK-106-C3-50
29.11.2023 18:21:14
!TGA-013332-15-MRANK-106-C3-50
TGA-013332-15-MRANK-106-C3-50, 4.0400 mg
? Step -0.5198 %
-20.9999e-03 mg
Left Limit 30.00 °C
Right Limit 120.00 °C
Lab: jxyj
Not signed
STARe SW 16.00

FIG. 6

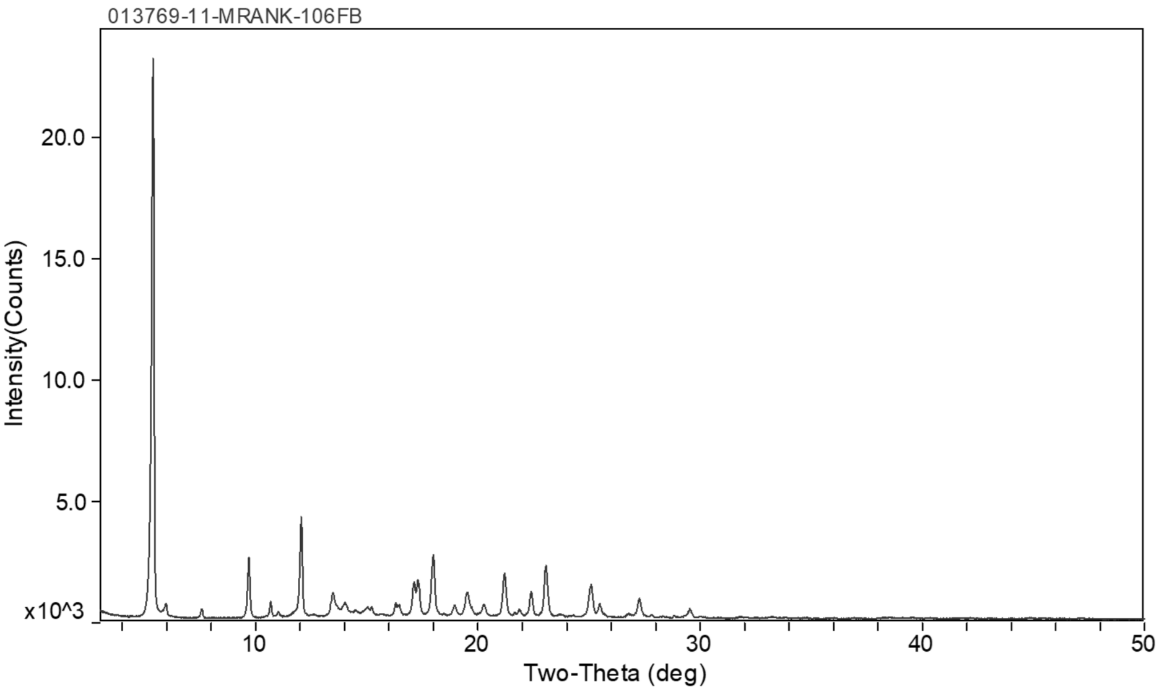
013769-11-MRANK-106FB
Intensity(Counts)
20.0
15.0
10.0
5.0
x10^3
10
20
30
40
50
Two-Theta (deg)

FIG. 7

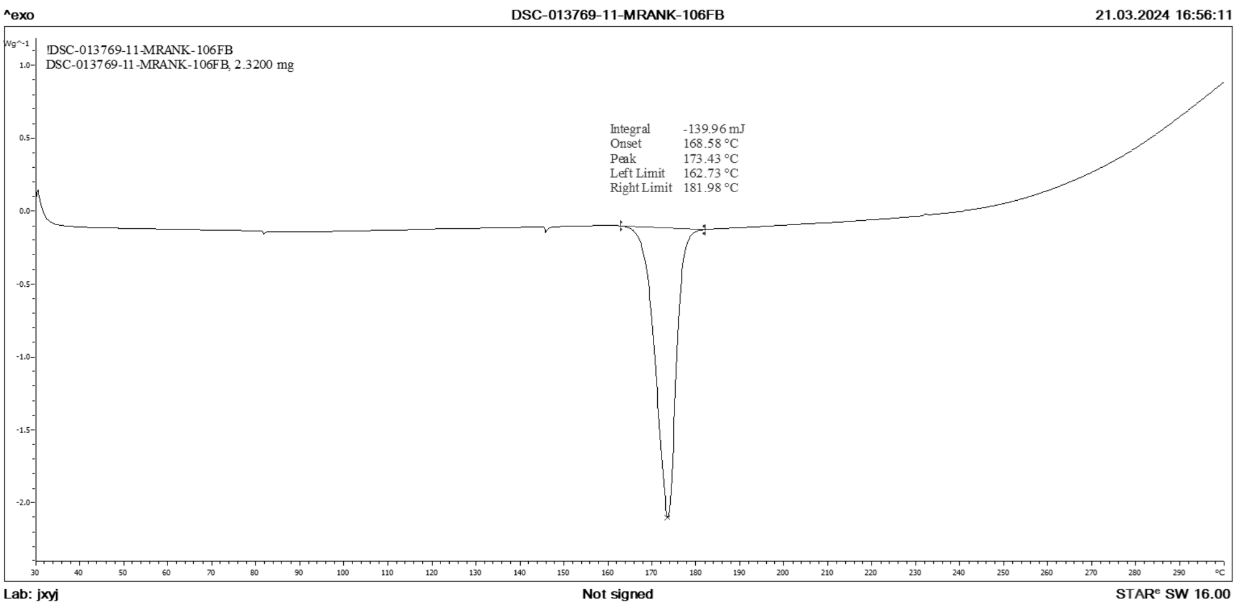
^exo
DSC-013769-11-MRANK-106FB
21.03.2024 16:56:11
!DSC-013769-11-MRANK-106FB
DSC-013769-11-MRANK-106FB, 2.3200 mg
Integral -139.96 mJ
Onset 168.58 °C
Peak 173.43 °C
Left Limit 162.73 °C
Right Limit 181.98 °C
Lab: jxyj
Not signed
STAR® SW 16.00

FIG. 8

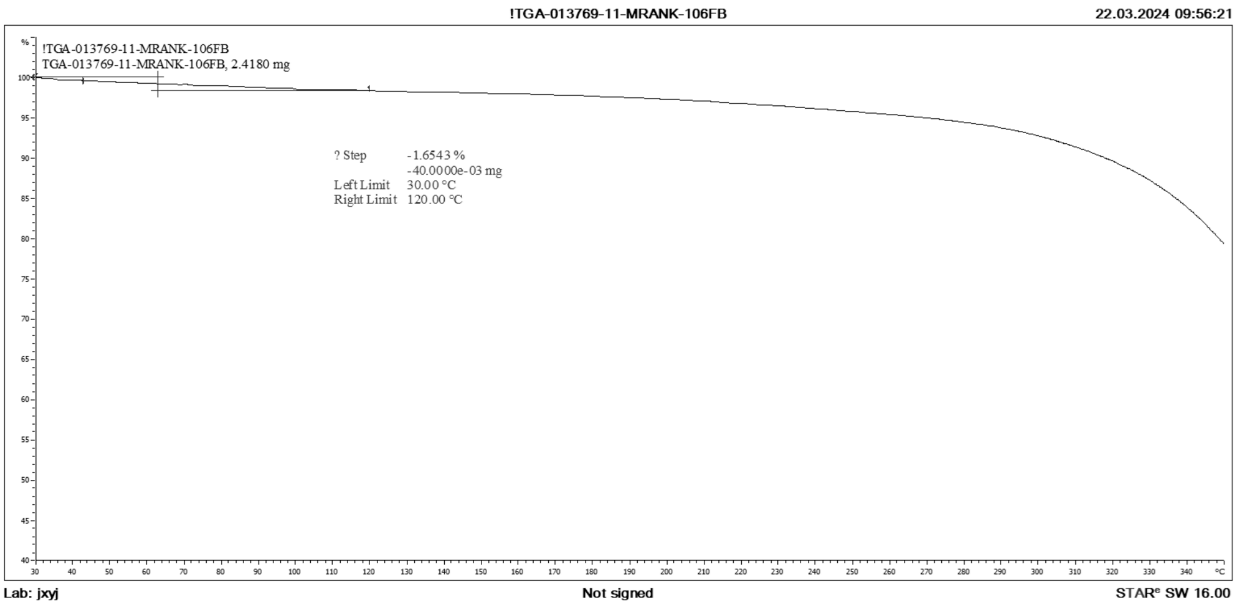
!TGA-013769-11-MRANK-106FB
22.03.2024 09:56:21
!TGA-013769-11-MRANK-106FB
TGA-013769-11-MRANK-106FB, 2.4180 mg
? Step -1.6543 %
-40.0000e-03 mg
Left Limit 30.00 °C
Right Limit 120.00 °C
Lab: jxyj
Not signed
STARe SW 16.00

FIG. 9

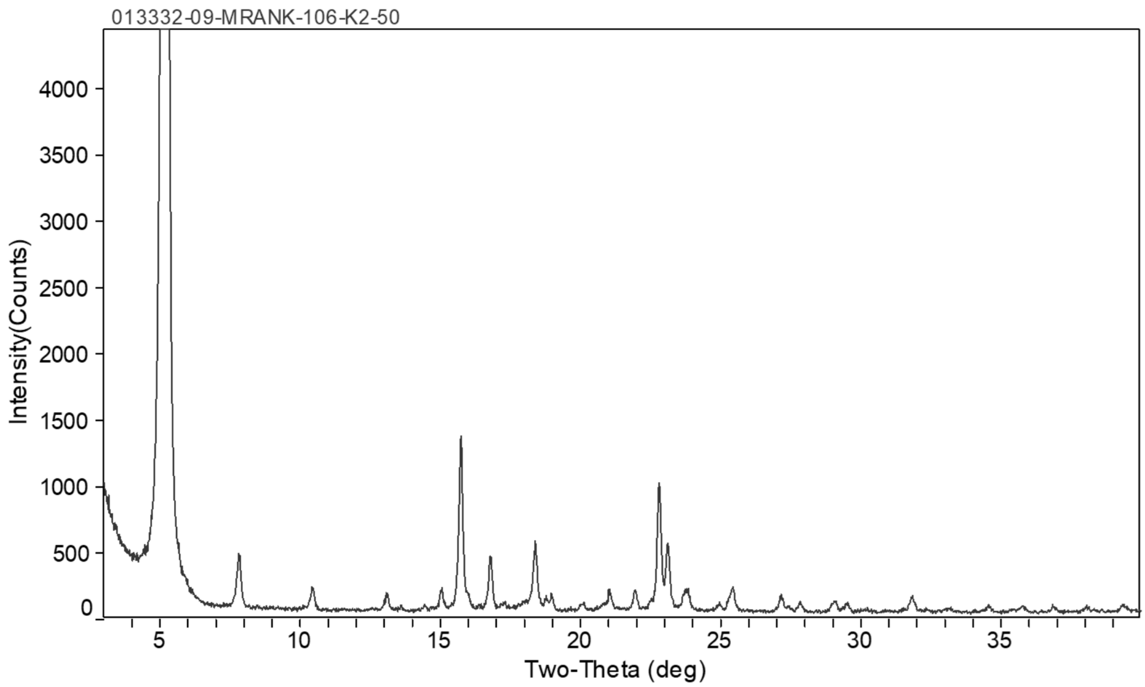
013332-09-MRANK-106-K2-50
Intensity(Counts)
Two-Theta (deg)

FIG. 10

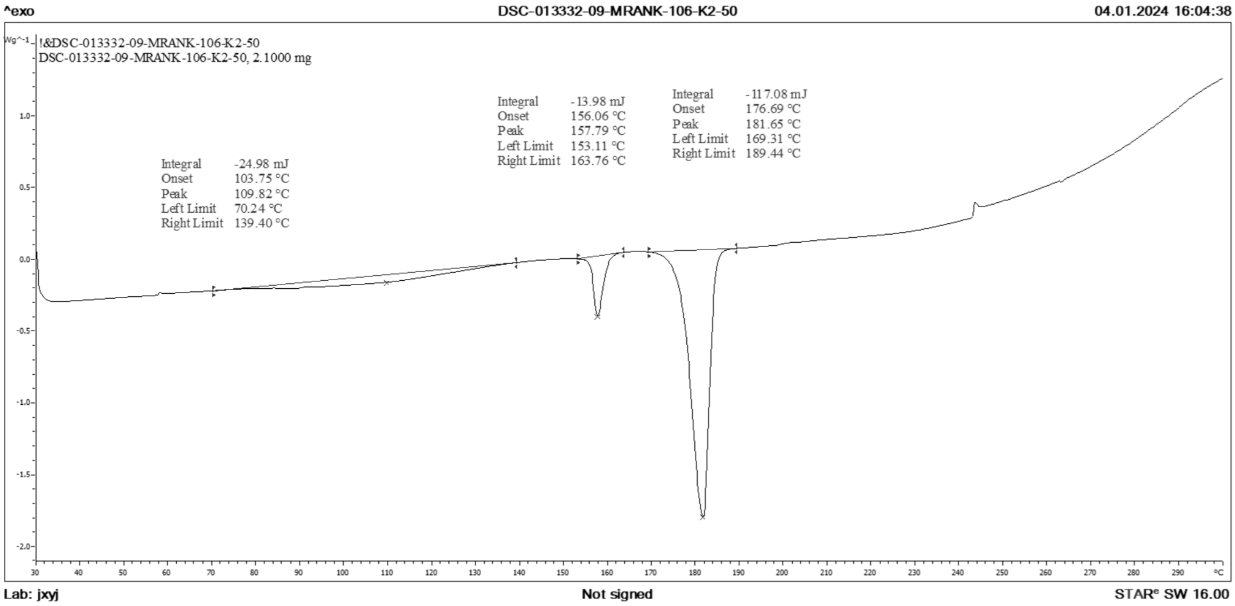
^exo
DSC-013332-09-MRANK-106-K2-50
04.01.2024 16:04:38
!&DSC-013332-09-MRANK-106-K2-50
DSC-013332-09-MRANK-106-K2-50, 2.1000 mg
Integral -24.98 mJ
Onset 103.75 °C
Peak 109.82 °C
Left Limit 70.24 °C
Right Limit 139.40 °C
Integral -13.98 mJ
Onset 156.06 °C
Peak 157.79 °C
Left Limit 153.11 °C
Right Limit 163.76 °C
Integral -117.08 mJ
Onset 176.69 °C
Peak 181.65 °C
Left Limit 169.31 °C
Right Limit 189.44 °C
Lab: jxyj
Not signed
STARe SW 16.00

FIG. 11

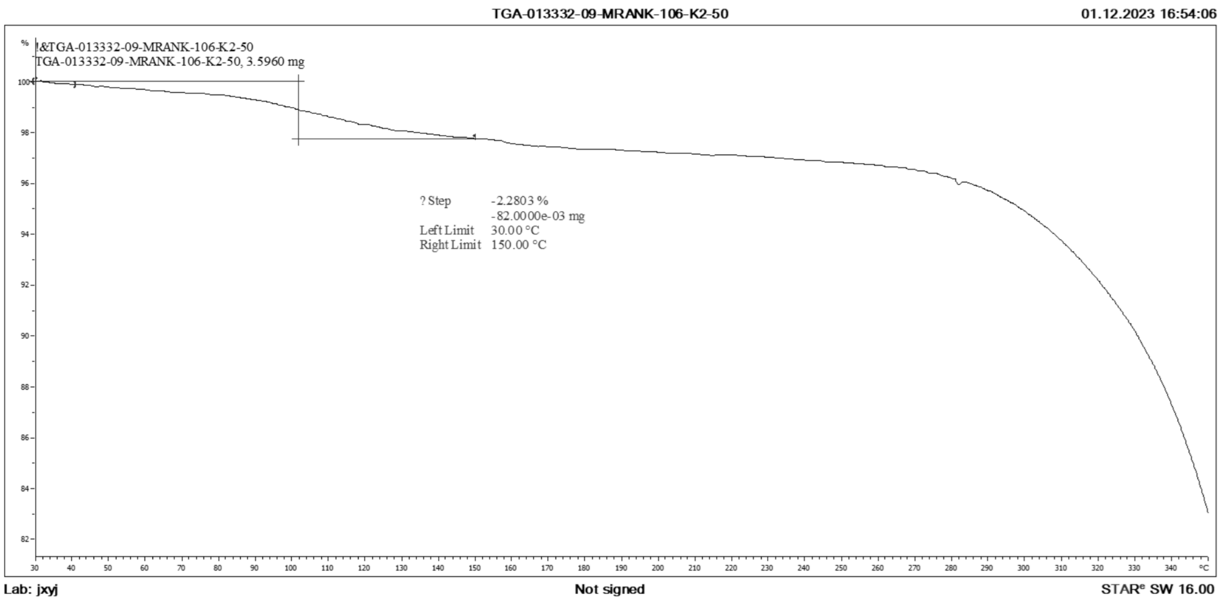
TGA-013332-09-MRANK-106-K2-50
01.12.2023 16:54:06
!&TGA-013332-09-MRANK-106-K2-50
TGA-013332-09-MRANK-106-K2-50, 3.5960 mg
? Step -2.2803 %
-82.0000e-03 mg
Left Limit 30.00 °C
Right Limit 150.00 °C
Lab: jxyj
Not signed
STARe SW 16.00

FIG. 12

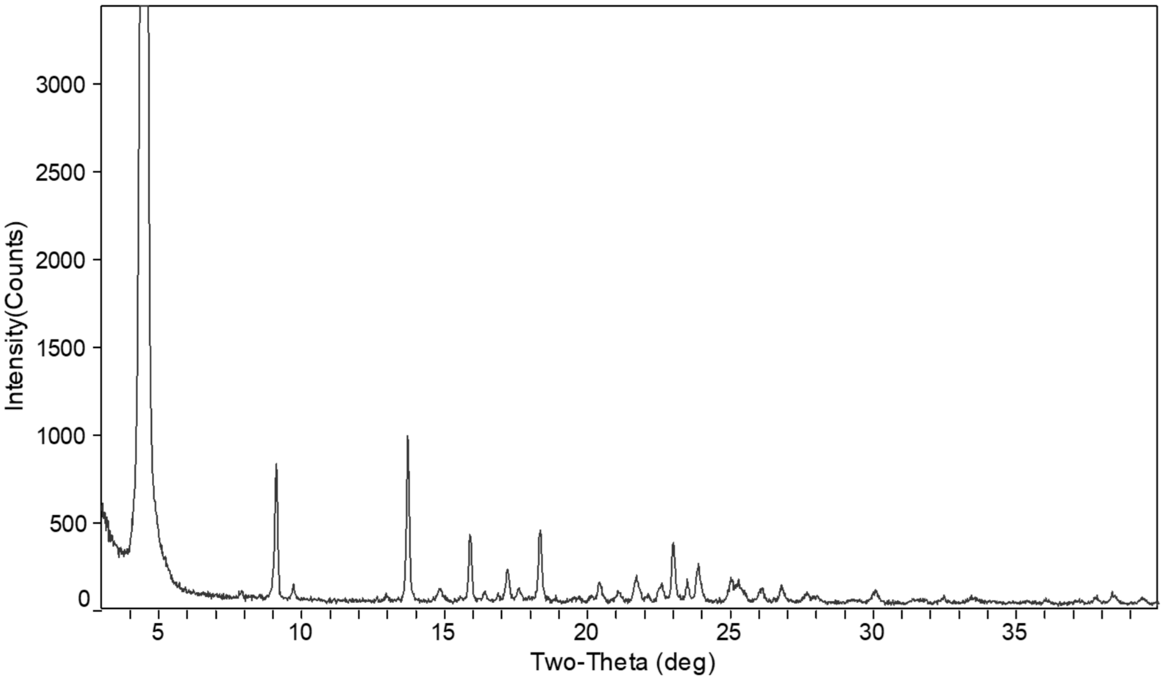
3000
2500
2000
1500
1000
500
0
Intensity(Counts)
5
10
15
20
25
30
35
Two-Theta (deg)

FIG. 13

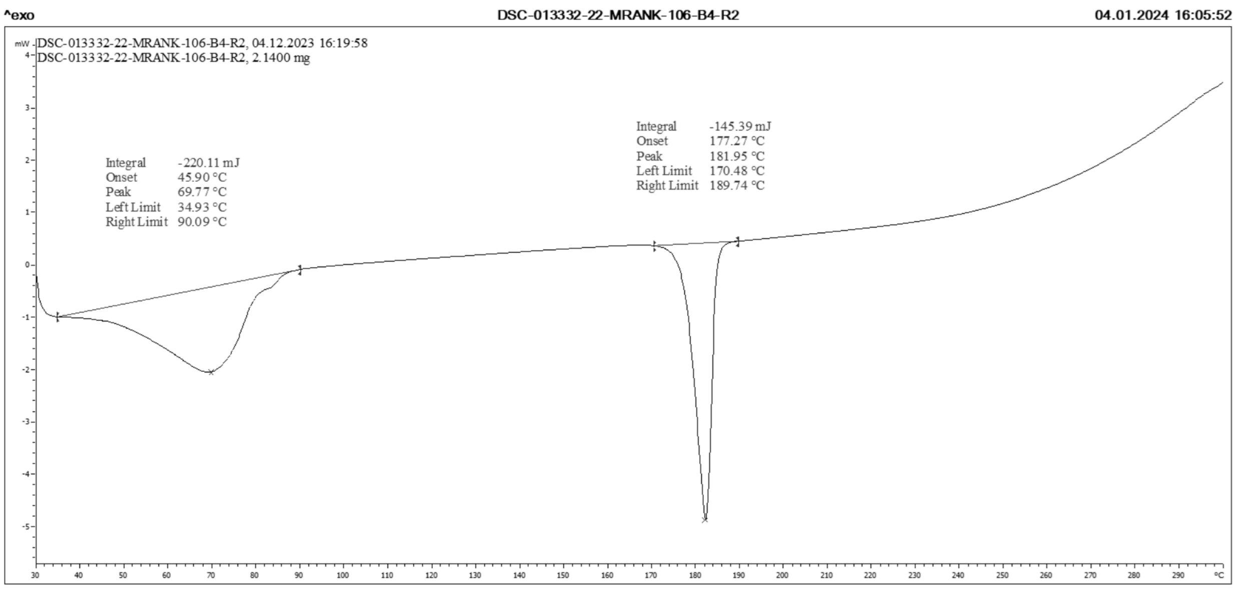
^exo
DSC-013332-22-MRANK-106-B4-R2
04.01.2024 16:05:52
DSC-013332-22-MRANK-106-B4-R2, 04.12.2023 16:19:58
DSC-013332-22-MRANK-106-B4-R2, 2.1400 mg
Integral -220.11 mJ
Onset 45.90 °C
Peak 69.77 °C
Left Limit 34.93 °C
Right Limit 90.09 °C
Integral -145.39 mJ
Onset 177.27 °C
Peak 181.95 °C
Left Limit 170.48 °C
Right Limit 189.74 °C
Lab: jxyj
Not signed
STARe SW 16.00

FIG. 14

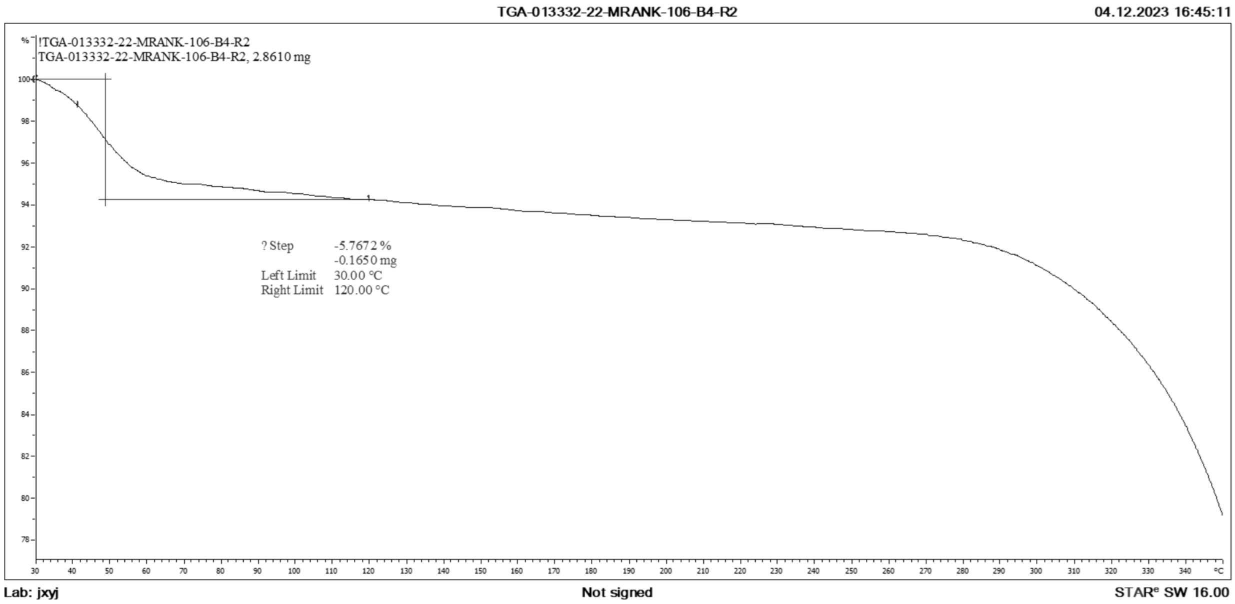
TGA-013332-22-MRANK-106-B4-R2
04.12.2023 16:45:11
!TGA-013332-22-MRANK-106-B4-R2
TGA-013332-22-MRANK-106-B4-R2, 2.8610 mg
? Step -5.7672 %
-0.1650 mg
Left Limit 30.00 °C
Right Limit 120.00 °C
Lab: jxyj
Not signed
STARe SW 16.00

FIG. 15

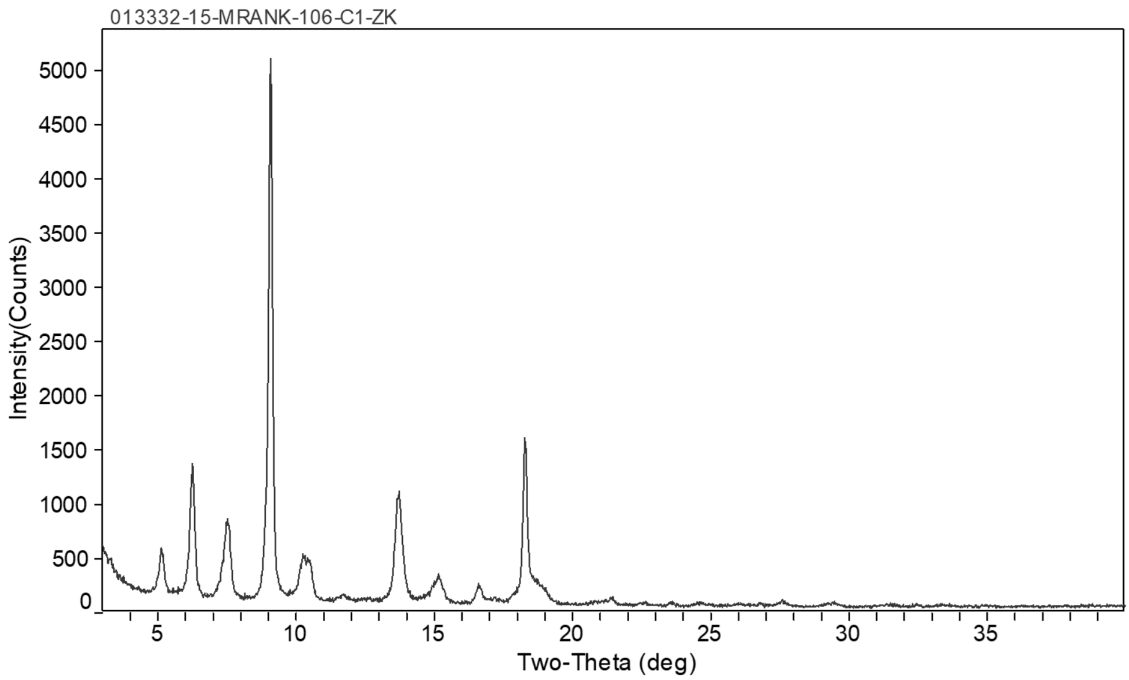
013332-15-MRANK-106-C1-ZK
Intensity(Counts)
Two-Theta (deg)

FIG. 16

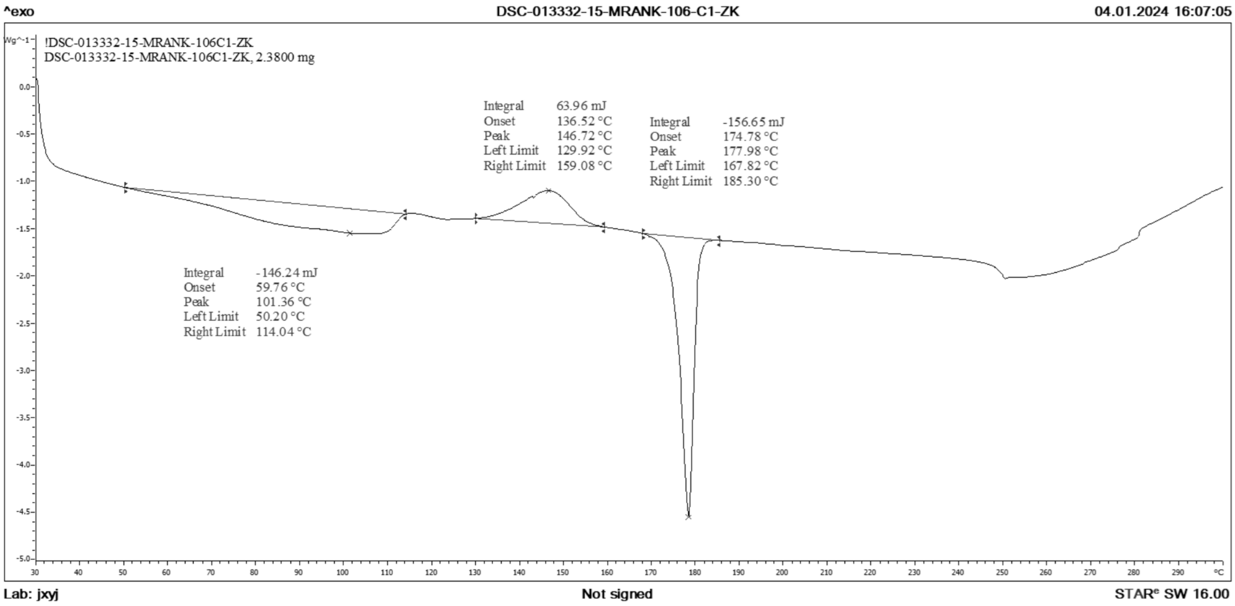
^exo
DSC-013332-15-MRANK-106-C1-ZK
04.01.2024 16:07:05
!DSC-013332-15-MRANK-106C1-ZK
DSC-013332-15-MRANK-106C1-ZK, 2.3800 mg
Integral 63.96 mJ
Onset 136.52 °C
Peak 146.72 °C
Left Limit 129.92 °C
Right Limit 159.08 °C
Integral -156.65 mJ
Onset 174.78 °C
Peak 177.98 °C
Left Limit 167.82 °C
Right Limit 185.30 °C
Integral -146.24 mJ
Onset 59.76 °C
Peak 101.36 °C
Left Limit 50.20 °C
Right Limit 114.04 °C
Lab: jxyj
Not signed
STARe SW 16.00

FIG. 17

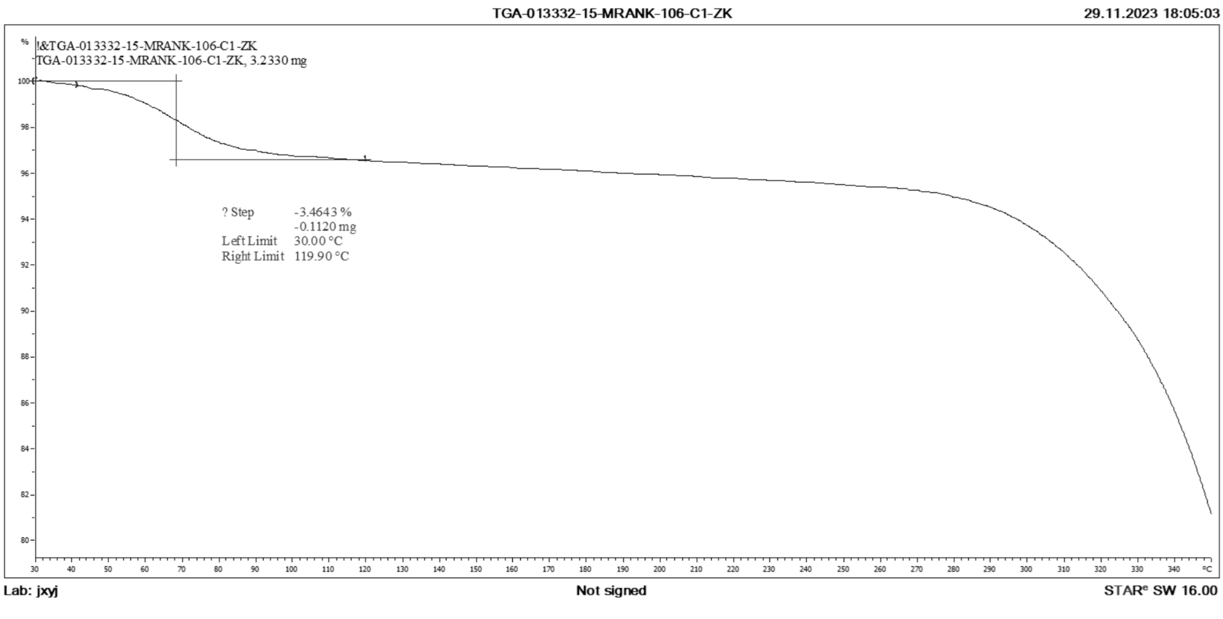
TGA-013332-15-MRANK-106-C1-ZK
29.11.2023 18:05:03
!&TGA-013332-15-MRANK-106-C1-ZK
TGA-013332-15-MRANK-106-C1-ZK, 3.2330 mg
? Step -3.4643 %
-0.1120 mg
Left Limit 30.00 °C
Right Limit 119.90 °C
Lab: jxyj
Not signed
STARe SW 16.00

FIG. 18

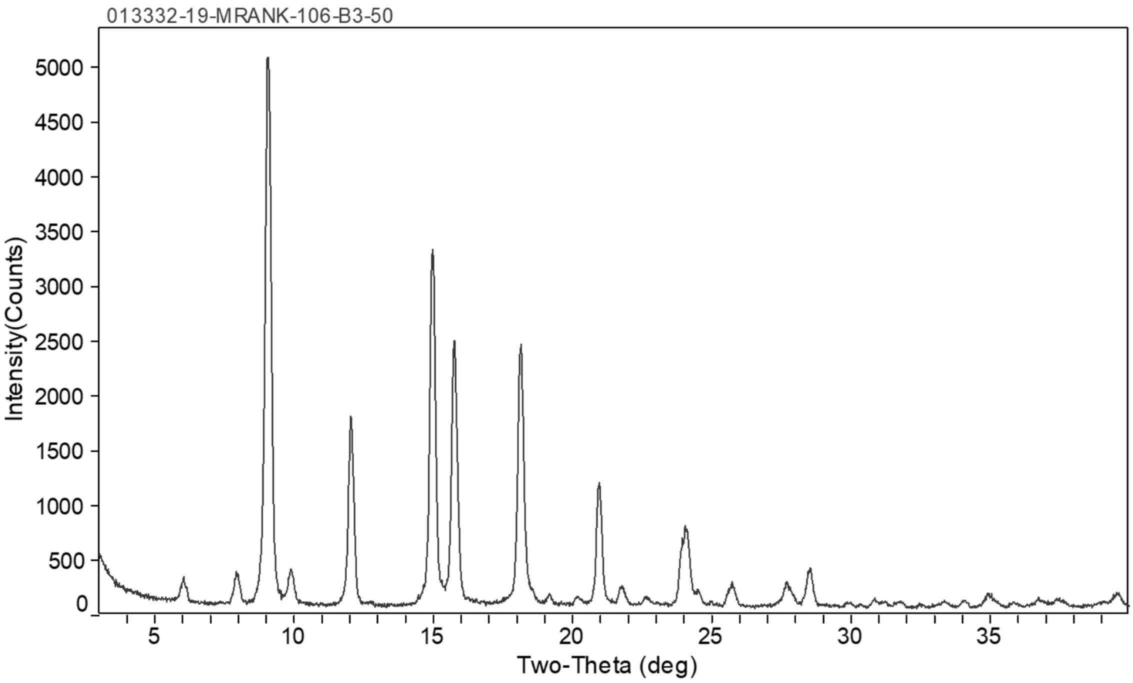
013332-19-MRANK-106-B3-50
Intensity(Counts)
0
500
1000
1500
2000
2500
3000
3500
4000
4500
5000
5
10
15
20
25
30
35
Two-Theta (deg)

FIG. 19

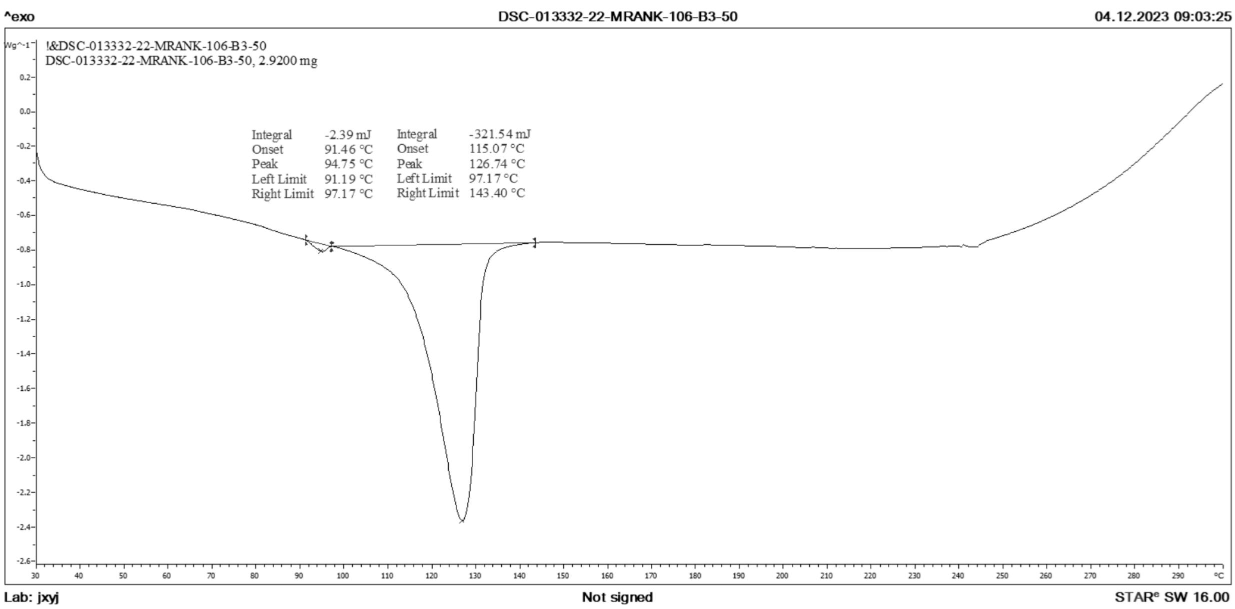
^exo
DSC-013332-22-MRANK-106-B3-50
04.12.2023 09:03:25
Wg^-1
!&DSC-013332-22-MRANK-106-B3-50
DSC-013332-22-MRANK-106-B3-50, 2.9200 mg
Integral -2.39 mJ
Onset 91.46 °C
Peak 94.75 °C
Left Limit 91.19 °C
Right Limit 97.17 °C
Integral -321.54 mJ
Onset 115.07 °C
Peak 126.74 °C
Left Limit 97.17 °C
Right Limit 143.40 °C
°C
Lab: jxyj
Not signed
STARe SW 16.00

FIG. 20

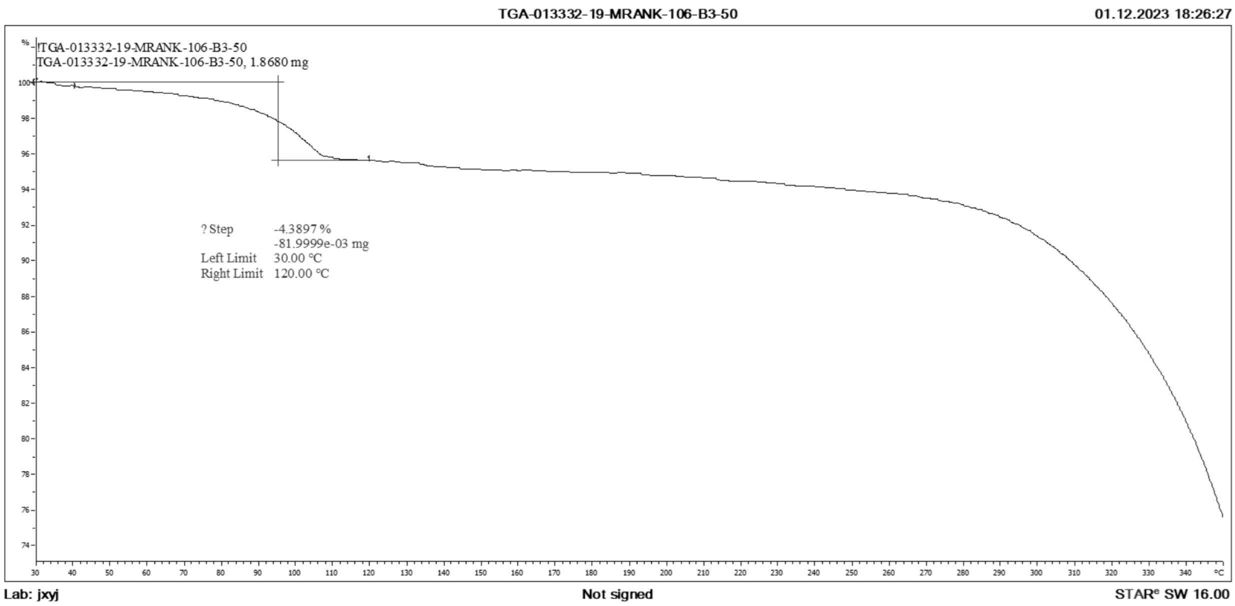
TGA-013332-19-MRANK-106-B3-50
01.12.2023 18:26:27
TGA-013332-19-MRANK-106-B3-50
TGA-013332-19-MRANK-106-B3-50, 1.8680 mg
? Step -4.3897 %
-81.9999e-03 mg
Left Limit 30.00 °C
Right Limit 120.00 °C
Lab: jxyj
Not signed
STARe SW 16.00

FIG. 21

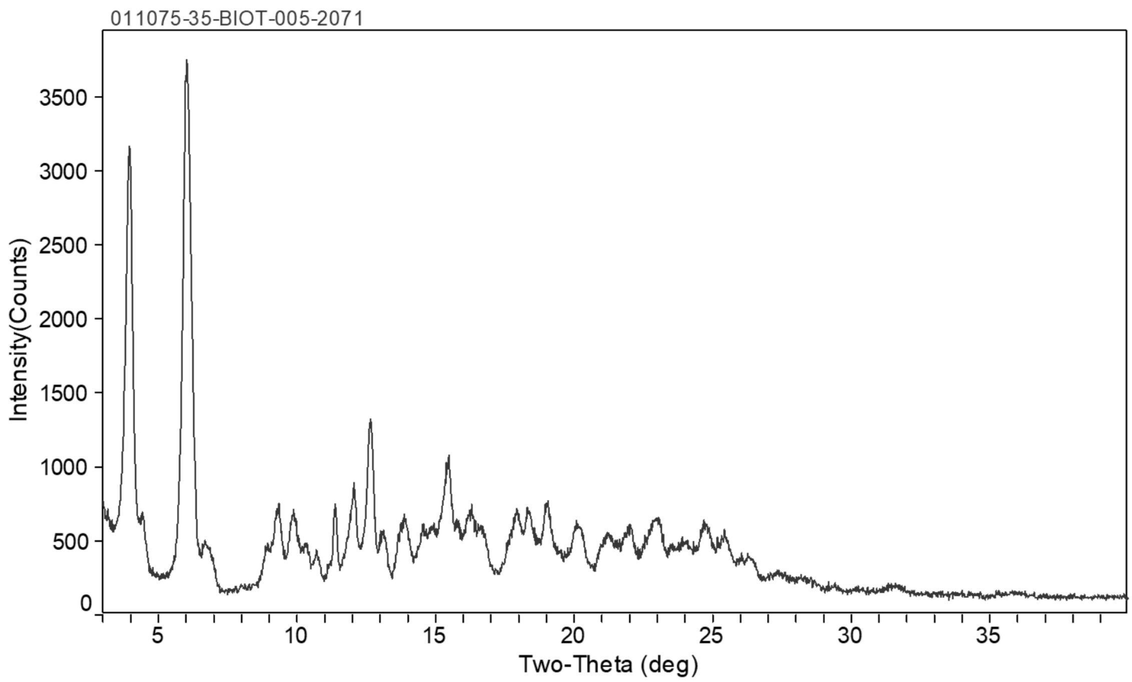
011075-35-BIOT-005-2071
Intensity(Counts)
Two-Theta (deg)

FIG. 22

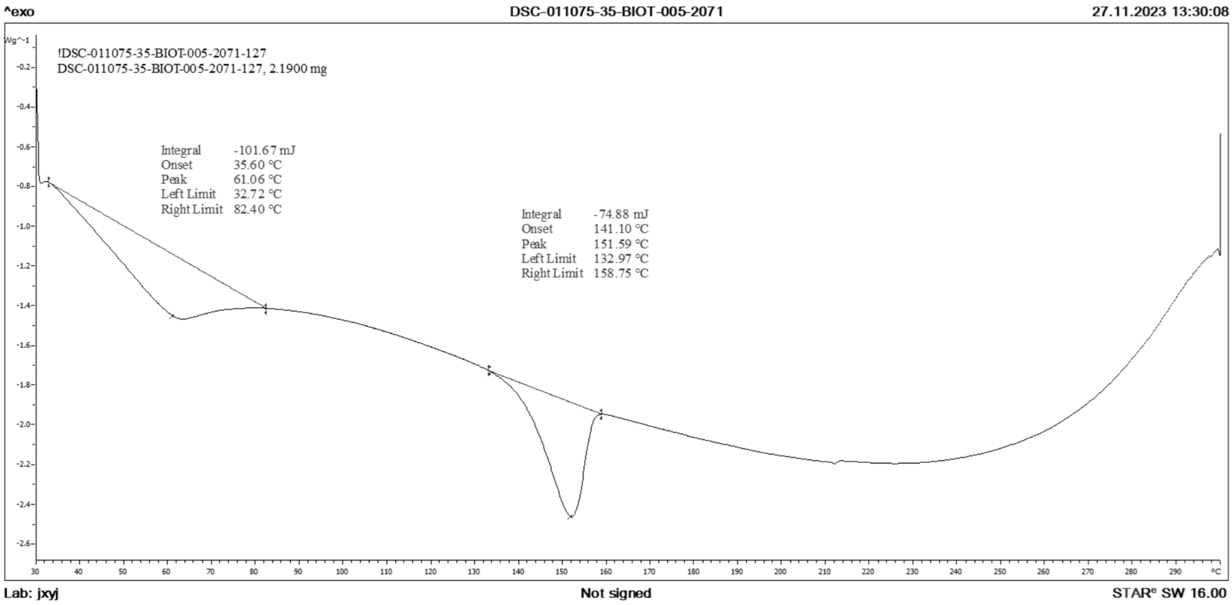
^exo
DSC-011075-35-BIOT-005-2071
27.11.2023 13:30:08
!DSC-011075-35-BIOT-005-2071-127
DSC-011075-35-BIOT-005-2071-127, 2.1900 mg
Integral -101.67 mJ
Onset 35.60 °C
Peak 61.06 °C
Left Limit 32.72 °C
Right Limit 82.40 °C
Integral -74.88 mJ
Onset 141.10 °C
Peak 151.59 °C
Left Limit 132.97 °C
Right Limit 158.75 °C
Lab: jxyj
Not signed
STARe SW 16.00

FIG. 23

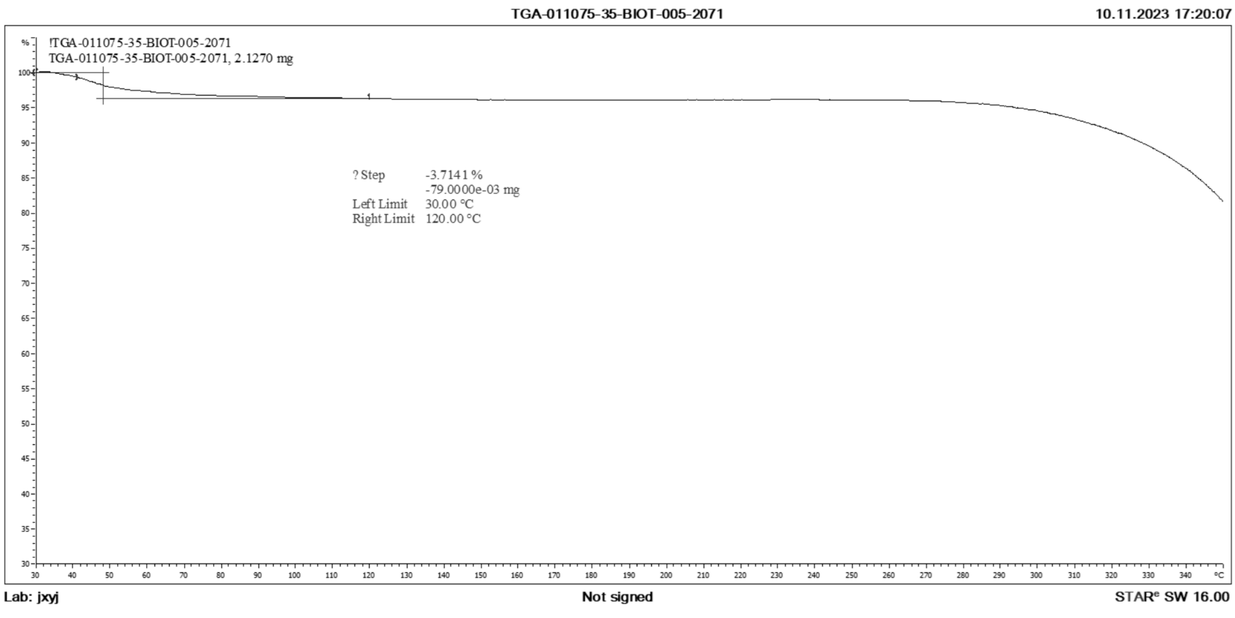
TGA-011075-35-BIOT-005-2071
10.11.2023 17:20:07
!TGA-011075-35-BIOT-005-2071
TGA-011075-35-BIOT-005-2071, 2.1270 mg
? Step -3.7141 %
-79.0000e-03 mg
Left Limit 30.00 °C
Right Limit 120.00 °C
Lab: jxyj
Not signed
STARe SW 16.00

FIG. 24

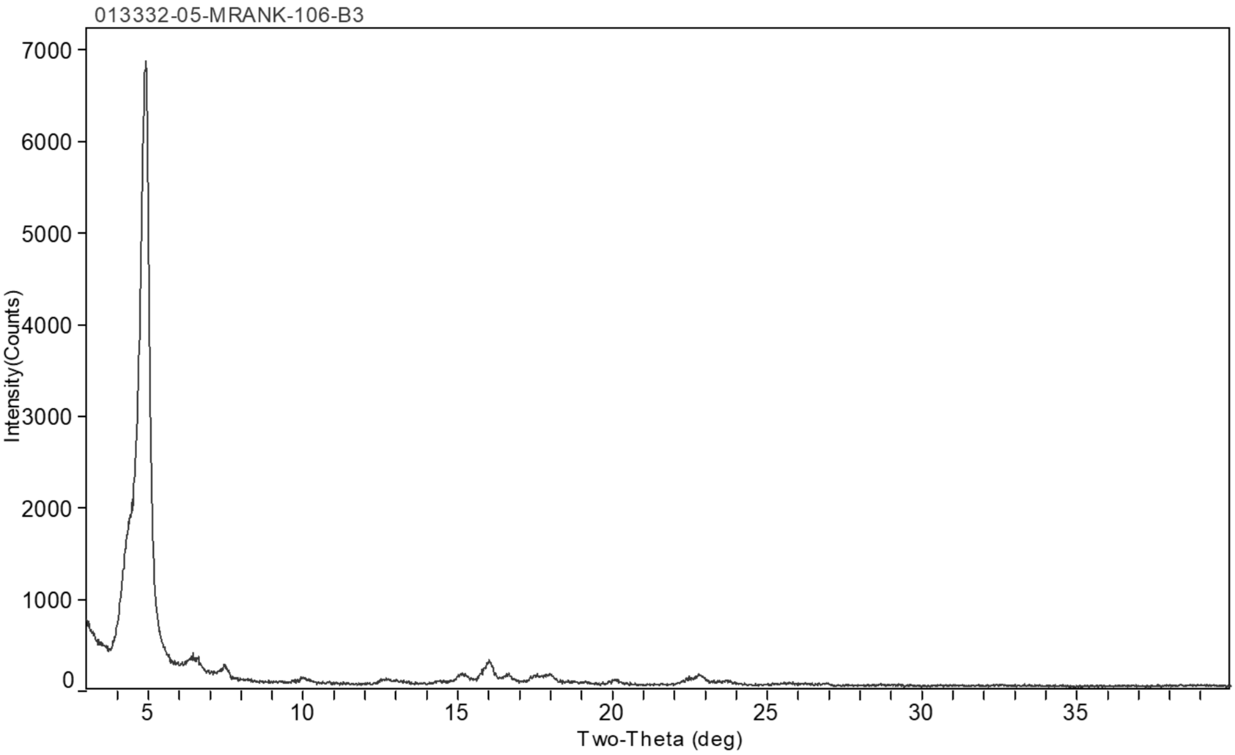
013332-05-MRANK-106-B3
7000
6000
5000
4000
3000
2000
1000
0
Intensity(Counts)
5
10
15
20
25
30
35
Two-Theta (deg)

FIG. 25

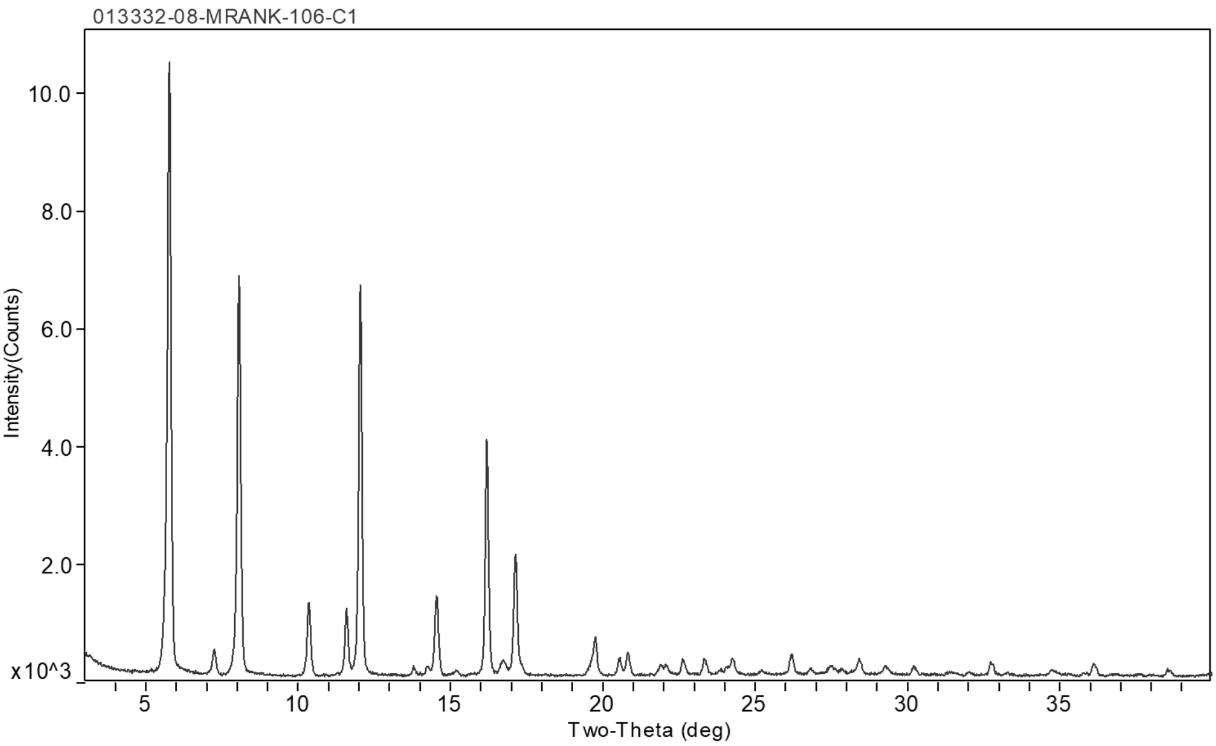
013332-08-MRANK-106-C1
10.0
8.0
6.0
4.0
2.0
x10^3
Intensity(Counts)
5
10
15
20
25
30
35
Two-Theta (deg)

FIG. 26

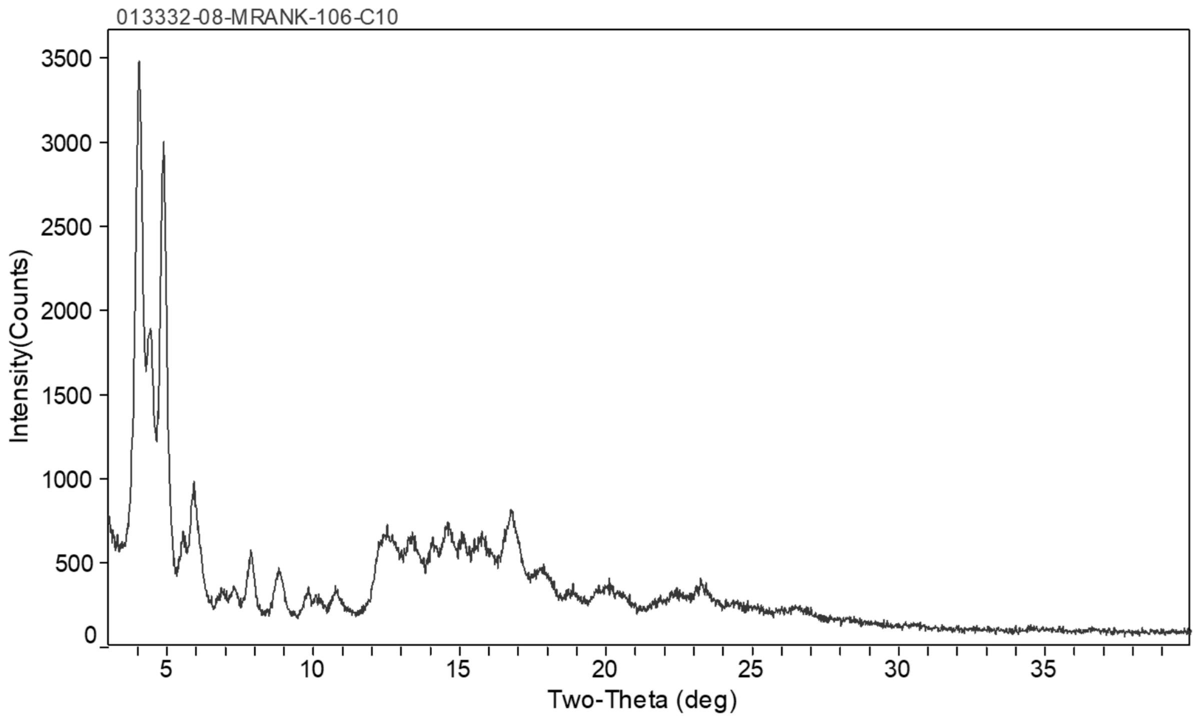
013332-08-MRANK-106-C10
3500
3000
2500
2000
1500
1000
500
0
Intensity(Counts)
5
10
15
20
25
30
35
Two-Theta (deg)

FIG. 27

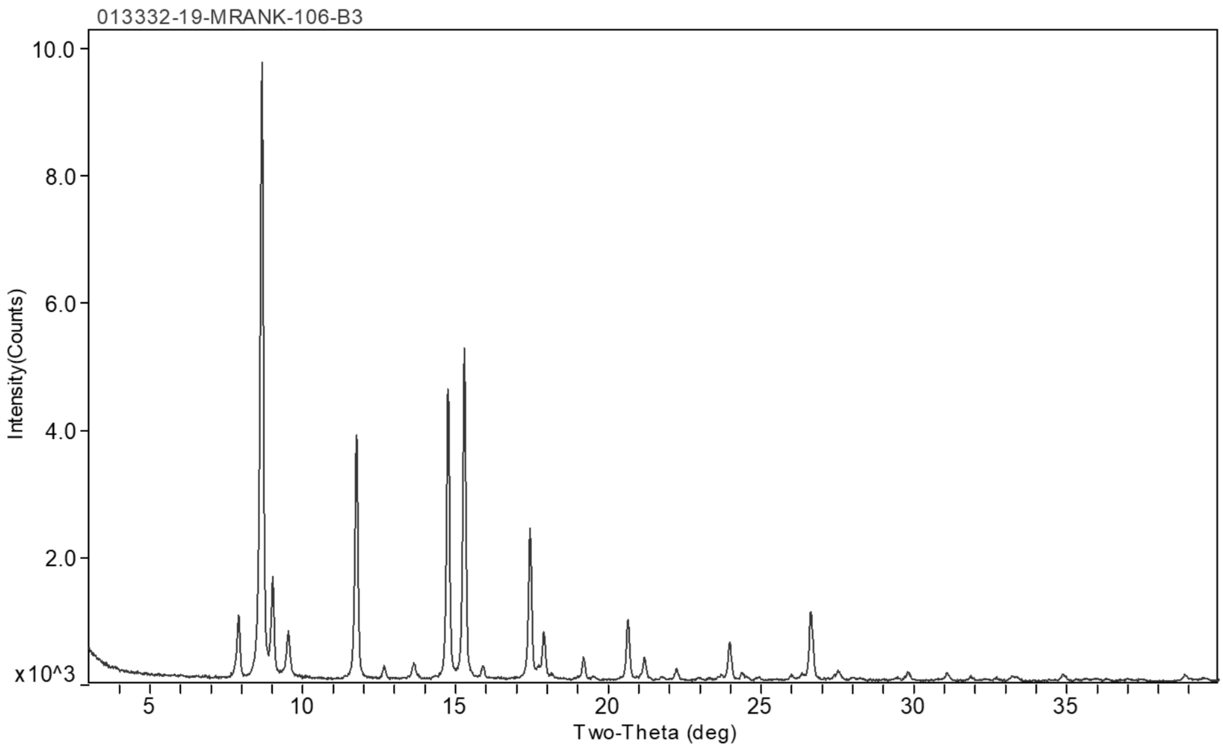
013332-19-MRANK-106-B3
10.0
8.0
6.0
4.0
2.0
x10^3
Intensity(Counts)
5
10
15
20
25
30
35
Two-Theta (deg)

FIG. 28

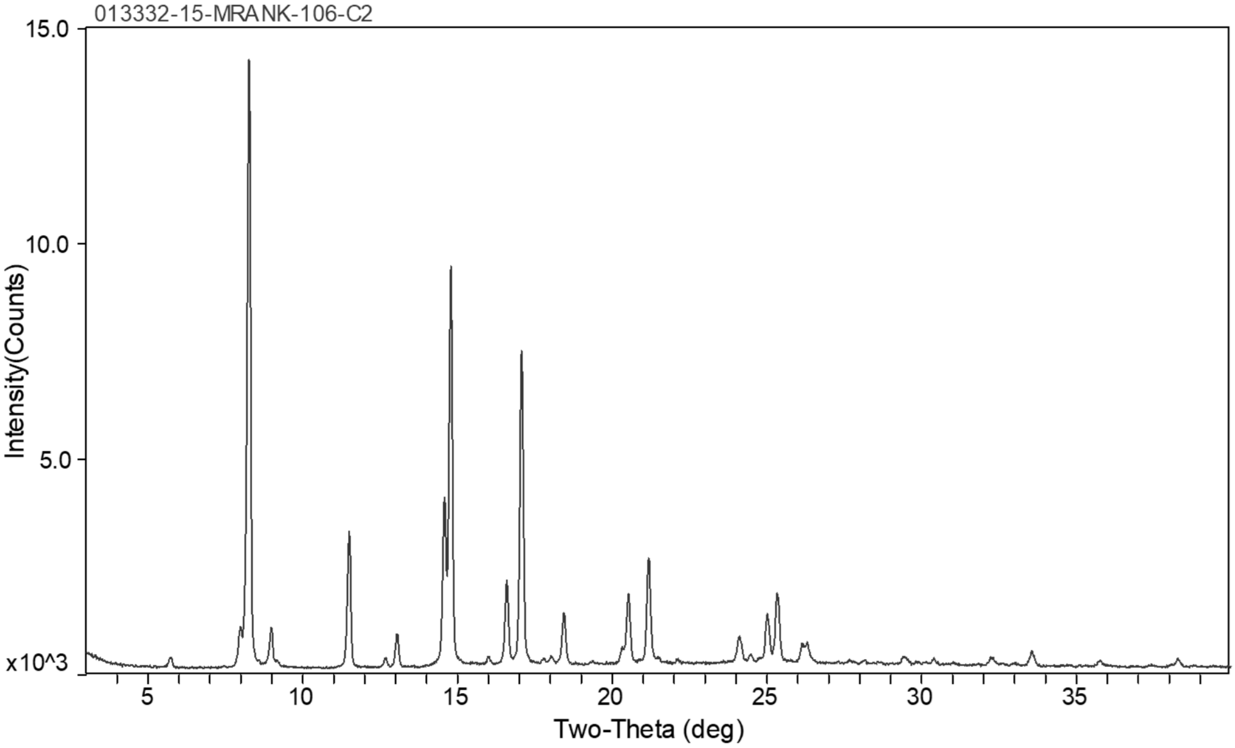
013332-15-MRANK-106-C2
15.0
10.0
5.0
x10^3
Intensity(Counts)
5
10
15
20
25
30
35
Two-Theta (deg)

FIG. 29

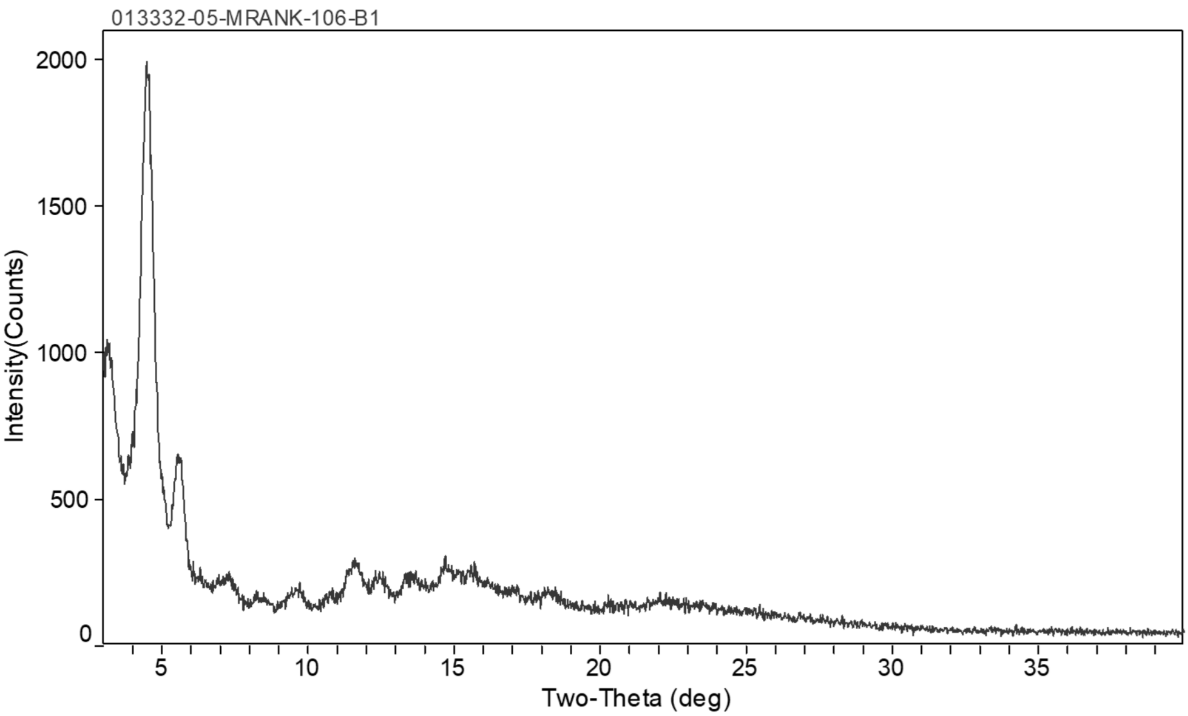
013332-05-MRANK-106-B1
2000
1500
1000
500
0
Intensity(Counts)
5
10
15
20
25
30
35
Two-Theta (deg)

FIG. 30

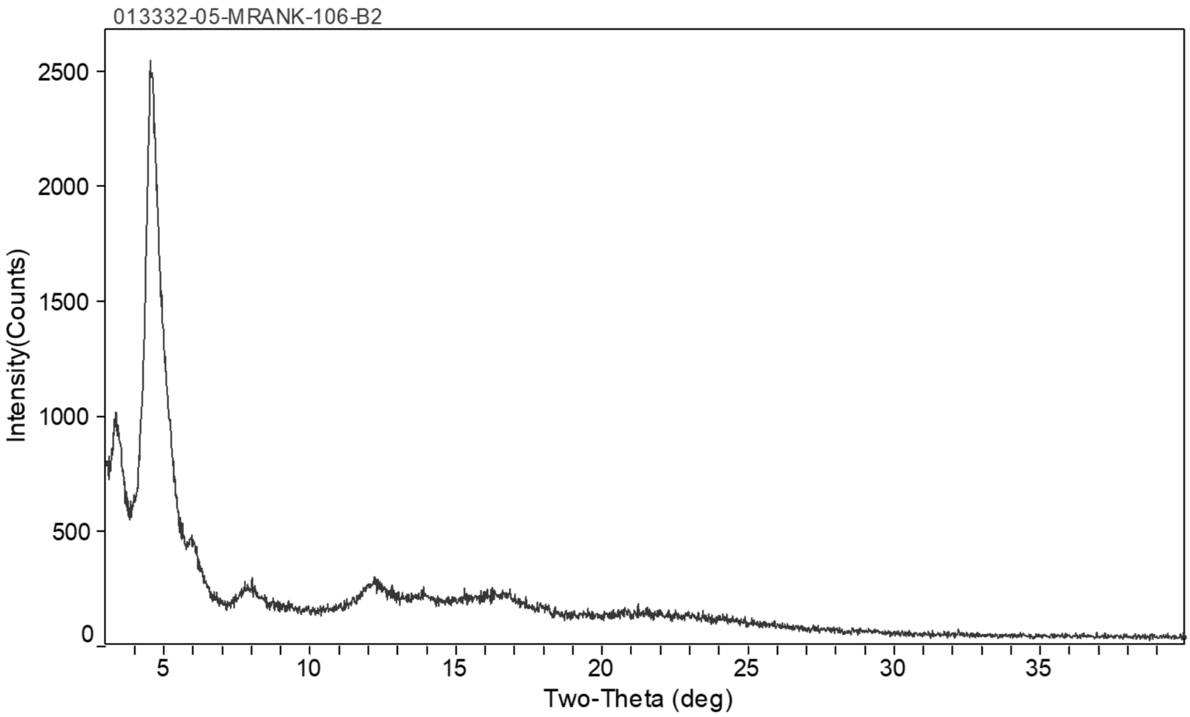
013332-05-MRANK-106-B2
2500
2000
1500
1000
500
0
Intensity(Counts)
5
10
15
20
25
30
35
Two-Theta (deg)

FIG. 31

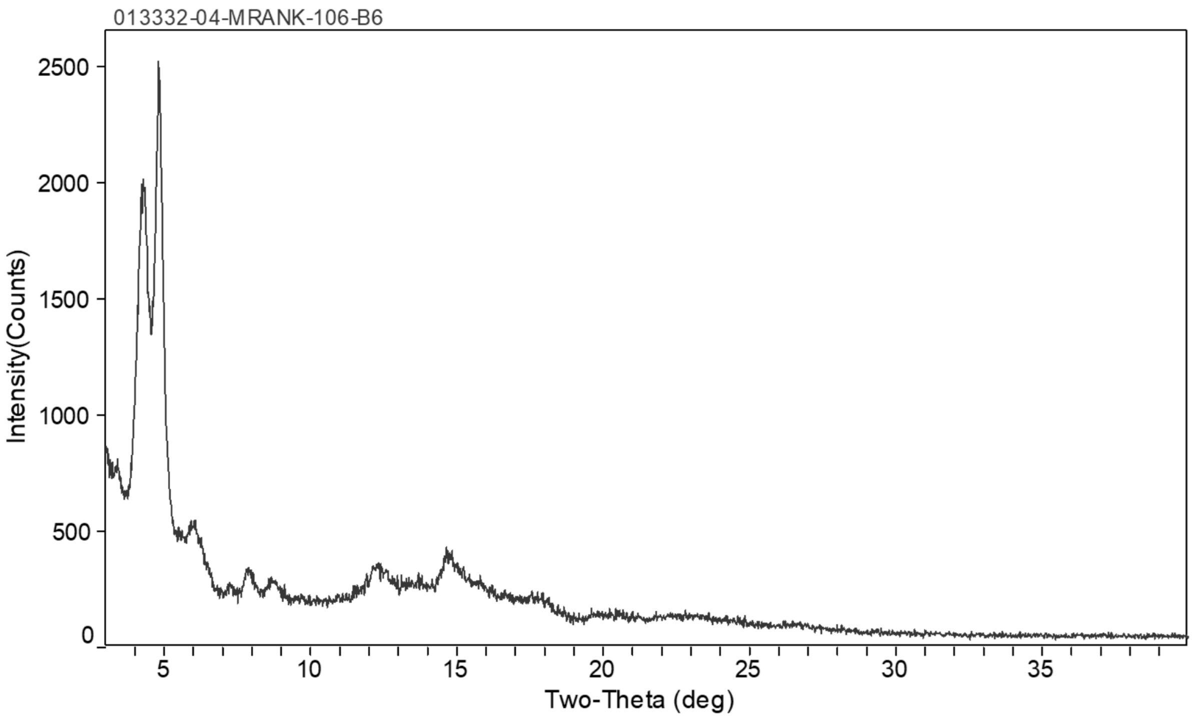
013332-04-MRANK-106-B6
2500
2000
1500
1000
500
0
Intensity(Counts)
5
10
15
20
25
30
35
Two-Theta (deg)

FIG. 32

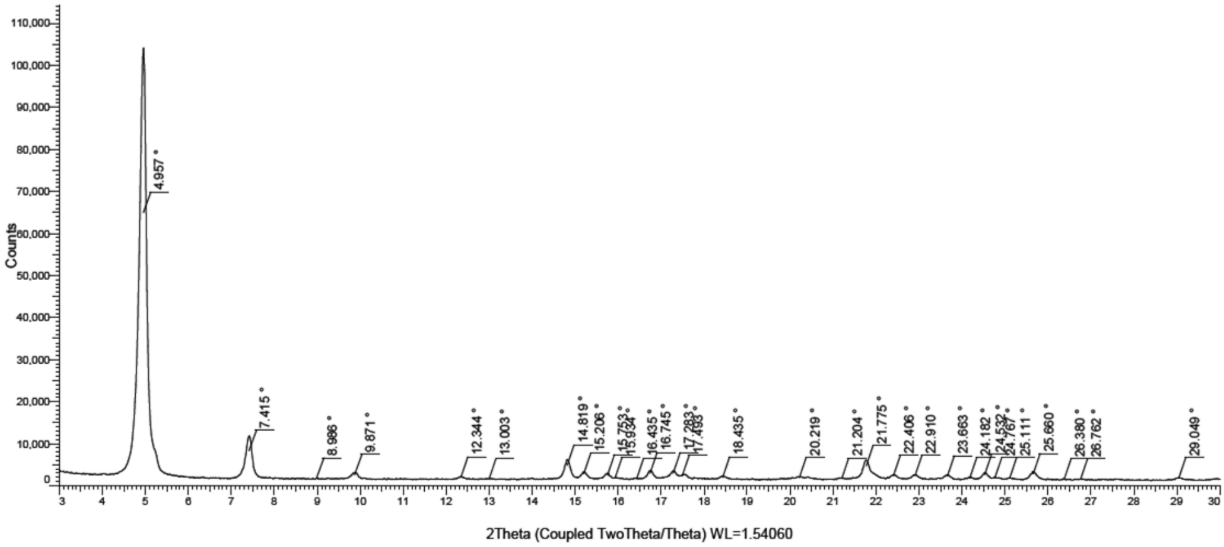
Counts
110,000
100,000
90,000
80,000
70,000
60,000
50,000
40,000
30,000
20,000
10,000
0
4.957 °
7.415 °
8.986 °
9.871 °
12.344 °
13.003 °
14.819 °
15.206 °
15.753 °
15.934 °
16.435 °
16.745 °
17.283 °
17.493 °
18.435 °
20.219 °
21.204 °
21.775 °
22.406 °
22.910 °
23.663 °
24.182 °
24.532 °
24.767 °
25.111 °
25.660 °
26.380 °
26.762 °
29.049 °
3 4 5 6 7 8 9 10 11 12 13 14 15 16 17 18 19 20 21 22 23 24 25 26 27 28 29 30
2Theta (Coupled TwoTheta/Theta) WL=1.54060

FIG. 33

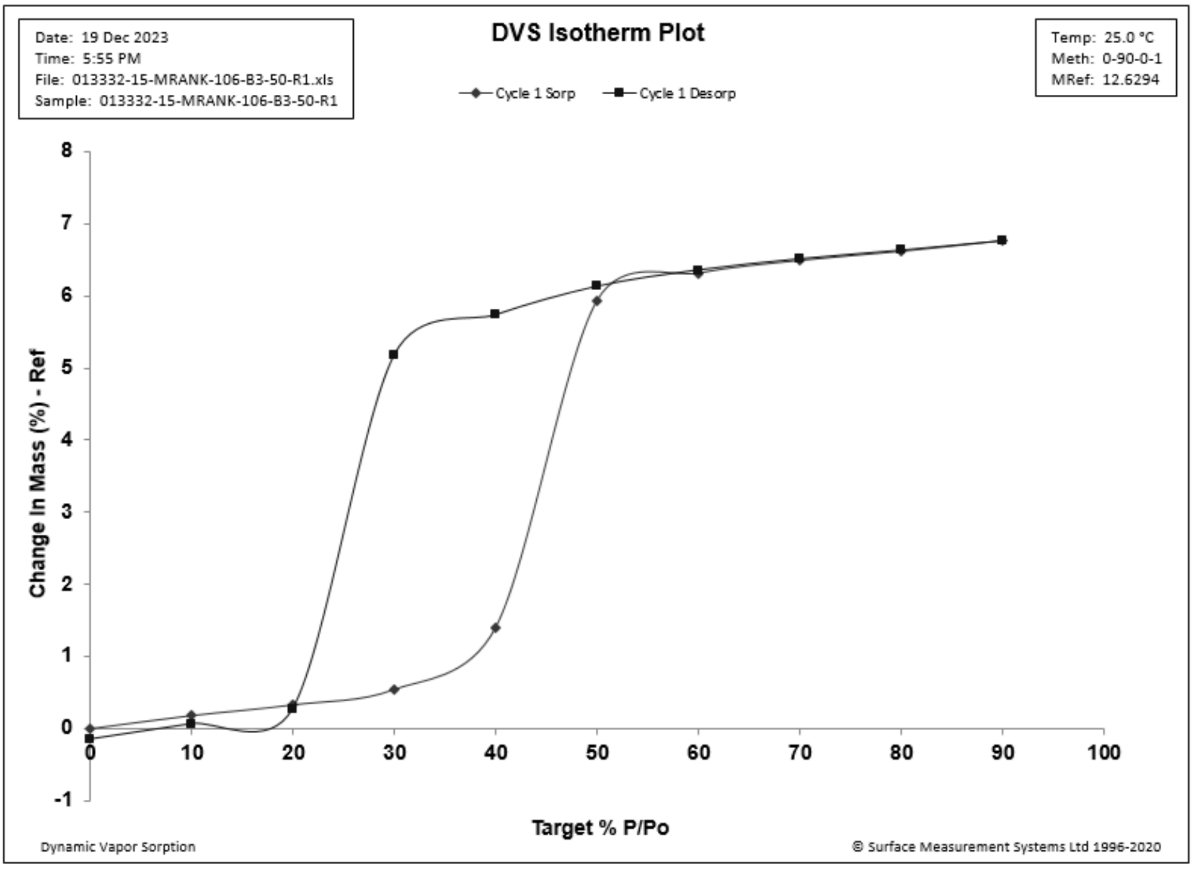
Date: 19 Dec 2023
Time: 5:55 PM
File: 013332-15-MRANK-106-B3-50-R1.xls
Sample: 013332-15-MRANK-106-B3-50-R1
DVS Isotherm Plot
Temp: 25.0 °C
Meth: 0-90-0-1
MRef: 12.6294
Cycle 1 Sorp
Cycle 1 Desorp
Change In Mass (%) - Ref
8
7
6
5
4
3
2
1
0
-1
0 10 20 30 40 50 60 70 80 90 100
Target % P/Po
Dynamic Vapor Sorption
© Surface Measurement Systems Ltd 1996-2020

FIG. 34

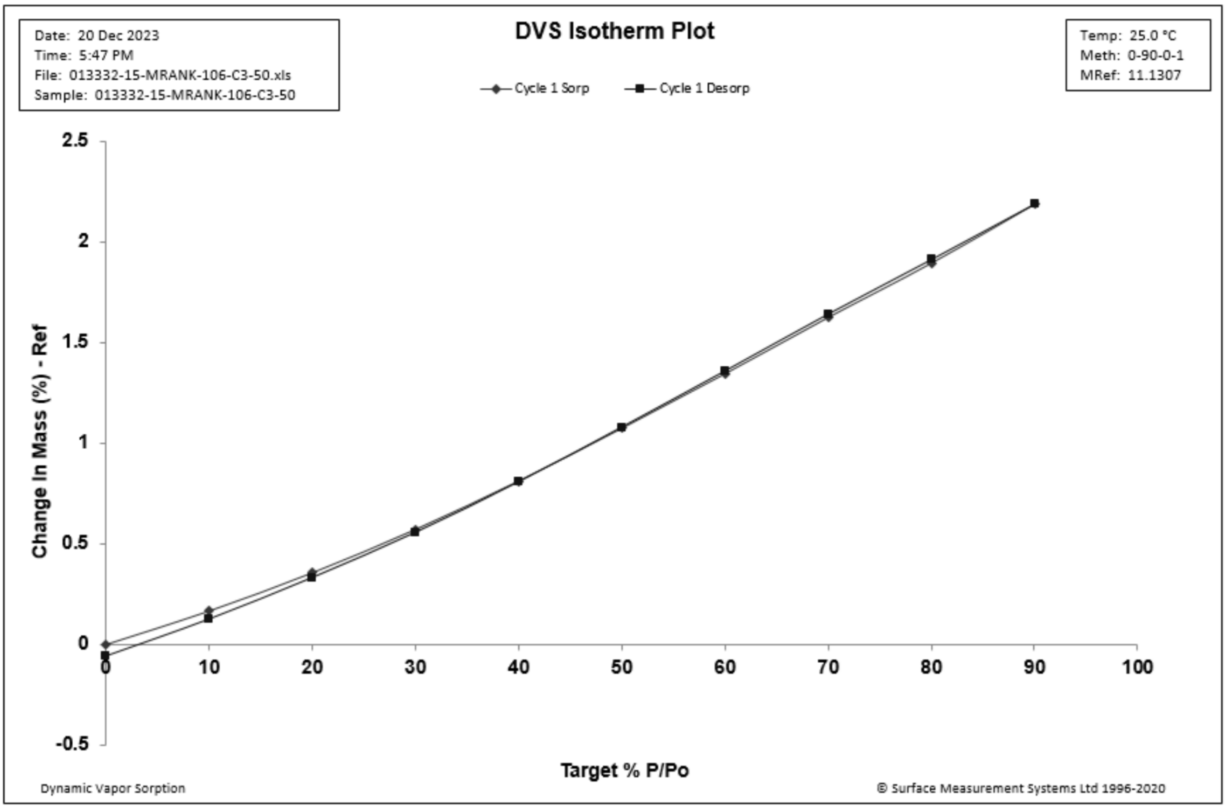
Date: 20 Dec 2023
Time: 5:47 PM
File: 013332-15-MRANK-106-C3-50.xls
Sample: 013332-15-MRANK-106-C3-50
DVS Isotherm Plot
Temp: 25.0 °C
Meth: 0-90-0-1
MRef: 11.1307
Cycle 1 Sorp
Cycle 1 Desorp
Change In Mass (%) - Ref
Target % P/Po
Dynamic Vapor Sorption
© Surface Measurement Systems Ltd 1996-2020

FIG. 35

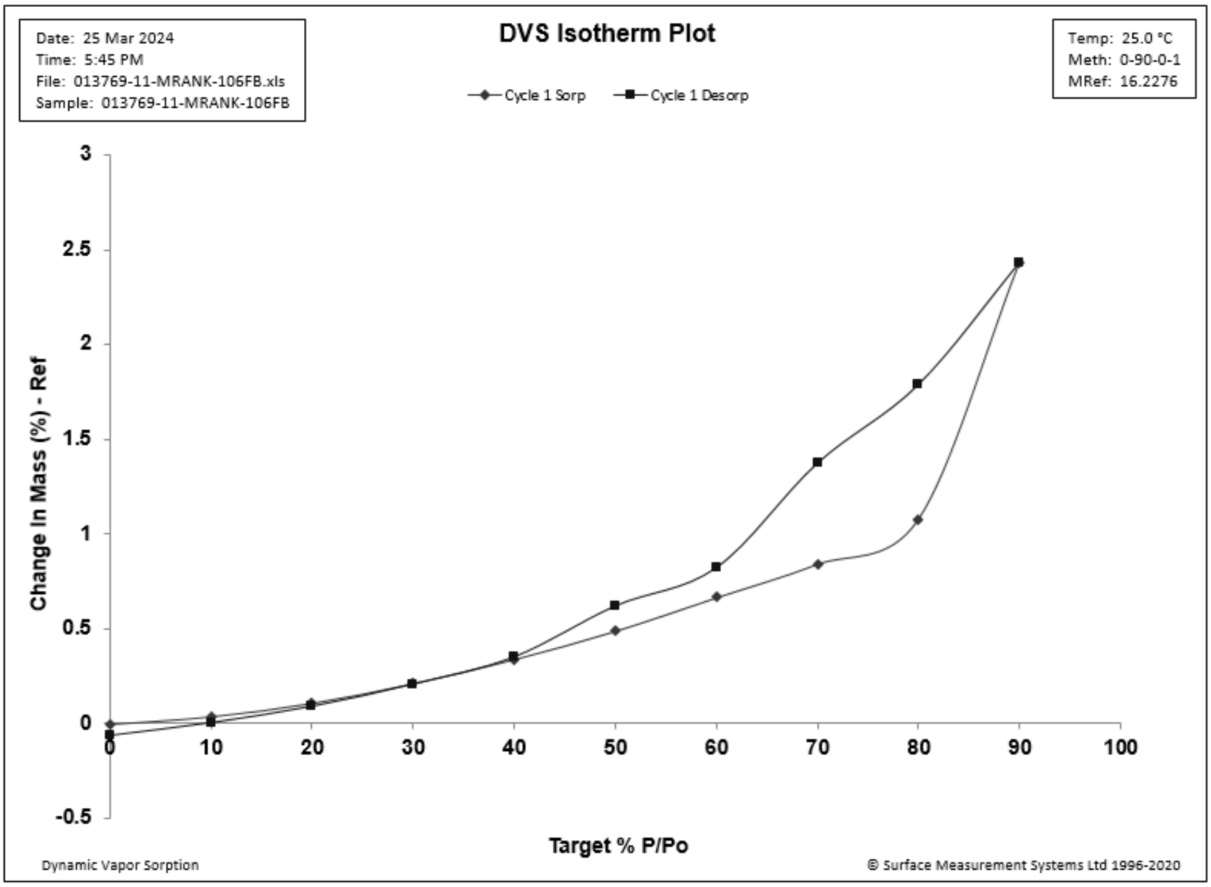
Date: 25 Mar 2024
Time: 5:45 PM
File: 013769-11-MRANK-106FB.xls
Sample: 013769-11-MRANK-106FB
DVS Isotherm Plot
Temp: 25.0 °C
Meth: 0-90-0-1
MRef: 16.2276
Cycle 1 Sorp
Cycle 1 Desorp
Change In Mass (%) - Ref
3
2.5
2
1.5
1
0.5
0
-0.5
0
10
20
30
40
50
60
70
80
90
100
Target % P/Po
Dynamic Vapor Sorption
© Surface Measurement Systems Ltd 1996-2020

FIG. 36

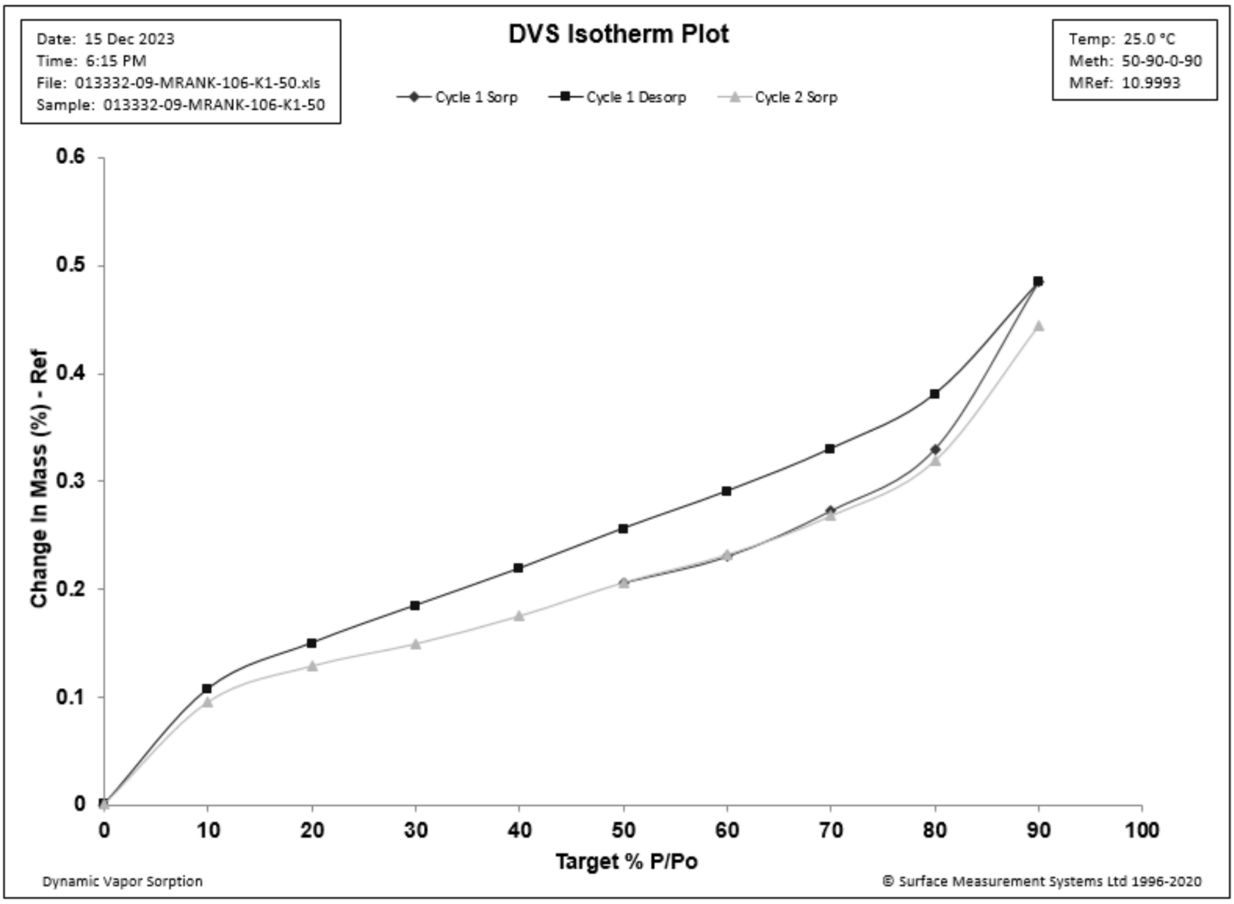
Date: 15 Dec 2023
Time: 6:15 PM
File: 013332-09-MRANK-106-K1-50.xls
Sample: 013332-09-MRANK-106-K1-50
DVS Isotherm Plot
Cycle 1 Sorp
Cycle 1 Desorp
Cycle 2 Sorp
Temp: 25.0 °C
Meth: 50-90-0-90
MRef: 10.9993
Change In Mass (%) - Ref
0.6
0.5
0.4
0.3
0.2
0.1
0
0
10
20
30
40
50
60
70
80
90
100
Target % P/Po
Dynamic Vapor Sorption
© Surface Measurement Systems Ltd 1996-2020

FIG. 37

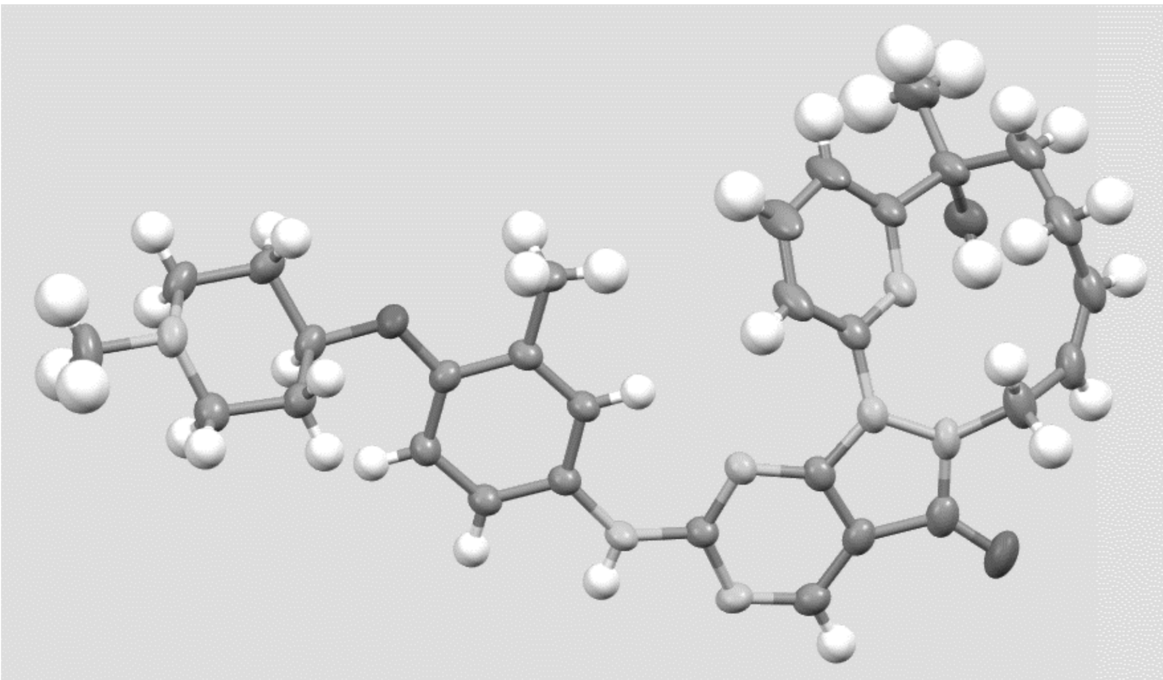

FIG. 38

## INTERNATIONAL SEARCH REPORT

International application No.
**PCT/CN2024/092244**

**A. CLASSIFICATION OF SUBJECT MATTER**

C07D 487/04(2006.01)i; C07D487/14(2006.01)i; A61K31/519(2006.01)i; A61K31/4162(2006.01)i; A61P35/00(2006.01)i; A61P37/00(2006.01)i

According to International Patent Classification (IPC) or to both national classification and IPC

**B. FIELDS SEARCHED**

Minimum documentation searched (classification system followed by classification symbols)

C07D 487/-,C07D 471/-,A61K,A61P

Documentation searched other than minimum documentation to the extent that such documents are included in the fields searched

Electronic data base consulted during the international search (name of data base and, where practicable, search terms used)

CNKI, CNABS, CNTXT, ENTXT, DWPI, CAPLUS, REGISTRY: Wee, 抑制剂, inhibit, 嘧啶, 大杂环, pyrimidin, 根据式I进行的结构式限制检索, structural formula limited search based on formula I.

**C. DOCUMENTS CONSIDERED TO BE RELEVANT**

| Category* | Citation of document, with indication, where appropriate, of the relevant passages | Relevant to claim No. |
|---|---|---|
| PX | WO 2023217201 A2 (MINDRANK AI LTD.) 16 November 2023 (2023-11-16)<br>embodiments 23-25, 28, 29, 89, 90, 130, 134, 140, 141, 143, 144, and 146, claims 9-10, general formula I and 2-IB, description, page 14, first line, and description, page 17, definitions of R4 and R5. | 1-11 |
| X | WO 2019085933 A1 (MEDSHINE DISCOVERY INC.) 09 May 2019 (2019-05-09)<br>claims 25-27 | 1, 2, 4, 5, 6, 10, 11 |
| X | US 2021403451 A1 (QIAN WENYUAN; YANG CHUNDAO; LI ZHENGWEI; LI JIAN; CHEN SHUHUI) 30 December 2021 (2021-12-30)<br>claims 20-23, and general formula I | 1, 2, 4, 5, 6, 10, 11 |
| X | WO 2020221358 A1 (SHIJIAZHUANG SAGACITY NEW DRUG DEVELOPMENT CO., LTD.) 05 November 2020 (2020-11-05)<br>embodiment 1, and experiment 2-5 | 1, 2, 4, 5, 6, 10, 11 |
| PX | WO 2023083194 A1 (HANGZHOU GLUBIO PHARMACEUTICAL CO., LTD.) 19 May 2023 (2023-05-19)<br>see embodiment 141, Intermediates 141-5 | 1, 2, 4, 5, 6 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 August 2024** | **04 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/092244**

| Patent document cited in search report | Publication date (day/month/year) | Patent family member(s) | Publication date (day/month/year) |
|---|---|---|---|
| WO 2023217201 A2 | 16 November 2023 | WO 2023217201 A3 | 28 December 2023 |
| WO 2019085933 A1 | 09 May 2019 | CA 3080842 A1 | 09 May 2019 |
| | | US 2020325145 A1 | 15 October 2020 |
| | | US 11613545 B2 | 28 March 2023 |
| | | SG 11202003974 XA | 28 May 2020 |
| | | BR 112020008664 A2 | 27 October 2020 |
| | | BR 112020008664 A8 | 17 January 2023 |
| | | FI 3712150 T3 | 31 July 2024 |
| | | EP 3712150 A1 | 23 September 2020 |
| | | EP 3712150 A4 | 10 March 2021 |
| | | EP 3712150 B1 | 12 June 2024 |
| | | RU 2020117443 A3 | 01 December 2021 |
| | | IL 274357 A | 30 June 2020 |
| | | IL 274357 B1 | 01 January 2023 |
| | | IL 274357 B2 | 01 May 2023 |
| | | JP 2021501191 A | 14 January 2021 |
| | | JP 7290638 B2 | 13 June 2023 |
| | | LT 3712150 T | 12 August 2024 |
| | | DK 3712150 T3 | 29 July 2024 |
| | | AU 2018361010 A1 | 11 June 2020 |
| | | AU 2018361010 B2 | 12 January 2023 |
| US 2021403451 A1 | 30 December 2021 | WO 2020083404 A1 | 30 April 2020 |
| | | JP 2022505756 A | 14 January 2022 |
| | | JP 7481336 B2 | 10 May 2024 |
| | | EP 3875460 A1 | 08 September 2021 |
| | | EP 3875460 A4 | 20 July 2022 |
| WO 2020221358 A1 | 05 November 2020 | EP 3964510 A1 | 09 March 2022 |
| | | EP 3964510 A4 | 26 April 2023 |
| | | EP 3964510 B1 | 03 July 2024 |
| | | LT 3964510 T | 12 August 2024 |
| | | AU 2020266956 A1 | 28 October 2021 |
| | | SG 11202111315 XA | 29 November 2021 |
| | | US 2022220120 A1 | 14 July 2022 |
| | | JP 2022530812 A | 01 July 2022 |
| | | IL 287472 A | 01 December 2021 |
| | | CA 3138240 A1 | 05 November 2020 |
| | | FI 3964510 T3 | 24 July 2024 |
| | | BR 112021021230 A2 | 21 December 2021 |
| | | BR 112021021230 A8 | 25 April 2023 |
| | | DK 3964510 T3 | 29 July 2024 |
| WO 2023083194 A1 | 19 May 2023 | CA 3237756 A1 | 19 May 2023 |
| | | AU 2022384370 A1 | 13 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023093349 W


### Non-patent literature cited in the description


- **BERGE et al.** Pharmaceutical Salts. *Journal of Pharmaceutical Science*, 1977, vol. 66, 1-19
- Theory and Origin of Polymorphism. *Polymorphism in Pharmaceutical Solids*, 1999, ISBN 8247-0237
- **MAEHR**. *J. Chem. Ed.*, 1985, vol. 62, 114-120
- Remington: The Science and Practice of Pharmacy. Lippincott, Williams & Wilkins, 2005